# EUROPEAN PATENT APPLICATION

(11) **EP 2 096 108 A1**
(43) Date of publication of application: **02.09.2009**
(21) Application number: 09250537.9
(22) Date of filing: 27.02.2009
(51) Int. Cl.: C07D 213/74, C07D 237/20, C07D 239/42, C07D 241/20, C07D 251/42, C07D 307/66, C07D 333/36, C07D 401/14, C07D 403/14, C07D 405/14, C07D 409/14, C09K 11/06, H05B 33/14

(54) **Novel organic electroluminescent compounds and organic electroluminscent device using the same**

(30) Priority: 29.02.2008 KR 20080019367
(71) Applicant: Gracel Display Inc., Seoul 133-833 (KR)
(72) Inventor: Kim, Chi Sik, Seoul 156-825 (KR); Cho, Young Jun, Seoul 136-060 (KR); Kwon, Hyuck Joo, Seoul 130-100 (KR); Kim, Bong Ok, Seoul 135-090 (KR); Kim, Sung Min, Seoul-city 157-886 (KR); Yoon, Seung Soo, Seoul 135-884 (KR)
(74) Representative: Kent, Venetia Katherine

(57) **Abstract**

The present invention relates to novel organic electroluminescent compounds, and organic electroluminescent devices employing the same in an electroluminescent layer. Specifically, the organic electroluminescent compounds according to the invention are **characterized in that** they are represented by Chemical Formula (1) or Chemical Formula (2):

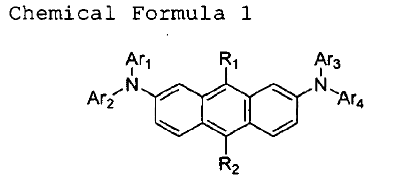

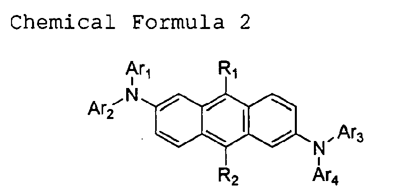

Since the organic electroluminescent compounds according to the invention have good luminous efficiency and excellent life property of material, OLED's having high color purity, high luminance and long life can be manufactured therefrom.

## Description

The present invention relates to novel organic electroluminescent compounds, and organic electroluminescent devices employing the same in an electroluminescent layer. Specifically, the organic electroluminescent compounds according to the invention are **characterized in that** they are represented by Chemical Formula (1) or Chemical Formula (2): 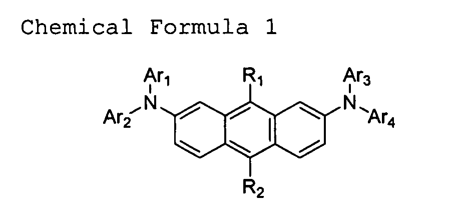 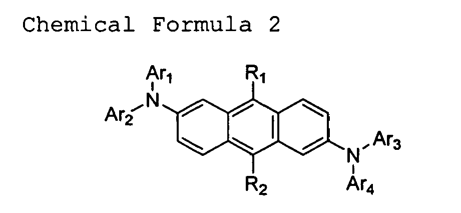 wherein,
R₁ and R₂ independently represent hydrogen, deuterium, (C1-C60)alkyl, (C2-C60)alkenyl, (C2-C60)alkynyl, (C3-C60)cycloalkyl, (C4-C60)tricycloalkyl, (C7-C60)bicycloalkyl, (C6-C60)aryl, (C4-C60)heteroaryl, 5- or 6-membered heterocycloalkyl containing one or more heteroatom(s) selected from N, O and S, spirobifluorenyl, halogen, cyano, (C1-C60)alkoxy, tri(C1-C60)alkylsilyl, di(C1-C60)alkyl(C6-C60)arylsilyl or tri(C6-C60)arylsilyl; and the alkyl, alkenyl, alkynyl, cycloalkyl, tricycloalkyl, bicycloalkyl, aryl or heteroaryl of R₁ and R₂ may be further substituted by one or more substituent(s) selected from deuterium, (C1-C60)alkyl, (C1-C60)alkoxy, halogen, tri(C1-C60)alkylsilyl, di(C1-C60)alkyl(C6-C60)arylsilyl, tri(C6-C60)arylsilyl, cyano, (C6-C60)aryl, (C6-C60)ar(C1-C60)alkyl and (C6-C60)ar(C1-C60)alkoxy; and
Ar₁ through Ar₄ independently represent 5- or 6-membered heteroaryl containing from 1 to 4 heteroatom(s) selected from N, O and S, provided that at least two of Ar₁, Ar₂, Ar₃ and Ar₄ represent pyridyl if the heteroaryl of Ar₁ through Ar₄ represents pyridyl.

### BACKGROUND OF THE INVENTION

The most important factor in developing organic electroluminescent devices of high efficiency and long life is development of electroluminescent material of high performance. In view of current development of electroluminescent material, green electroluminescent materials show superior electroluminescent property to red or blue electroluminescent materials. However, conventional green electroluminescent materials still have many problems to achieve manufacturing panels of large size with low power consumption. In view of practical efficiency and life, various kinds of electroluminescent materials for green have been reported up to now. Though they exhibit from 2 to 5 times of electroluminescent property as compared to red or blue electroluminescent materials, development of green electroluminescent material is getting challenged by the improvement of properties of red or blue electroluminescent material. In the meanwhile, enhancement of device life of the green material is still insufficient, so that a green electroluminescent material providing long life is seriously required.

As green fluorescent material, a coumarin derivative (Compound D), a quinacridone derivative (Compound E), DPT (Compound F) and the like have been known. Compound D is the structure of C545T that is the most widely used coumarin derivative up to the present. In general, those materials are doped by using Alq as the host, at a concentration of several % to about several ten %, to form an electroluminescent device.

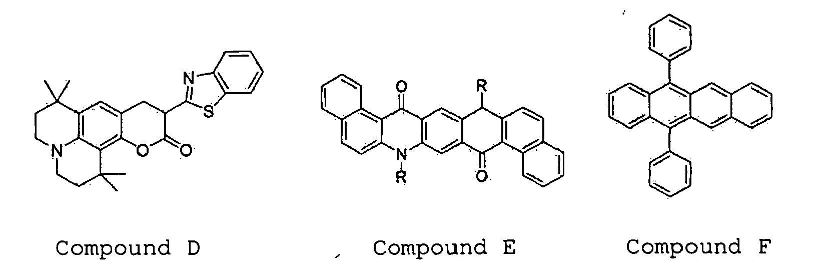

Japanese Patent Laid-Open No. 2001-131541 discloses bis(2,6-diarylamino)-9,10-diphenylanthracene derivatives represented by Compound G shown below, wherein diarylamino groups are directly substituted at 2- and 6-position of anthracene, respectively.

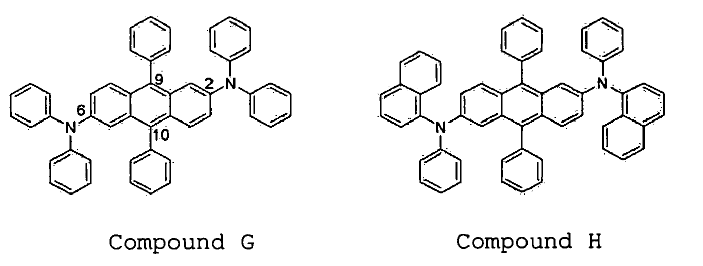

Japanese Patent Laid-Open No. 2003-146951 (which discloses compounds for a hole transport layer) does not mention the compounds wherein diarylamino groups are directly substituted at 2- and 6-position, respectively, but simply describing the compounds having phenyl substituents at 9- and 10-position of anthracene. As considering that Japanese Patent Laid-Open No. 2003-146951 indicated the problem of Compound (H) (wherein diarylamino groups are directly substituted at 2-and 6-position of the anthracene ring, respectively) having poor luminous efficiency, it is found that the invention of Japanese Patent Laid-Open No. 2003-146951 did not recognize the compounds other than those having phenyl substituents at 9- and 10-position of anthracene.

In the meanwhile, Japanese Patent Laid-Open No. 2004-91334 suggested the organic electroluminescent compounds represented by Compound (J), which overcomes poor luminous efficiency of conventional compounds but exhibits low ionization potential and excellent hole transportation, by further substituting the aryl group of the diarylamino group with diarylamino group, even though the diarylamino groups are directly substituted on the anthracene group.

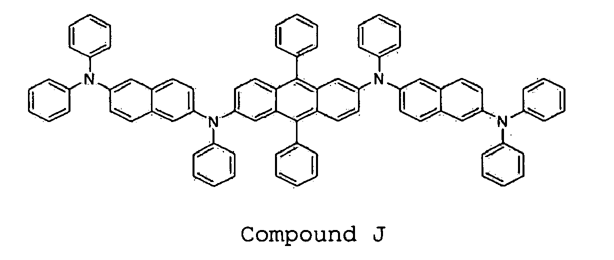

The compounds suggested by Japanese Patent Laid-Open No. 2004-91334 (applied as a hole transport layer), however, show the problem of shortened operation life as a hole transport layer because of too many amine functional groups, even though they showed lowered ionization potential due to many amine functional groups and overcame the problem of increase in hole transporting property.

### [Summary of Invention]

The present inventors found that incorporation of alkyl, alkenyl, alkynyl, cycloalkyl, adamantyl, bicycloalkyl, aryl, heteroaryl, heterocycloalkyl or spirobifluorenyl at 9- and 10-position of anthracene, with direct substitution of amino groups substituted by two 5- or 6-membered heteroaryls at 2-and 6-position, or 2- and 7-position of anthracene, respectively, provides far improved electroluminescent properties to the compounds, and completed the present invention.

Thus, the inventors have intensively endeavored to overcome the problems described above and to develop a novel electroluminescent compound which can realize an organic electroluminescent device having excellent color purity and luminous efficiency and noticeably improved device life.

The object of the invention is to provide novel organic electroluminescent compounds wherein alkyl, alkenyl, alkynyl, cycloalkyl, adamantyl, bicycloalkyl, aryl, heteroaryl, heterocycloalkyl or spirobifluorenyl group is incorporated at 9- and 10-position of anthracene, and amino groups having two 5- or 6-membered heteroaryl substituents on each of them are directly substituted at the 2- and 6-, or 2- and 7-position of anthracene.

Another object of the present invention is to provide an organic electroluminescent device comprising an electroluminescent region which employs one or more compound(s) selected from anthracene derivatives and benz[a]anthracene derivatives as an electroluminescent host, in addition to one or more organic electroluminescent compound(s) as mentioned above.

Still another object of the invention is to provide organic electroluminescent compounds exhibiting excellent color purity with high luminous efficiency and very good device life, and to provide organic electroluminescent devices comprising the novel organic electroluminescent compounds.

The present invention relates to novel organic electroluminescent compounds, and organic electroluminescent devices employing the same in an electroluminescent layer. Specifically, the organic electroluminescent compounds according to the invention are **characterized in that** they are represented by Chemical Formula (1) or Chemical Formula (2): 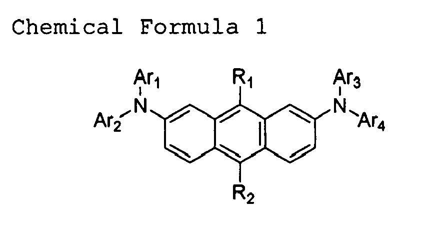 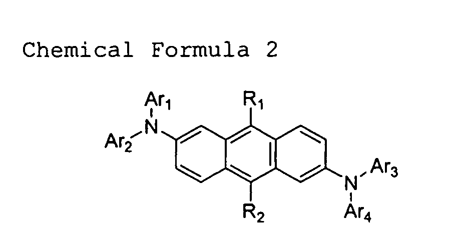 wherein,
R₁ and R₂ independently represent hydrogen, deuterium, (C1-C60)alkyl, (C2-C60)alkenyl, (C2-C60)alkynyl, (C3-C60)cycloalkyl, (C4-C60)tricycloalkyl, (C7-C60)bicycloalkyl, (C6-C60)aryl, (C4-C60)heteroaryl, 5- or 6-membered heterocycloalkyl containing one or more heteroatom(s) selected from N, O and S, spirobifluorenyl, halogen, cyano, (C1-C60)alkoxy, tri(C1-C60)alkylsilyl, di(C1-C60)alkyl(C6-C60)arylsilyl or tri(C6-C60)arylsilyl; and the alkyl, alkenyl, alkynyl, cycloalkyl, tricycloalkyl, bicycloalkyl, aryl or heteroaryl of R₁ and R₂ may be further substituted by one or more substituent(s) selected from deuterium, (C1-C60)alkyl, (C1-C60)alkoxy, halogen, tri(C1-C60)alkylsilyl, di(C1-C60)alkyl(C6-C60)arylsilyl, tri(C6-C60)arylsilyl, cyano, (C6-C60)aryl, (C6-C60)ar(C1-C60)alkyl and (C6-C60)ar(C1-C60)alkoxy; and

Ar₁ through Ar₄ independently represent 5- or 6-membered heteroaryl containing from 1 to 4 heteroatom(s) selected from N, O and S, provided that at least two of Ar₁, Ar₂, Ar₃ and Ar₄ represent pyridyl if the heteroaryl of Ar₁ through Ar₄ represents pyridyl.

### BRIEF DESCRIPTION OF DRAWINGS

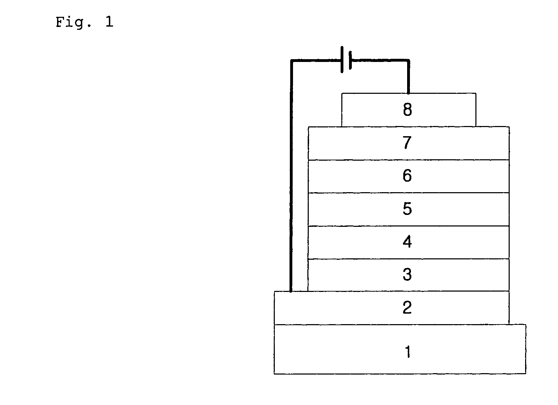

Fig. 1 is a cross-sectional view of an organic light emitting diode (OLED).

### DETAILED DESCRIPTION OF THE INVENTION

Referring now to the Drawings, Fig. 1 illustrates a cross-sectional view of an OLED of the present invention comprising a Glass 1, Transparent electrode 2, Hole injection layer 3, Hole transport layer 4, Electroluminescent layer 5, Electron transport layer 6, Electron injection layer 7 and Al cathode 8.

The term "heteroaryl" of R₁ and R₂ means an aryl group containing from 1 to 4 heteroatom(s) selected from N, O and S as the aromatic cyclic backbone atom(s), and carbon atom(s) for remaining aromatic cyclic backbone atoms. The heteroaryl may be a 5- or 6-membered monocyclic heteroaryl or a polycyclic heteroaryl which is fused with one or more benzene ring(s), and may be partially saturated. The heteroaryl group may comprise a bivalent aryl group, of which the heteroatoms may be oxidized or quaternized to form N-oxide and quaternary salt. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinolizinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The term "heteroaryl" of Ar₁ through Ar₄ means an aryl group containing from 1 to 4 heteroatom(s) selected from N, O and S as the 5- or 6-membered aromatic cyclic backbone atom(s), and carbon atom(s) for remaining aromatic cyclic backbone atoms. The heteroaryl may be a 5- or 6-membered monocyclic heteroaryl, and may be partially saturated. Specific examples include furyl, thiophenyl, pyrrolyl, pyranyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl and pyridazinyl.

The term "alkyl", "alkoxy" and other substituents comprising "alkyl" moiety may be linear or branched one.

The term "aryl" described herein means an organic radical derived from aromatic hydrocarbon via elimination of one hydrogen atom. Each ring comprises a monocyclic or fused ring system containing from 4 to 7, preferably from 5 to 6 cyclic atoms. The term "aryl" even includes multiple aryl groups connected via single bonds. Specific examples include phenyl, naphthyl, biphenyl, anthryl, indenyl, fluorenyl, phenanthryl, triphenylenyl, pyrenyl, perylenyl, chrysenyl, naphthacenyl and fluoranthenyl, but they are not restricted thereto.

The naphthyl may be 1-naphthyl or 2-naphthyl; the anthryl may be 1-anthryl, 2-anthryl or 9-anthryl; and the fluorenyl may be 1-fluorenyl, 2-fluorenyl, 3-fluorenyl, 4-fluorenyl or 9-fluorenyl.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms. The substituents comprising "(C3-C60)cycloalkyl" moiety may contain 3 to 60 carbon atoms, 3 to 20 carbon atoms, or 3 to 7 carbon atoms. The substituents comprising "(C2-C60)alkenyl or alkynyl" moiety may contain 2 to 60 carbon atoms, 2 to 20 carbon atoms, or 2 to 10 carbon atoms.

The organic electroluminescent compounds of Chemical Formula (1) or Chemical Formula (2) according to the present invention are characterized by their structure of novel concept which maximizes luminous efficiency of green electroluminescent devices resulted from those compounds and their device life, being unexpected by conventional inventions.

The organic electroluminescent compounds of Chemical Formula (1) or Chemical Formula (2) according to the invention adopted a structure showing an efficient energy transfer mechanism between the host and dopant, which can realize electroluminescent property with a reliably high efficiency on the basis of improvement in electron density distribution. The structure of the novel compounds according to the present invention can provide a skeletal which can also tune an electroluminescent property with high efficiency in the range from blue to red, not only for green electroluminescence. Beyond the concept of using a host material with high electron conductivity such as Alq, the invention applies a host having appropriate balance of hole conductivity and electron conductivity, thereby overcoming the problems of conventional materials including low initial efficiency and short lifetime, and ensures electroluminescent properties with high performance having high efficiency and long life for each color.

In Chemical Formula (1) or (2), R₁ and R₂ independently represent methyl, ethyl, n-propyl, i-propyl, n-butyl, i-butyl, tert-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, benzyl, ethenyl, phenylethenyl, ethynyl, phenylethynyl, cyclopentyl, cyclohexyl, cycloheptyl, cyclooctyl, bicyclo[1.1.0]butyl, bicyclo[1.1.1]pentyl, bicyclo[2.1.0]pentyl, bicyclo[2.1.1]hexyl, bicyclo[3.2.0]hexyl, bicyclo[3.2.0]heptyl, bicyclo[3.1.1]heptyl, bicyclo[4.1.0]heptyl, bicyclo[2.2.1]heptyl, octahydropentalenyl, bicyclo[2.2.2]octyl, bicyclo[4.2.0]octyl, bicyclo[4.1.1]octyl, bicyclo[3.2.1]octyl, octahydro-1H-indenyl, bicyclo[5.2.0]nonyl, bicyclo[4.2.1]nonyl, bicyclo[3.3.1]nonyl, bicyclo[3.3.2]decyl, bicyclo[4.3.1]decyl, bicyclo[4.2.2]decyl, decahydronaphthalenyl, bicyclo[3.3.3]undecyl, bicyclo[4.3.2]undecyl, bicyclo[4.3.3]dodecyl, 4-pentylbicyclo[2.2.2]octyl, tricyclo[2.2.1.0]heptyl, tricyclo[5.2.1.0^{2,6}]decyl, tricyclo[5.3.1.1]dodecyl, tricyclo[5.4.0.0^{2,9}]undecyl, adamantyl, tricyclo[5.3.2.0^{4,9}]dodecyl, tricyclo[4.4.1.1^{1,5}]dodecyl, tricyclo[5.5.1.0^{3,11}]tridecyl, phenyl, naphthyl, biphenyl, indenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, tetrazolyl, pyrazinyl, pyrimidyl, quinolyl, benzofuranyl, benzothiophenyl, pyrazolyl, indolyl, carbazolyl, thiazolyl, oxazolyl, benzothiazolyl, benzoxazolyl, morpholino, thiomorpholino, spirobifluorenyl, fluoro, cyano, methoxy, ethoxy, n-propoxy, i-propoxy, n-butoxy, tert-butoxy, hexyloxy, trimethylsilyl, triethylsilyl, tripropylsilyl, tri(tert-butyl)silyl, tert-butyldimethylsilyl, dimethylphenylsilyl or triphenylsilyl; and the phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl may be further substituted by one or more substituent(s) selected from methyl, ethyl, n-propyl, i-propyl, n-butyl, i-butyl, tert-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, fluoro, cyano, phenyl, naphthyl, 9,9-dimethyl-9H-fluorenyl, 9,9-diphenyl-9H-fluorenyl, anthryl, trimethylsilyl, triethylsilyl, tripropylsilyl, tri(tert-butyl)silyl, tert-butyldimethylsilyl, dimethylphenylsilyl, triphenylsilyl, triphenylmethyl and triphenylmethoxy.

In Chemical Formula (1) or Chemical Formula (2), Ar₁ through Ar₄ independently represent pyrrolyl, pyrazolyl, imidazolyl, thiazolyl, isothiazolyl, thiadiazolyl, oxazolyl, isoxazolyl, oxadiazolyl, furanyl, furazanyl, thienyl, tetrazolyl, triazolyl, pyridyl, pyrimidinyl, pyridazinyl, pyrazinyl, triazinyl or tetrazinyl; and they are preferably selected from 1-pyrrolyl, 2-pyrrolyl, 3-pyrrolyl, 1-pyrazolyl, 3-pyrazolyl, 4-pyrazolyl, 5-pyrazolyl, 1-imidazolyl, 2-imidazolyl, 4-imidazolyl, 5-imidazolyl, 2-thiazolyl, 4-thiazolyl, 5-thiazolyl, 3-isothiazolyl, 4-isothiazolyl, 5-isothiazolyl, 1,2,3-thiadiazol-4-yl, 1,2,3-thiadiazol-5-yl, 1,3,4-thiadiazol-2-yl, 1,3,5-thiadiazol-2-yl, 1,3,5-thiadiazol-4-yl, 2-oxazolyl, 4-oxazolyl, 5-oxazolyl, 3-isoxazolyl, 4-isoxazolyl, 5-isoxazolyl, 1,2,3-oxadiazol-4-yl, 1,2,3-oxadiazol-5-yl, 1,3,4-oxadiazol-2-yl, 1,3,5-oxadiazol-2-yl, 1,3,5-oxadiazol-4-yl, 2-furanyl, 3-furanyl, 3-furazanyl, 2-thienyl, 3-thienyl, 1H-tetrazol-5-yl, 1H-tetrazol-1-yl, 2H-tetrazol-5-yl, 2H-tetrazol-2-yl, 1H-1,2,3-triazol-1-yl, 1H-1,2,3-triazole-4-yl, 1H-1,2,3-triazol-5-yl, 1H-1,2,4-triazol-1-yl, 1H-1,2,4-triazol-3-yl, 1H-1,2,4-triazol-5-yl, 2H-1,2,3-triazol-2-yl, 2H-1,2,3-triazol-4-yl, 2H-1,2,3-triazol-5-yl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1,2,3-triazin-4-yl, 1,2,3-triazin-5-yl, 1,2,4-triazin-3-yl, 1,2,4-triazin-5-yl, 1,2,4-triazin-6-yl, 1,3,5-triazin-2-yl, 1,2,3,4-tetrazin-5-yl and 1,2,3,5-tetrazin-4-yl.

The organic electroluminescent compounds according to the present invention can be specifically exemplified by the following compounds, but are not restricted thereto: 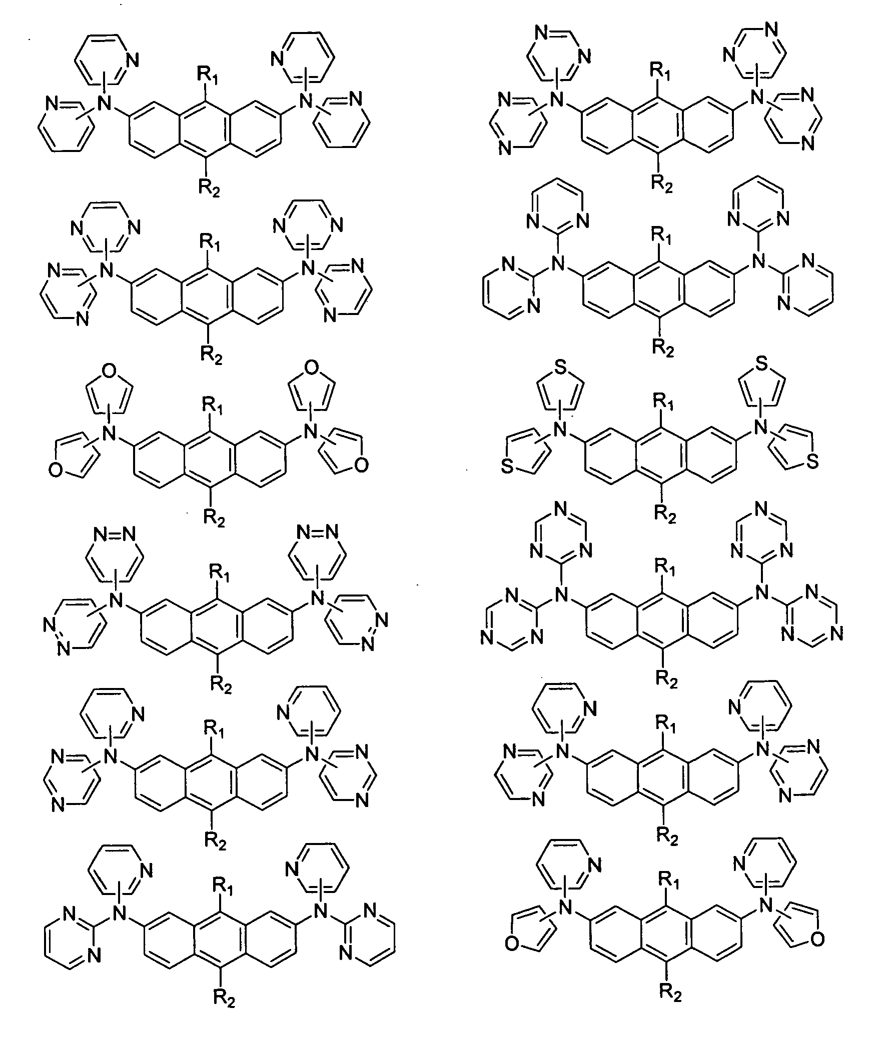 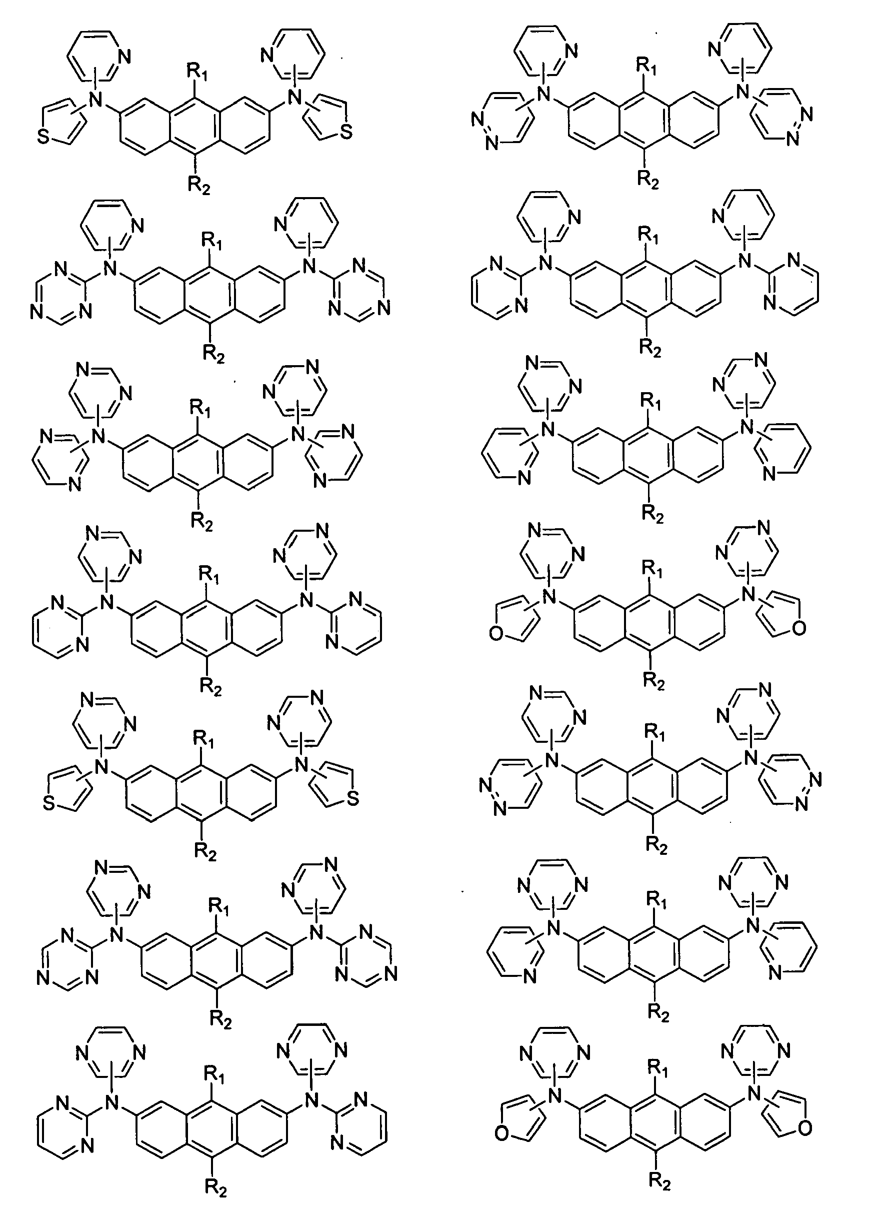 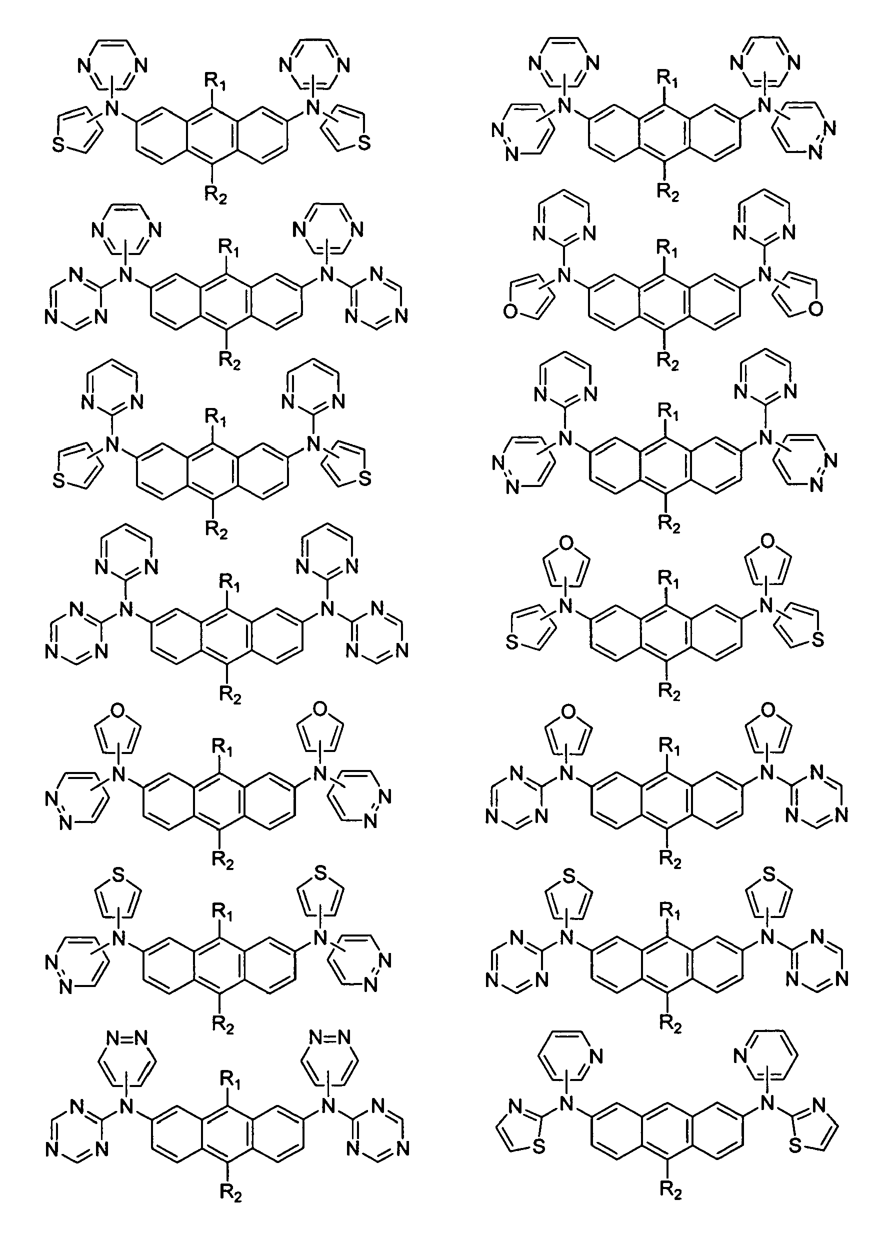 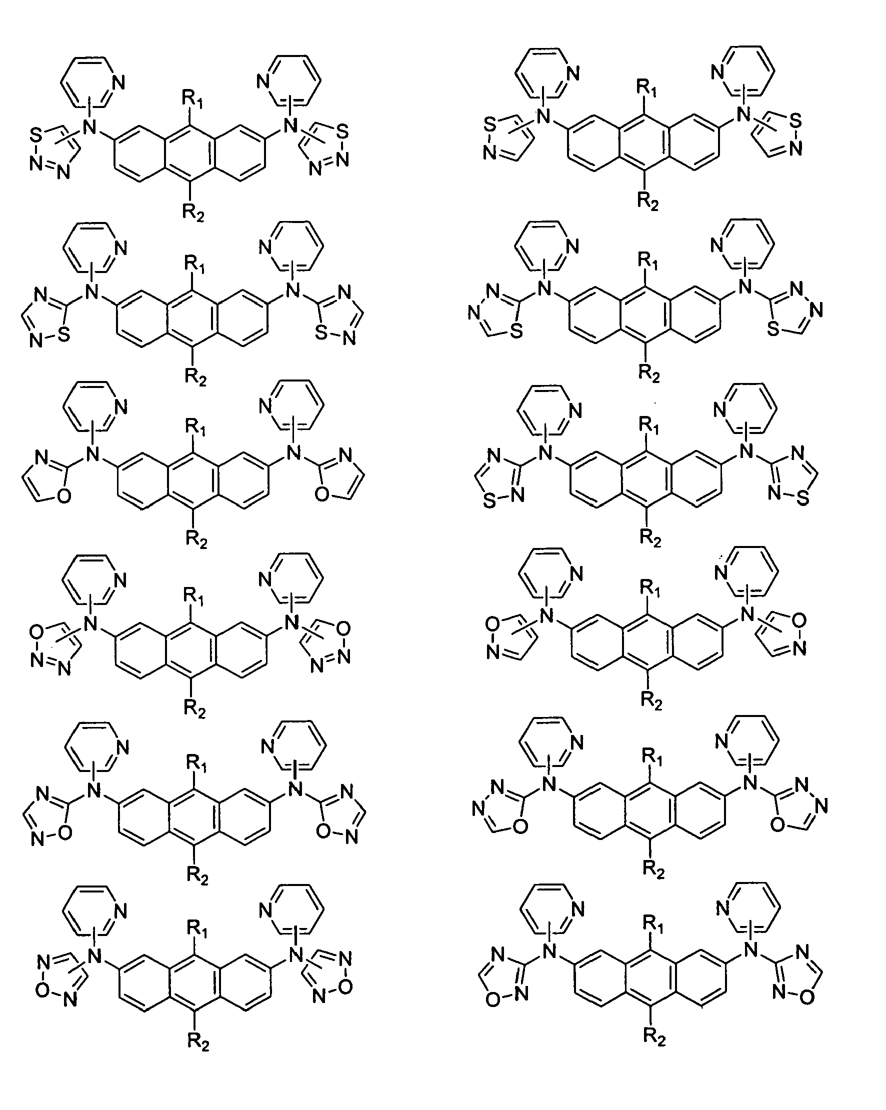 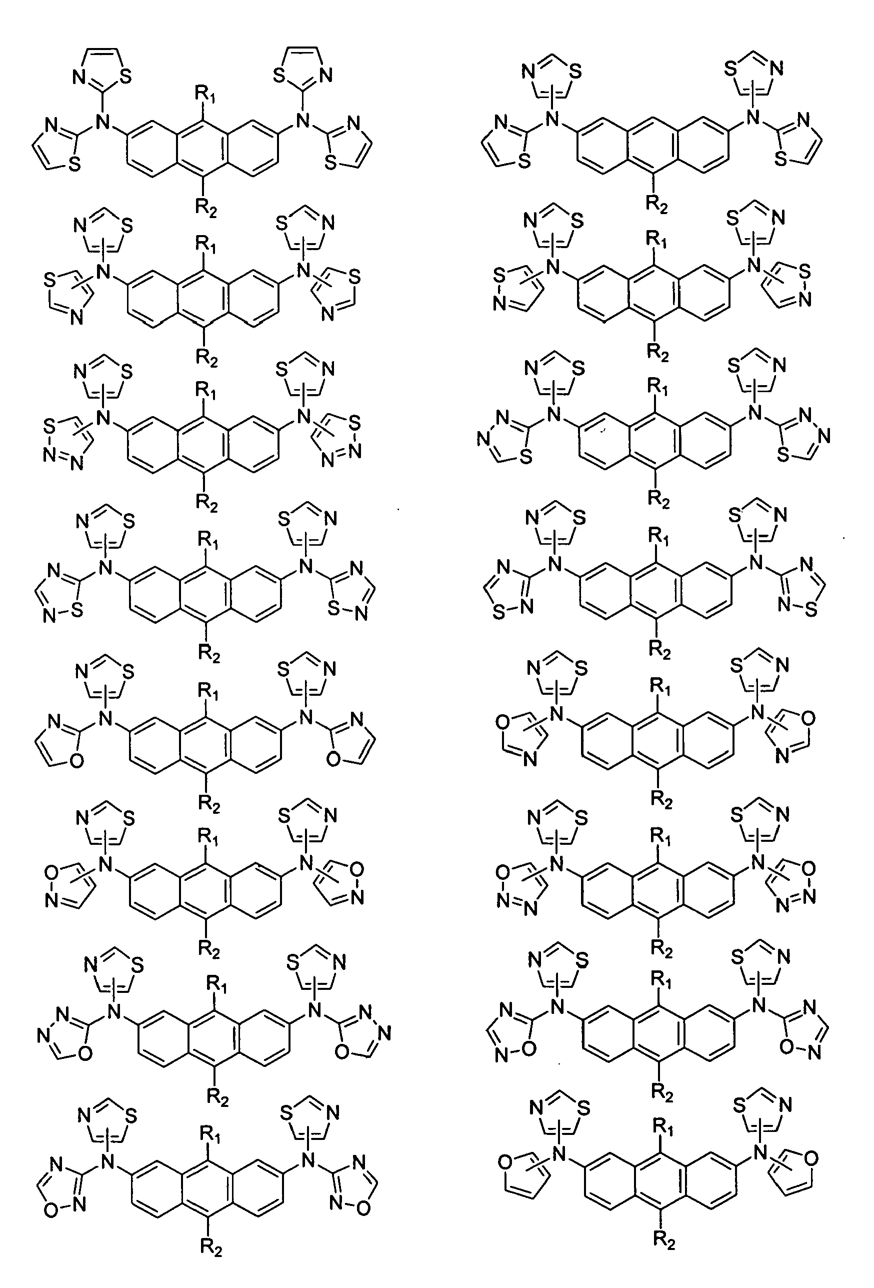 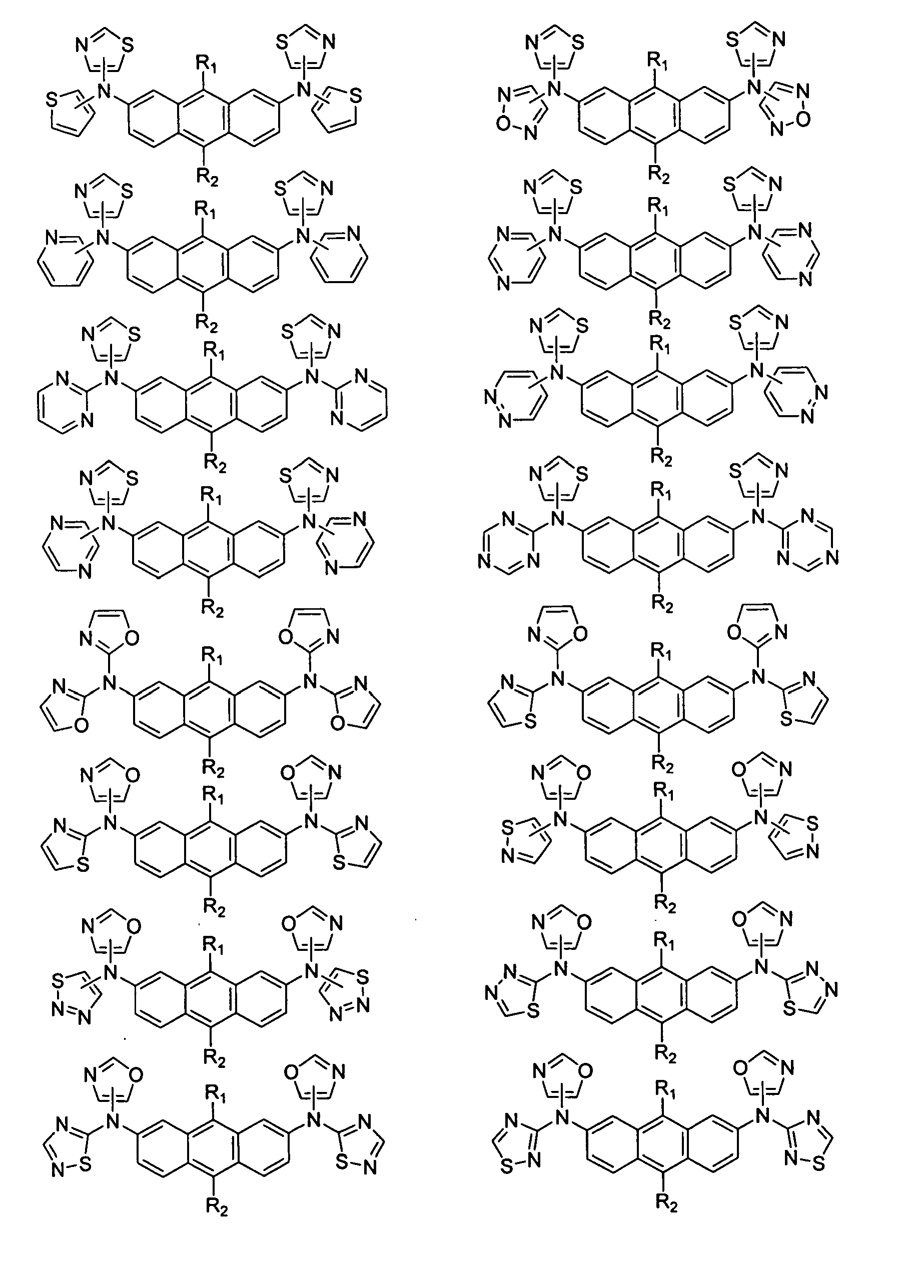 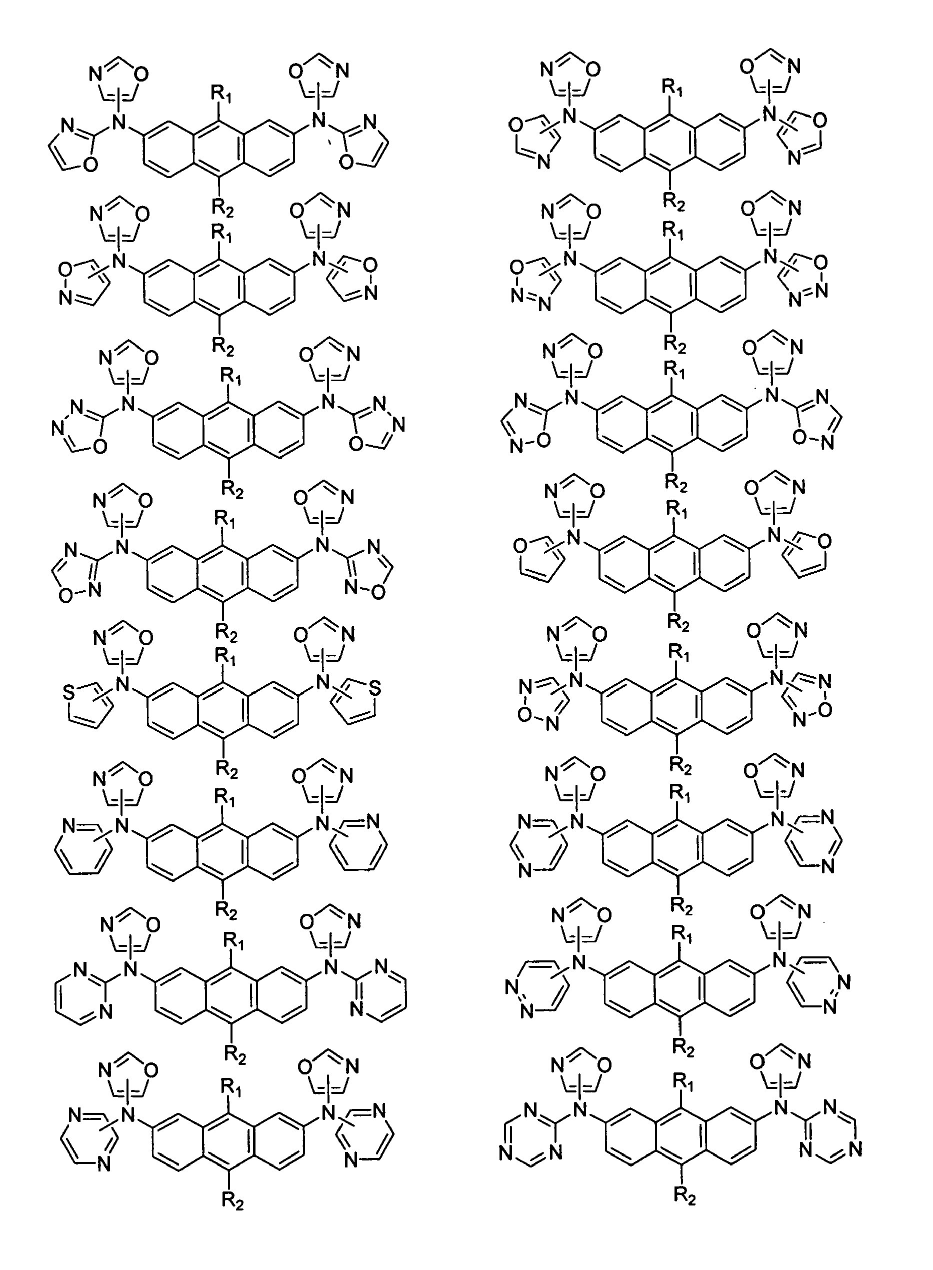 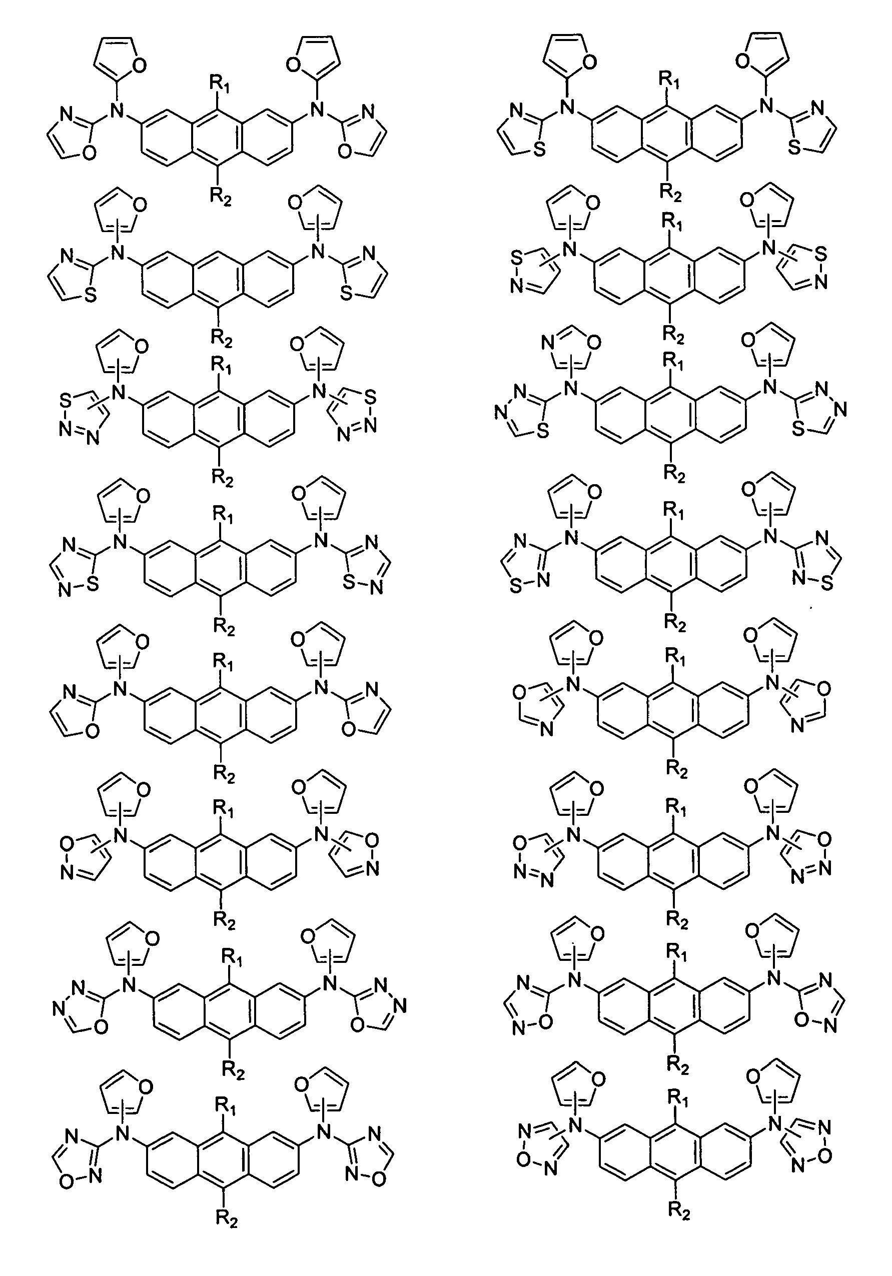 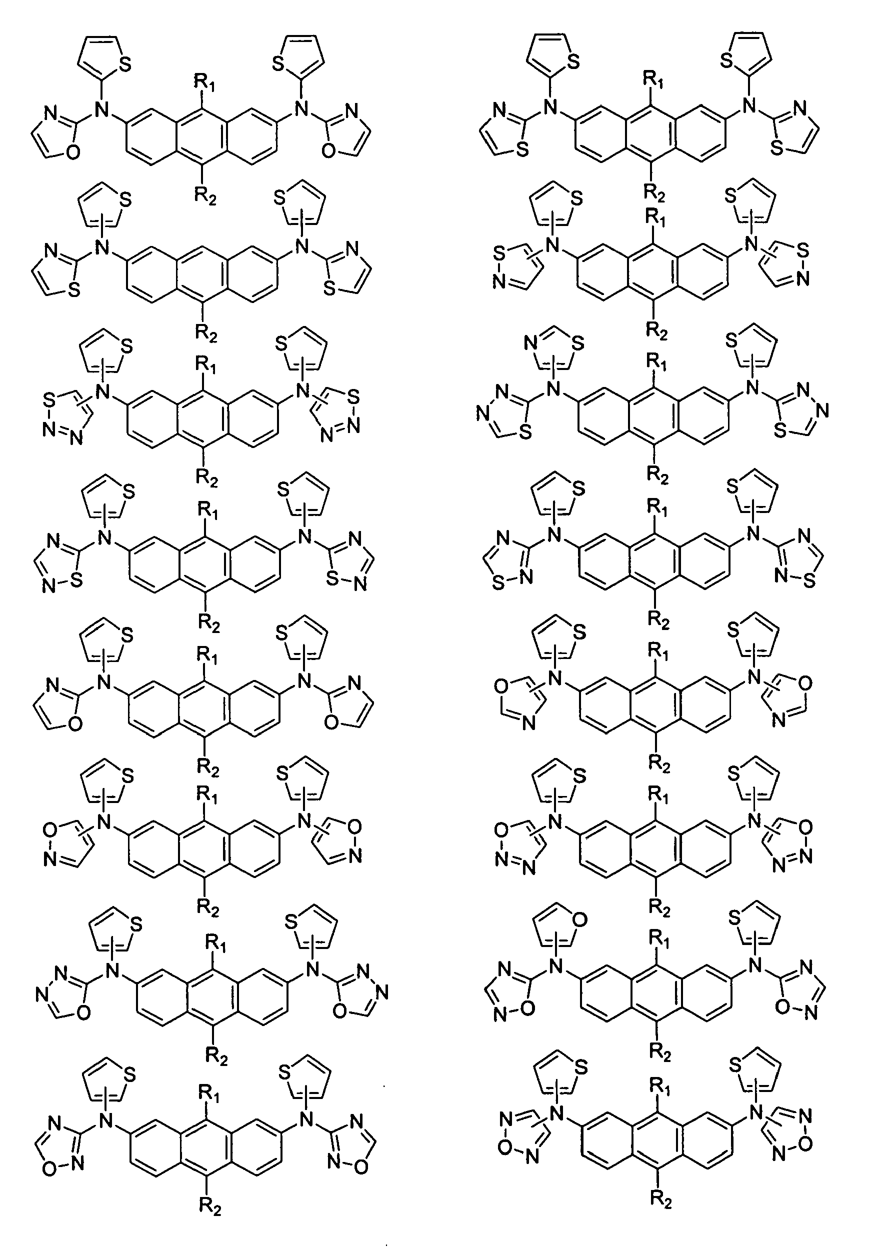 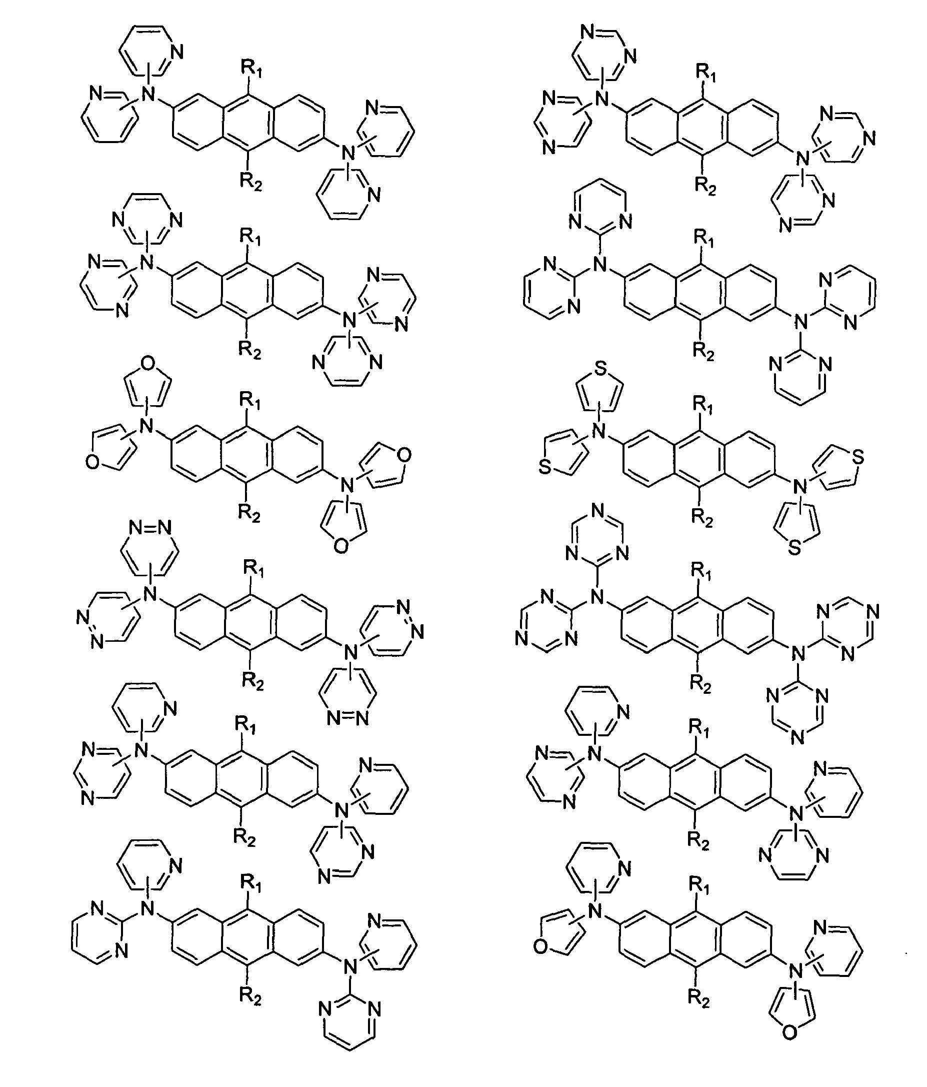 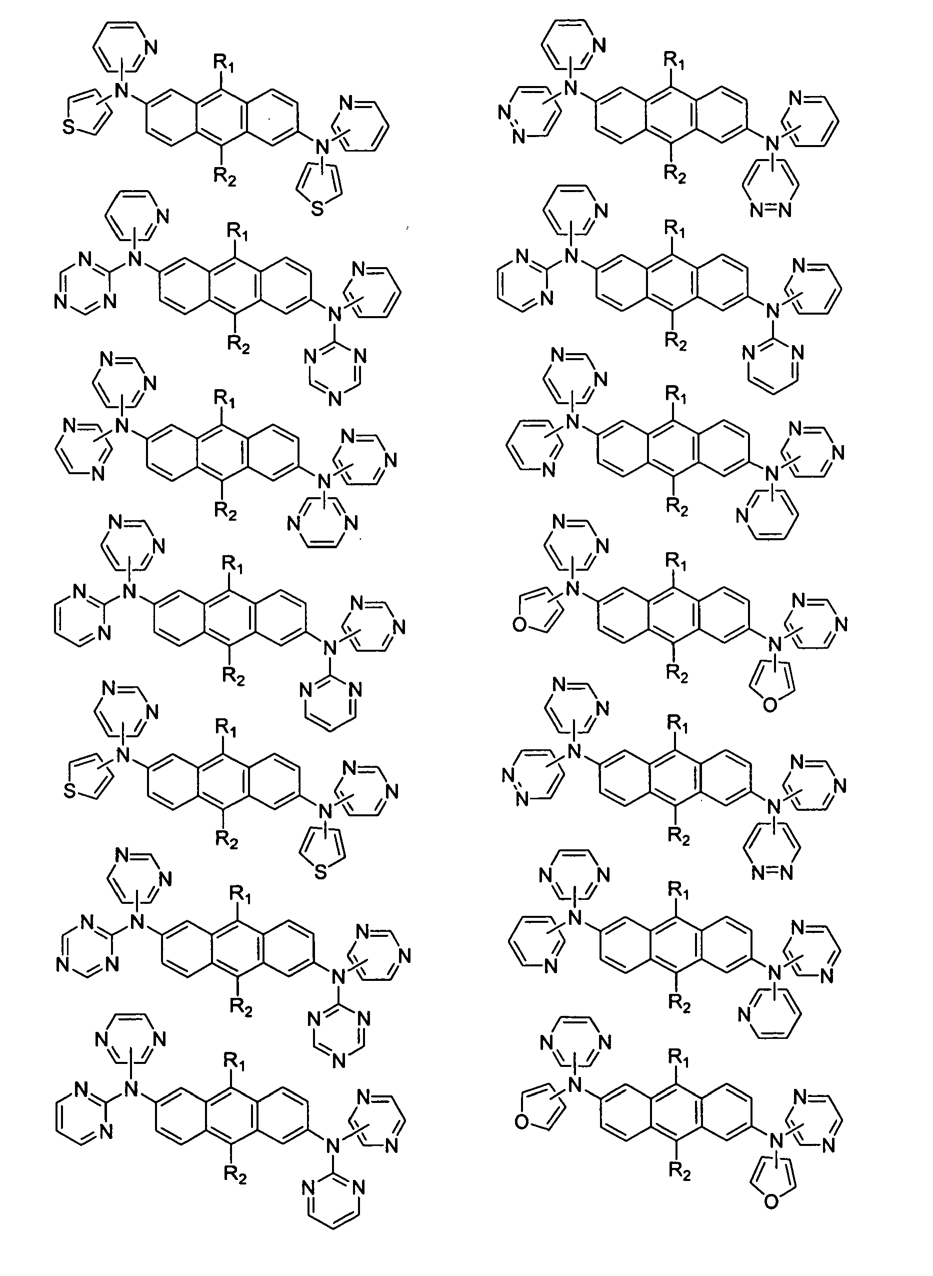 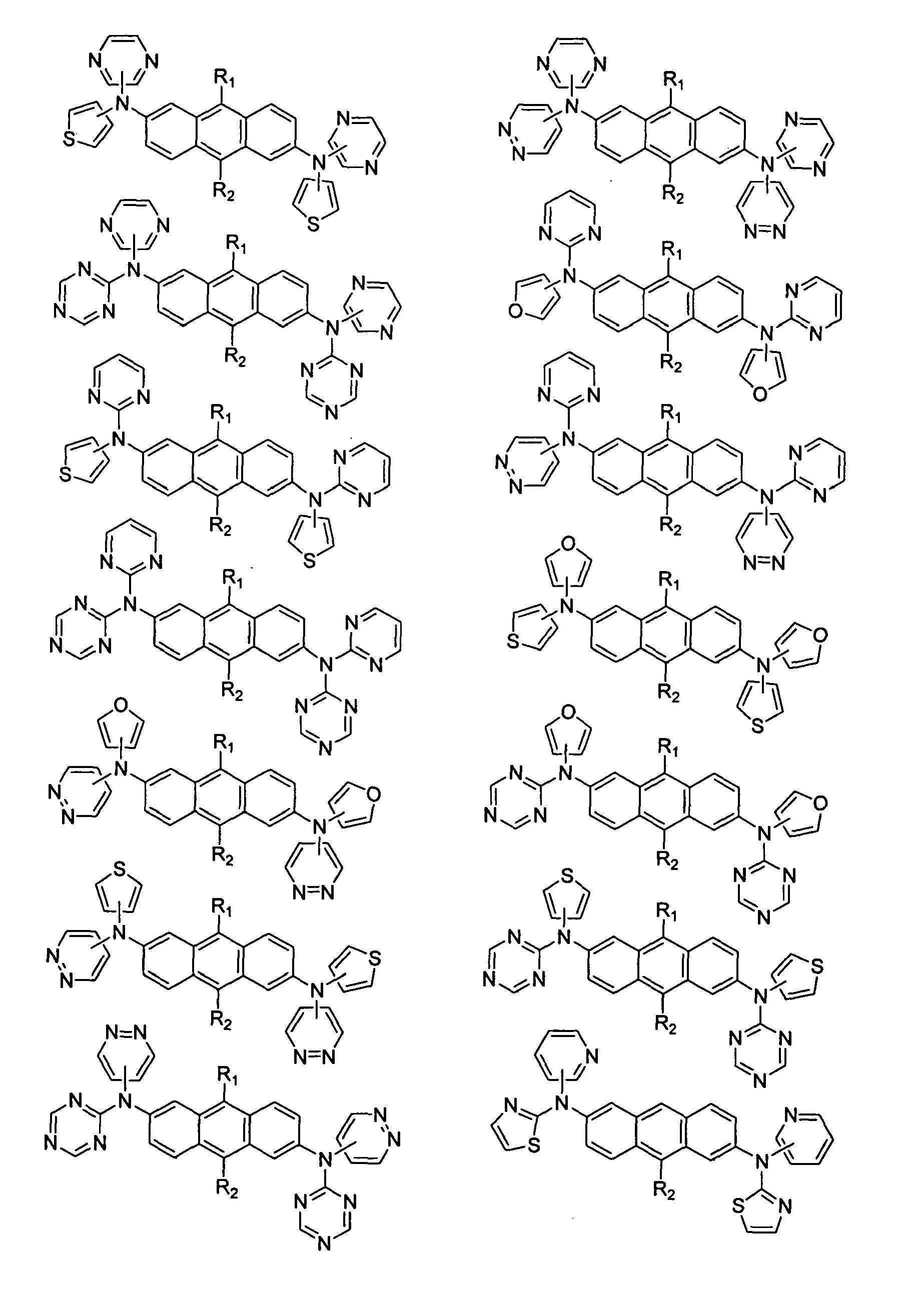 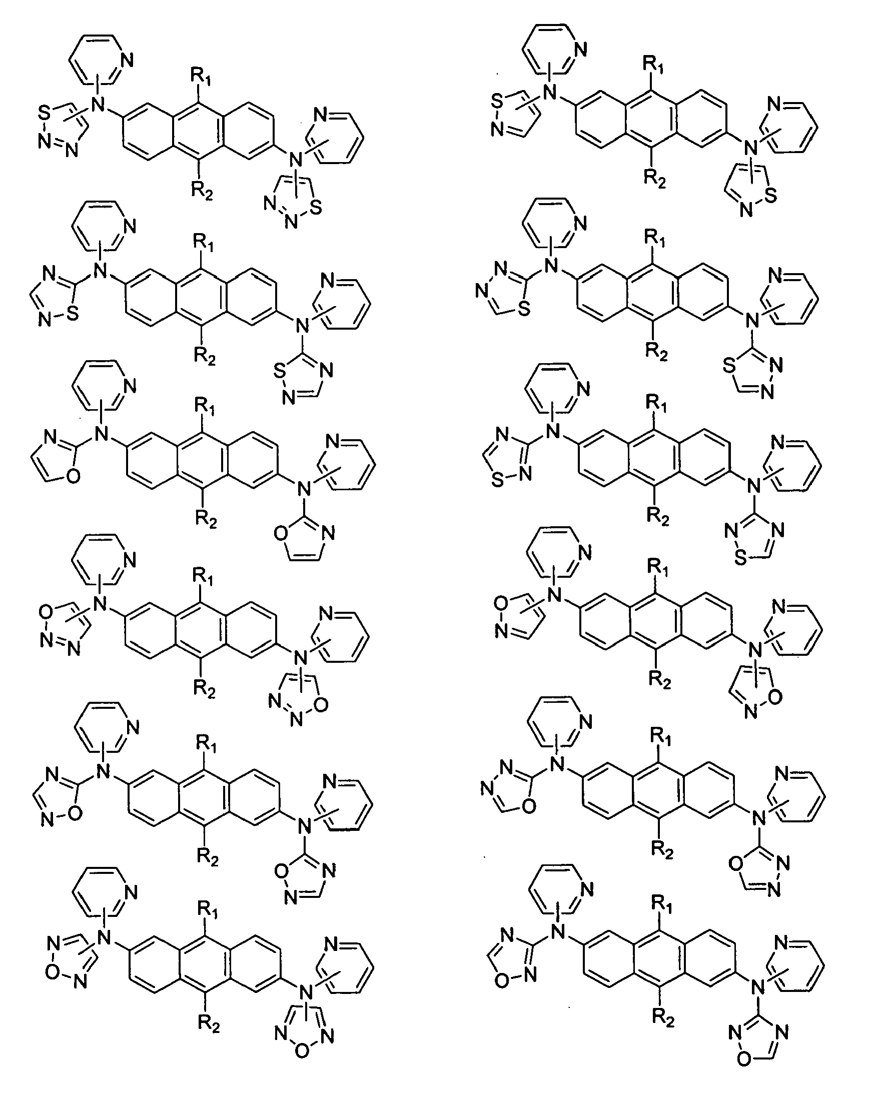 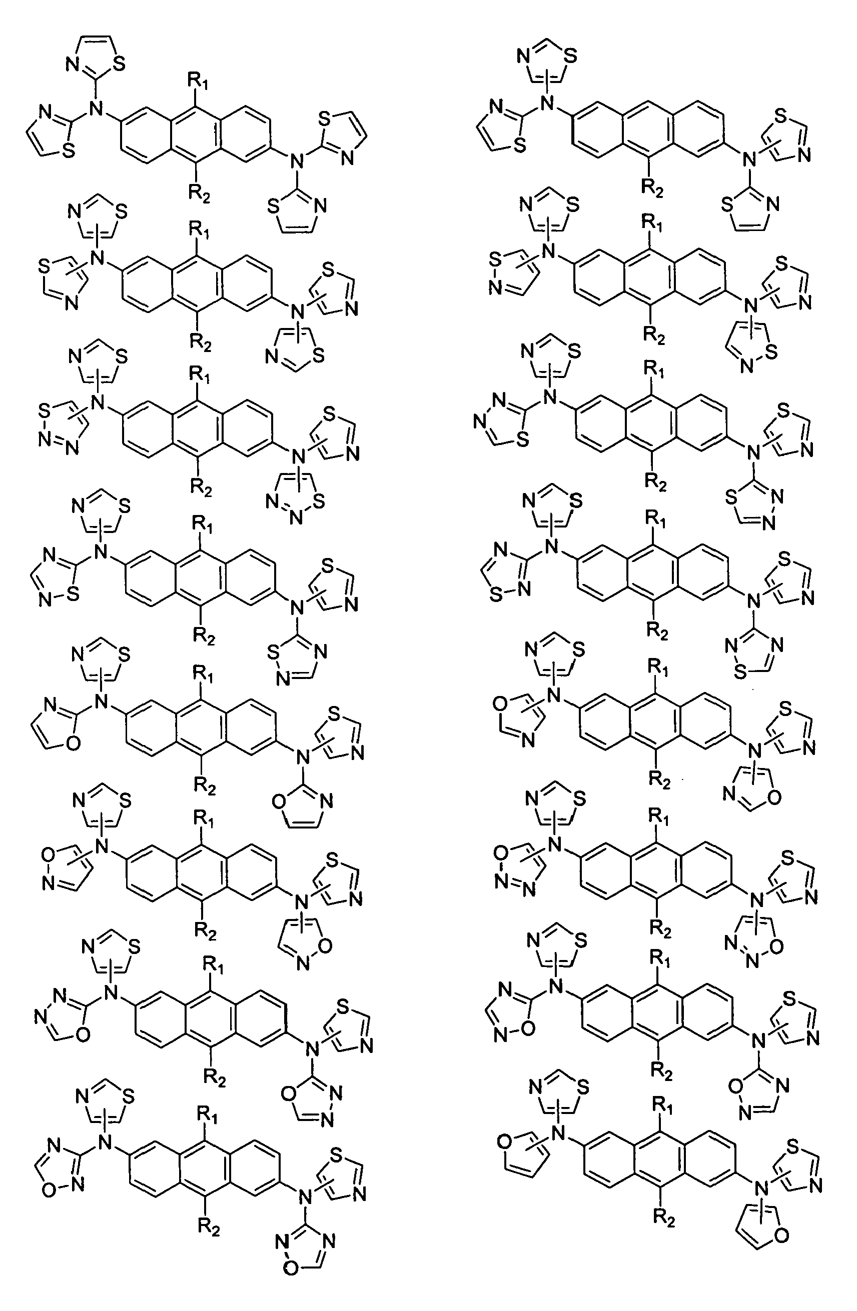 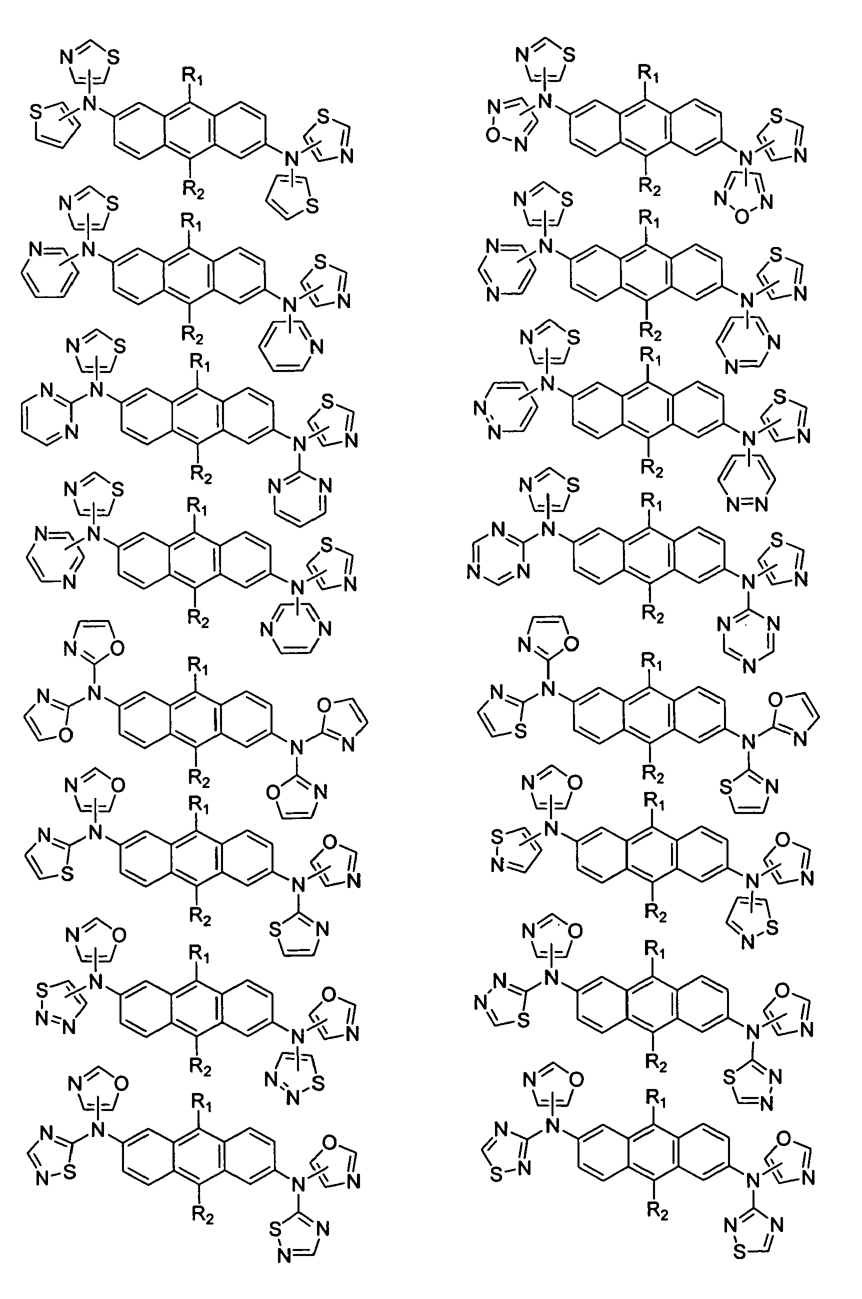 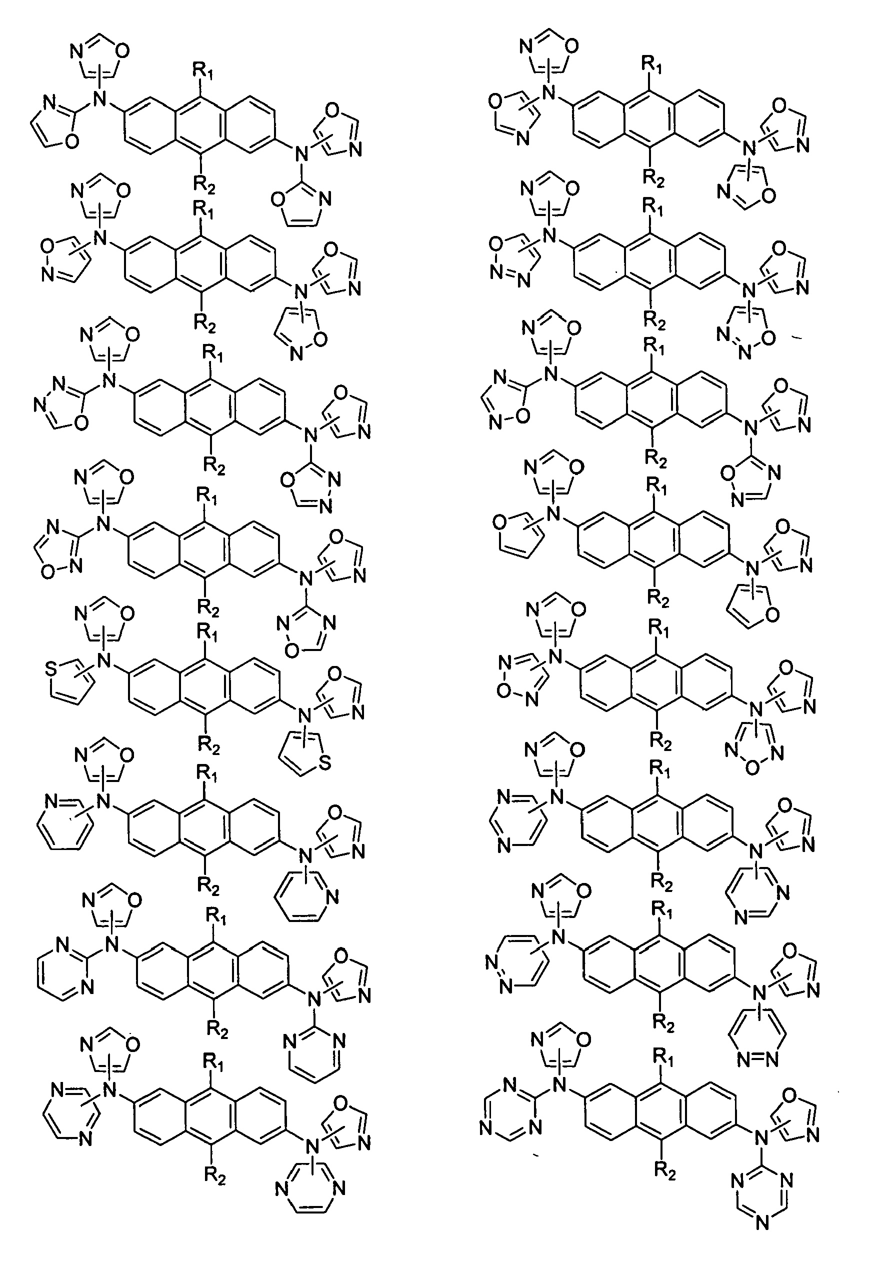 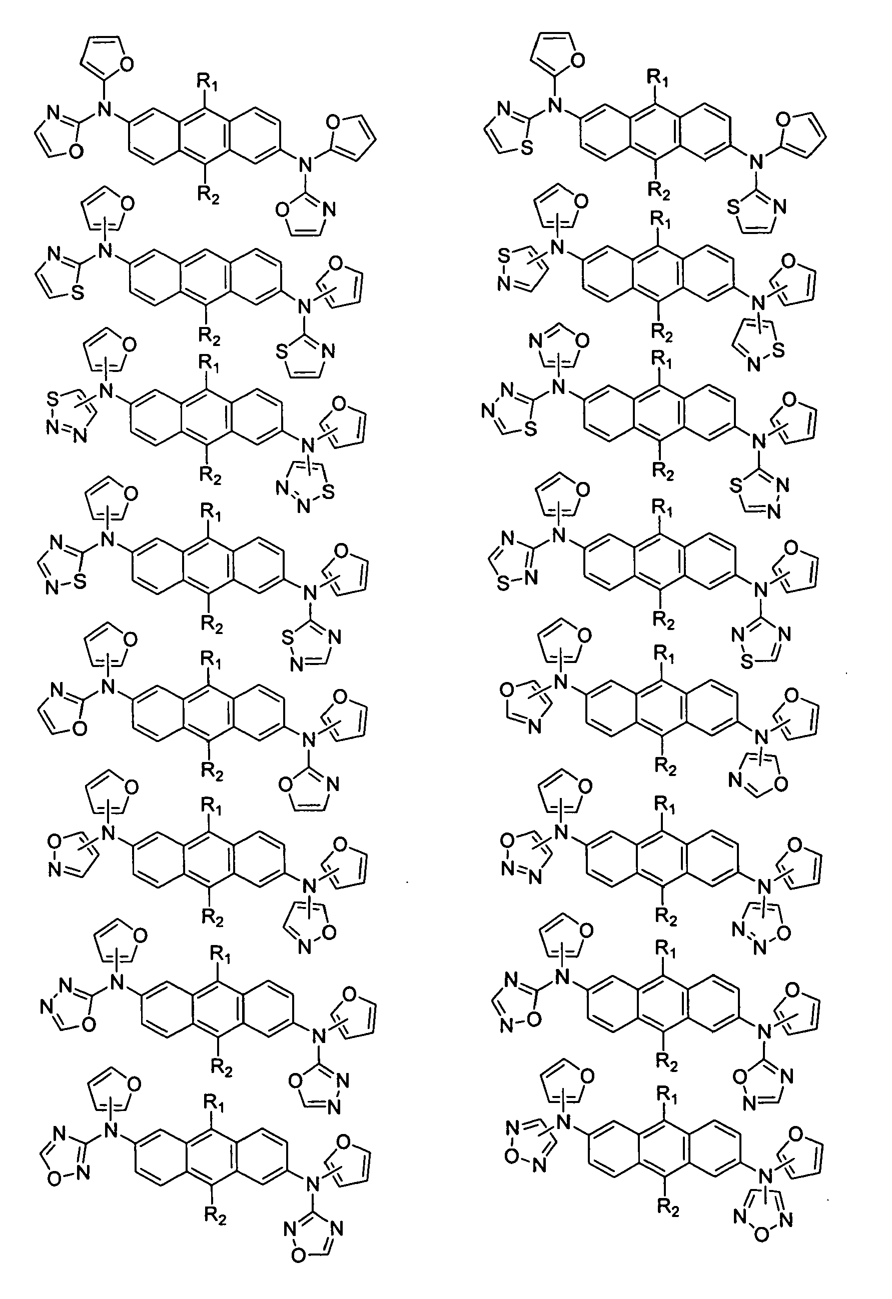 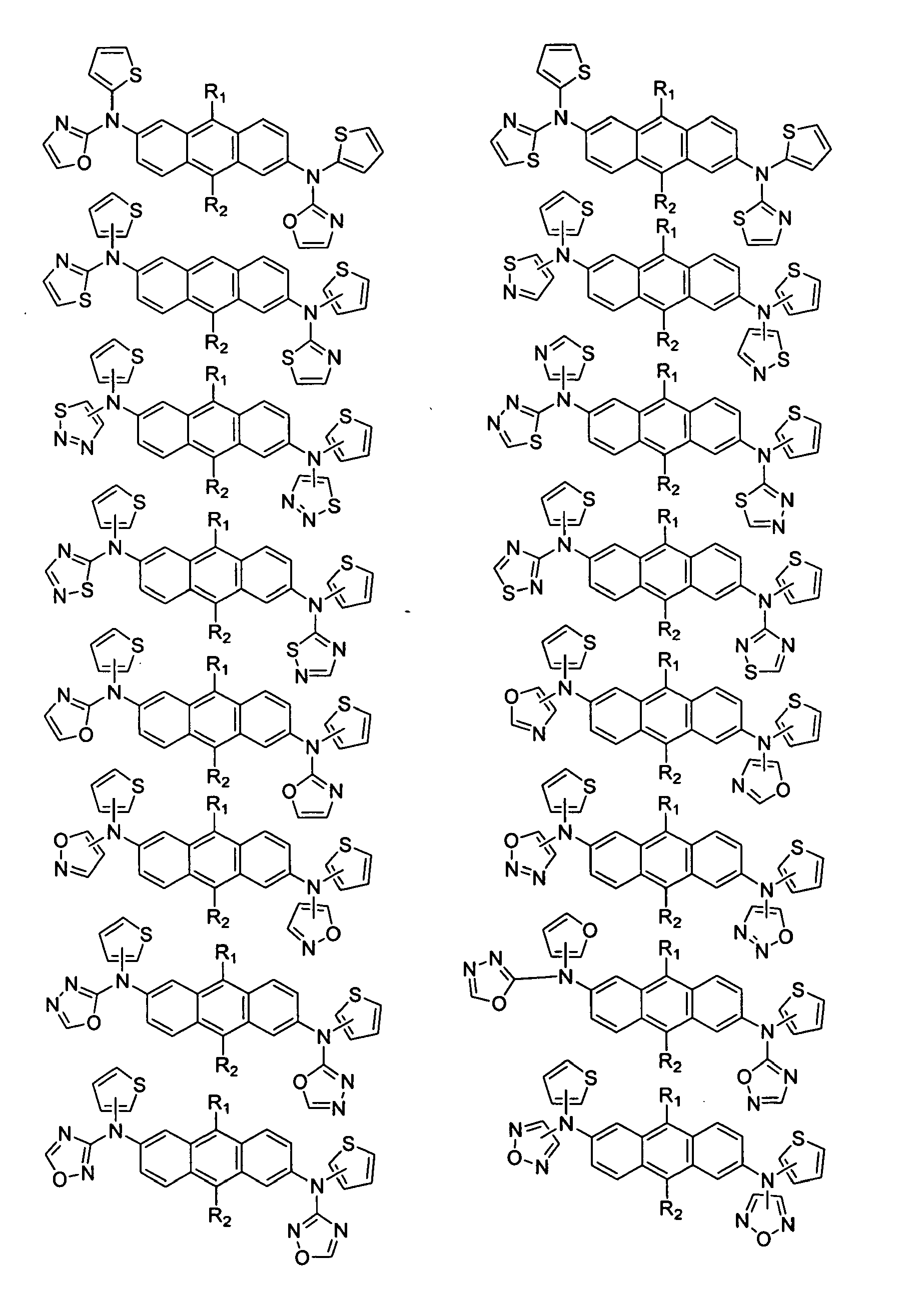 wherein, R₁ and R₂ independently represent methyl, ethyl, n-propyl, i-propyl, n-butyl, i-butyl, tert-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, benzyl, ethenyl, phenylethenyl, ethynyl, phenylethynyl, cyclopentyl, cyclohexyl, cycloheptyl, cyclooctyl, bicyclo[1.1.0]butyl, bicyclo[1.1.1]pentyl, bicyclo[2.1.0]pentyl, bicyclo[2.1.1]hexyl, bicyclo[3.2.0]hexyl, bicyclo[3.2.0]heptyl, bicyclo[3.1.1]heptyl, bicyclo[4.1.0]heptyl, bicyclo[2.2.1]heptyl, octahydropentalenyl, bicyclo[2.2.2]octyl, bicyclo[4.2.0]octyl, bicyclo[4.1.1]octyl, bicyclo[3.2.1]octyl, octahydro-1H-indenyl, bicyclo[5.2.0]nonyl, bicyclo[4.2.1]nonyl, bicyclo[3.3.1]nonyl, bicyclo[3.3.2]decyl, bicyclo[4.3.1]decyl, bicyclo[4.2.2]decyl, decahydronaphthalenyl, bicyclo[3.3.3]undecyl, bicyclo[4.3.2]undecyl, bicyclo[4.3.3]dodecyl, 4-pentylbicyclo[2.2.2]octyl, tricyclo[2.2.1.0]heptyl, tricyclo[5.2.1.0^{2,6}]decyl, tricyclo[5.3.1.1]dodecyl, tricyclo[5.4.0.0^{2,9}]undecyl, adamantyl, tricyclo[5.3.2.0^{4,9}]dodecyl, tricyclo[4.4.1.1^{1,5}]dodecyl, tricyclo[5.5.1.0^{3,11}]tridecyl, phenyl, naphthyl, biphenyl, indenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, tetrazolyl, pyrazinyl, pyrimidyl, quinolyl, benzofuranyl, benzothiophenyl, pyrazolyl, indolyl, carbazolyl, thiazolyl, oxazolyl, benzothiazolyl, benzoxazolyl, morpholino, thiomorpholino or spirobifluorenyl; and
the phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl may be further substituted by one or more substituent(s) selected from methyl, ethyl, n-propyl, i-propyl, n-butyl, i-butyl, tert-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, fluoro, cyano, phenyl, naphthyl, 9,9-dimethyl-9H-fluorenyl, 9,9-diphenyl-9H-fluorenyl, anthryl, trimethylsilyl, triethylsilyl, tripropylsilyl, tri(tert-butyl)silyl, tert-butyldimethylsilyl, dimethylphenylsilyl, triphenylsilyl, triphenylmethyl and triphenylmethoxy.

More specifically, the organic electroluminescent compounds according to the present invention can be exemplified by the following compounds, but the invention is not restricted to them.

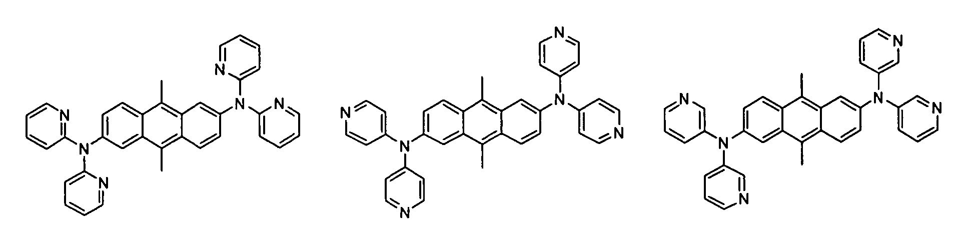

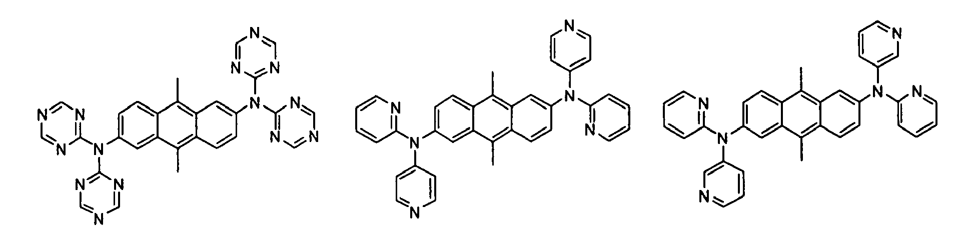

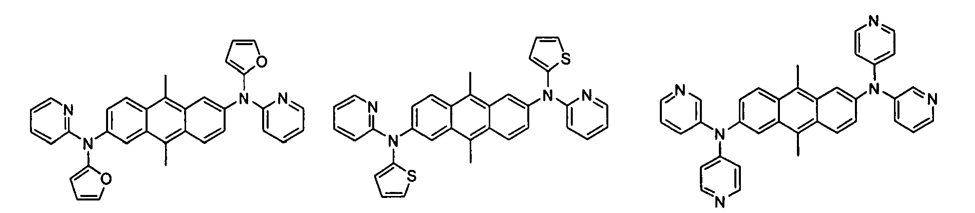

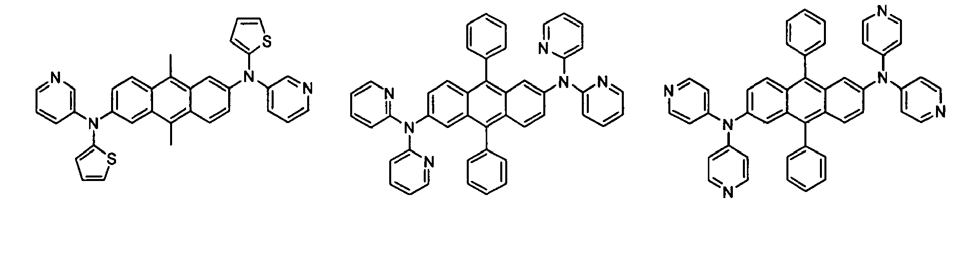

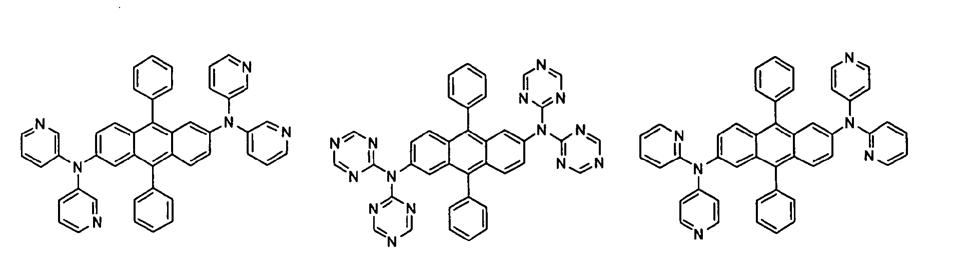

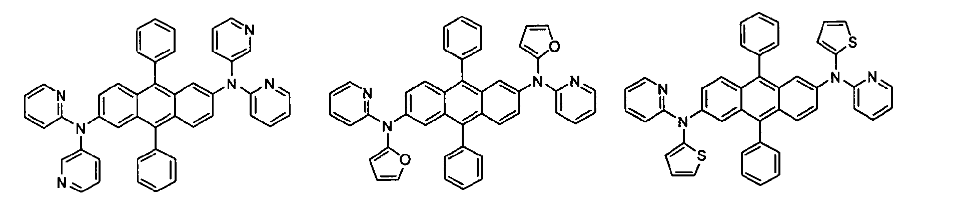

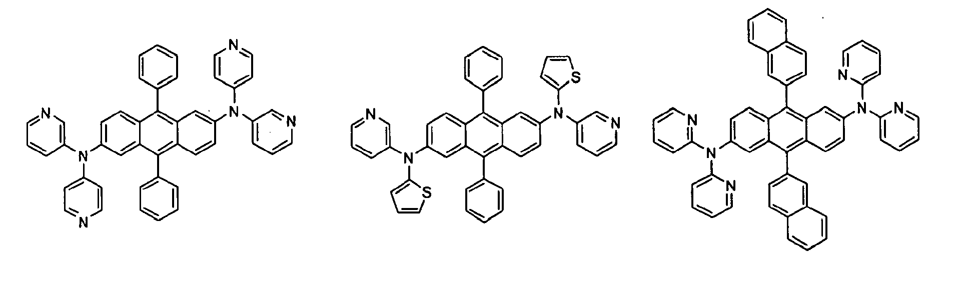

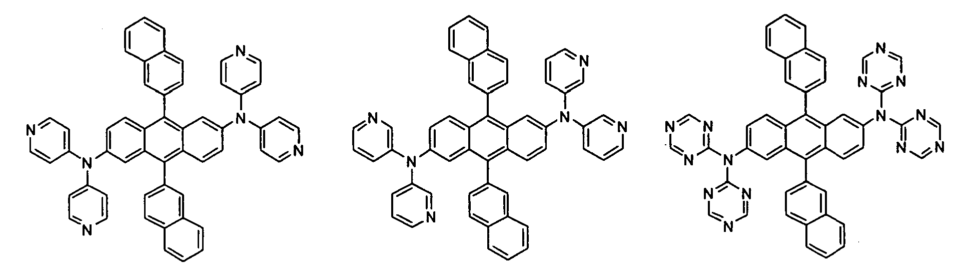

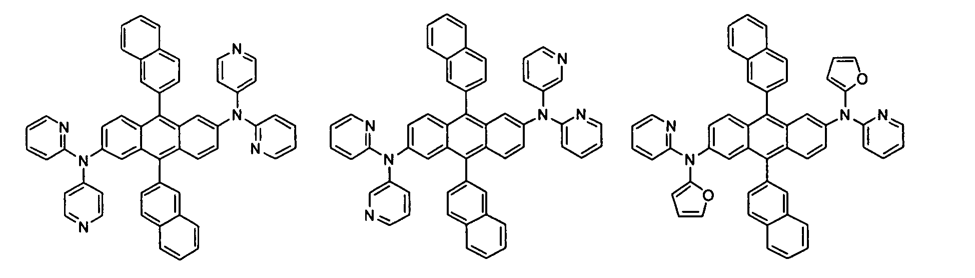

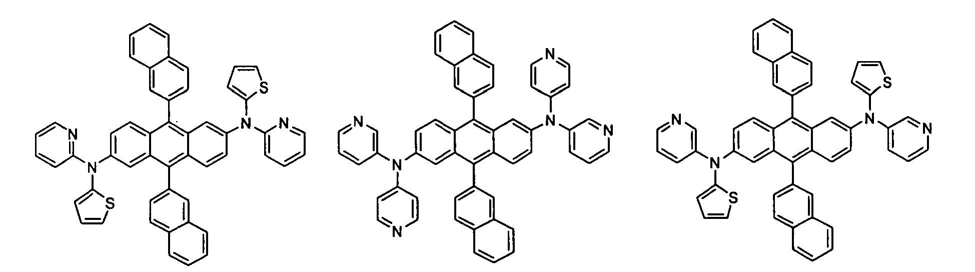

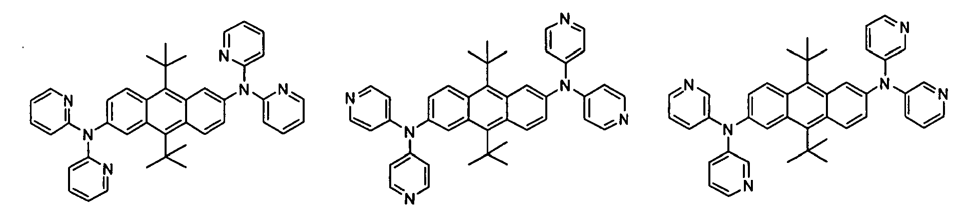

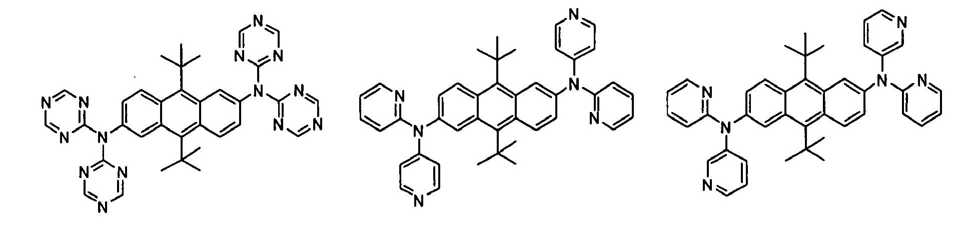

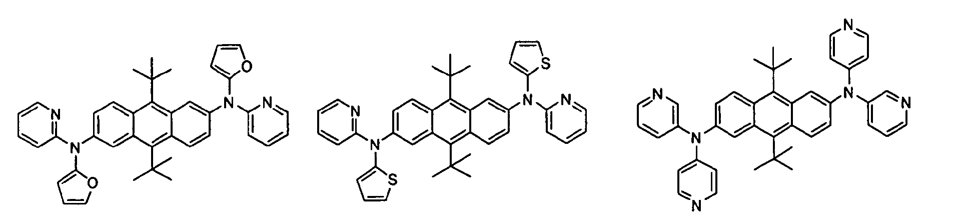

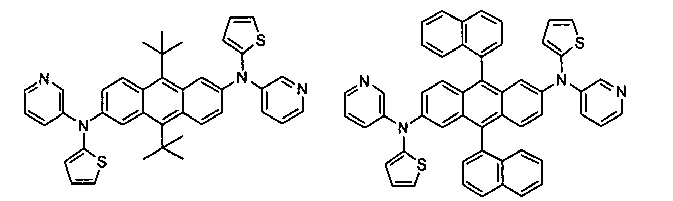

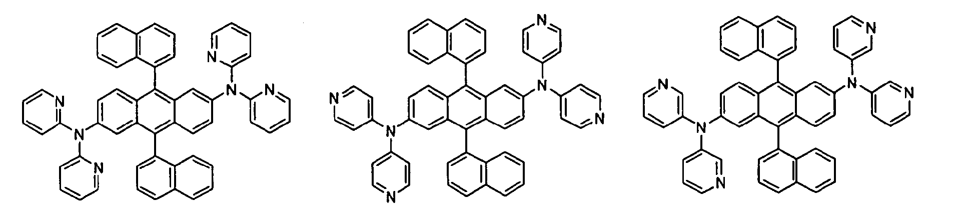

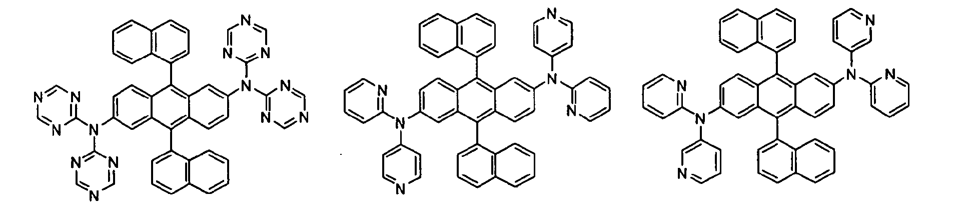

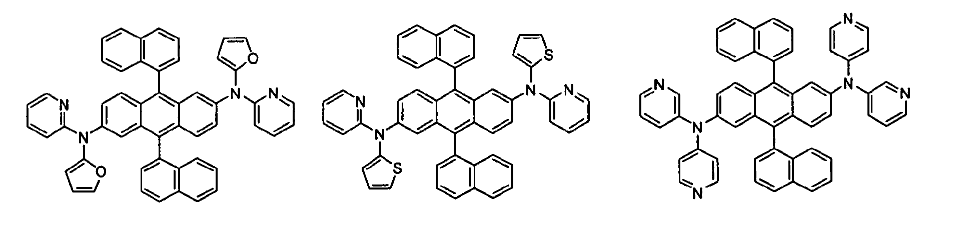

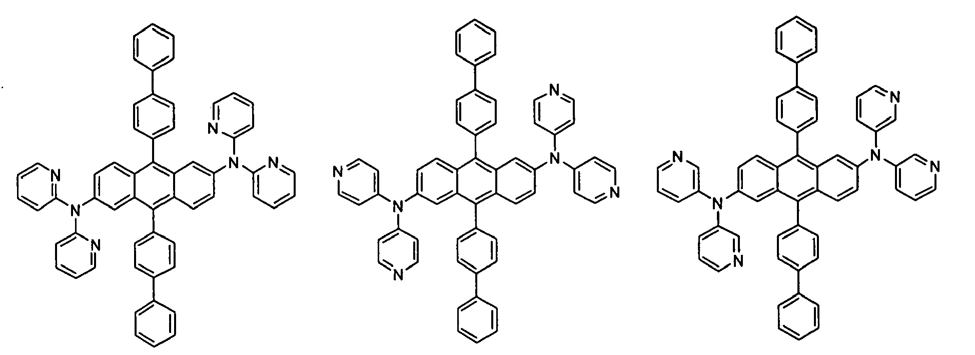

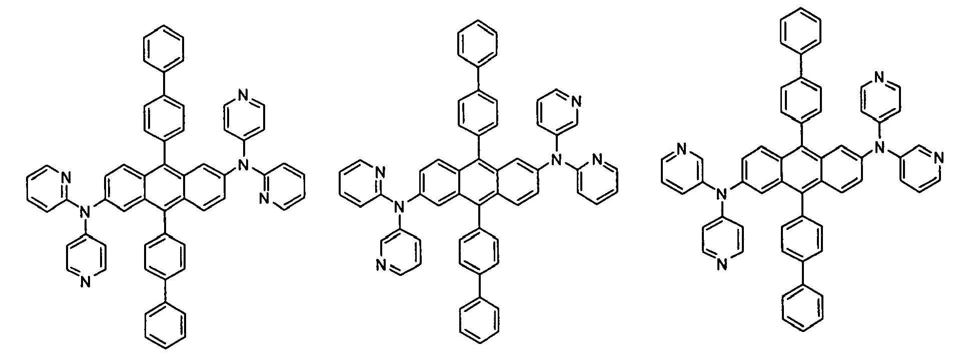

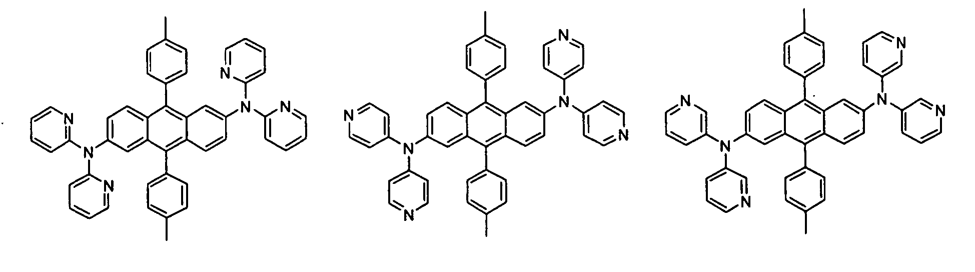

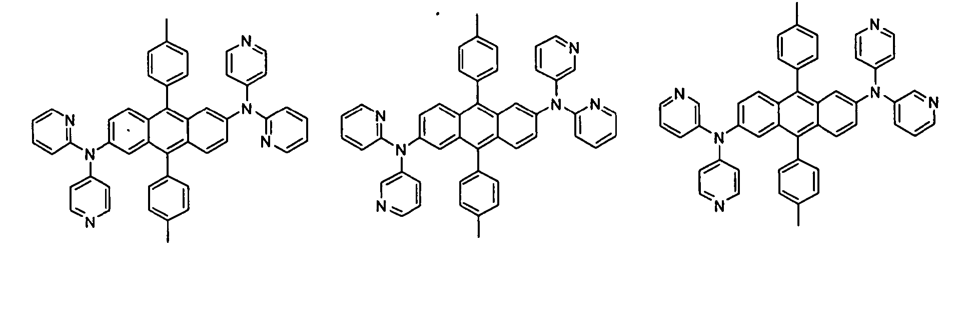

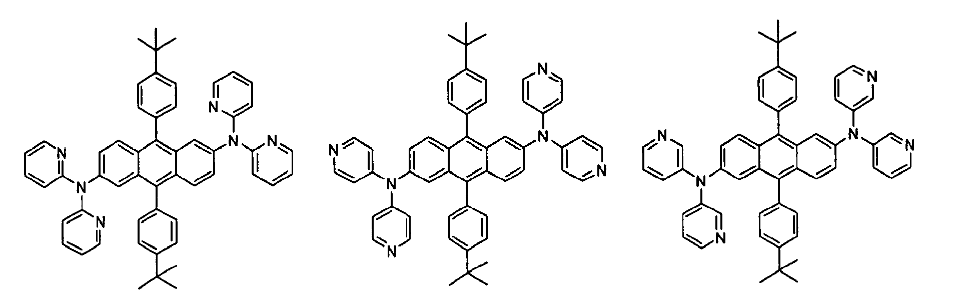

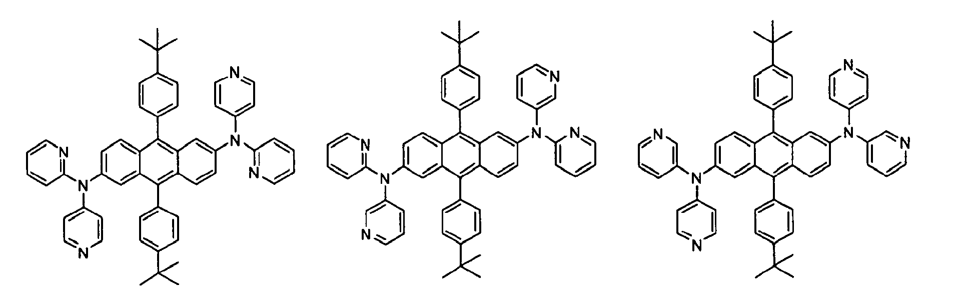

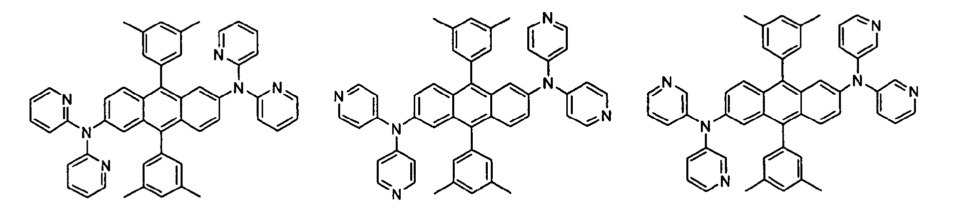

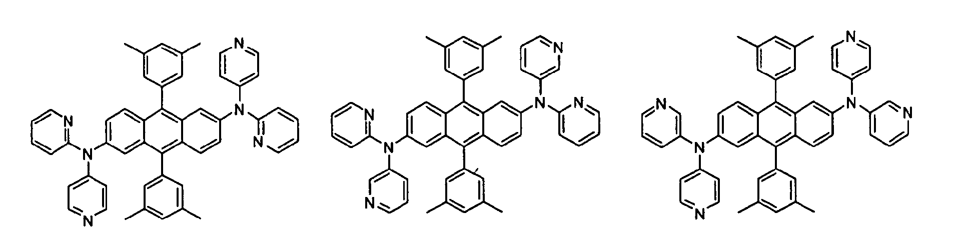

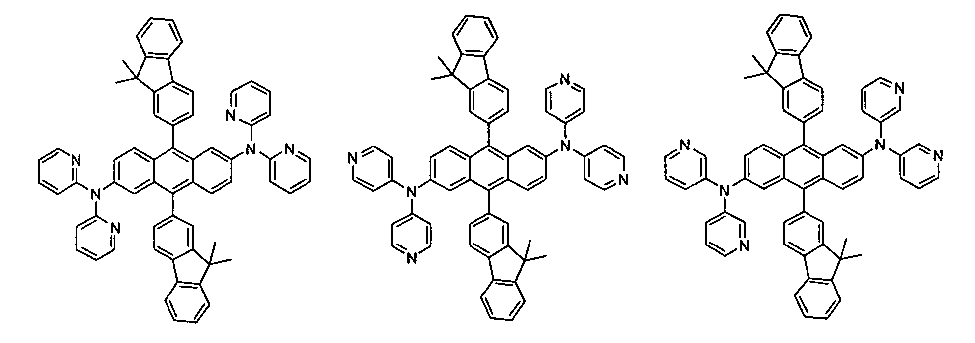

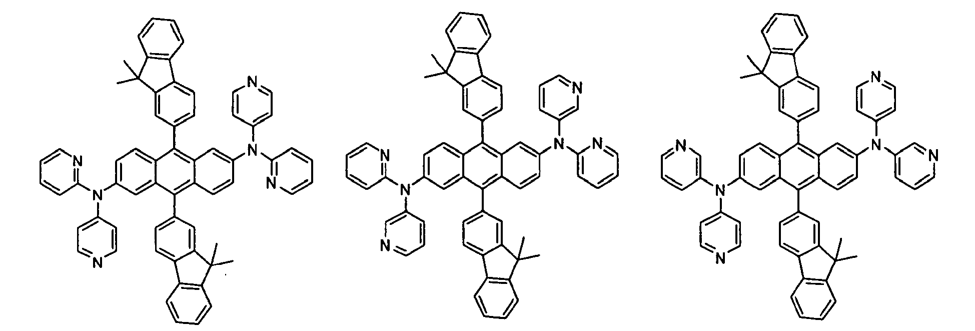

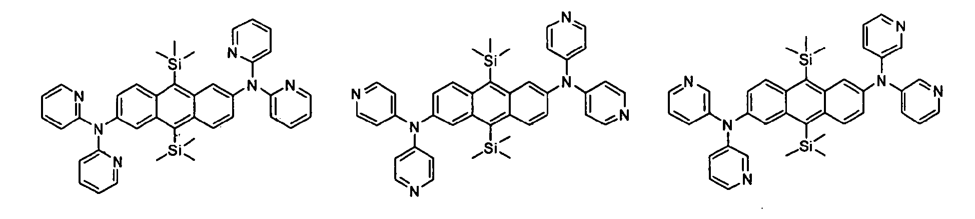

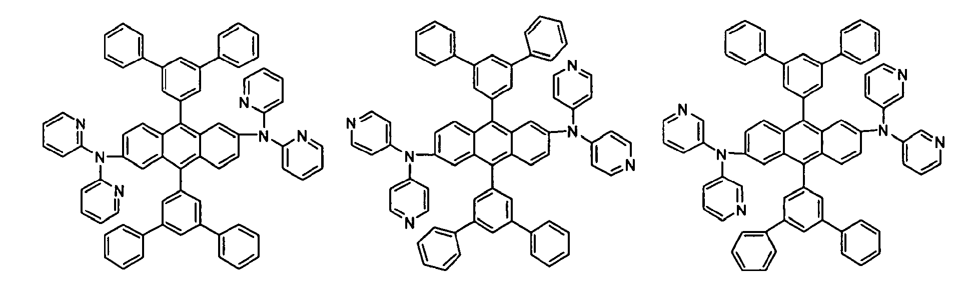

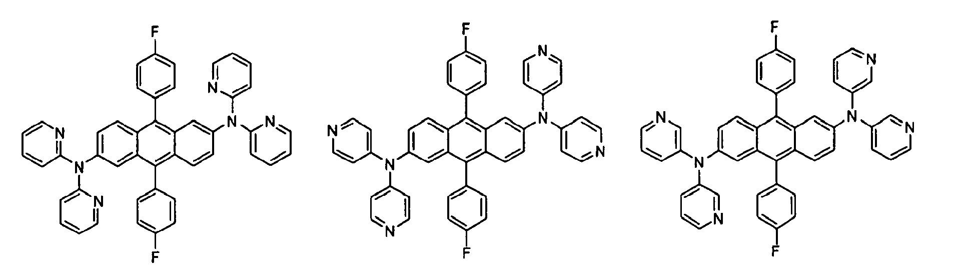

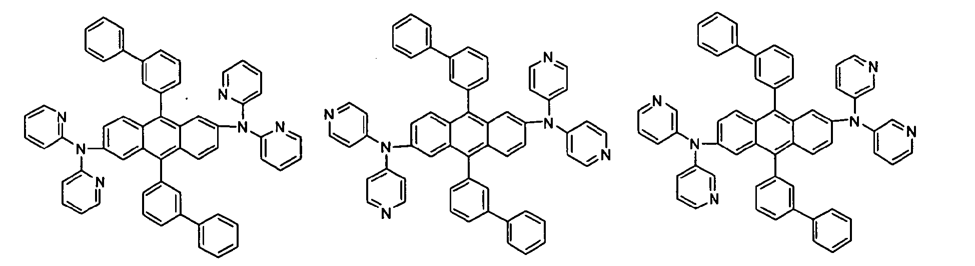

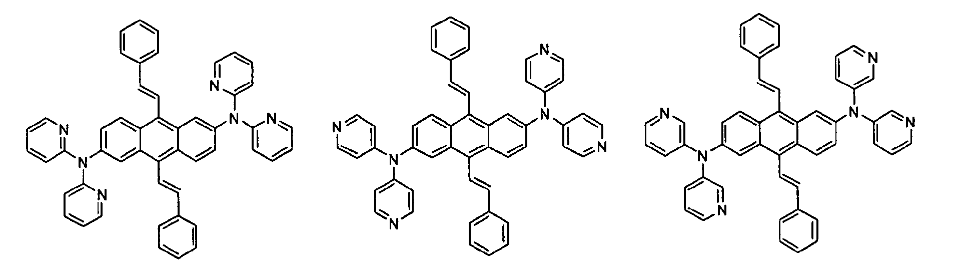

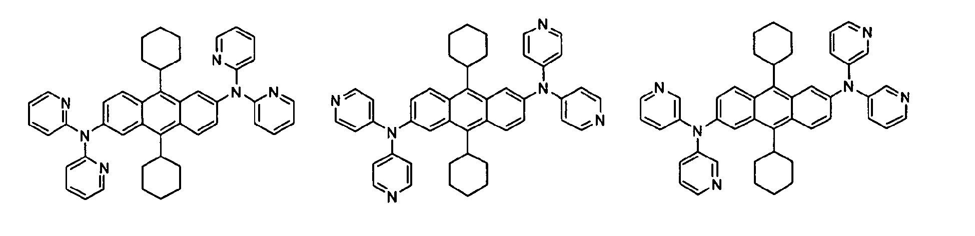

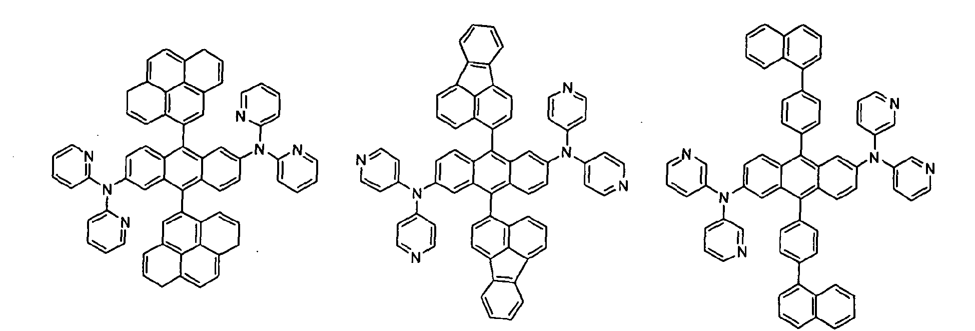

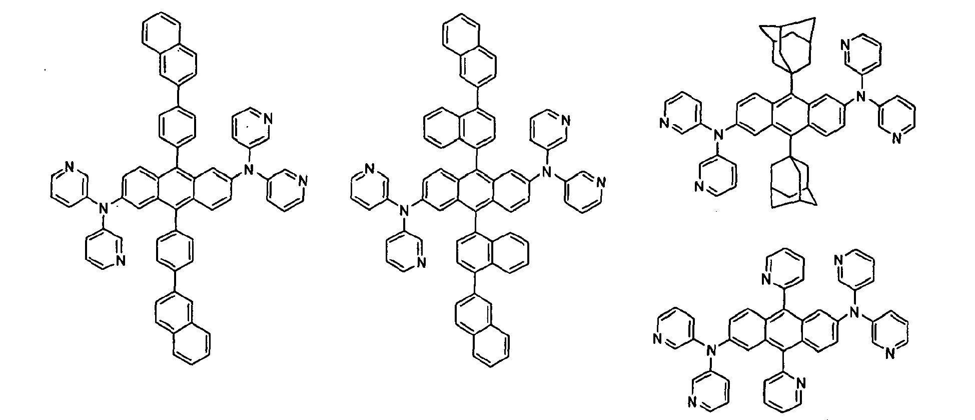

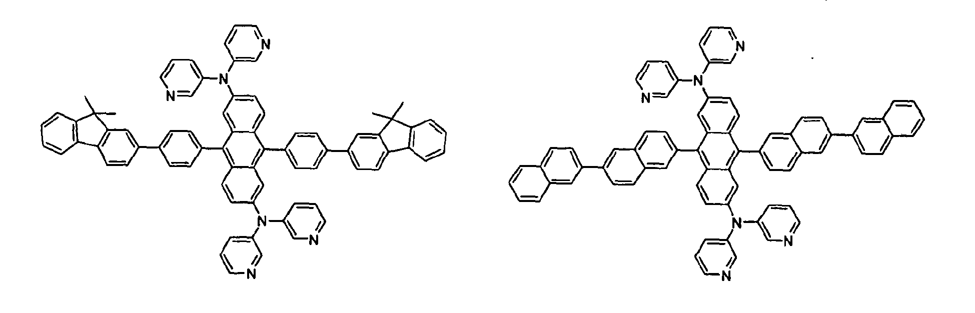

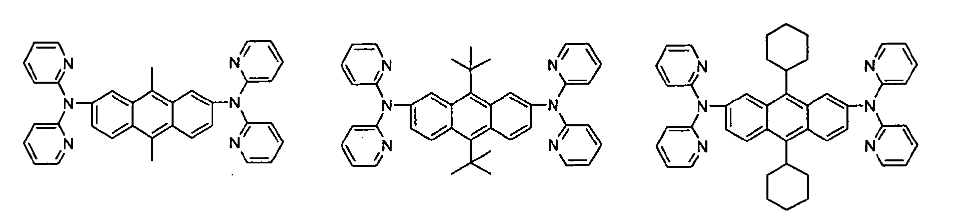

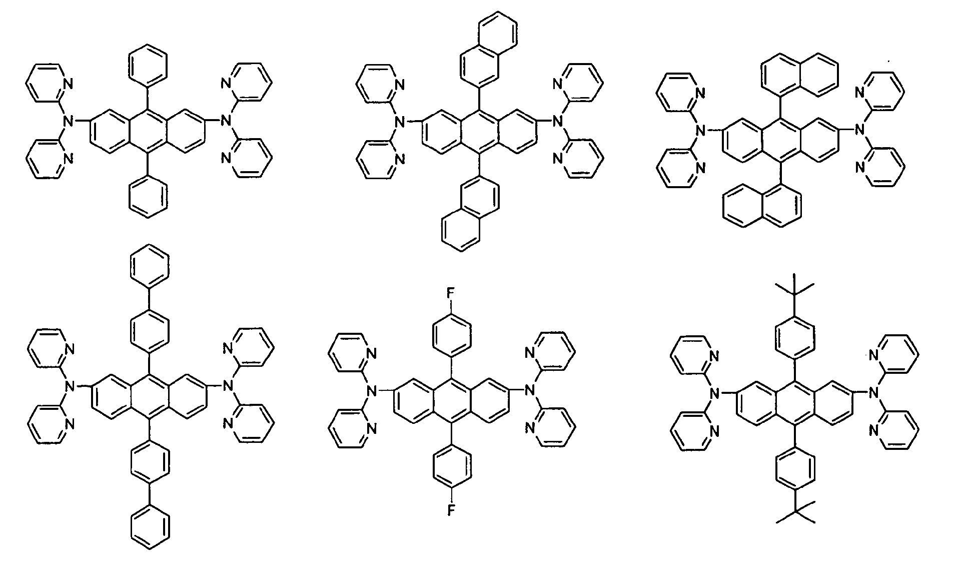

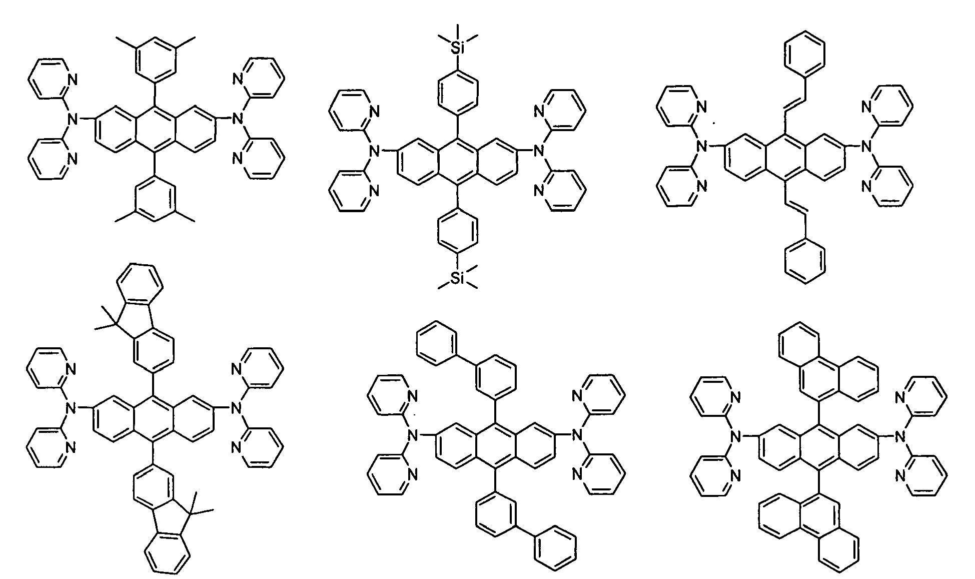

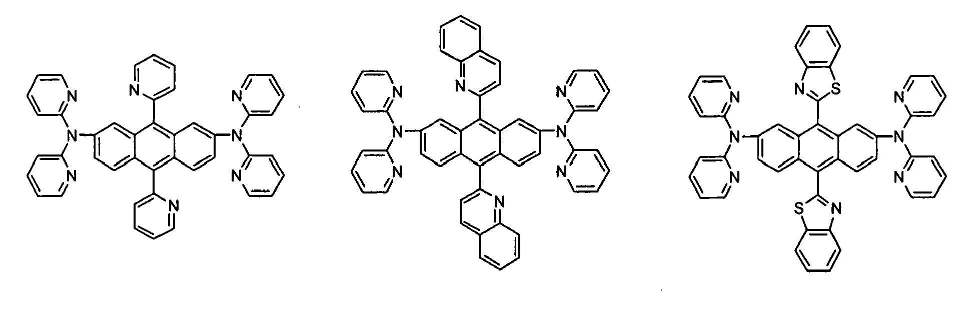

The organic electroluminescent compounds according to the present invention can be prepared according to the procedure illustrated by Reaction Scheme (1) or Reaction Scheme (2): 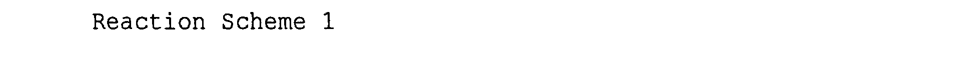 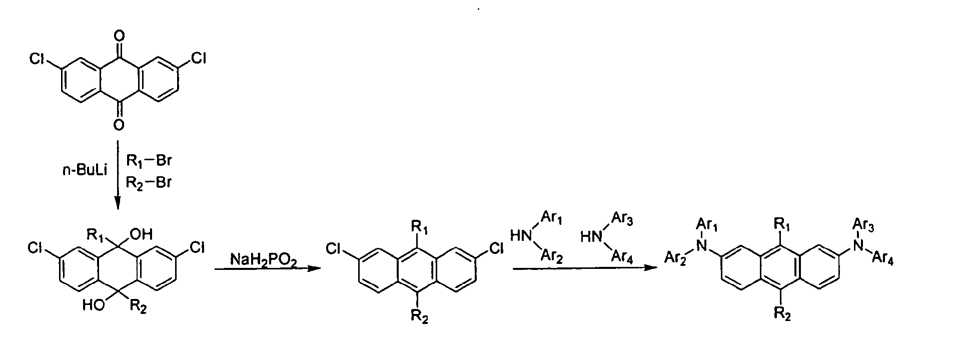 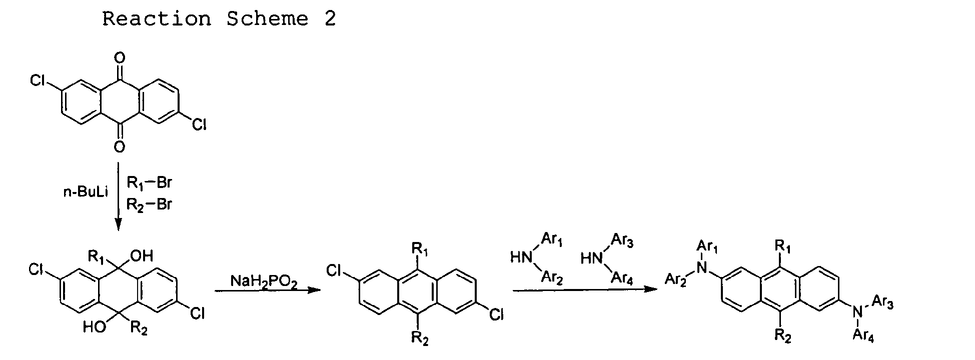 wherein, R₁, R₂, Ar₁, Ar₂, Ar₃ and Ar₄ are defined as in Chemical Formulas (1) and (2).

In addition, the present invention provides organic solar cells, which comprise one or more organic electroluminescent compound(s) represented by Chemical Formula (1) or Chemical Formula (2).

The present invention also provides an organic electroluminescent device which is comprised of a first electrode; a second electrode; and at least one organic layer(s) interposed between the first electrode and the second electrode; wherein the organic layer comprises one or more organic electroluminescent compound(s) represented by Chemical Formula (1) or Chemical Formula (2).

The organic electroluminescent device according to the present invention is **characterized in that** the organic layer comprises an electroluminescent layer, and the electroluminescent layer comprises one or more compound(s) represented by Chemical Formula (1) or Chemical Formula (2) as electroluminescent dopant, and one or more host(s).

The host applied to the electroluminescent device according to the invention is not particularly restricted, but preferably selected from the compounds represented Chemical Formula (3) or (4):

Chemical Formula 3 (Ar₁₁)_{b}-L₁-(Ar₁₂)_{c}

Chemical Formula 4 (Ar₁₃)_{d}-L₂-(Ar₁₄)ₑ

wherein,
L₁ represents (C6-C60)arylene or (C4-C60)heteroarylene;
L₂ represents anthracenylene;
Ar₁₁ through Ar₁₄ are independently selected from hydrogen, deuterium, (C1-C60)alkyl, (C1-C60)alkoxy, halogen, (C4-C60)heteroaryl, (C5-C60)cycloalkyl and (C6-C60)aryl; and the cycloalkyl, aryl or heteroaryl of Ar₁₁ through Ar₁₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, deuterium, (C3-C60)cycloalkyl, halogen, cyano, tri(C1-C60)alkylsilyl, di(C1-C60)alkyl(C6-C60)arylsilyl and tri(C6-C60)arylsilyl; and b, c, d and e independently represent an integer from 0 to 4.

The hosts represented by Chemical Formula (3) or (4) can be exemplified by the derivatives represented by one of Chemical Formulas (5) to (7).

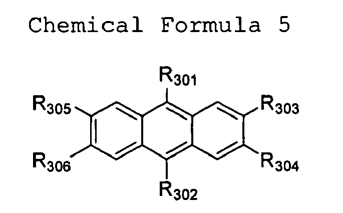

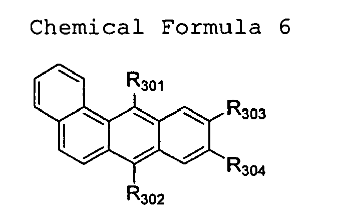

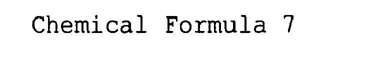

In Chemical Formulas (5) to (7),

R₃₀₁ and R₃₀₂ independently represent (C6-C60)aryl, (C4-C60)heteroaryl, a 5- or 6-membered heterocycloalkyl containing one or more heteroatom(s) selected from N, O and S, or (C3-C60)cycloalkyl, and the aryl or heteroaryl of R₃₀₁ and R₃₀₂ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, deuterium, (C3-C60)cycloalkyl, halogen, cyano, tri(C1-C60)alkylsilyl, di(C1-C60)alkyl(C6-C60)arylsilyl and tri(C6-C60)arylsilyl;

R₃₀₃ through R₃₀₆ independently represent hydrogen, deuterium, (C1-C60)alkyl, (C1-C60)alkoxy, halogen, (C4-C60)heteroaryl, (C5-C60)cycloalkyl or (C6-C60)aryl, and the heteroayl, cycloalkyl or aryl of R₃₀₃ through R₃₀₆ may be further substituted by one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;

B and D independently represent a chemical bond, or (C6-C60)arylene with or without one or more substituent(s) selected from deuterium, (C1-C60)alkyl, (C1-C60)alkoxy, (C6-C60)aryl, (C4-C60)heteroaryl and halogen;

Ar₁₀ and Ar₃₀ represent aryl selected from the following structures, or (C4-C60)heteroaryl, and the aryl or heteroaryl of Ar₁₀ or Ar₃₀ may be substituted by one or more substituent(s) selected from deuterium, (C1-C60)alkyl, (C1-C60)alkoxy, (C6-C60)aryl and (C4-C60)heteroaryl: 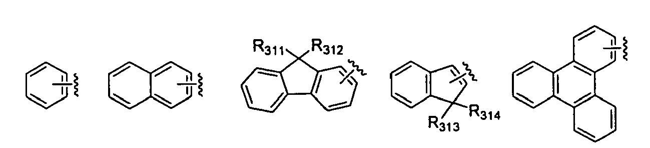 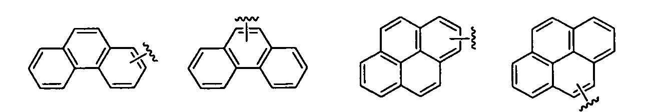 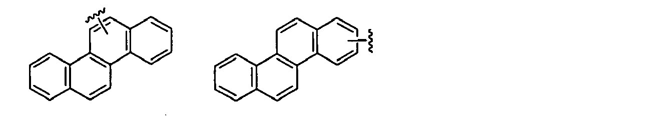 wherein, Ar₂₀ is selected from (C6-C60)arylene or (C4-C60)heteroarylene, preferably from phenylene, naphthylene, anthrylene, fluorenylene, phenanthrylene, tetracenylene, naphthacenylene, chrysenylene, pentacenylene, pyrenylene, heteroarylene and the compounds represented by the following structural formulas; and the arylene or heteroarylene of Ar₂₀ may be substituted by one or more substituent(s) selected from deuterium, (C1-C60)alkyl, (C1-C60)alkoxy, (C6-C60)aryl, (C4-C60)heteroaryl and halogen;

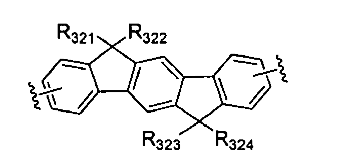

R₃₁₁ through R₃₁₄ independently represent hydrogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;

R₃₂₁ through R₃₂₄ independently represent hydrogen, deuterium, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

The electroluminescent layer means the layer where electroluminescence occurs, and it may be a single layer or a multi-layer consisting of two or more layers laminated. When a mixture of host-dopant is used according to the construction of the present invention, noticeable improvement in luminous efficiency due to the inventive electroluminescent host could be confirmed, as compared to the device simply employing the electroluminescent compound represented by Chemical Formula (1) or Chemical Formula (2). This can be achieved by the doping concentration of 2 to 5wt%. The host according to the present invention exhibits higher hole and electron conductivity, and excellent stability of the material as compared to other conventional host materials, and provides improved device life as well as luminous efficiency.

Thus, it can be described that use of the compound represented by one of Chemical Formulas (3) to (7) as an electroluminescent host significantly supplements electronic drawback of the organic electroluminescent compounds of Chemical Formula (1) or Chemical Formula (2) according to the present invention.

The host compounds represented by one of Chemical Formulas (3) to (7) can be exemplified by the following compounds, but are not restricted thereto.

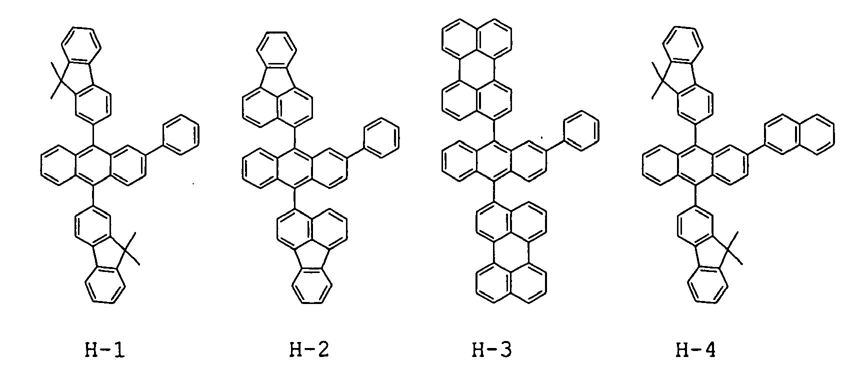

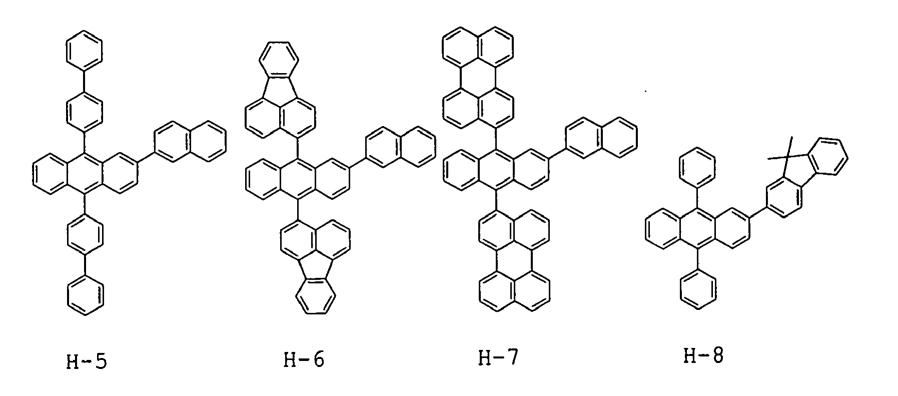

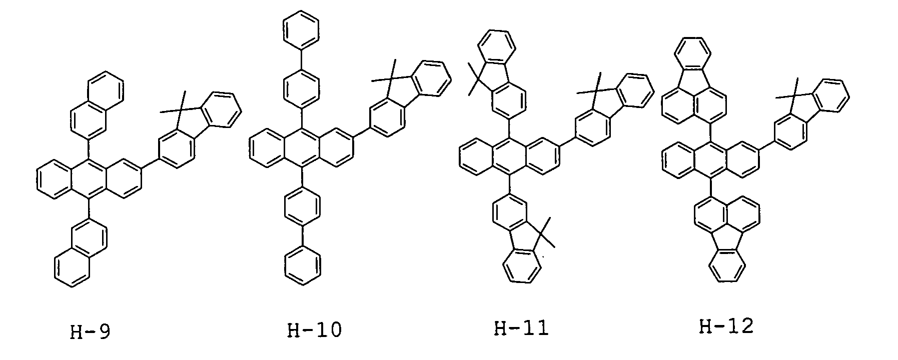

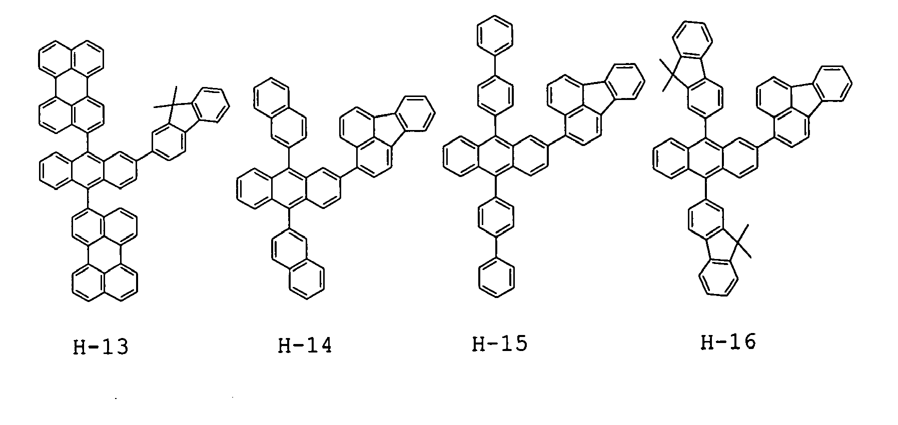

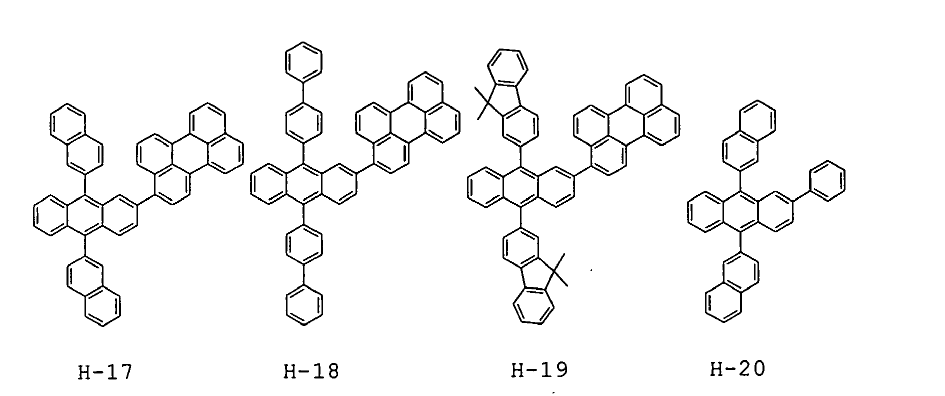

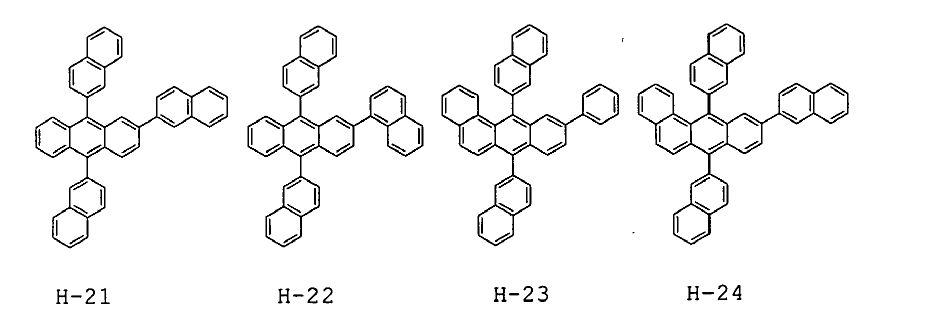

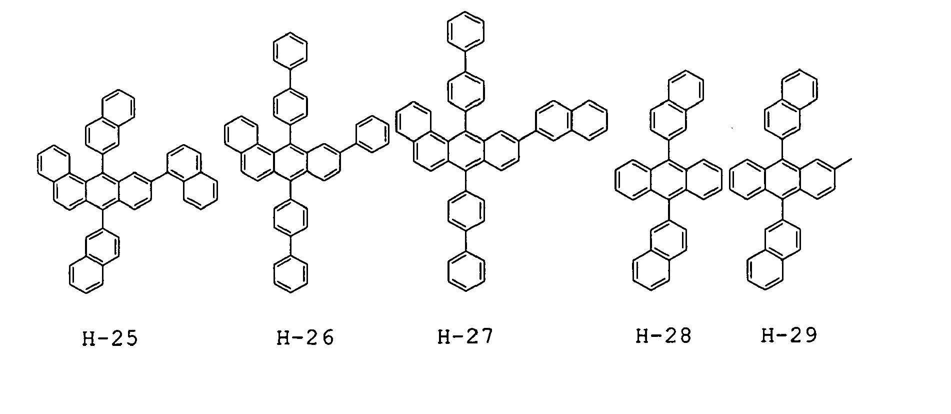

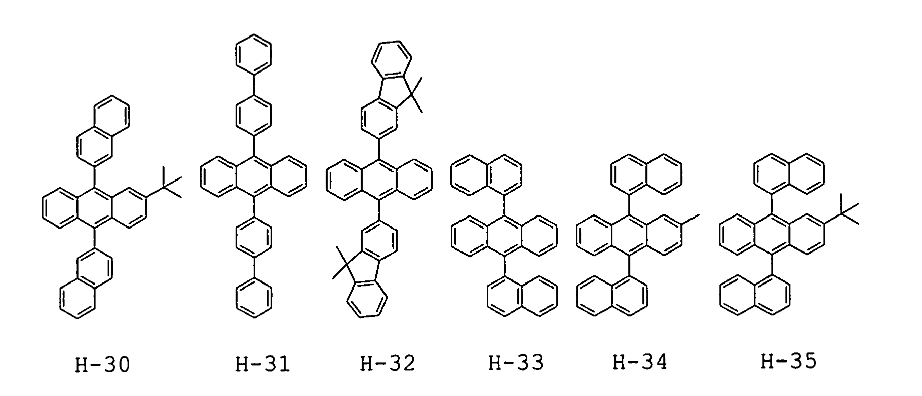

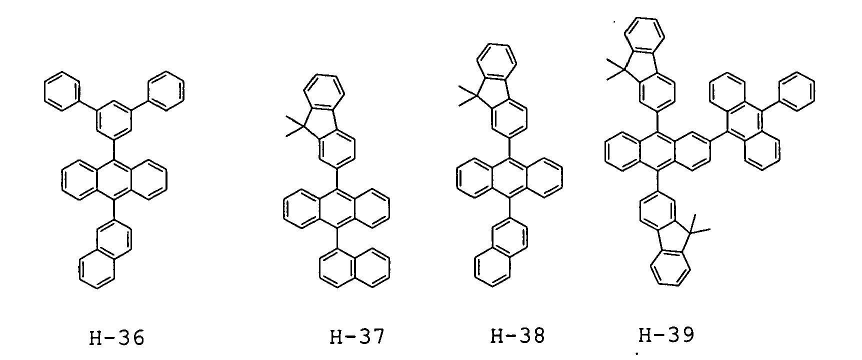

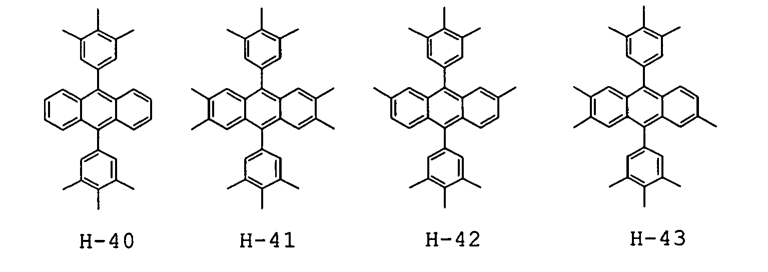

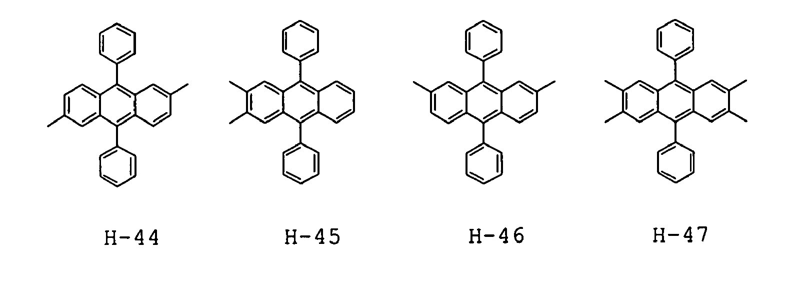

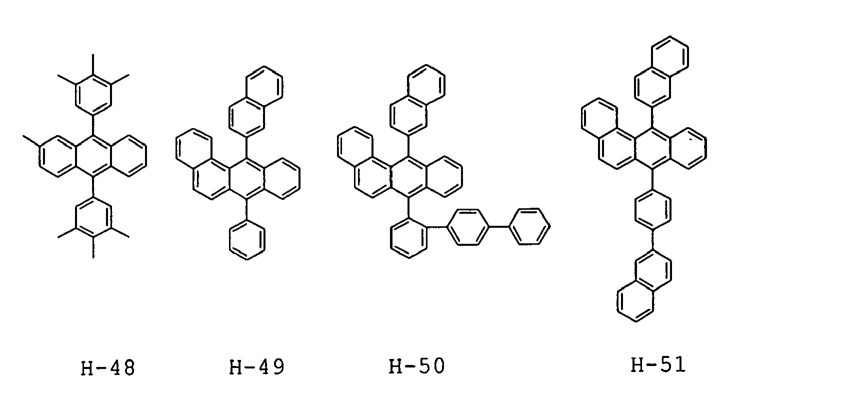

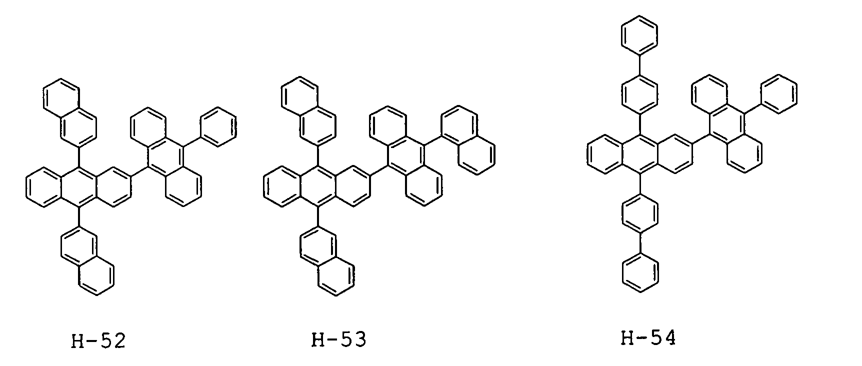

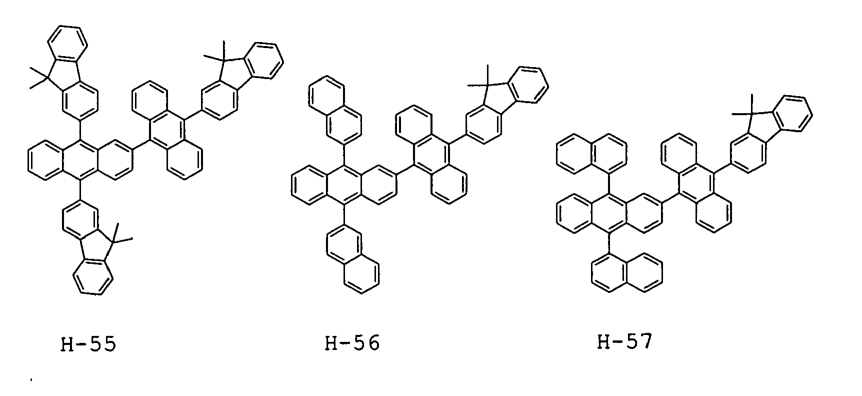

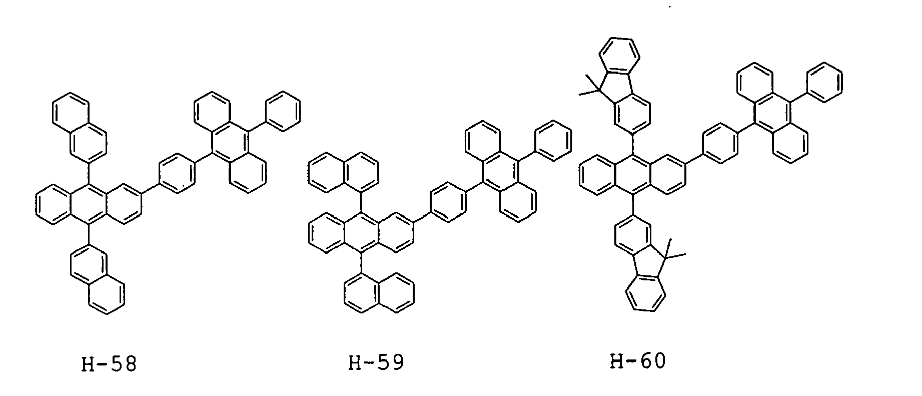

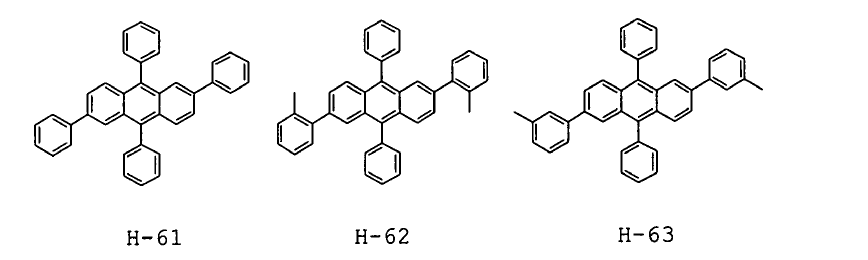

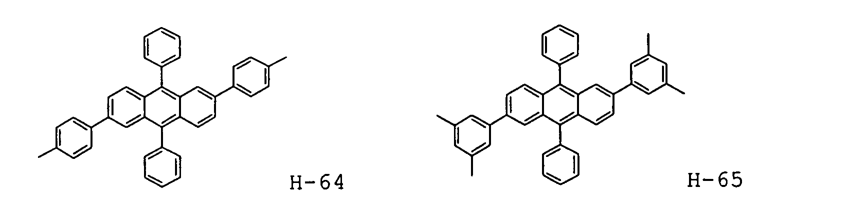

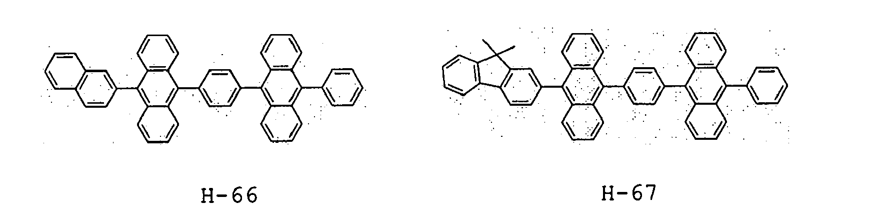

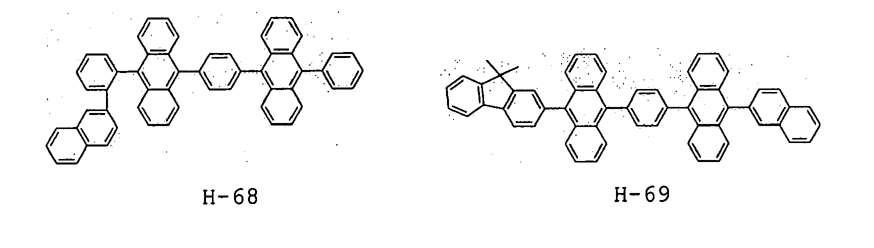

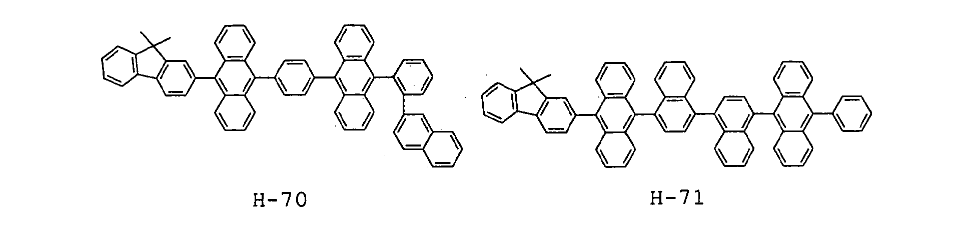

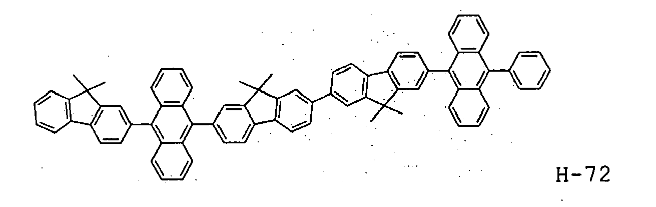

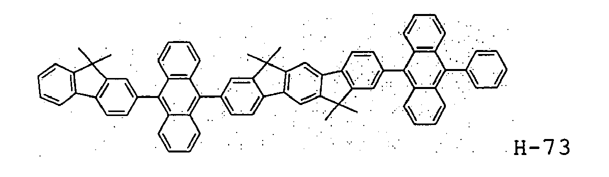

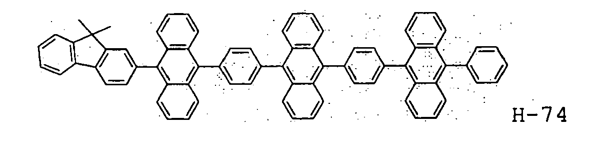

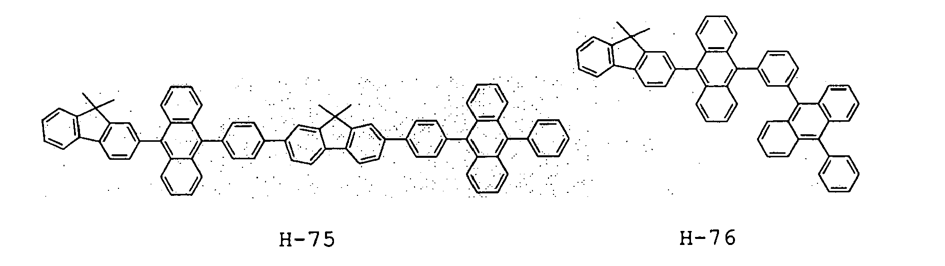

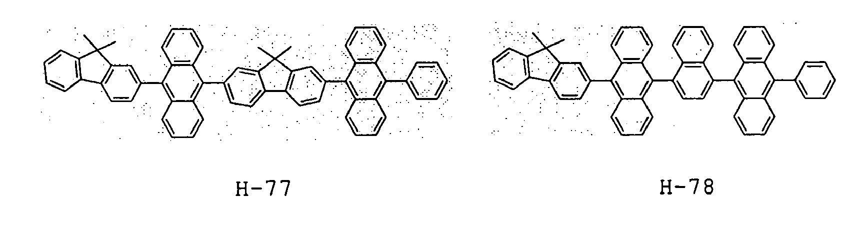

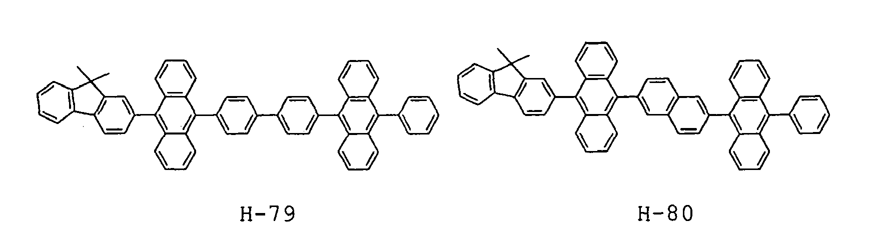

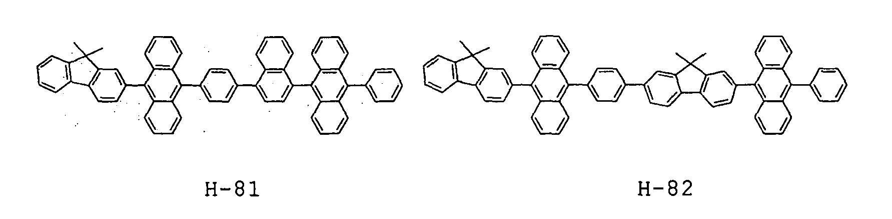

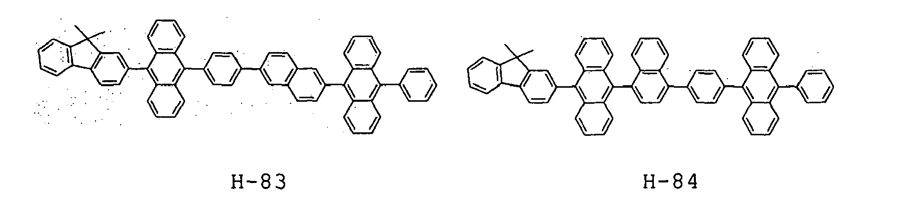

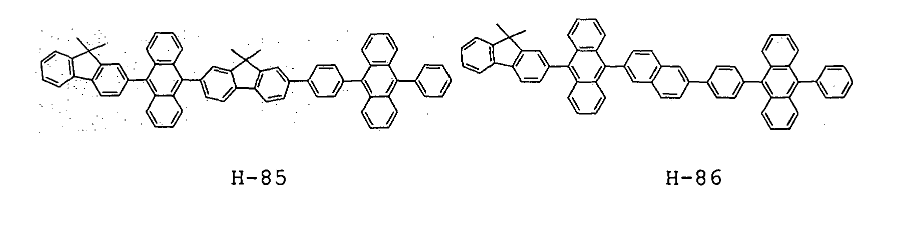

The organic electroluminescent device according to the invention may further comprise one or more compound(s) selected from arylamine compounds and styrylarylamine compounds, as well as the organic electroluminescent compound represented by Chemical Formula (1) or Chemical Formula (2). Examples of the arylamine or styrylarylamine compounds include the compounds represented by Chemical Formula (8), but they are not restricted thereto: 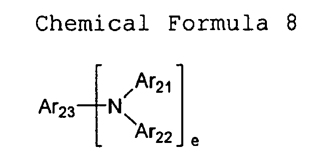 wherein, Ar₂₁ and Ar₂₂ independently represent hydrogen, deuterium, halogen, (C1-C60)alkyl, (C6-C60)aryl, (C4-C60)heteroaryl, mono or di-(C6-C60)arylamino, mono or di-(C1-C60)alkylamino, a 5- or 6-membered heterocycloalkyl containing one or more heteroatom(s) selected from N, O and S, or (C3-C60)cycloalkyl, or Ar₂₁ and Ar₂₂ may be linked via (C3-C60)alkylene or (C3-C60)alkenylene with or without a fused ring to form an alicyclic ring, or a monocyclic or polycyclic aromatic ring;
when e is 1, Ar₂₃ represents (C6-C60) aryl, (C4-C60)heteroaryl, or a substituent represented by one of the following structural formulas: 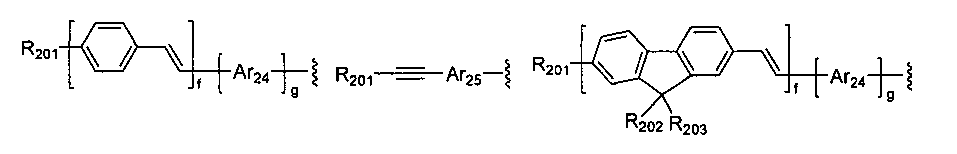 when e is 2, Ar₂₃ represents (C6-C60)arylene, (C4-C60)heteroarylene, or a substituent selected from the following structures: 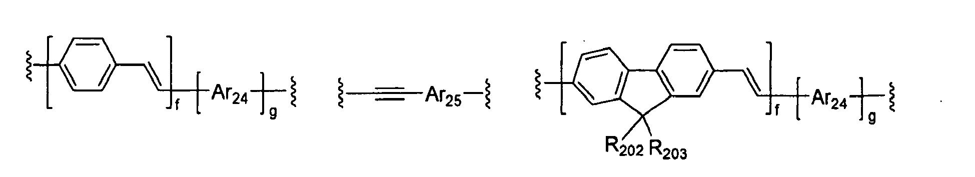 wherein Ar₂₄ and Ar₂₅ independently represent (C6-C60)arylene or (C4-C60)heteroarylene;
R₂₀₁ through R₂₀₃ independently represent hydrogen, halogen, deuterium, (C1-C60)alkyl or (C6-C60)aryl;
f is an integer from 1 to 4, g is an integer of 0 or 1; and
the alkyl, aryl, heteroaryl, arylamino, alkylamino, cycloalkyl or heterocycloalkyl of Ar₂₁ and Ar₂₂, or the aryl, heteroaryl, arylene or heteroarylene of Ar₂₃, or the arylene or heteroarylene of Ar₂₄ and Ar₂₅, or the alkyl or aryl of R₂₀₁ through R₂₀₃ may be further substituted by one or more substituent(s) selected from a group consisting of deuterium, halogen, (C1-C60)alkyl, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono or di-(C1-C60)alkylamino, mono or di-(C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

The arylamine compounds and styrylarylamine compounds may be more specifically exemplified by the following compounds, but are not restricted thereto.

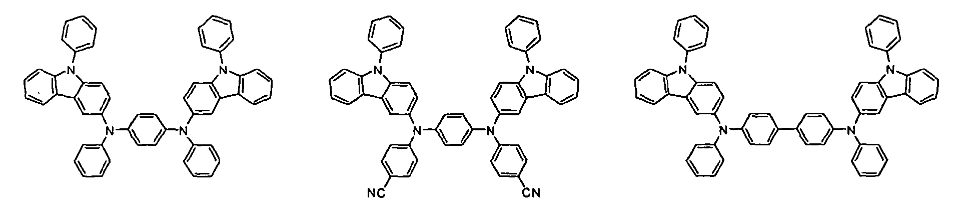

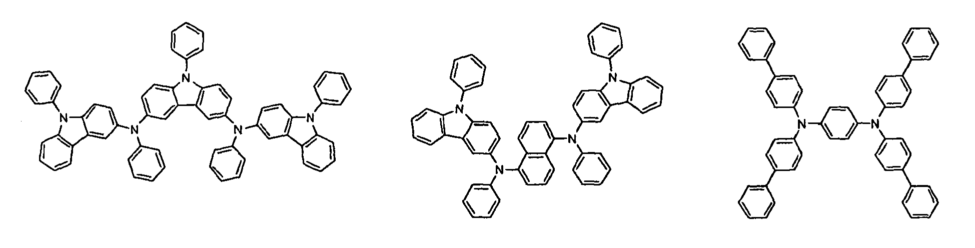

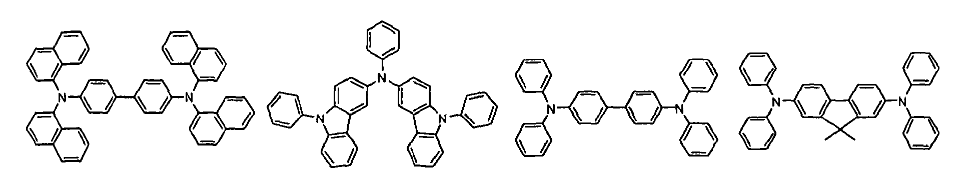

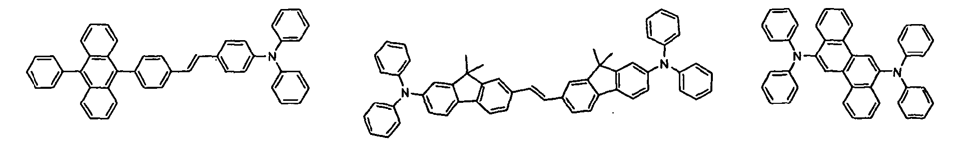

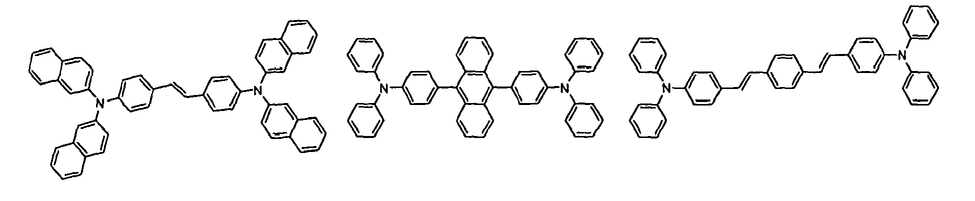

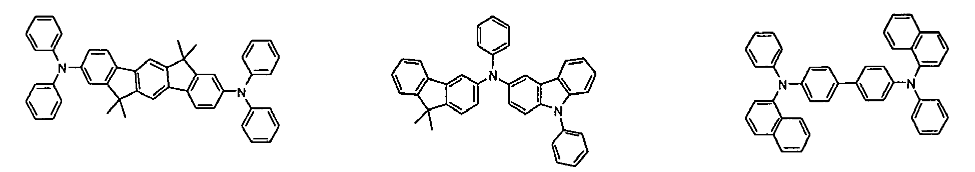

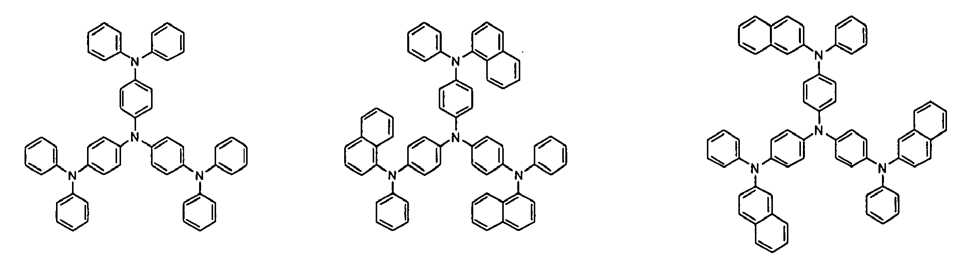

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements from the Periodic Table of Elements, as well as the organic electroluminescent compound represented by Chemical Formula (1) or Chemical Formula (2). The organic layer may comprise an electroluminescent layer and a charge generating layer at the same time.

The present invention can realize an electroluminescent device having a pixel structure of independent light-emitting mode, which comprises an organic electroluminescent device containing the compound of Chemical Formula (1) or Chemical Formula (2) as a sub-pixel, and one or more sub-pixel(s) comprising one or more compounds selected from a group consisting of Ir, Pt, Pd, Rh, Re, Os, Tl, Pb, Bi, In, Sn, Sb, Te, Au and Ag, patterned in parallel at the same time.

Further, the organic electroluminescent device may be an organic display wherein the organic layer comprises, in addition to the organic electroluminescent compound described above, one or more compound(s) selected from compounds having the electroluminescent peak of wavelength of not more than 500 nm, and those having the electroluminescent peak of wavelength of not less than 560 nm, at the same time. The compounds having electroluminescent peak of wavelength of not more than 500 nm, or those having the electroluminescent peak of wavelength of not less than 560 nm may be exemplified by the compounds represented by one of Chemical Formulas (9) to (15), but they are not restricted thereto.
Chemical Formula 9
M¹L³L⁴L⁵

In Chemical Formula (9), M¹ is selected from Group 7, 8, 9, 10, 11, 13, 14, 15 and 16 metals in the Periodic Table of Elements, and ligands L³, L⁴ and L⁵ are independently selected from the following structures: 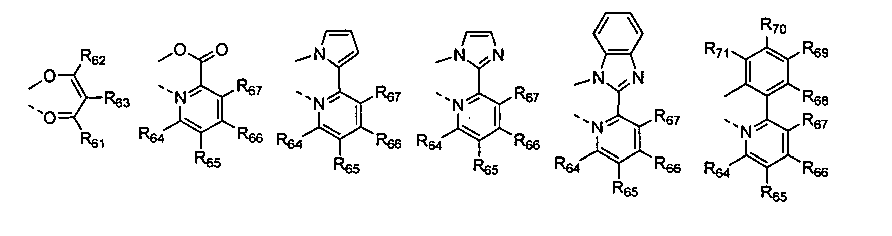 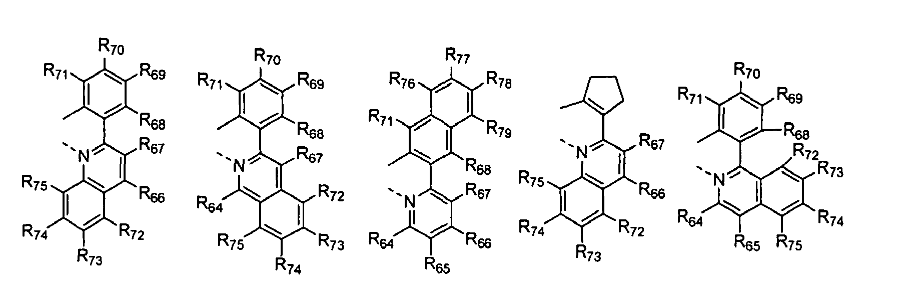 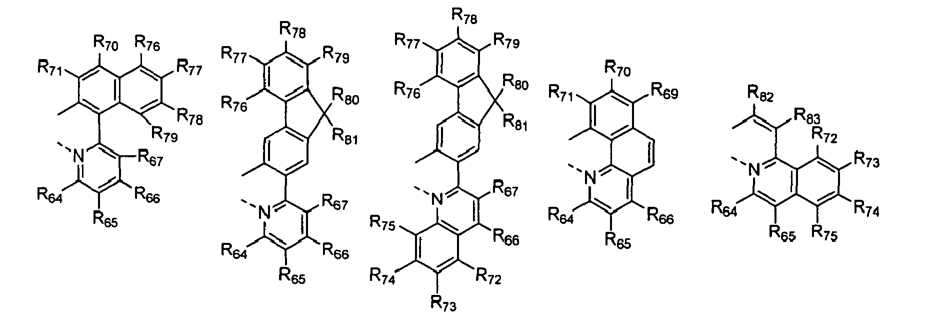 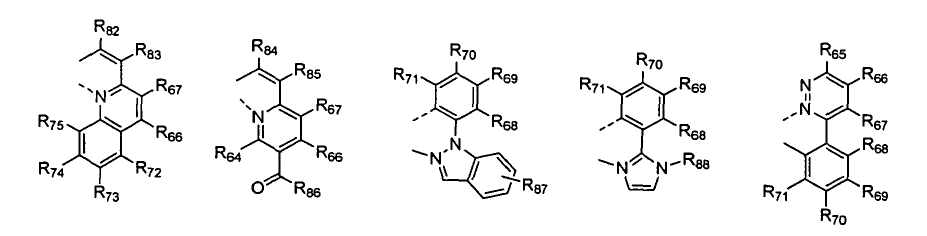 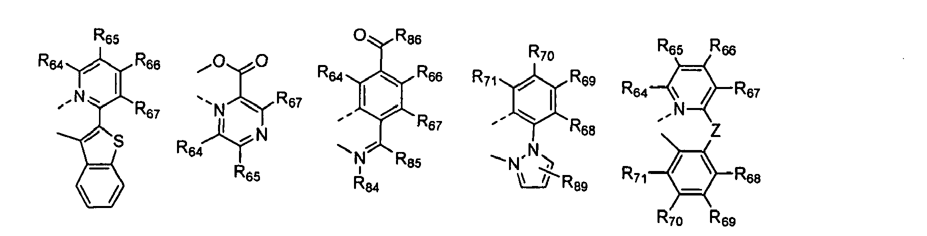 wherein, R₆₁ through R₆₃ independently represent hydrogen, deuterium, (C1-C60)alkyl with or without halogen substituent(s), (C6-C60)aryl with or without (C1-C60)alkyl substituent(s), or halogen;

R₆₄ through R₇₉ independently represent hydrogen, deuterium, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen, and R₇₀ and R₇₆ may be linked to an adjacent substituent via (C2-C12)alkylene or (C2-C12)alkenylene to form a fused ring or a multi-fused ring; and the alkyl, cycloalkyl, alkenyl or aryl of R₆₄ through R₇₉, or the fused ring or multi-fused ring formed from R₇₀ and R₇₆ via alkylene or alkenylene may be further substituted by one or more substituent(s) selected from deuterium, (C1-C60)alkyl, (C6-C60)aryl and halogen;

R₈₀ through R₈₃ independently represent hydrogen, deuterium, (C1-C60)alkyl with or without halogen substituent(s), or (C6-C60)aryl with or without (C1-C60)alkyl substituent(s);

R₈₄ and R₈₅ independently represent hydrogen, deuterium, (C1-C60)alkyl, (C6-C60)aryl or halogen, or R₈₄ and R₈₅ may be linked via (C3-C12)alkylene or (C3-C12)alkenylene with or without a fused ring to form an alicyclic ring, or a monocyclic or polycyclic aromatic ring; and the alkyl or aryl of R₈₄ and R₈₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from deuterium, (C1-C60)alkyl with or without halogen substituent(s), (C1-C30)alkoxy, halogen, tri(C1-C30)alkylsilyl, tri(C6-C30)arylsilyl and (C6-C60)aryl;

R₈₆ represents (C1-C60)alkyl, (C6-C60)aryl, (C5-C60)heteroaryl or halogen;

R₈₇ through R₈₉ independently represent hydrogen, deuterium, (C1-C60)alkyl, (C6-C60)aryl or halogen, and the alkyl or aryl of R₈₆ through R₈₉ may be further substituted by halogen or (C1-C60)alkyl; Z represents 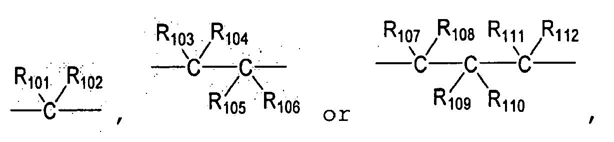 and R₁₀₁ through R₁₁₂ independently represent hydrogen, deuterium, (C1-C60)alkyl with or without halogen substituent(s), (C1-C30)alkoxy, halogen, (C6-C60)aryl, cyano or (C5-C60)cycloalkyl, or each of R₁₀₁ through R₁₁₂ may be linked to an adjacent substituent via alkylene or alkenylene to form a (C5-C7) spiro-ring or (C5-C9) fused ring, or each of them may be linked to R₆₇ or R₆₈ via alkylene or alkenylene to form a (C5-C7) fused ring.

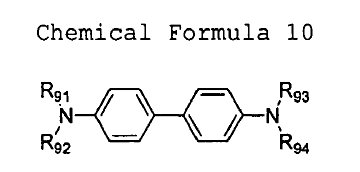

In Chemical Formula (10), R₉₁ through R₉₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₉₁ through R₉₄, or the alicyclic ring, or the monocyclic or polycyclic aromatic ring formed therefrom by linkage via (C3-C60)alkylene or (C3-C60)alkenylene with or without a fused ring may be further substituted by one or more substituent(s) selected from deuterium, (C1-C60)alkyl with or without halogen substituent(s), (C1-C60)alkoxy, halogen, tri(C1-C60)alkylsilyl, tri(C6-C60)arylsilyl and (C6-C60)aryl.

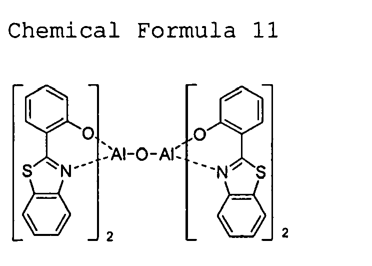

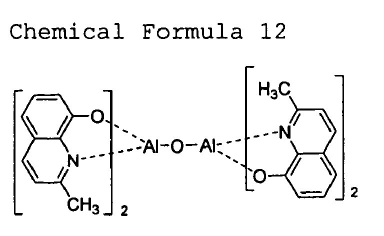

Chemical Formula 13 L¹¹L¹²M²(Q)_{y}

In Chemical Formula (13), the ligands, L¹¹ and L¹² are independently selected from the following structures:

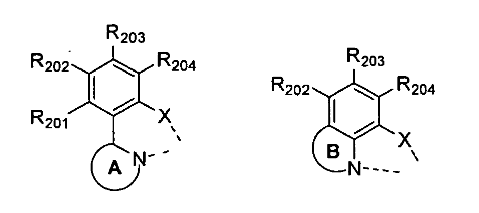

M² is a bivalent or trivalent metal; y is 0 when M² is a bivalent metal, while y is 1 when M² is a trivalent metal;

Q represents (C6-C60)aryloxy or tri(C6-C60)arylsilyl, and the aryloxy and triarylsilyl of Q may be further substituted by (C1-C60)alkyl or (C6-C60)aryl;

X represents O, S or Se; ring A represents oxazole, thiazole, imidazole, oxadiazole, thiadiazole, benzoxazole, benzothiazole, benzimidazole, pyridine or quinoline;

ring B represents pyridine or quinoline, and ring B may be further substituted by (C1-C60)alkyl, or phenyl or naphthyl with or without (C1-C60)alkyl substituent(s);

R₂₀₁ through R₂₀₄ independently represent hydrogen, deuterium, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₂₀₁ to form a fused ring;

ring A or the aryl group of R₂₀₁ through R₂₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

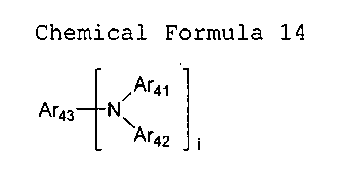

In Chemical Formula (14), Ar₄₁ and Ar₄₂ independently represent hydrogen, deuterium, halogen, (C1-C60)alkyl, (C6-C60)aryl, (C4-C60)heteroaryl, mono or di-(C6-C60)arylamino, mono or di-(C1-C60)alkylamino, 5- or 6-membered heterocycloalkyl containing one or more heteroatom(s) selected from N, O and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring;
when i is 1, Ar₄₃ represents (C6-C60)aryl, (C4-C60)heteroaryl, or an aryl group represented by one of the following structural formulas: 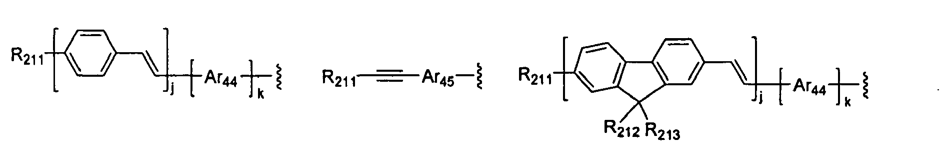 when i is 2, Ar₄₃ represents (C6-C60)arylene, (C4-C60)heteroarylene, or arylene represented by one of the following structural formulas: 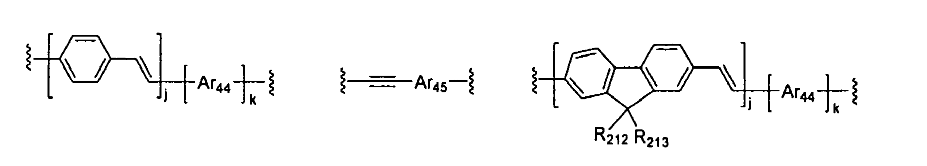 wherein Ar₄₄ and Ar₄₅ independently represent (C6-C60)arylene or (C4-C60)heteroarylene;

R₂₁₁ through R₂₁₃ independently represent hydrogen, deuterium, (C1-C60)alkyl or (C6-C60)aryl;

j is an integer from 1 to 4, k is an integer of 0 or 1; and the alkyl, aryl, heteroaryl, arylamino, alkylamino, cycloalkyl or heterocycloalkyl of Ar₄₁ and Ar₂₂; the alicyclic ring, or the monocyclic or polycyclic aromatic ring formed from Ar₄₁ and Ar₄₂ via alkylene or alkenylene; the aryl, heteroaryl, arylene or heteroarylene of Ar₄₃, or the arylene or heteroarylene of Ar₄₄ and Ar₄₅; or the alkyl or aryl of R₂₁₁ through R₂₁₃ may be further substituted by one or more substituent(s) selected from a group consisting of halogen, deuterium, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, mono or di-(C1-C60)alkylamino, mono or di-(C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, (C6-C60)aryloxycarbonyl, (C1-C60)alkoxycarbonyloxy, (C1-C60)alkylcarbonyloxy, (C6-C60)arylcarbonyloxy, (C6-C60)aryloxycarbonyloxy, (C1-C60)alkylcarbonyloxy, (C6-C60)arylcarbonyloxy, carboxyl, nitro and hydroxyl.

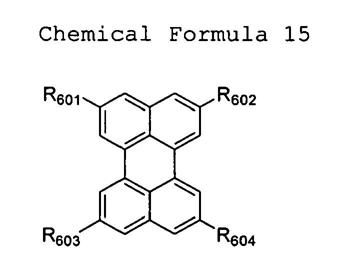

In Chemical Formula (15), R₆₀₁ through R₆₀₄ independently represent hydrogen, deuterium, halogen, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, (C1-C60)alkylcarbonyl, (C6-C60)arylcarbonyl, (C6-C60)aryloxycarbonyl, (C1-C60)alkoxycarbonyloxy, (C1-C60)alkylcarbonyloxy, (C6-C60)arylcarbonyloxy, (C6-C60)aryloxycarbonyloxy, (C1-C60)alkylcarbonyloxy, (C6-C60)arylcarbonyloxy, carboxyl, nitro or hydroxyl, or each of R₆₀₁ through R₆₀₄ may be linked to an adjacent substituent via (C3-C60)alkylene or (C3-C60)alkenylene with or without a fused ring to form an alicyclic ring, or a monocyclic or polycyclic aromatic ring; the alkyl, alkenyl, alkynyl, cycloalkyl, heterocycloalkyl, aryl, heteroaryl, arylsilyl, alkylsilyl, alkylamino or arylamino of R₆₀₁ through R₆₀₄, or the alicyclic ring, or the monocyclic or polycyclic aromatic ring formed therefrom by linkage to an adjacent substituent via (C3-C60)alkylene or (C3-C60)alkenylene with or without a fused ring may be further substituted by one or more substituent(s) selected from halogen, deuterium, (C1-C60)alkyl, (C6-C60)aryl, (C4-C60)heteroaryl, a 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, (C1-C60)alkylcarbonyl, (C6-C60)arylcarbonyl, (C6-C60)aryloxycarbonyl, (C1-C60)alkoxycarbonyloxy, (C1-C60)alkylcarbonyloxy, (C6-C60)arylcarbonyloxy, (C6-C60)aryloxycarbonyloxy, (C1-C60)alkylcarbonyloxy, (C6-C60)arylcarbonyloxy, carboxyl, nitro and hydroxyl.

The compounds having electroluminescent peak of wavelength of not more than 500 nm, or those having electroluminescent peak of wavelength of not less than 560 nm, may be exemplified by the following compounds, but they are not restricted thereto.

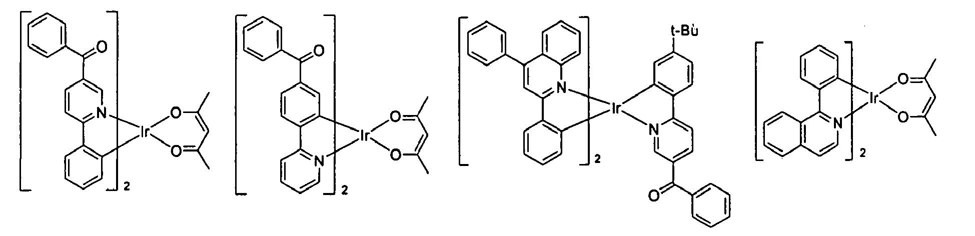

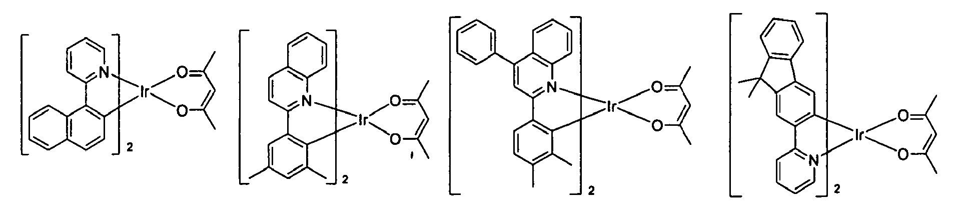

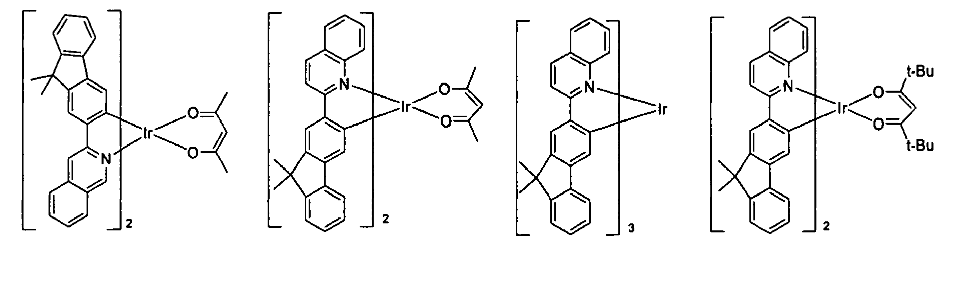

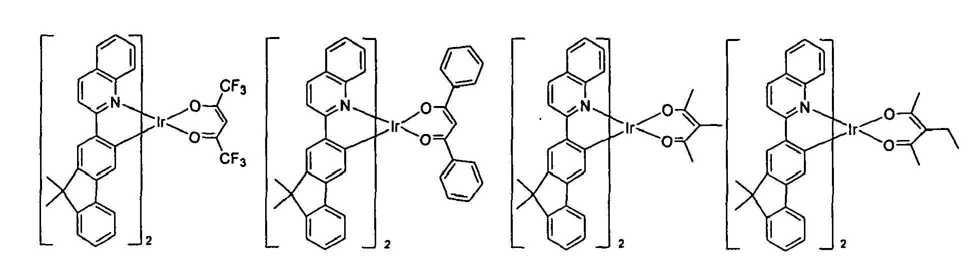

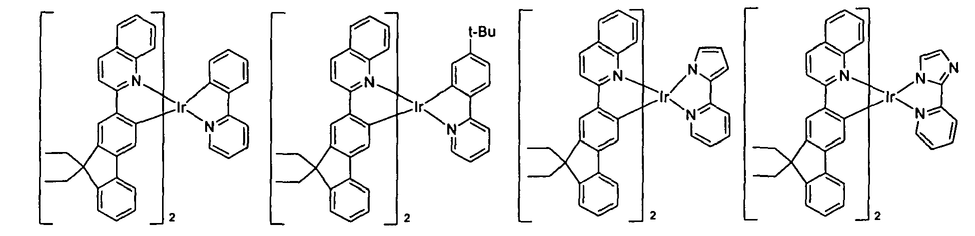

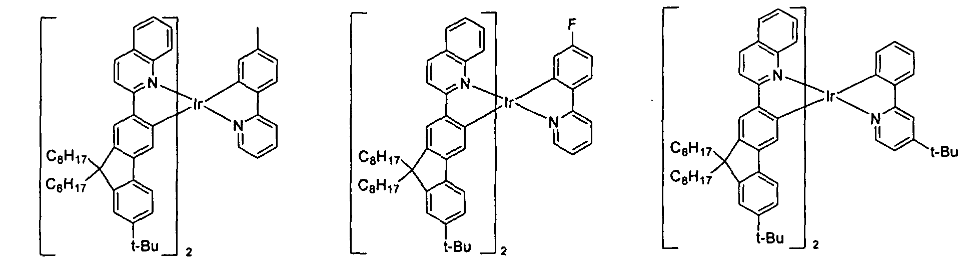

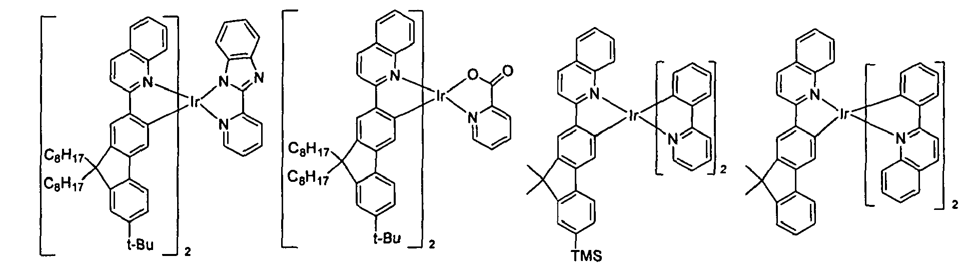

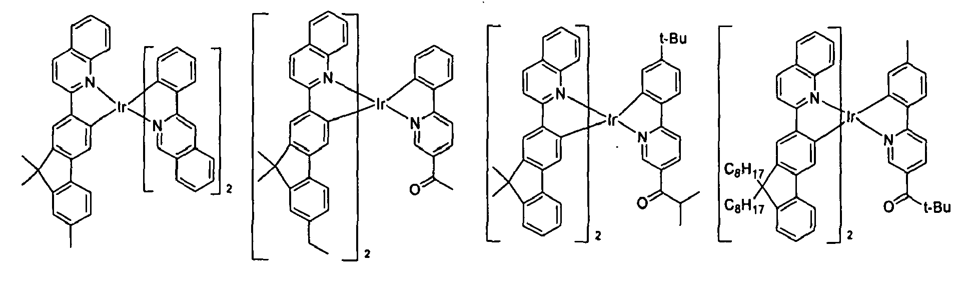

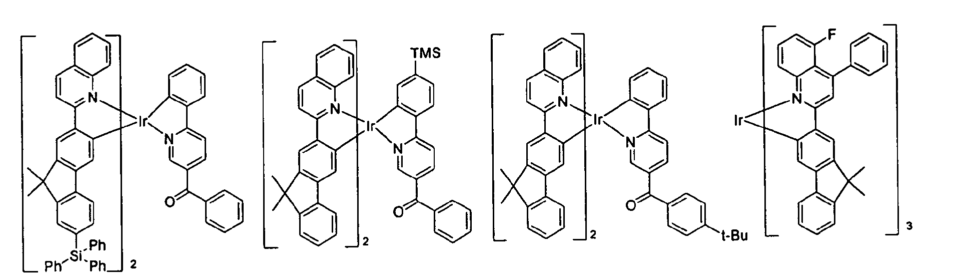

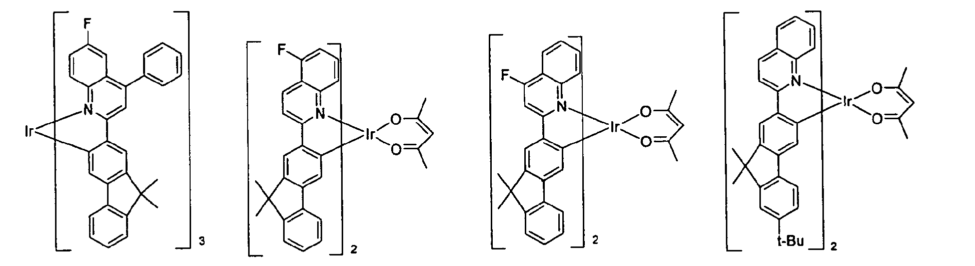

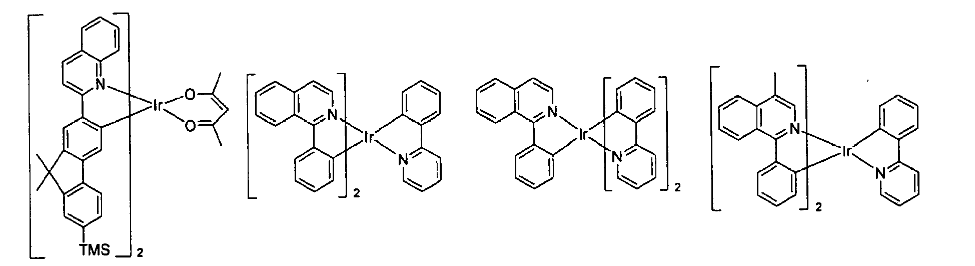

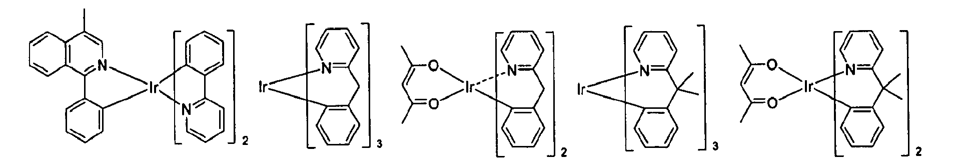

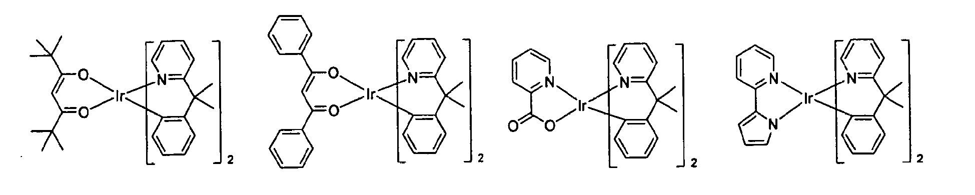

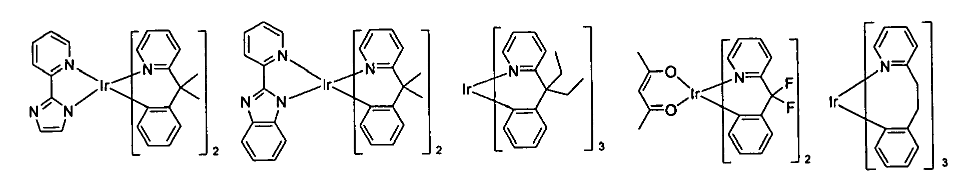

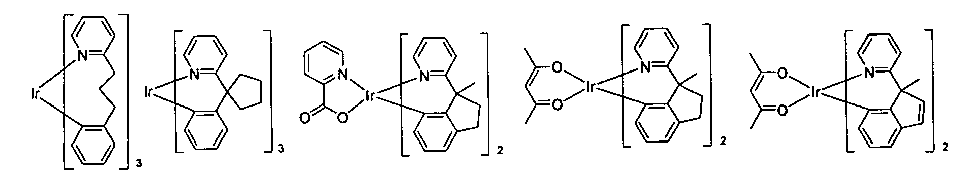

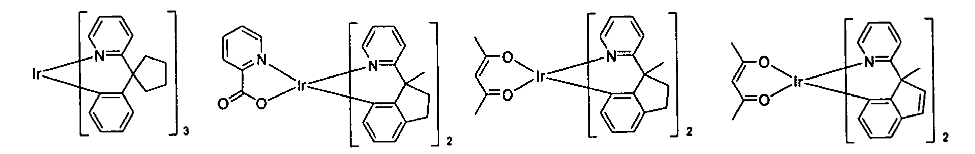

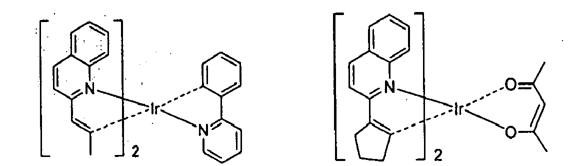

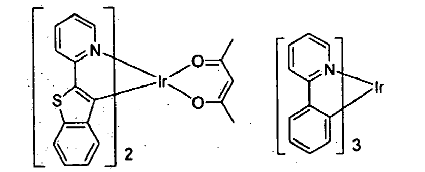

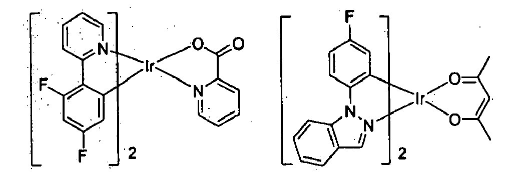

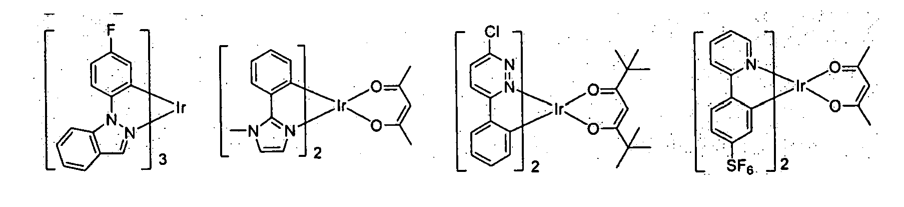

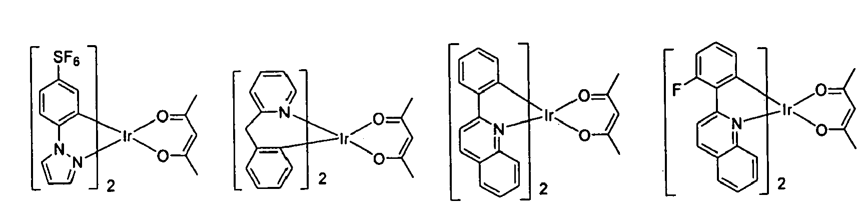

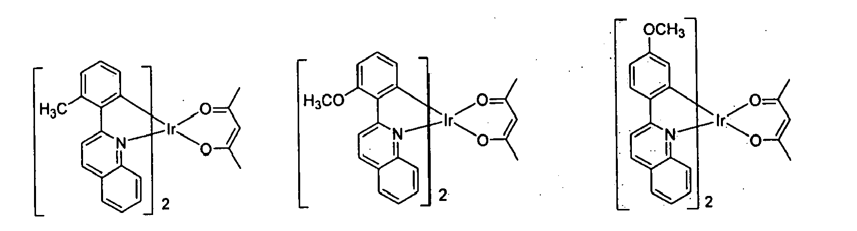

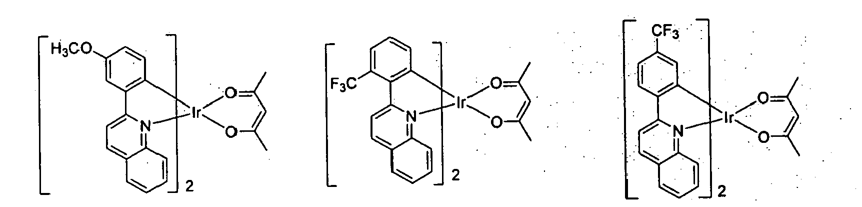

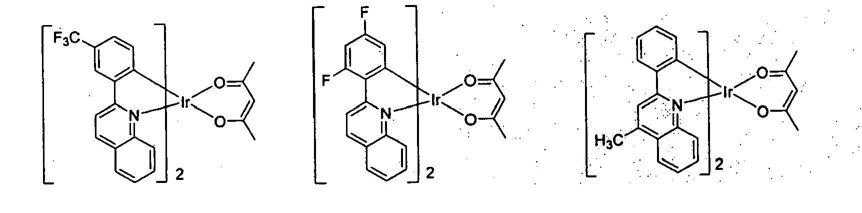

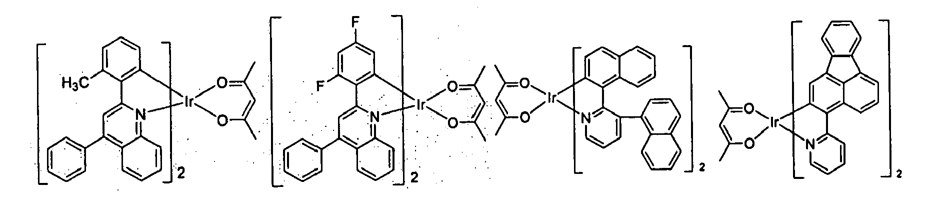

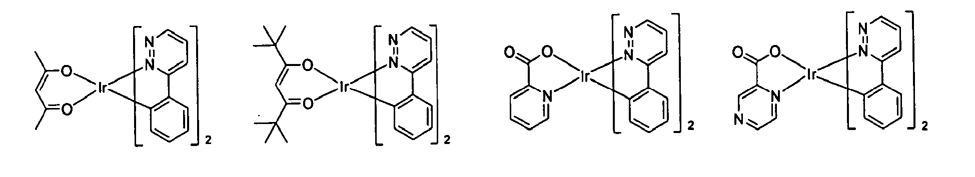

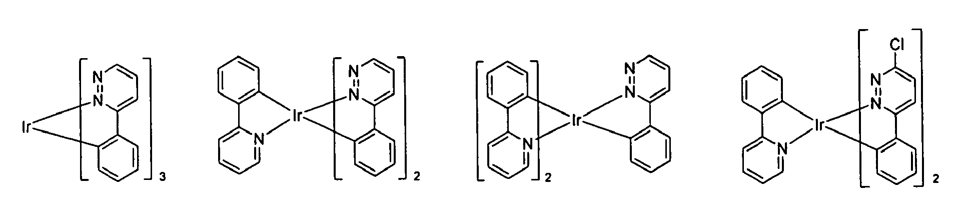

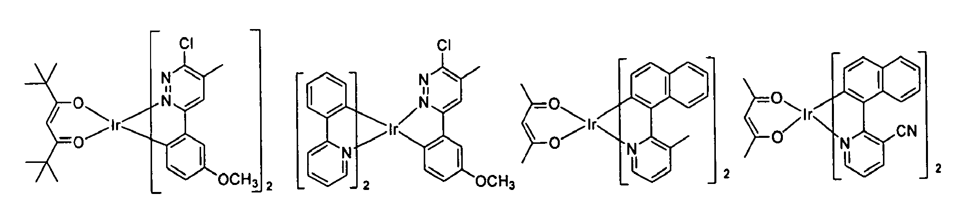

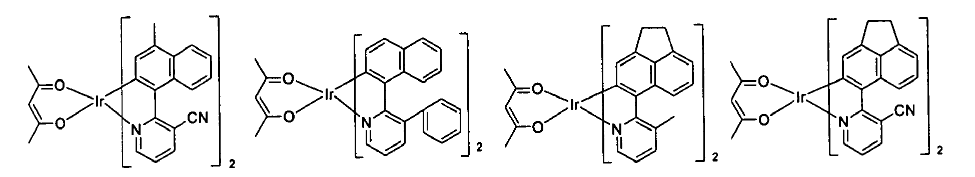

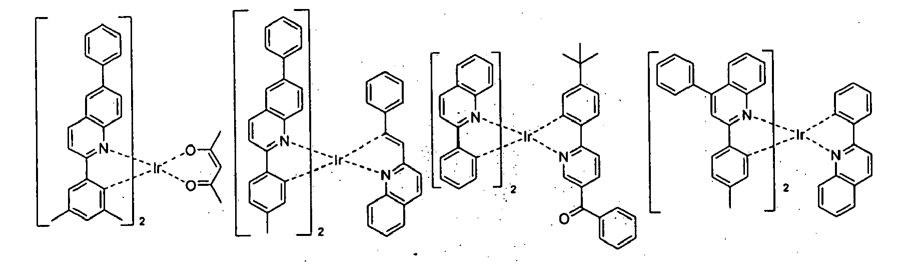

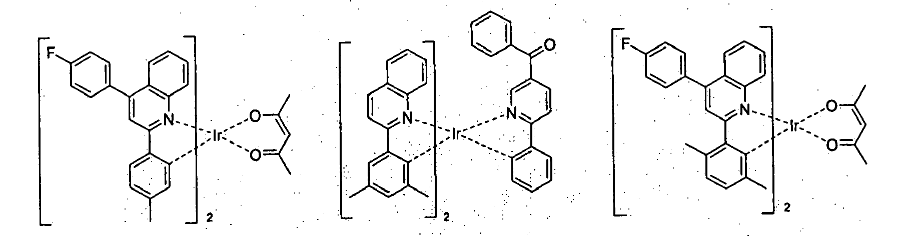

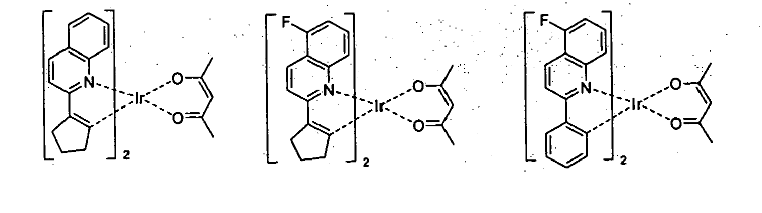

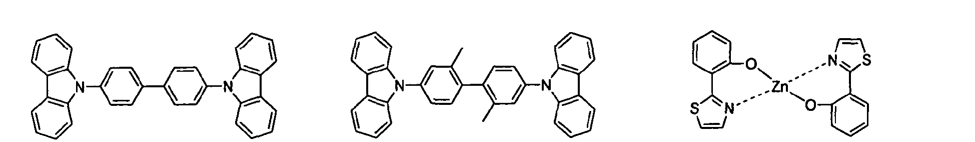

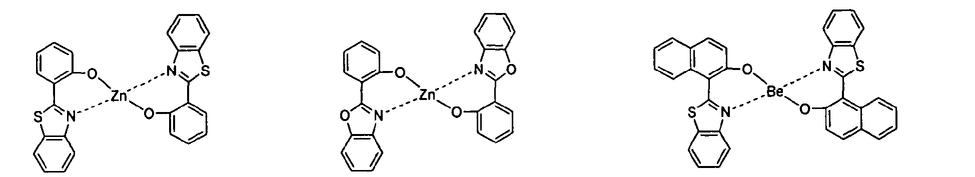

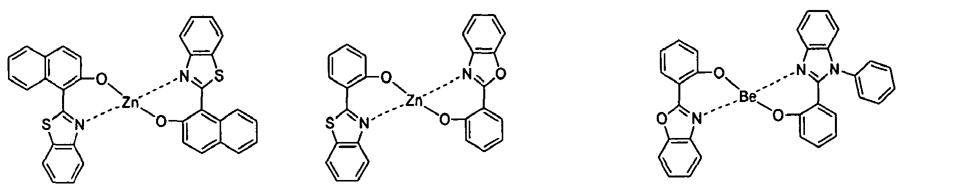

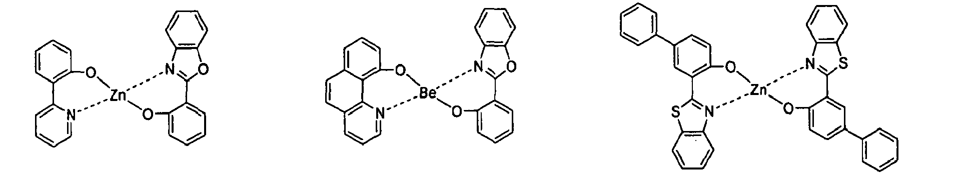

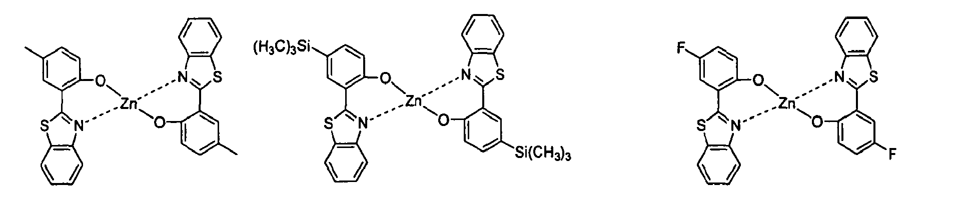

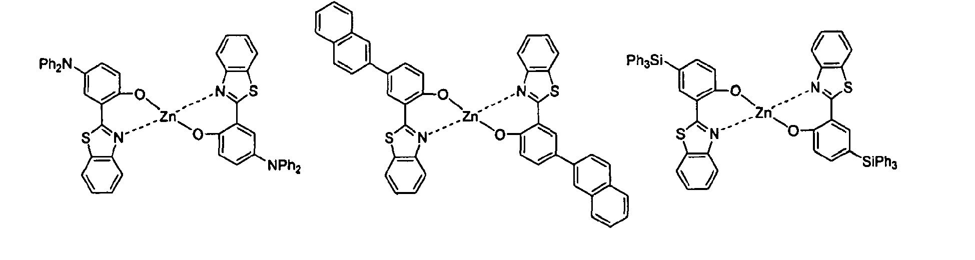

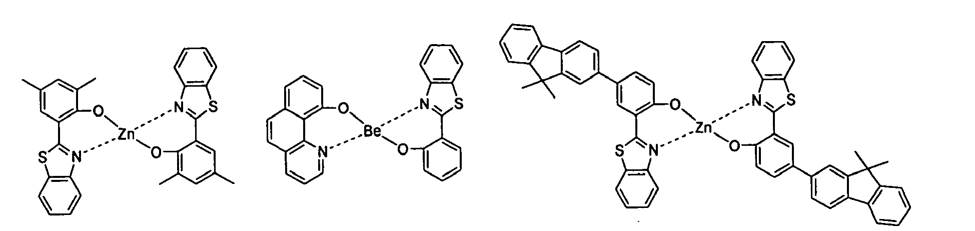

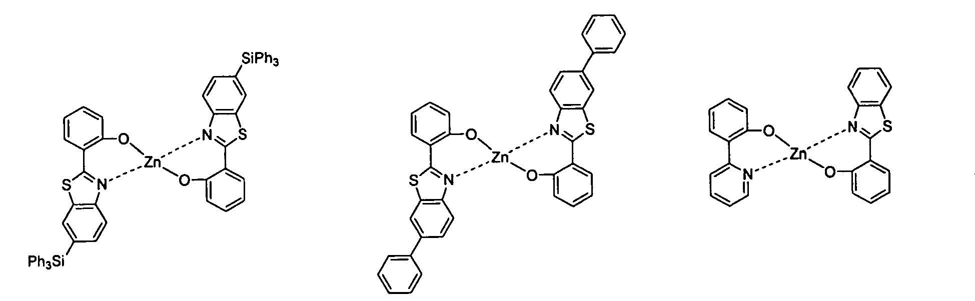

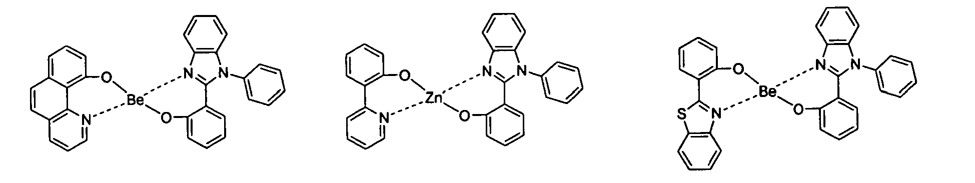

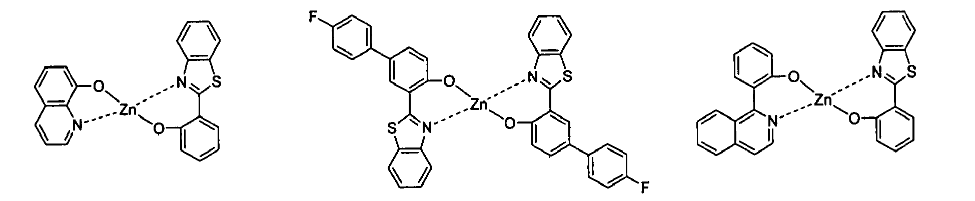

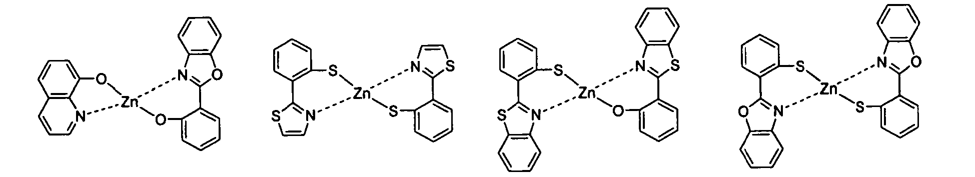

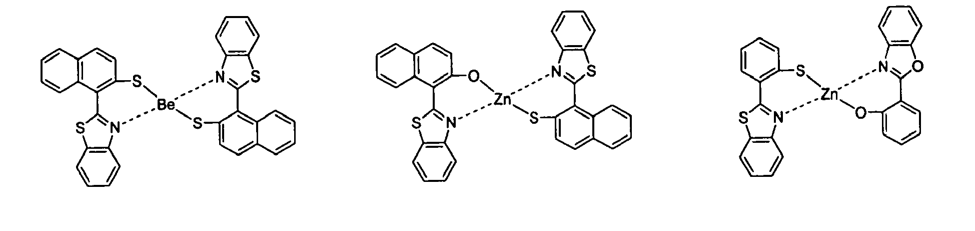

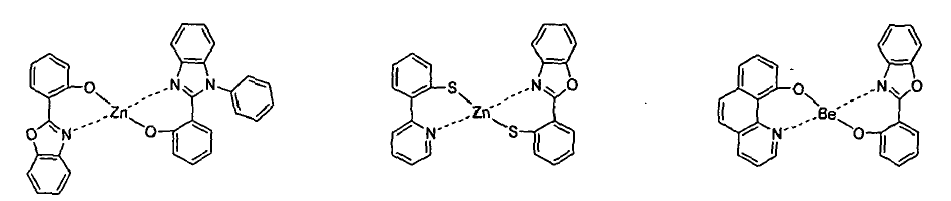

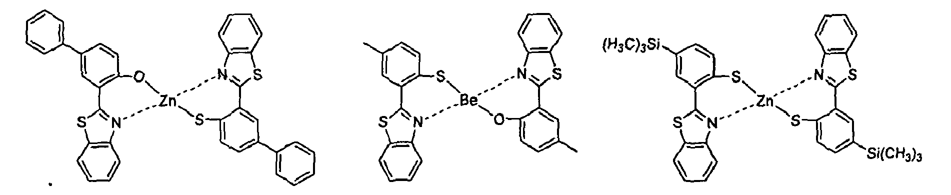

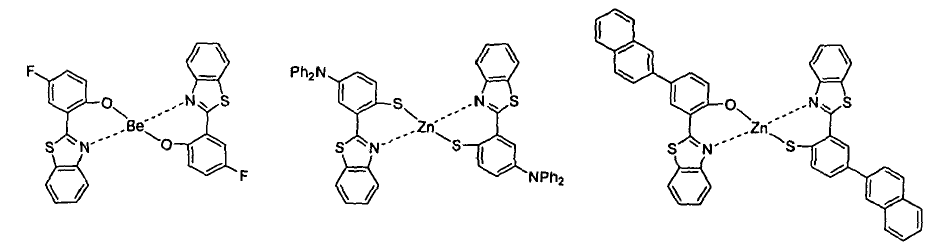

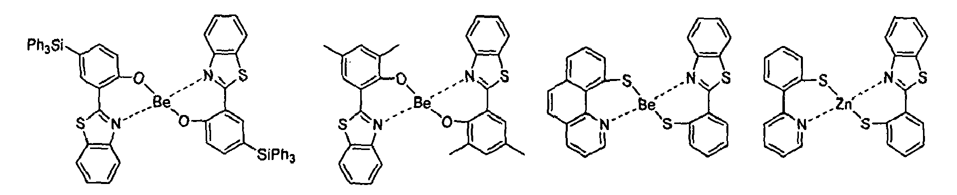

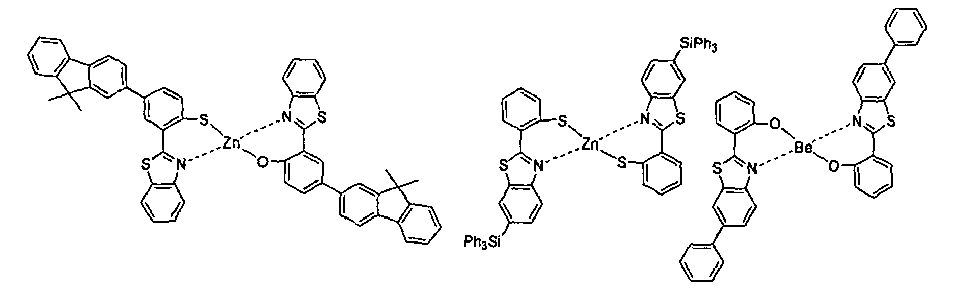

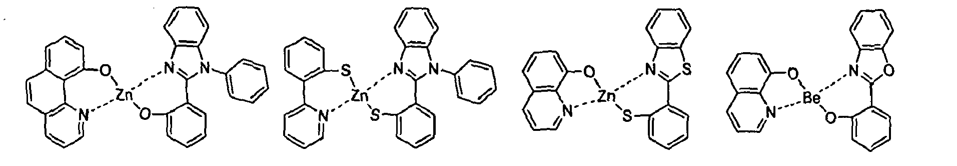

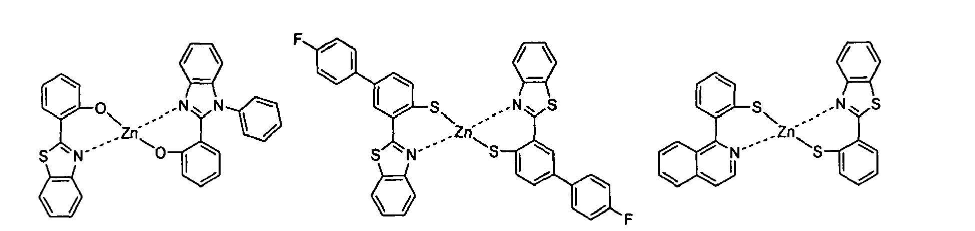

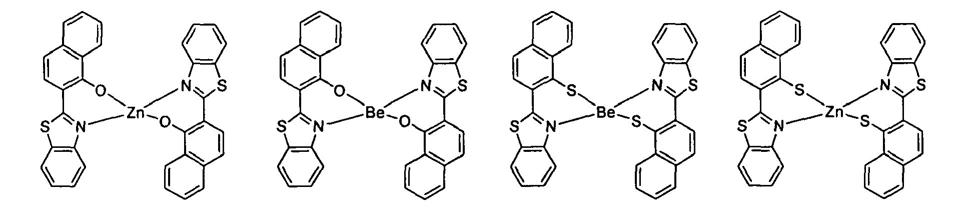

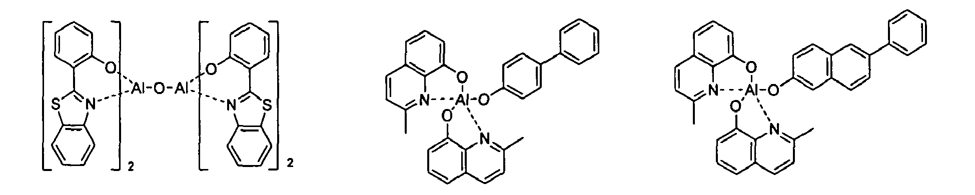

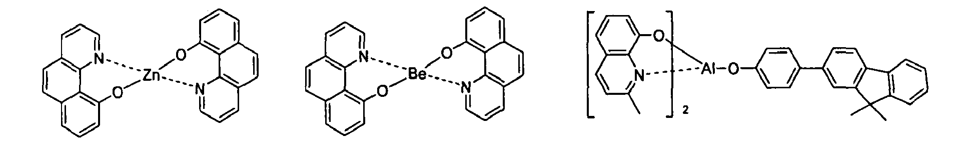

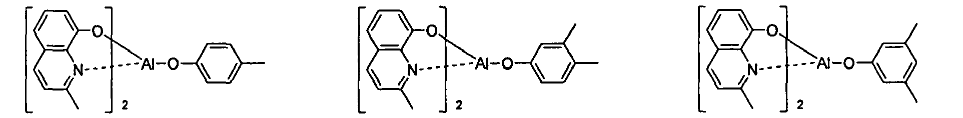

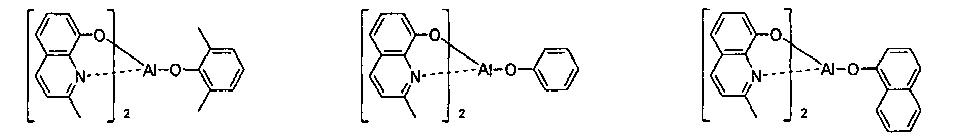

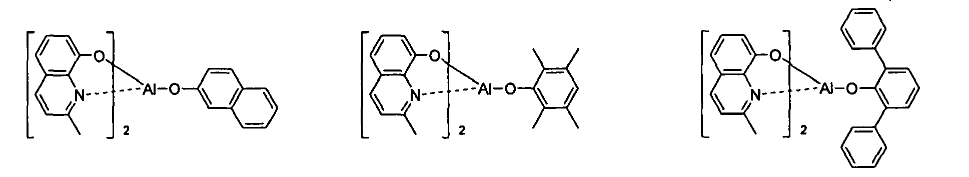

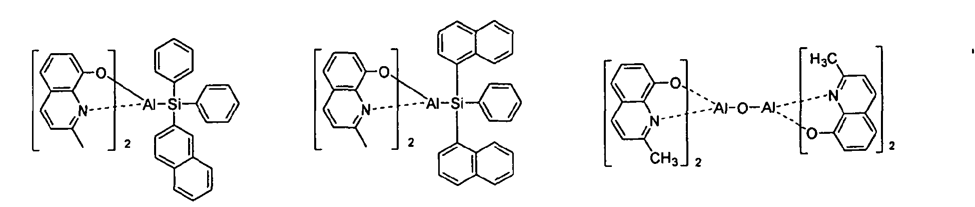

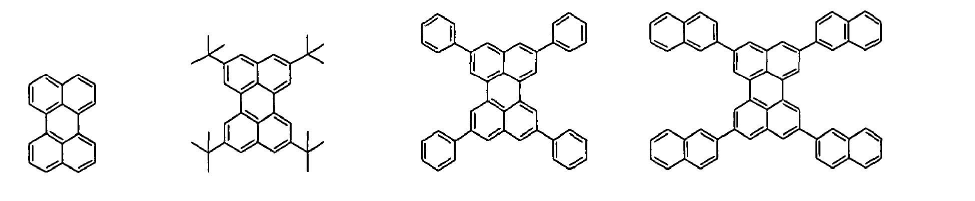

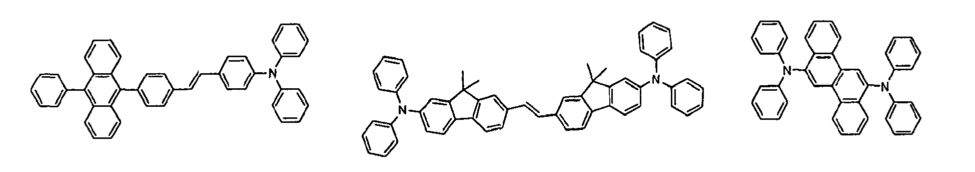

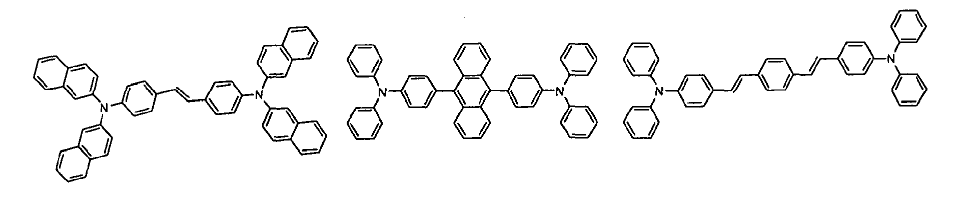

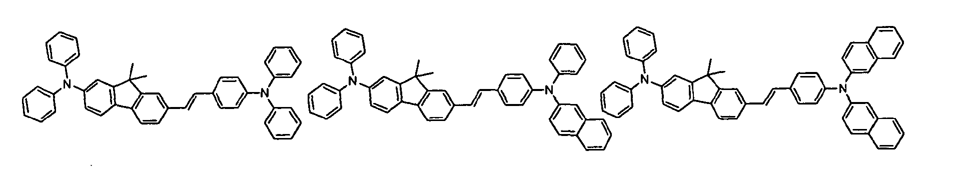

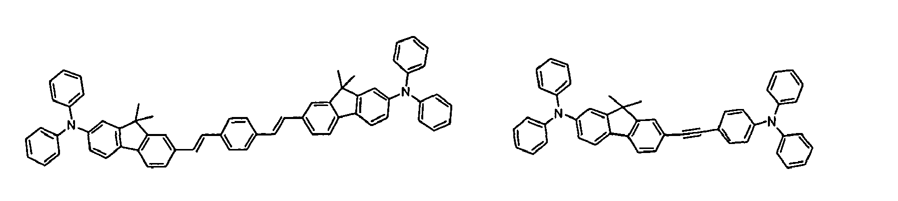

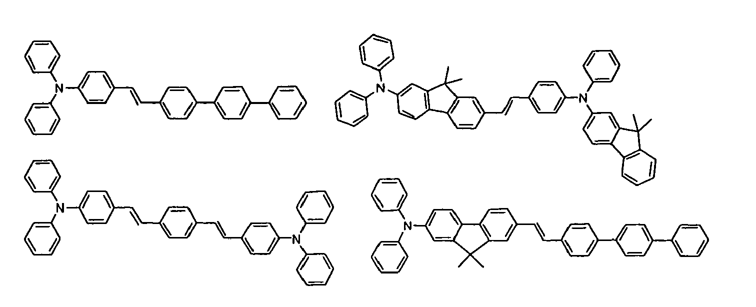

In an electroluminescent device according to the present invention, it is preferable to displace one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal or the like. Examples of metal oxides preferably include Cs₂O, Li₂O, MgO, SrO, BaO, CaO, or the like.

In an electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the present invention, having high luminous efficiency and excellent color purity and life property of material, are advantageous in that they can be employed to manufacture organic light emitting diodes (OLED's) having excellent operation life.

### Best Mode

The present invention is further described by referring to representative compounds with regard to the organic electroluminescent compounds according to the invention, preparation thereof and luminous properties of the devices manufactured therefrom, but those examples are provided for better understanding of the invention and illustration of the embodiments only, not being intended to limit the scope of the invention by any means.

### Preparation Examples

[Preparation Example 1] Preparation of Compound (213)

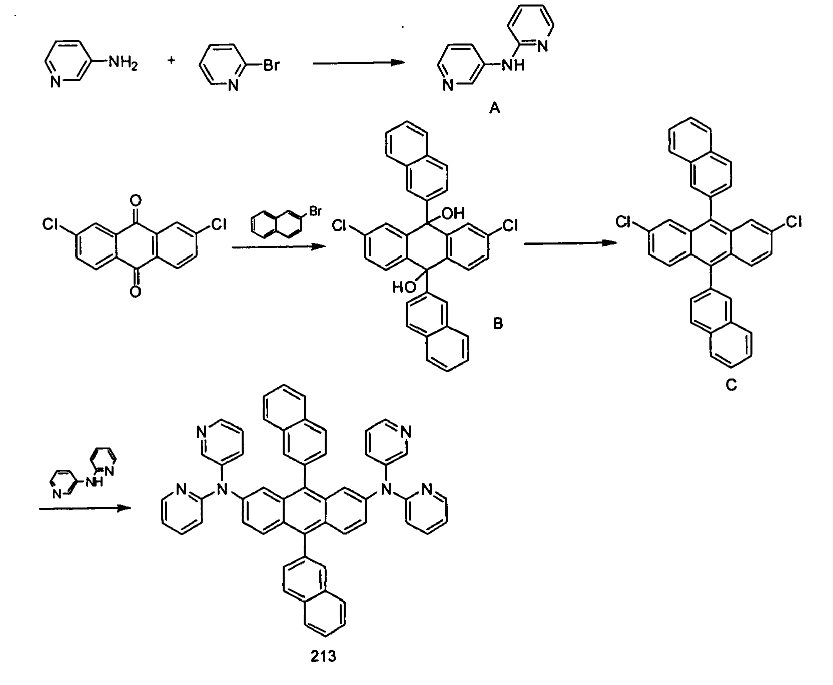

### Preparation of Compound A

A reaction vessel was charged with 3-aminopyridine (20 g, 212.5 mmol), 2-bromopyridine (31.09 ml, 318.7 mmol), Pd₂(dba)₃ (3.8 g, 4.25 mmol), Pcy₃(tricyclohexylphosphine) (2.38, 8.50 mmol), sodium tert-butoxide (61.28, 637.61 mmol) and toluene (500 mL), and the mixture was stirred at 110°C for 12 hours. The reaction mixture was cooled to ambient temperature and extracted with ethyl acetate. The extract was washed with distilled water, dried over anhydrous magnesium sulfate, and evaporated under reduced pressure. Purification via column chromatography (ethyl acetate: hexane = 1:1) gave Compound A (10 g, 27.48%).

### Preparation of Compound (B)

In dry tetrahydrofuran solvent (500 mL), dissolved was 2-bromonaphthalene (44.1 g, 270.7 mmol), and n-butyllithium (2.5 M solution in n-hexane) (130.0 mL, 324.9 mmol) was slowly added dropwise thereto at -78°C. After stirring for 1 hour, 2,7-dichloroanthracene-9,10-dione (30.0 g, 108.3 mmol) was added, and the mixture was stirred, while slowly raising the temperature to room temperature. After 17 hours, water was added thereto, and the resultant mixture was stirred for 30 minutes. The mixture was extracted with ethyl acetate (500 mL), and the organic layer was washed with water 500 mL and dried over magnesium sulfate. Distillation under reduced pressure and drying gave Compound (B) (21.3 g, 47.8 mmol).

### Preparation of Compound (C)

A reaction vessel was charged with Compound (B) (21.3 g, 47.8 mmol), potassium iodide (31.7 g, 191.2 mmol), sodium hydrophosphite (40.5 g, 382.4 mmol) and acetic acid (300 mL), and the mixture was stirred under reflux at 120°C. After 15 hours, water (500 mL) was added, and the resultant mixture was stirred for 1 hour. The precipitate collected by filtration under reduced pressure was washed with water (300 mL x 3) and acetone (300 mL x 1), and dried to obtain Compound (C) (12.5 g, 30.4 mmol).

### Preparation of Compound (213)

A reaction vessel was charged with Compound (C) (12.5 g, 30.4 mmol), Compound (A) (15.4 g, 91.2 mmol), palladium acetate (0.34 g, 1.52 mmol), tributylphosphine (0.6 g, 3.0 mmol), sodium tert-butoxide (9.3 g, 97.3 mmol) and toluene (250 mL), and the mixture was stirred under reflux under nitrogen atmosphere. After 8 hours, the reaction mixture was cooled to room temperature, and extracted with ethyl acetate (400 mL). The extract was dried under reduced pressure, and purified via column chromatography (dichloromethane: n-hexane = 5:1) to obtain the target compound (Compound 213) (9.2 g, 44%).

[Preparation Example 2] Preparation of Compound (2589)

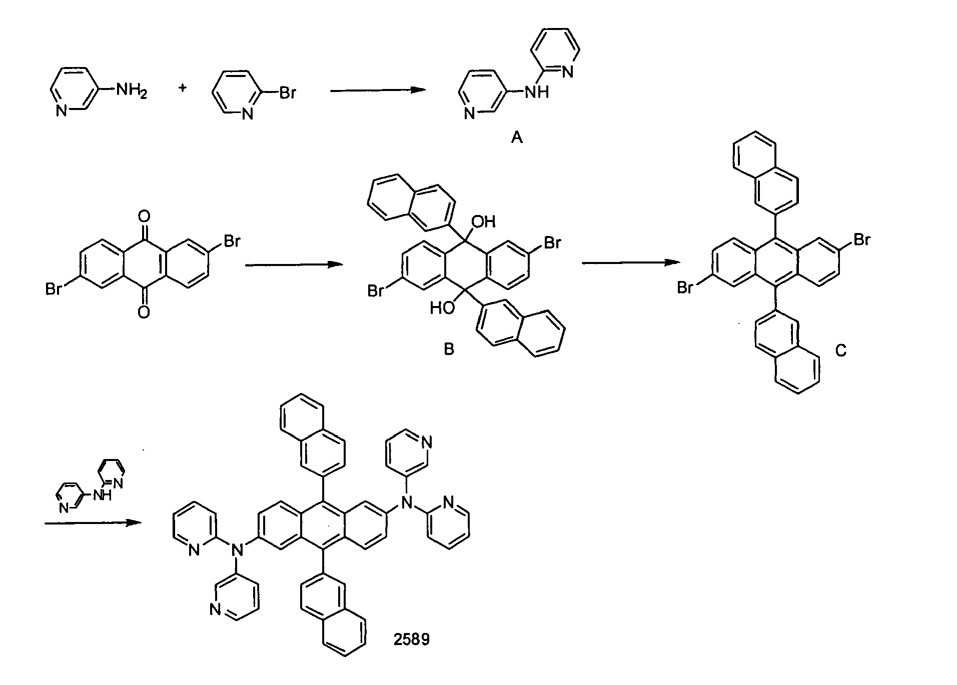

### Preparation of Compound (A)

A reaction vessel was charged with 3-aminopyridine (20 g, 212.5 mmol), 2-bromopyridine (31.09 mL, 318.7 mmol), Pd₂(dba)₃ (3.8 g, 4.25 mmol), Pcy₃ (tricyclohexylphosphine) (2.38, 8.50 mmol), sodium tert-butoxide (61.28, 637.61 mmol) and toluene (500 mL), and the mixture was stirred at 110°C for 12 hours.

After cooling to ambient temperature, the reaction mixture was extracted with ethyl acetate, and the extract washed with distilled water, dried over anhydrous magnesium sulfate, and distilled under reduced pressure. Purification via column chromatography (ethyl acetate: hexane = 1:1) gave Compound (A) (10 g, 27.48%).

### Preparation of Compound (B)

In dry tetrahydrofuran solvent (500 mL), dissolved was 2-bromonaphthalene (44.1 g, 270.7 mmol), and n-buthyllithium (2.5 M solution in n-hexane) (130.0 mL, 324.9 mmol) was slowly added thereto at -78°C. After stirring for 1 hour, 2,6-dibromoanthracene-9,10-dione (30.0 g, 108.3 mmol) was added thereto, and the resultant mixture was stirred while slowly raising the temperature to room temperature. After 17 hours, water was added, and the mixture was stirred for 30 minutes, and extracted with ethyl acetate (500 mL). The extract was washed with water (500 mL), and the organic layer obtained was dried over magnesium sulfate. Distillation under reduced pressure and drying gave Compound (B) (21.3 g, 47.8 mmol).

### Preparation of Compound (C)

In acetic acid (150 mL), dissolved were Compound (B) (7.0 g, 14.51 mmol), potassium iodide (KI) (9.64 g, 58.06 mmol) and sodium phosphate monohydrate (NaH₂PO₂· H₂O) (9.24 g, 87.12 mmol), and the solution was stirred under reflux. After 14 hours, the reaction mixture was cooled to 25°C, and sodium hydroxide solution (200 mL) was added to neutralize the mixture. The mixture was washed with water (400 mL), and extracted with dichloromethane solvent (300 mL). The extract was dried over magnesium sulfate, filtered through a filter, and the solvent was removed under reduced pressure. The compound thus obtained was purified via column chromatography (methylene chloride/hexane = 1/100) to obtain Compound (C) (4.49 g, 10.0 mmol).

### Preparation of Compound (2589)

A reaction vessel was charged with Compound (C) (7 g, 11.89 mmol), Compound A (6.1 g, 35.69 mmol), Pd(OAc)₂ (0.13 g, 0.59 mmol), tri(tert-butyl)phosphine (50% in toluene) (0.58 mL, 1.18 mmol), sodium tert-butoxide (4.57 g, 47.56 mmol) and DMF (100 mL), and the mixture was stirred at 140°C for 12 hours.
After cooling to ambient temperature, distilled water (100 mL) was added to the mixture, and the solid produced was filtered under reduced pressure. The filtered solid was purified via column chromatography (ethyl acetate: hexane = 2:1) to obtain Compound (2589) (3.7 g, 40.47%).

According to the same procedure as Preparation Examples 1 and 2, the organic electroluminescent compounds (Compounds 1 to 4752) listed in Table 1 and Table 2 were prepared, of which the ¹H NMR and MS/FAB data are listed in Table 3.

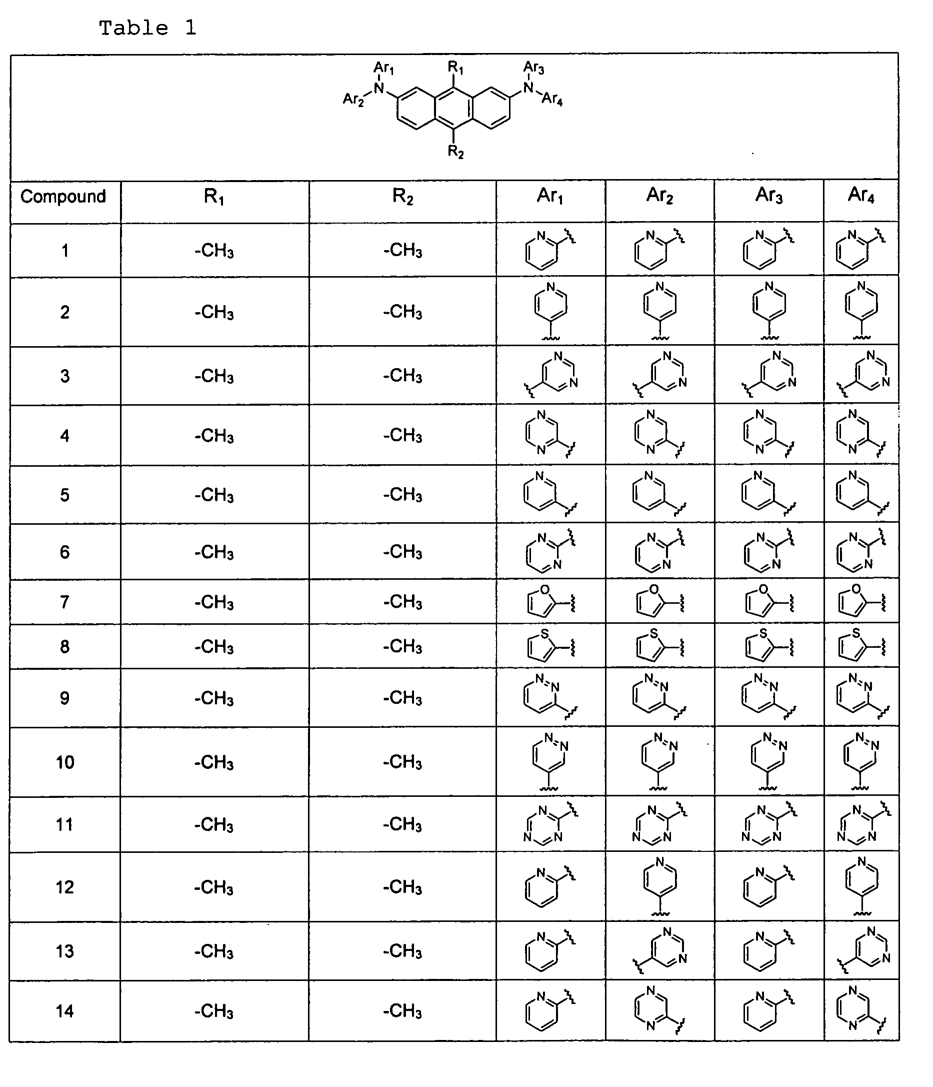

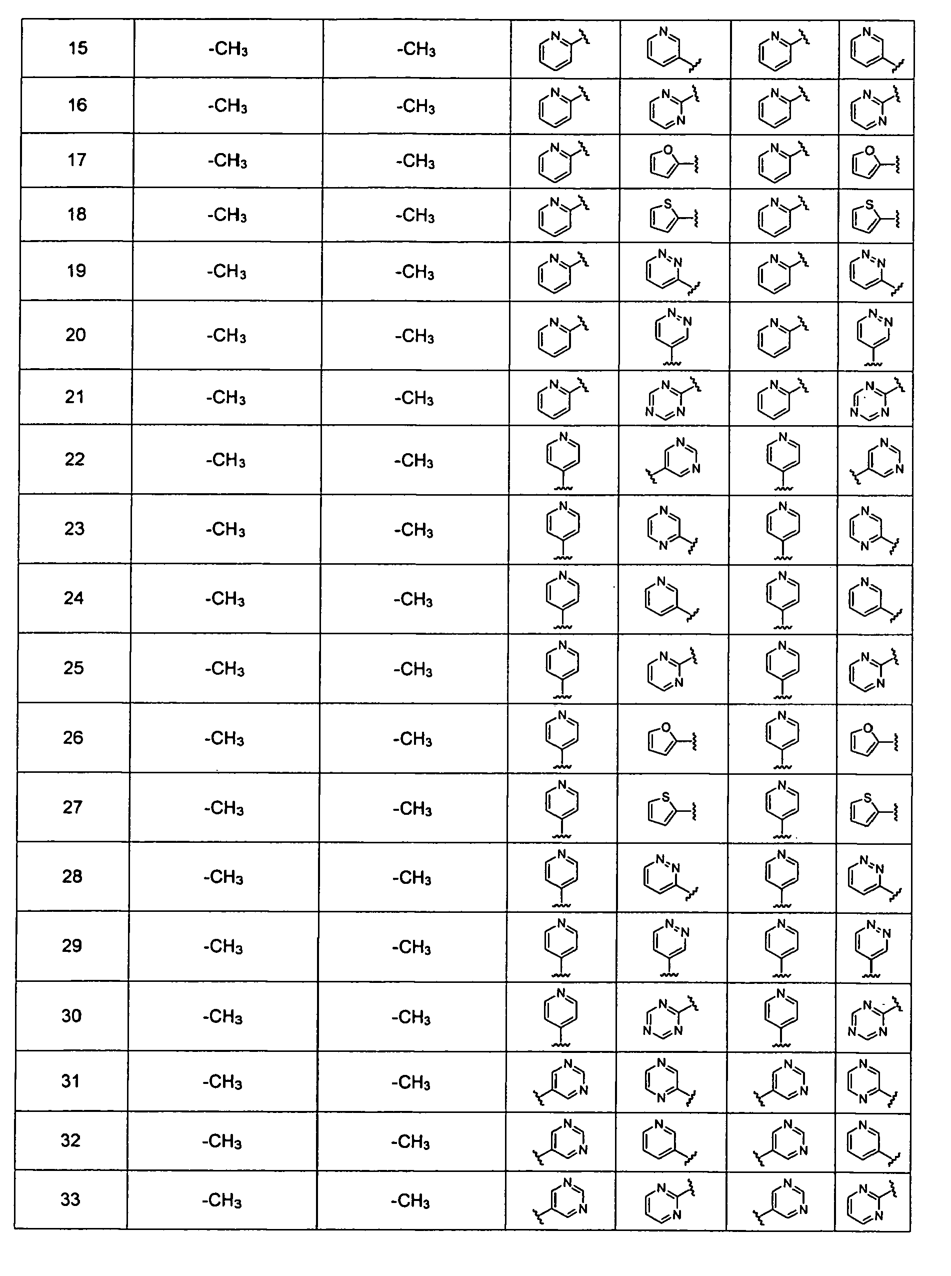

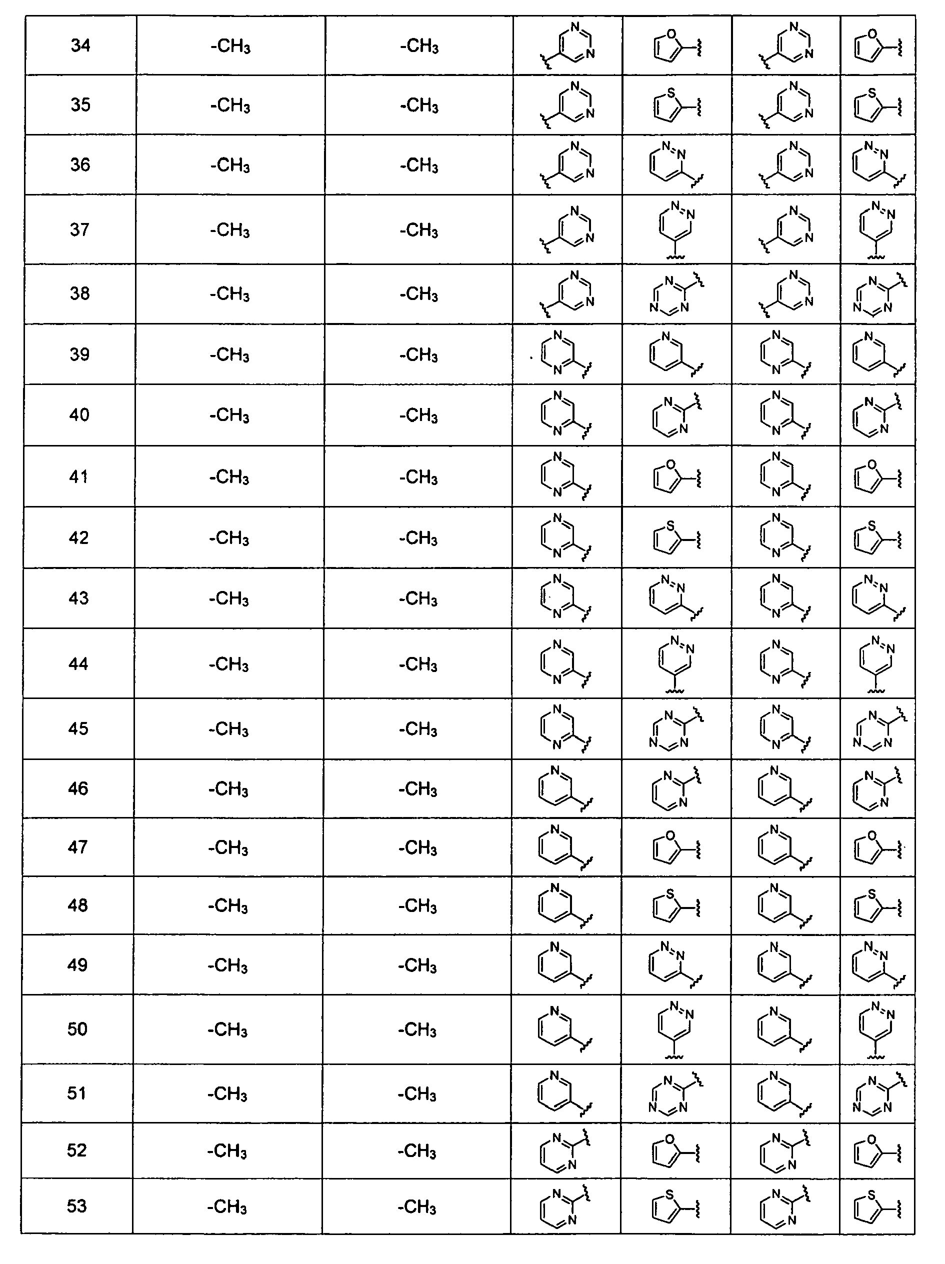

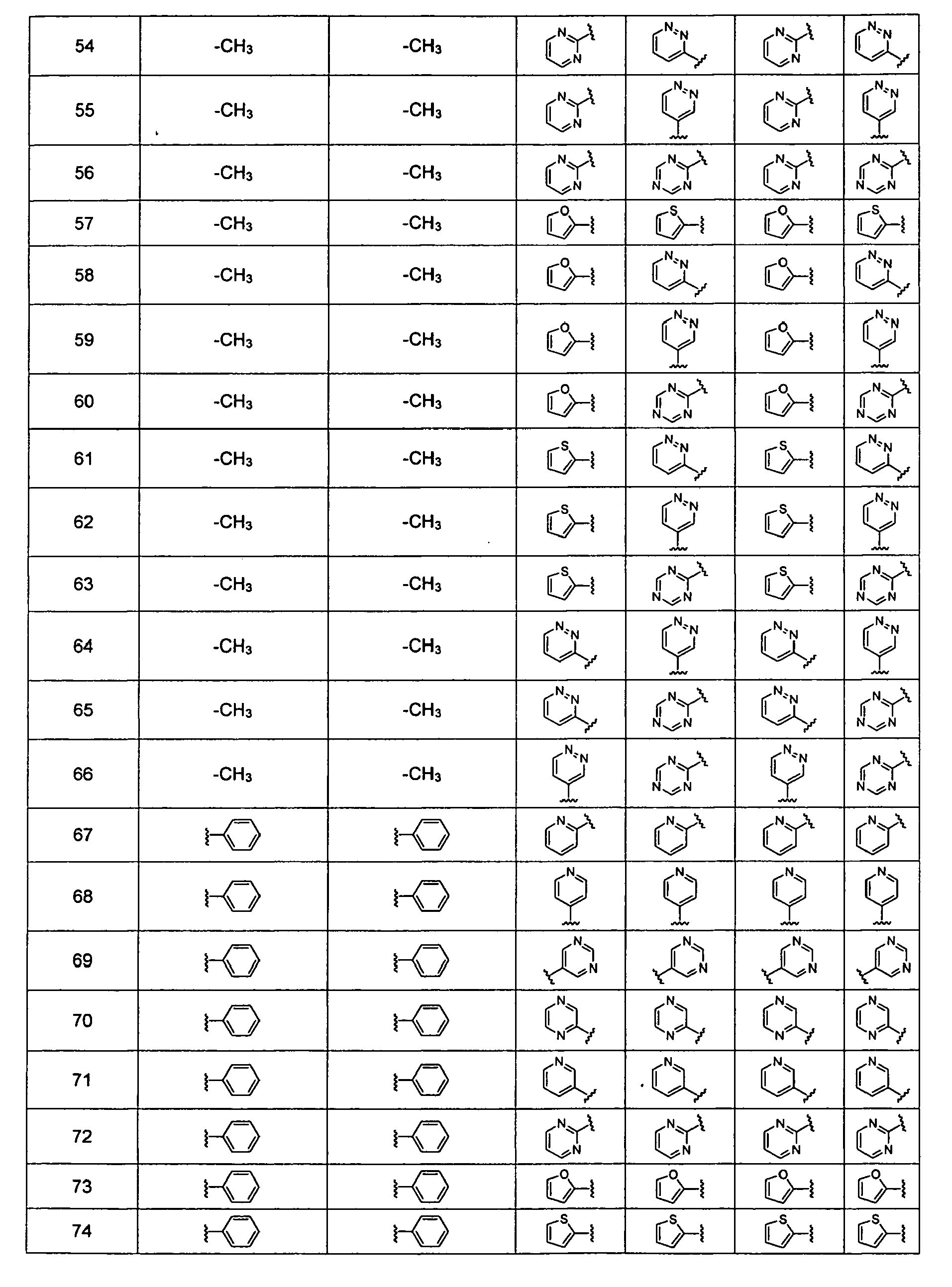

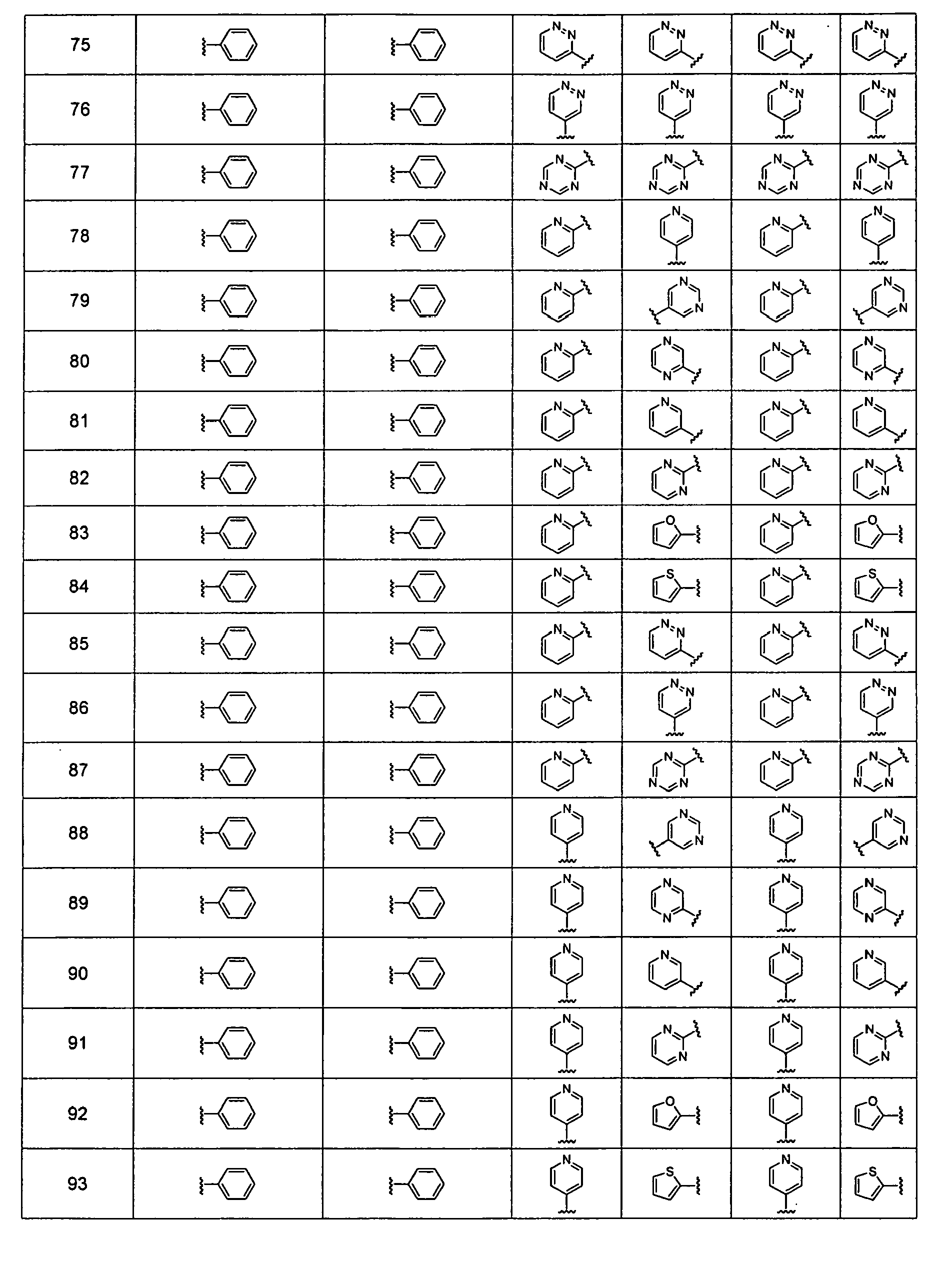

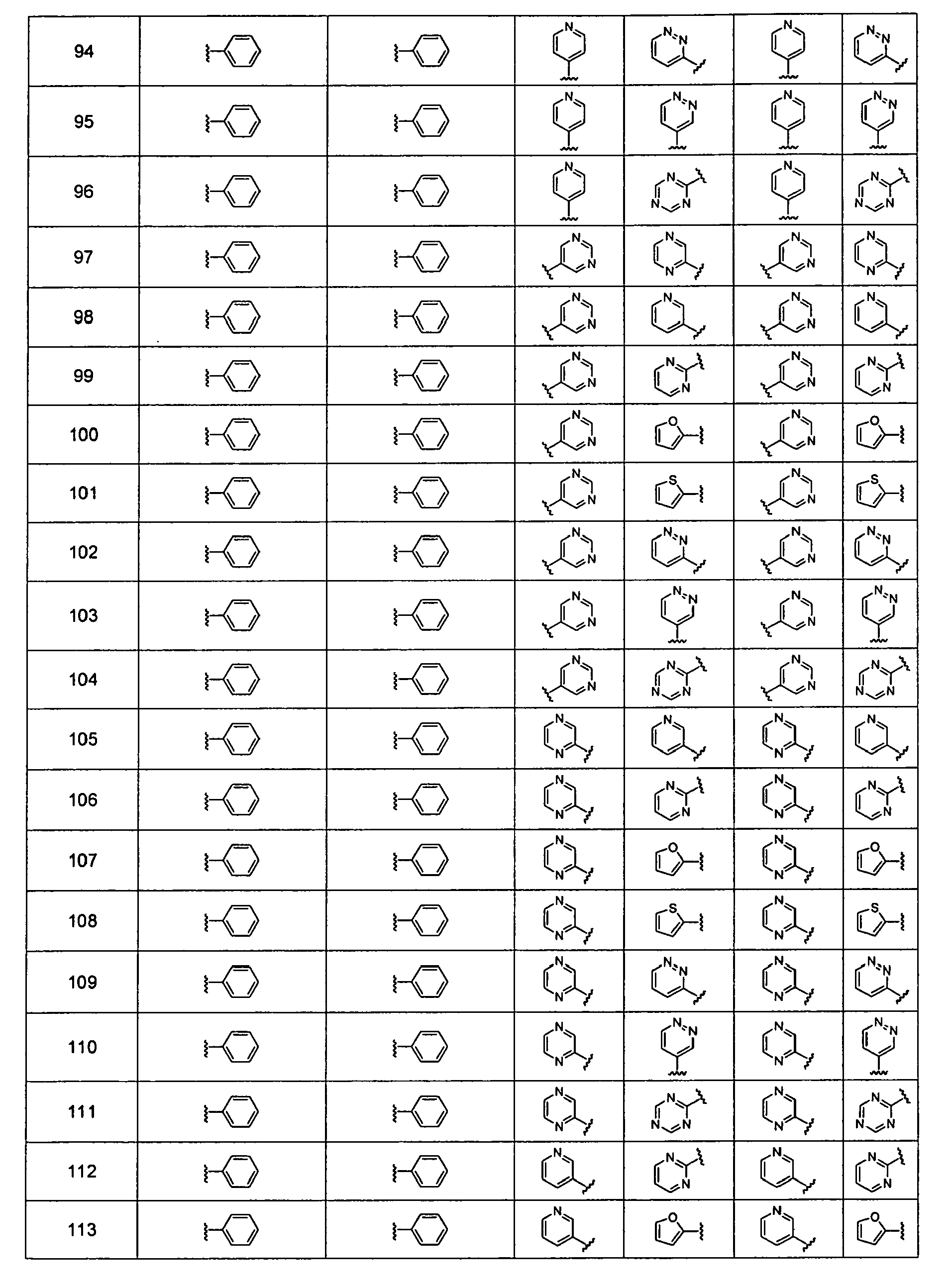

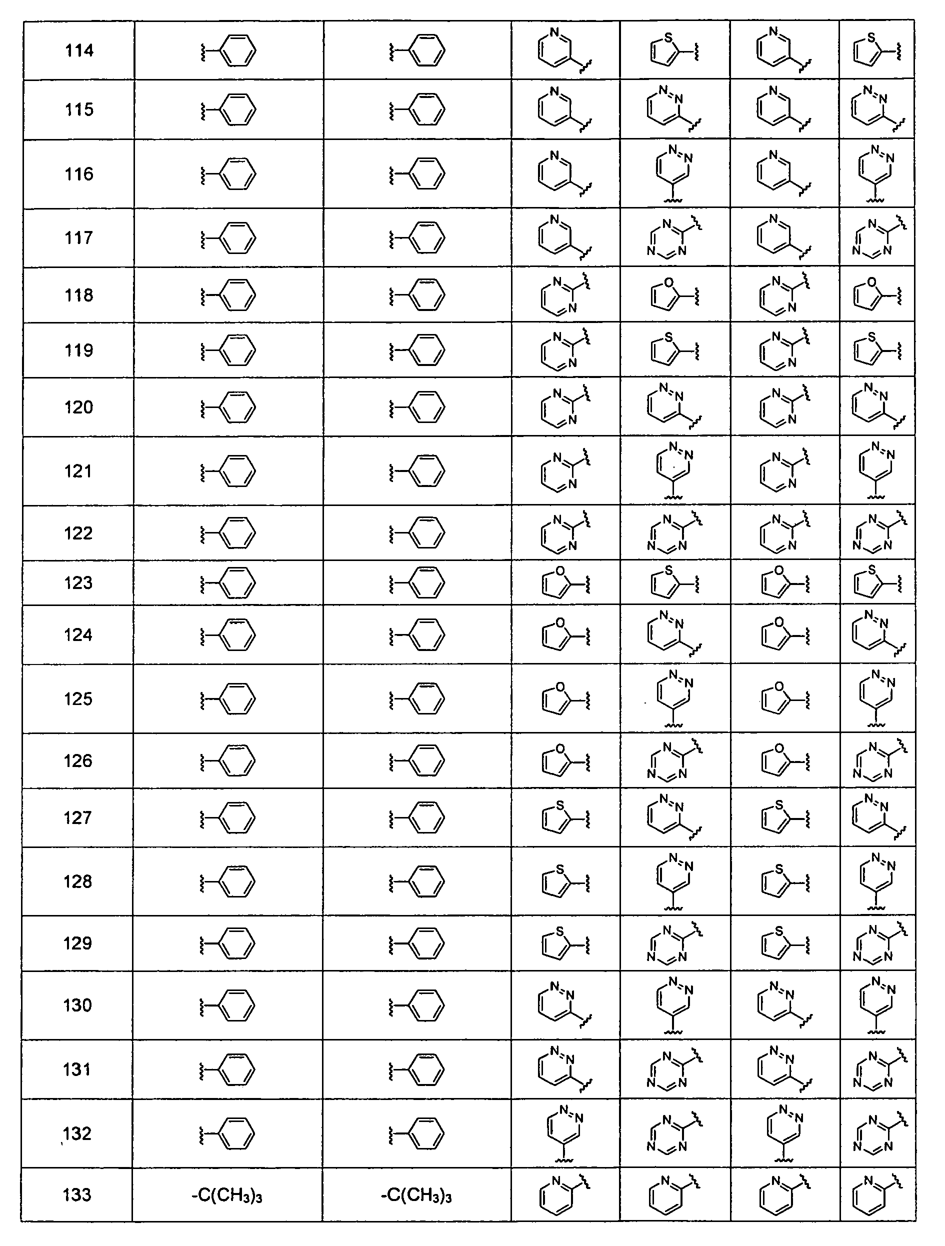

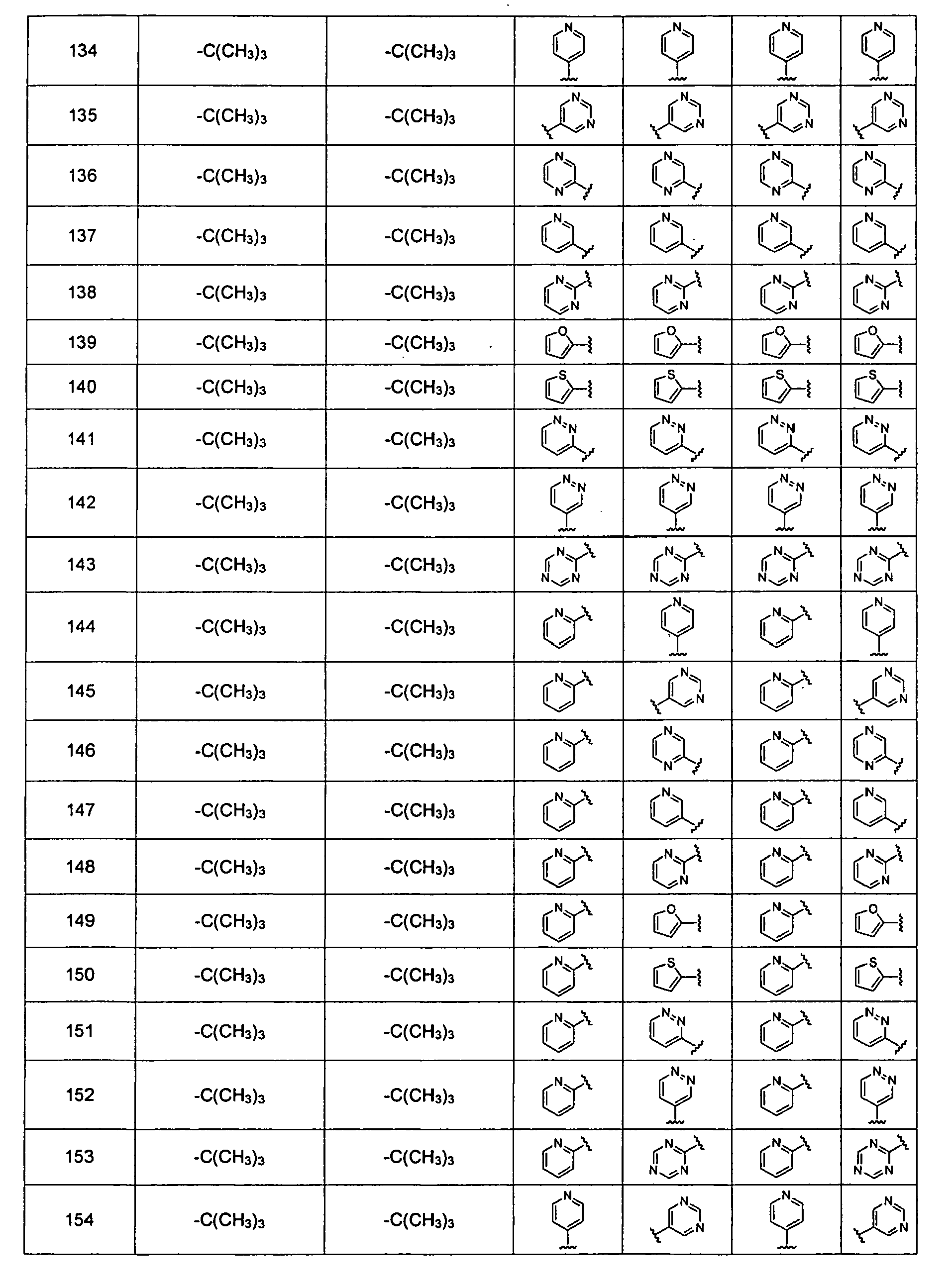

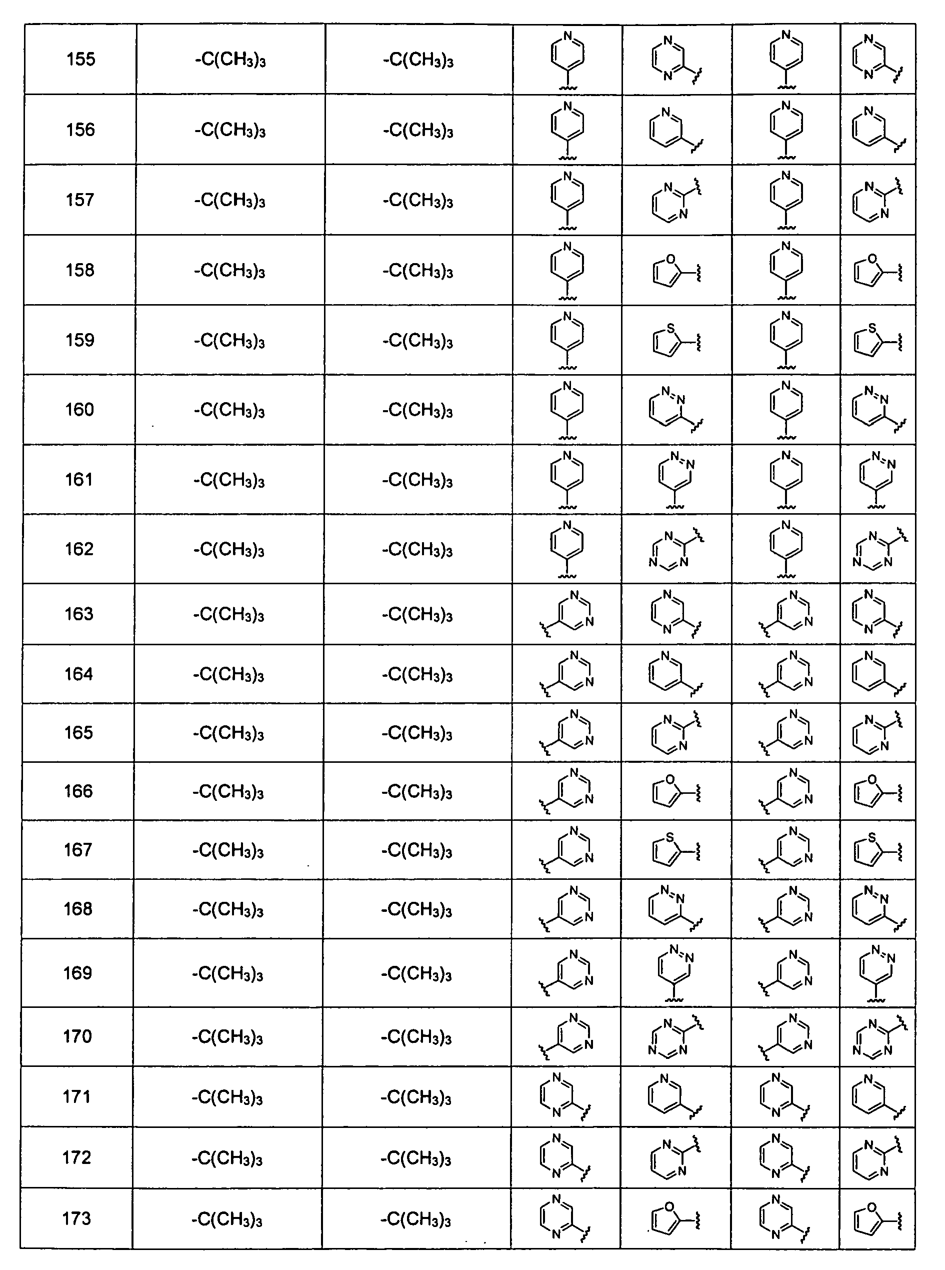

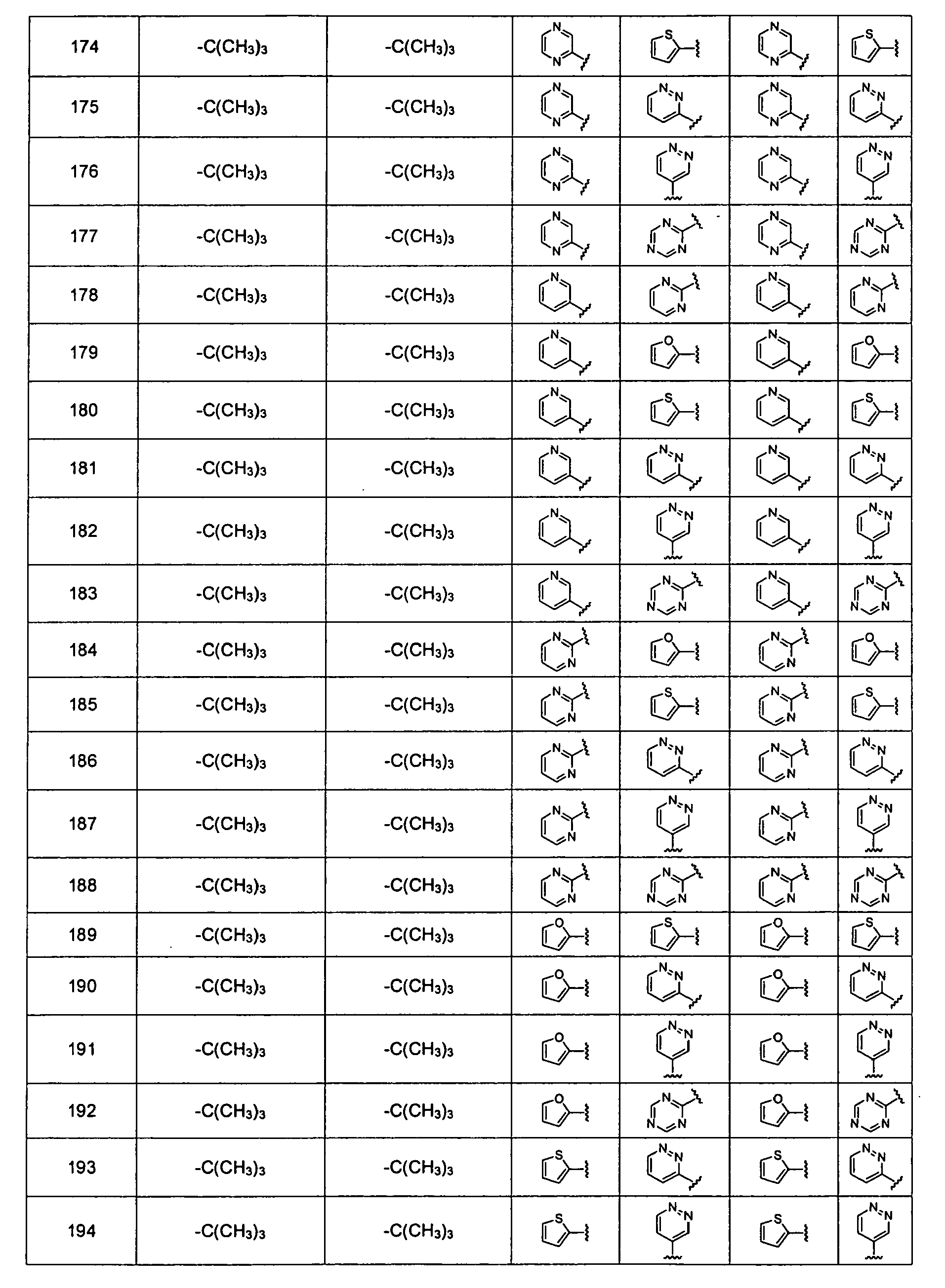

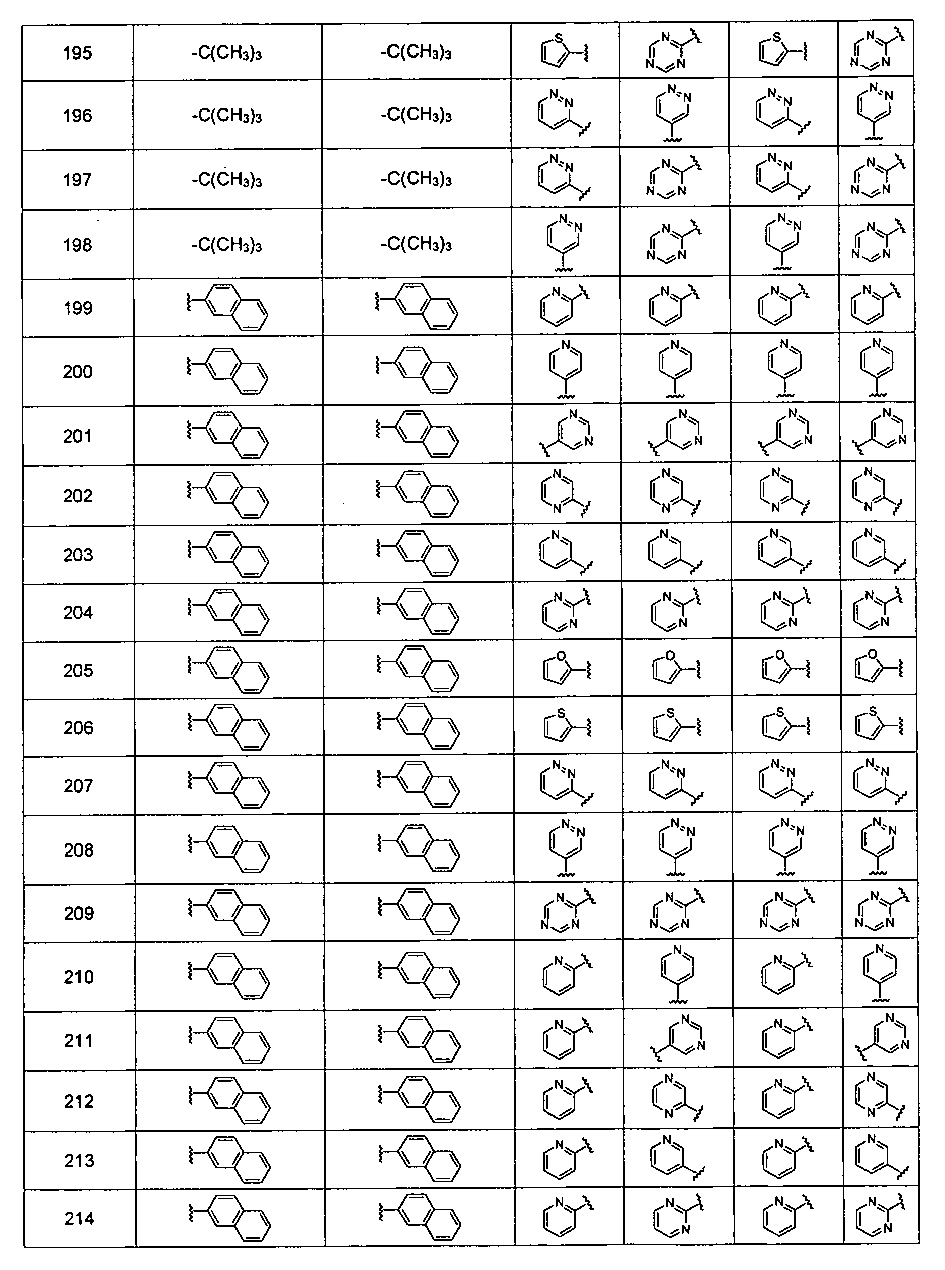

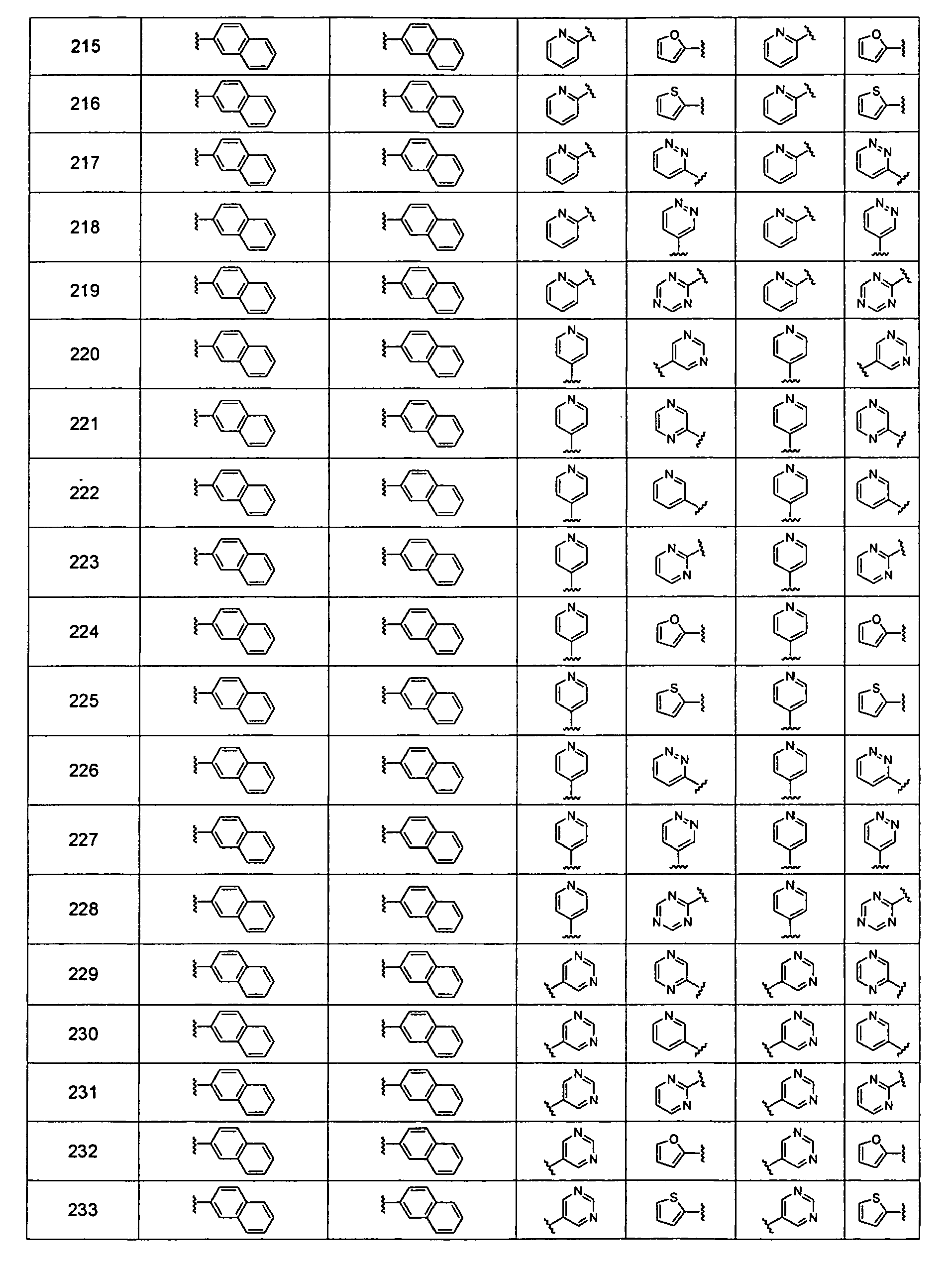

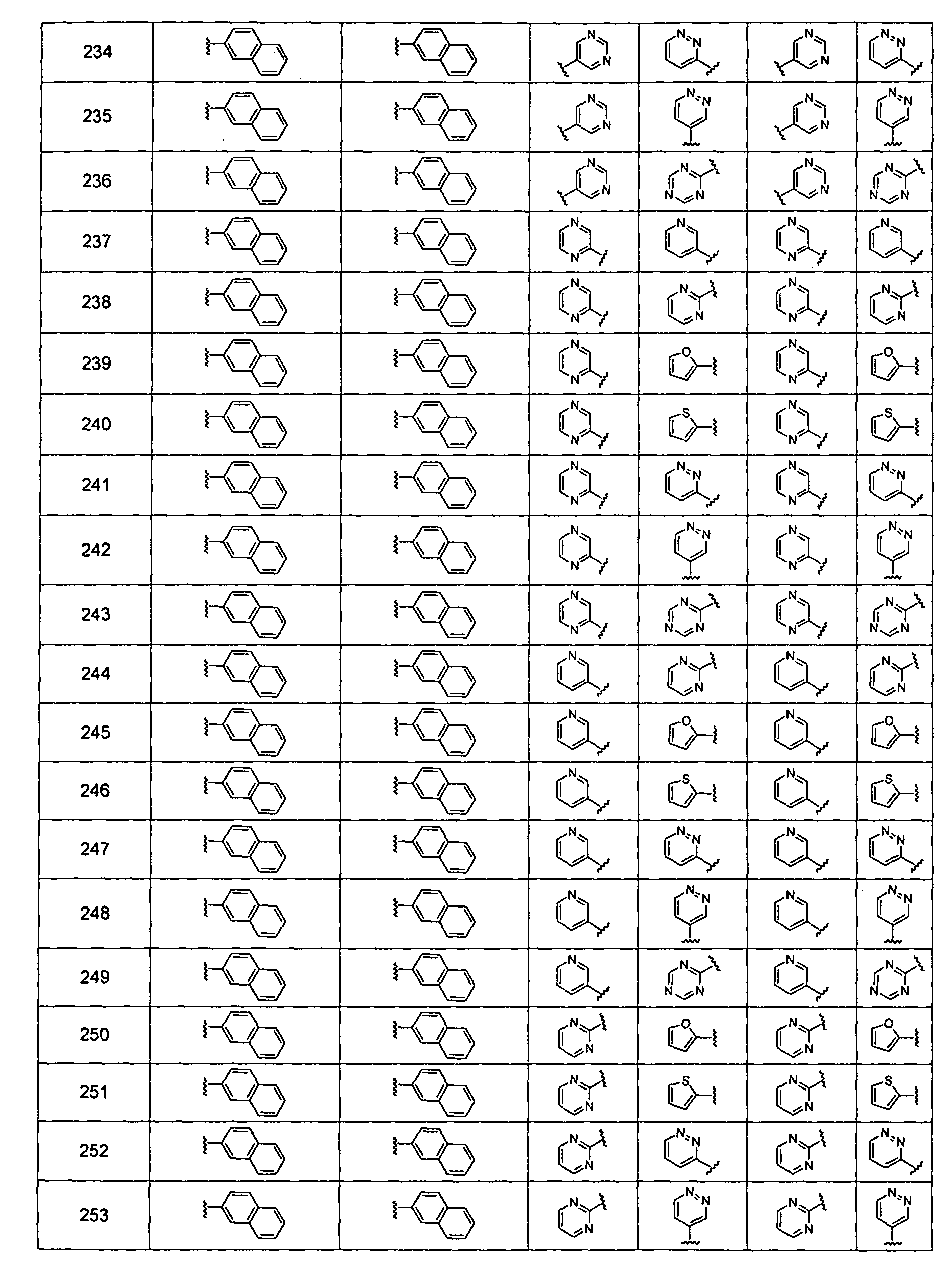

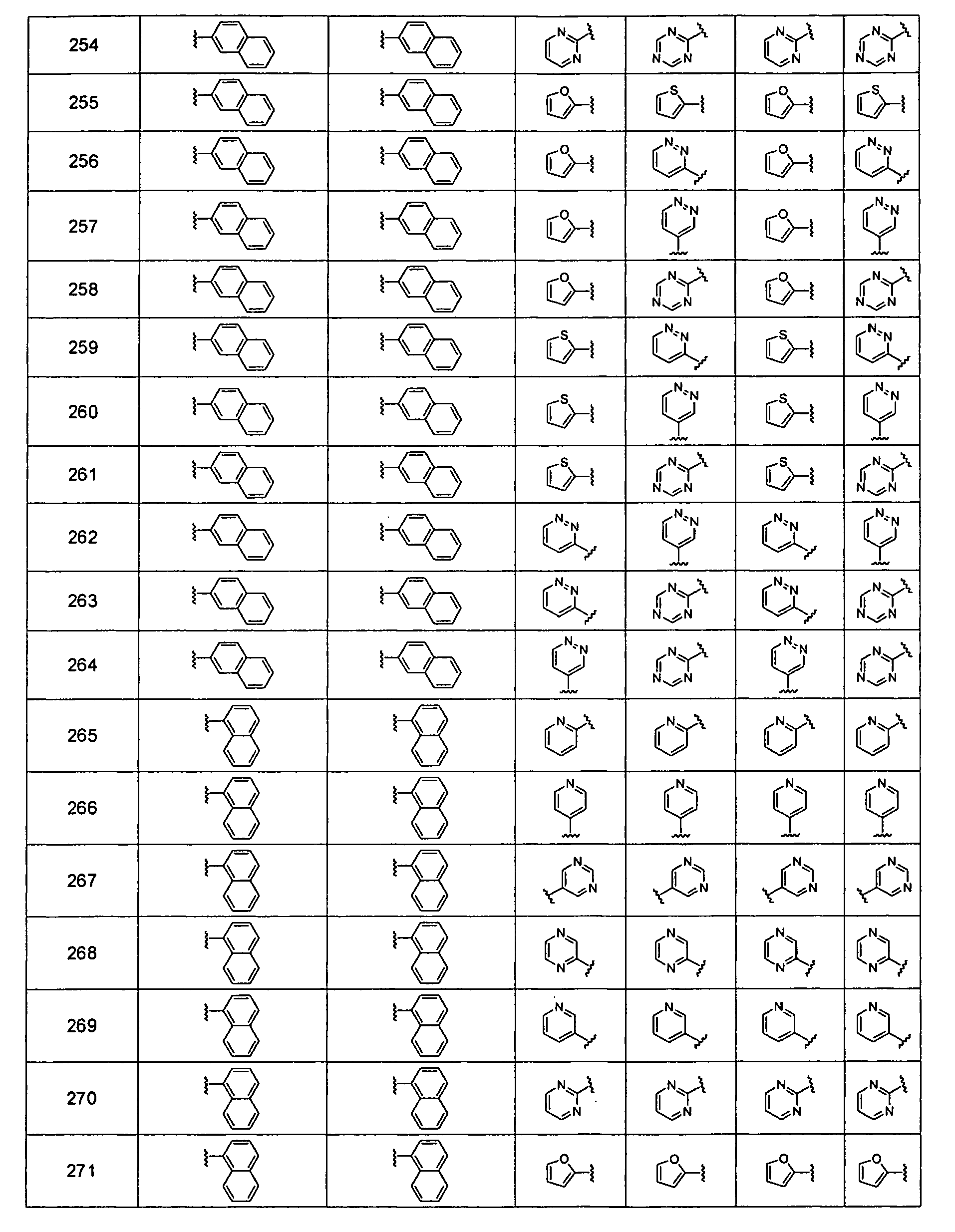

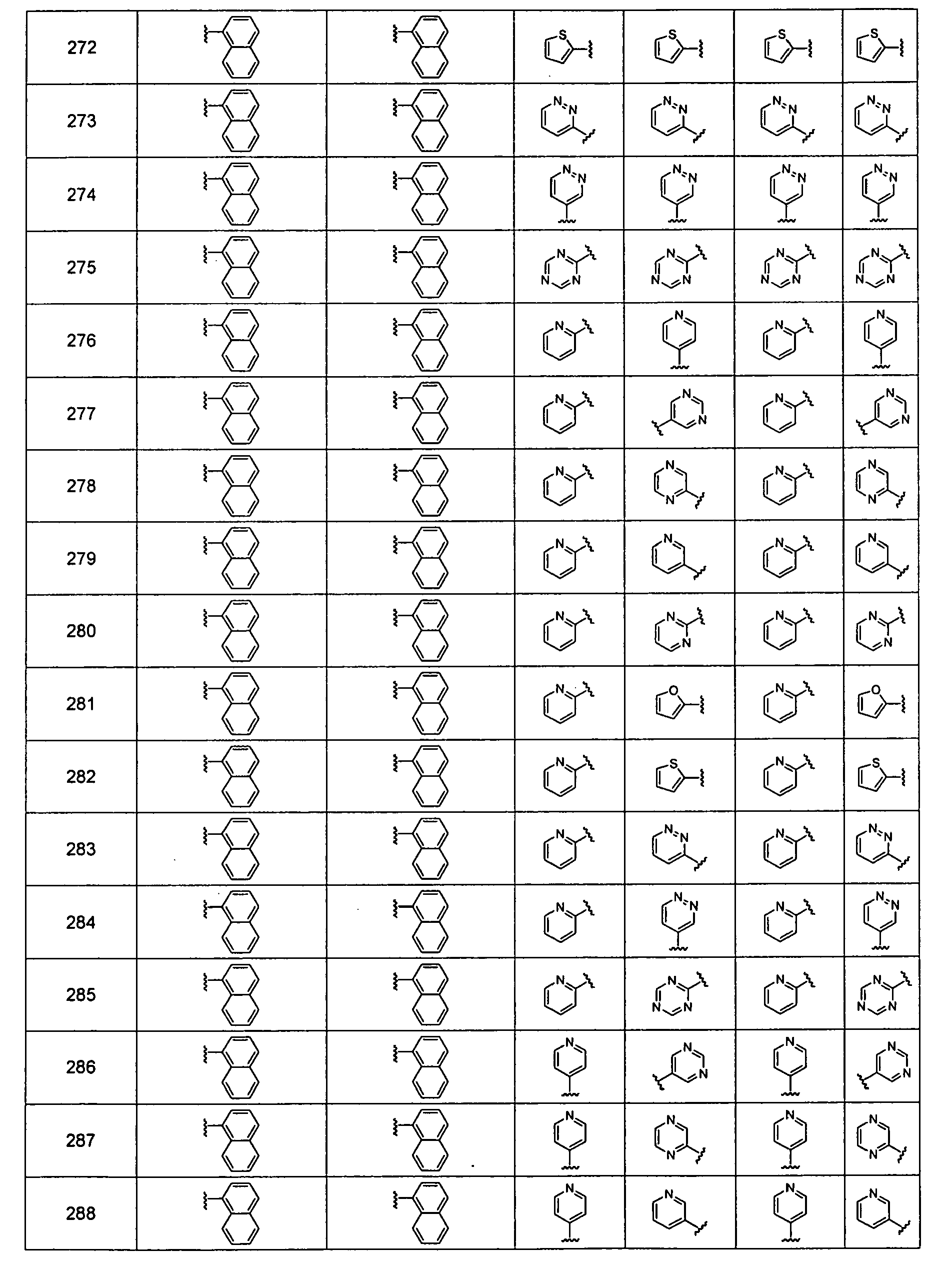

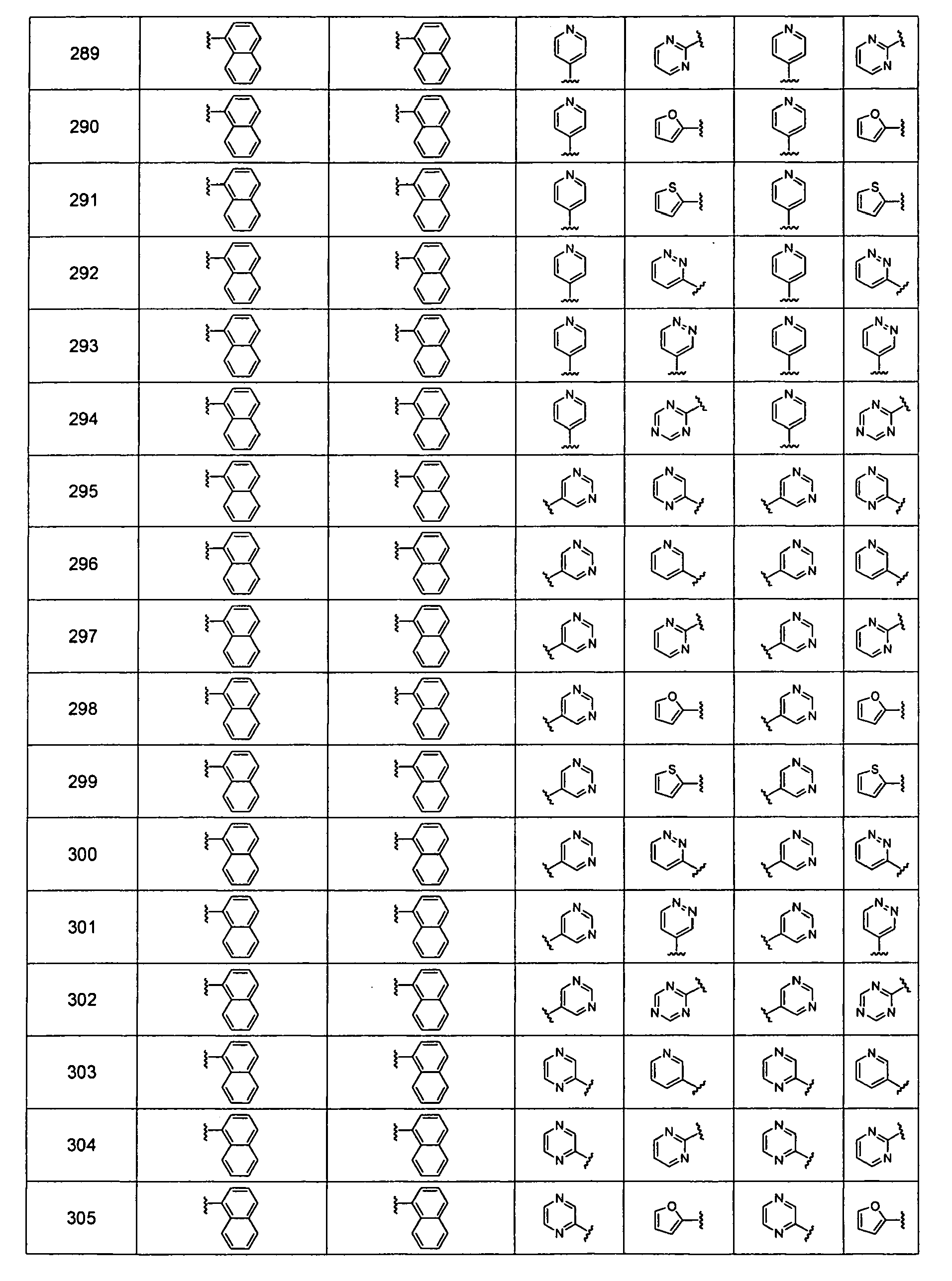

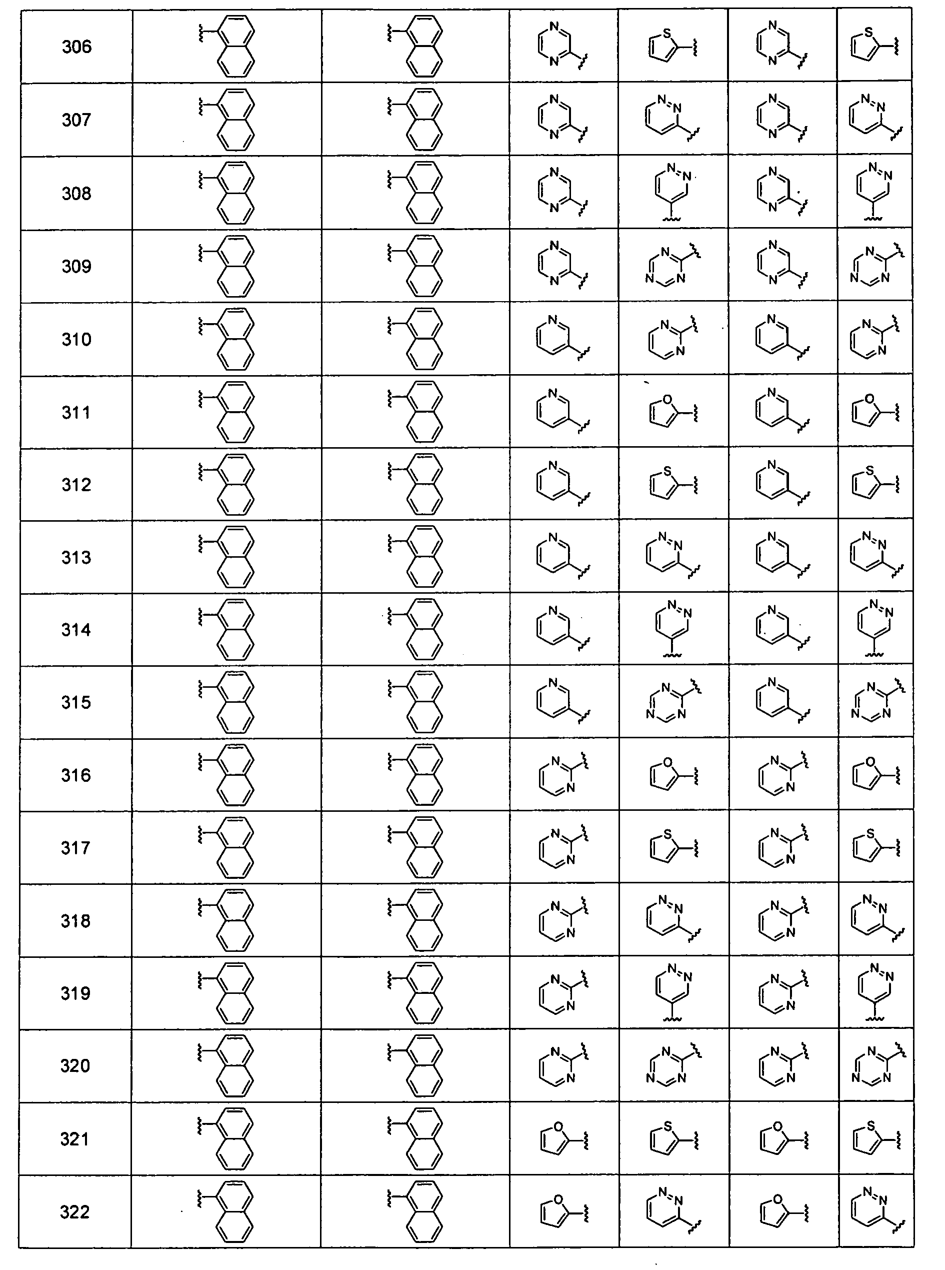

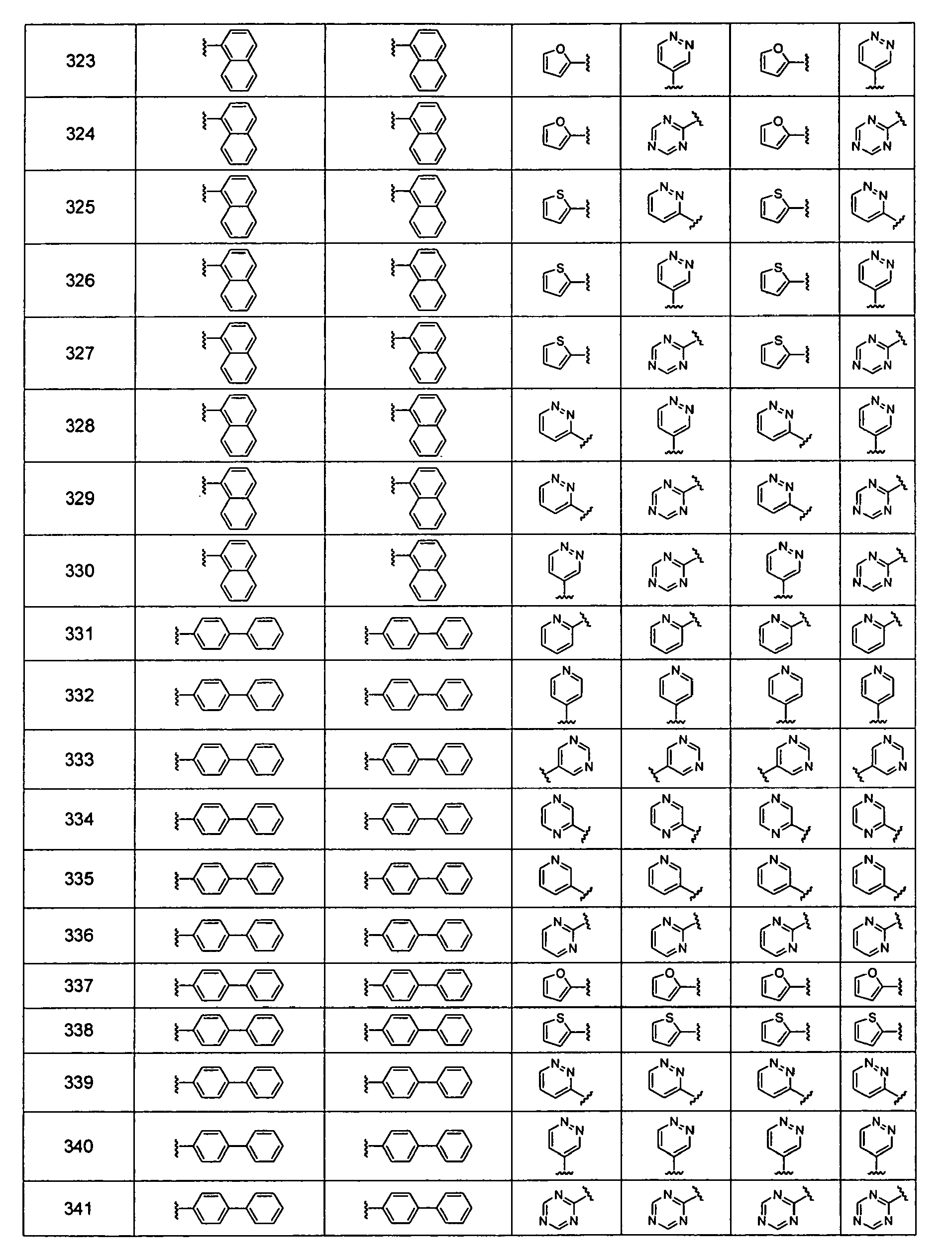

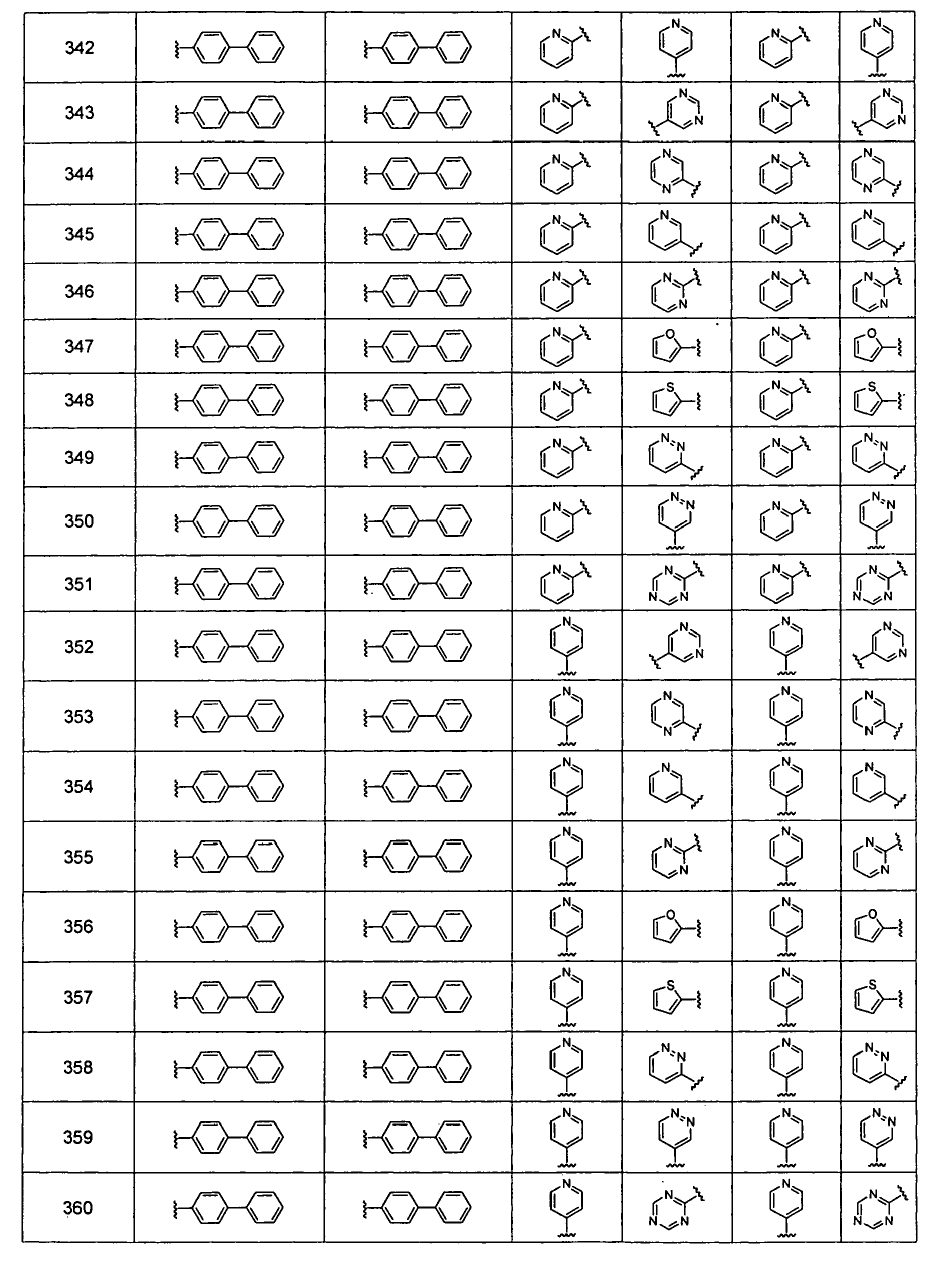

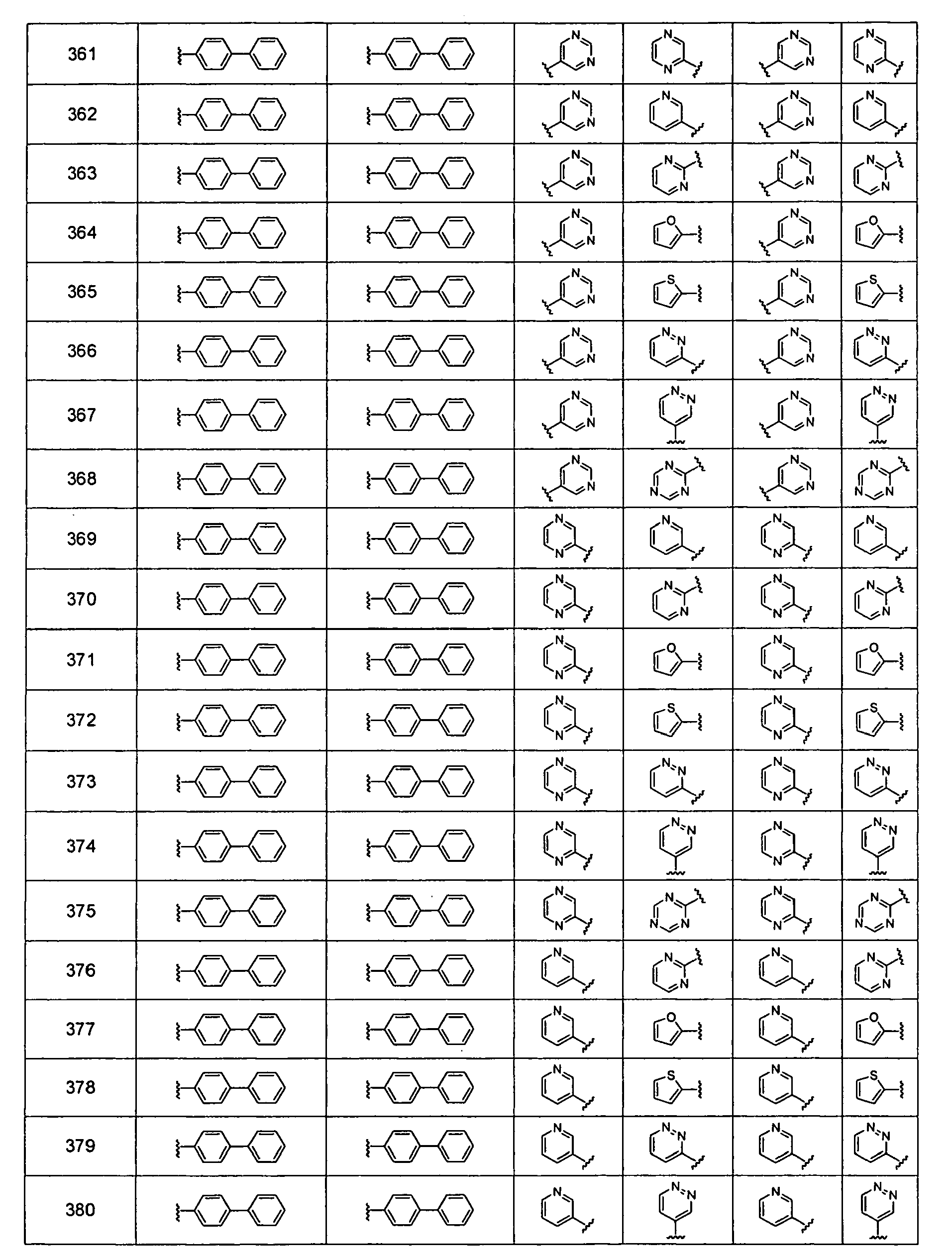

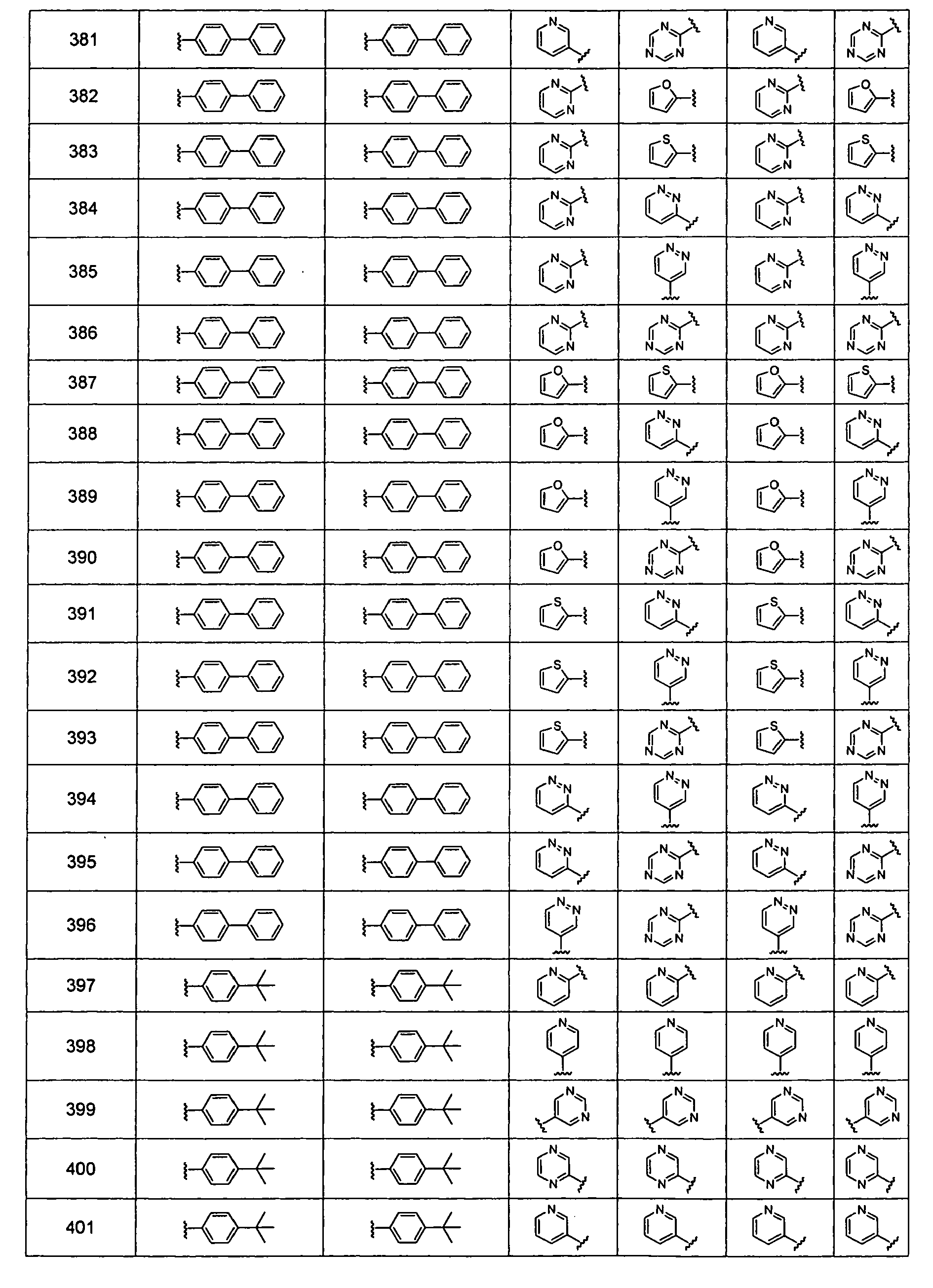

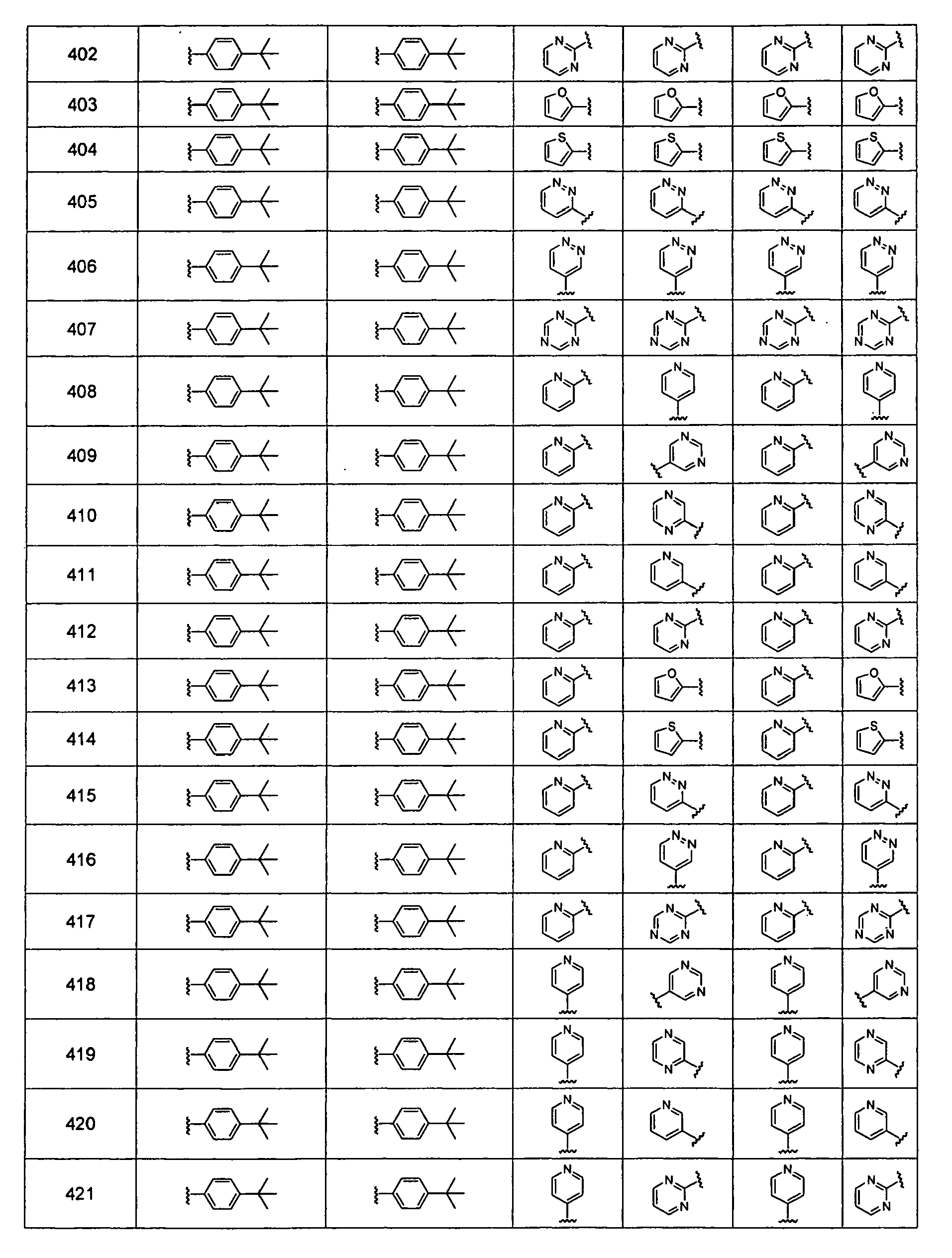

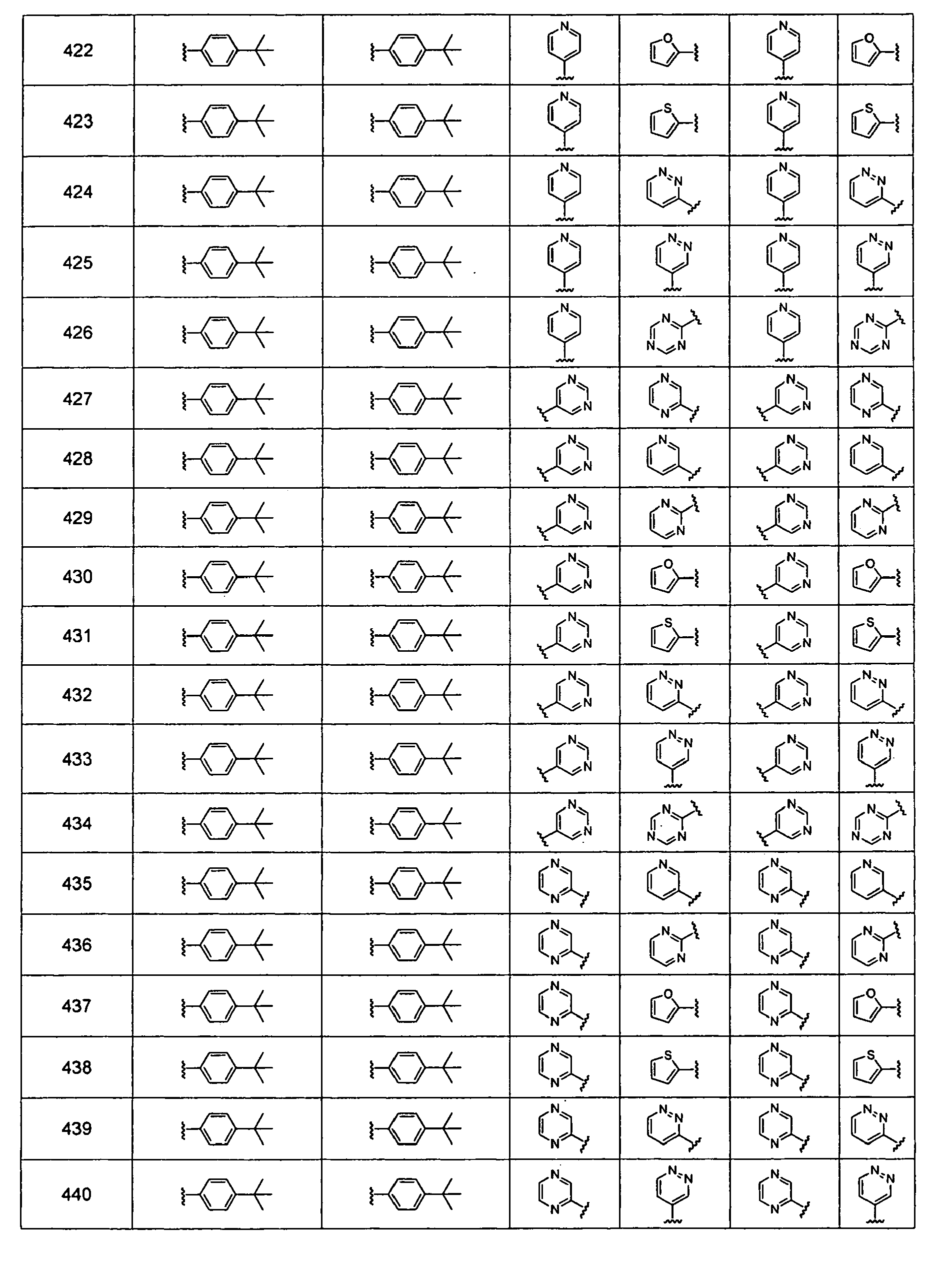

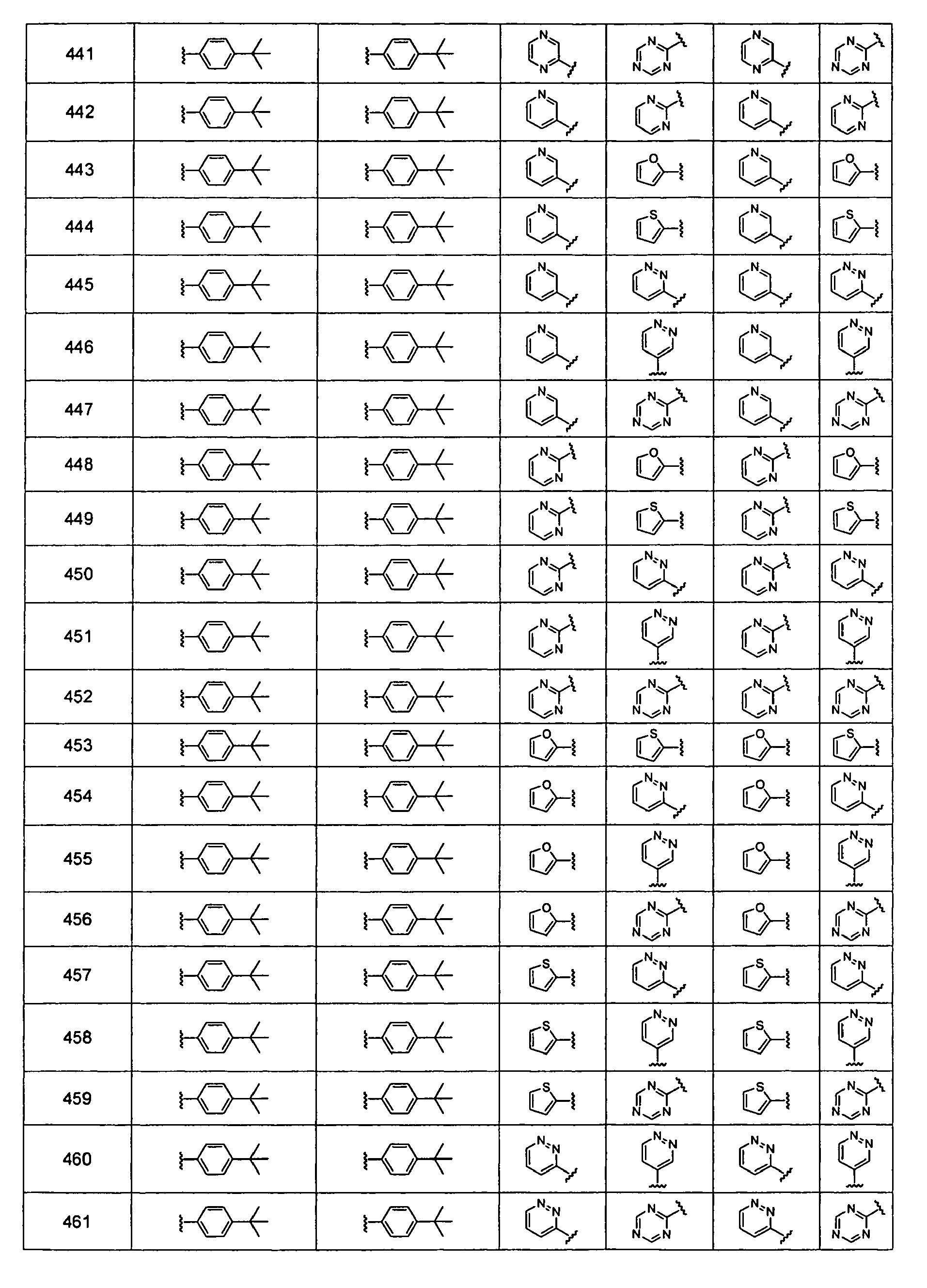

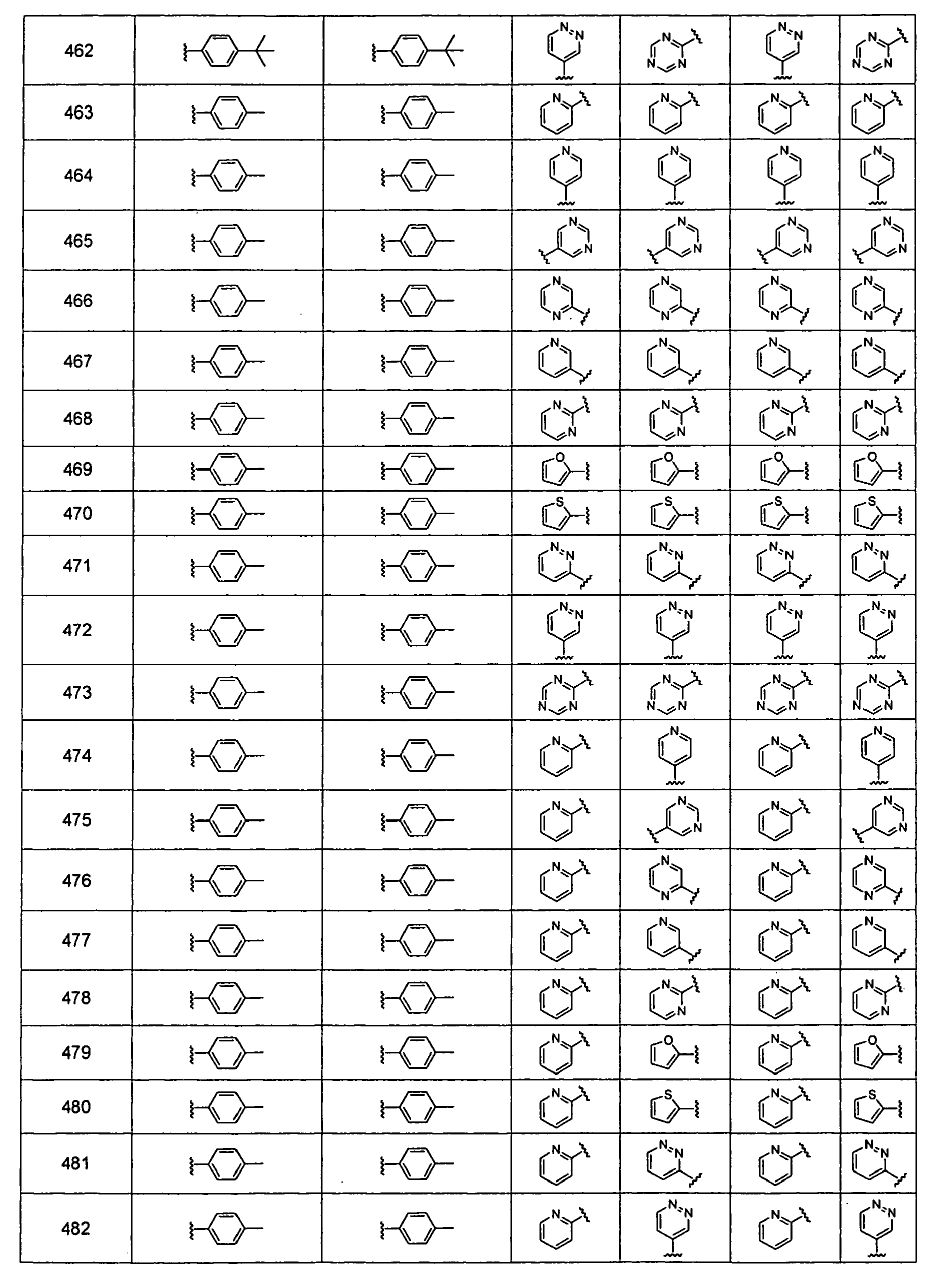

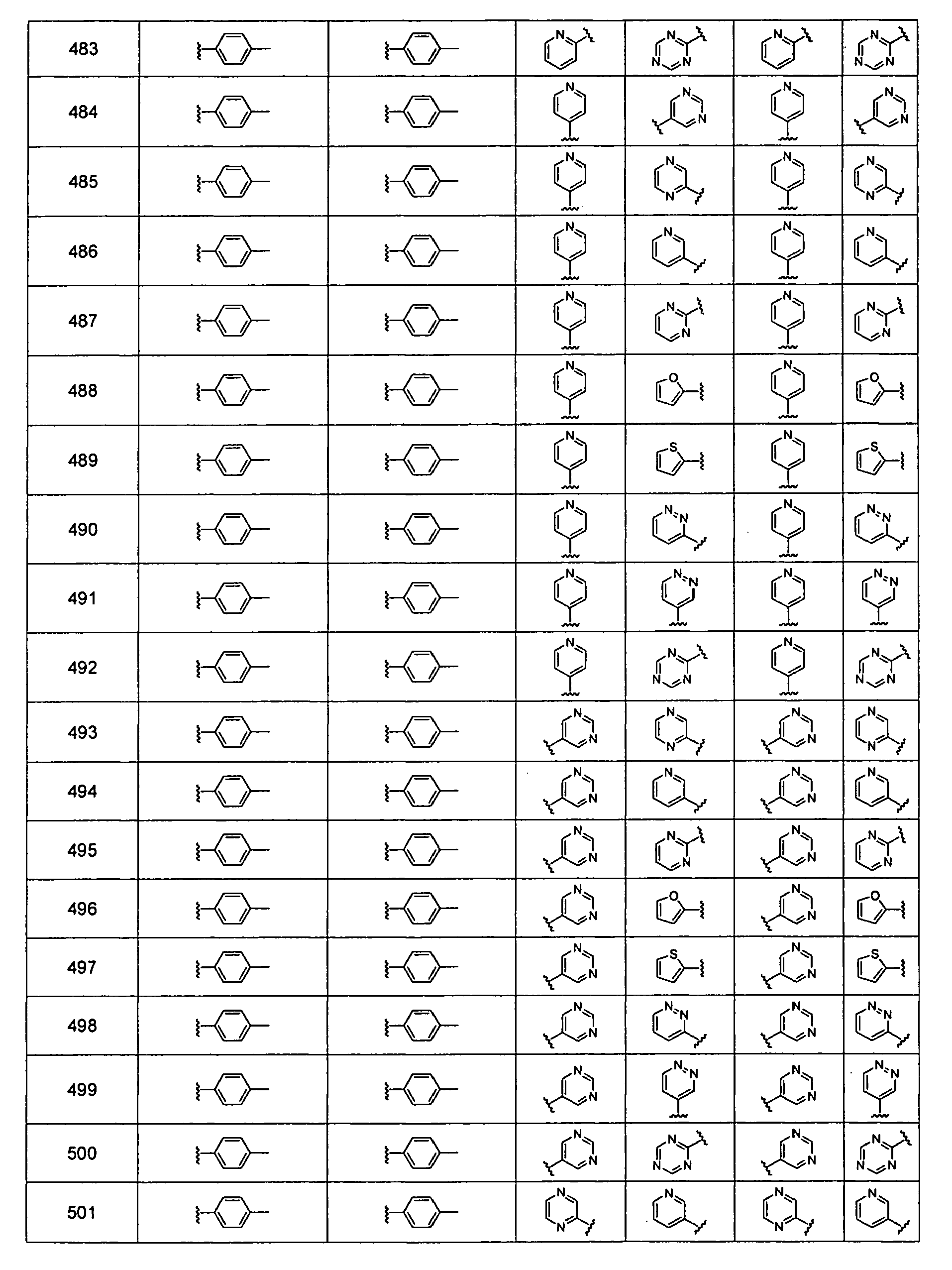

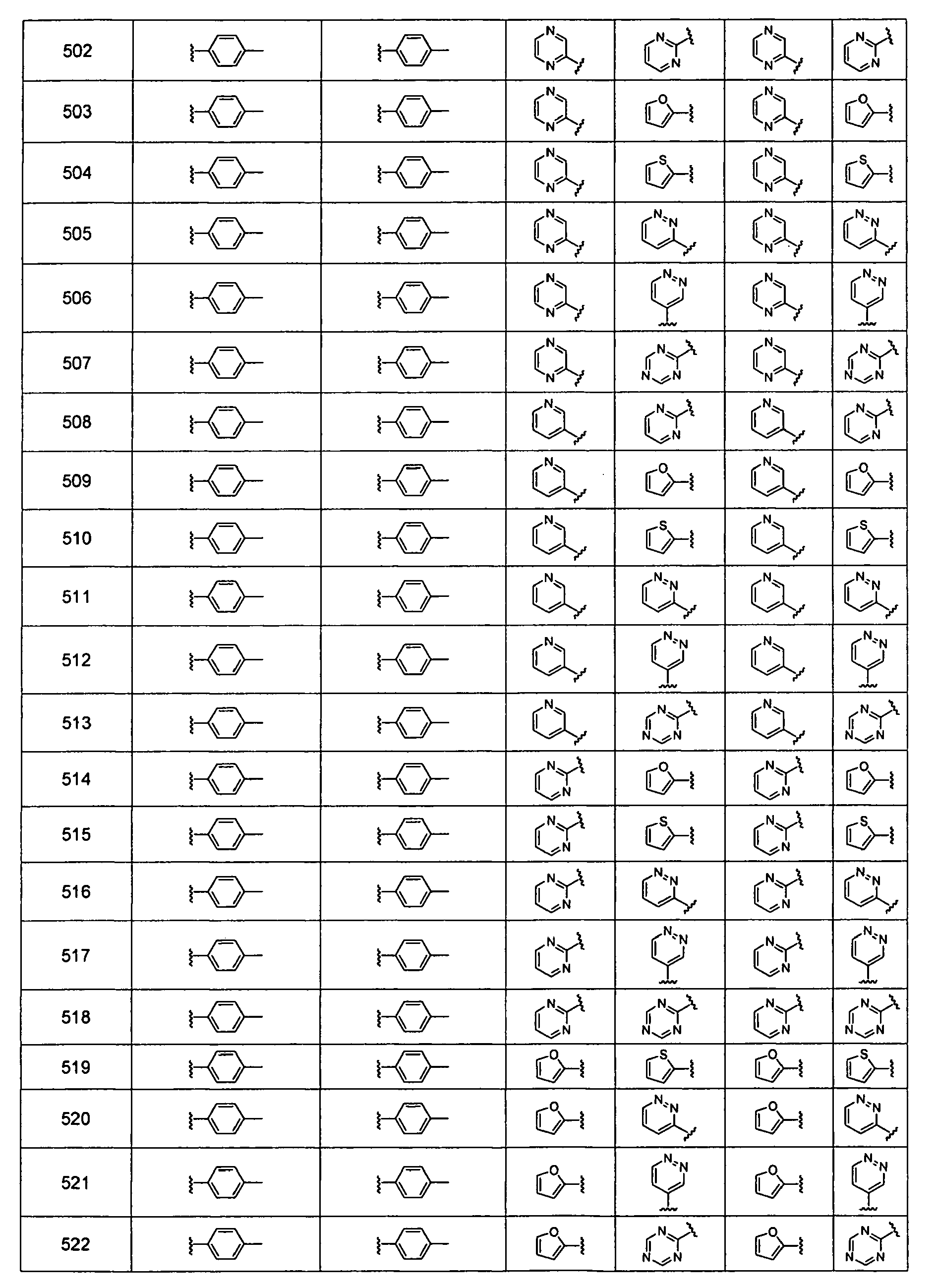

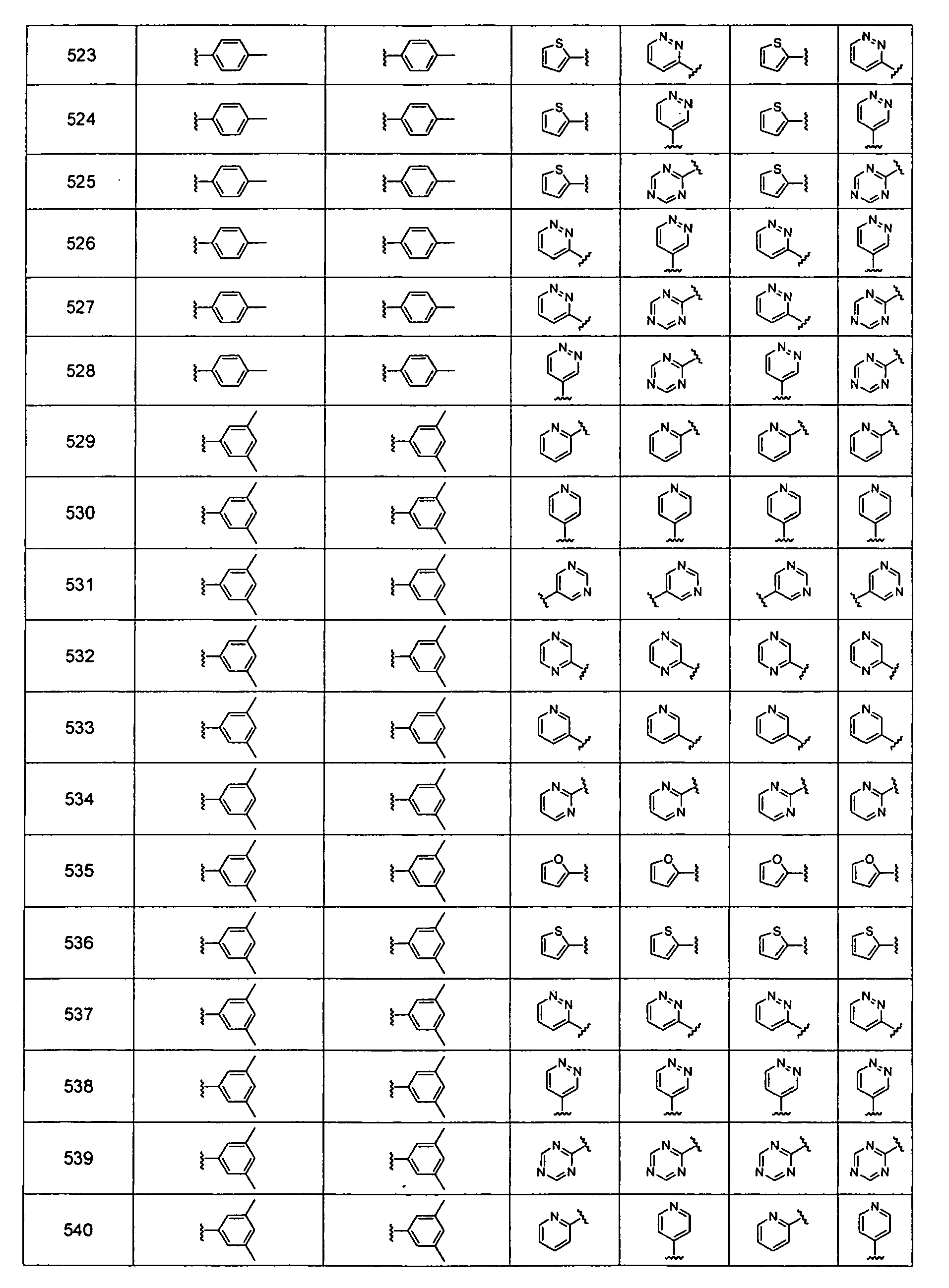

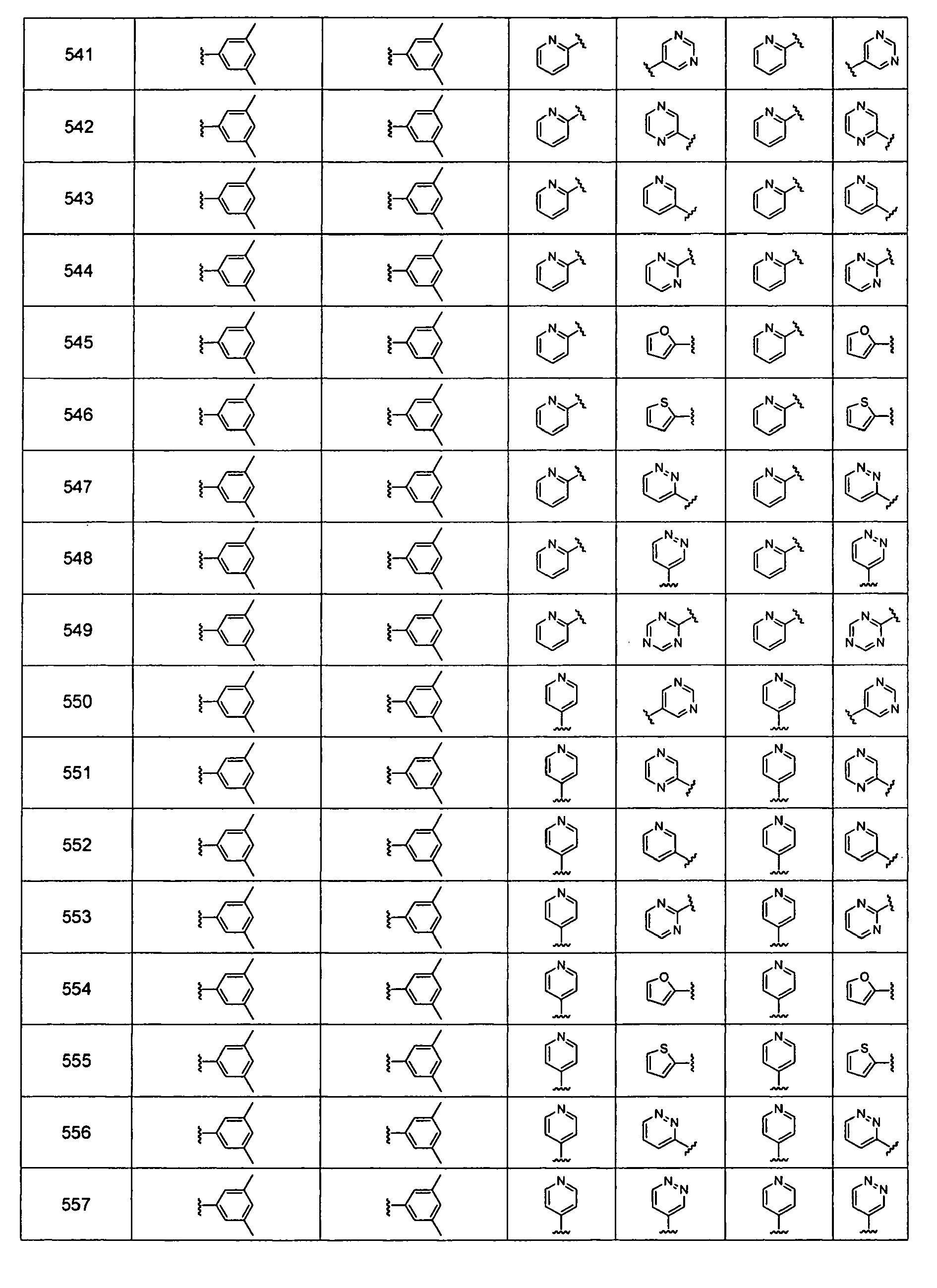

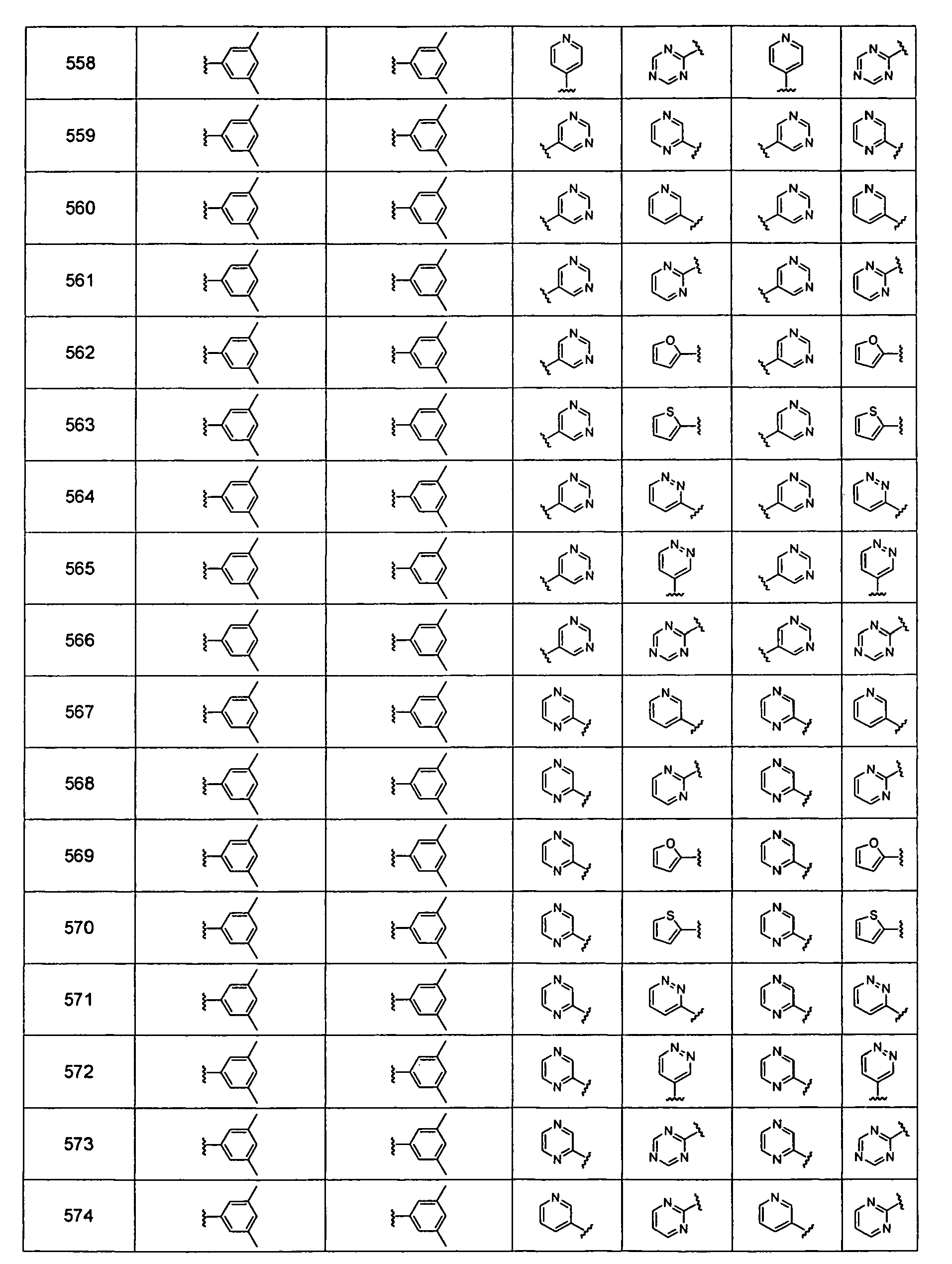

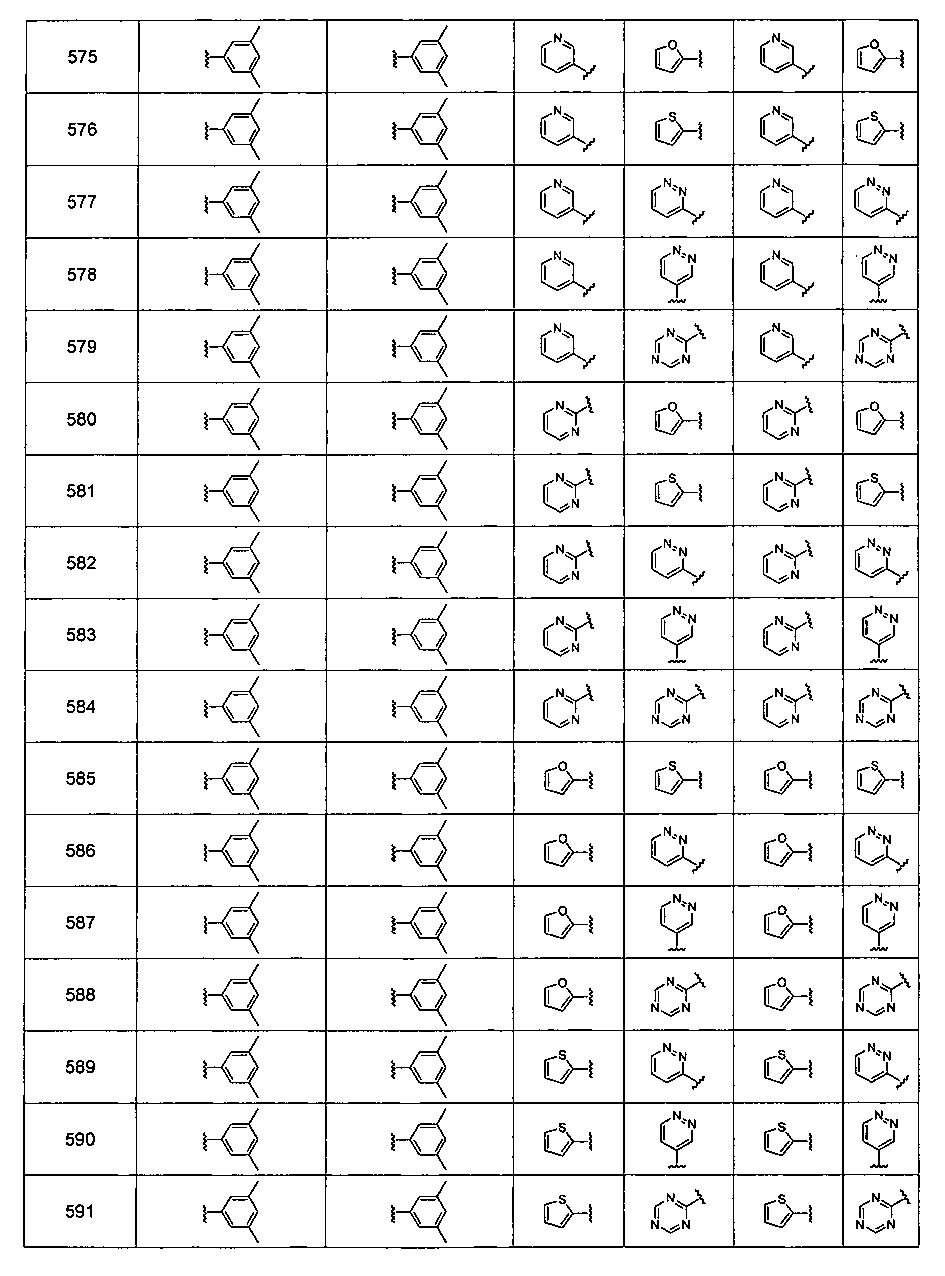

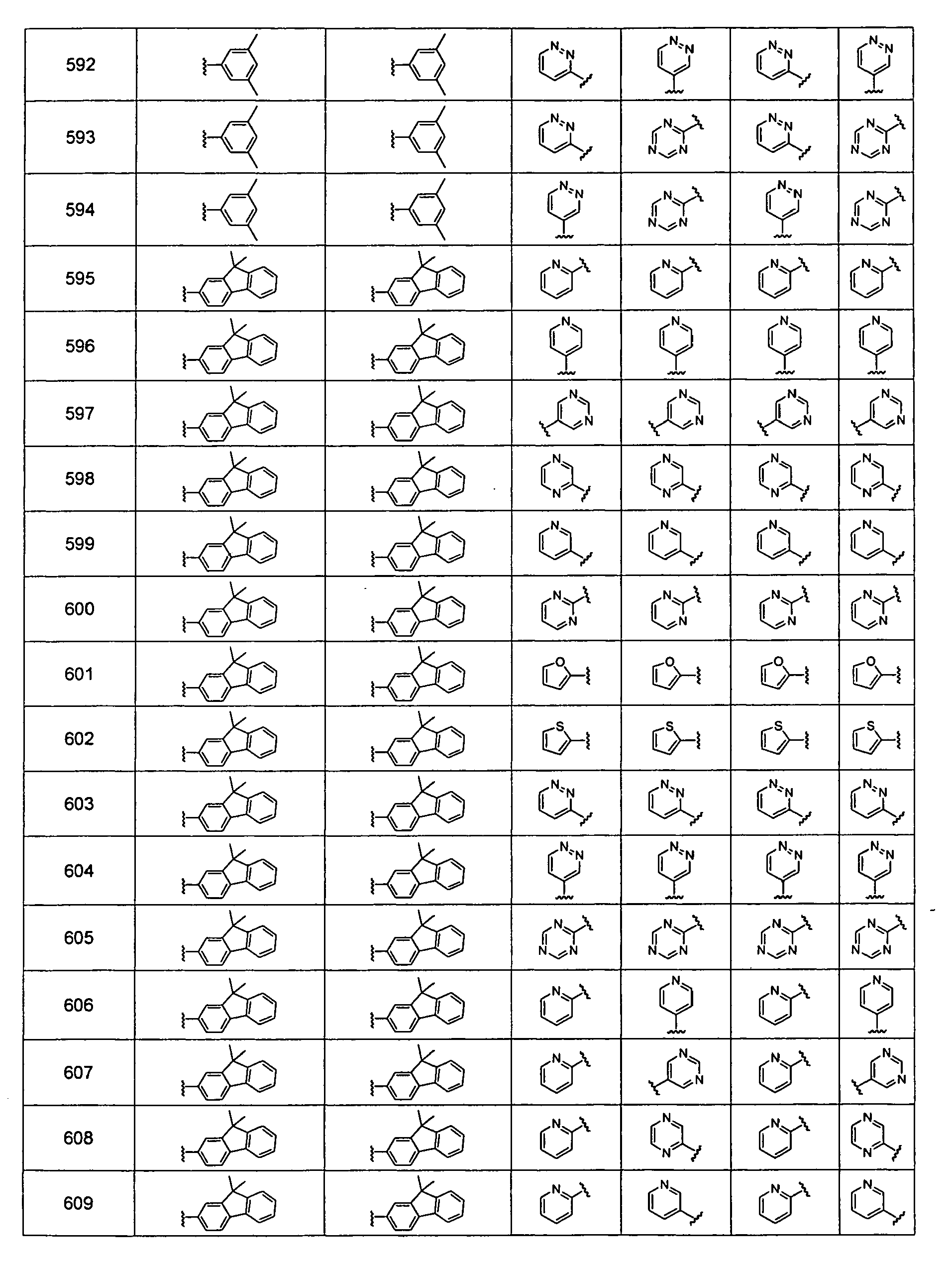

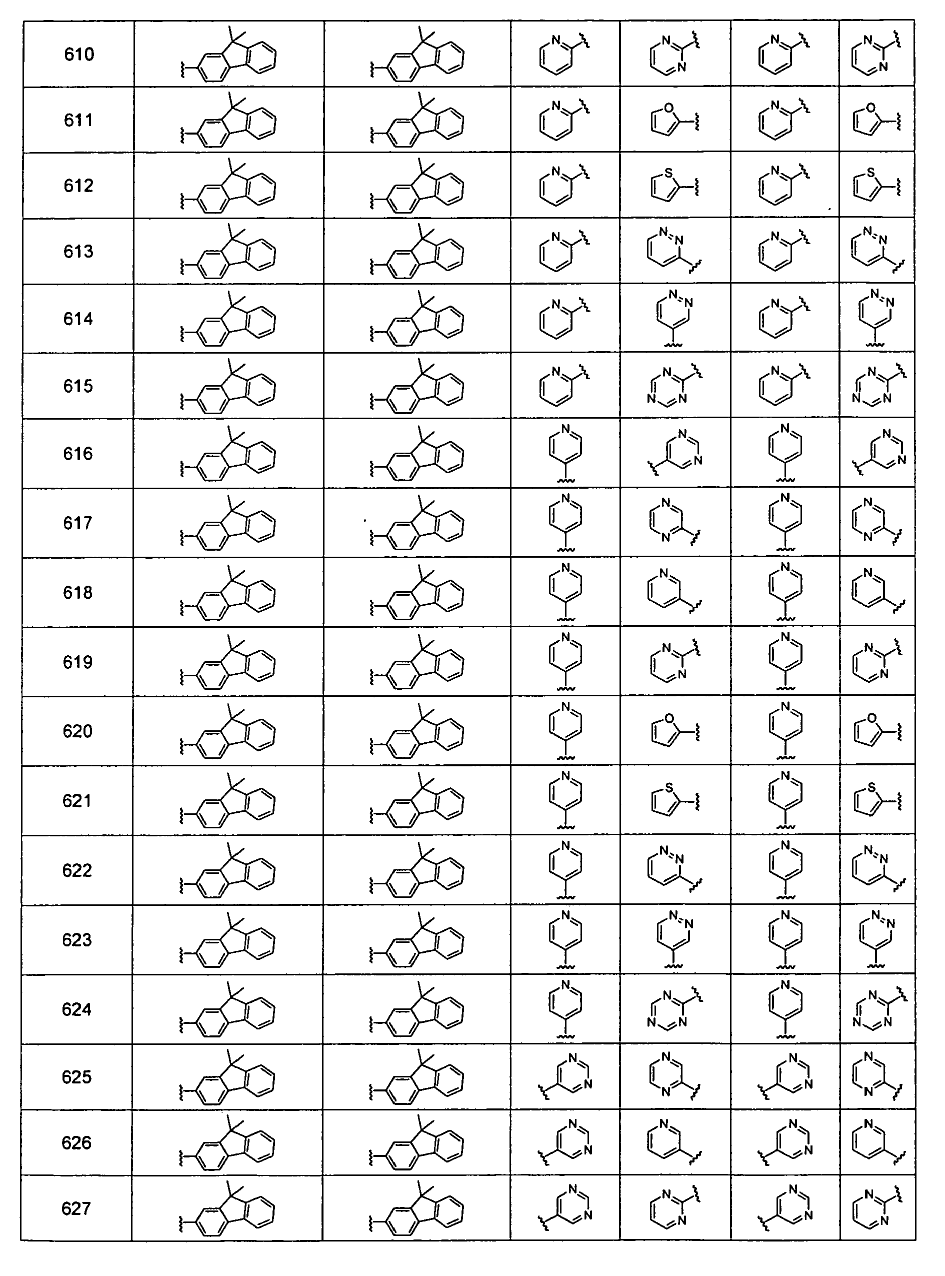

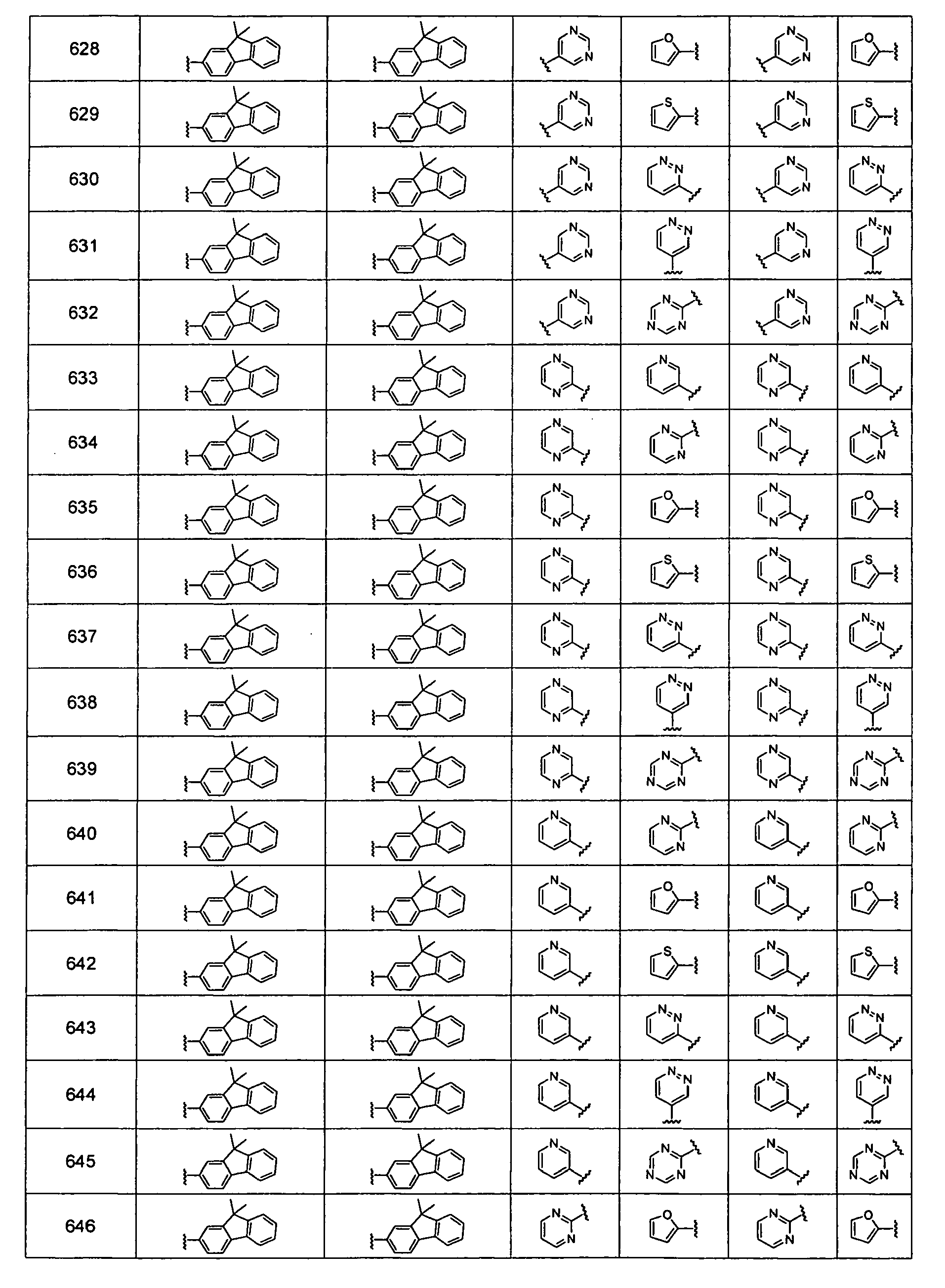

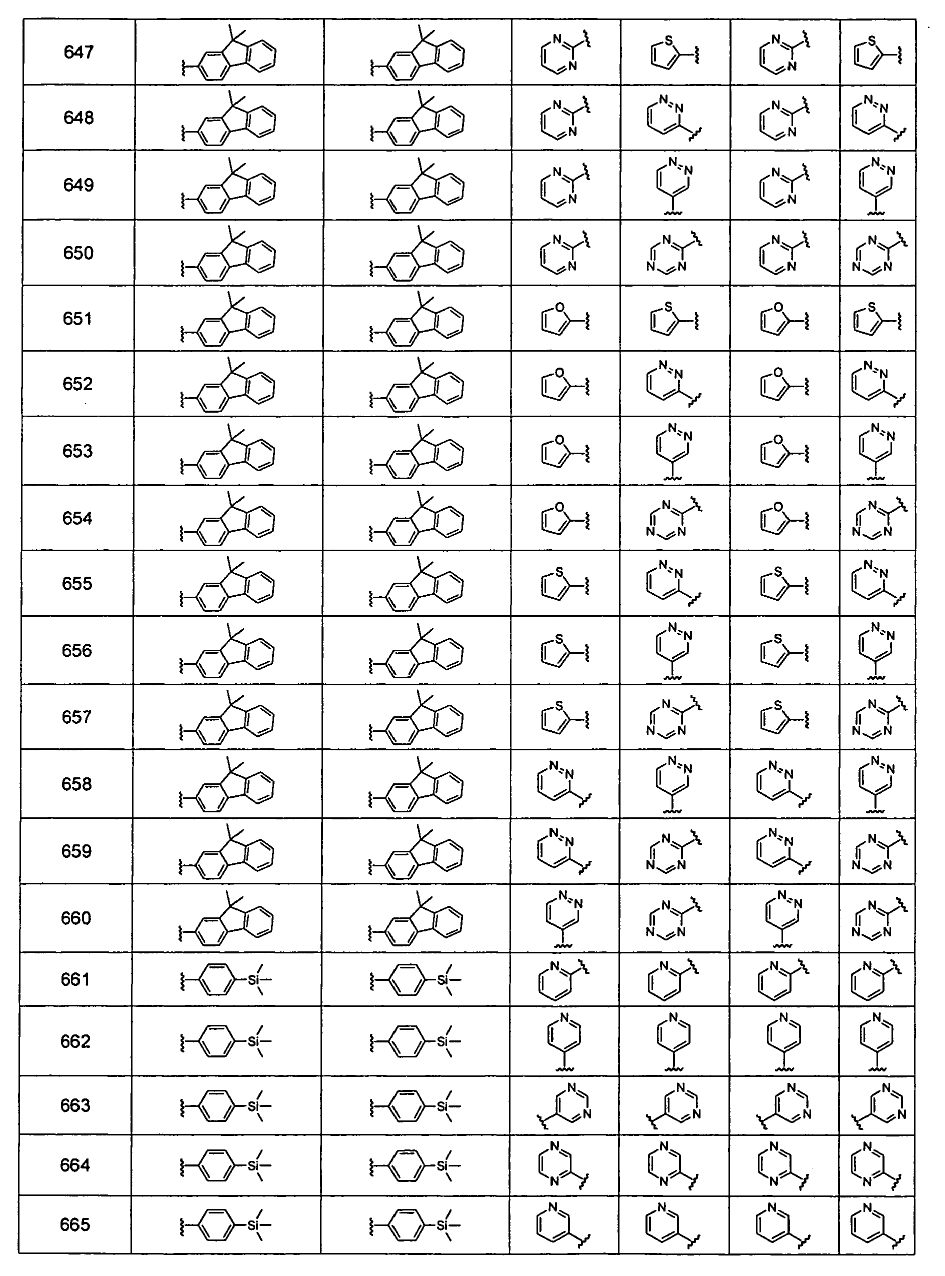

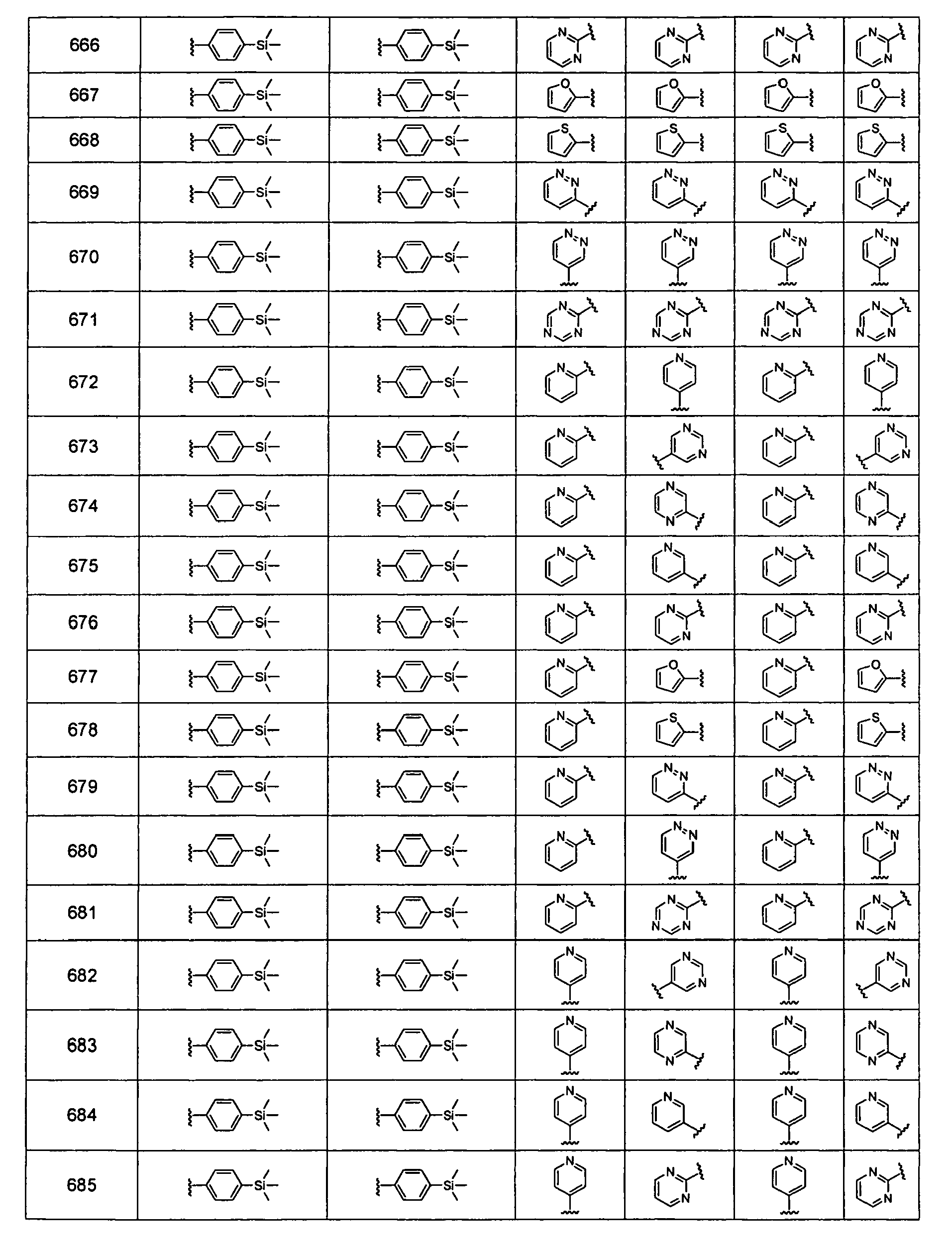

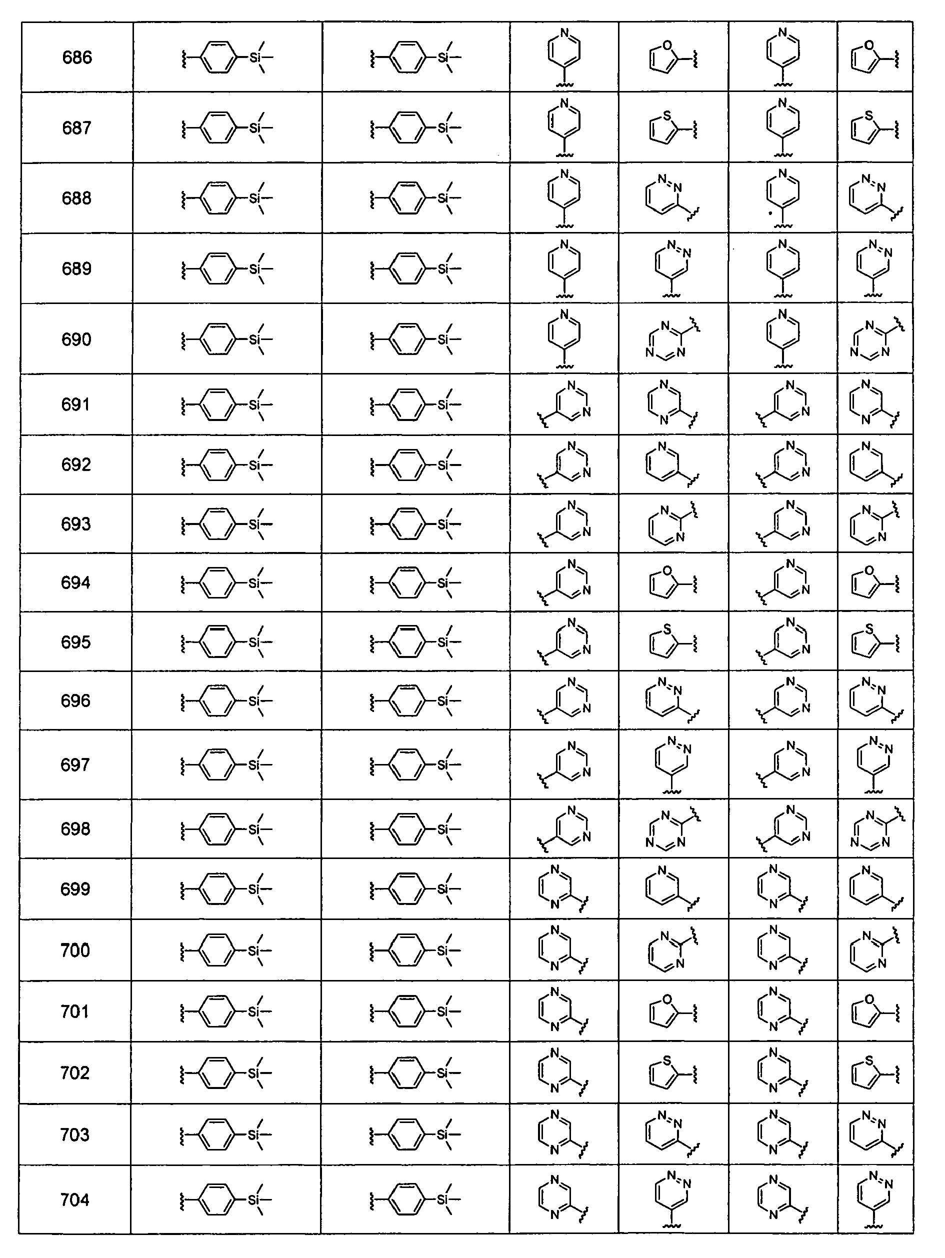

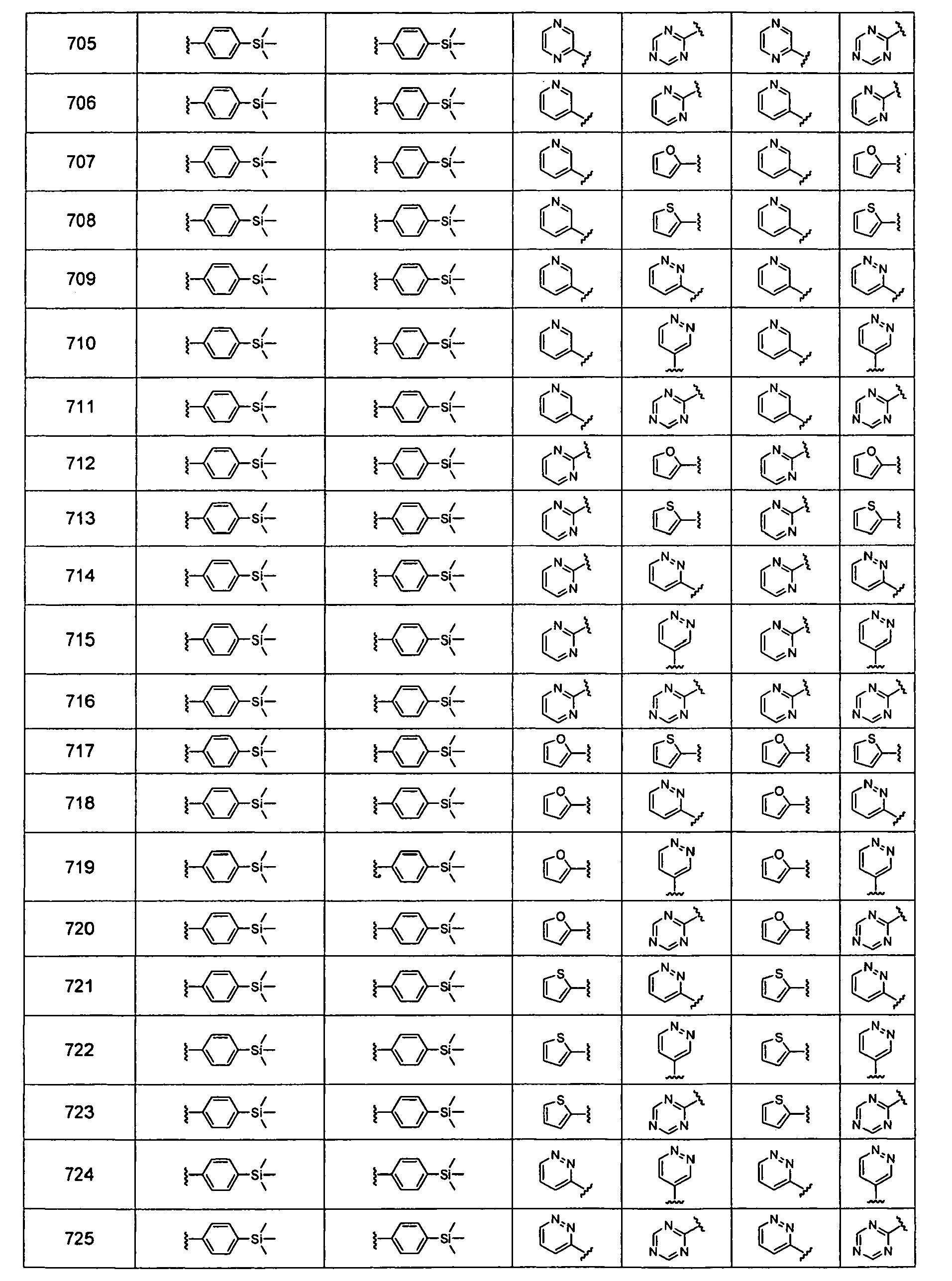

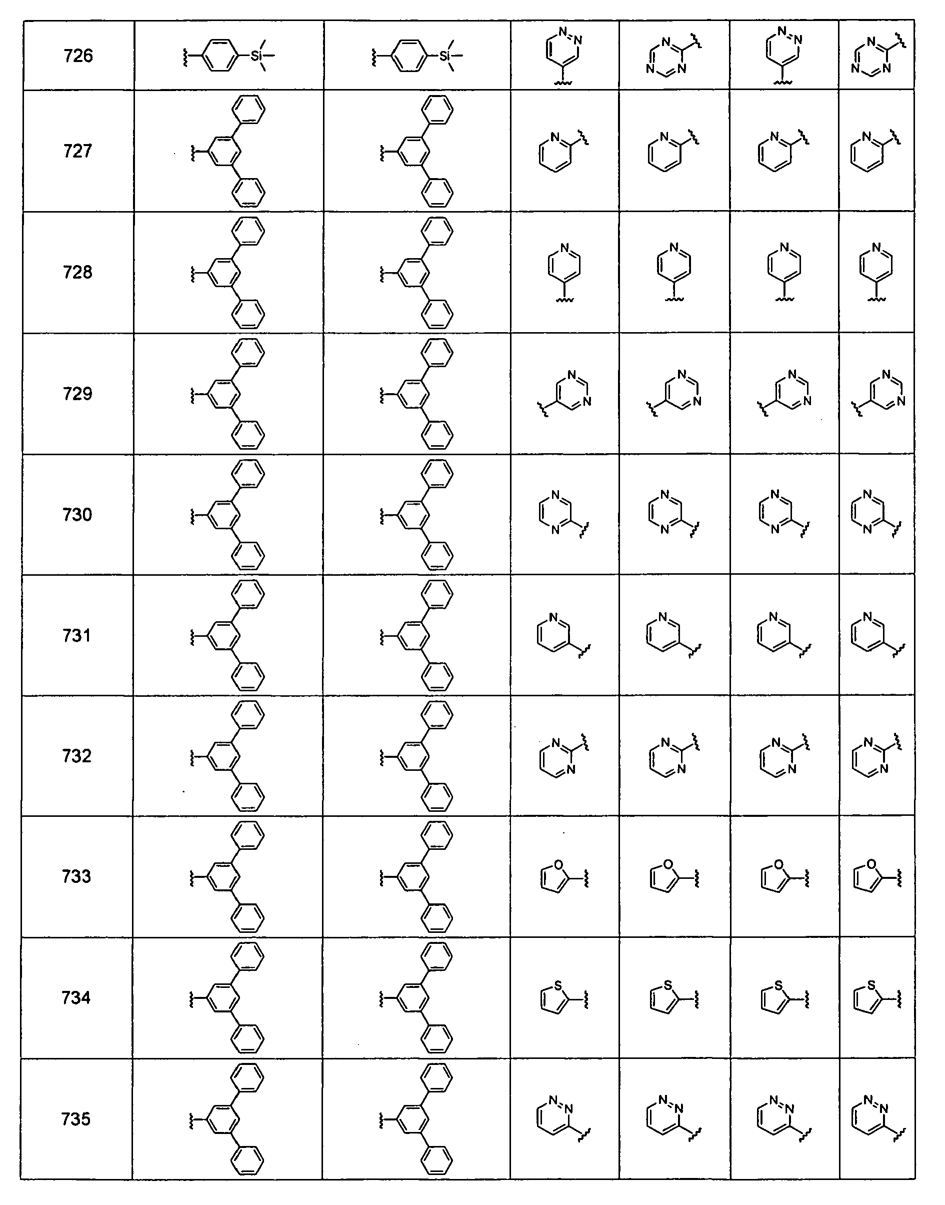

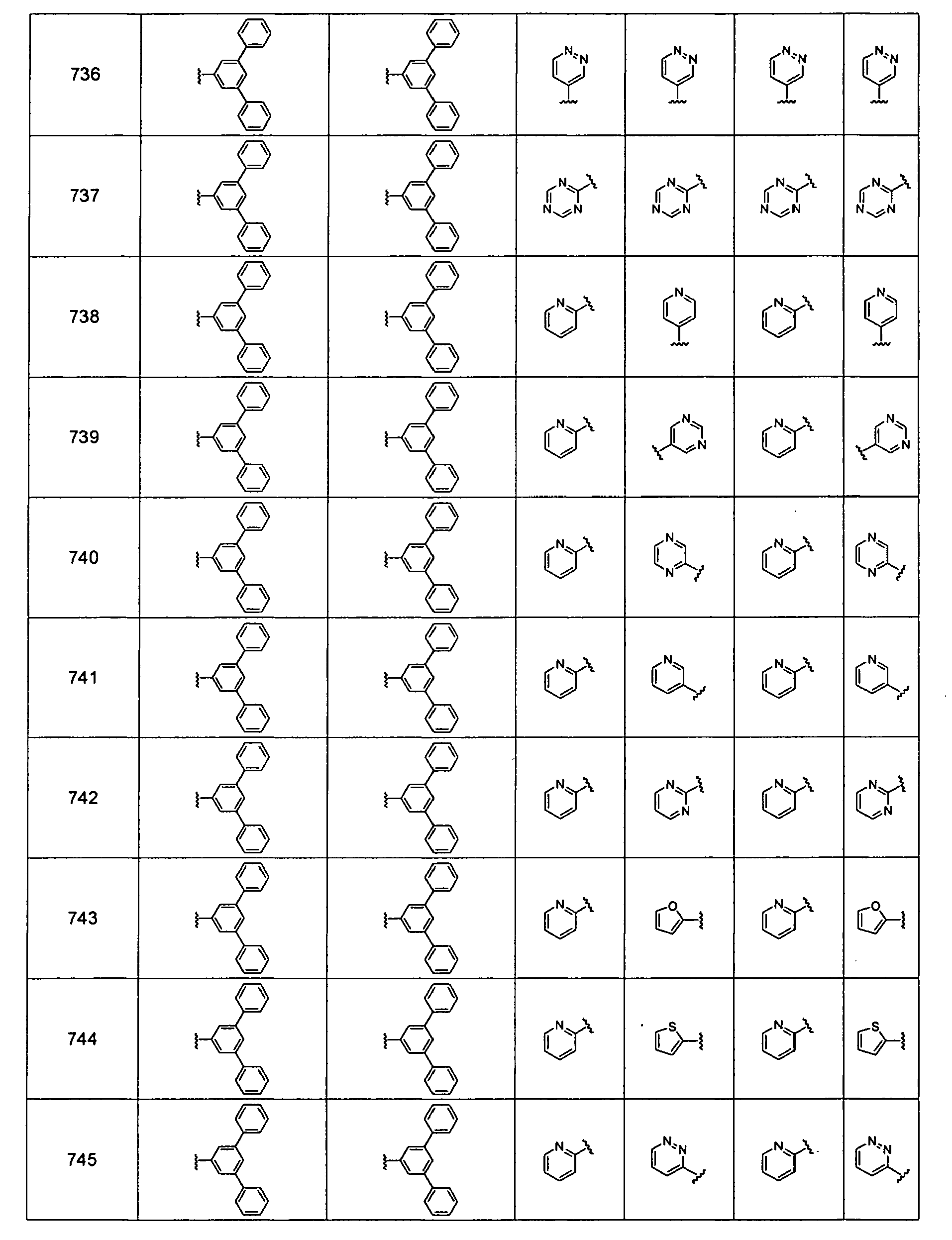

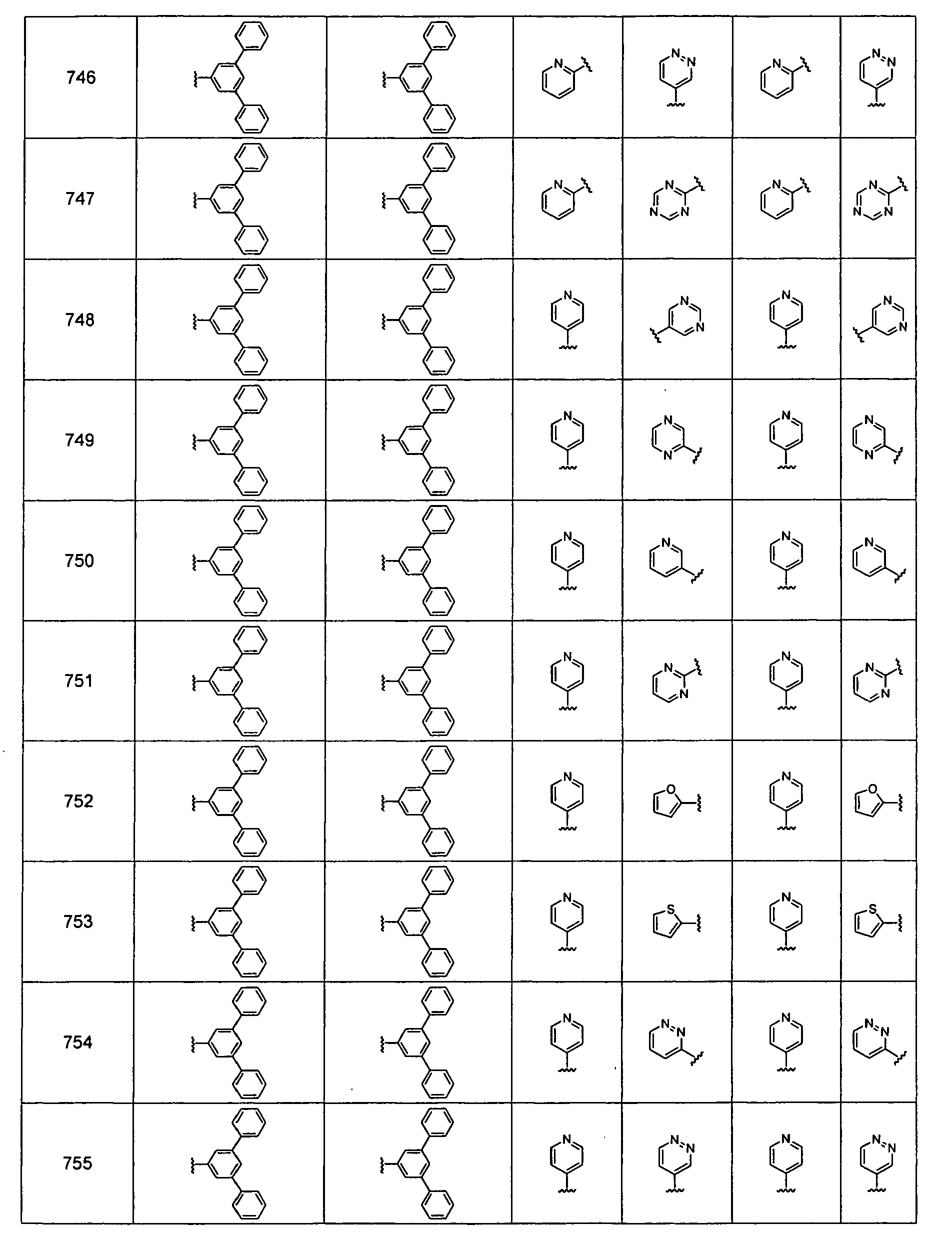

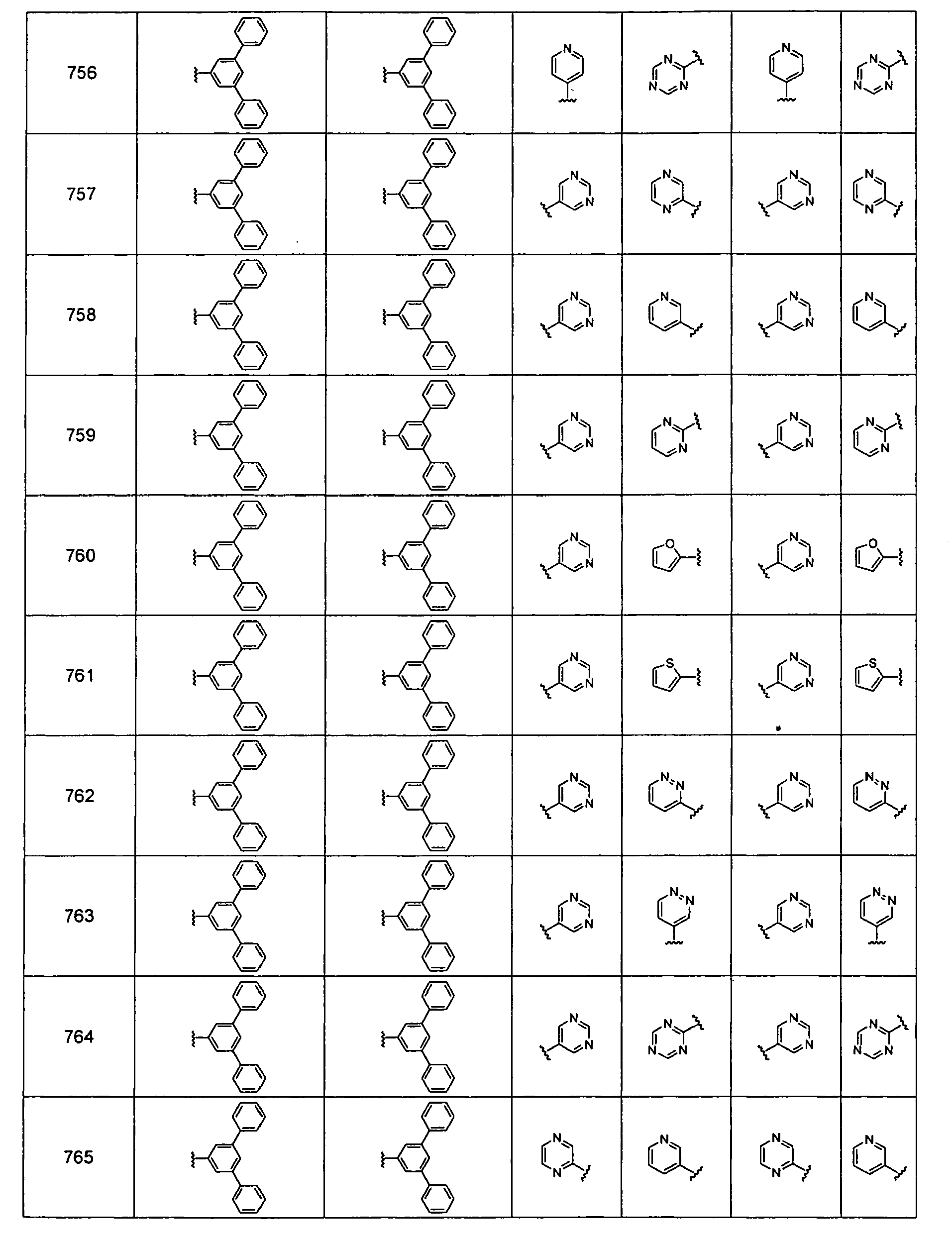

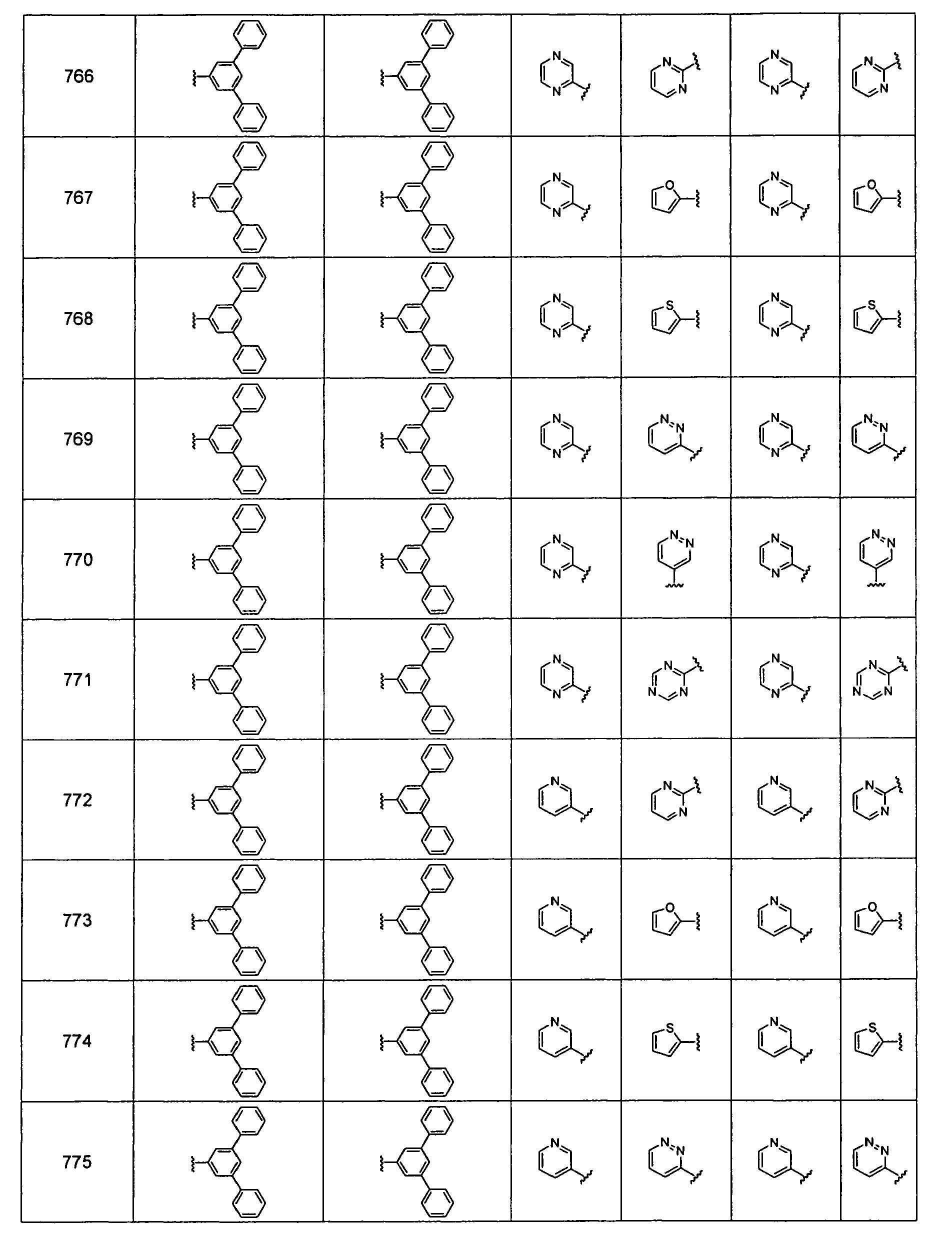

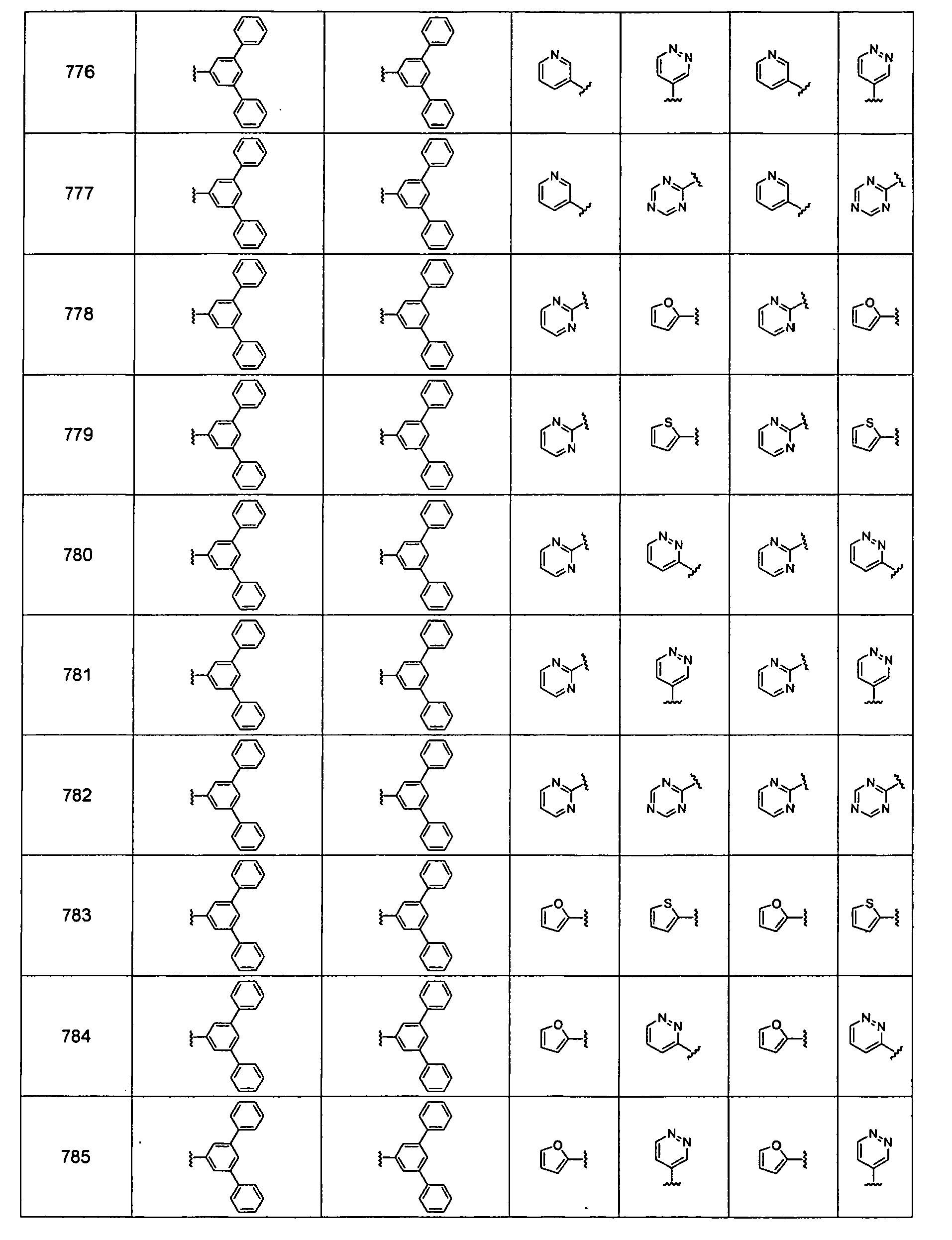

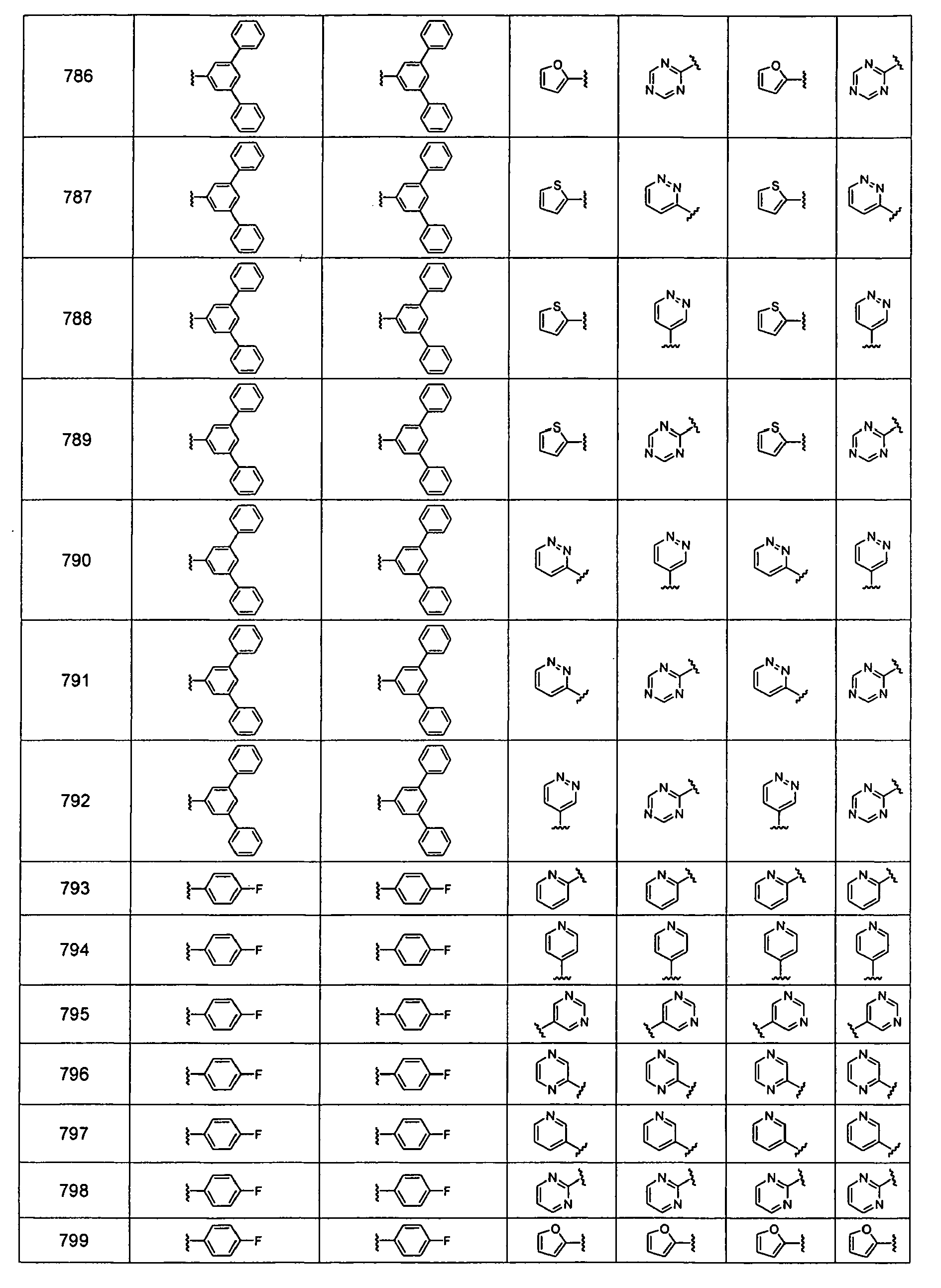

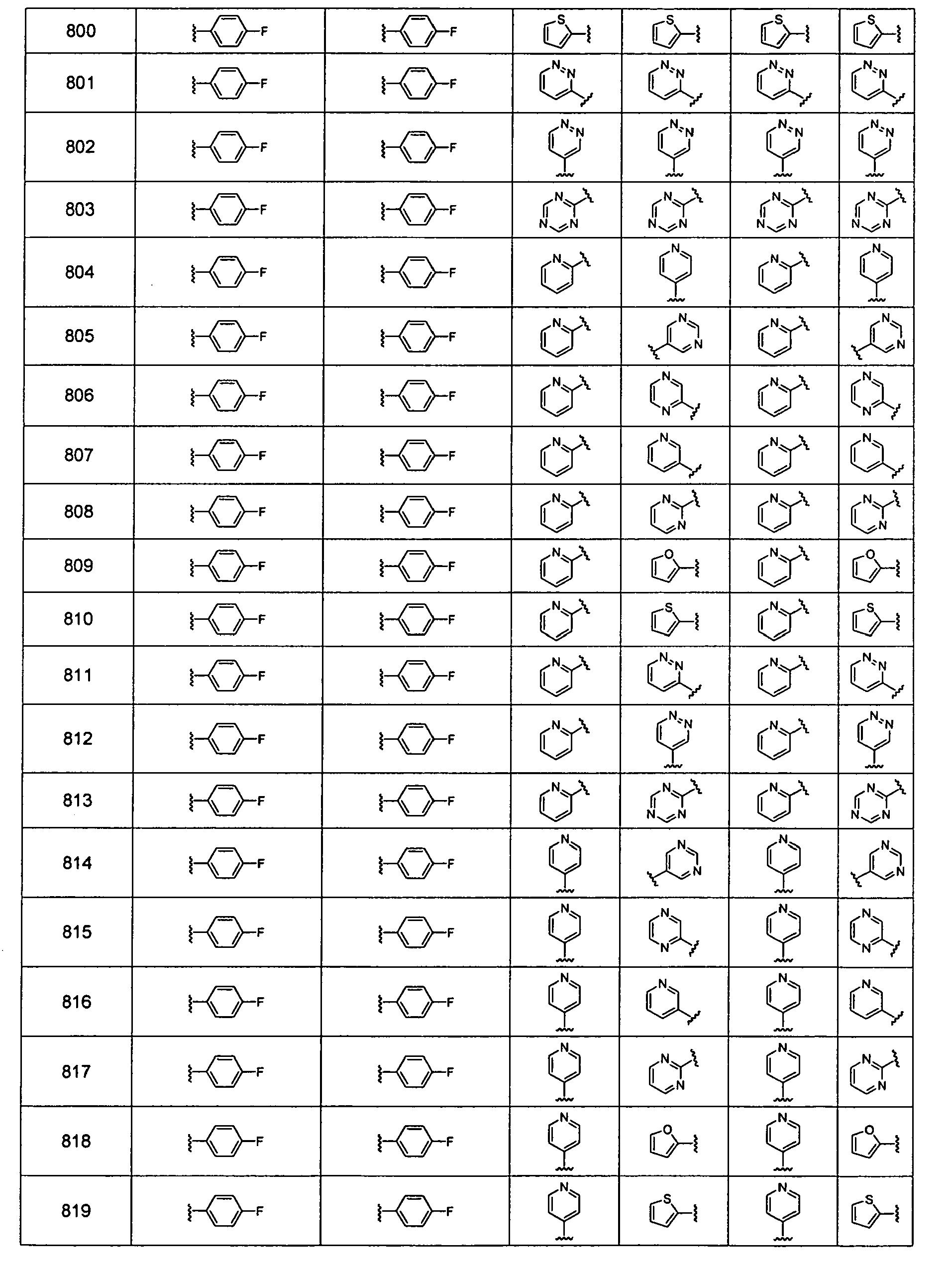

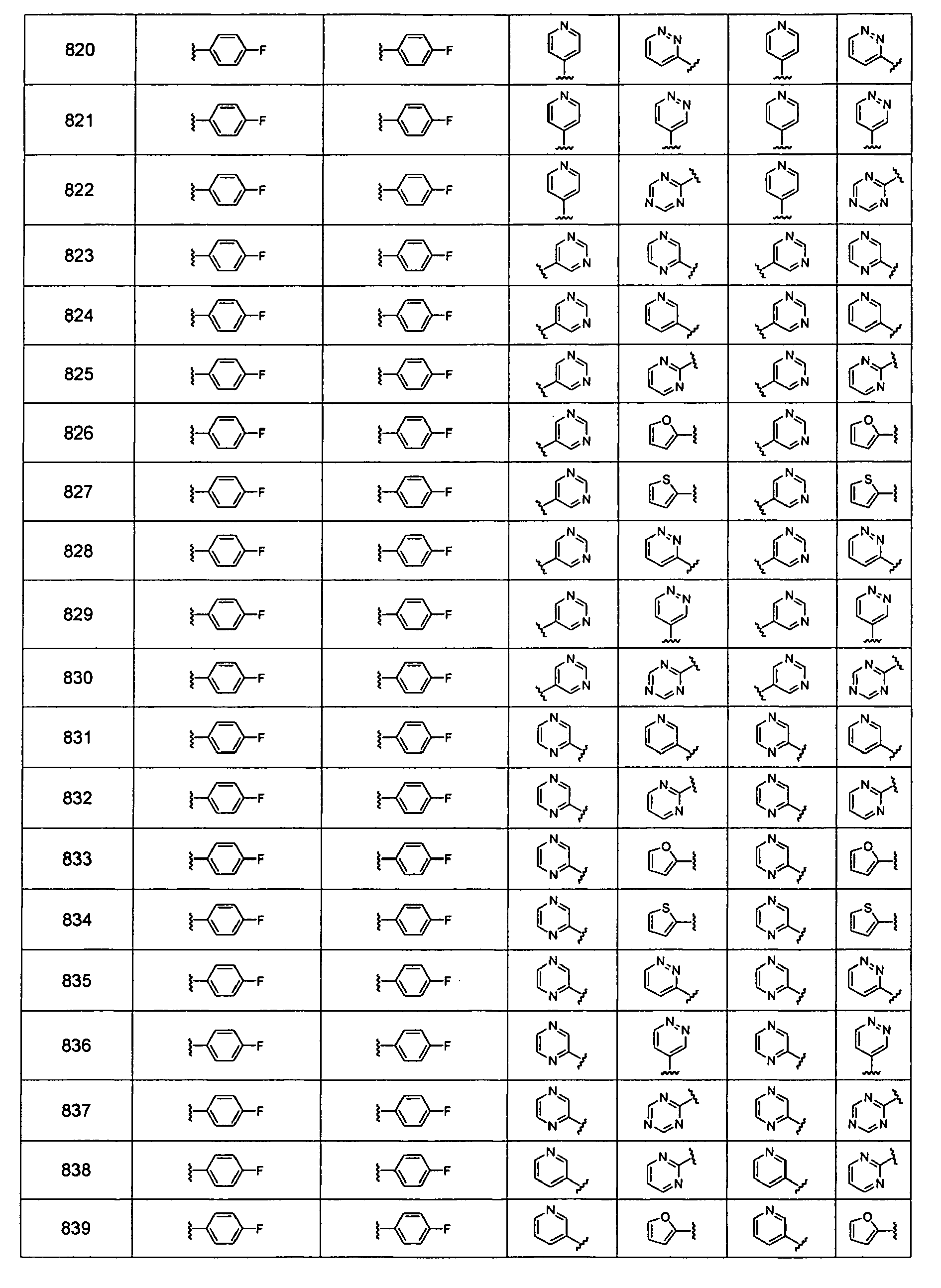

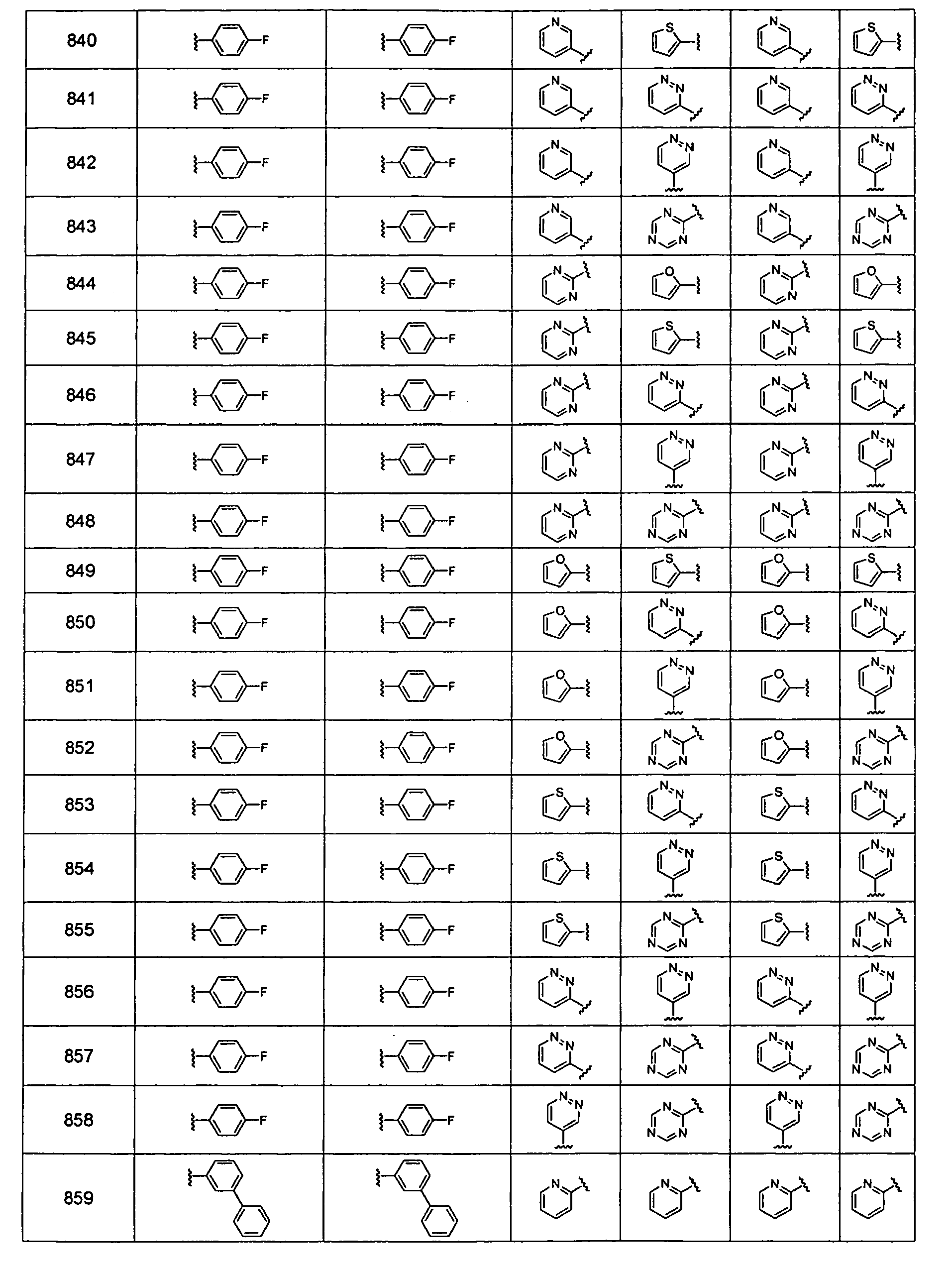

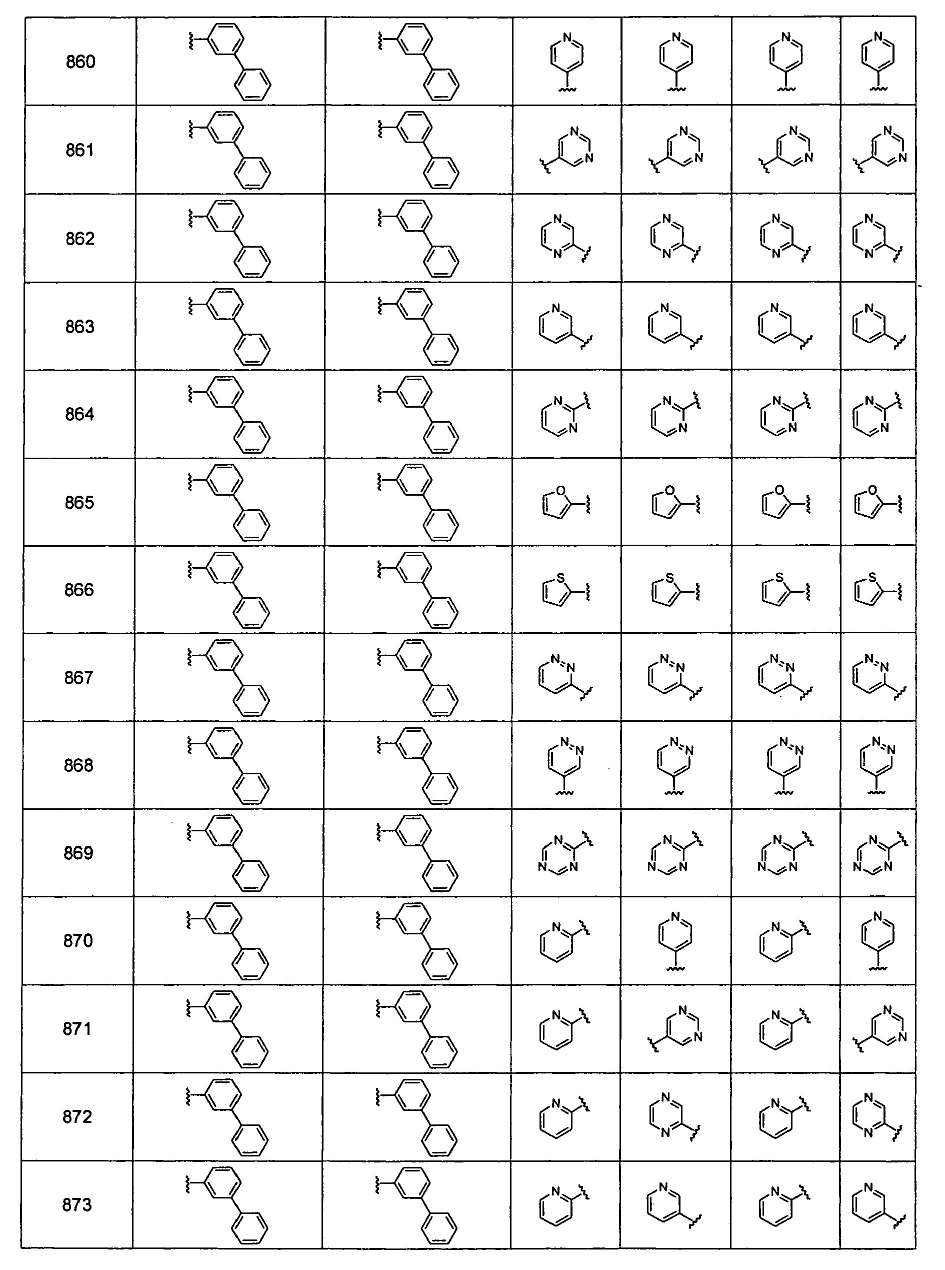

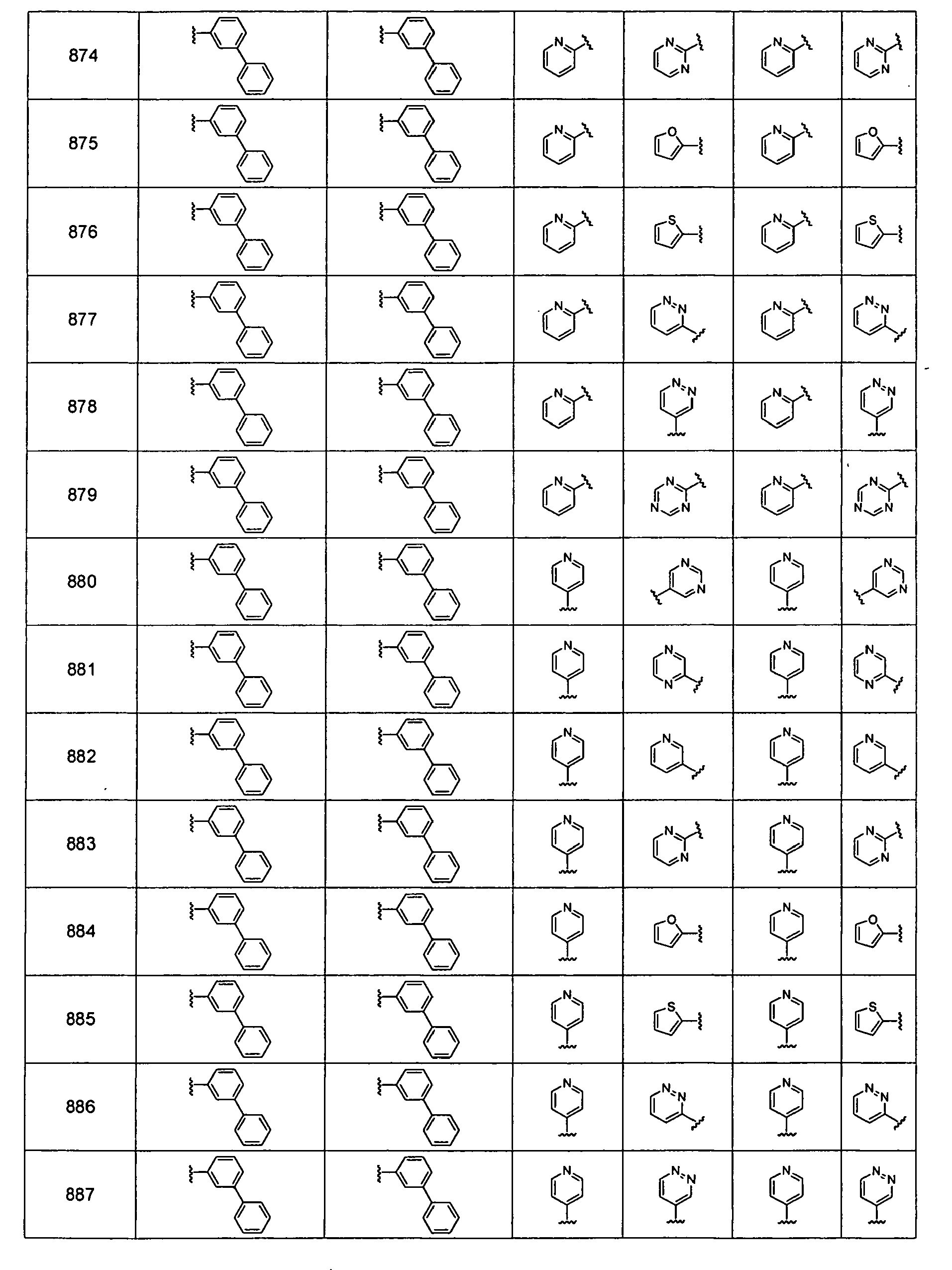

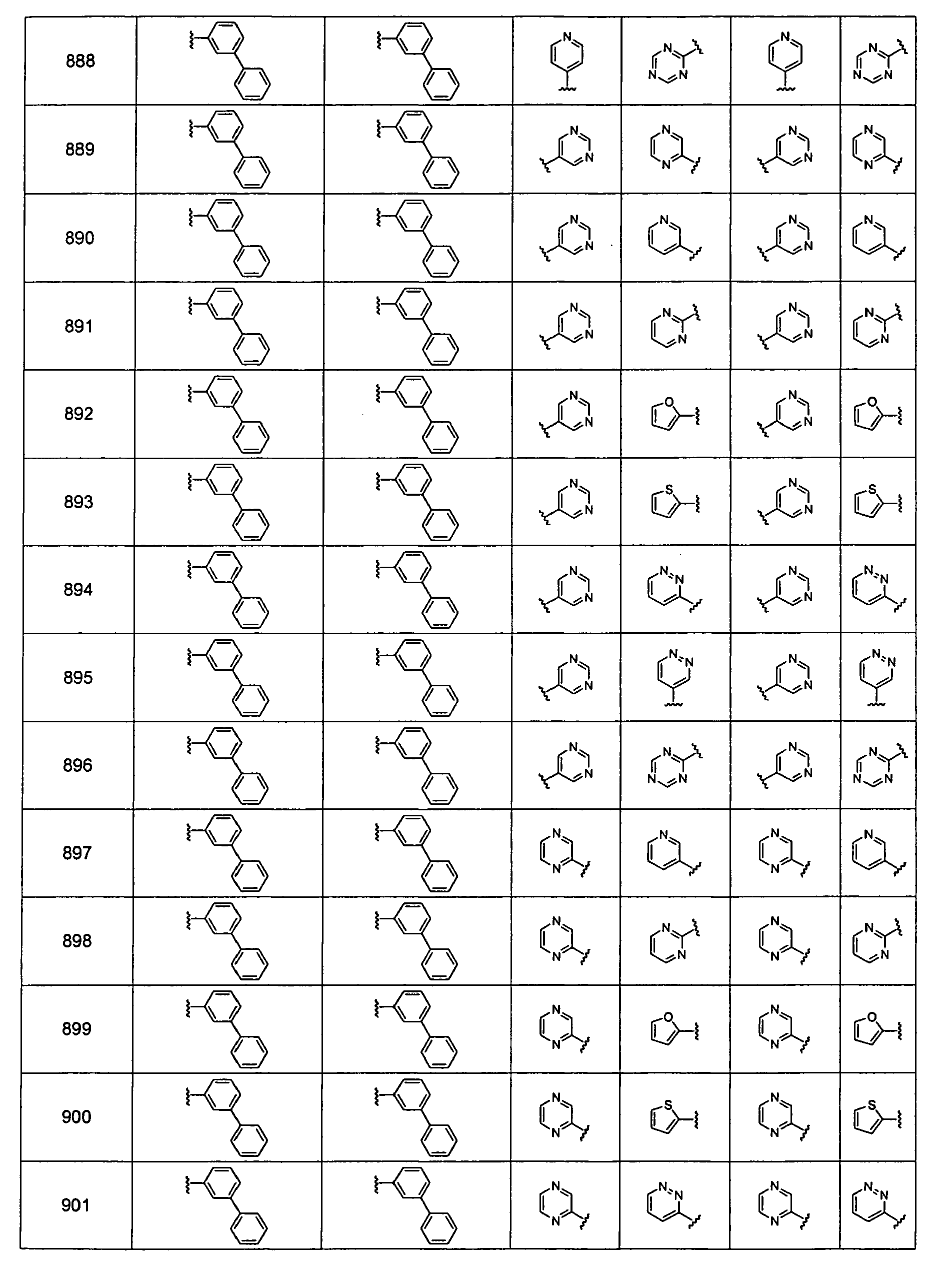

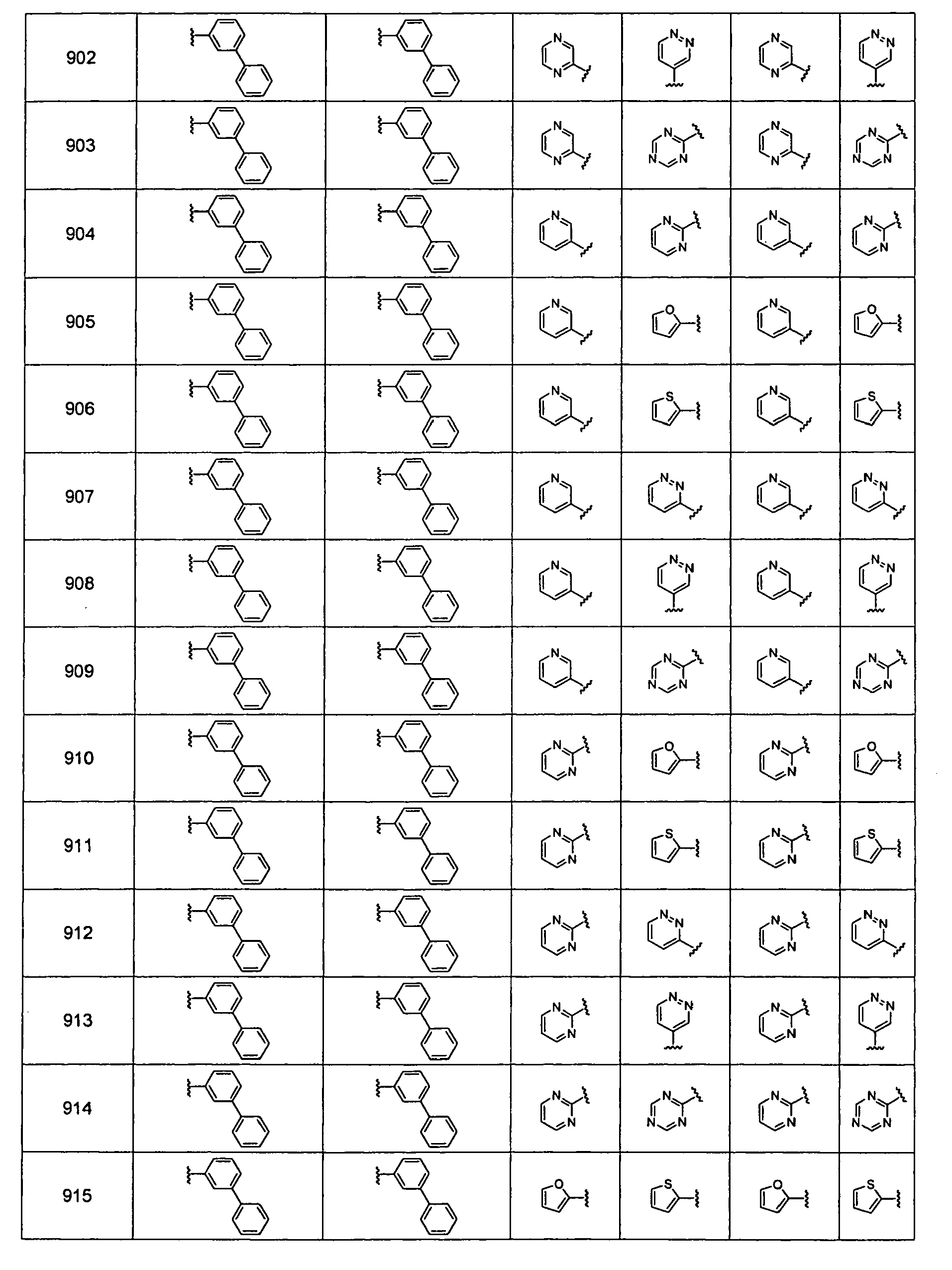

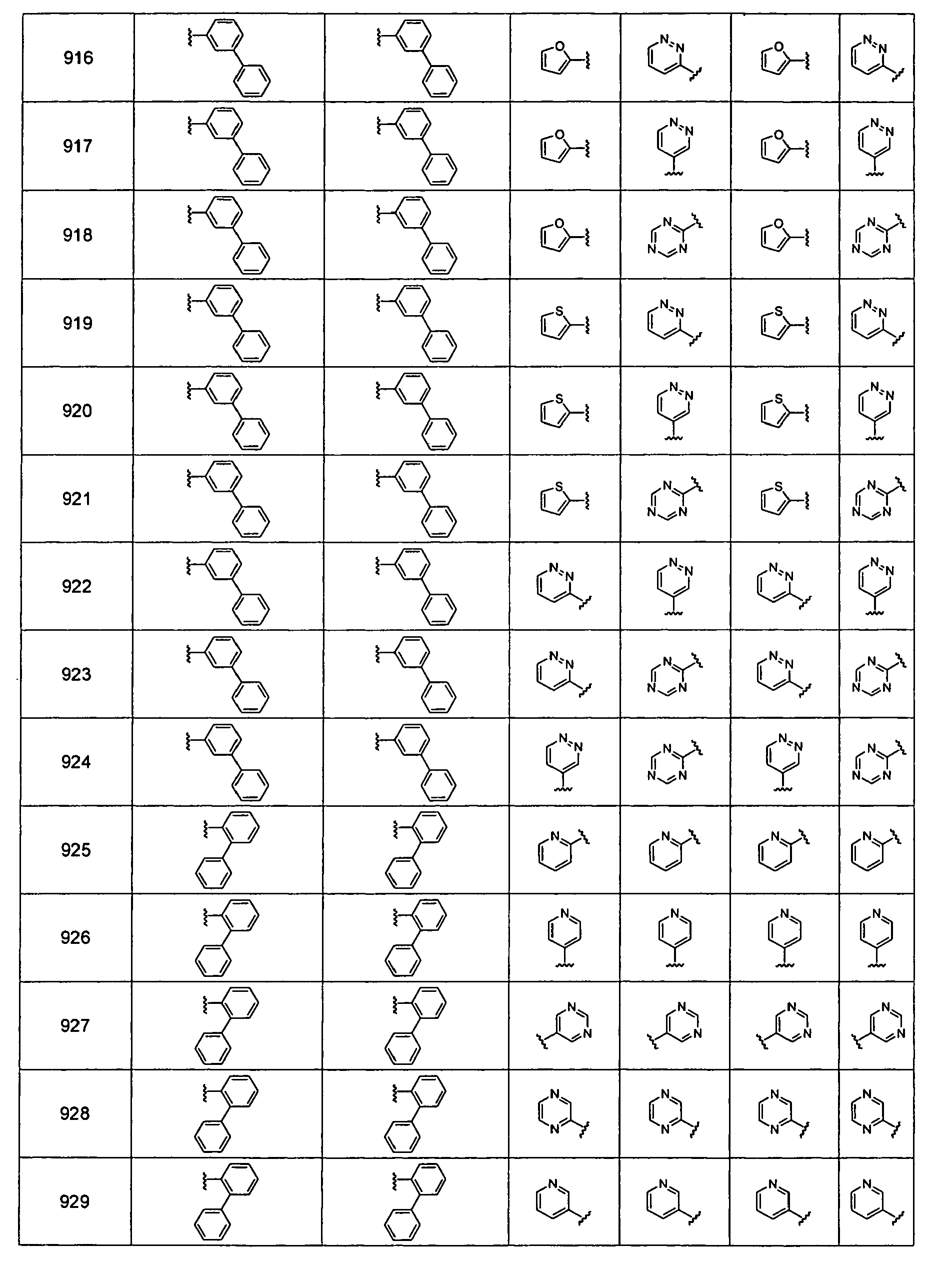

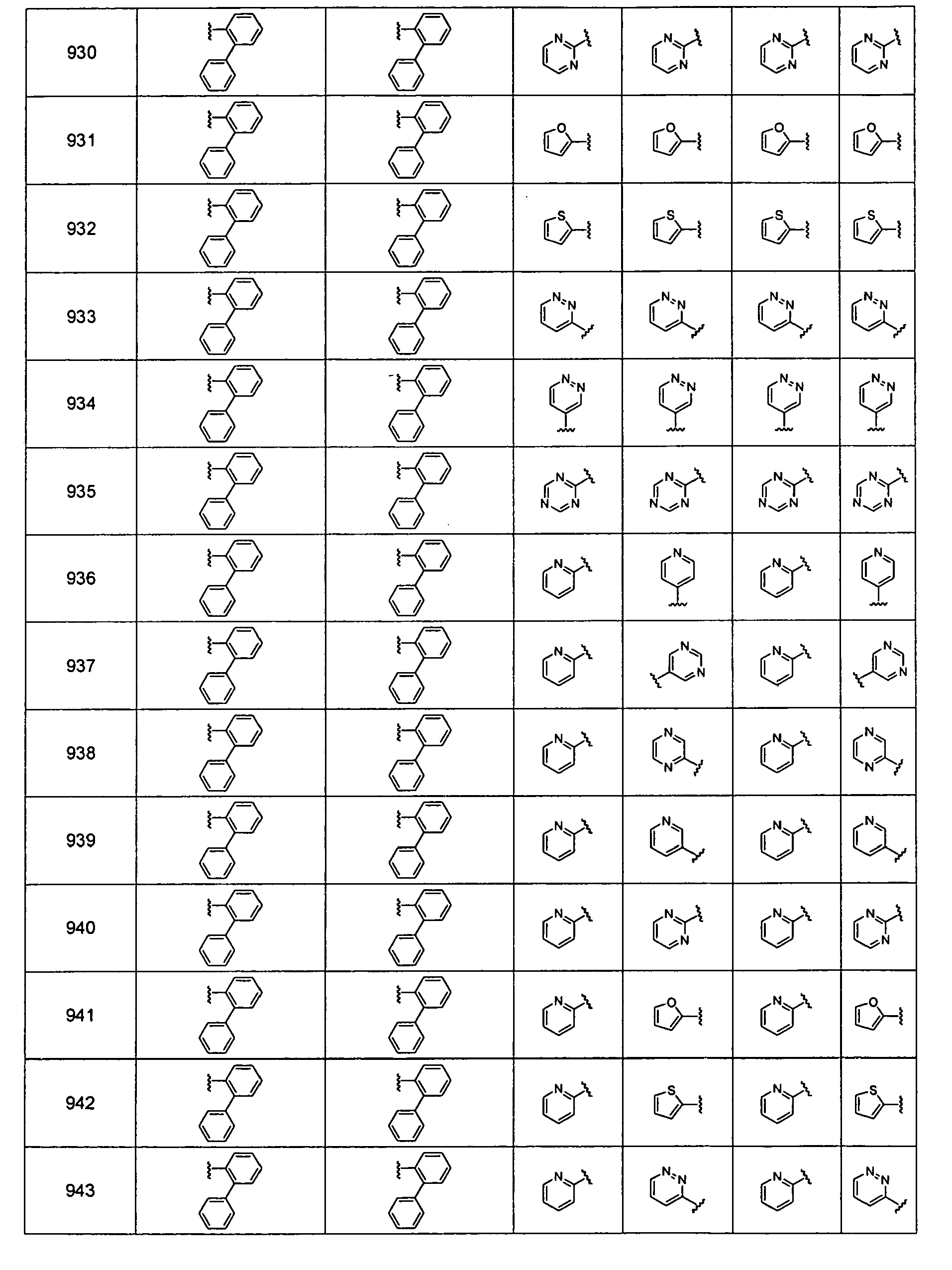

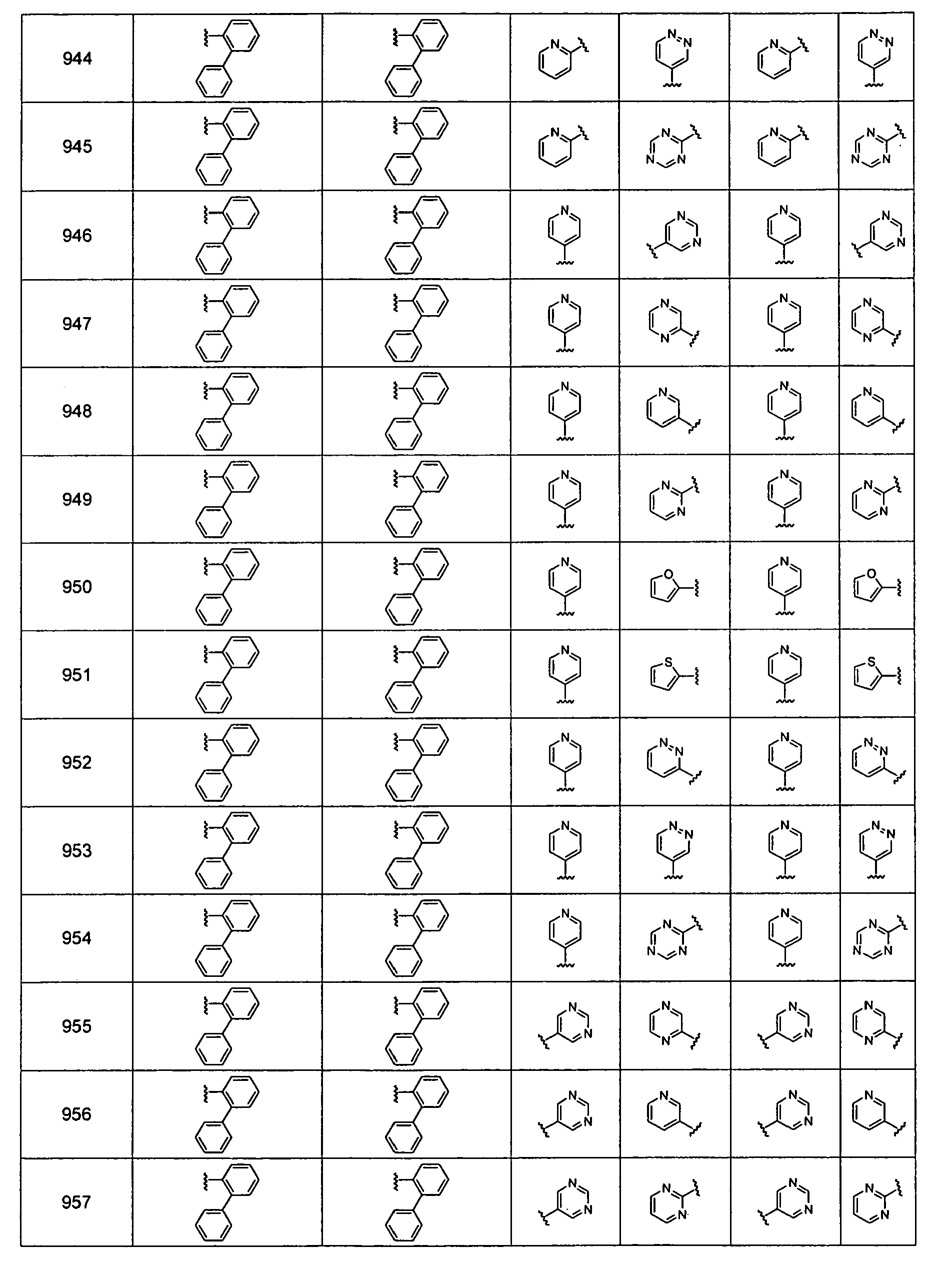

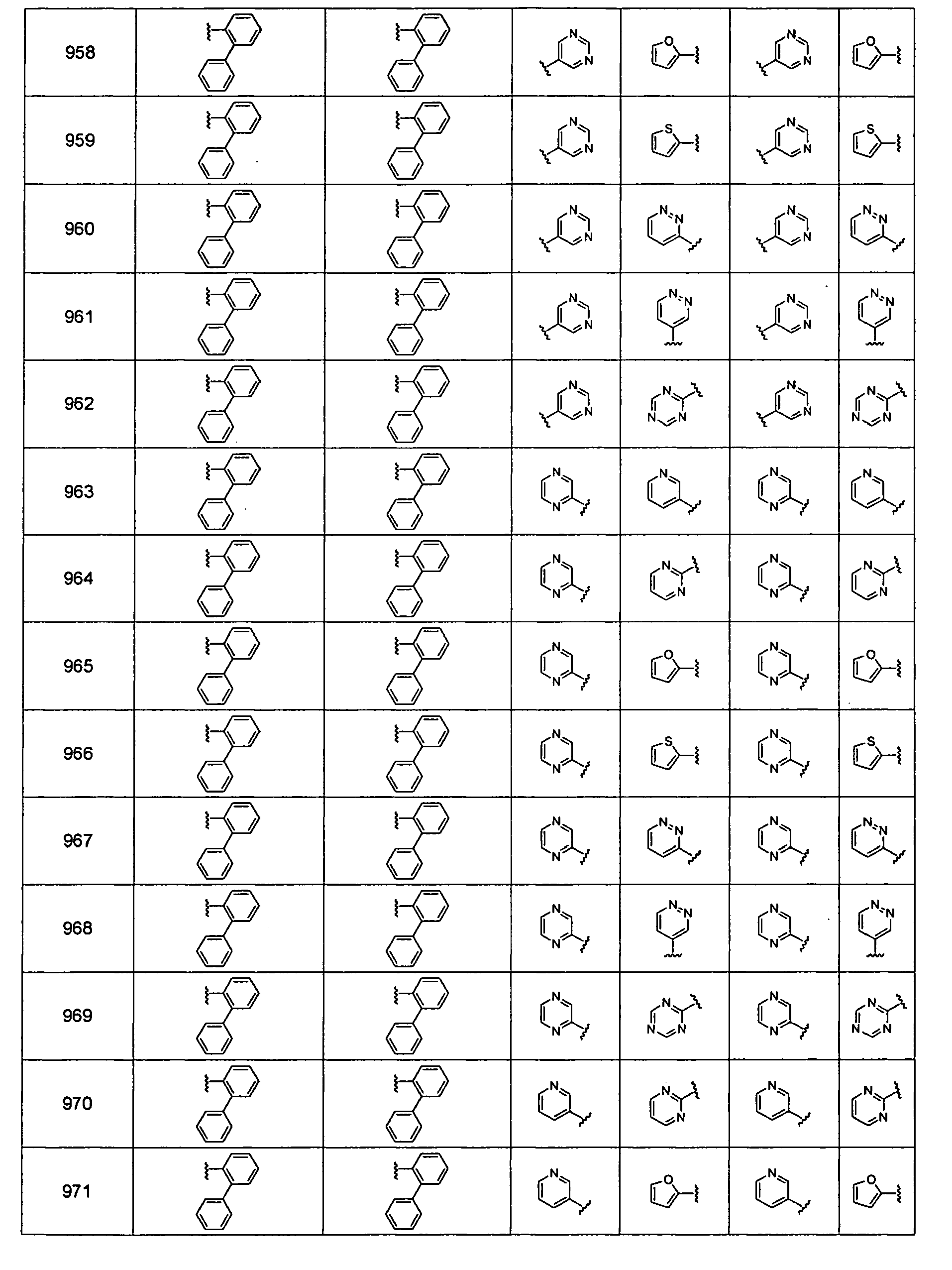

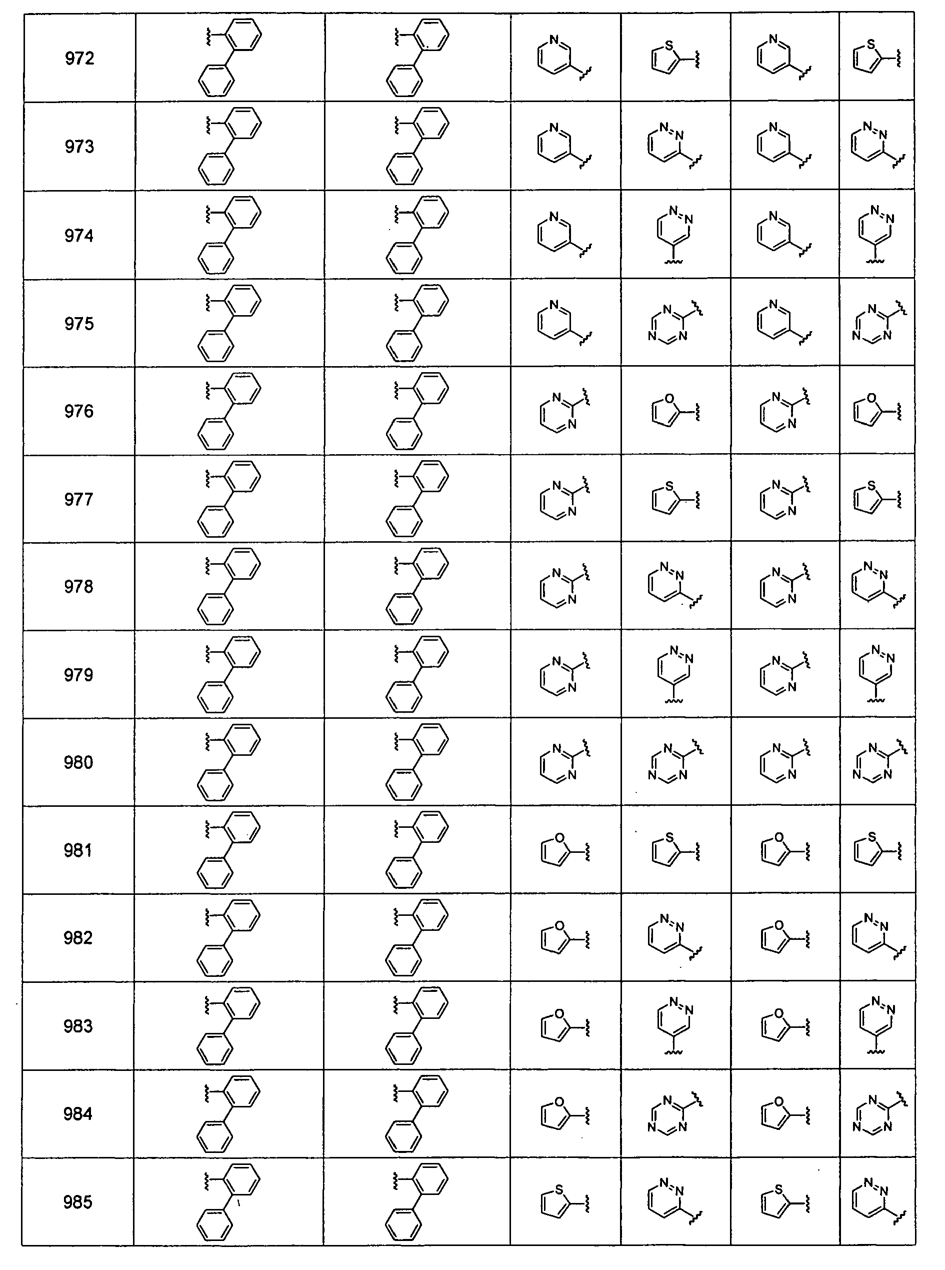

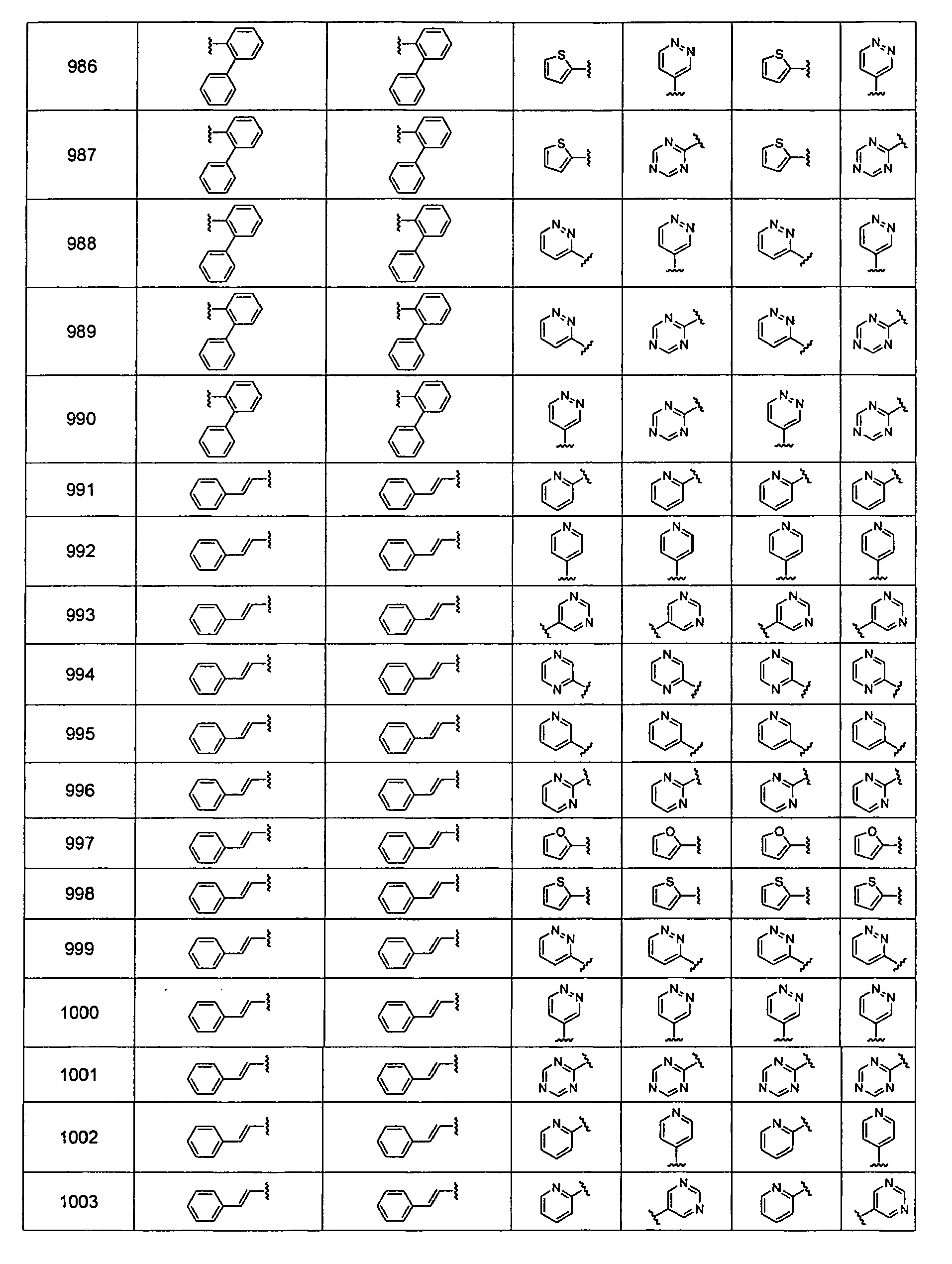

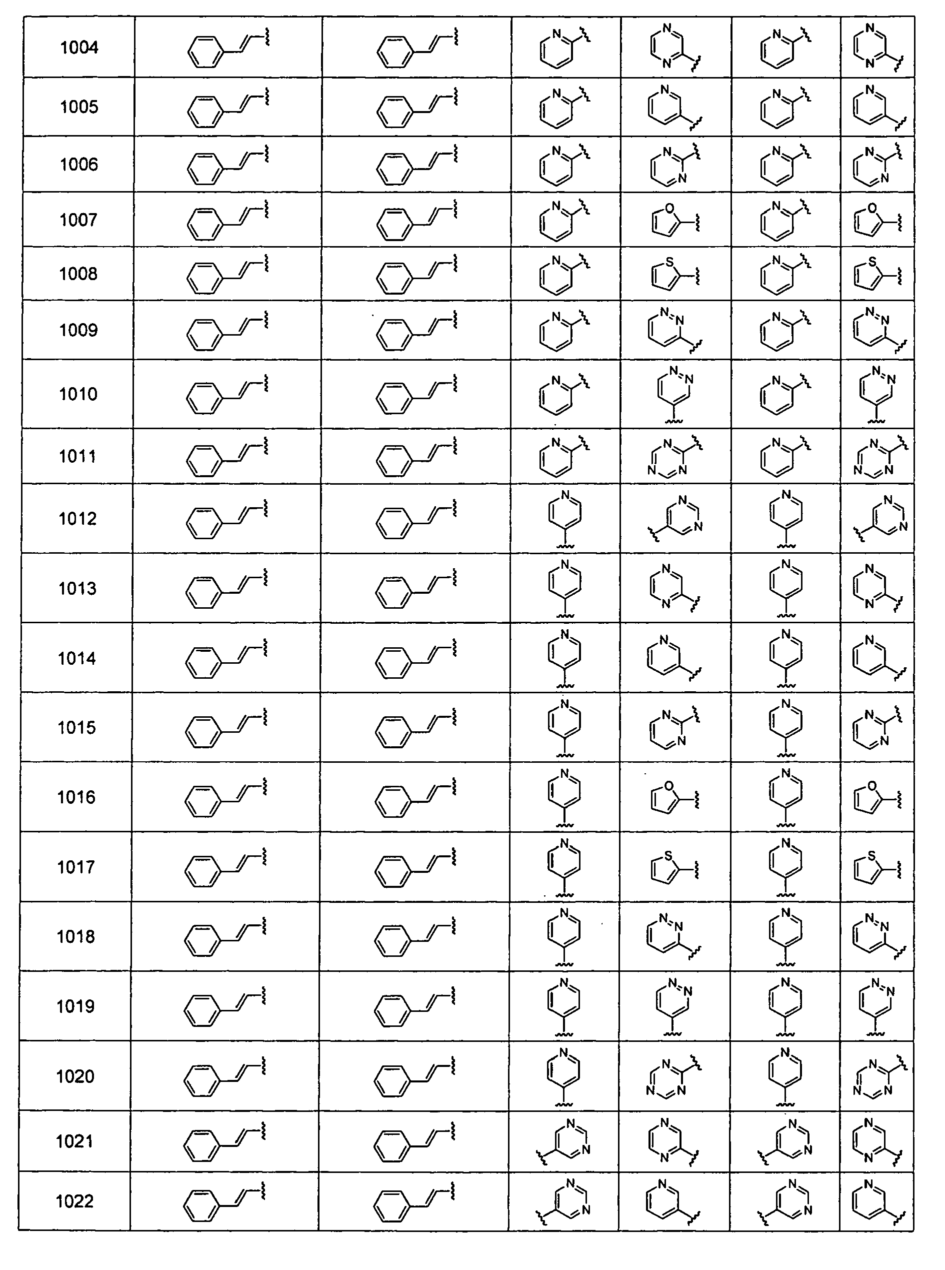

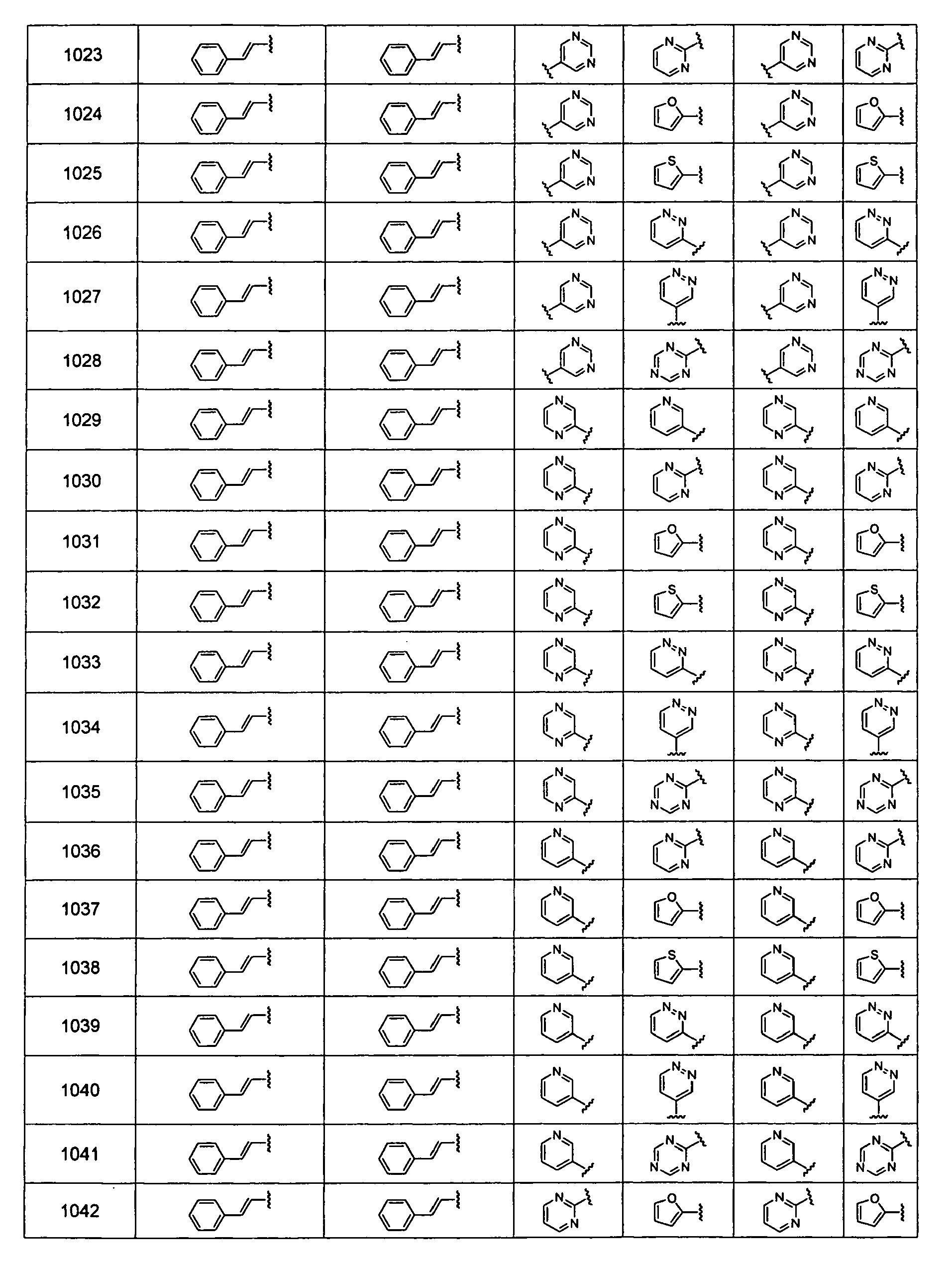

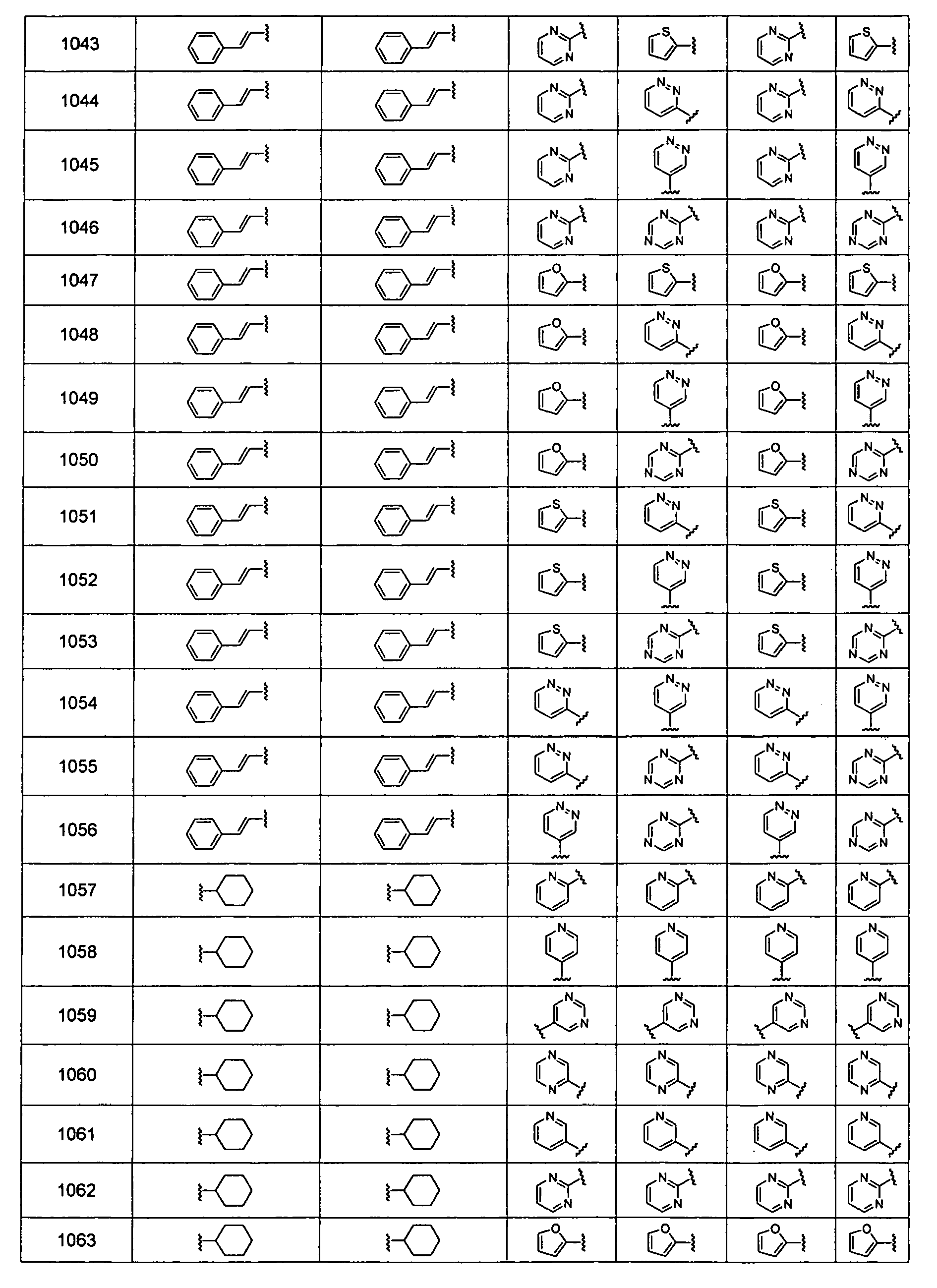

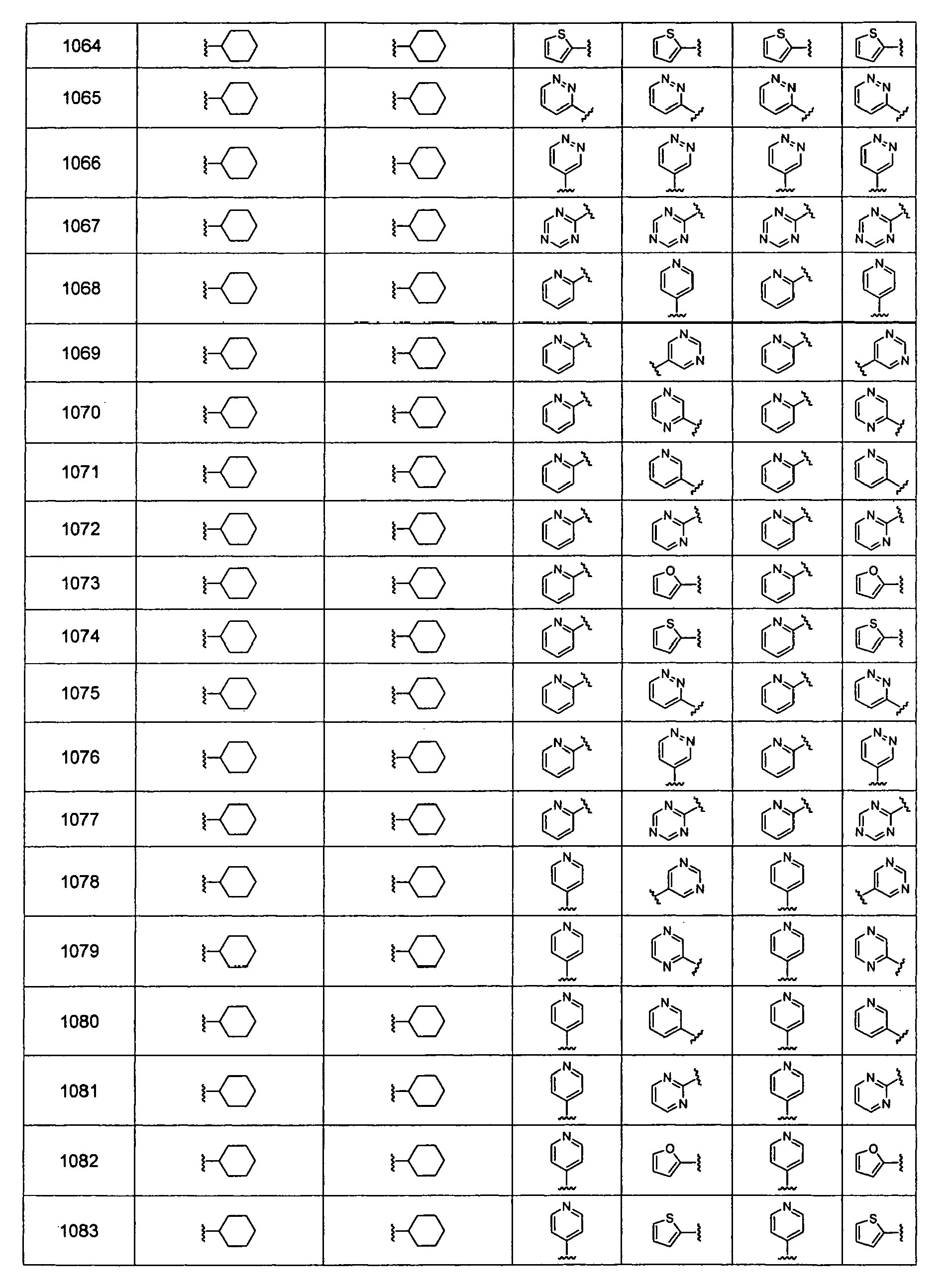

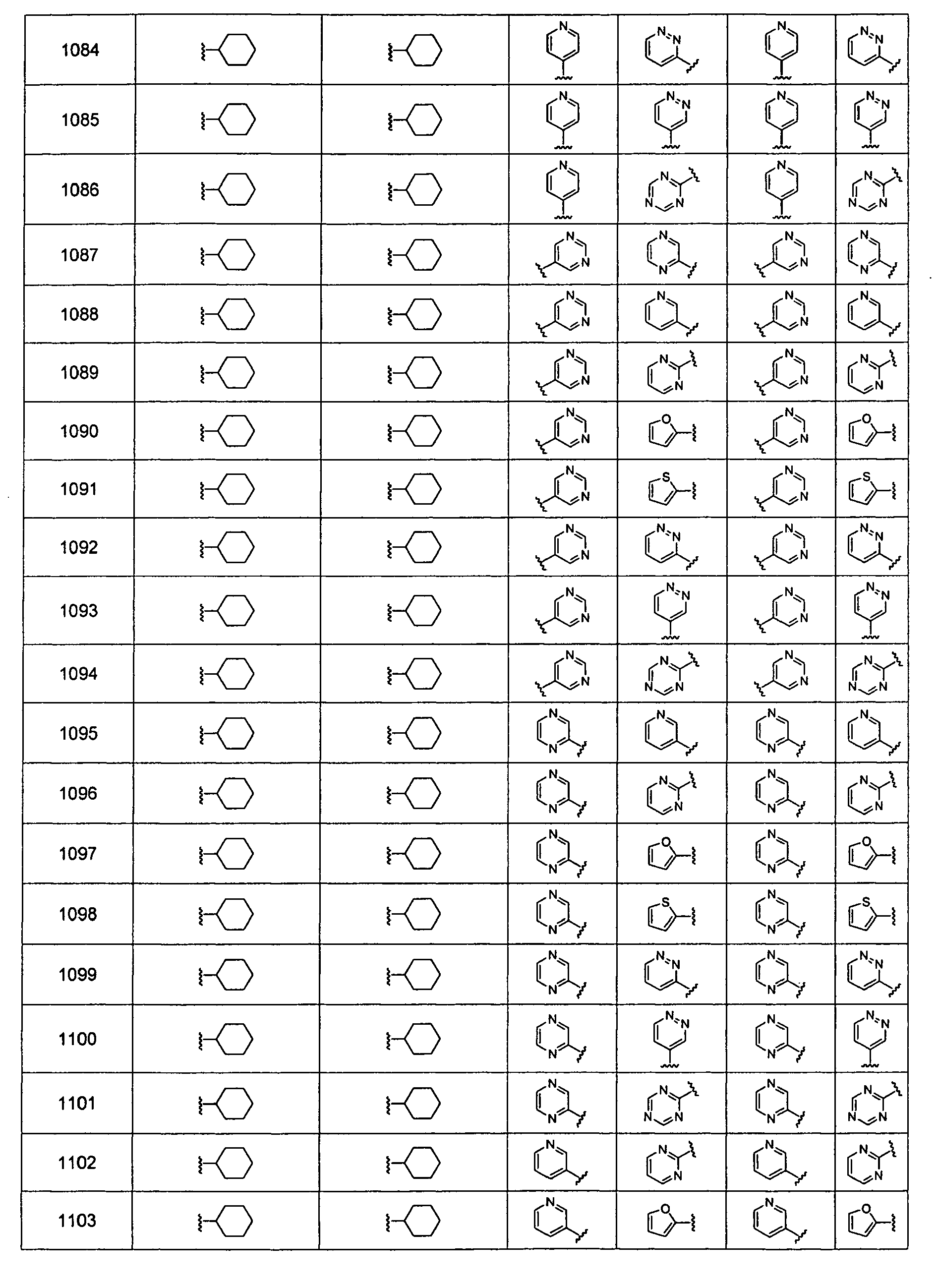

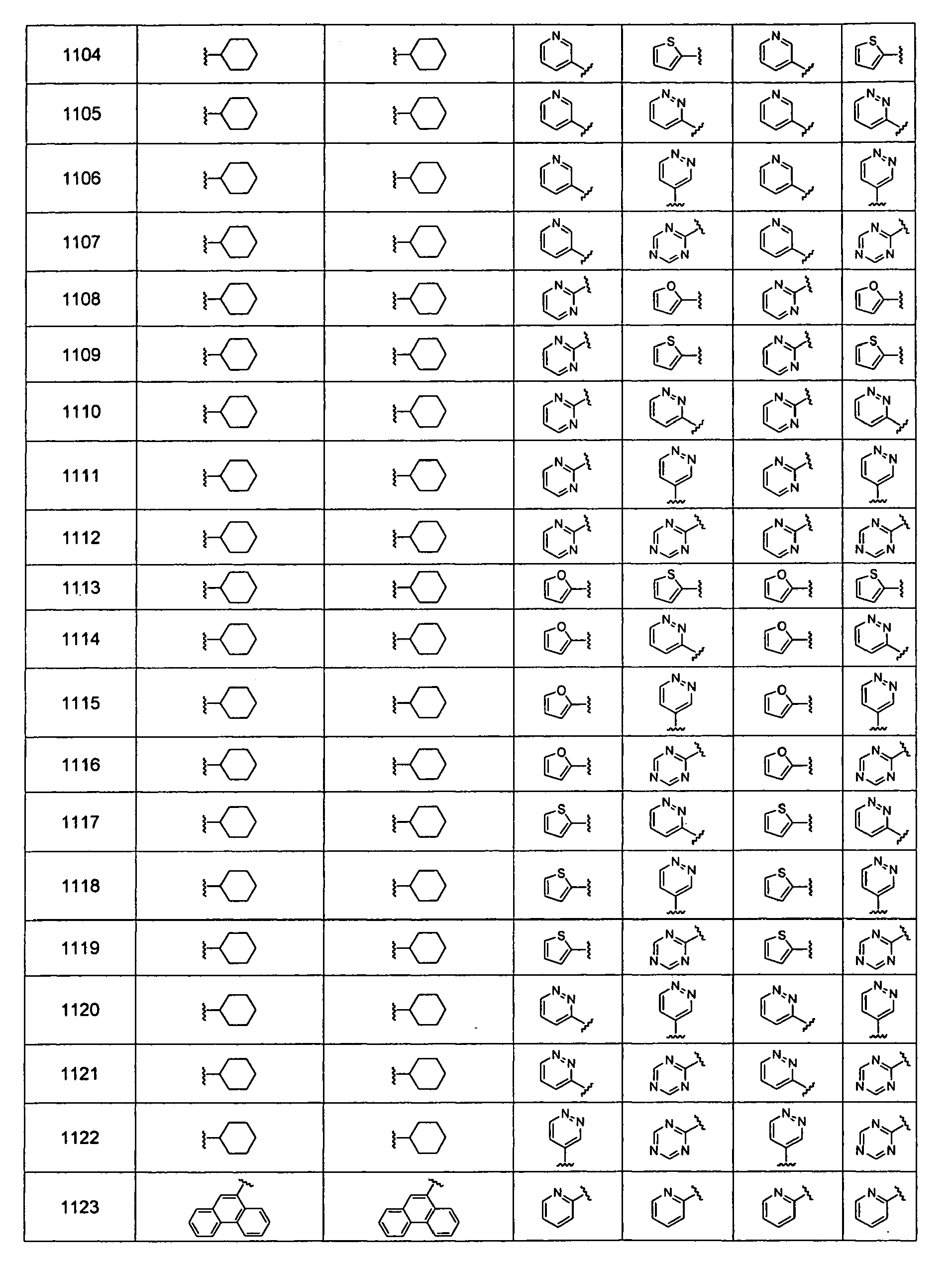

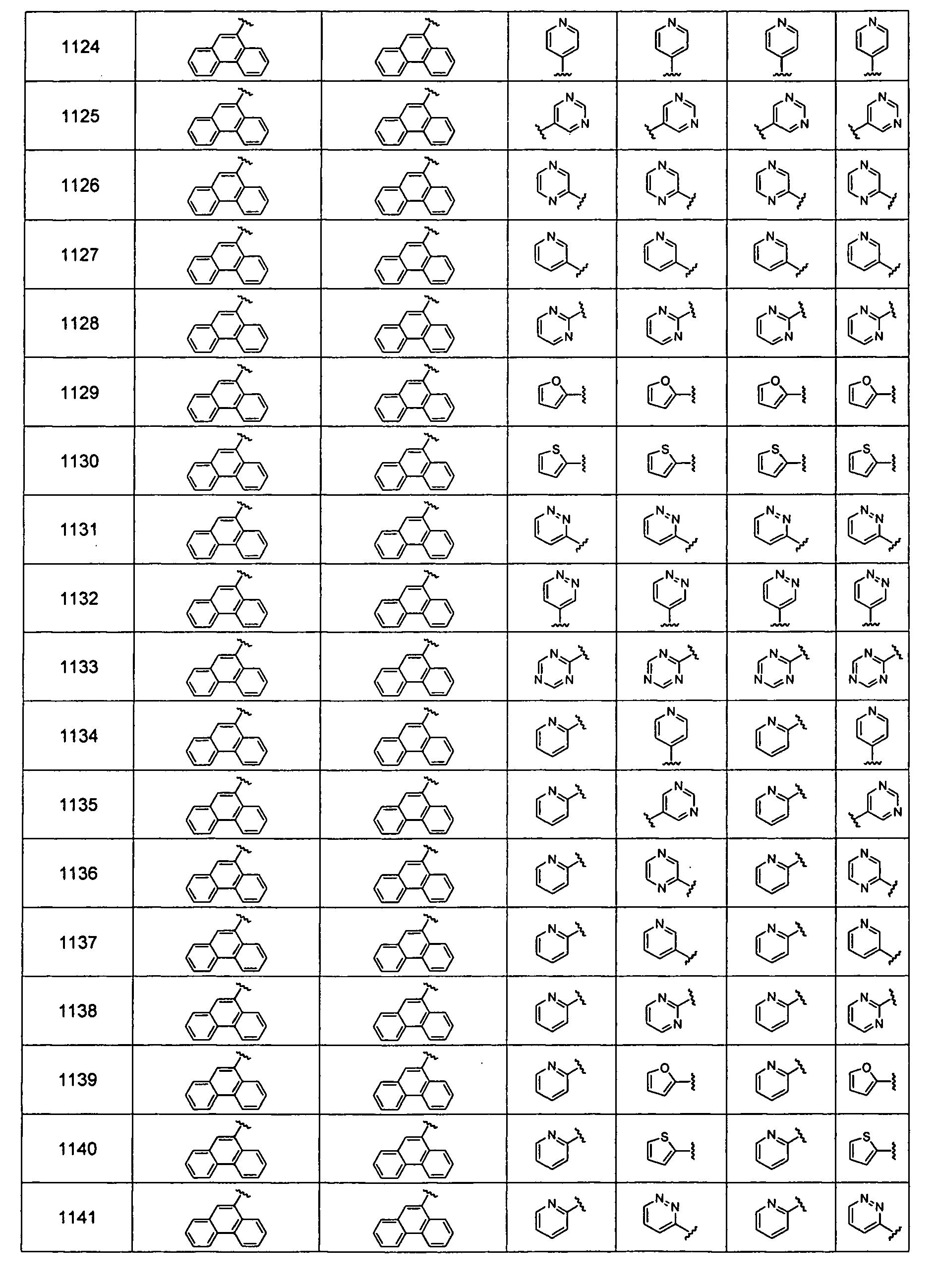

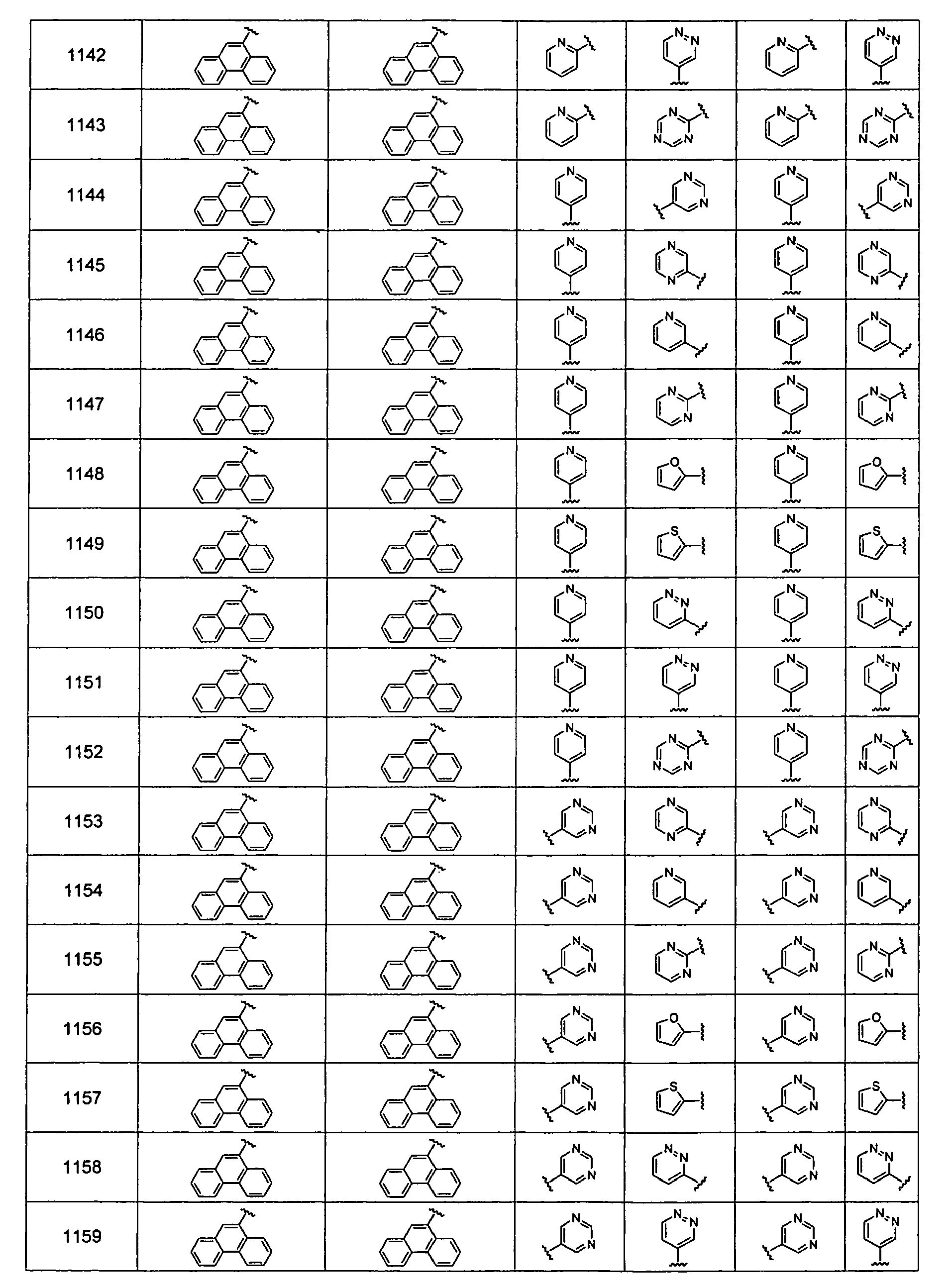

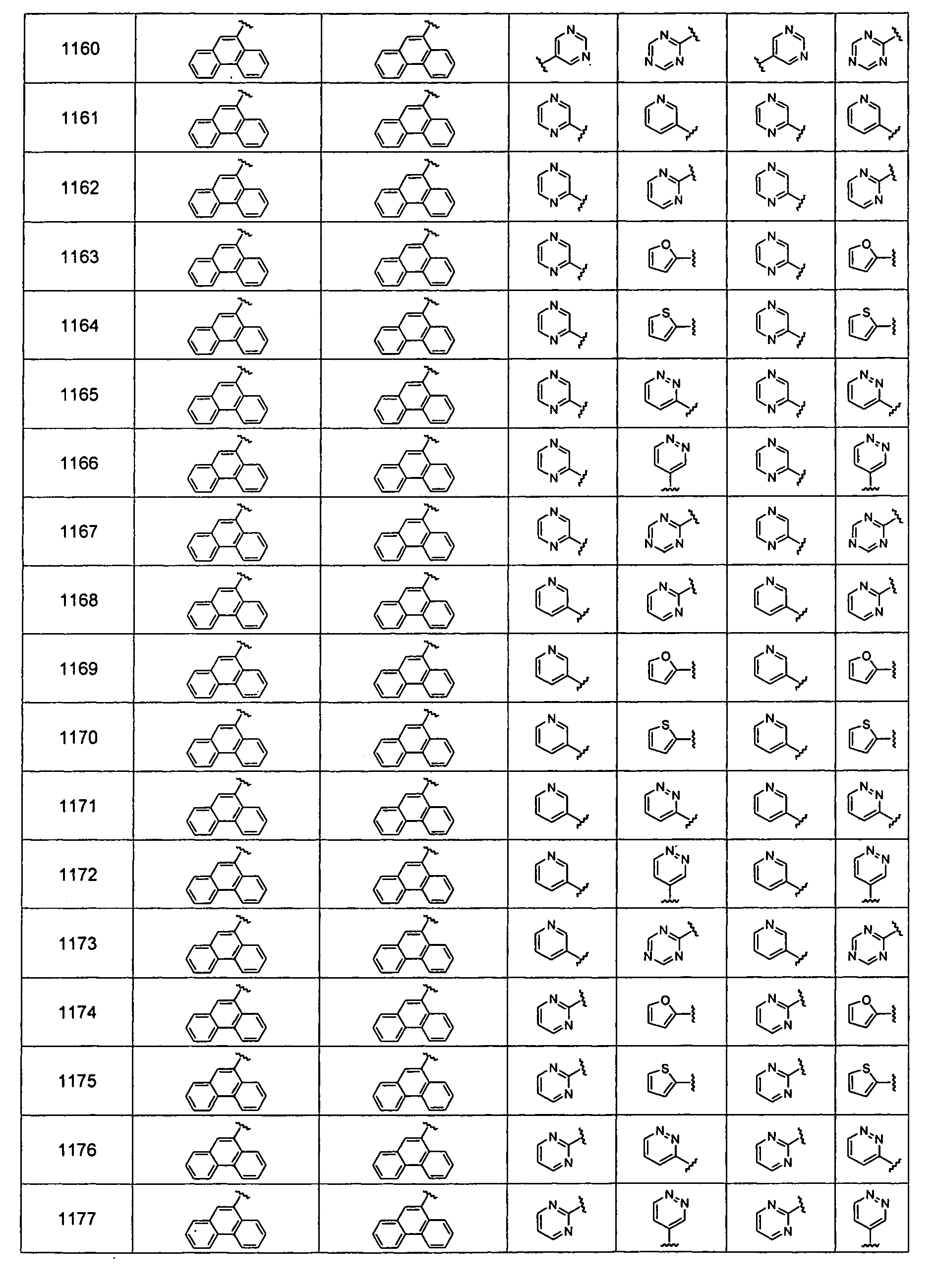

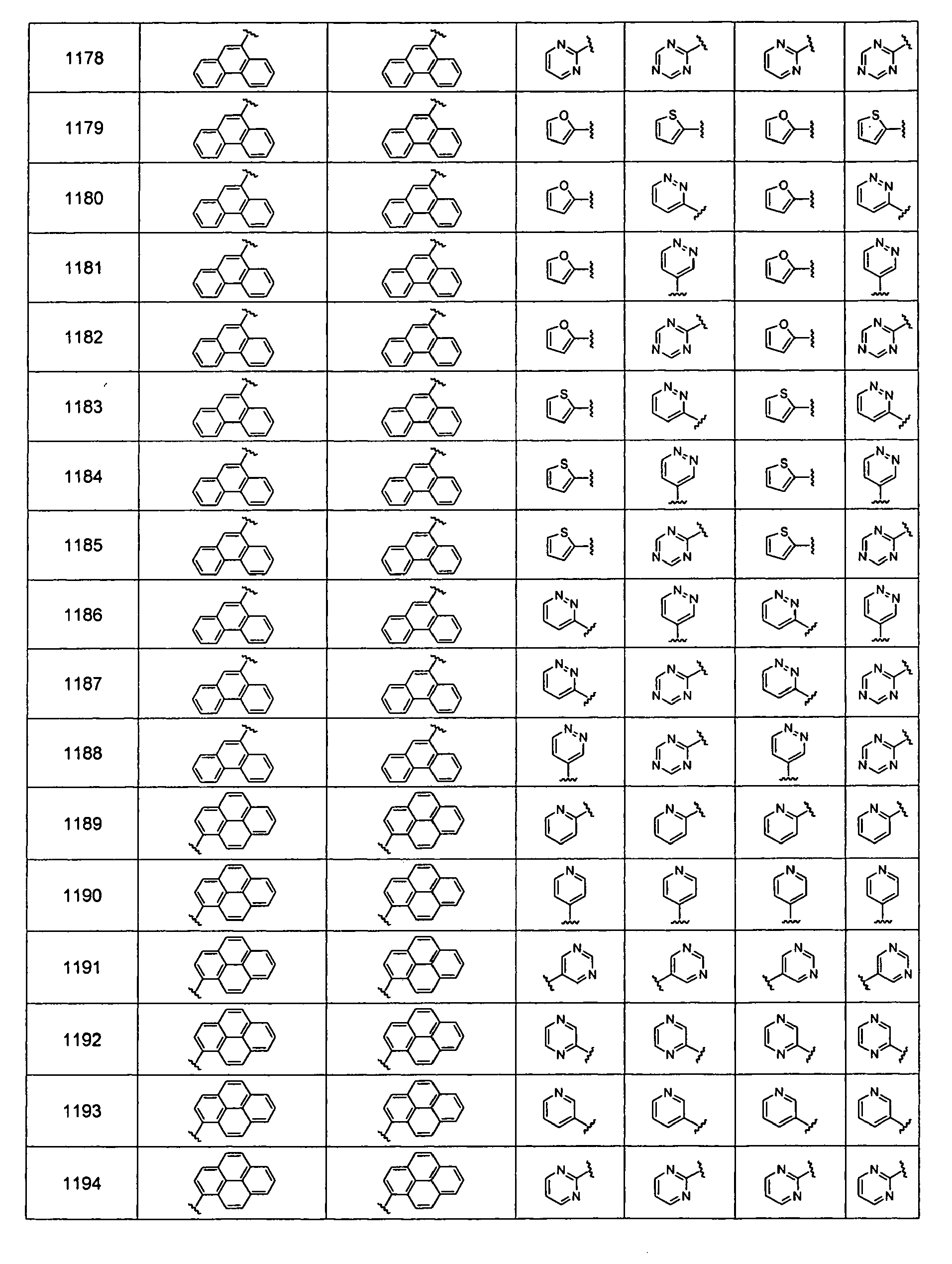

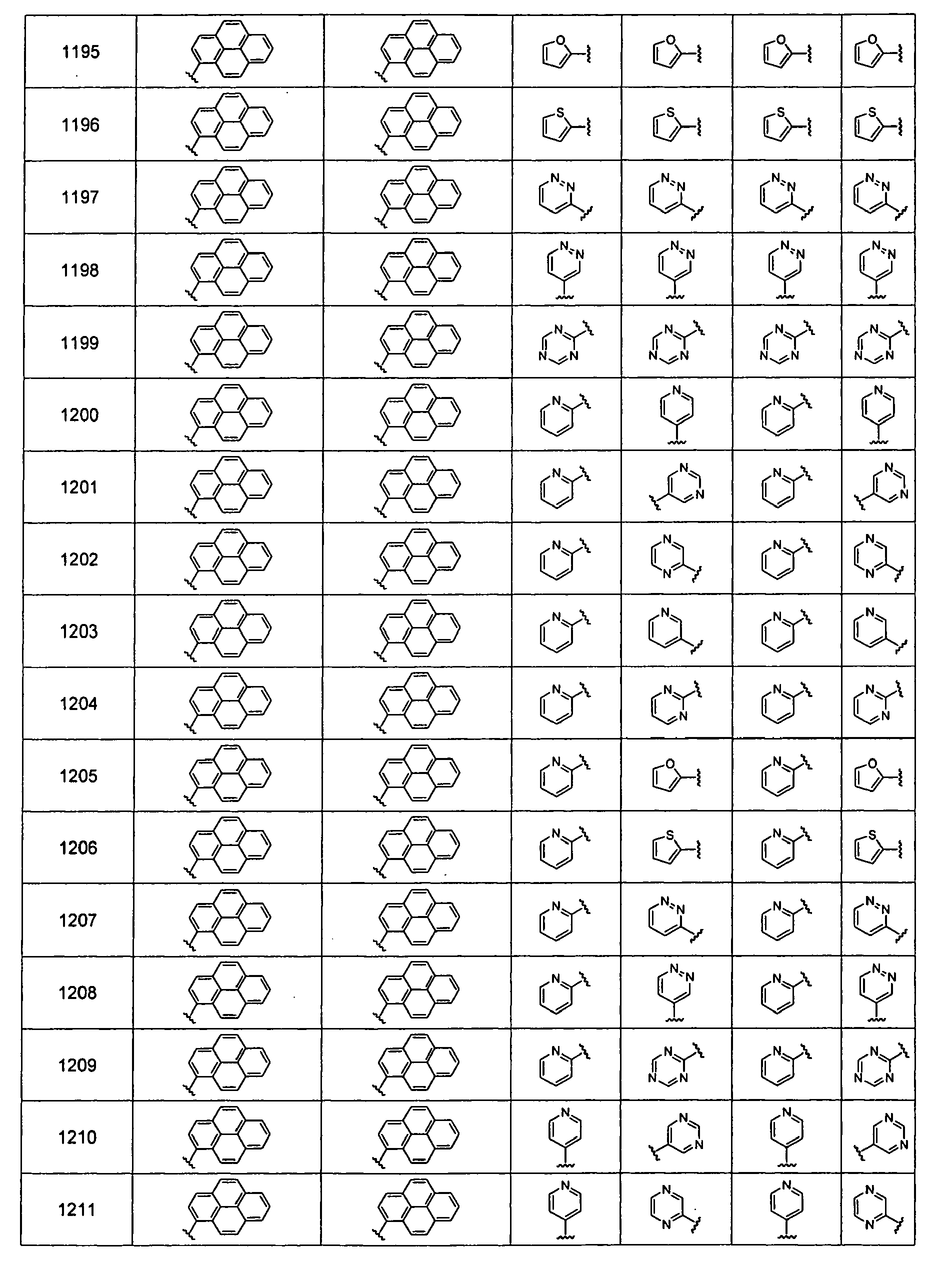

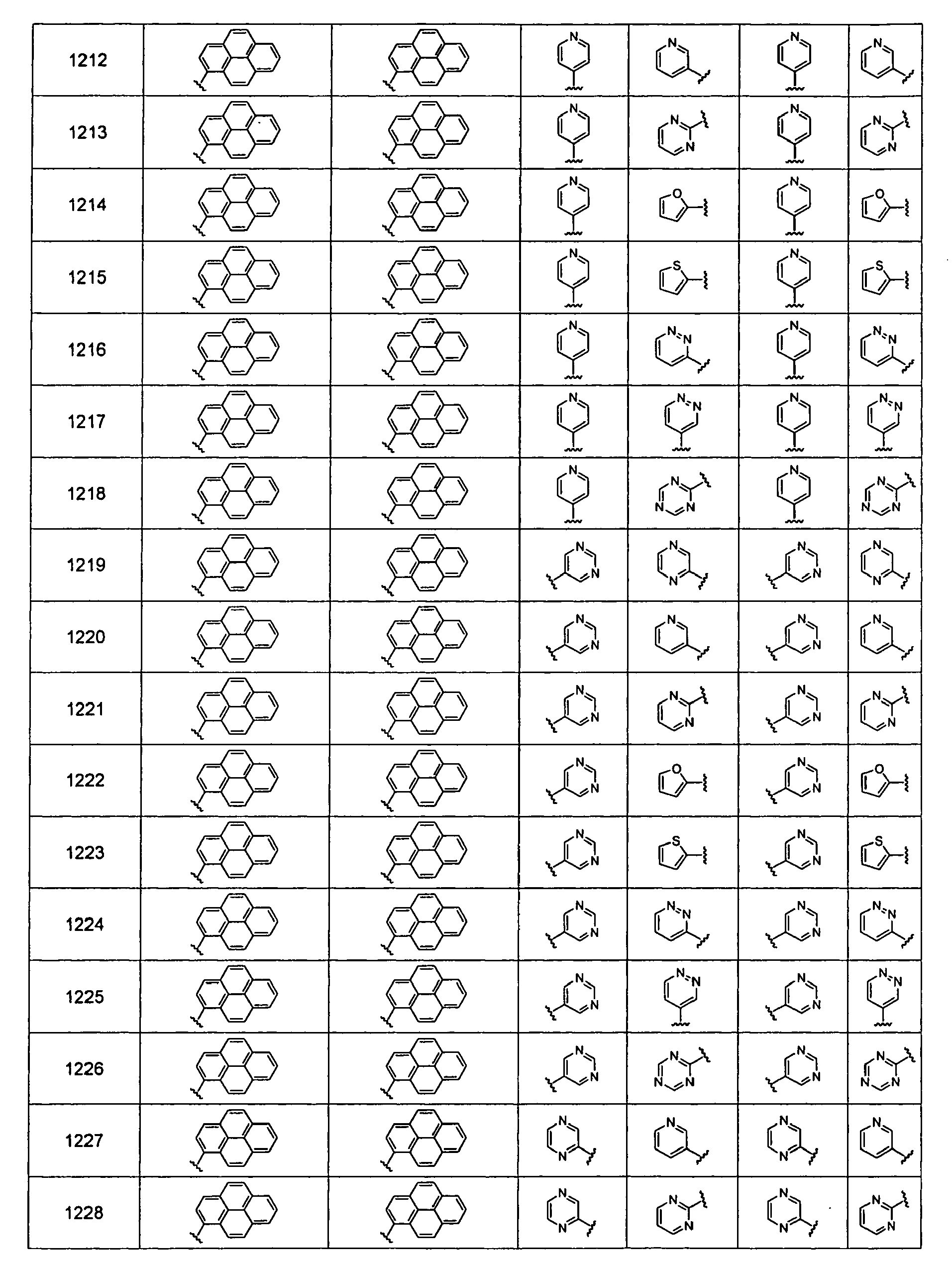

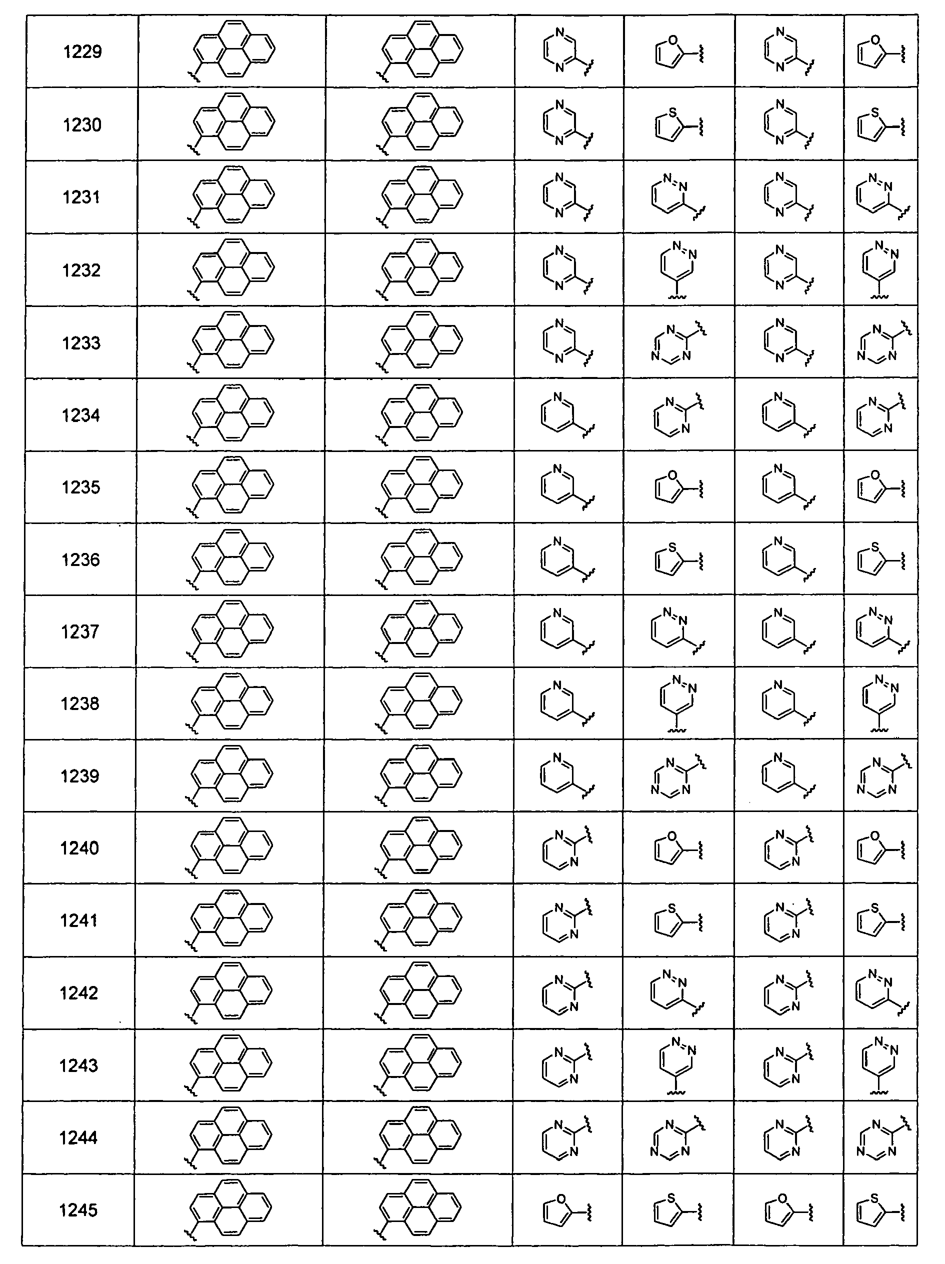

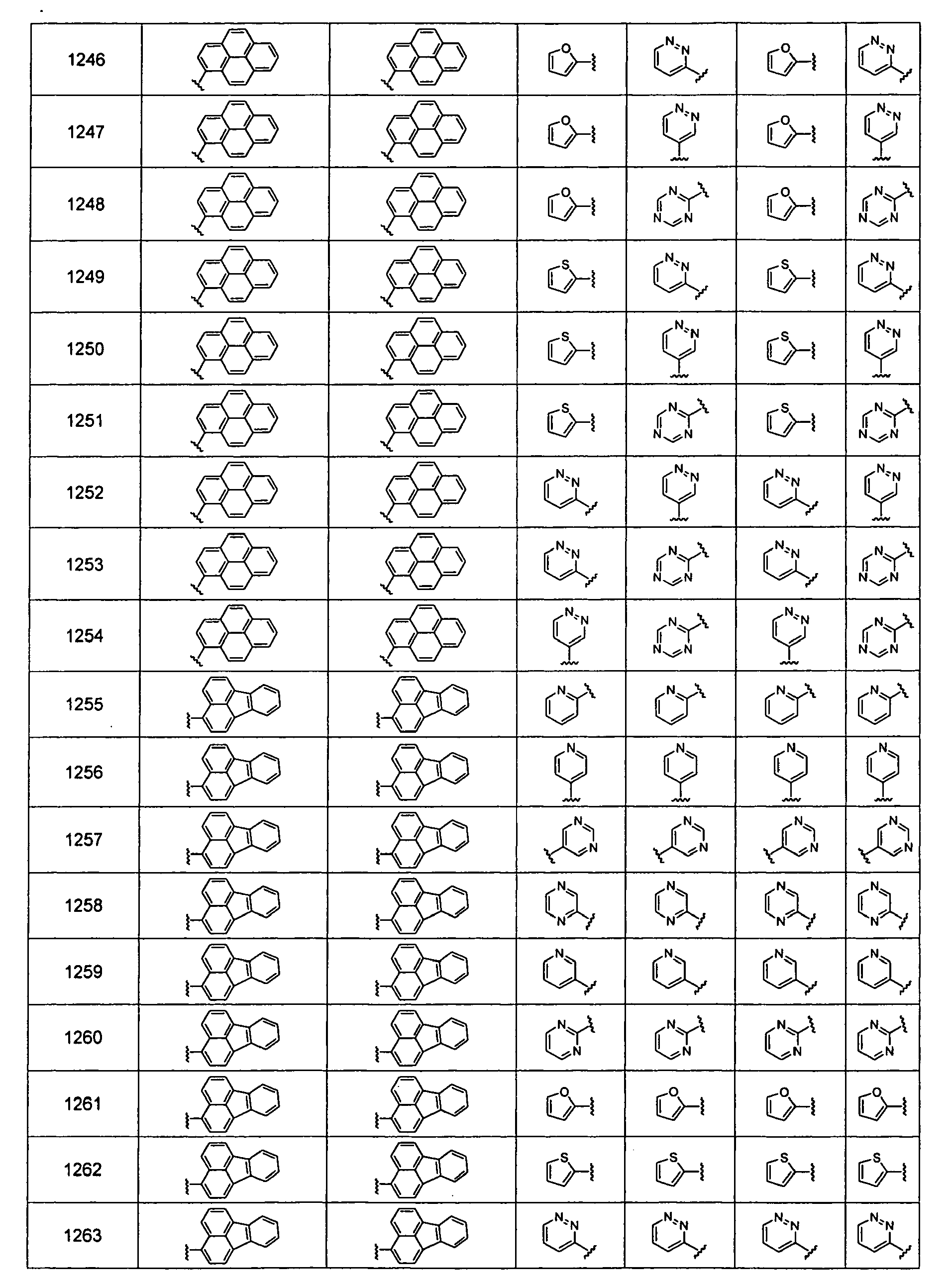

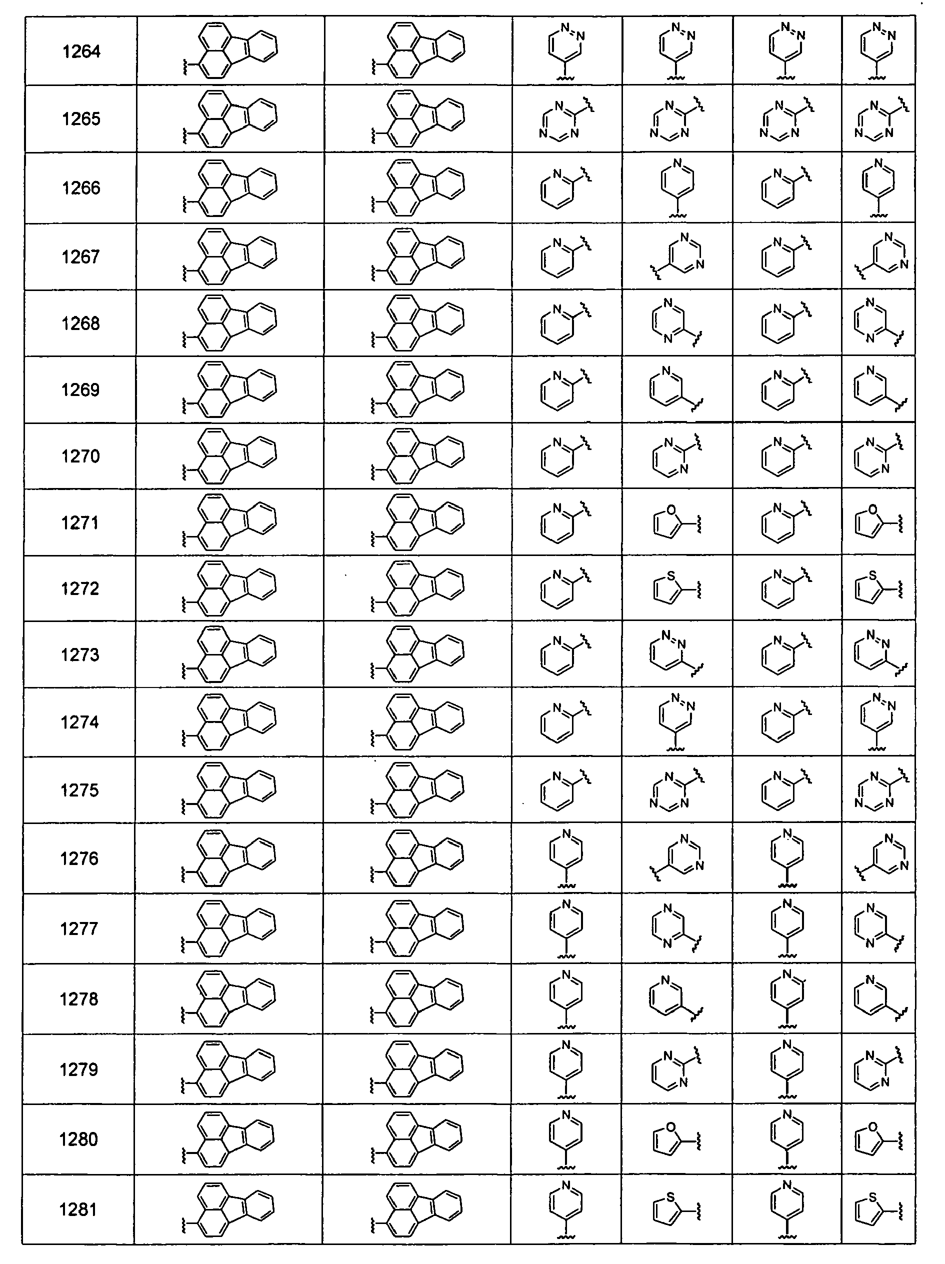

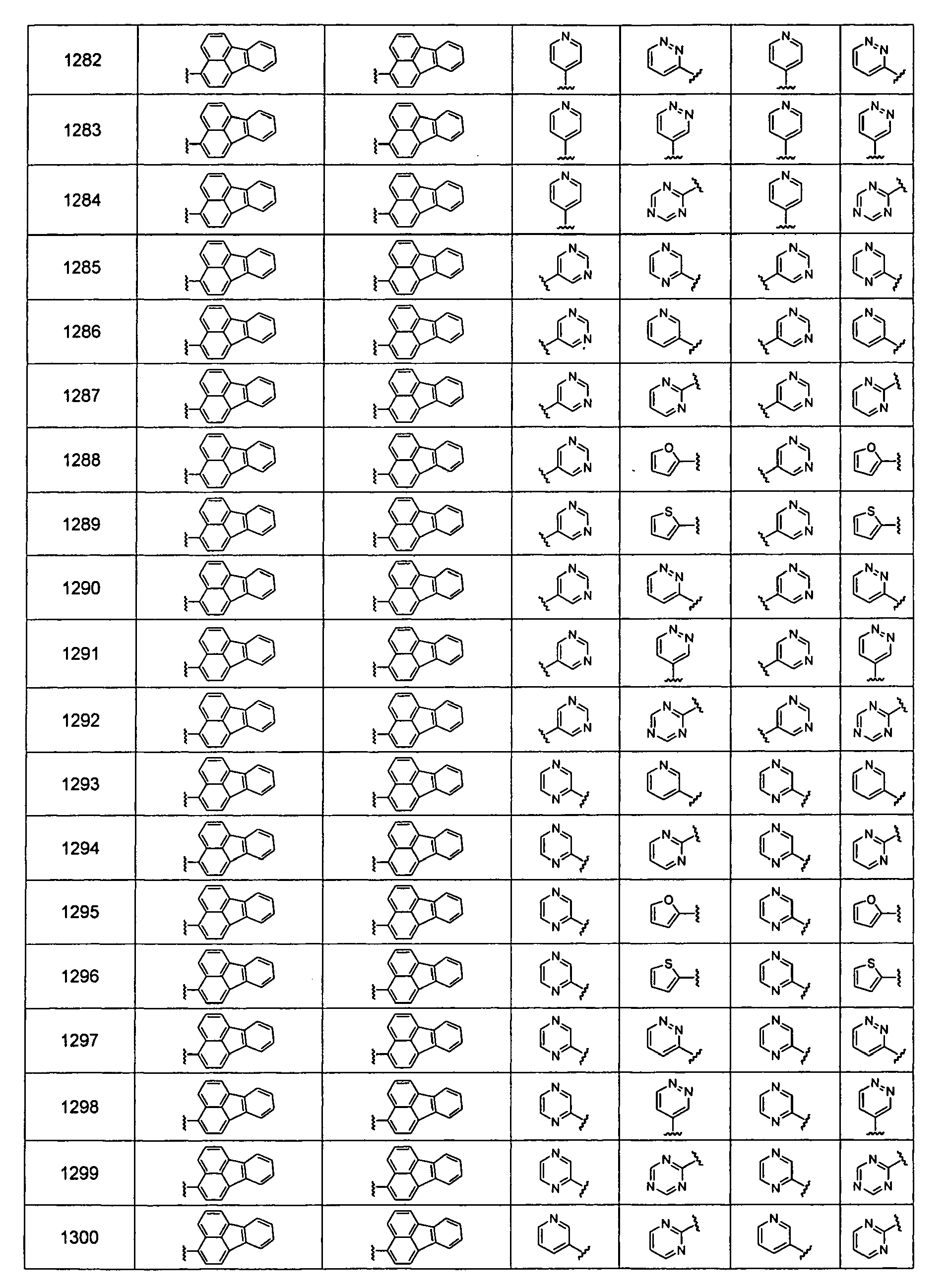

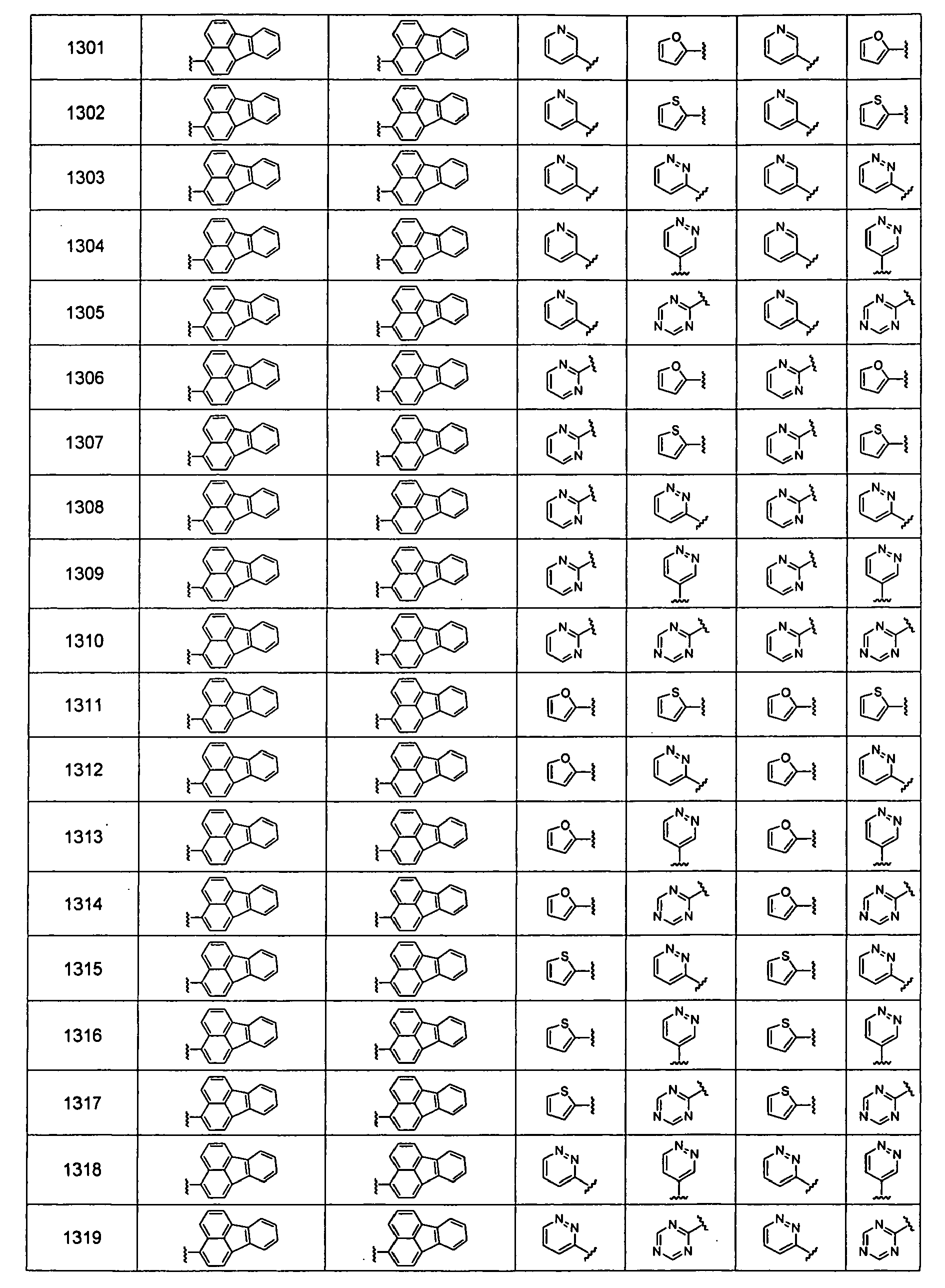

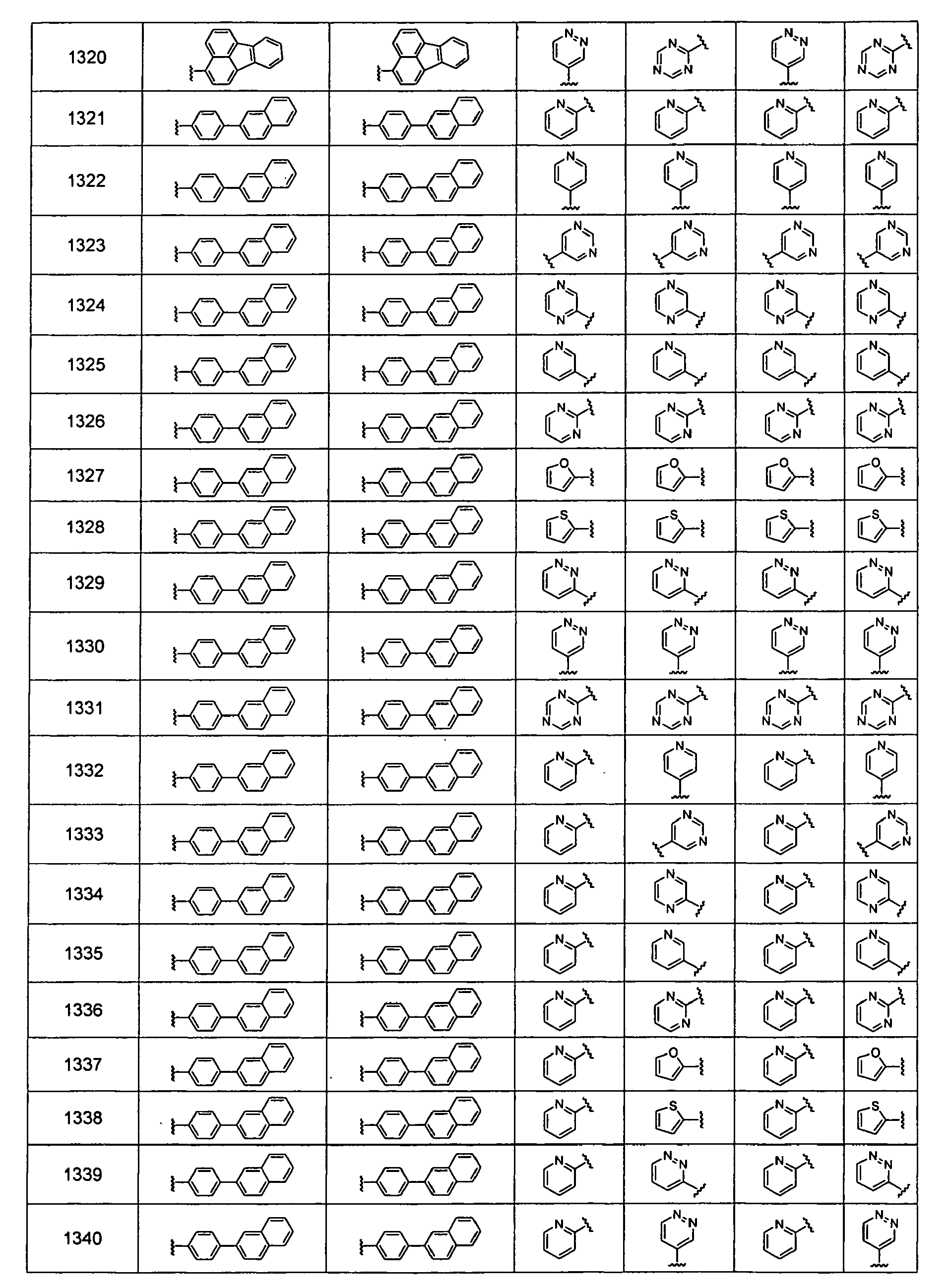

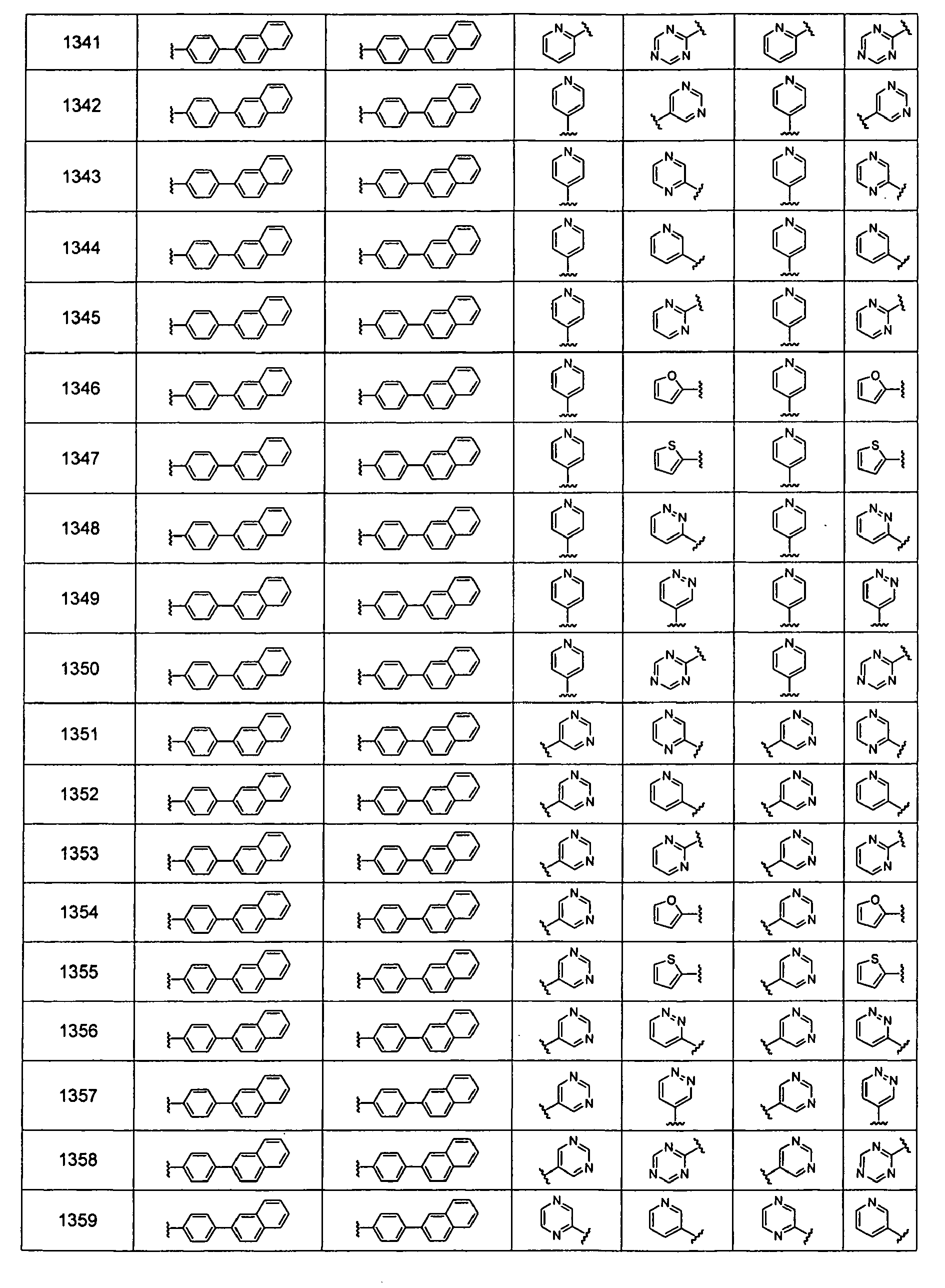

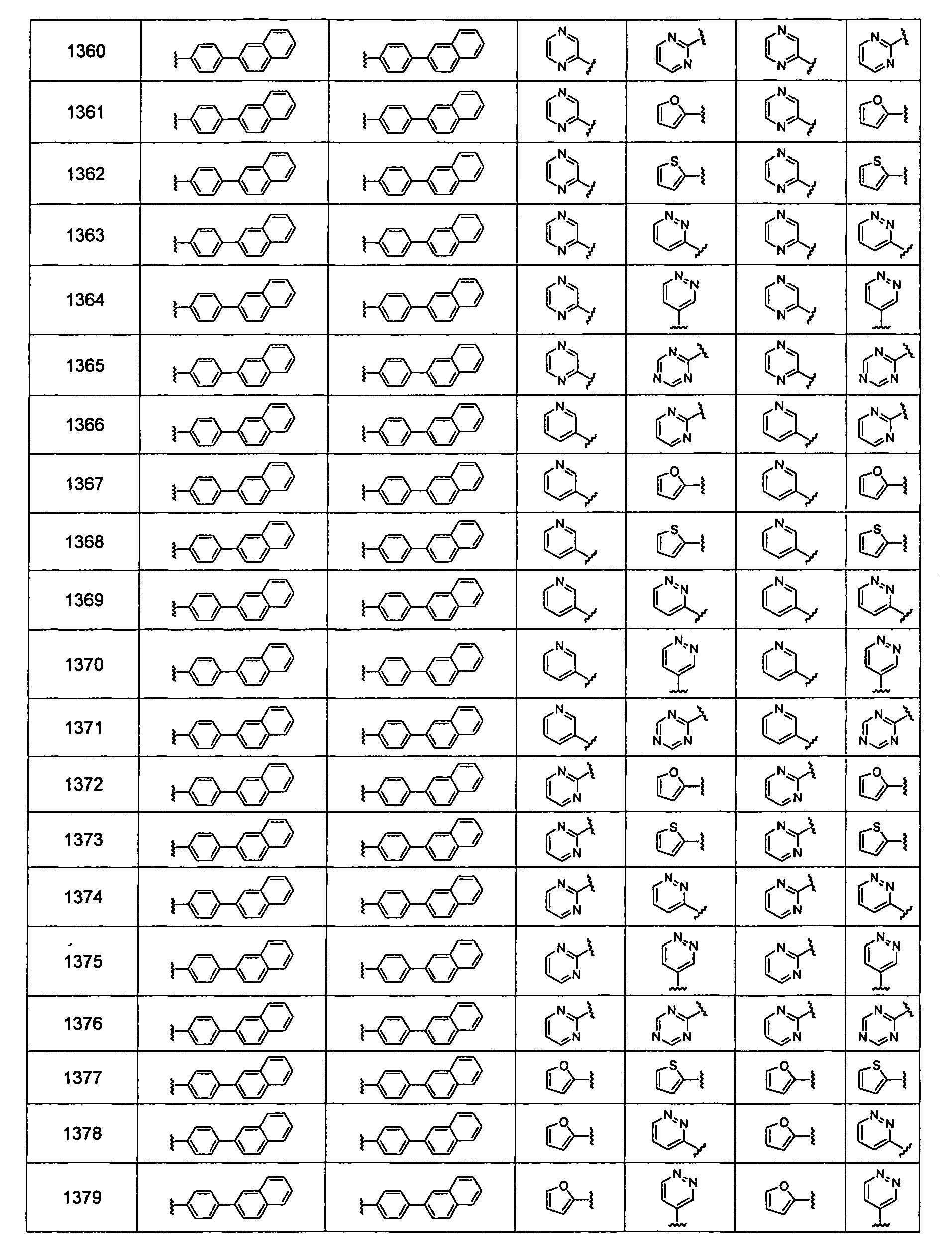

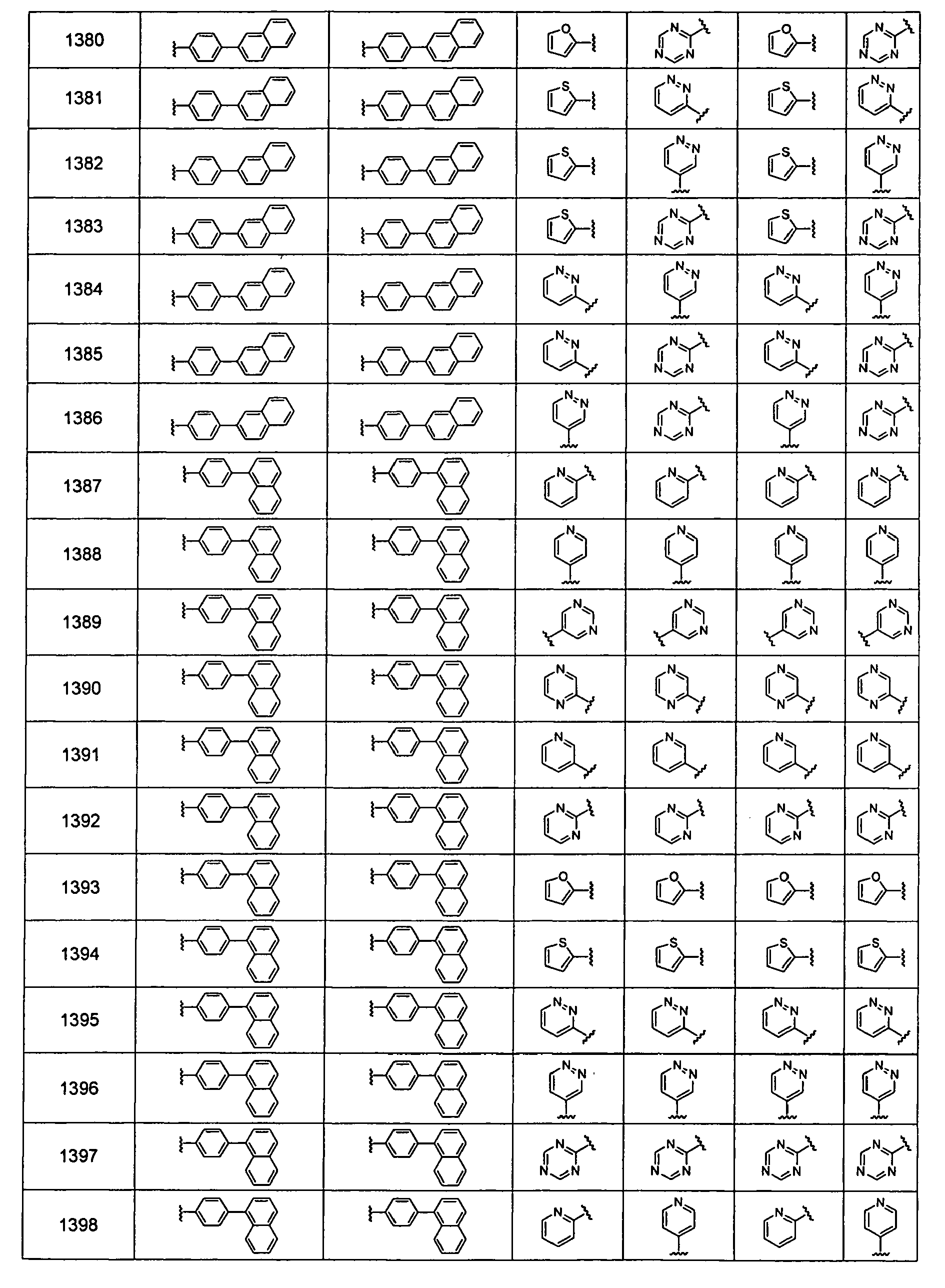

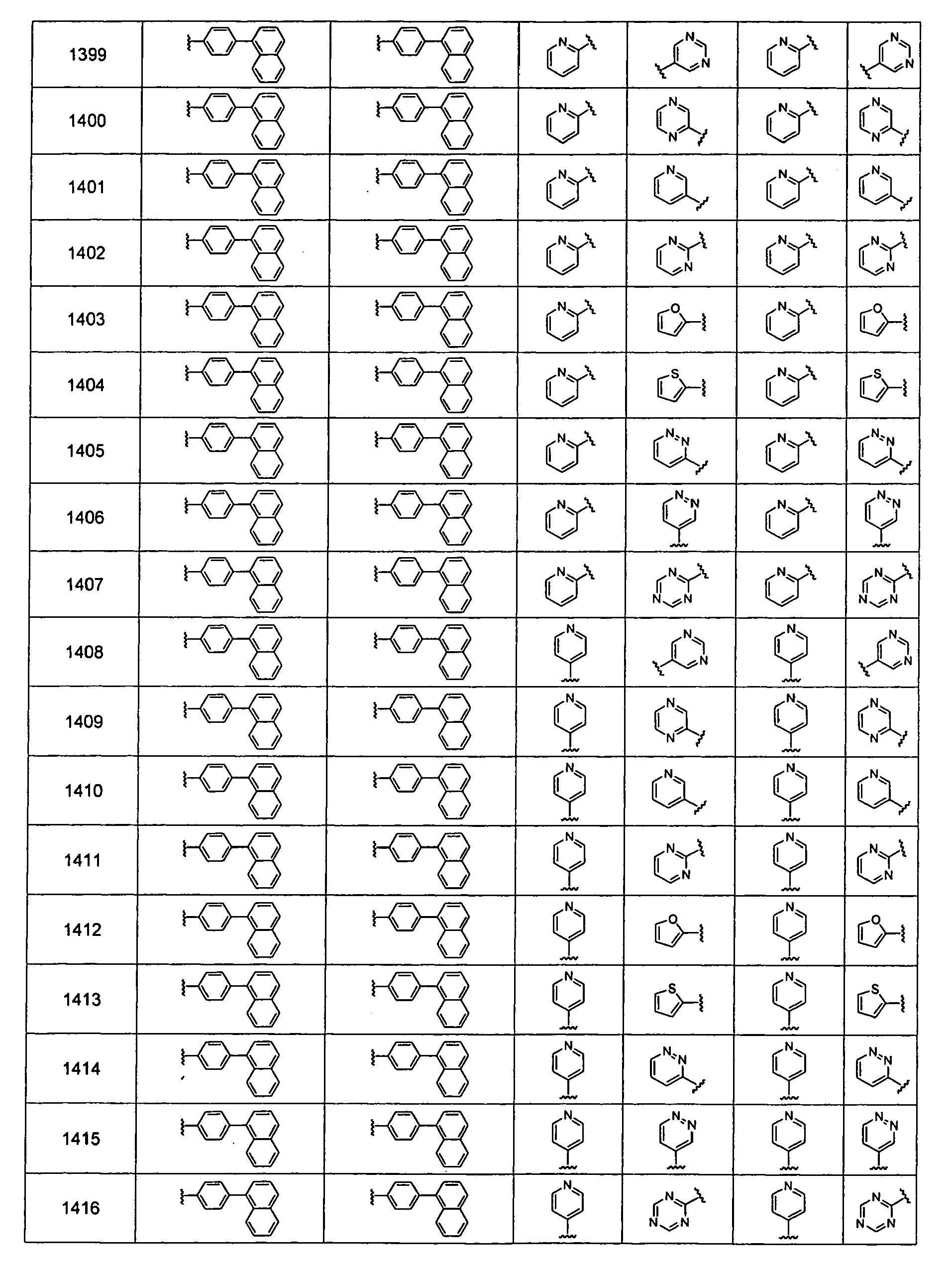

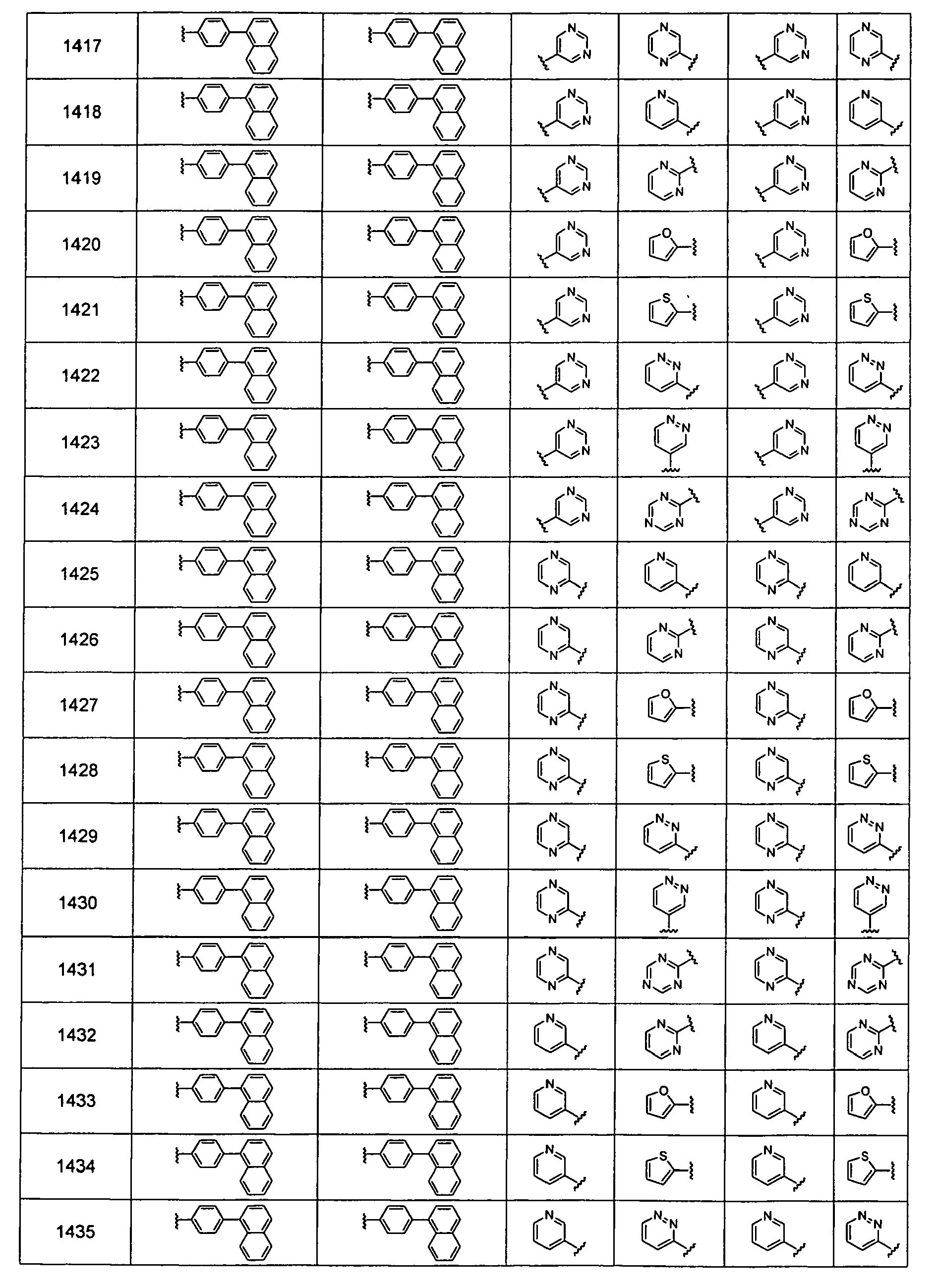

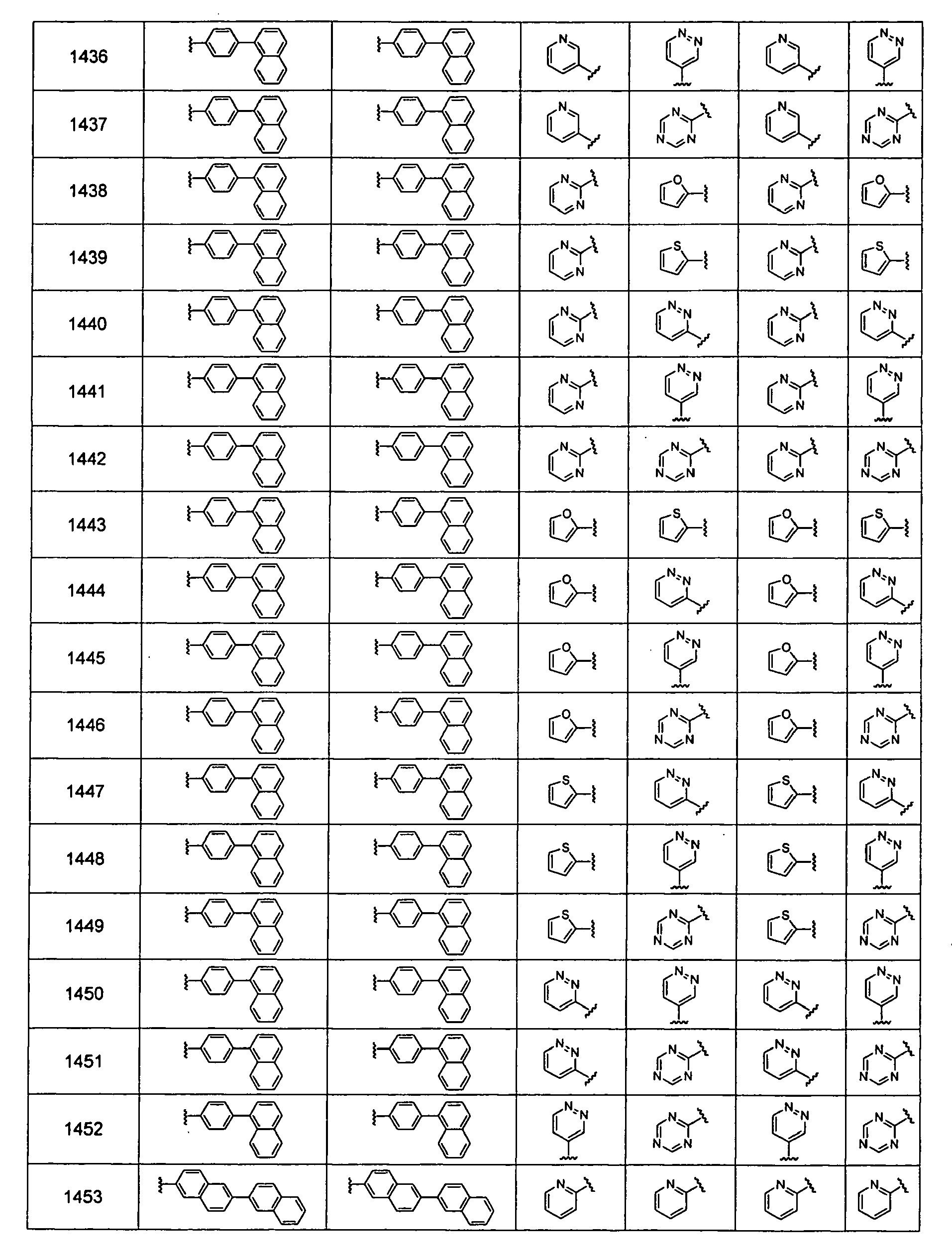

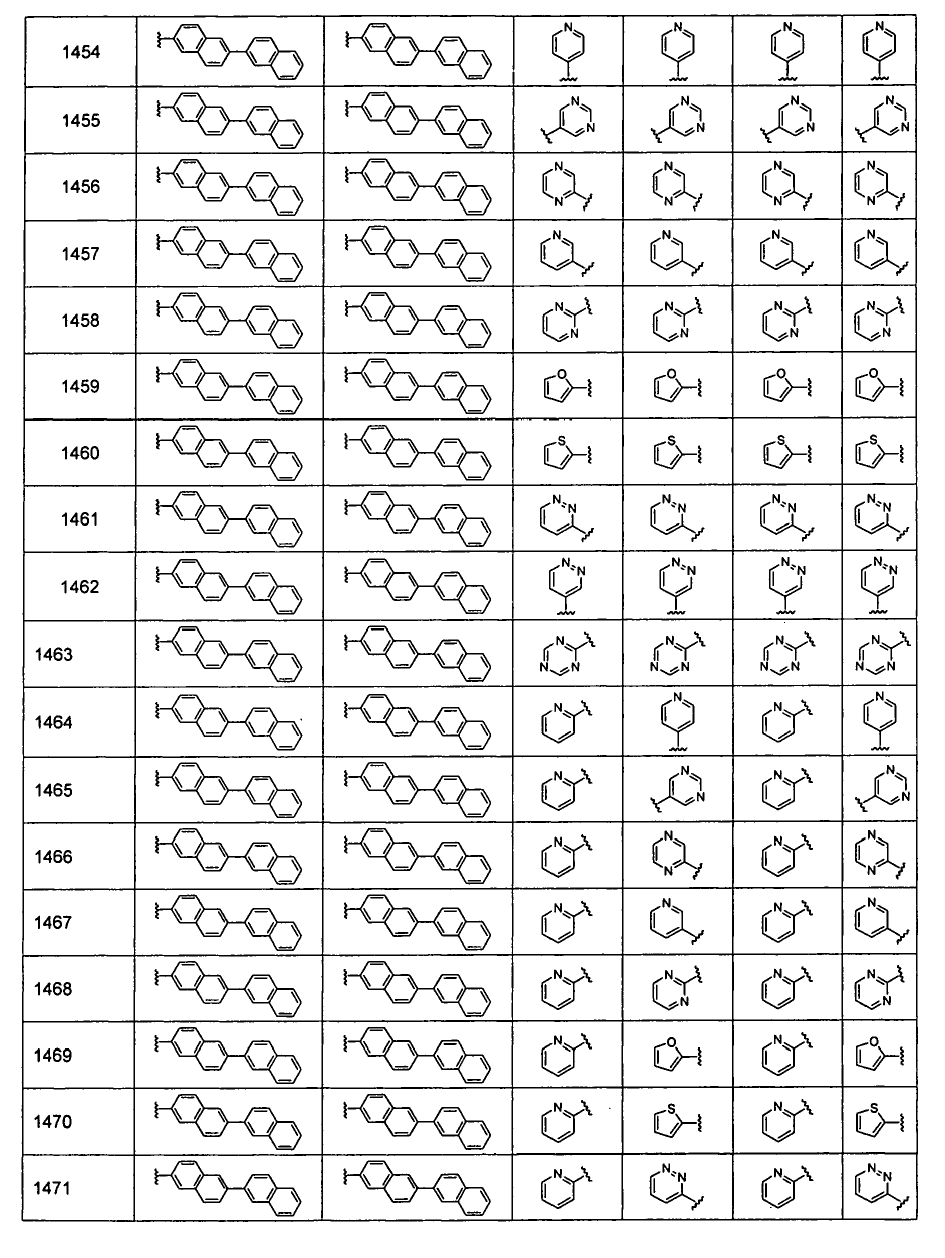

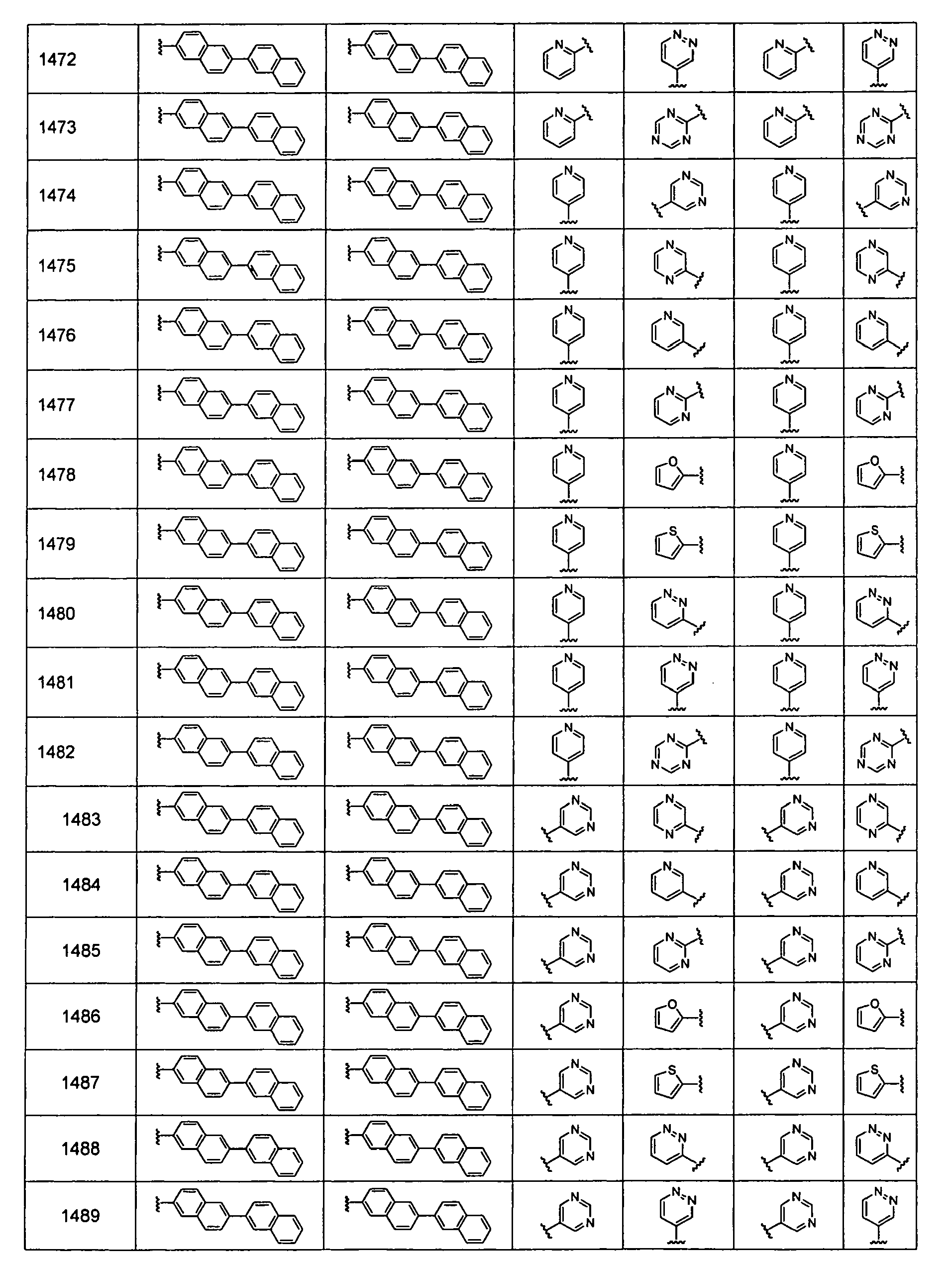

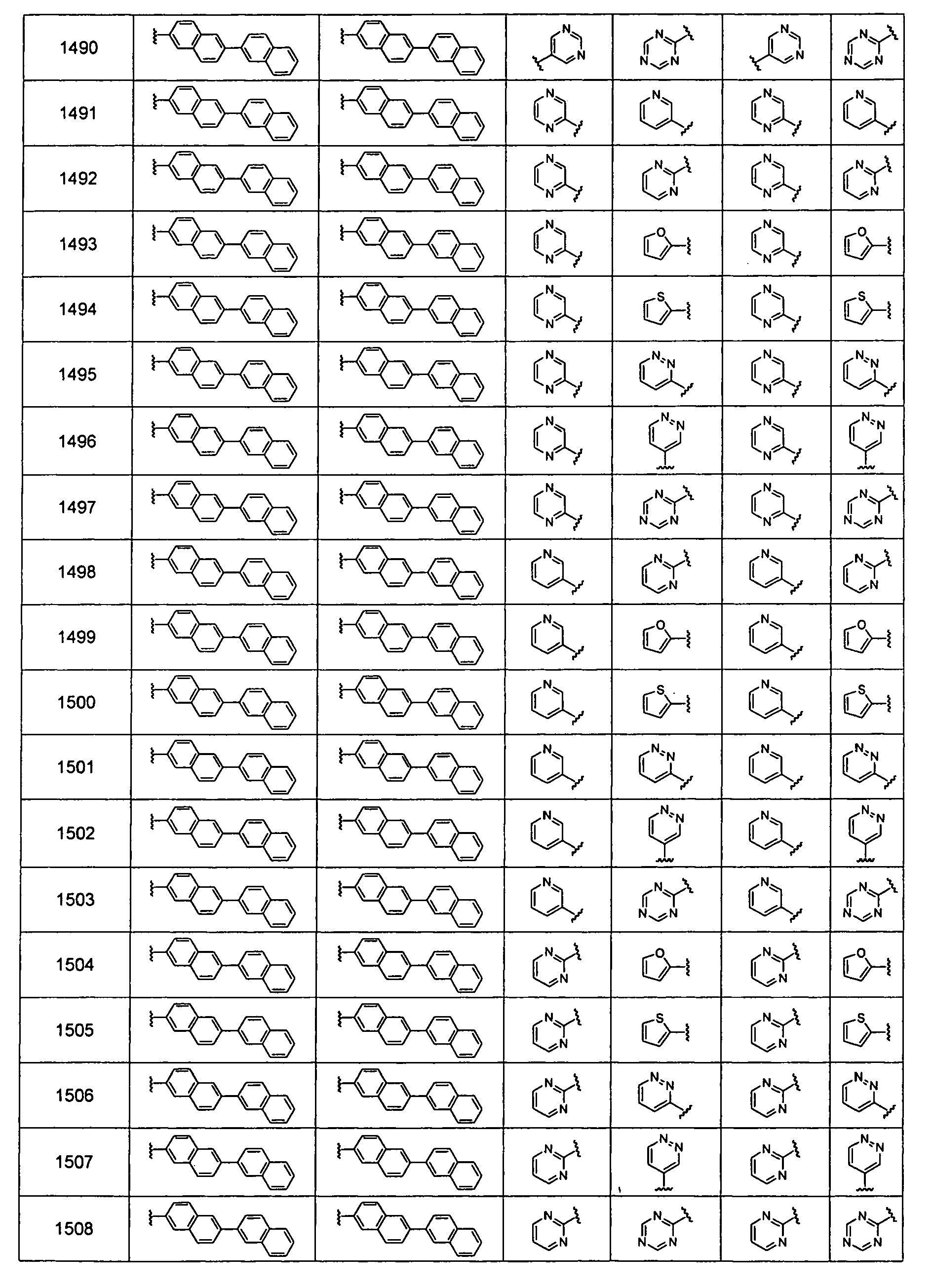

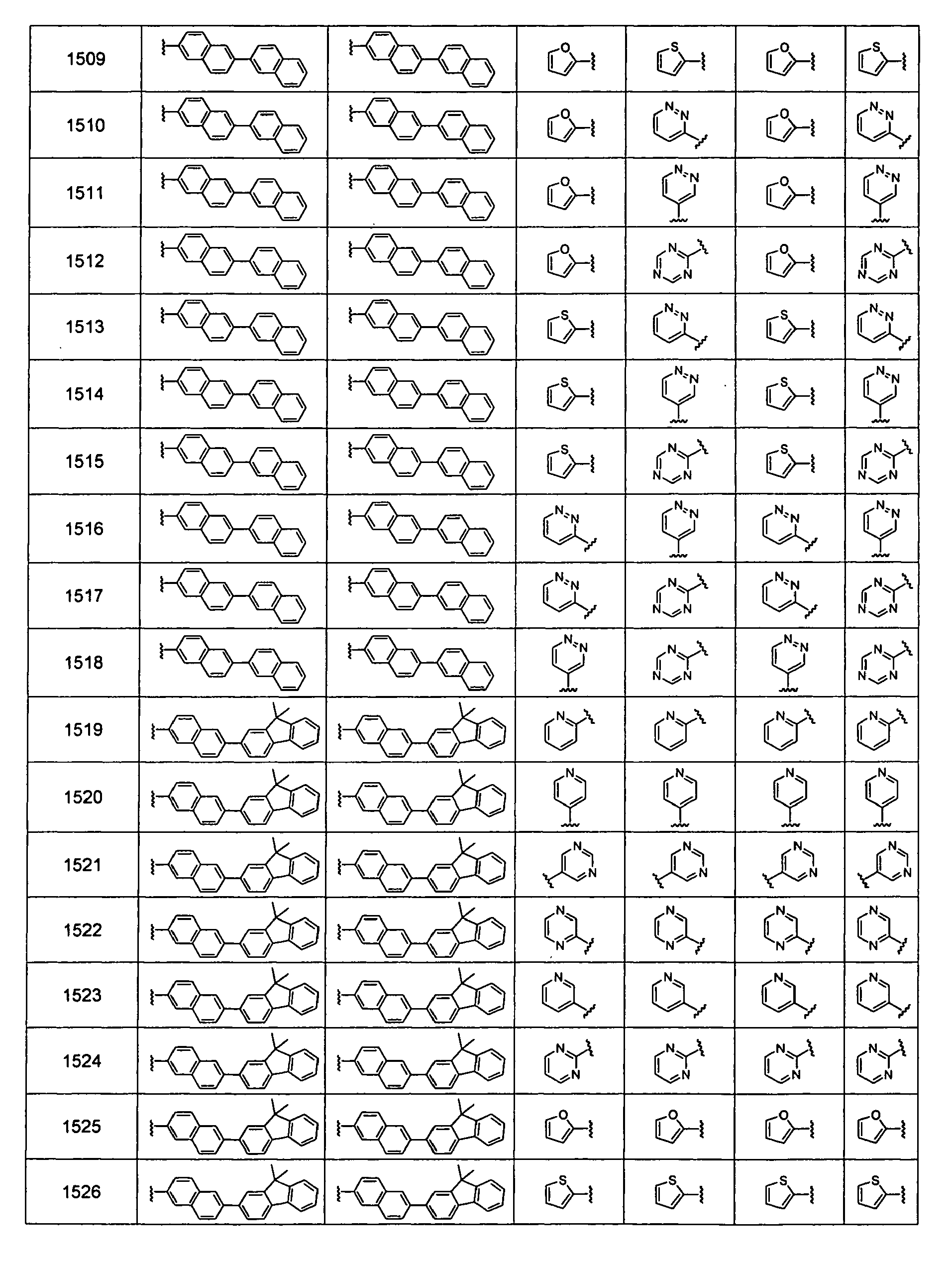

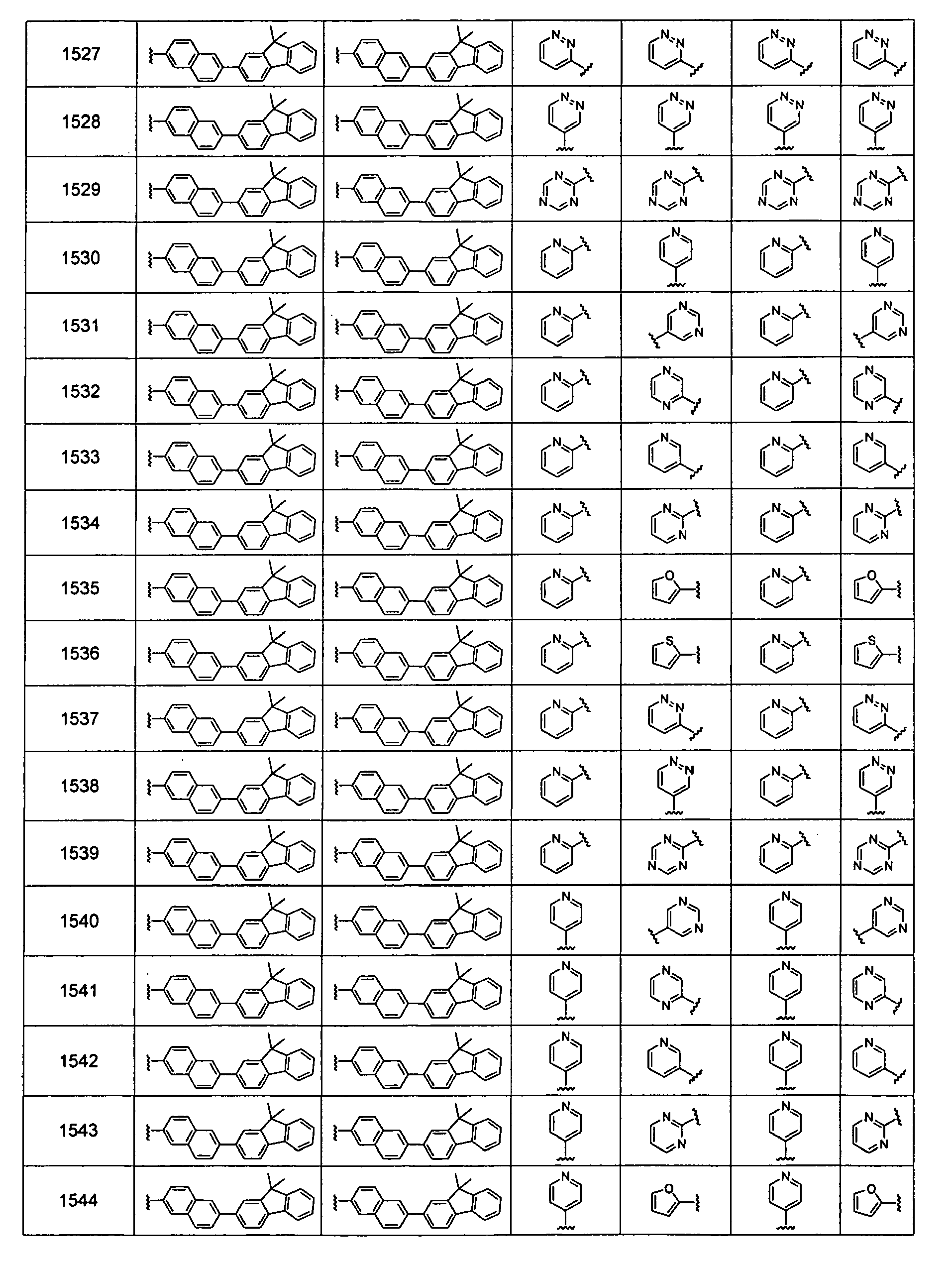

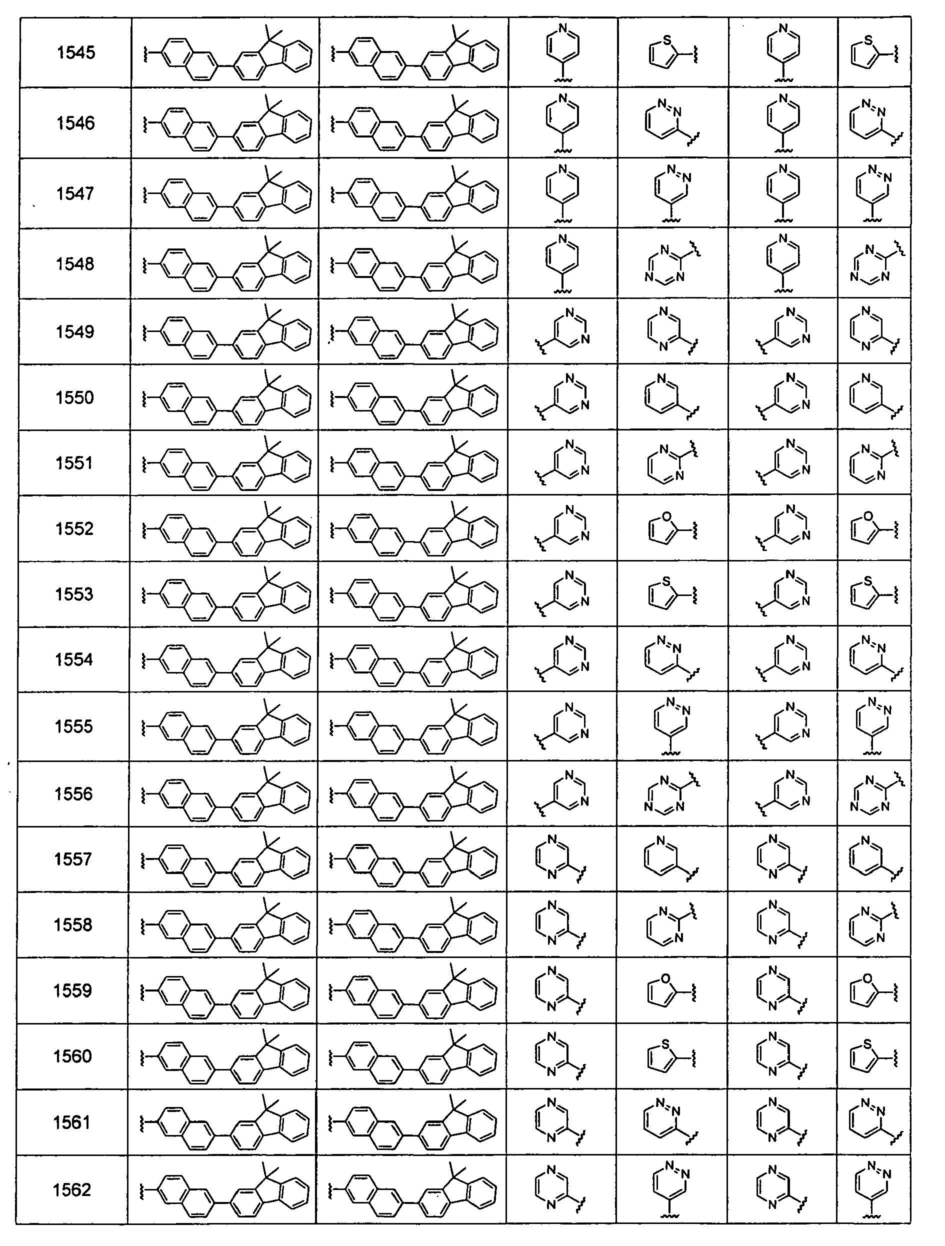

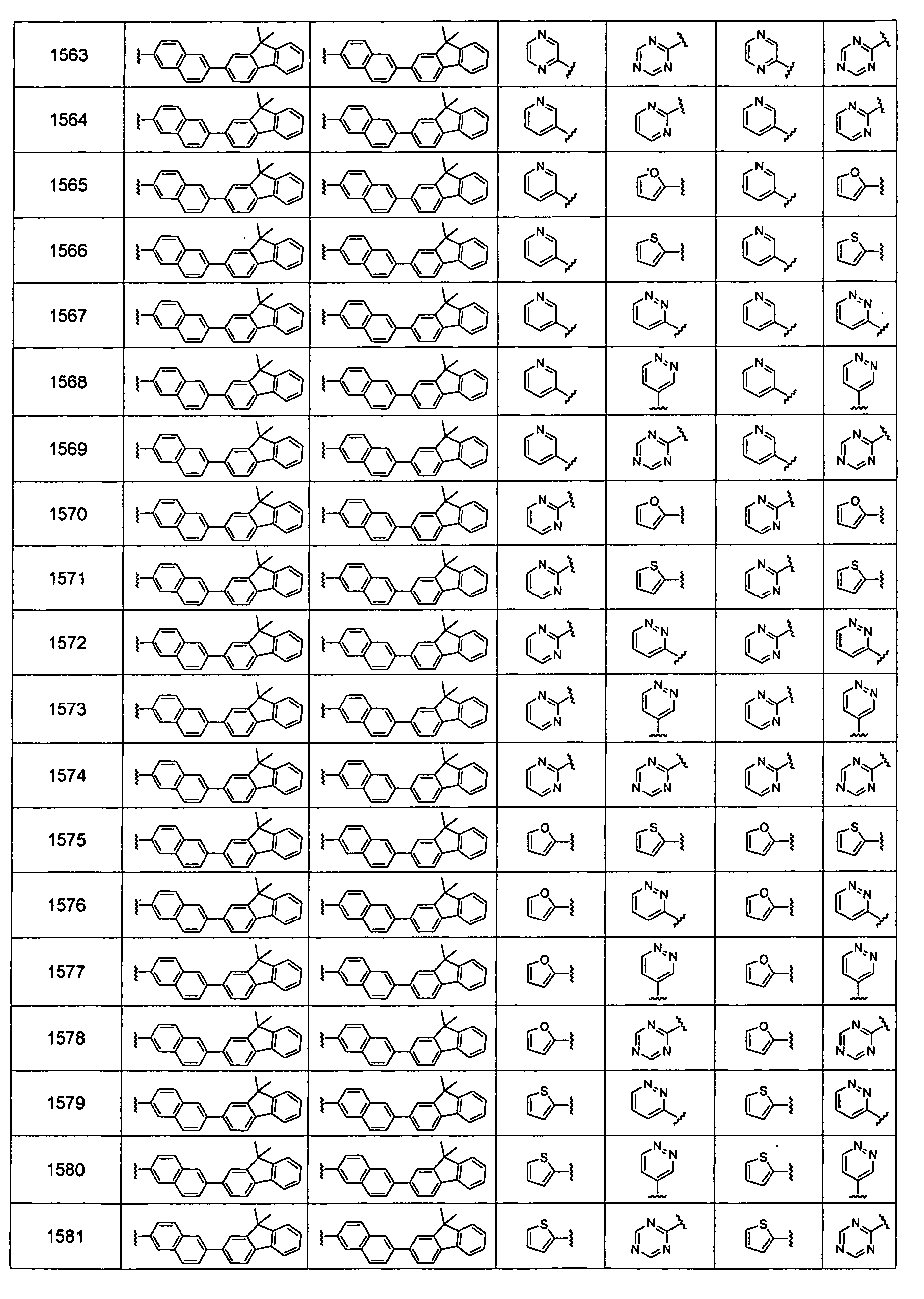

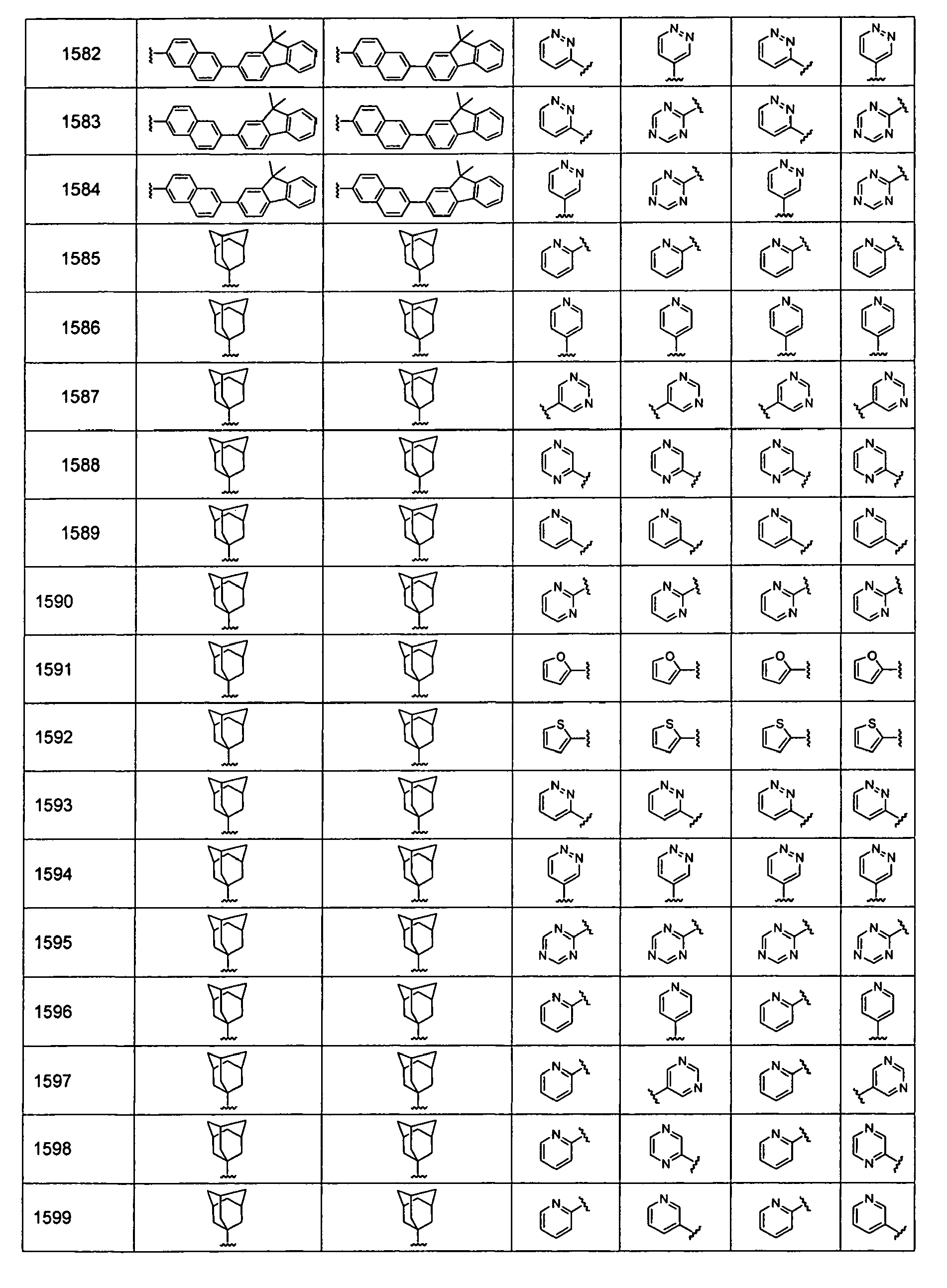

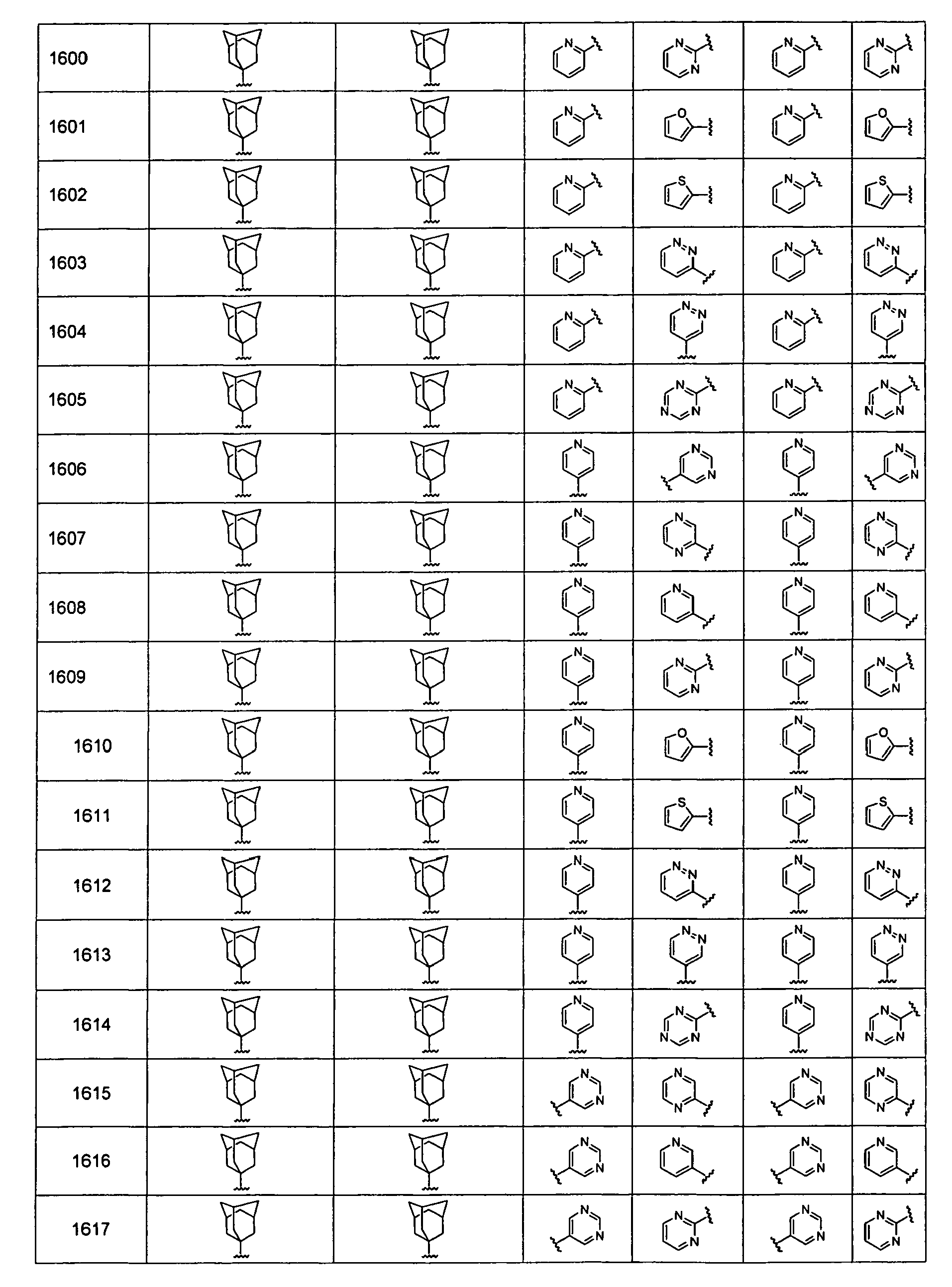

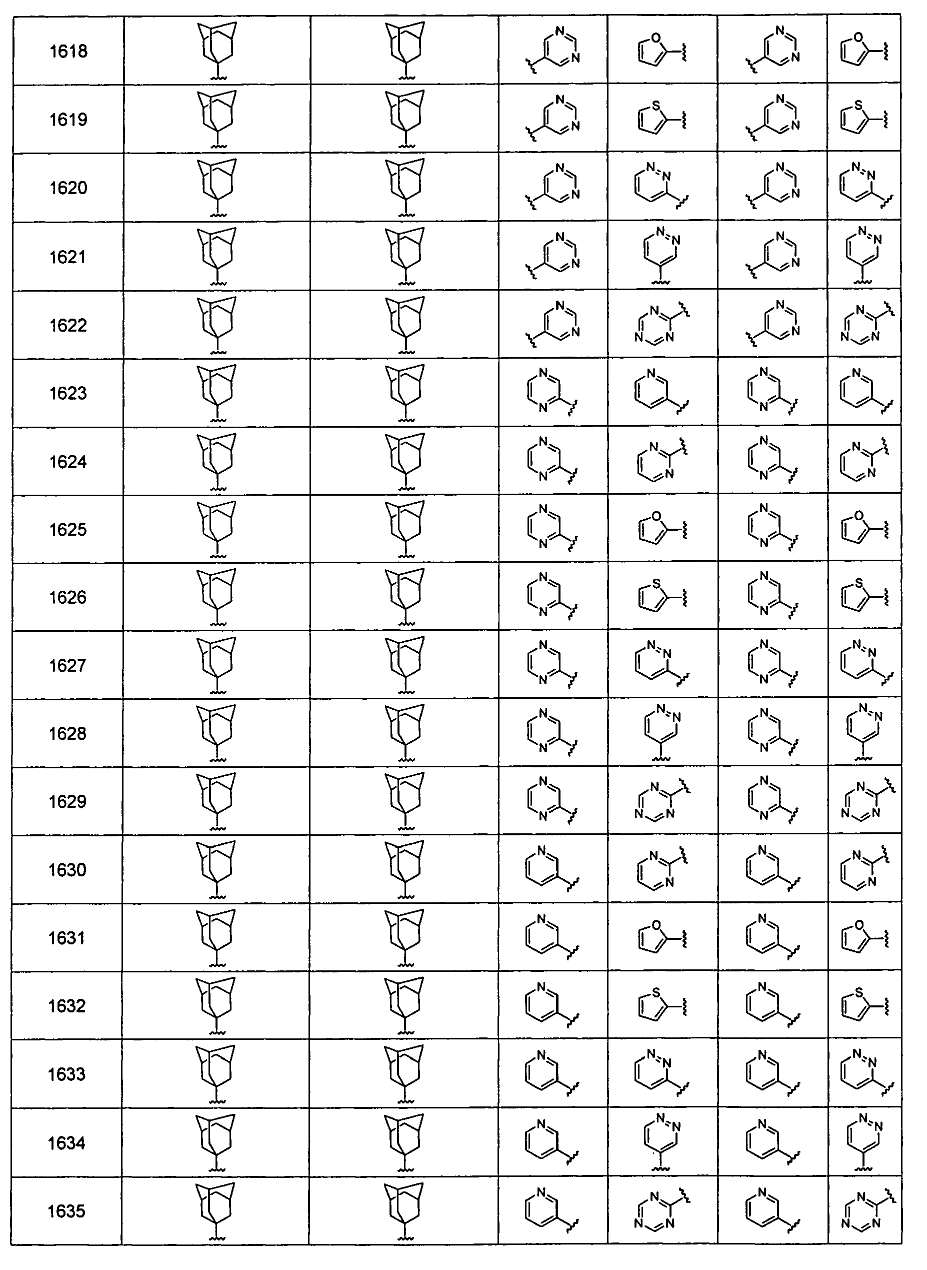

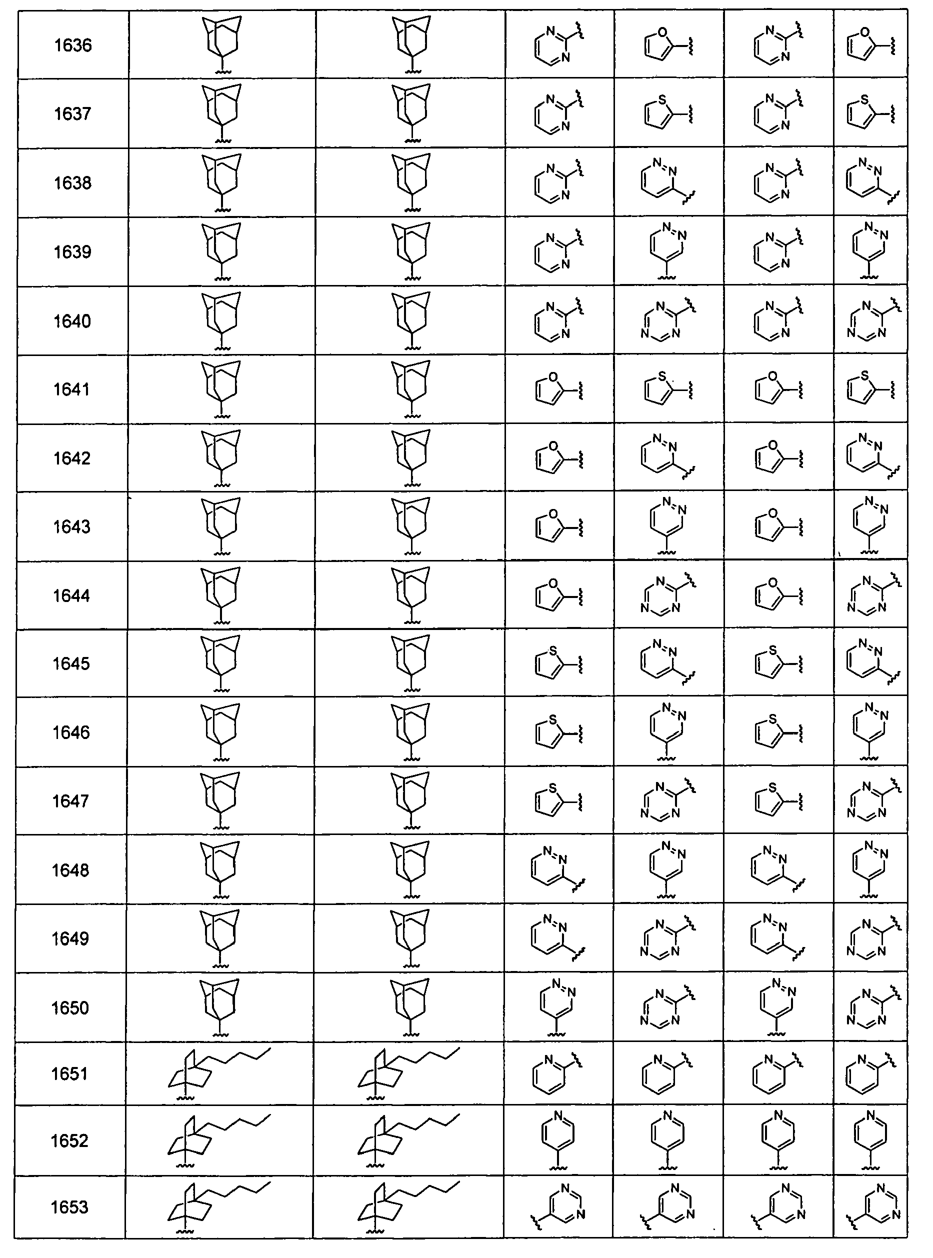

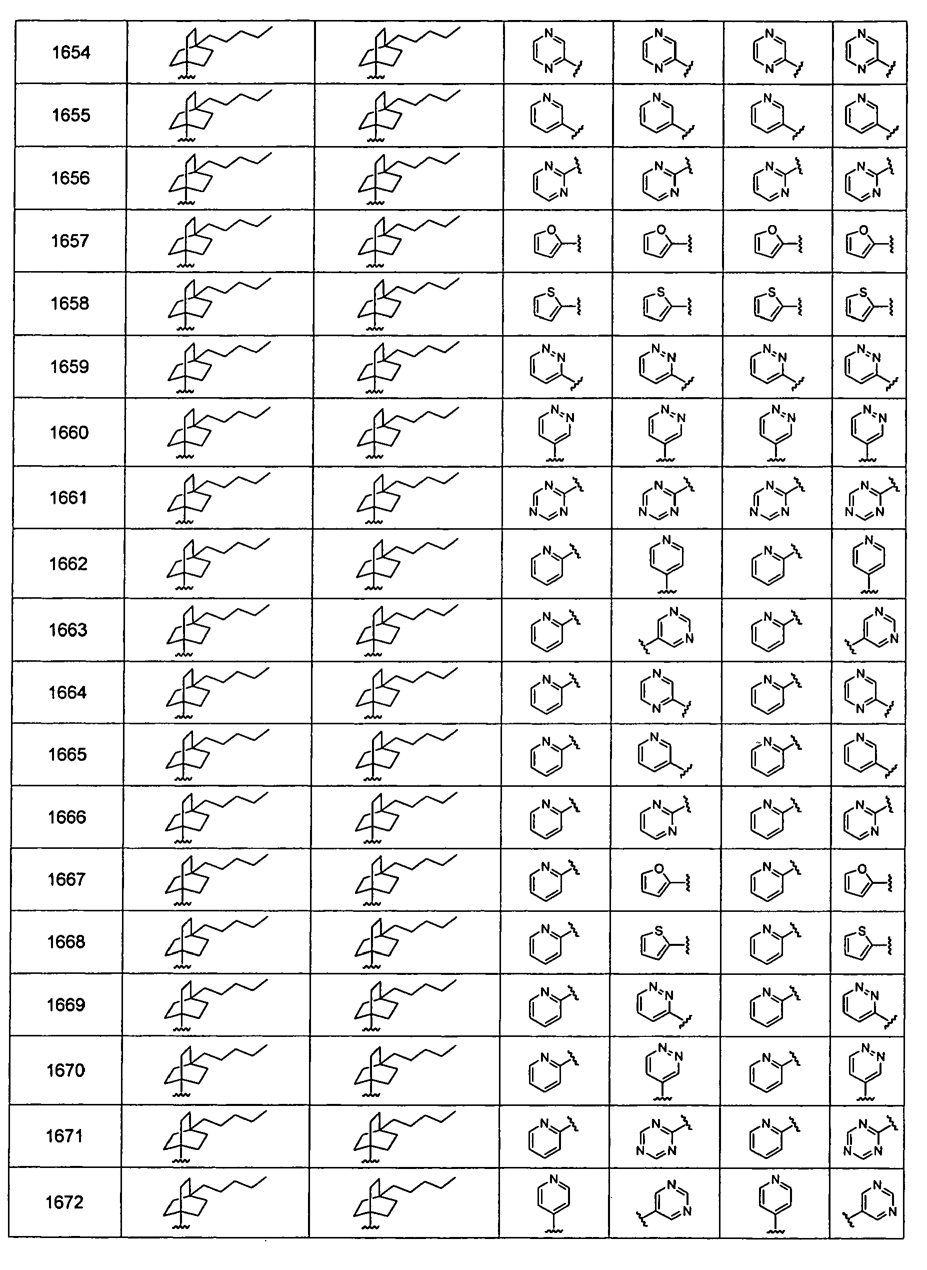

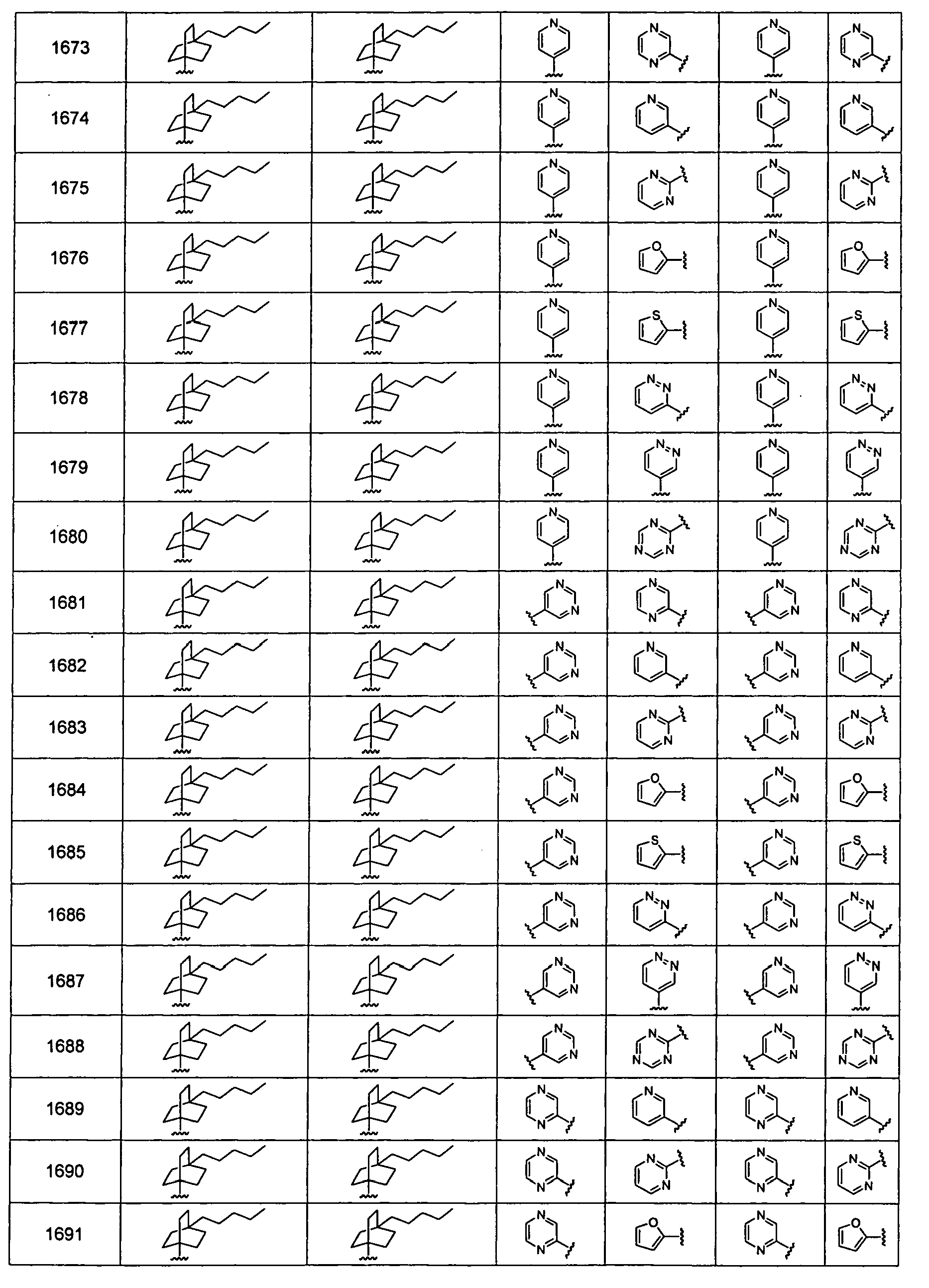

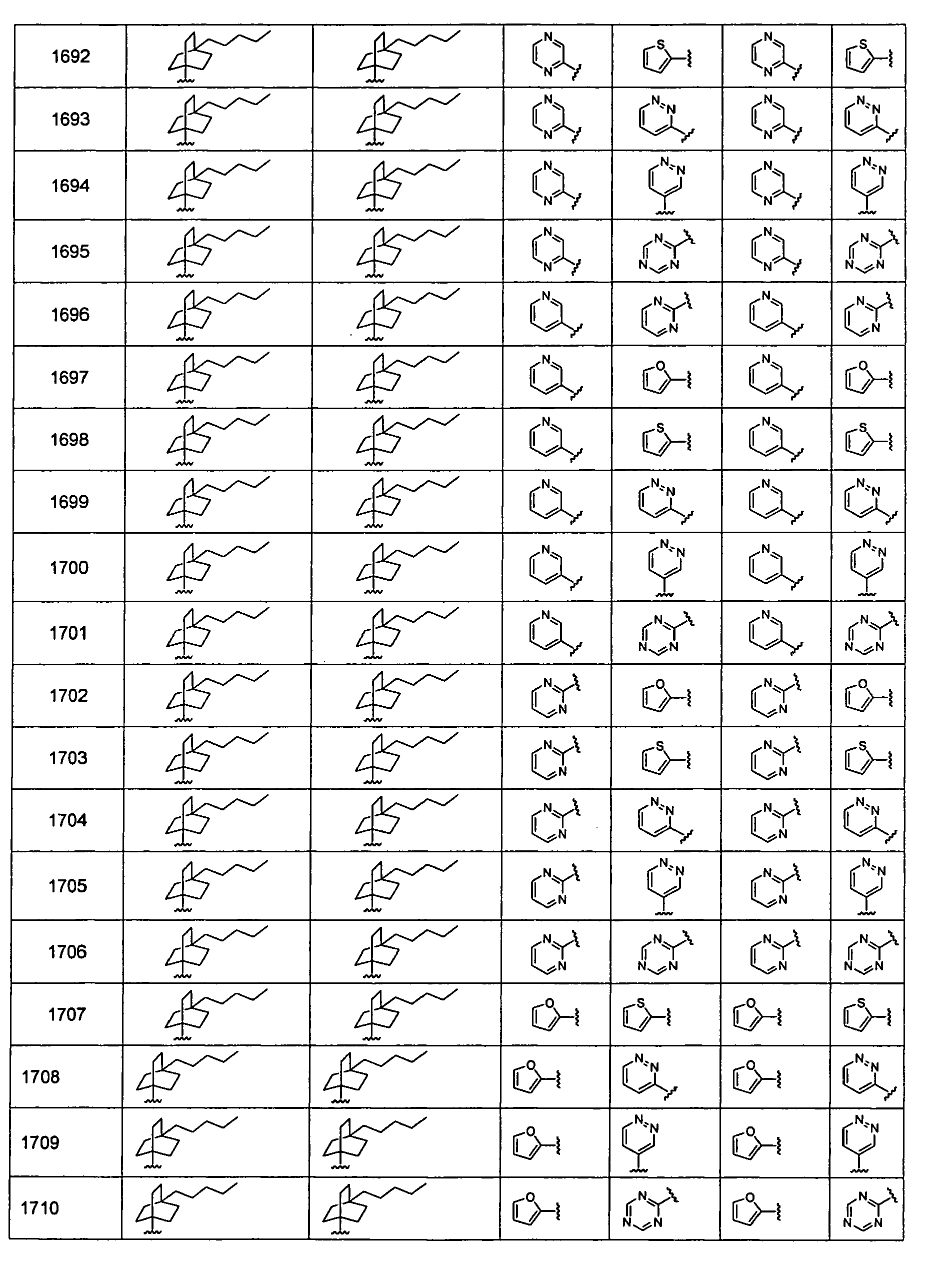

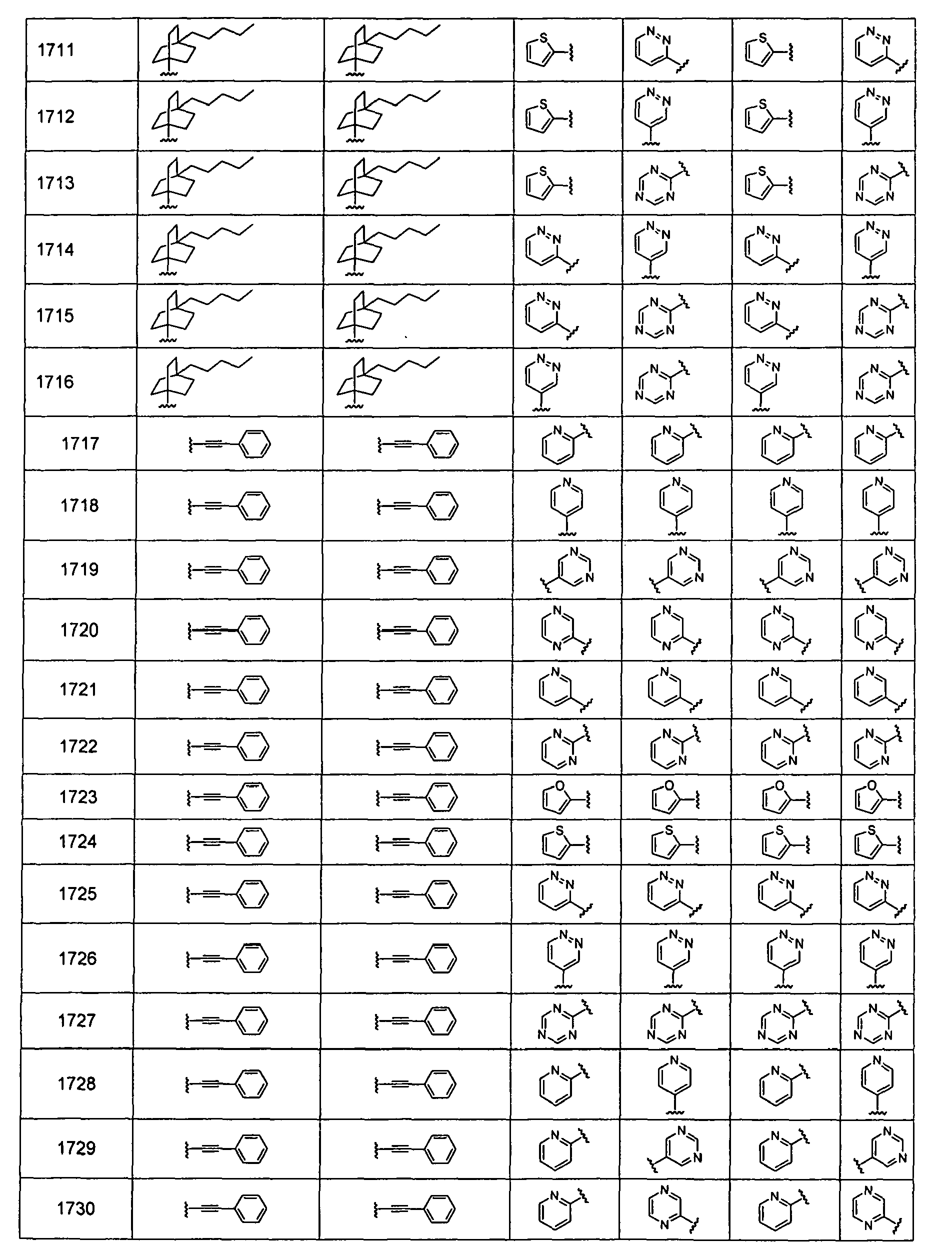

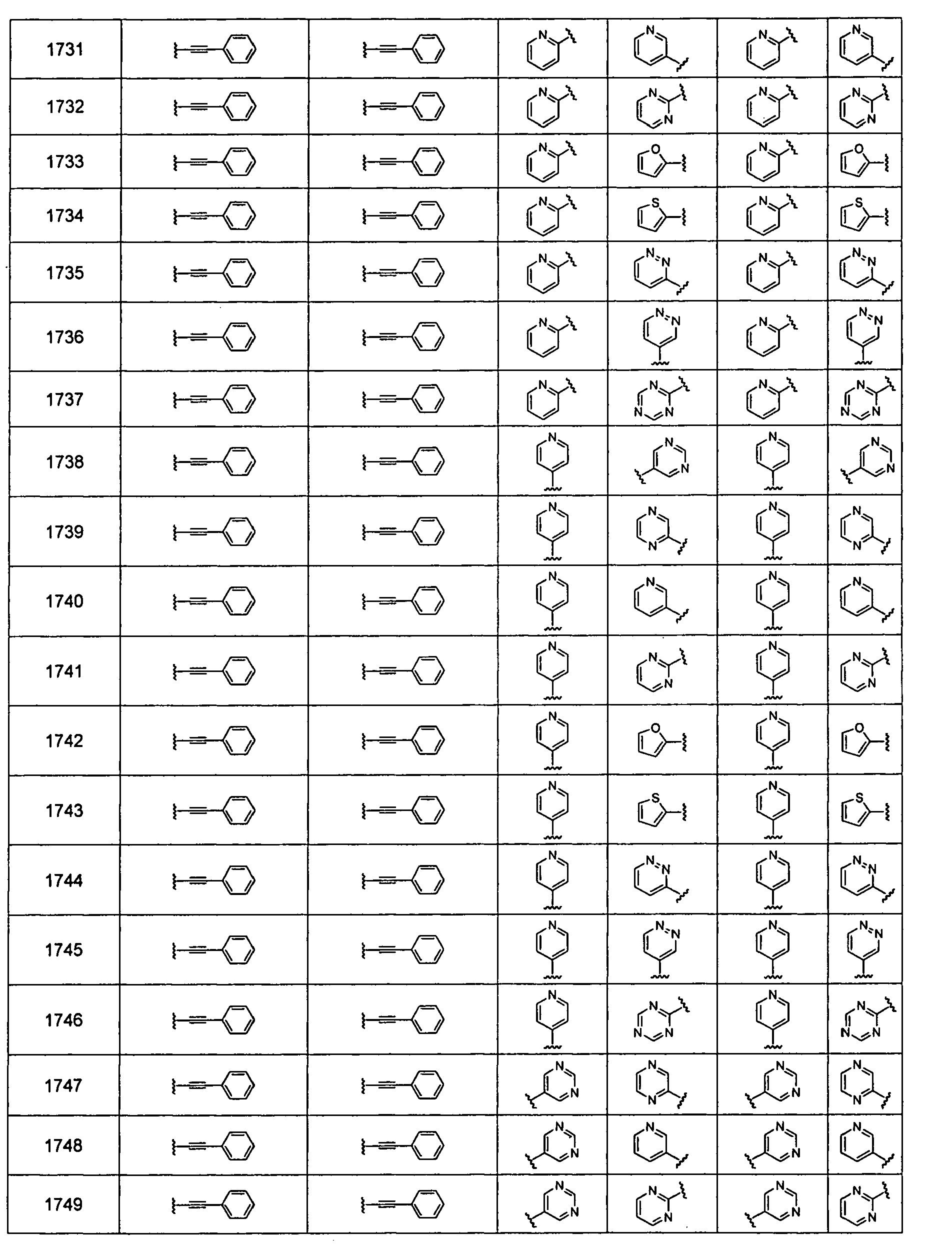

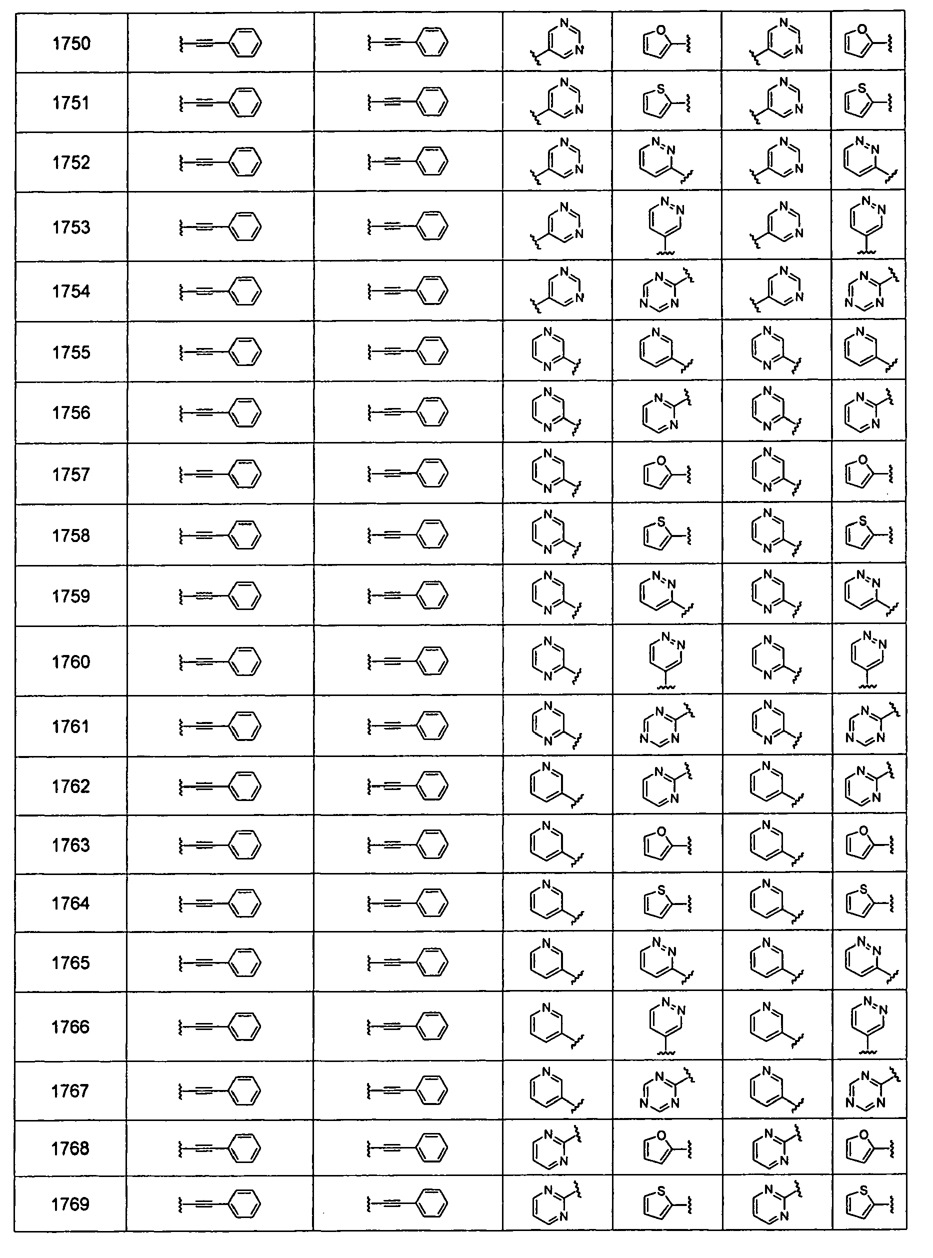

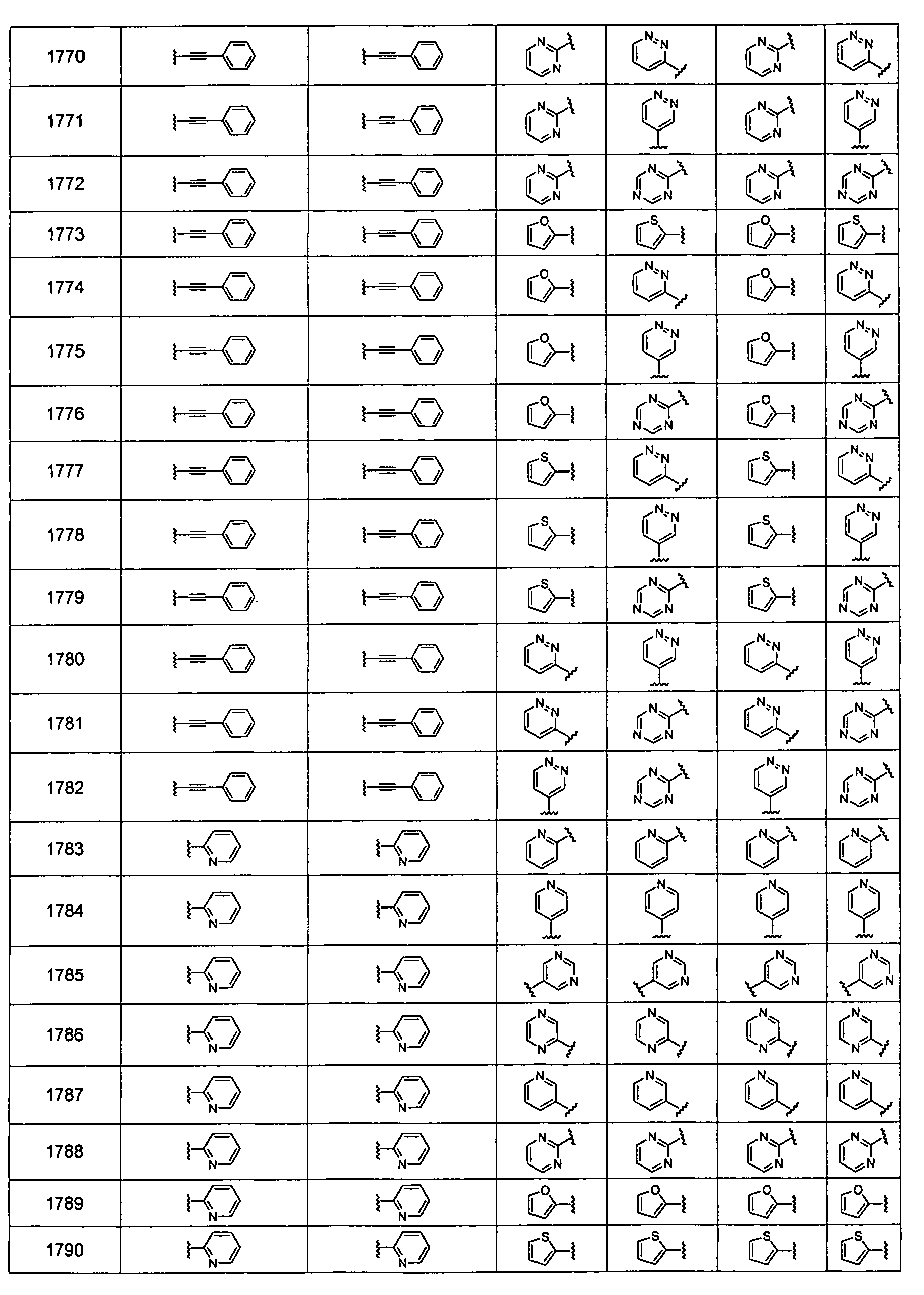

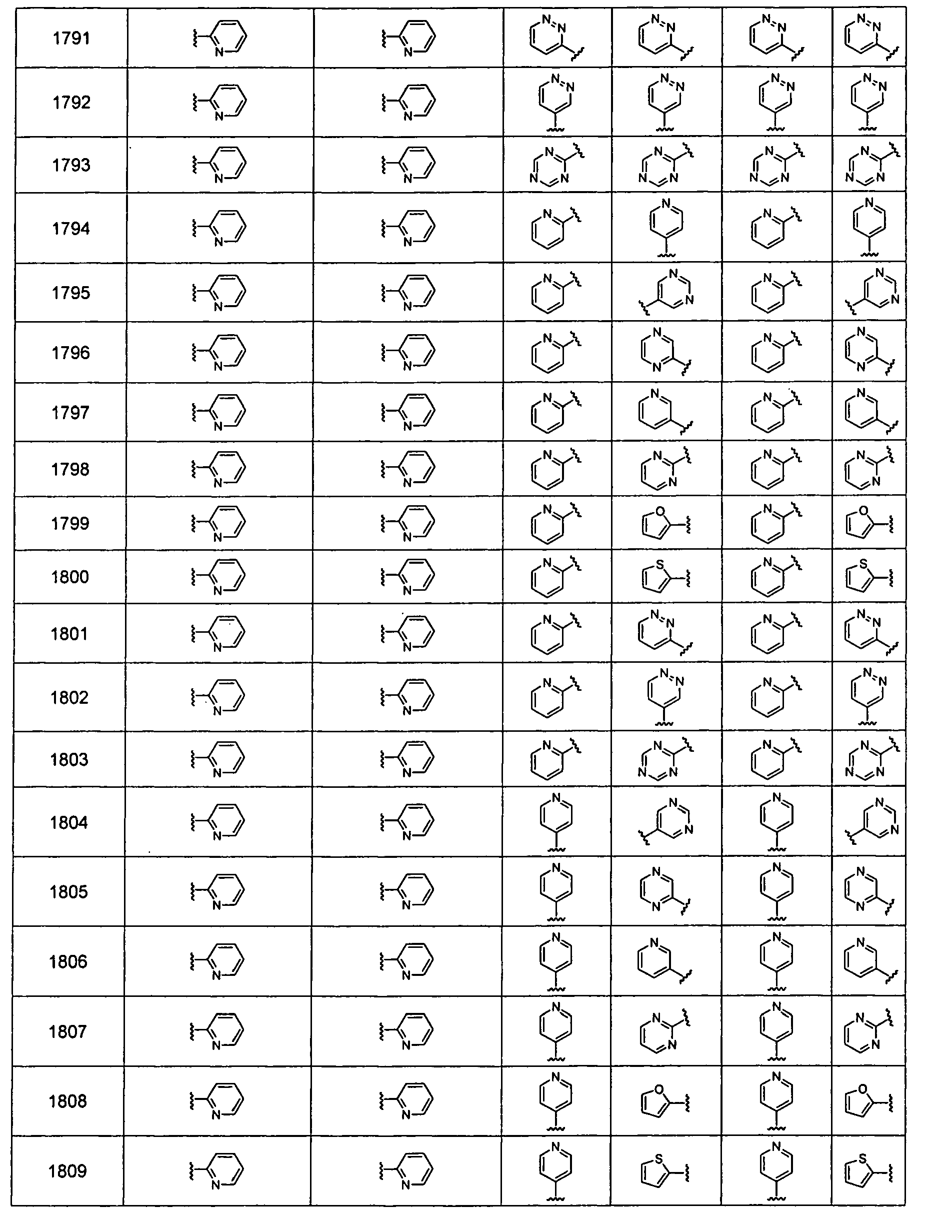

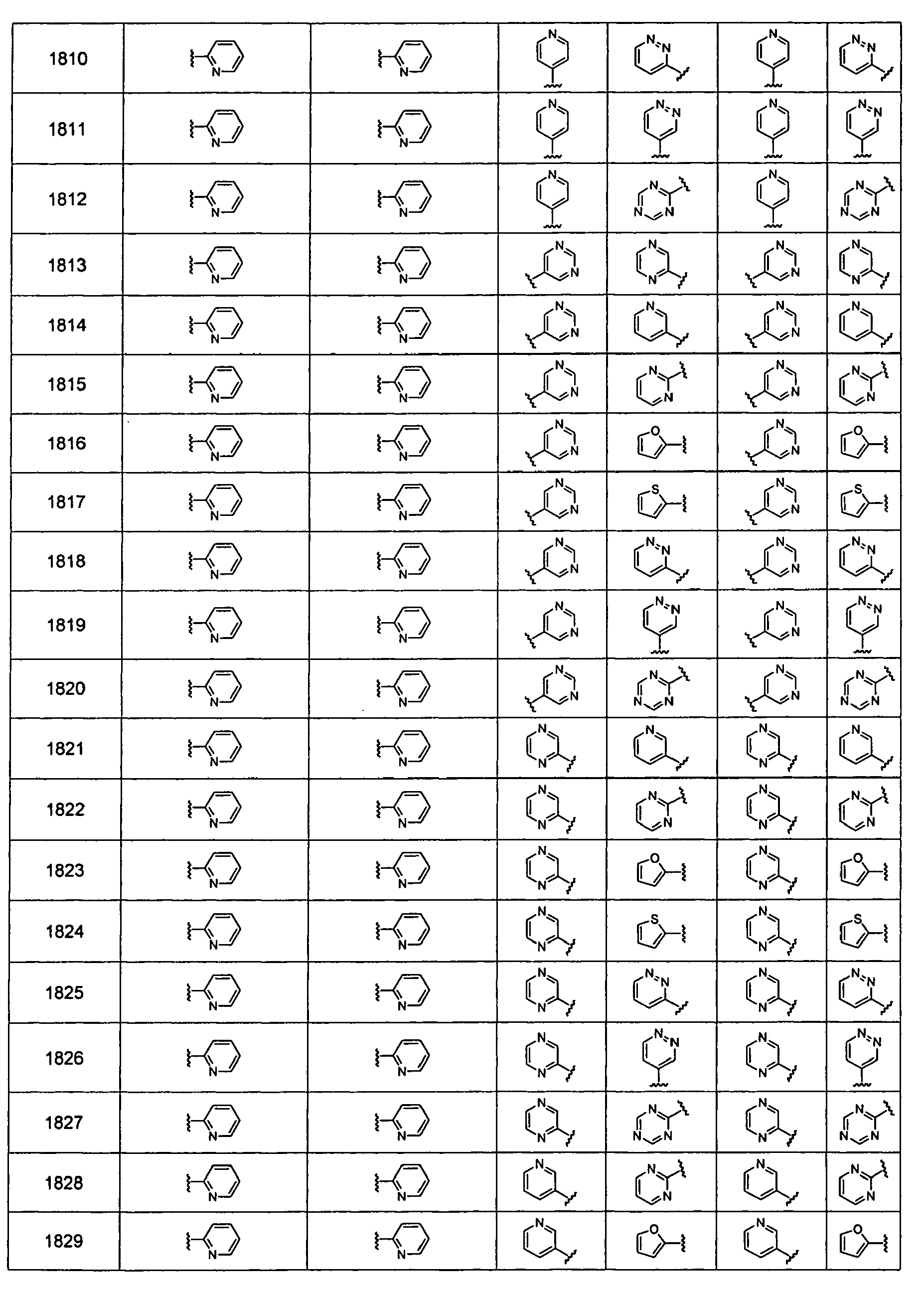

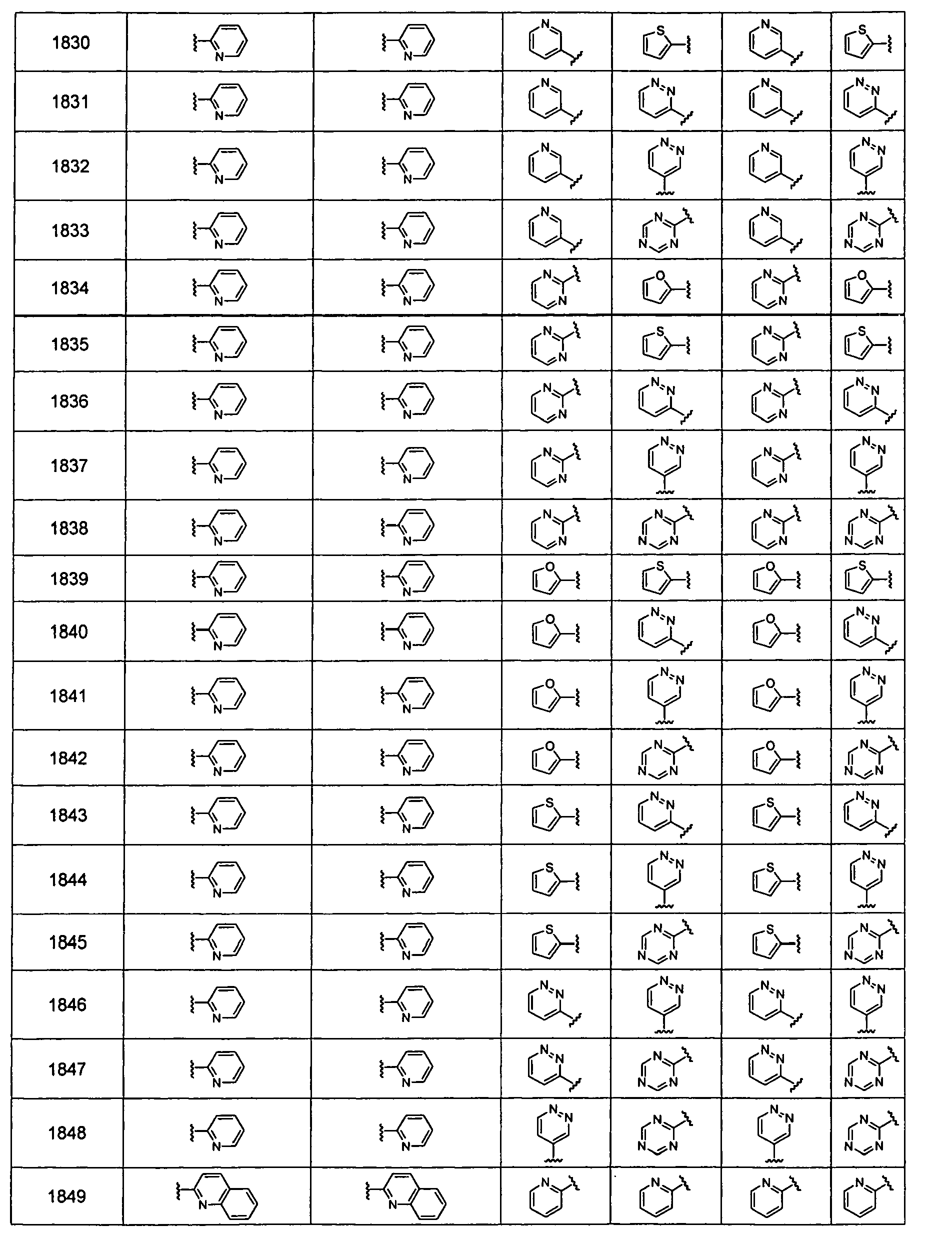

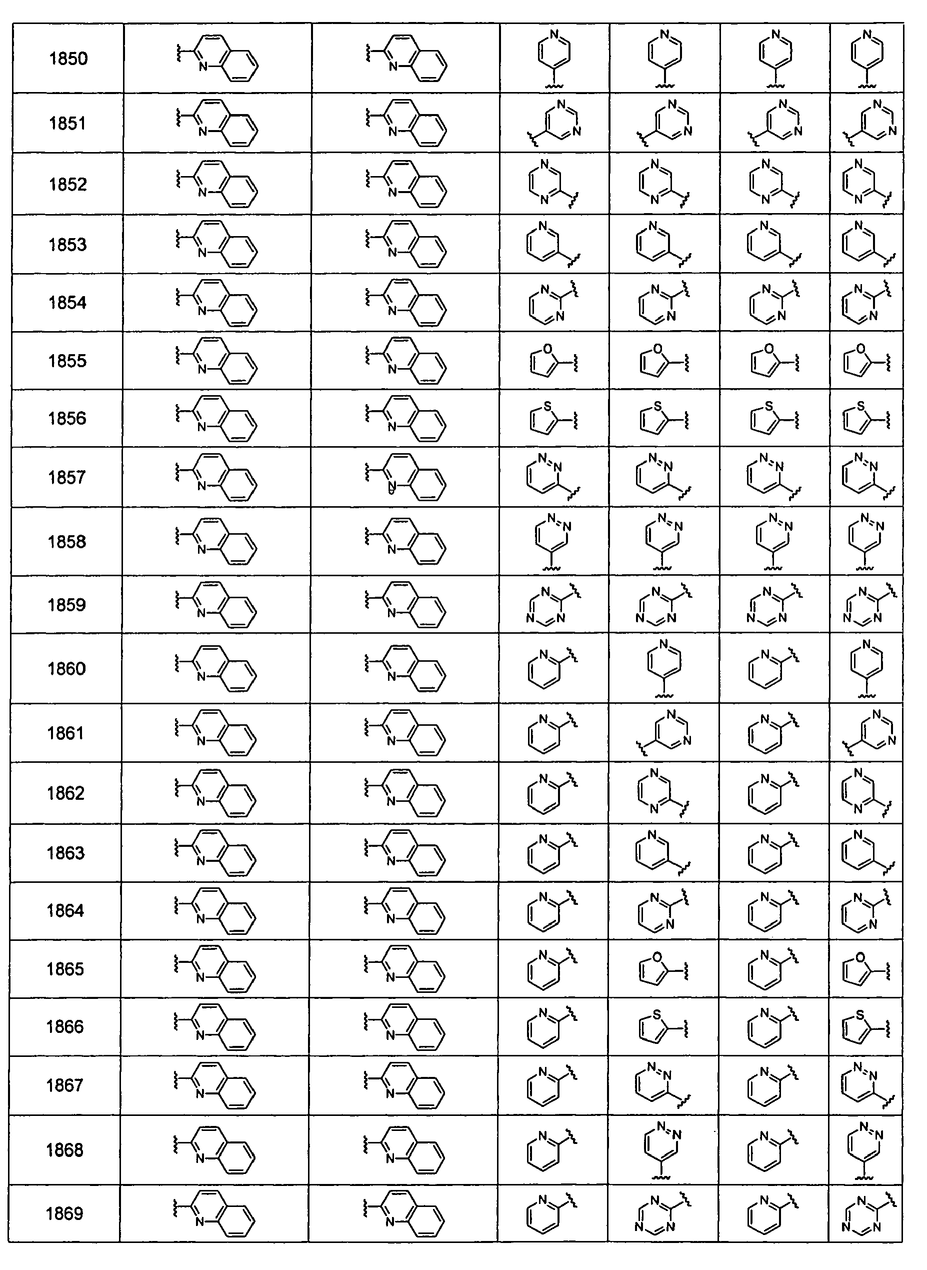

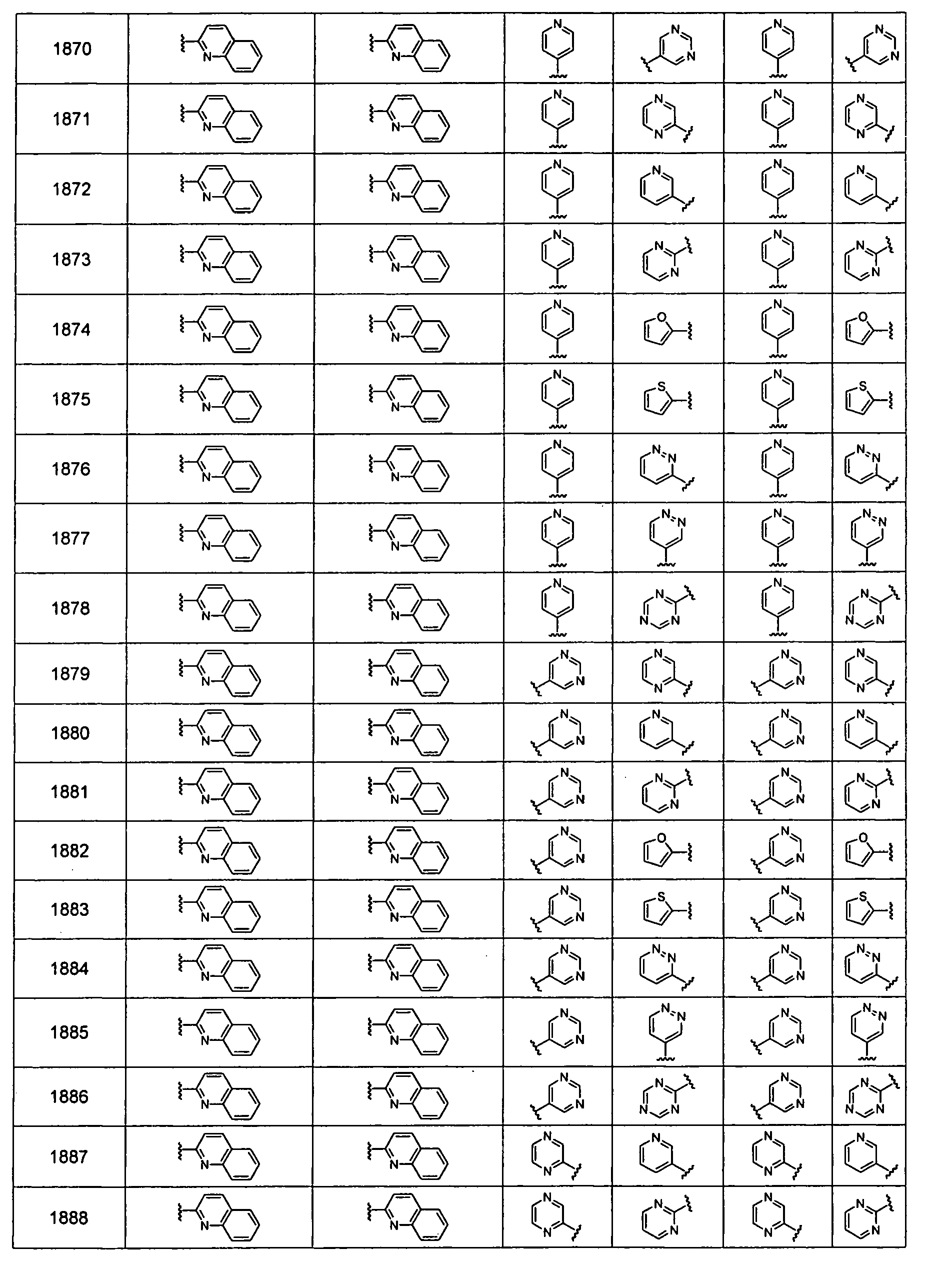

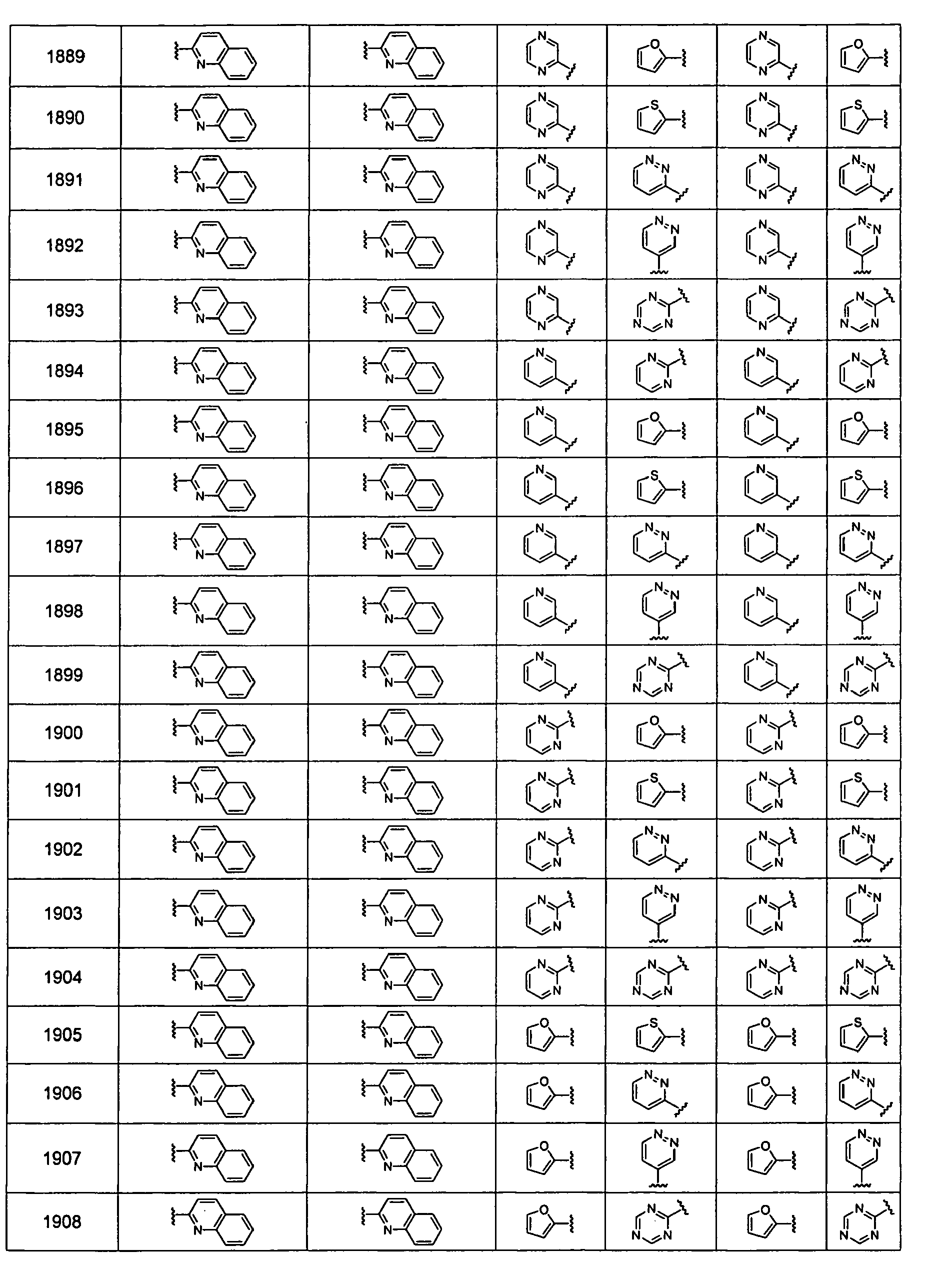

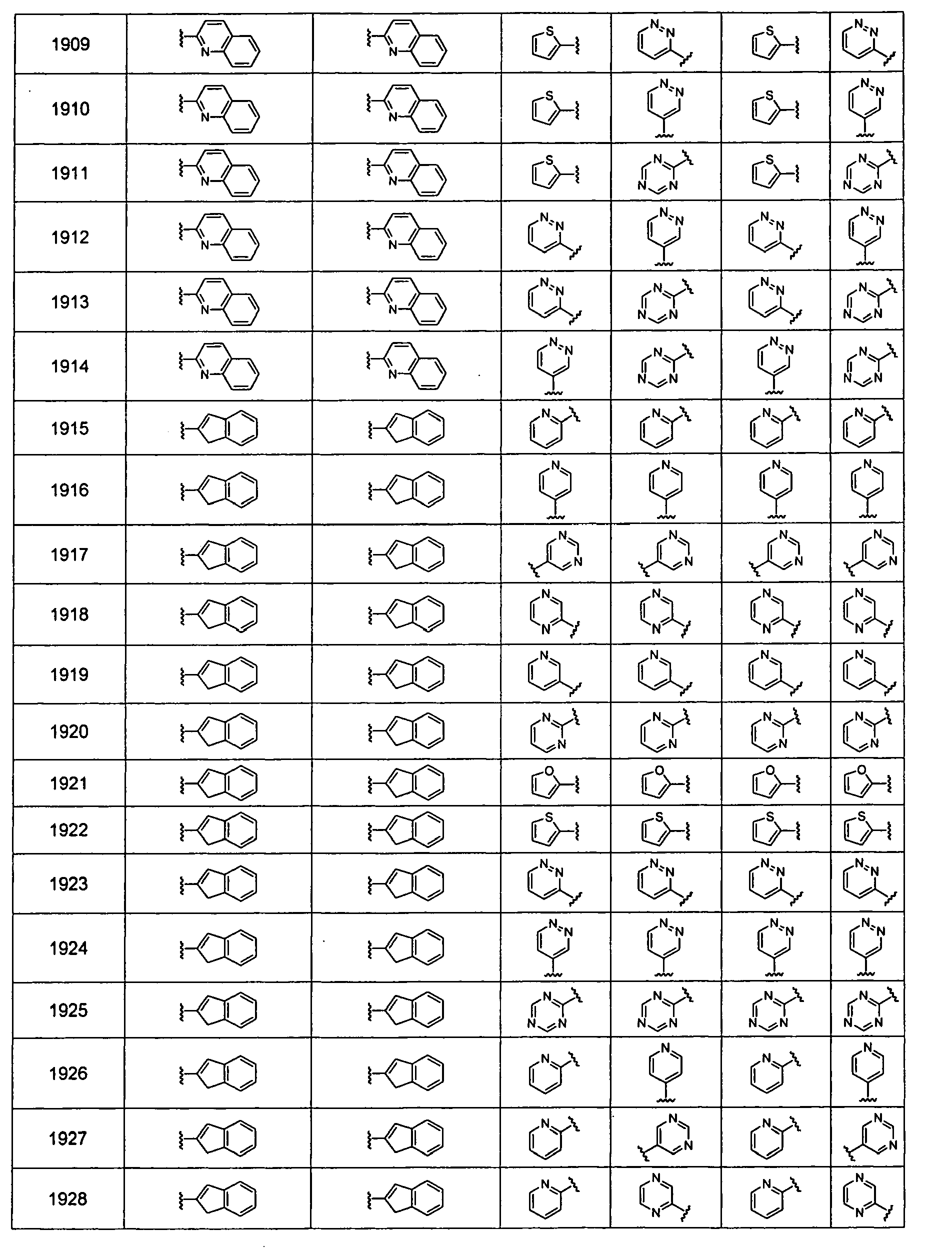

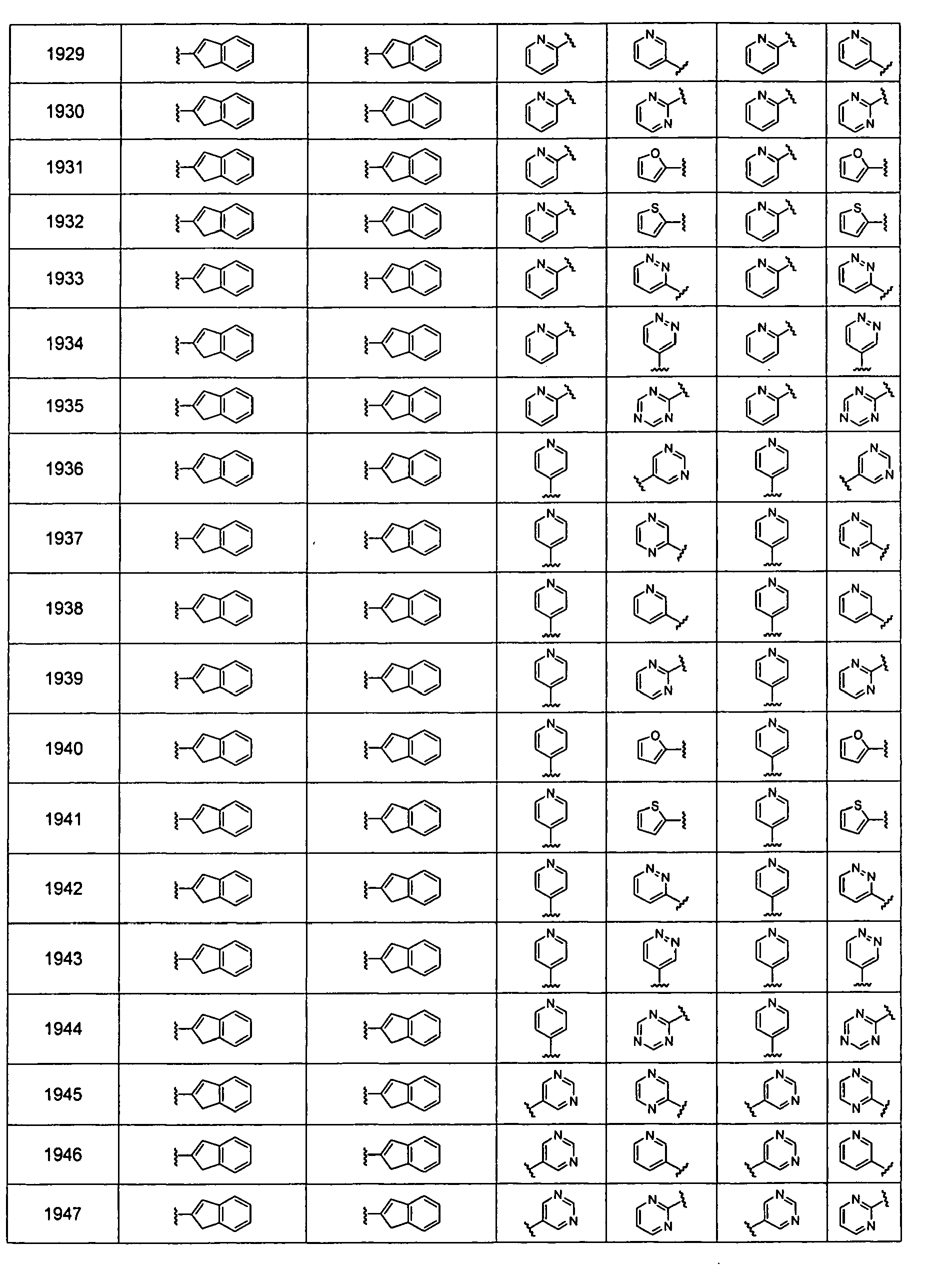

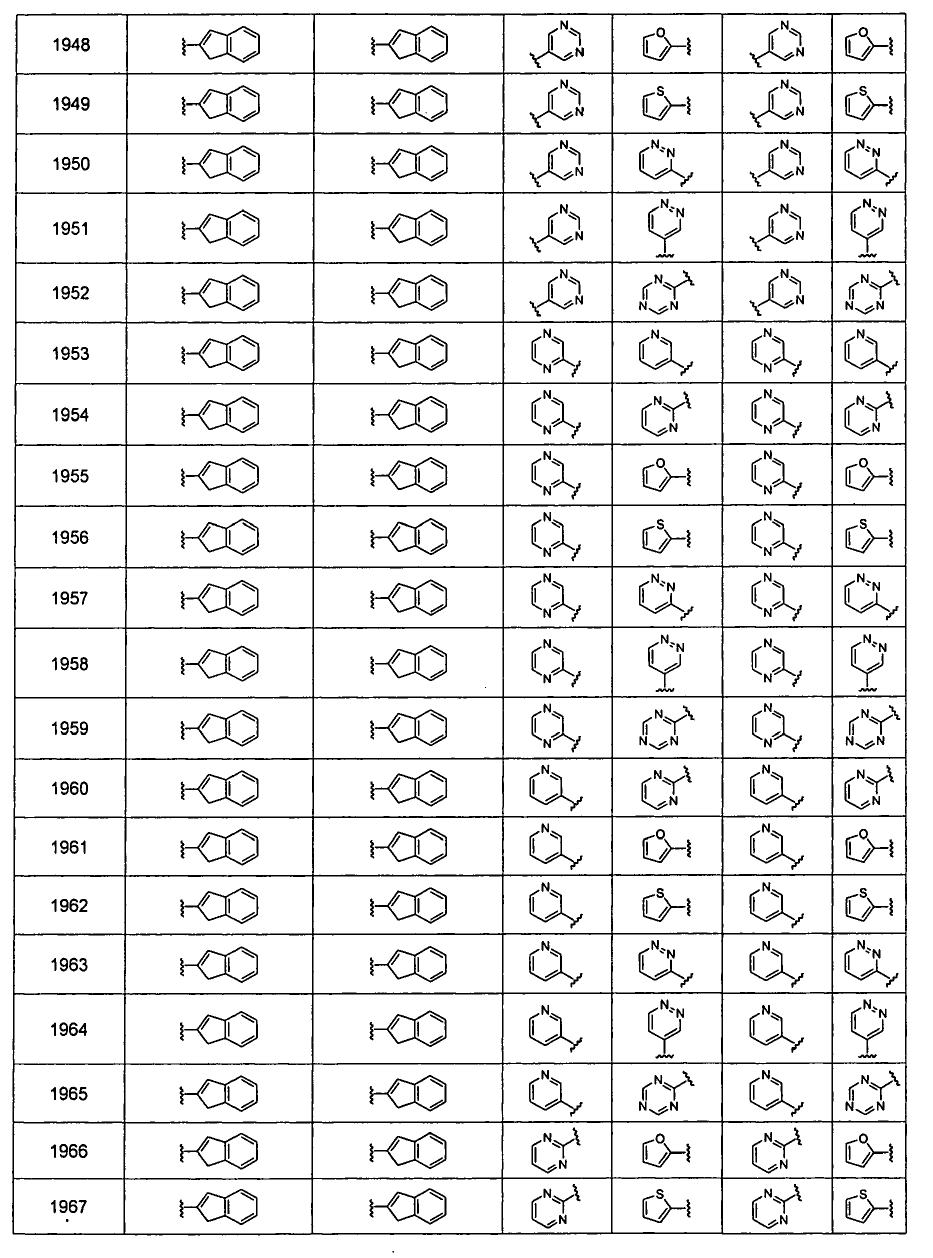

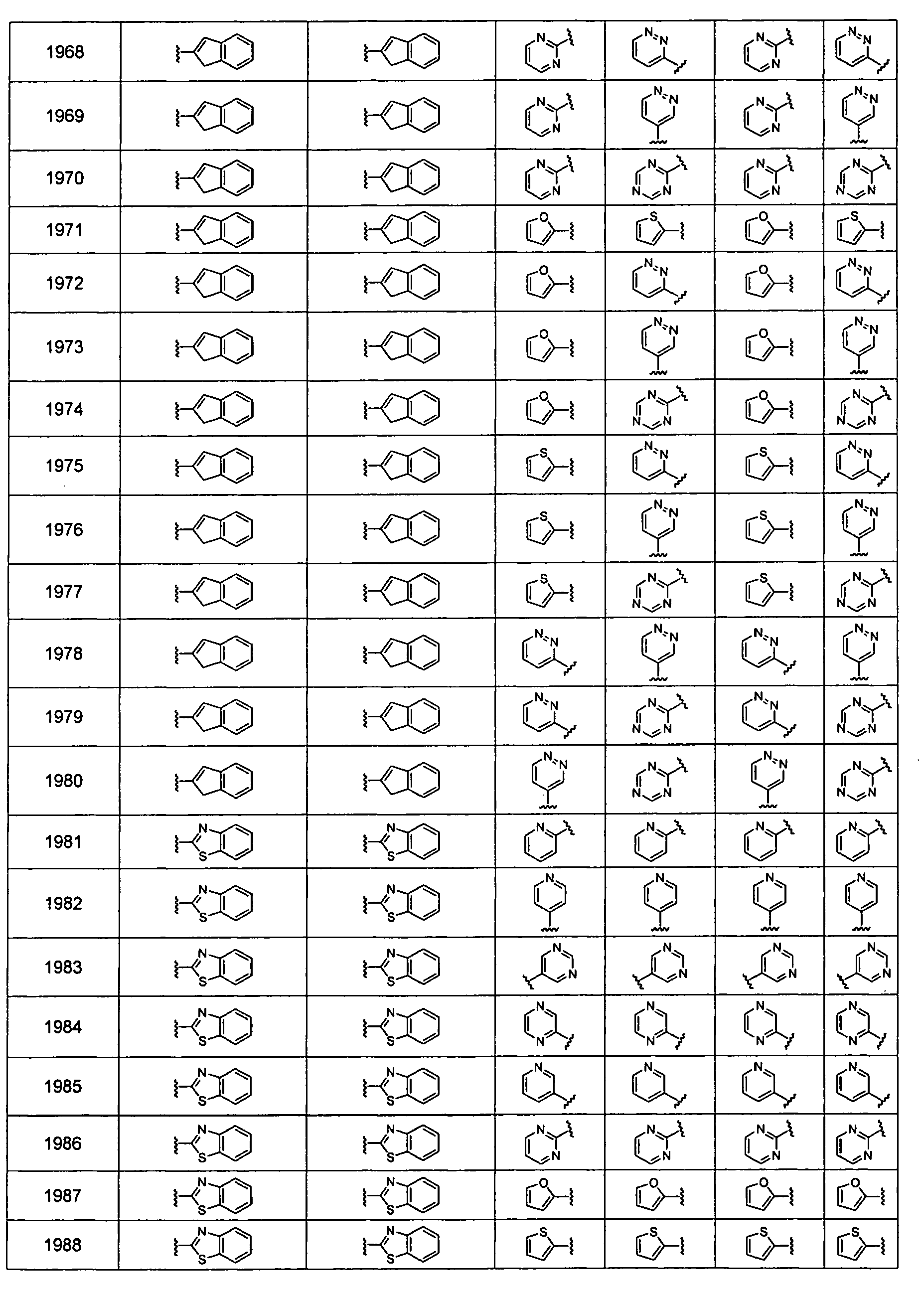

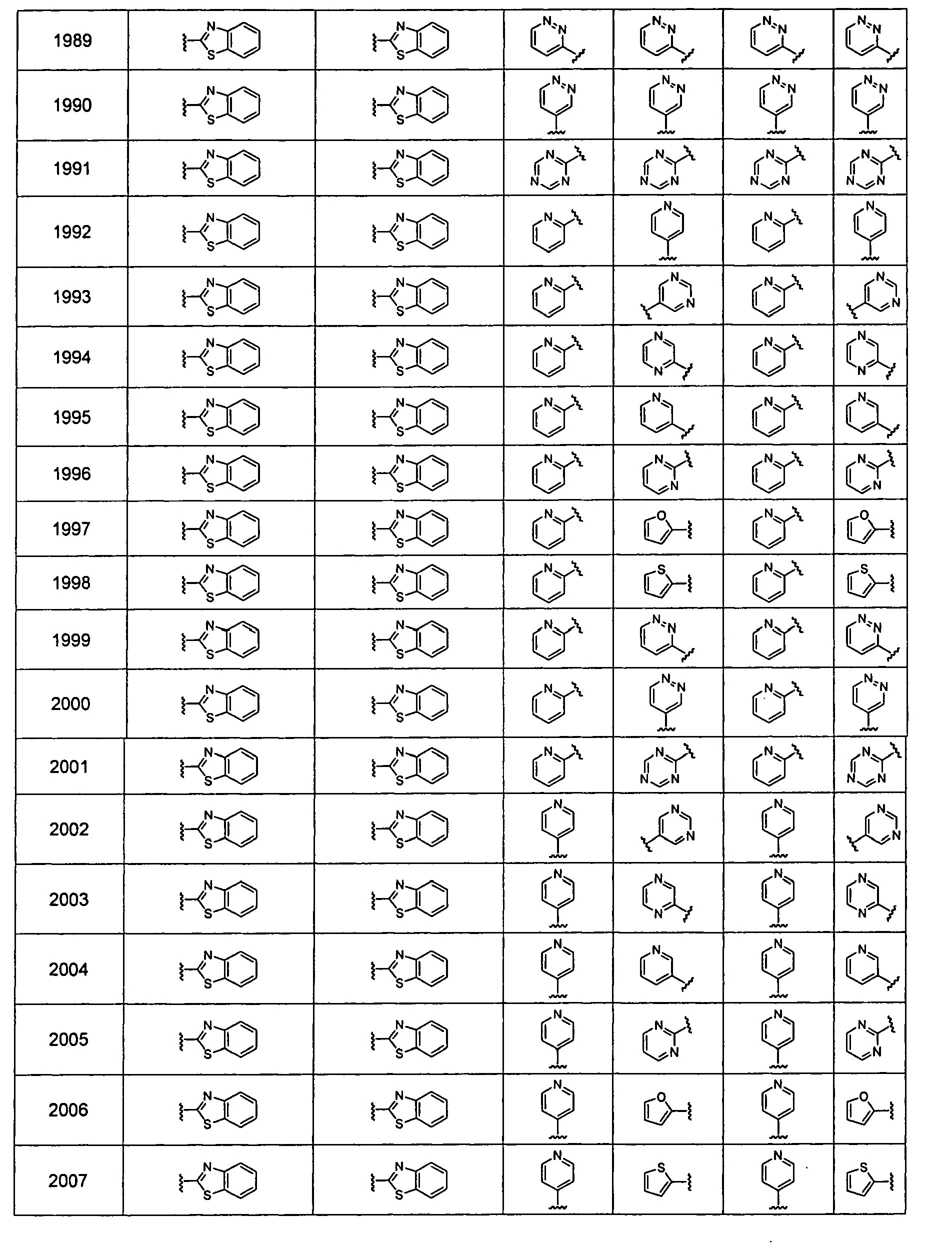

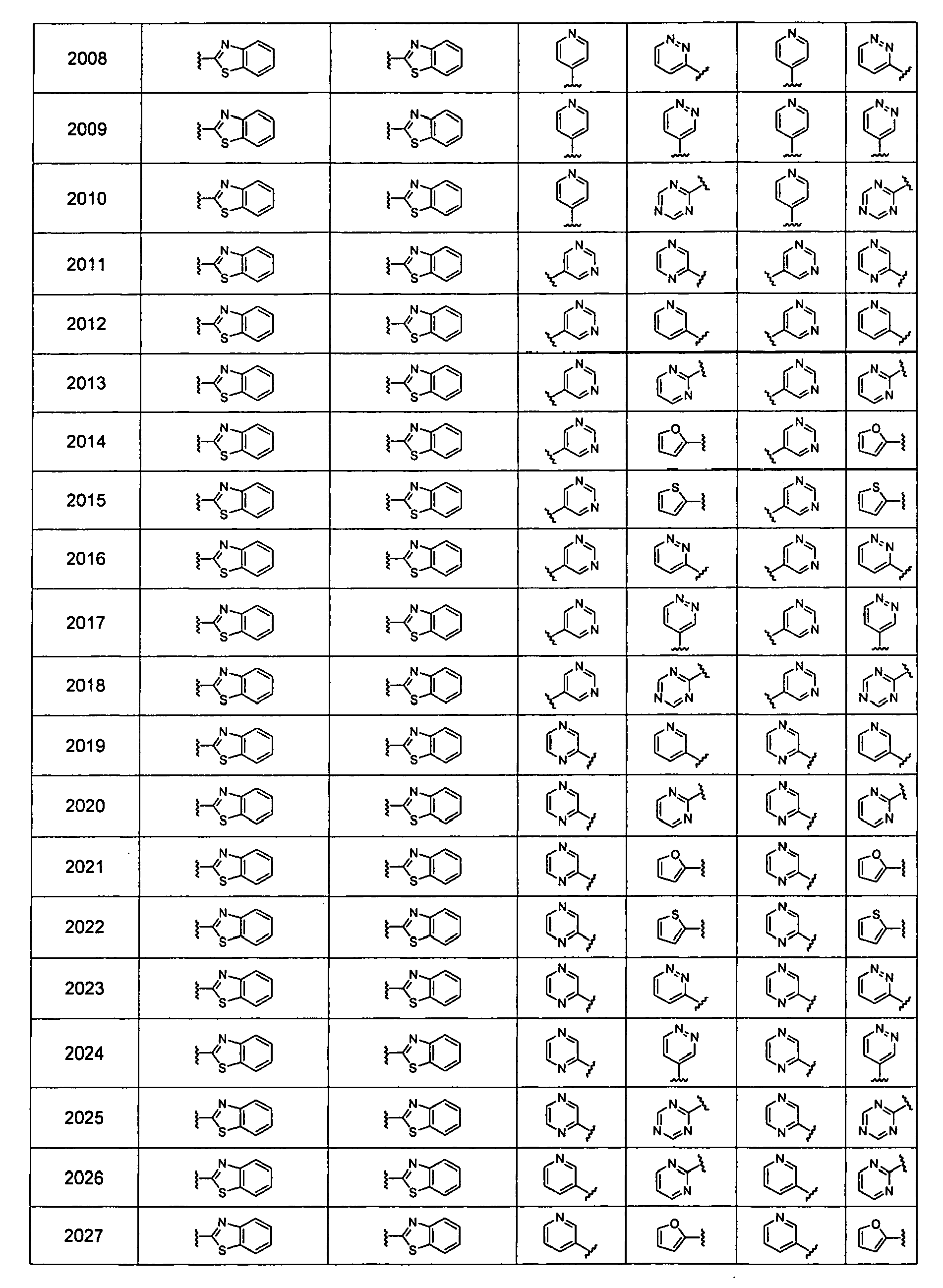

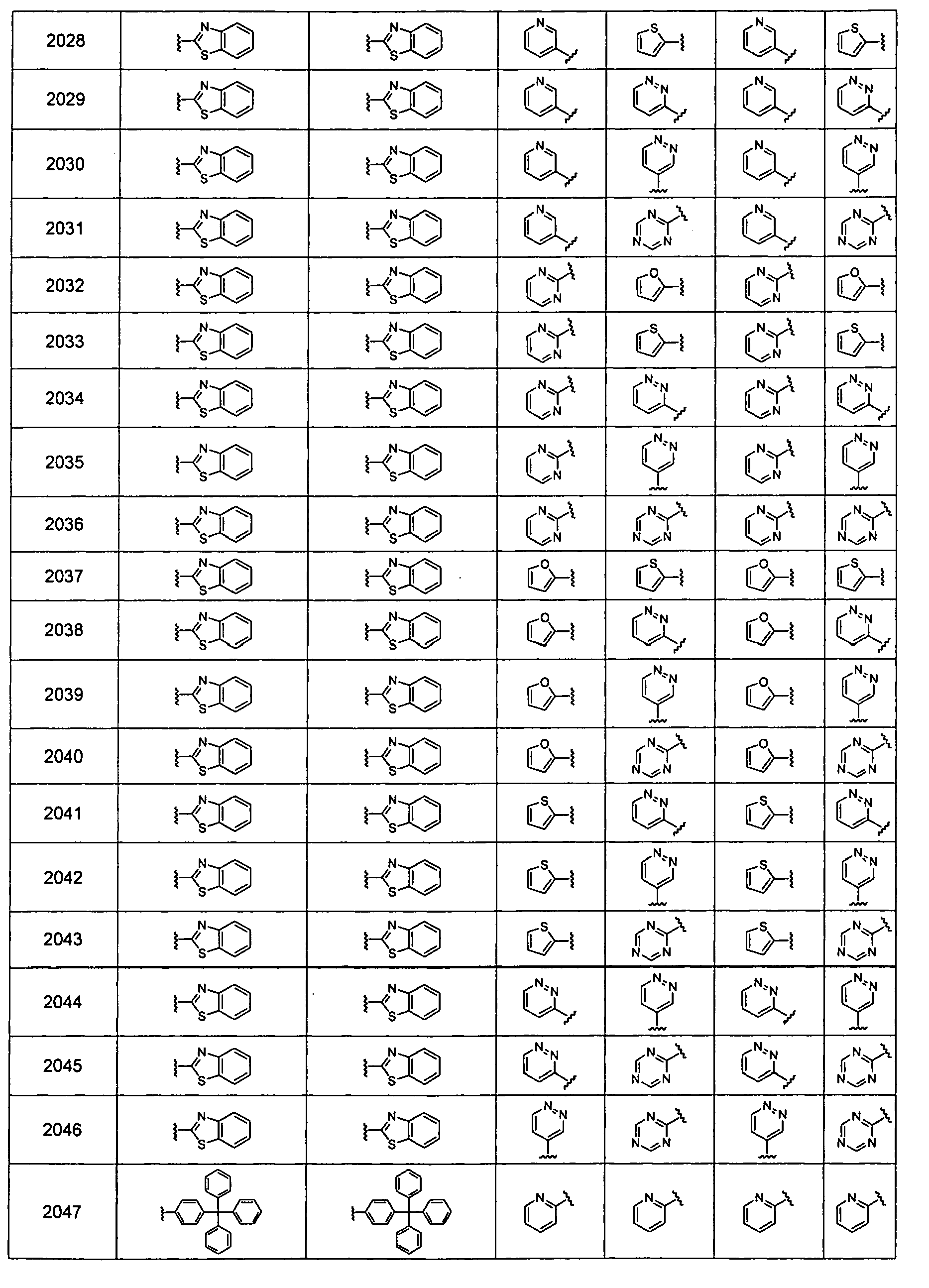

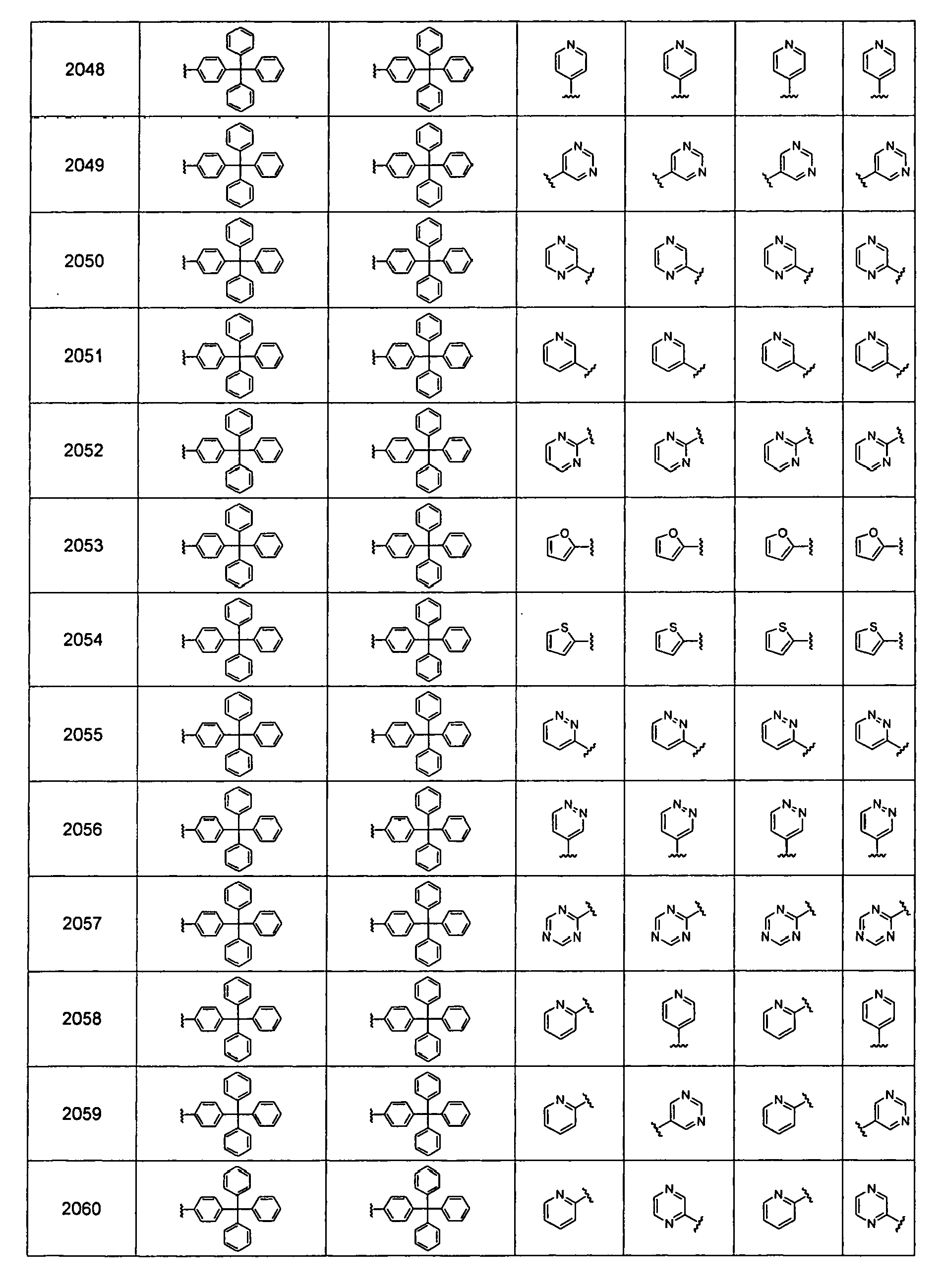

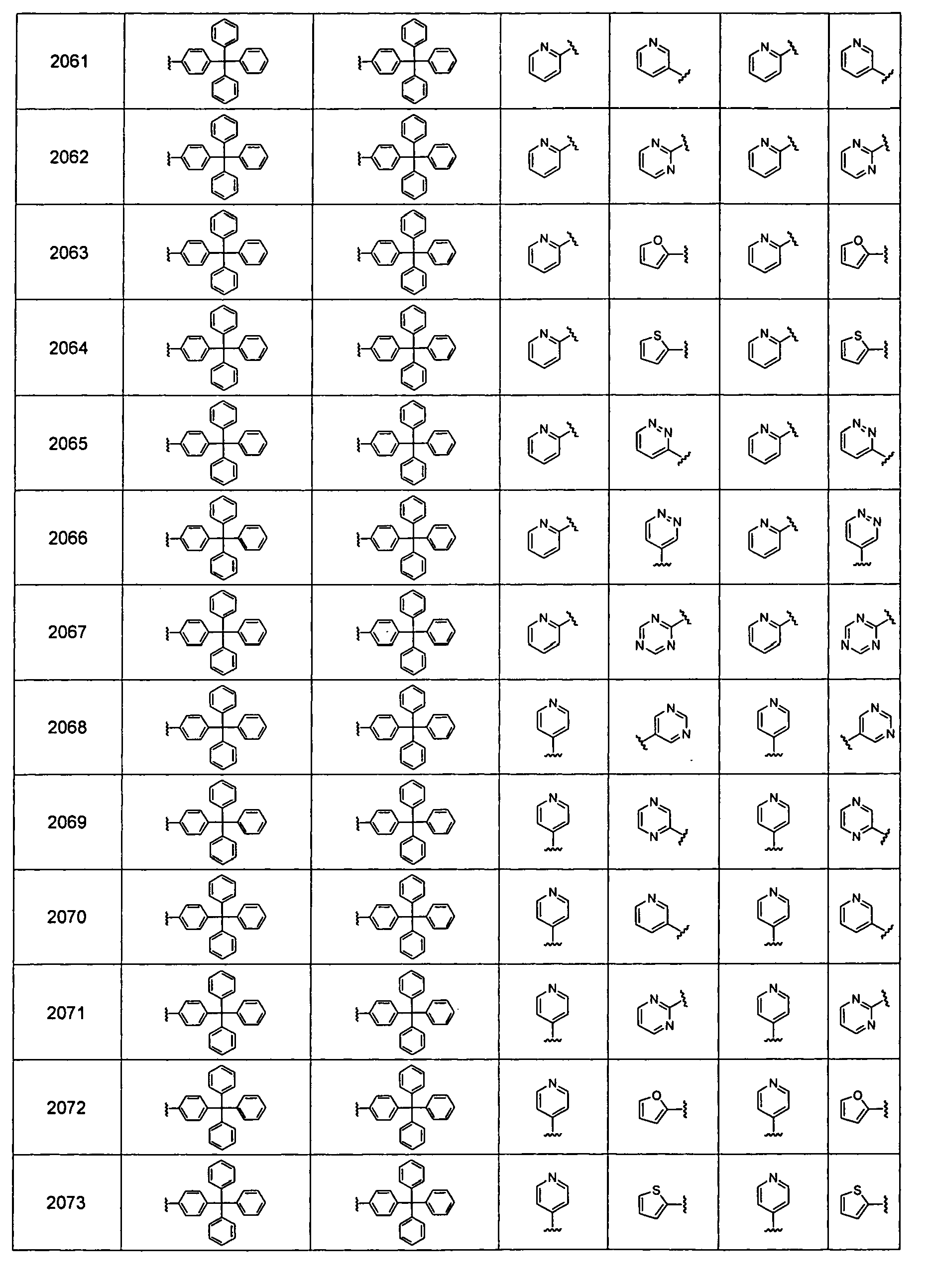

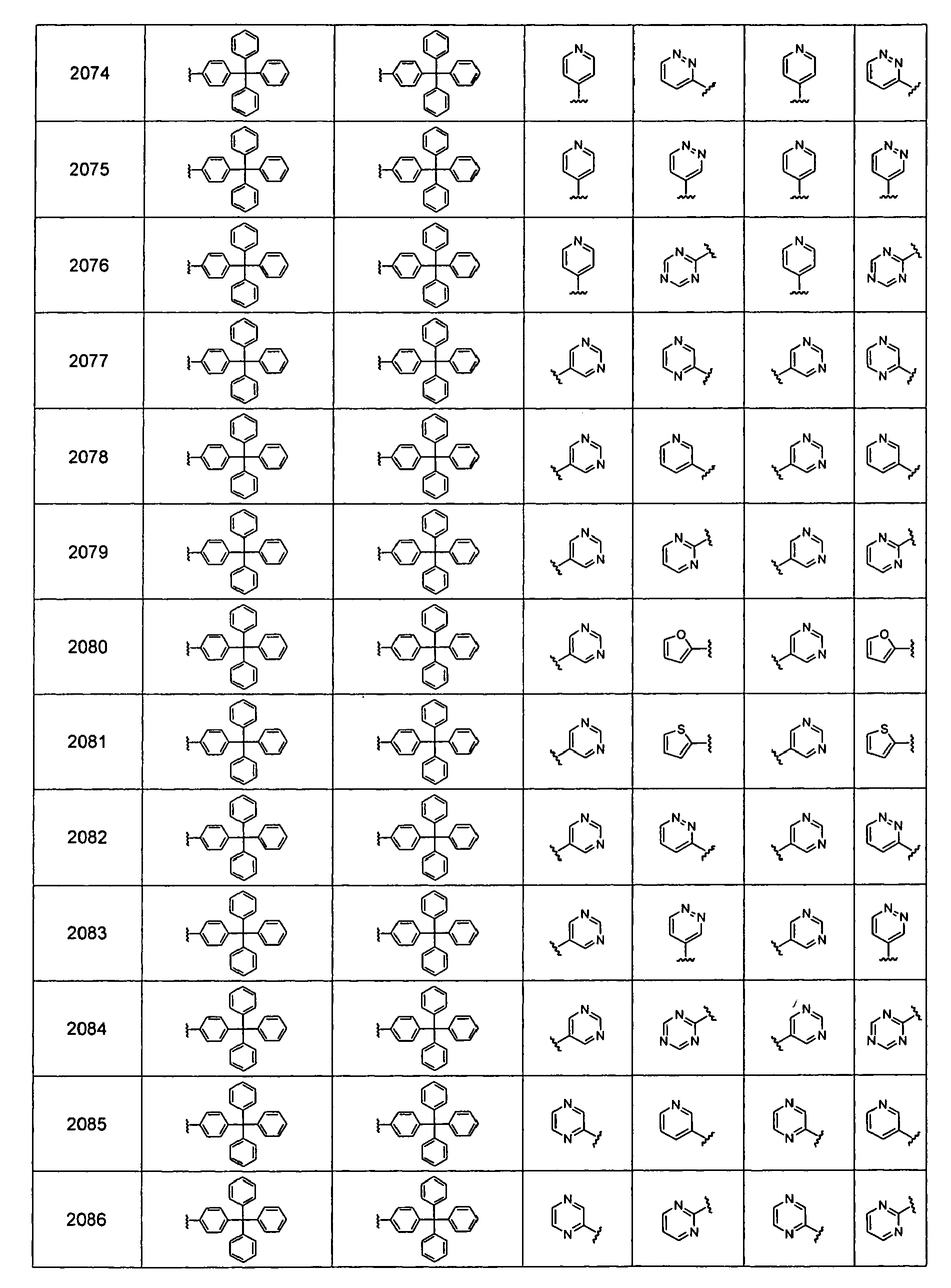

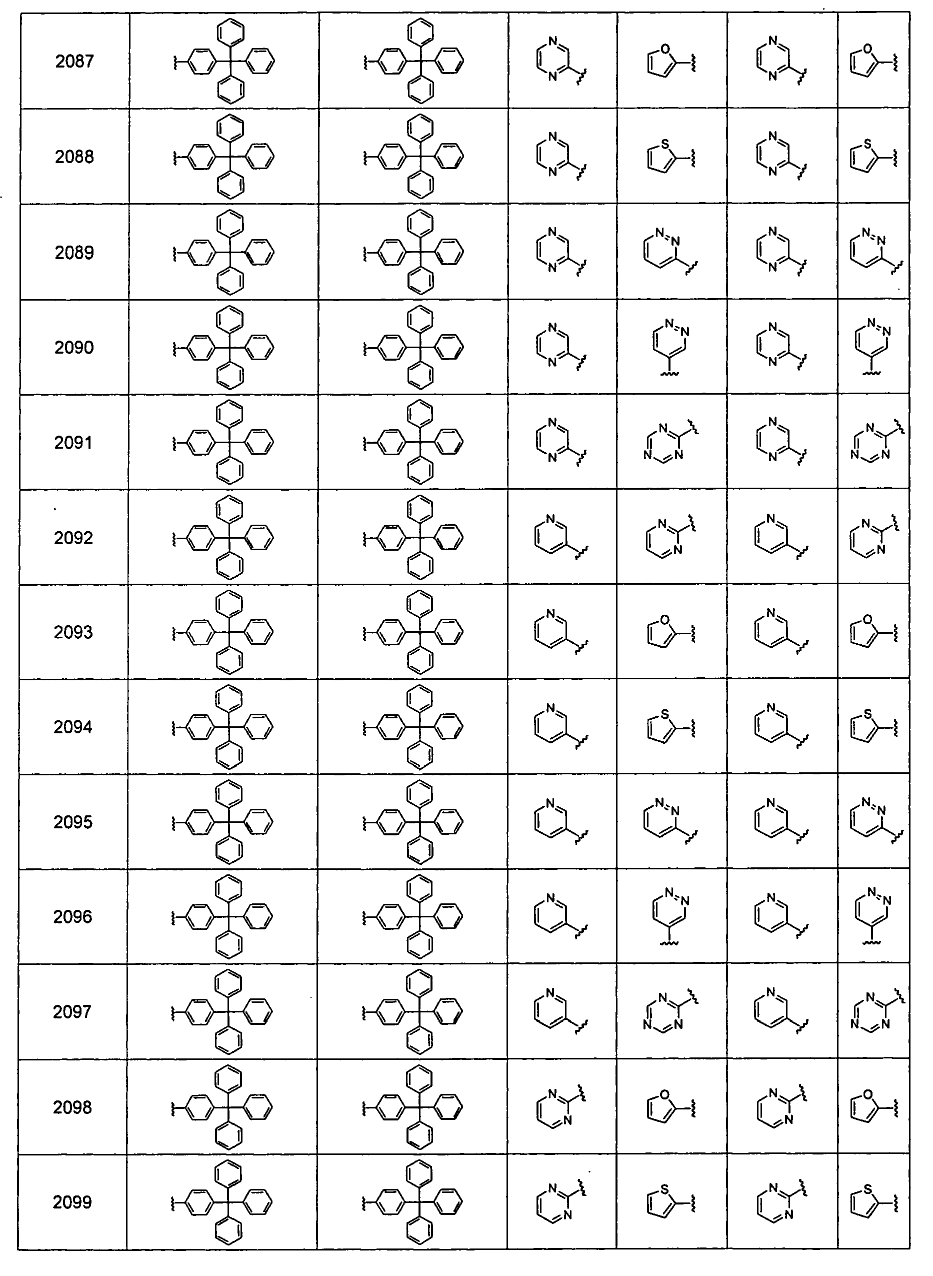

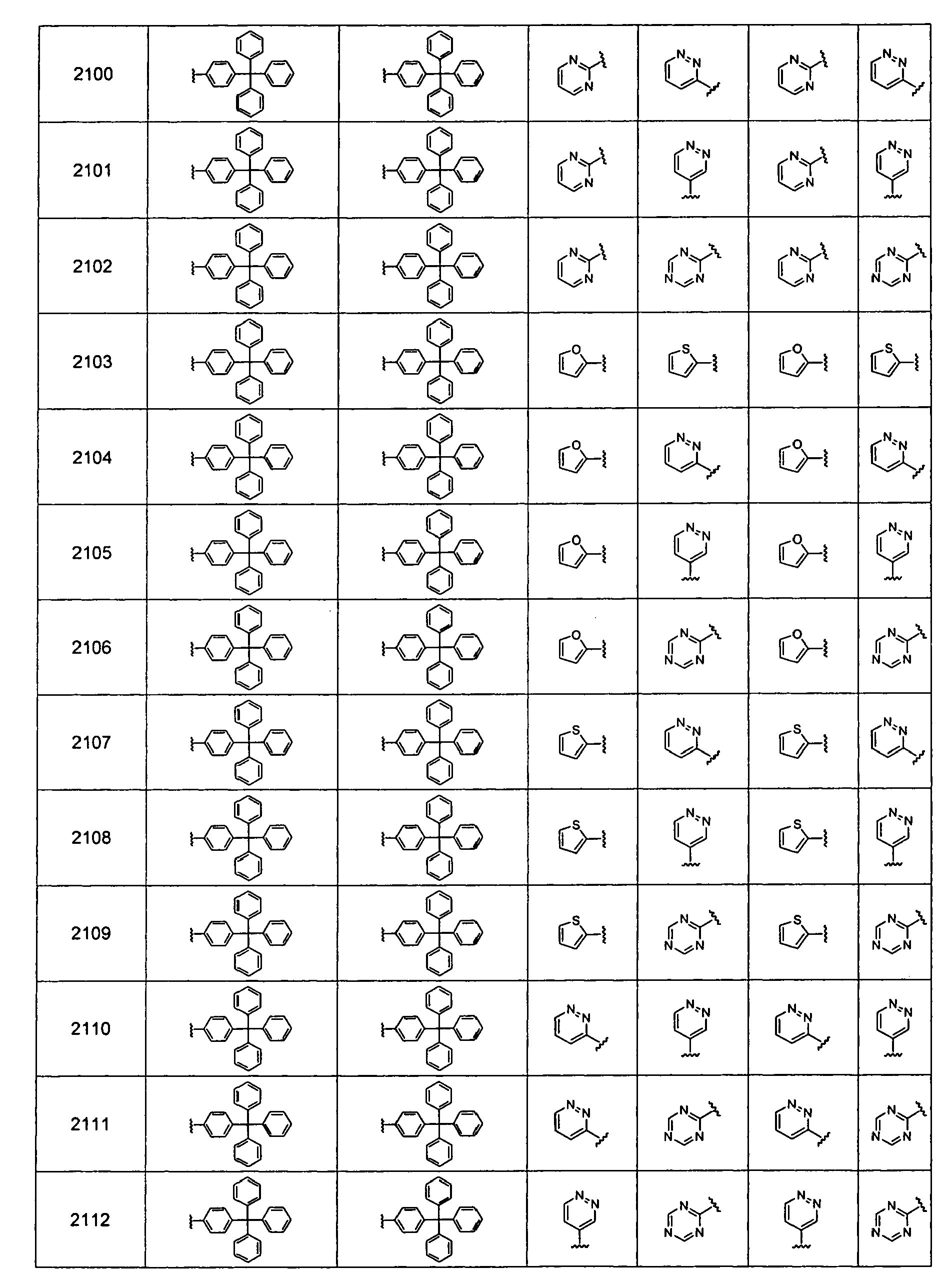

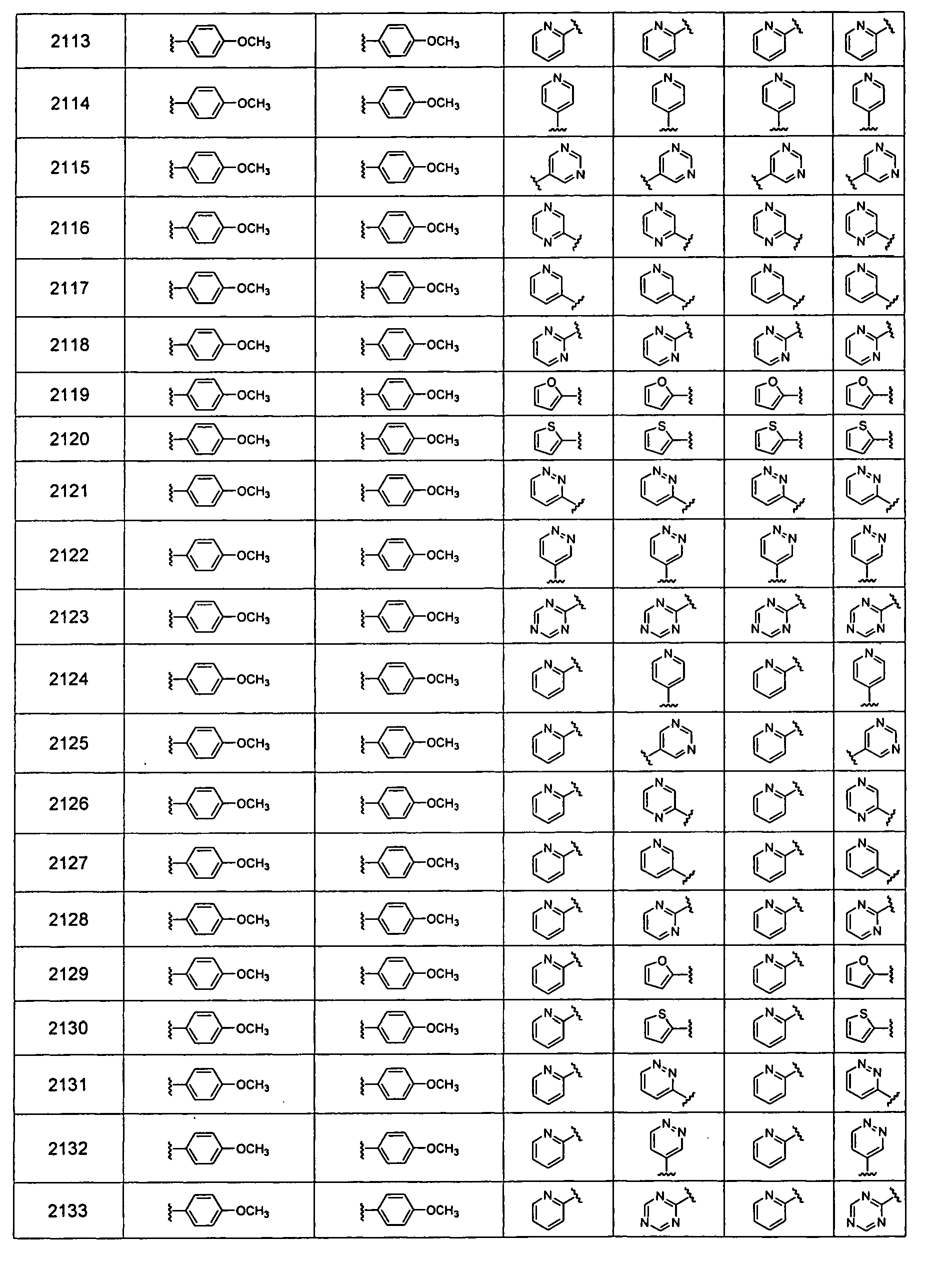

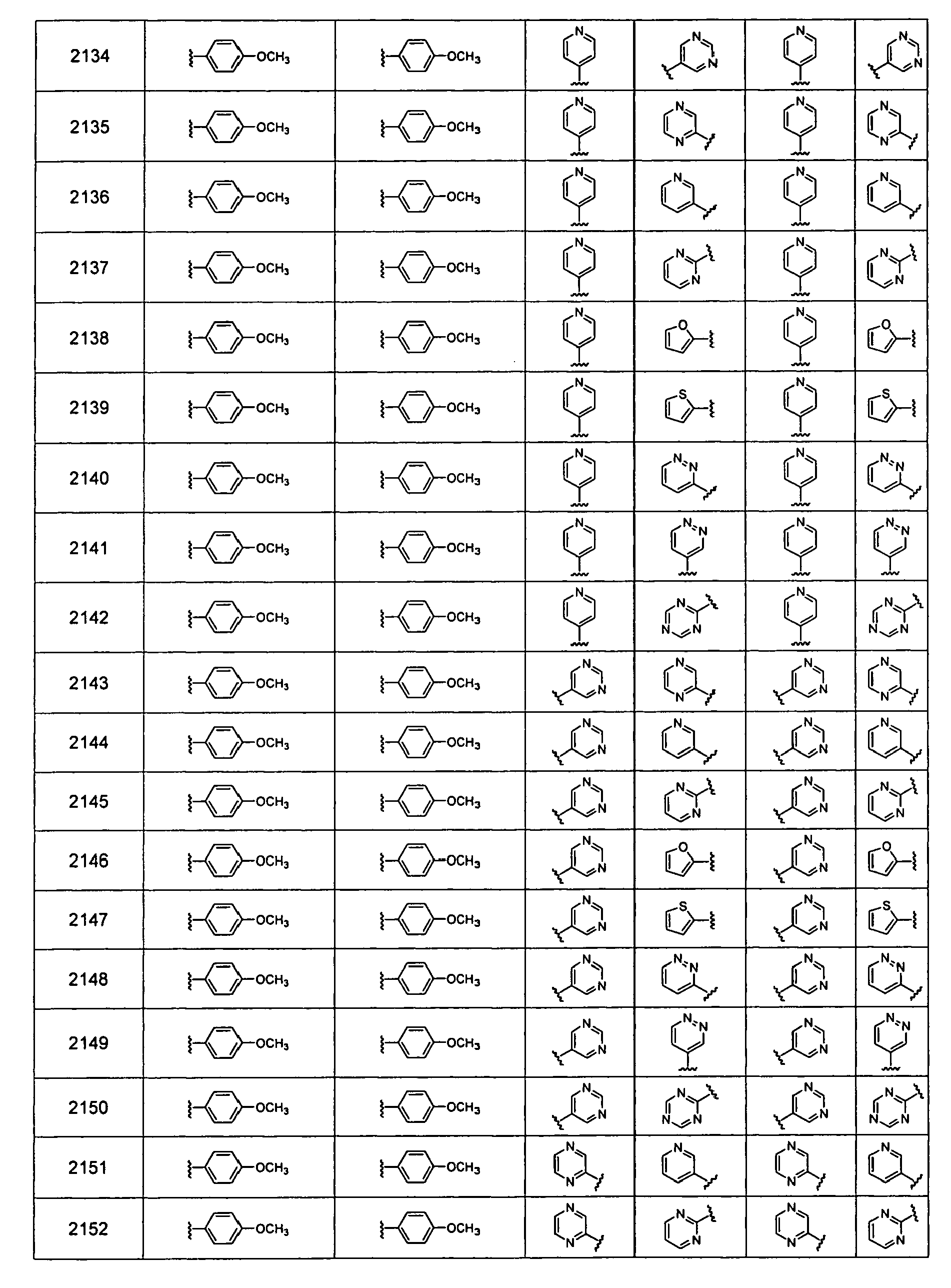

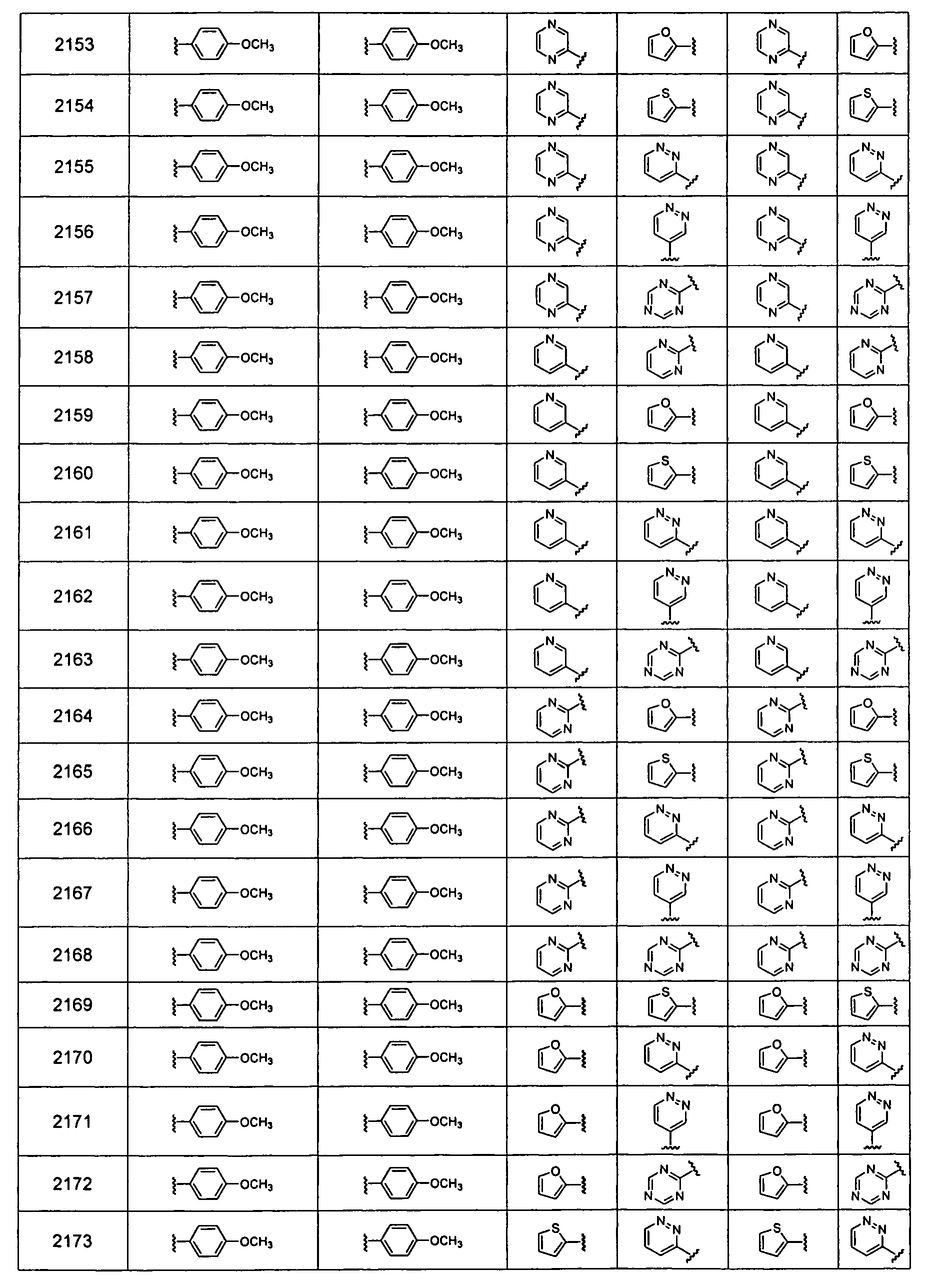

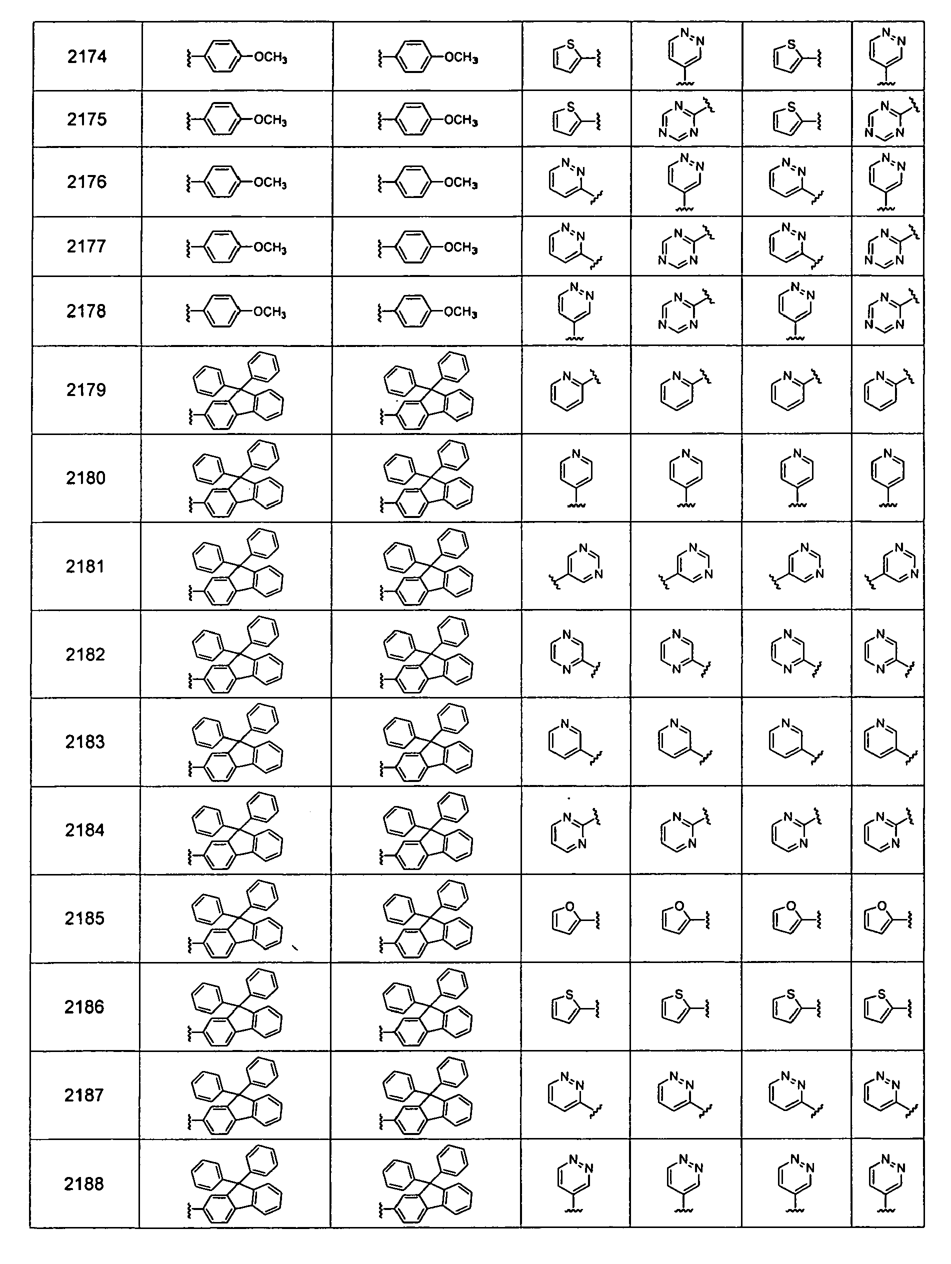

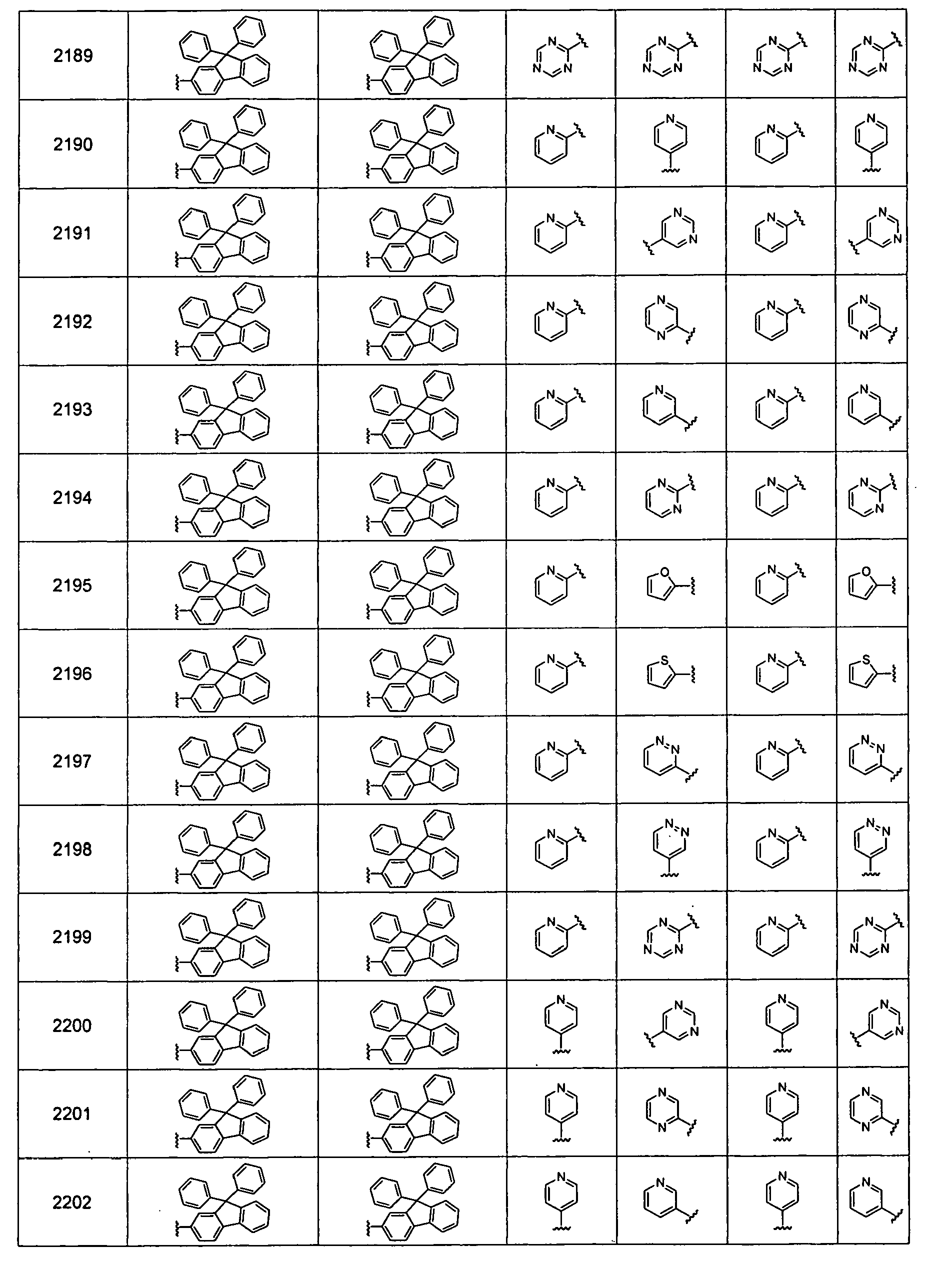

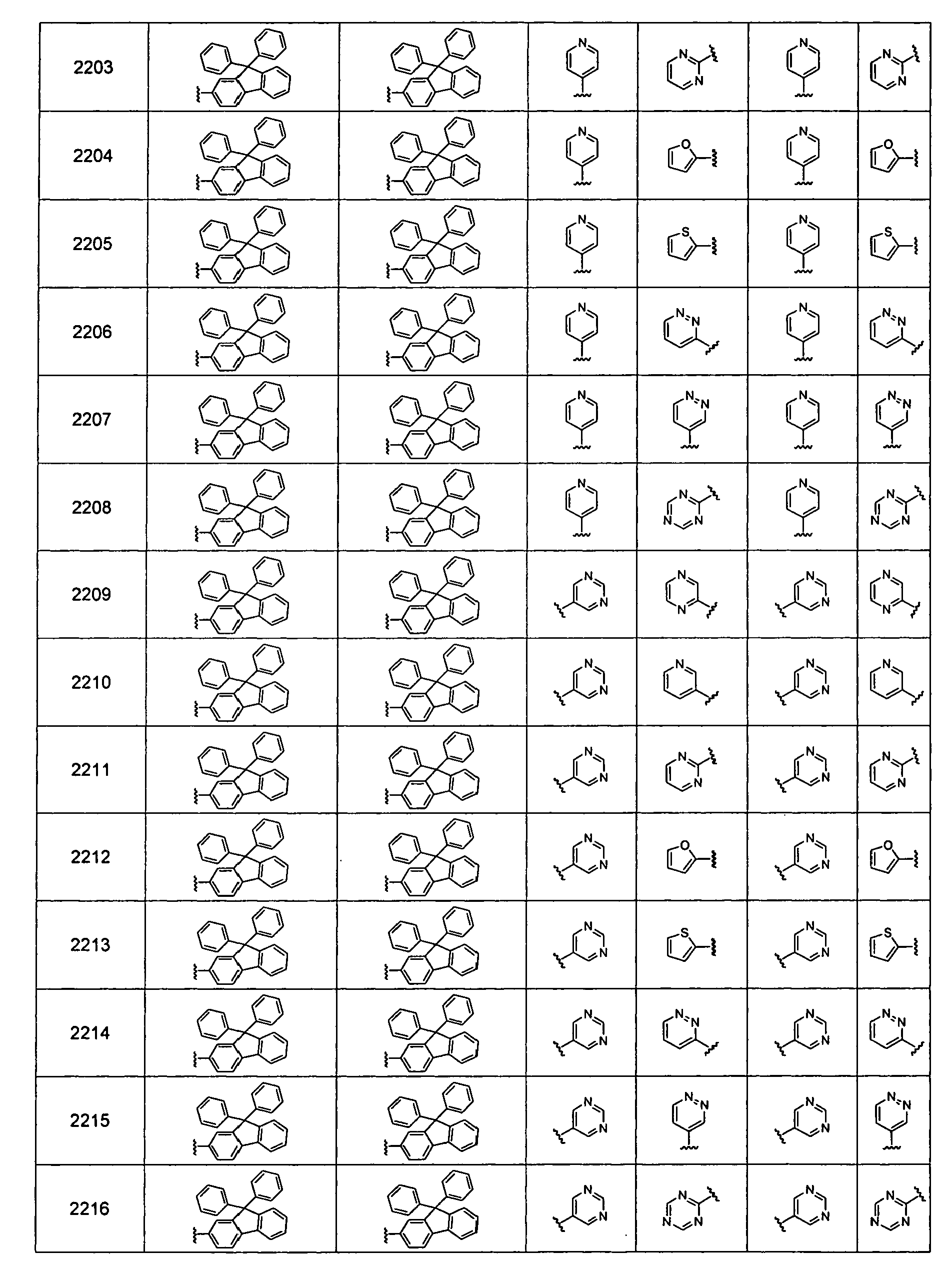

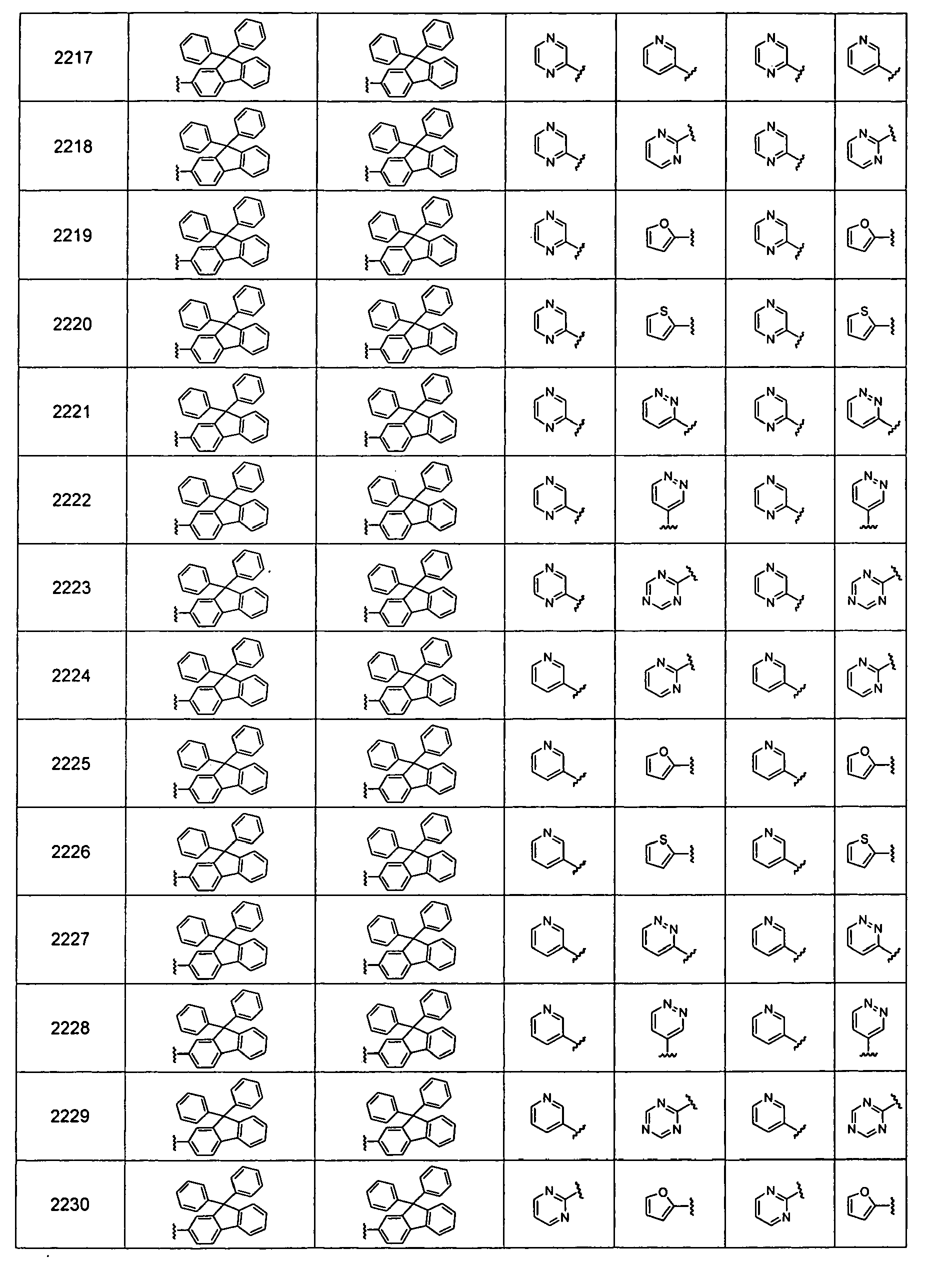

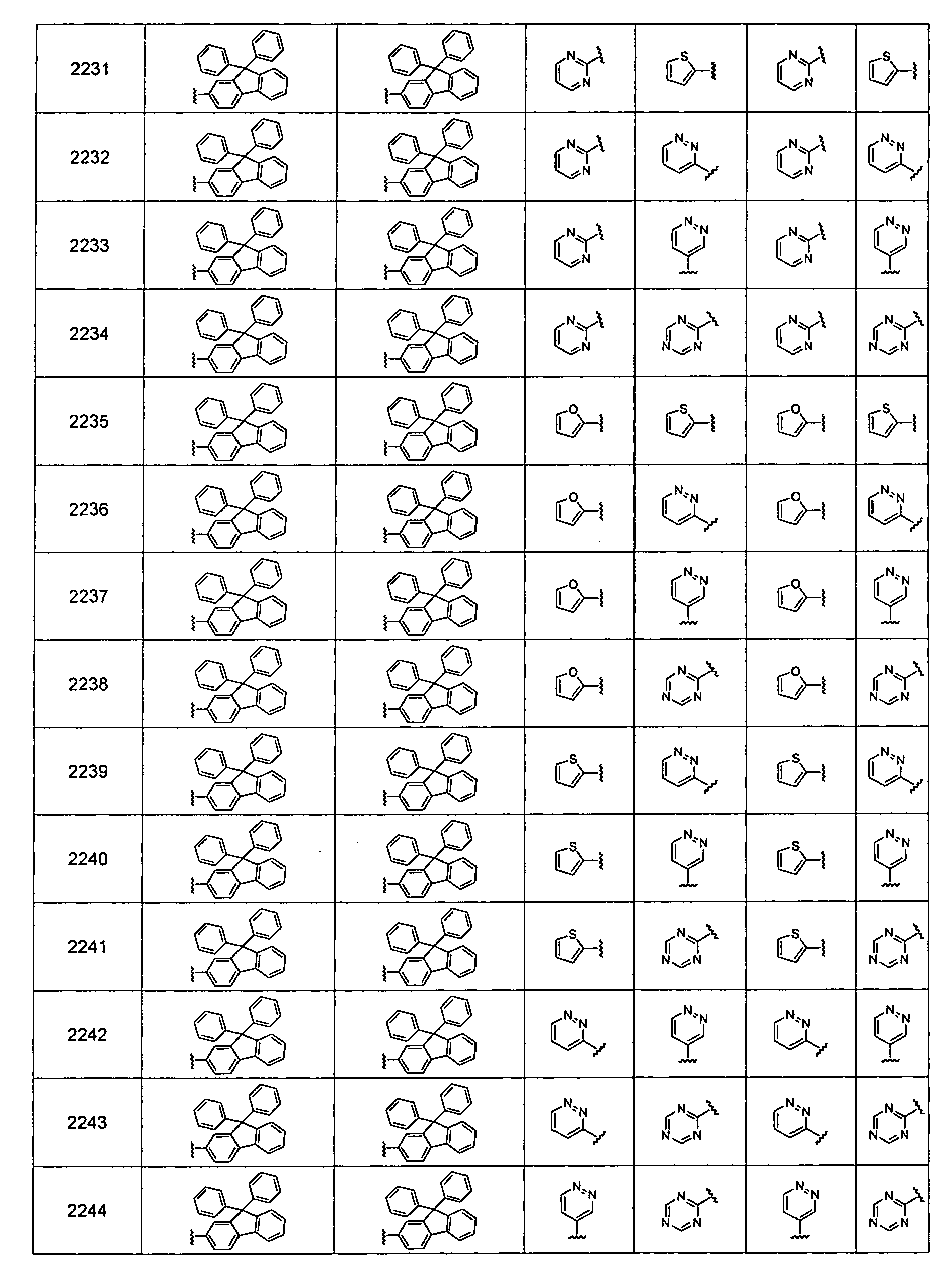

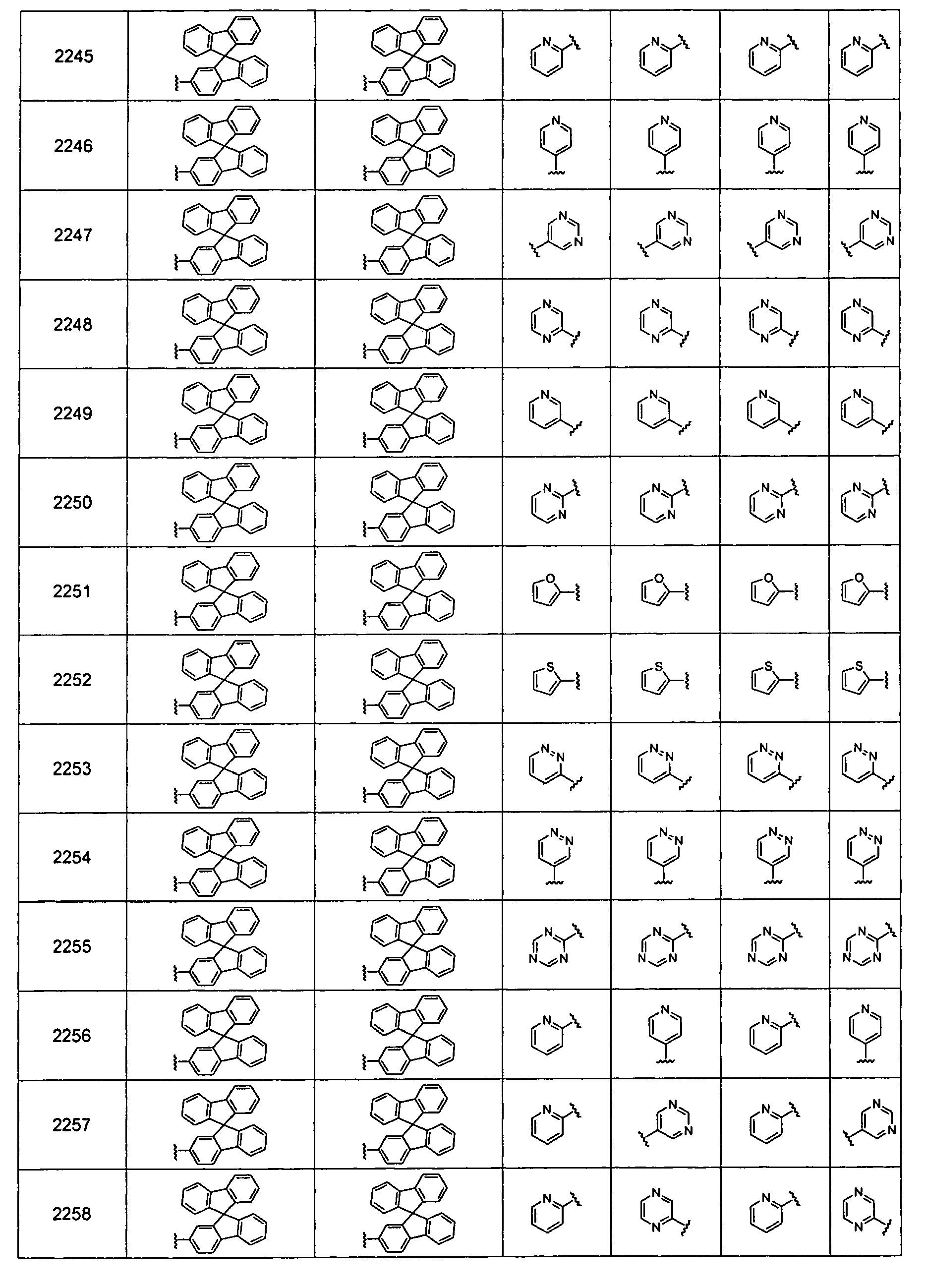

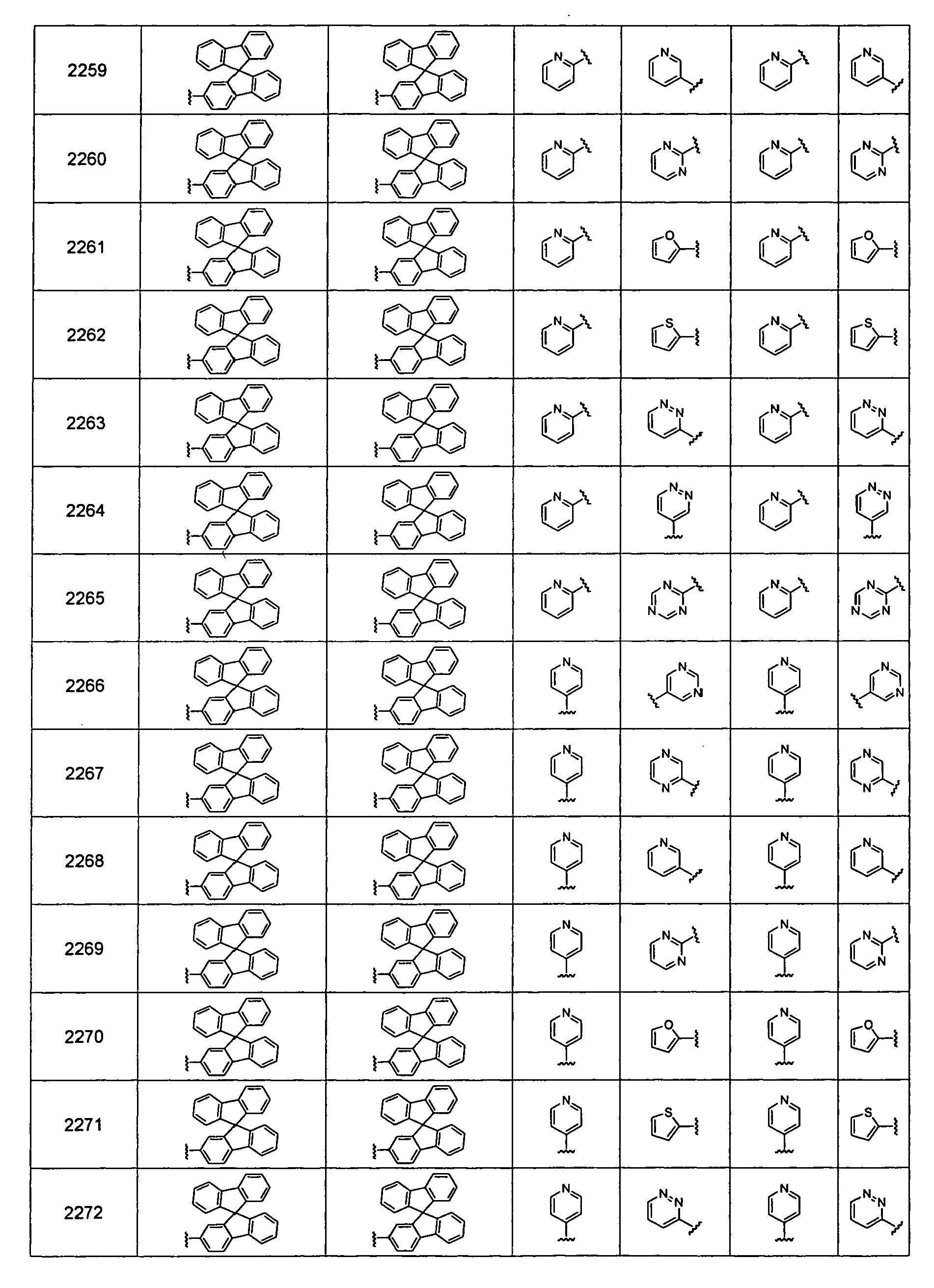

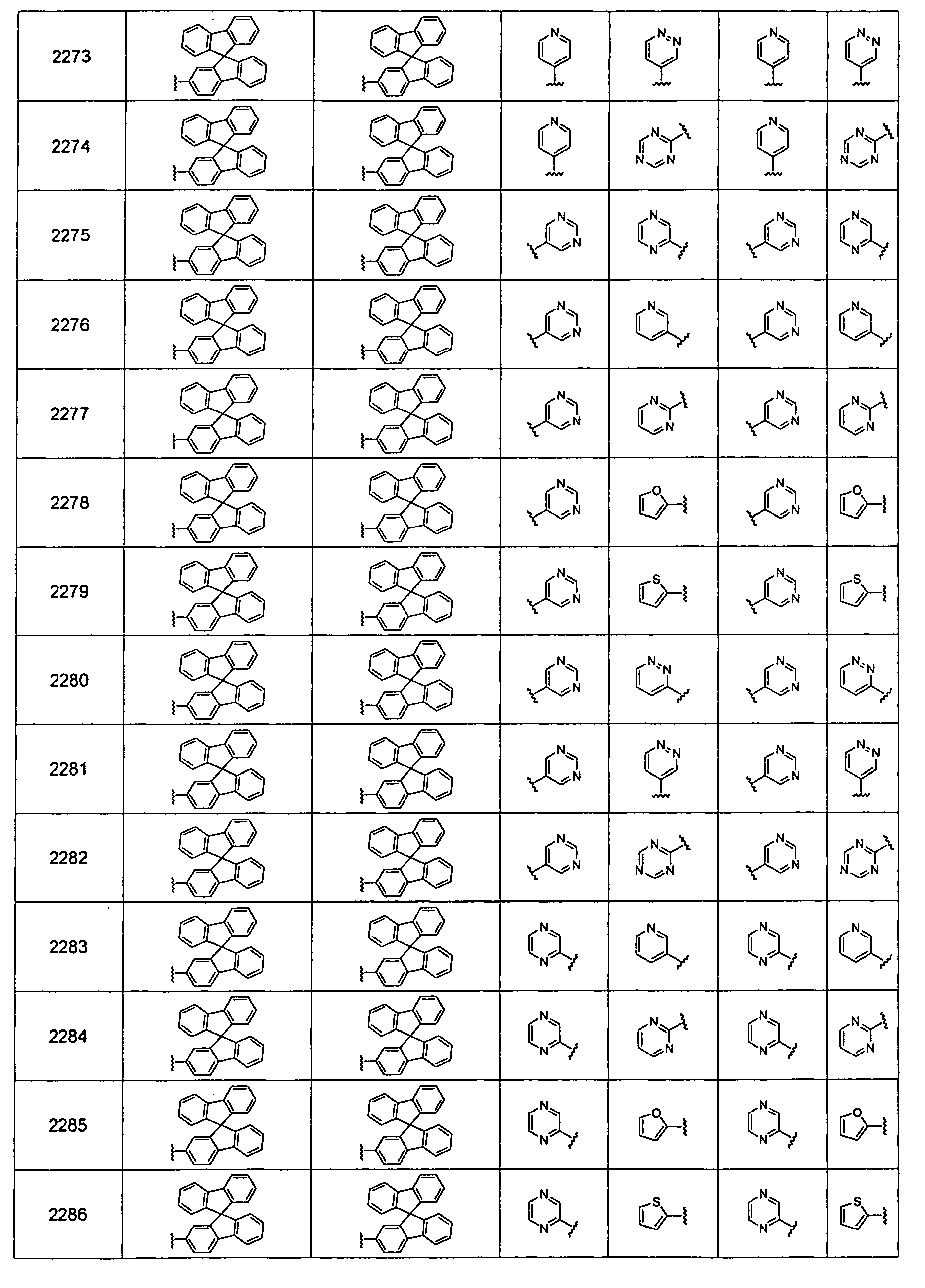

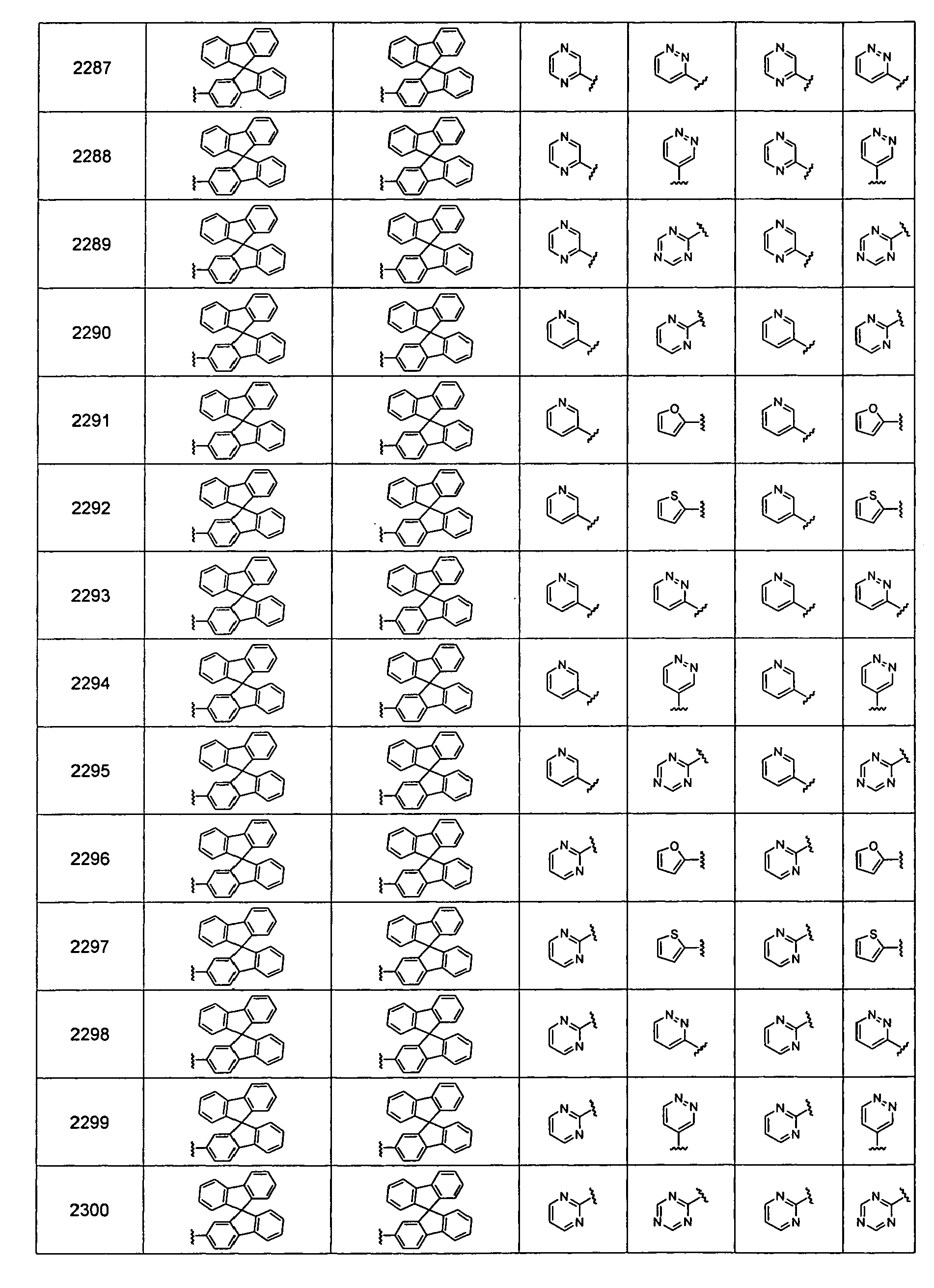

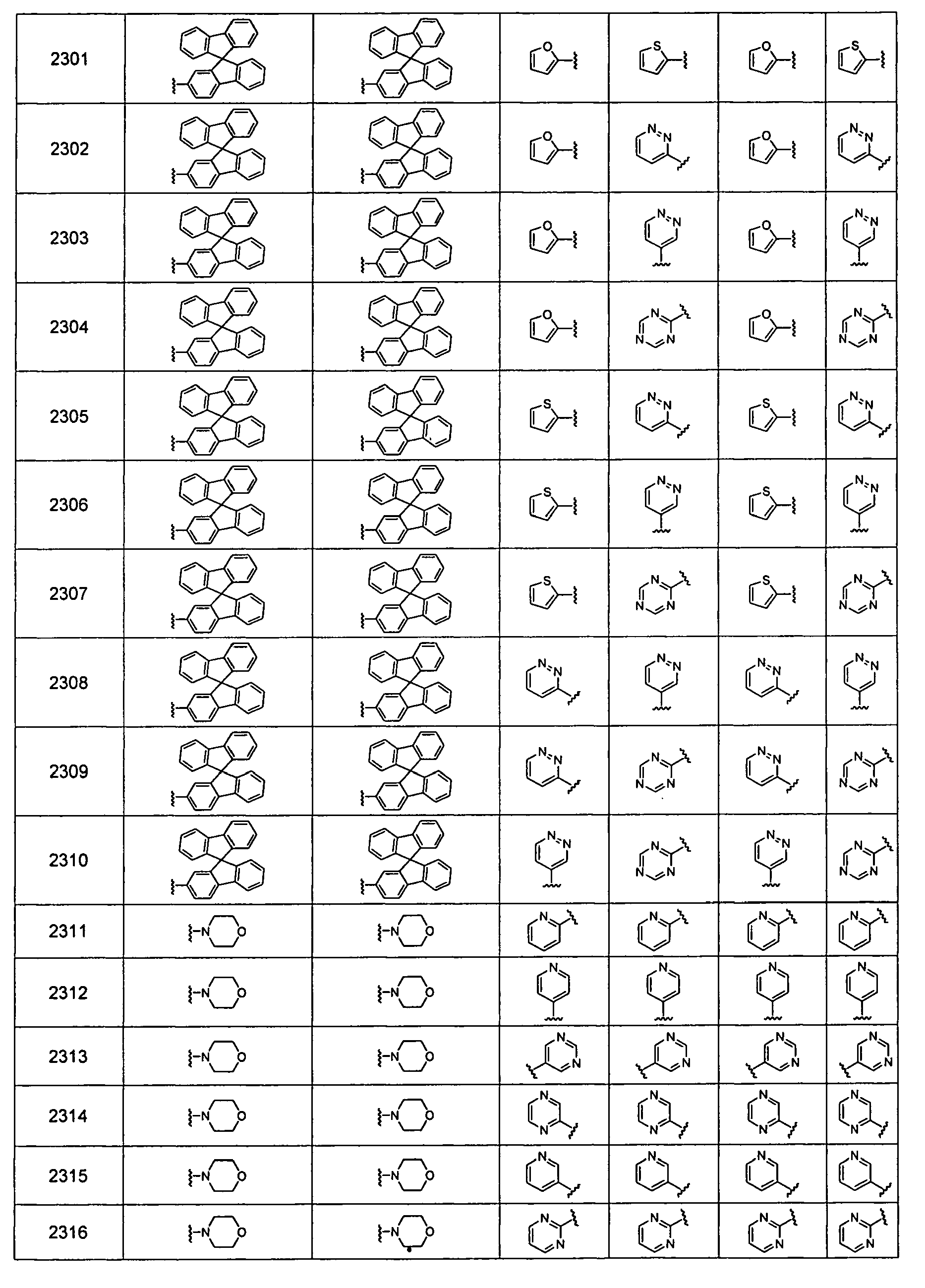

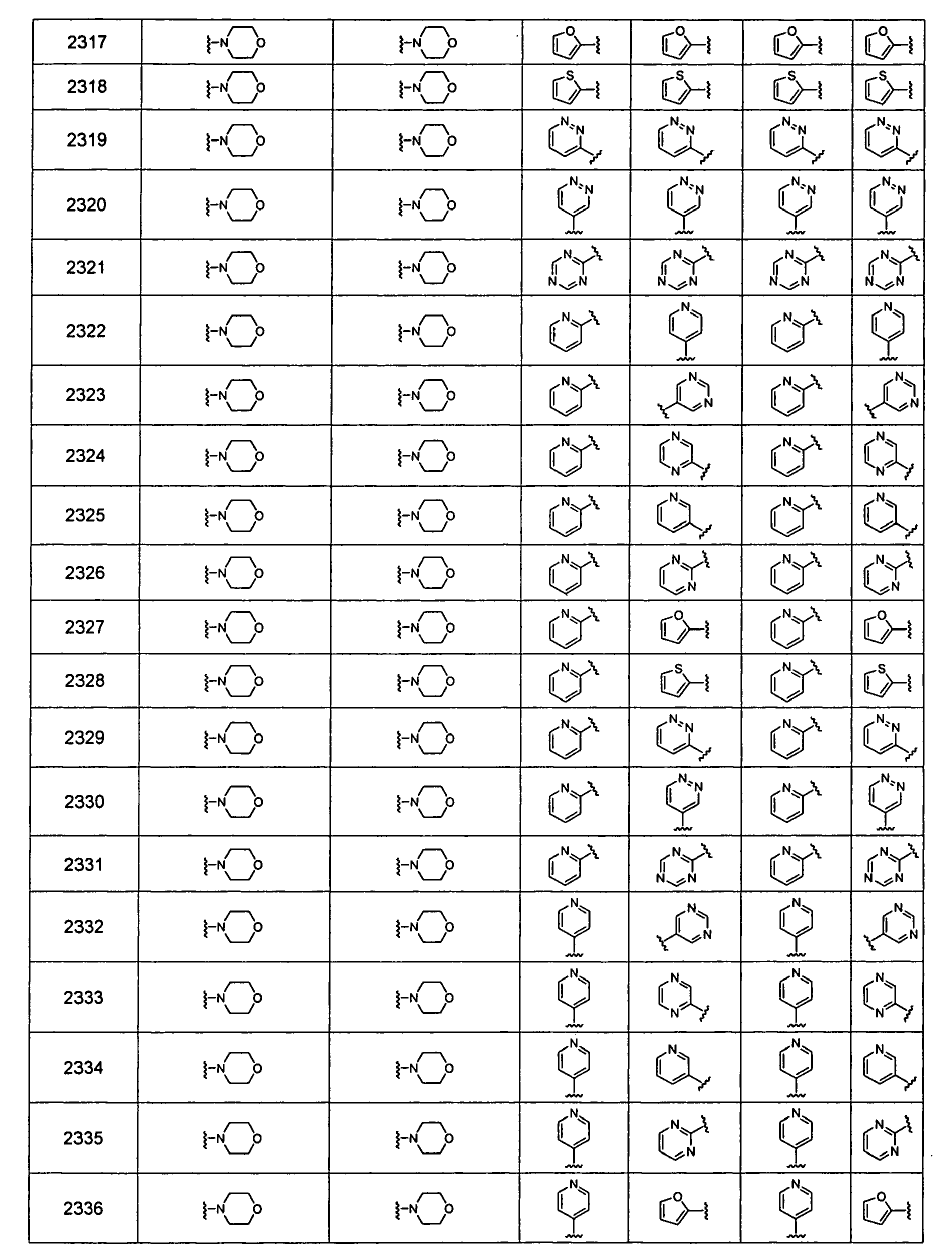

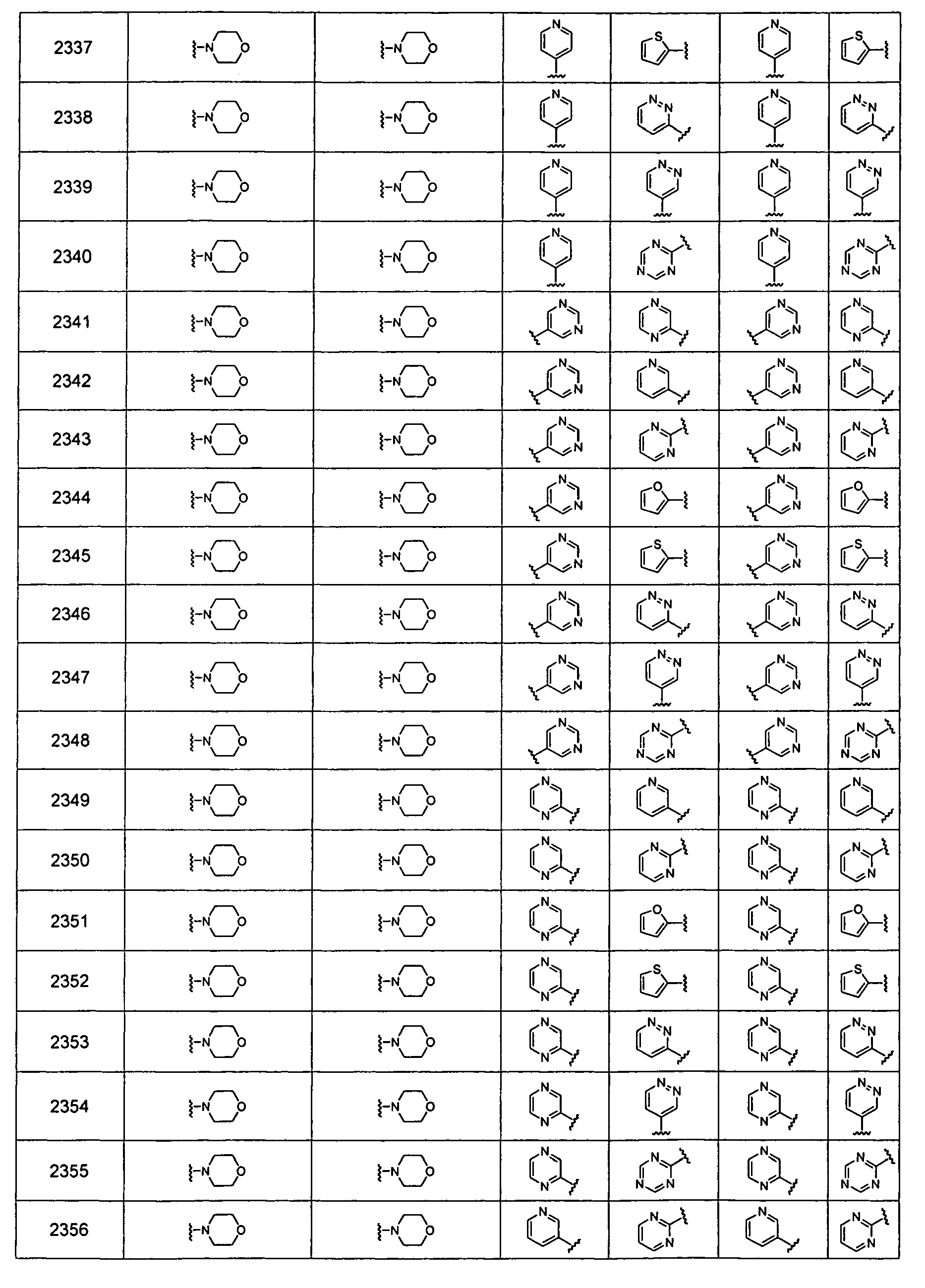

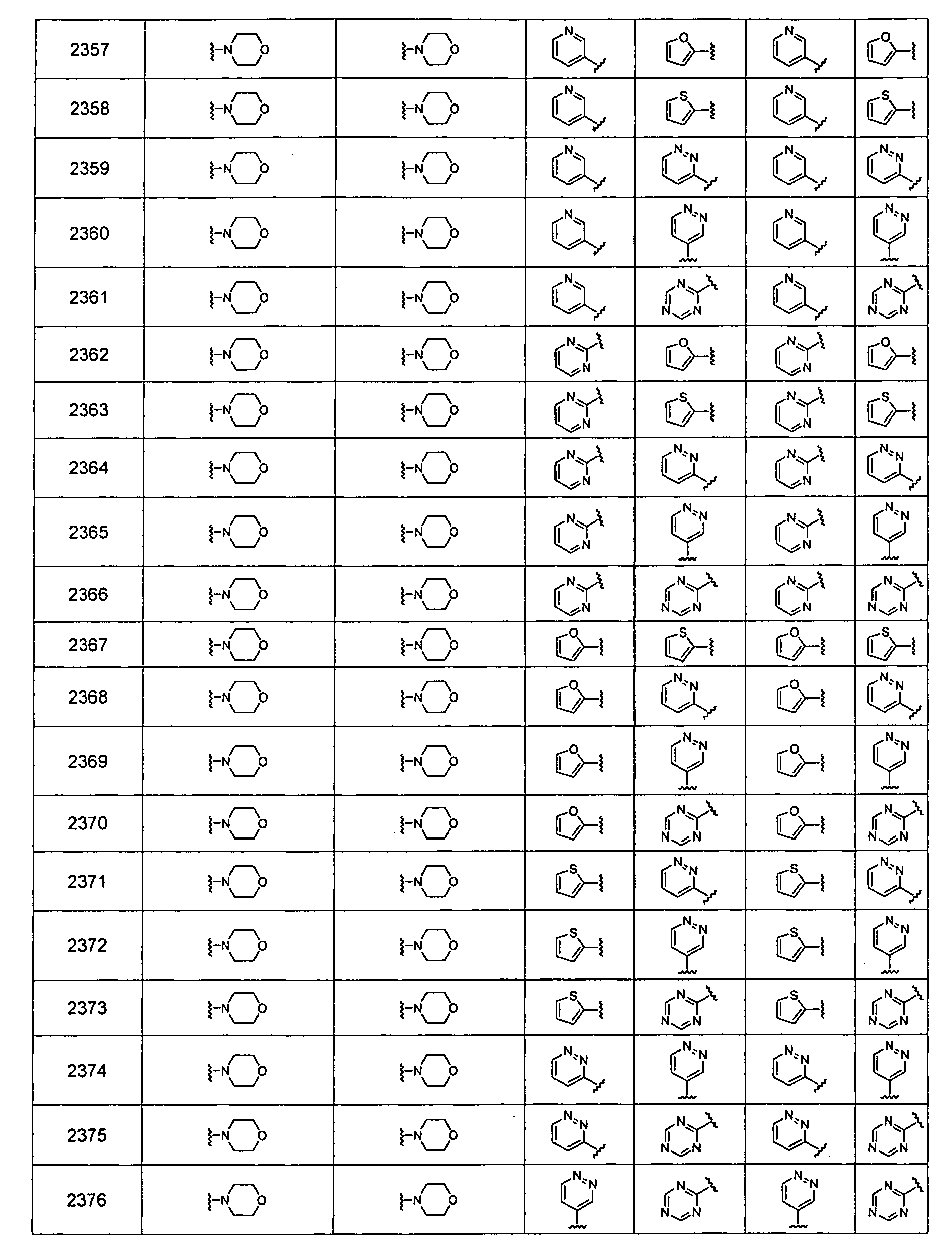

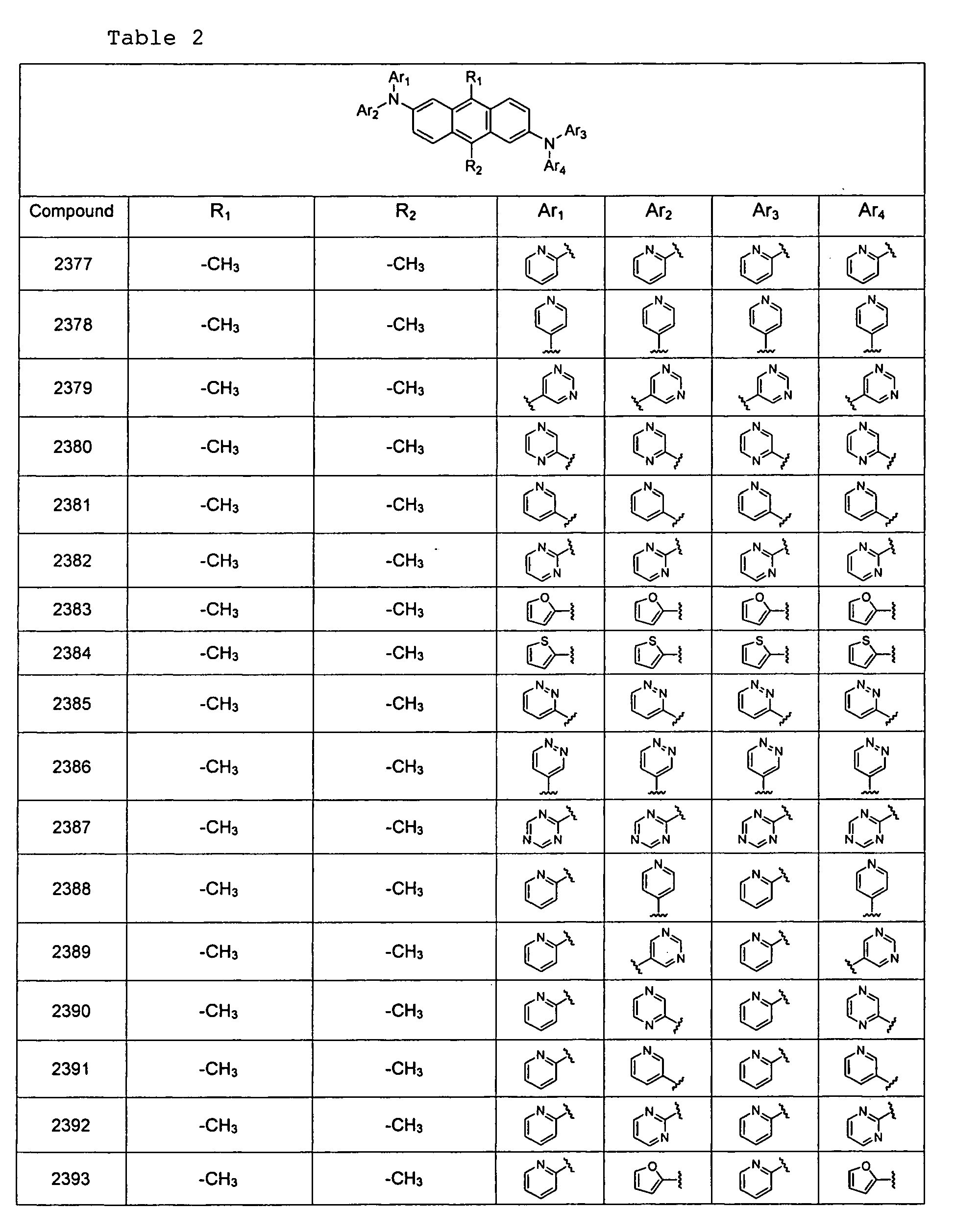

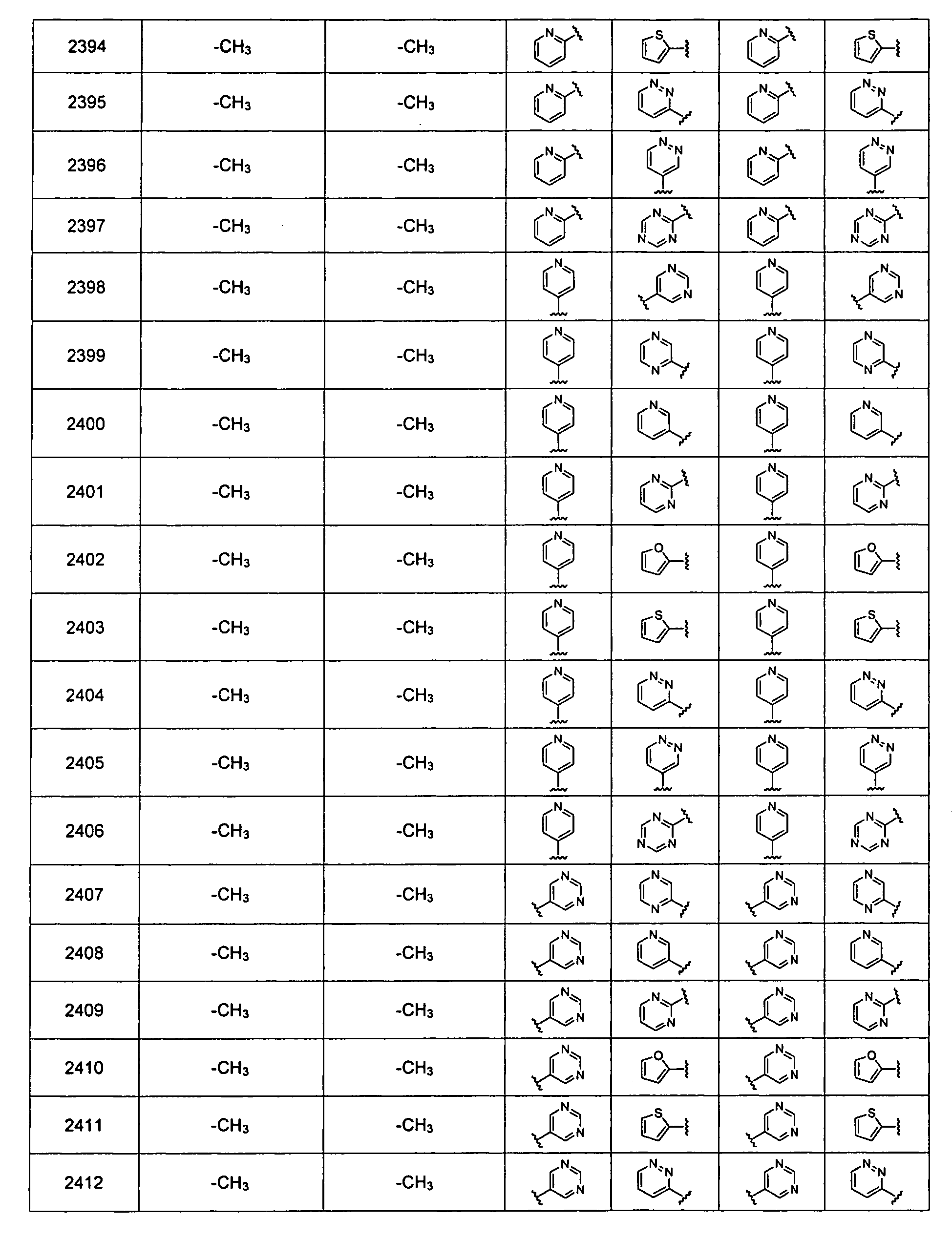

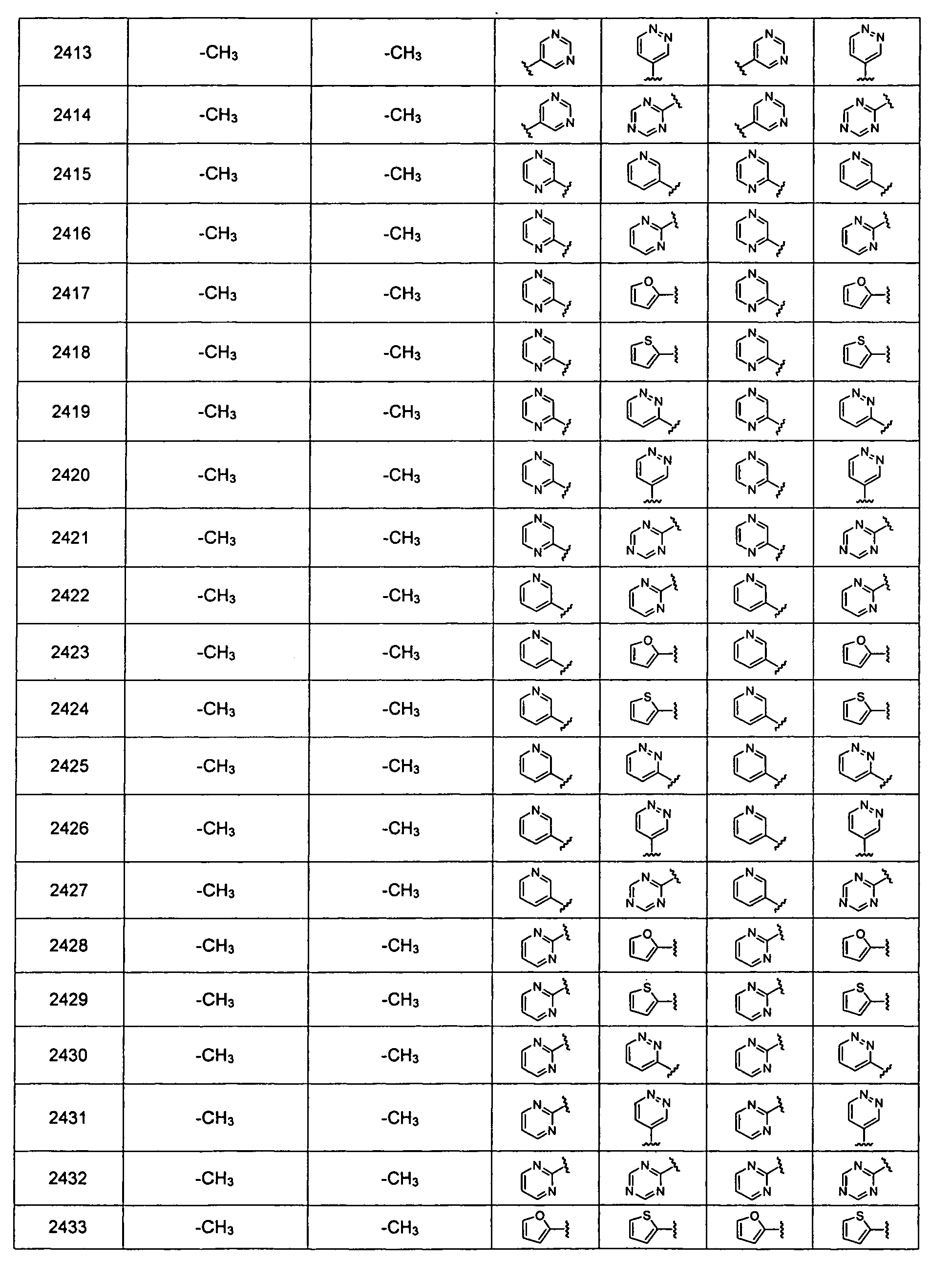

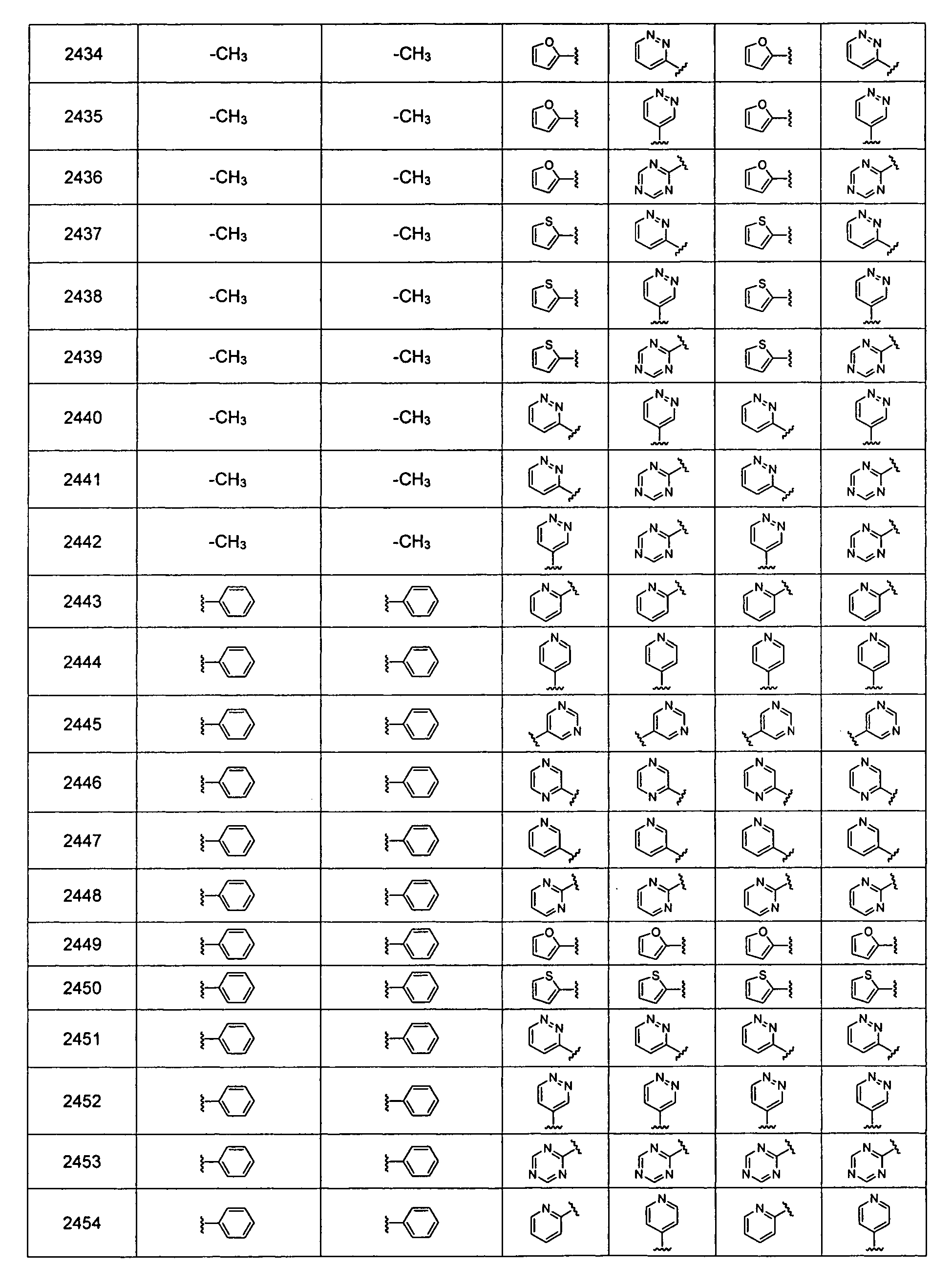

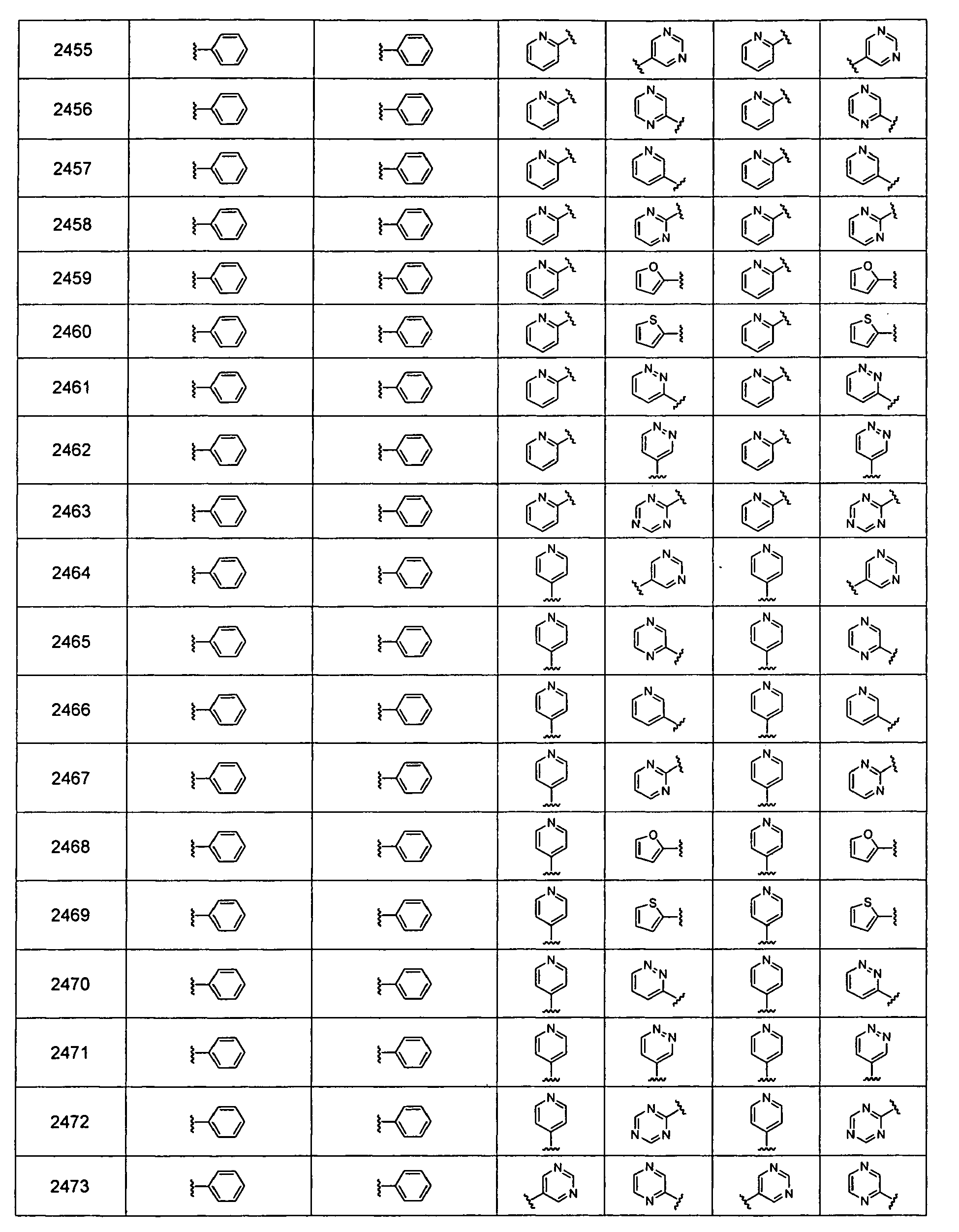

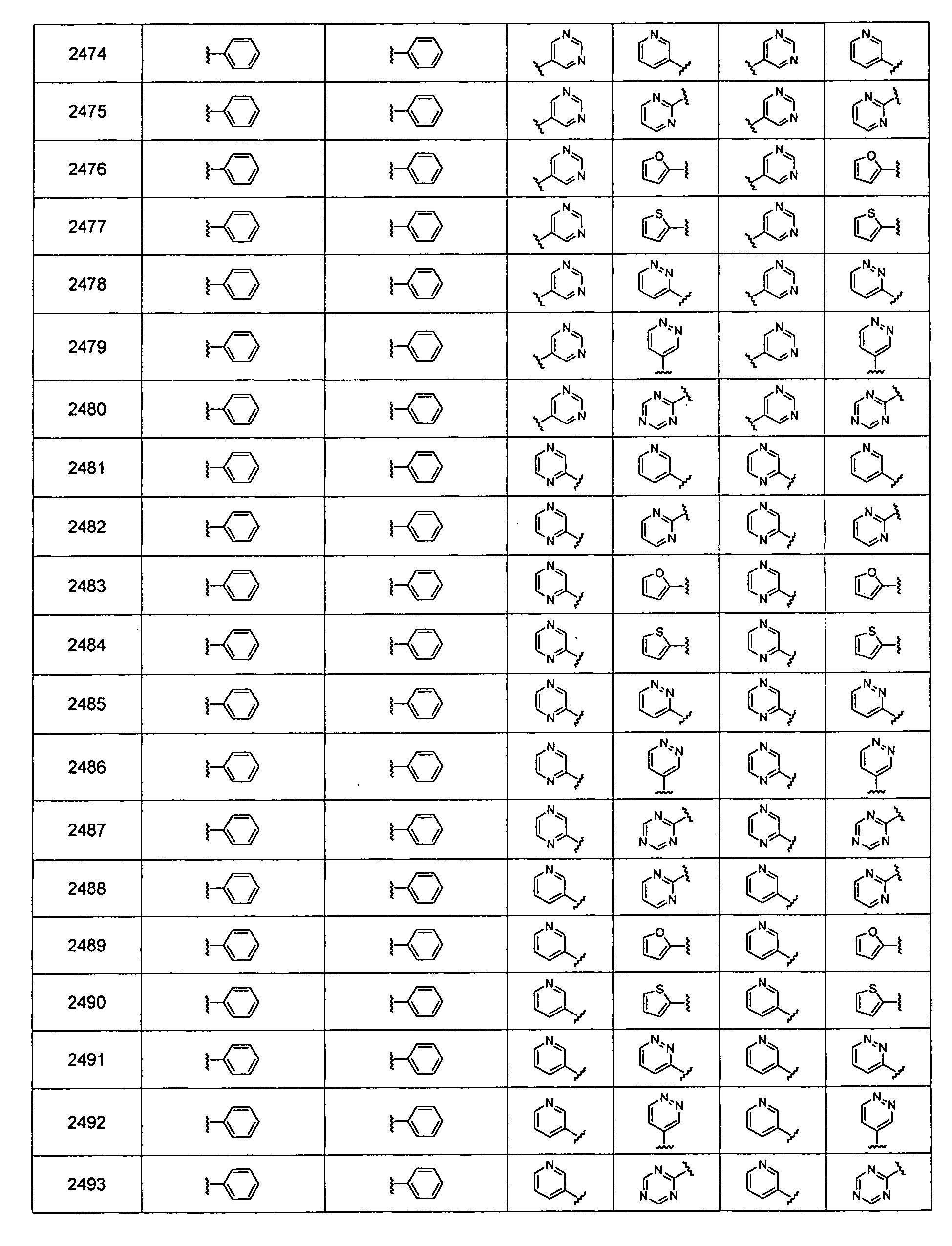

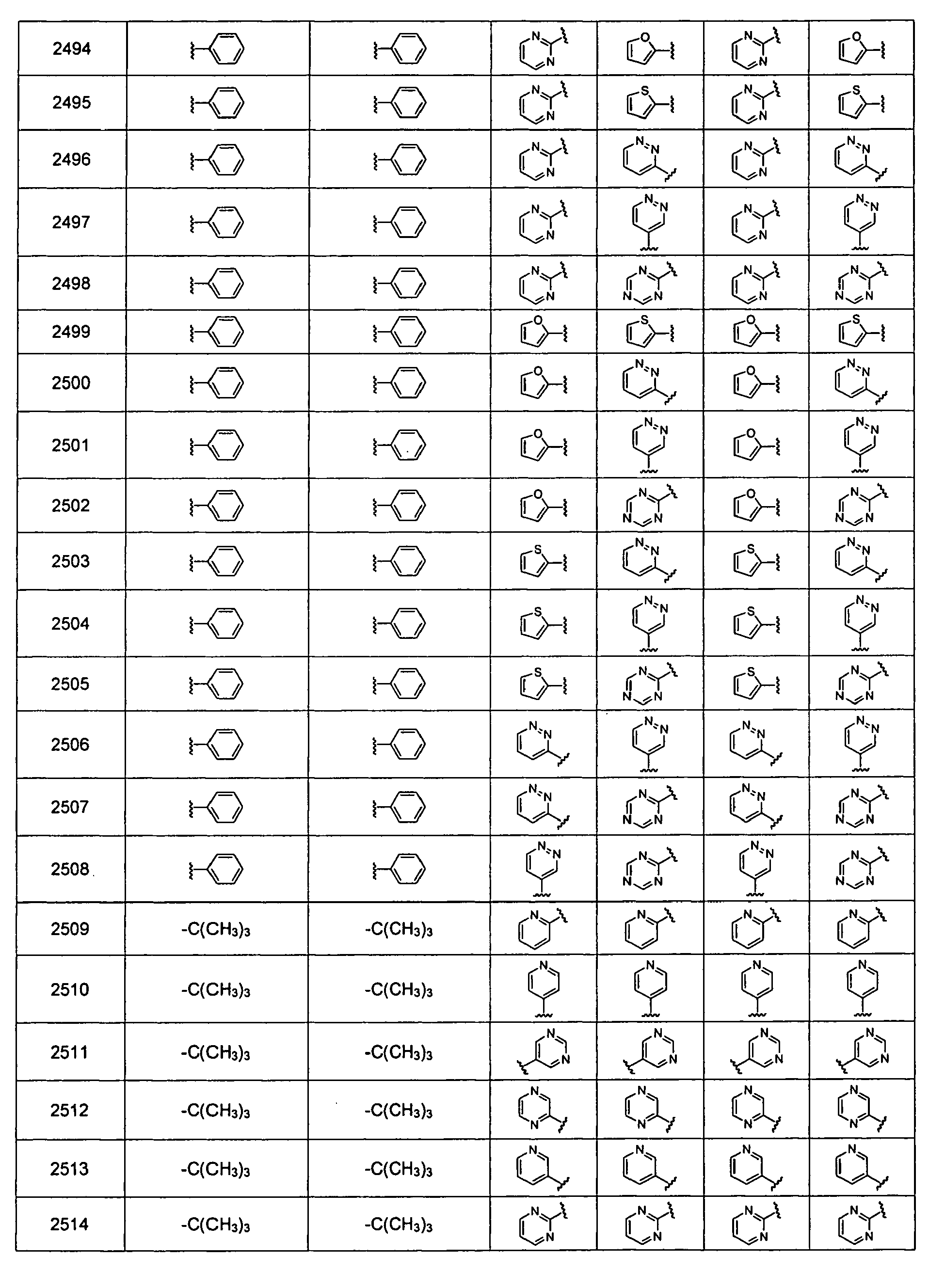

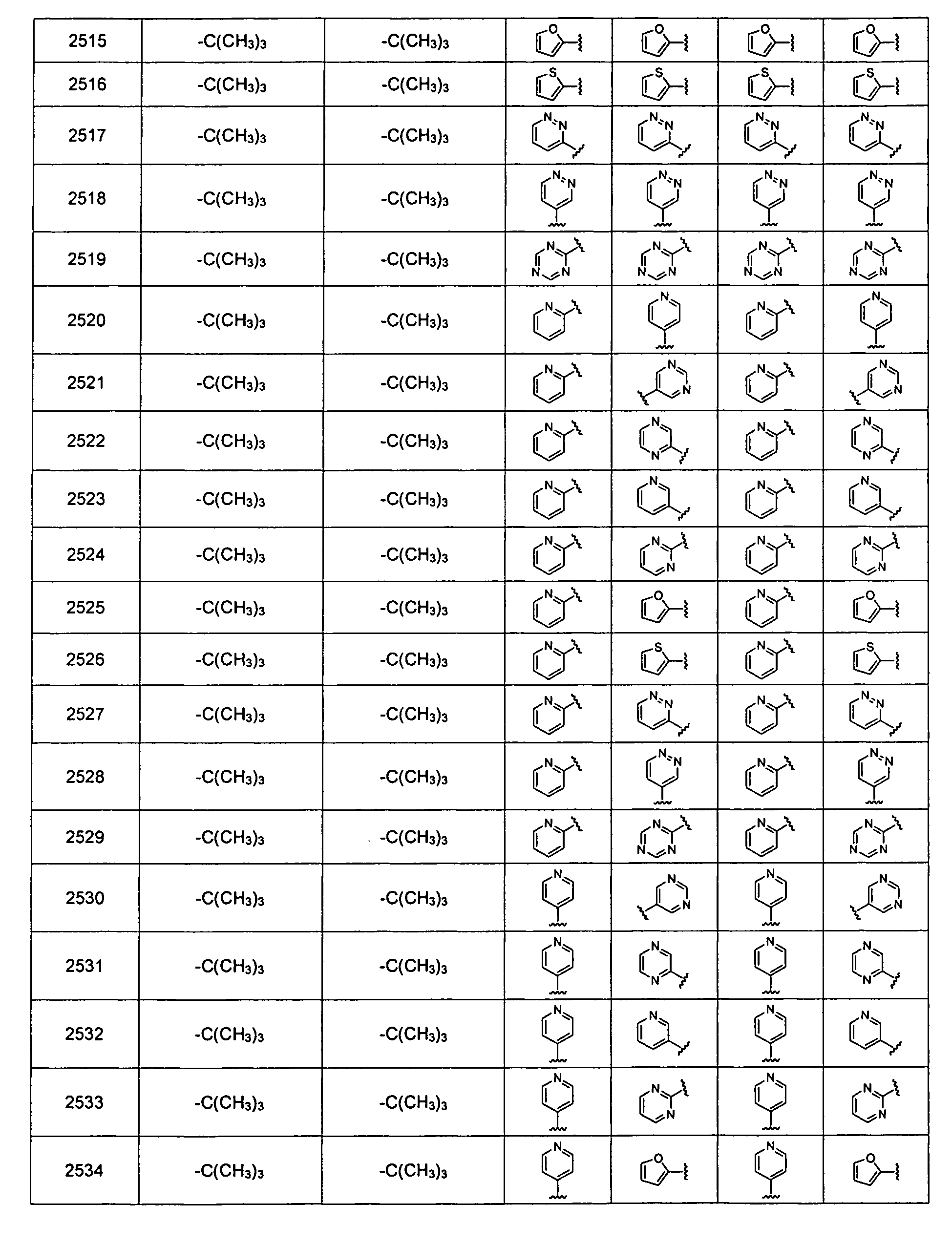

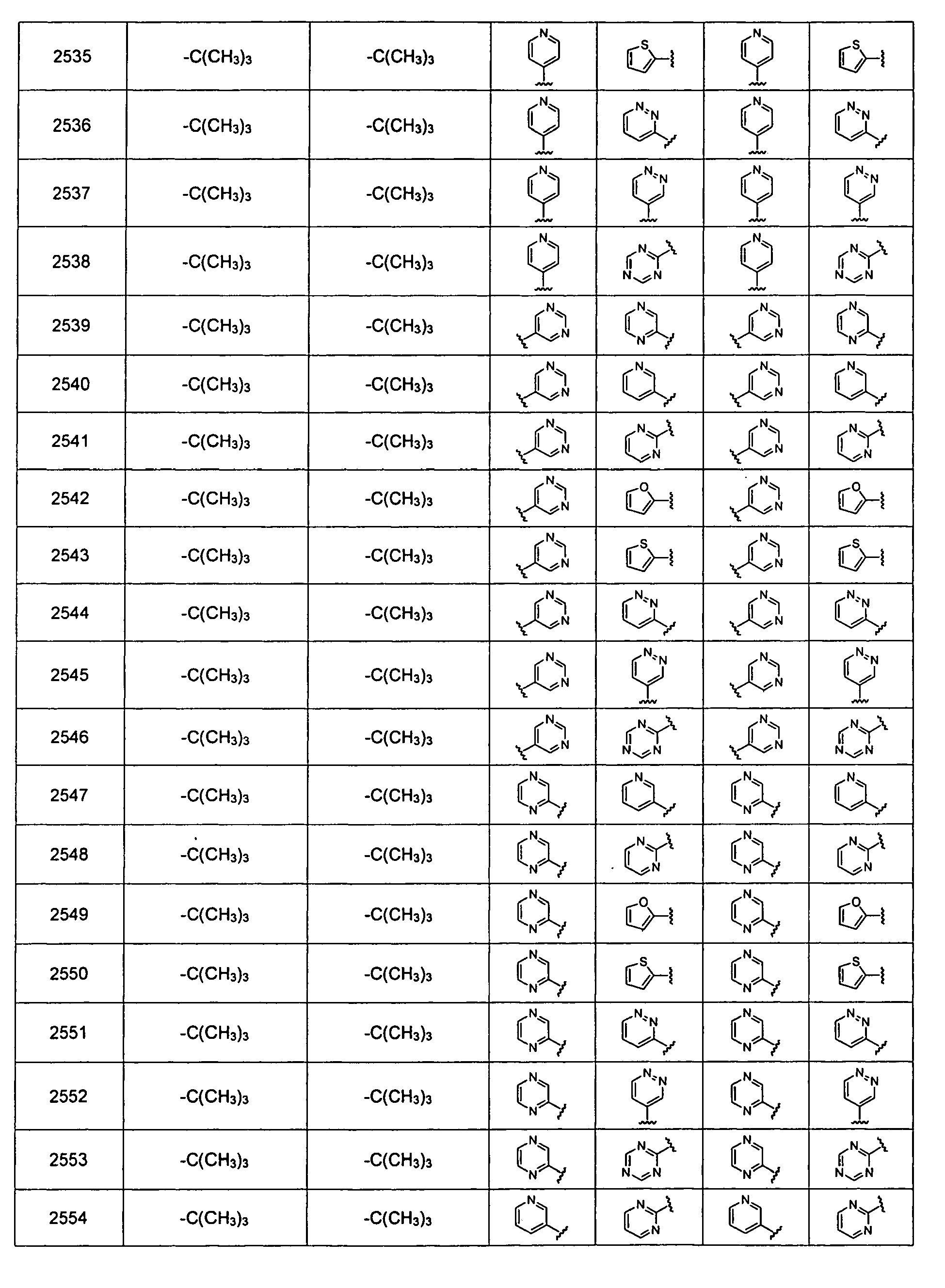

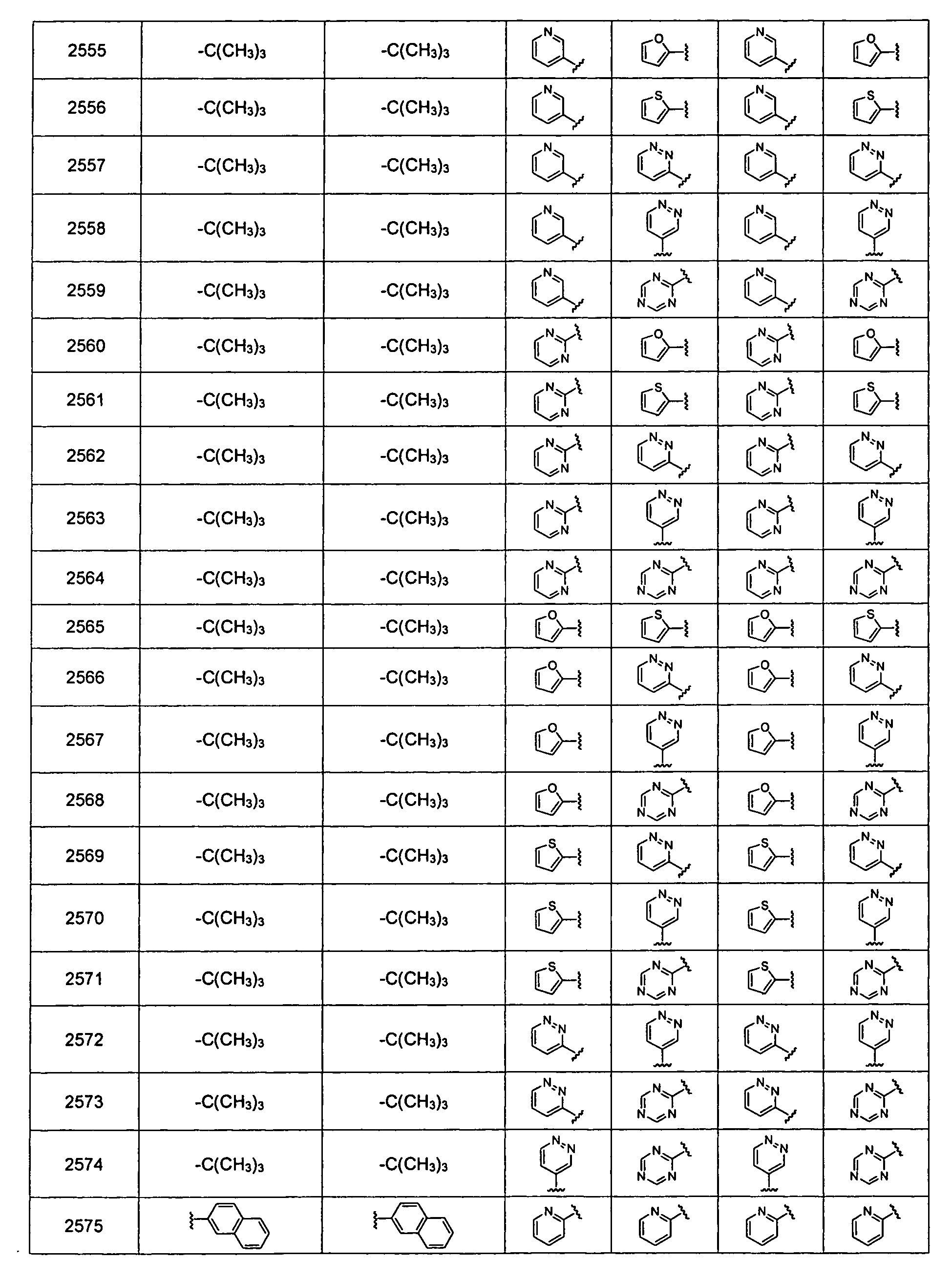

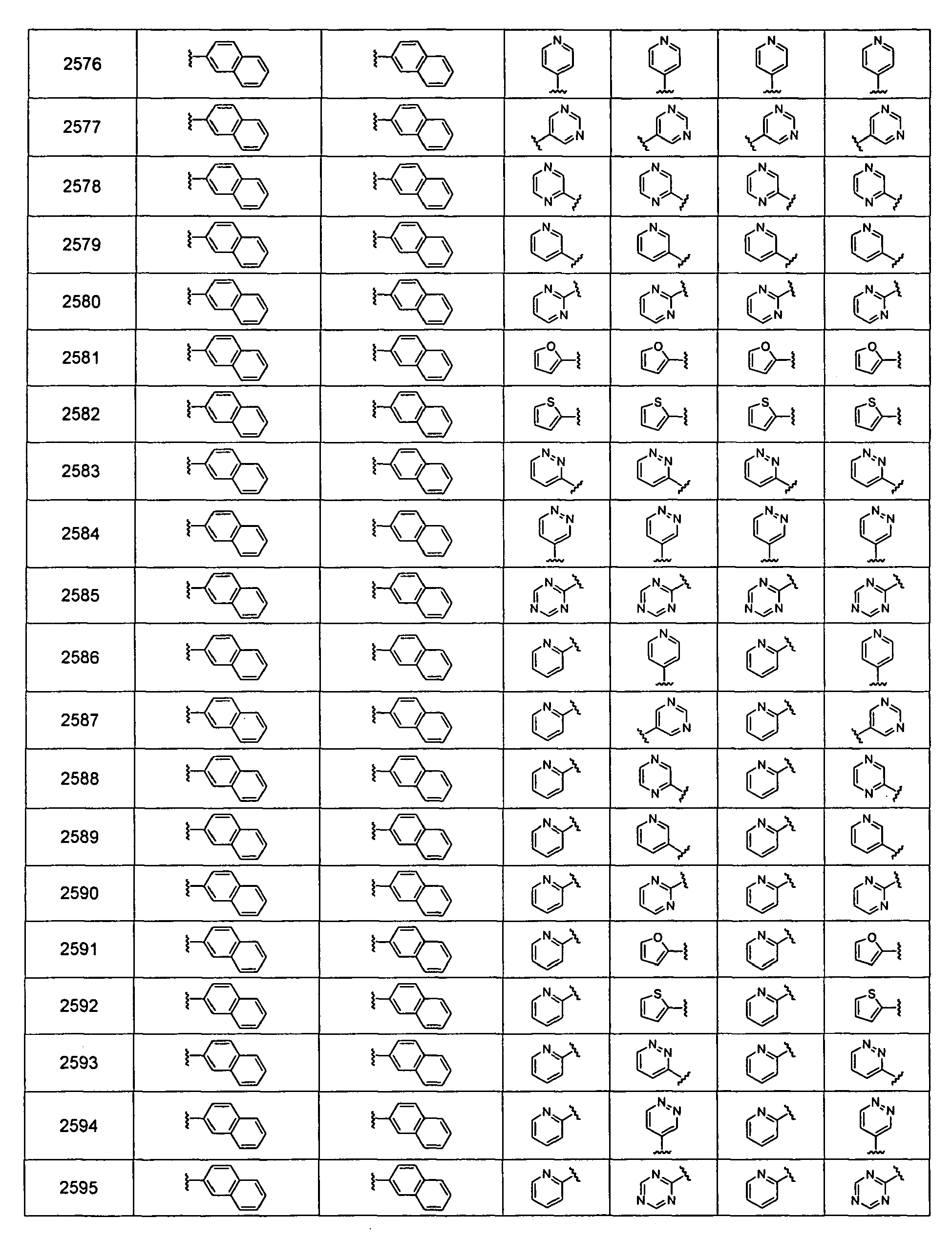

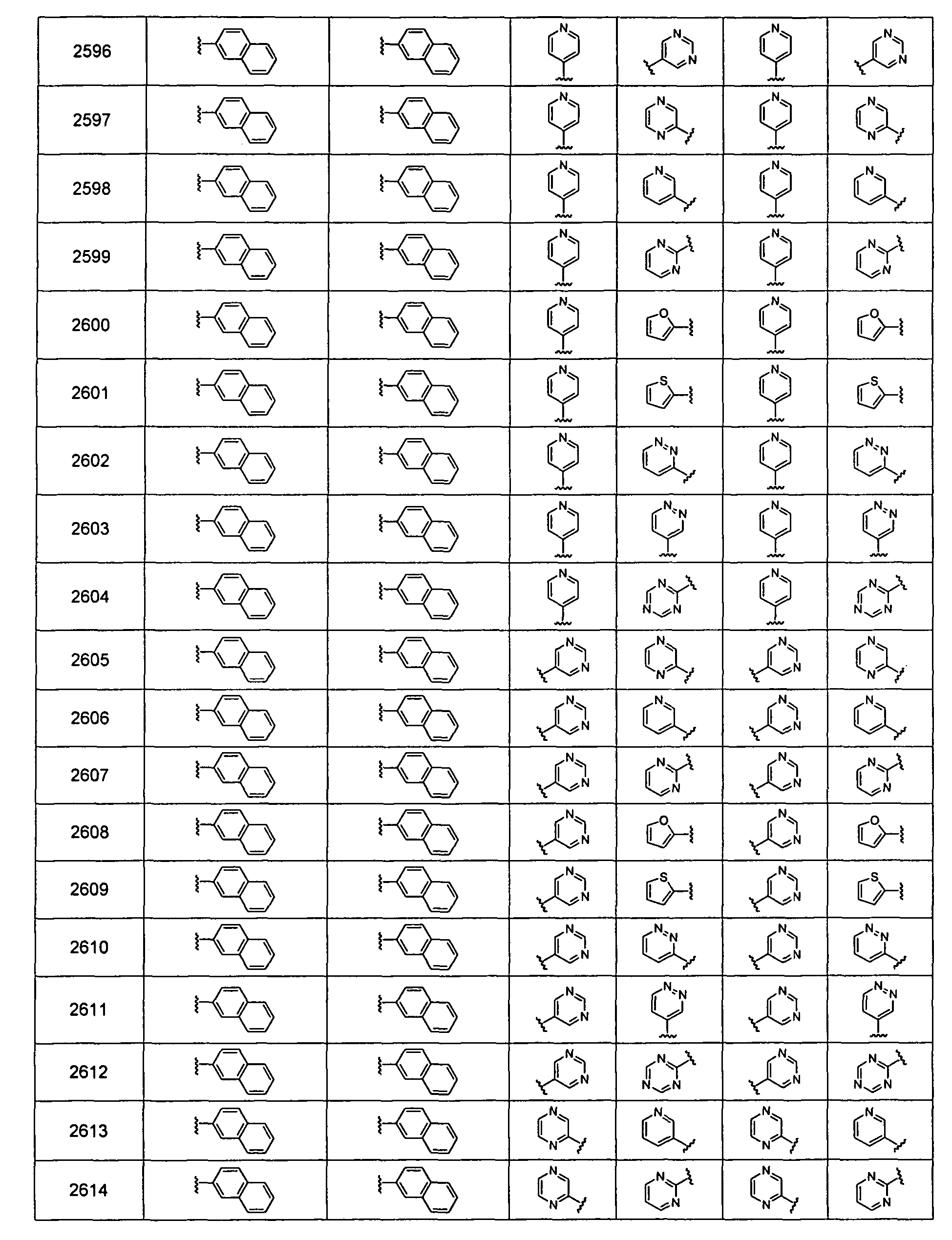

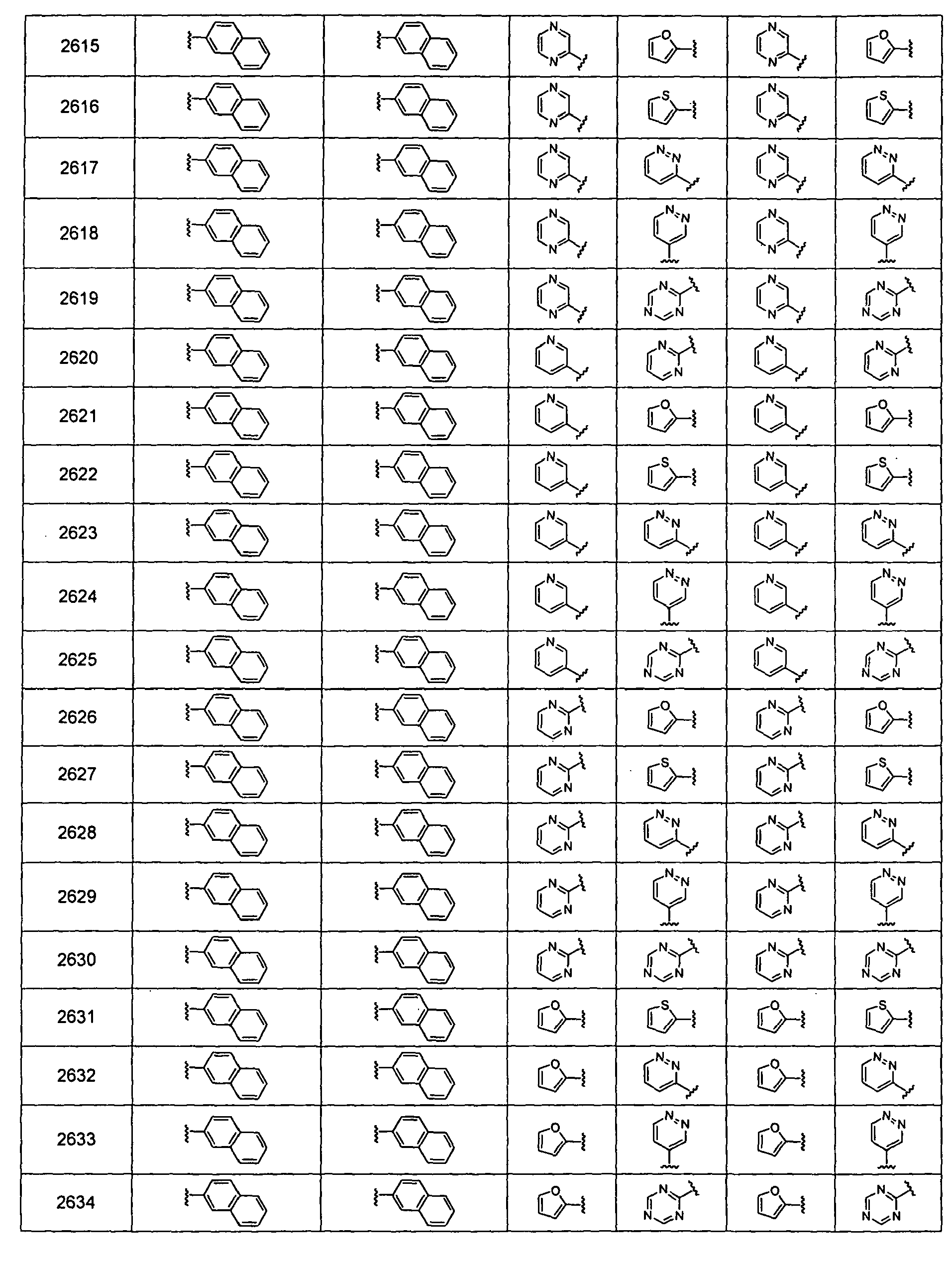

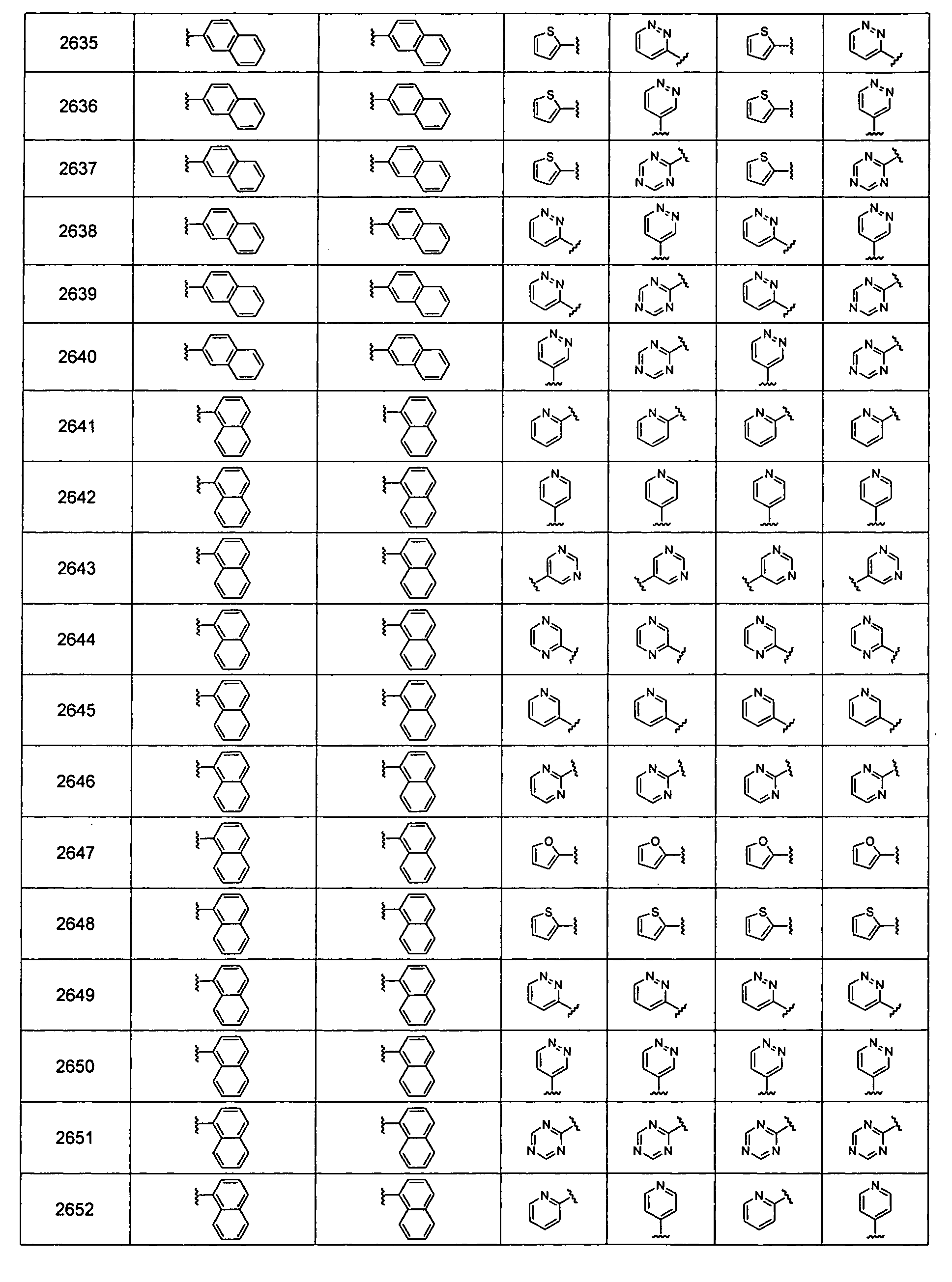

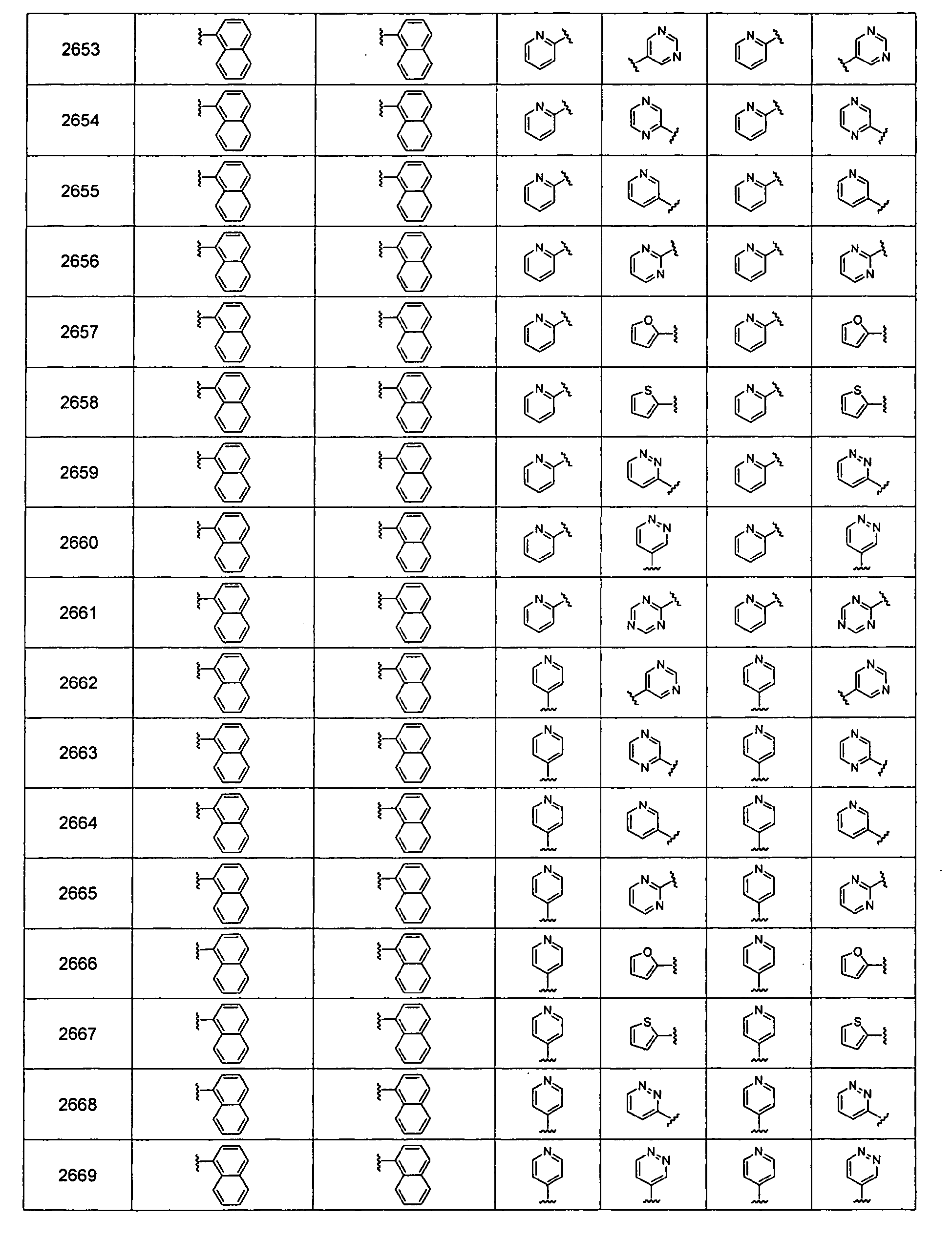

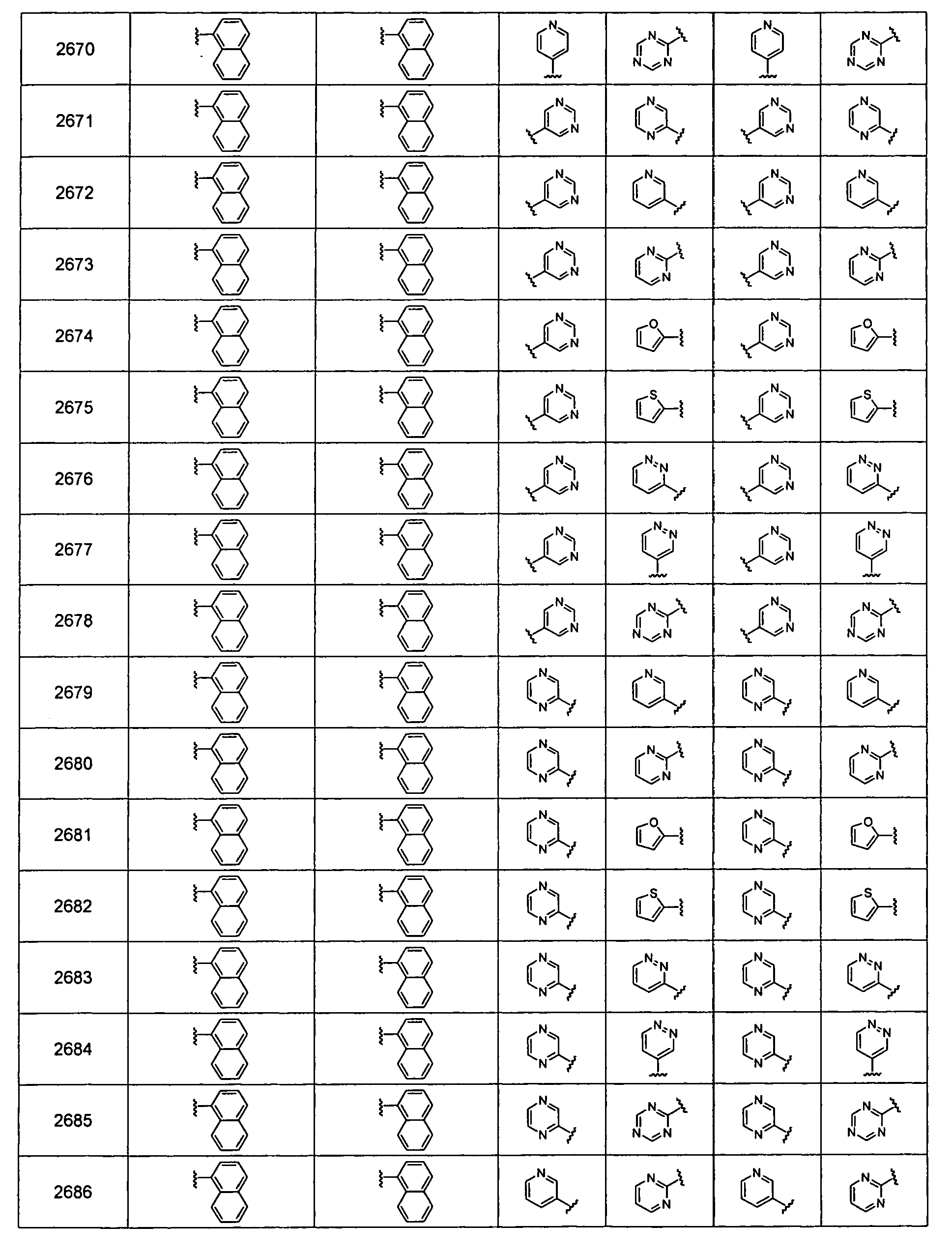

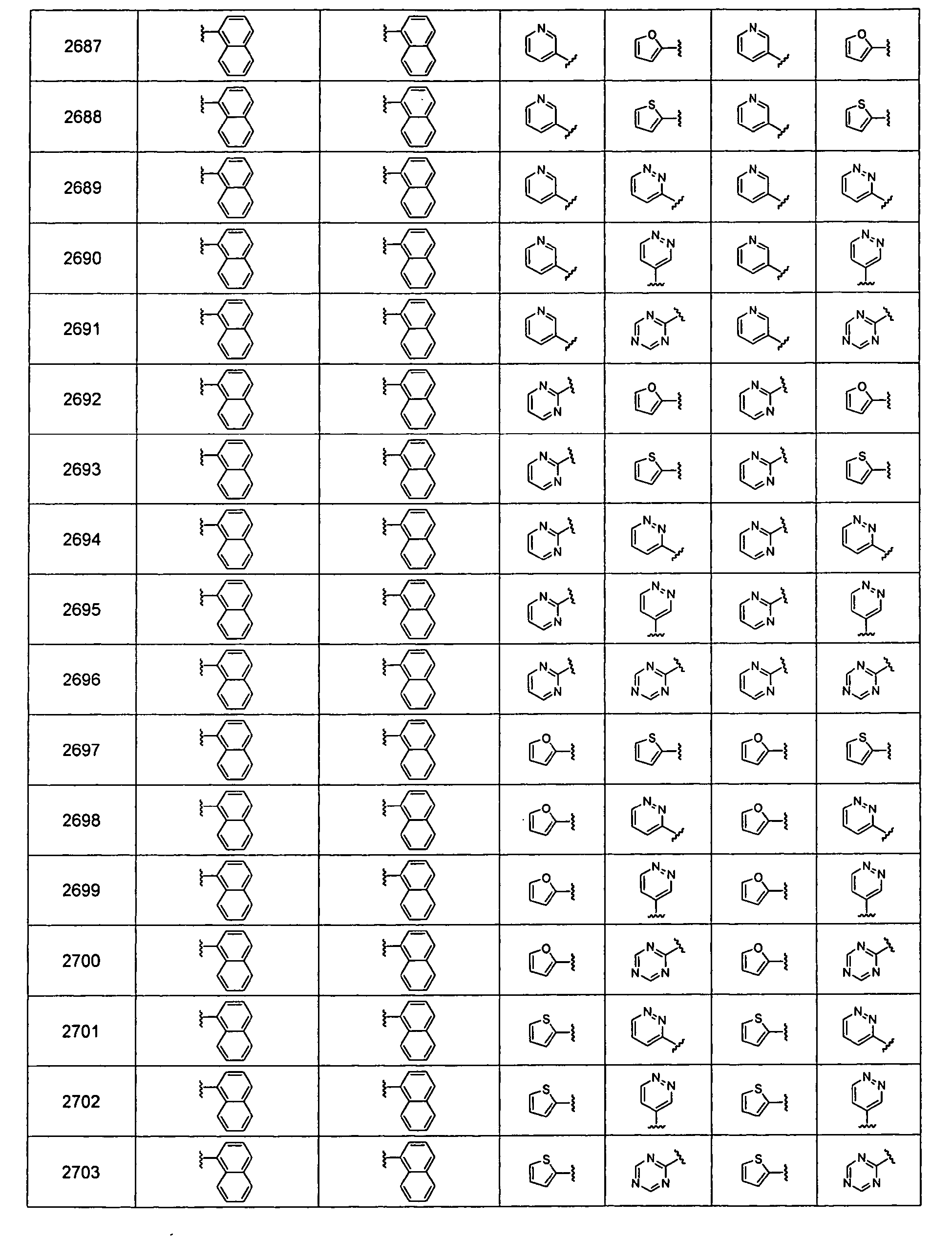

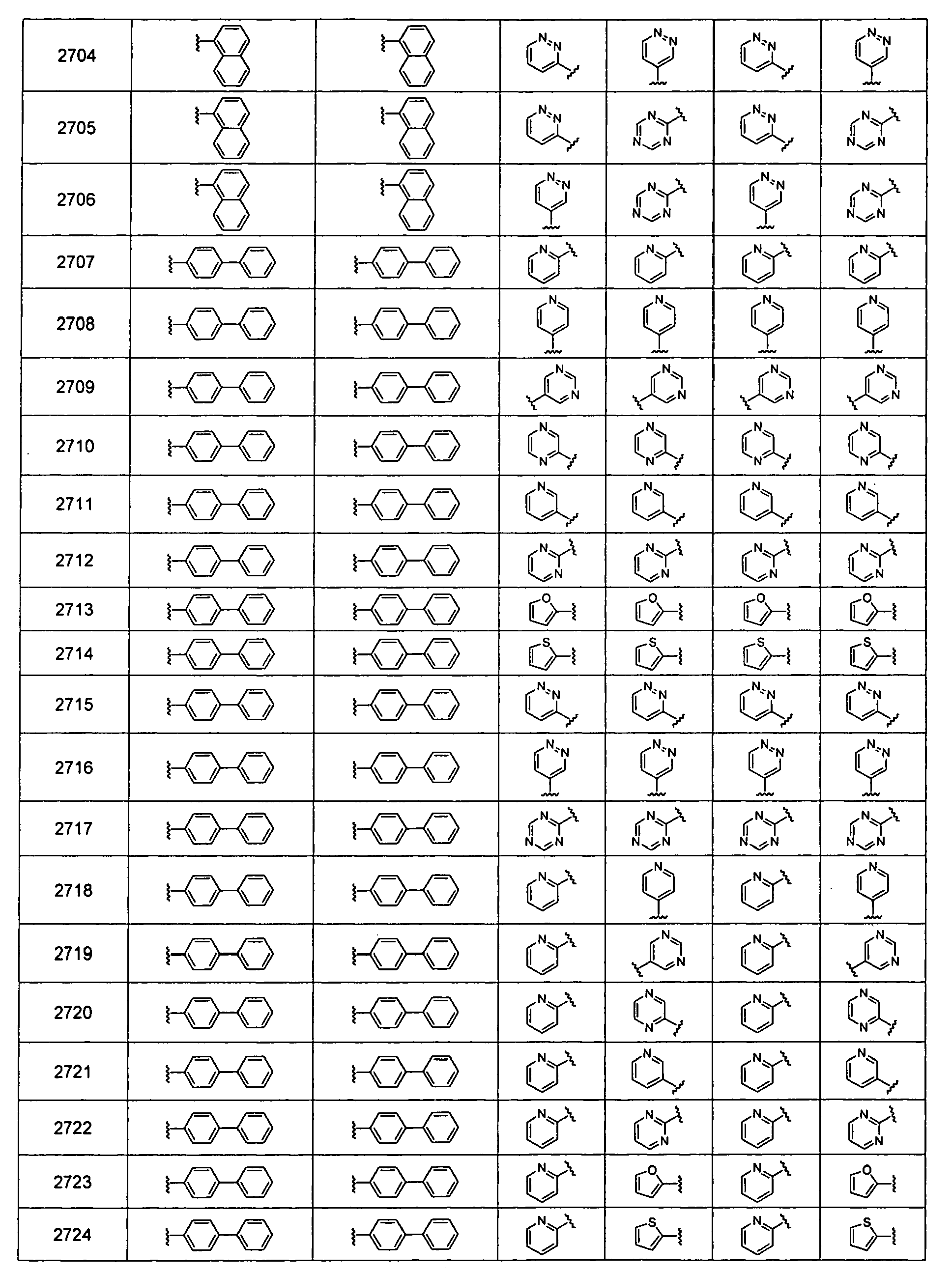

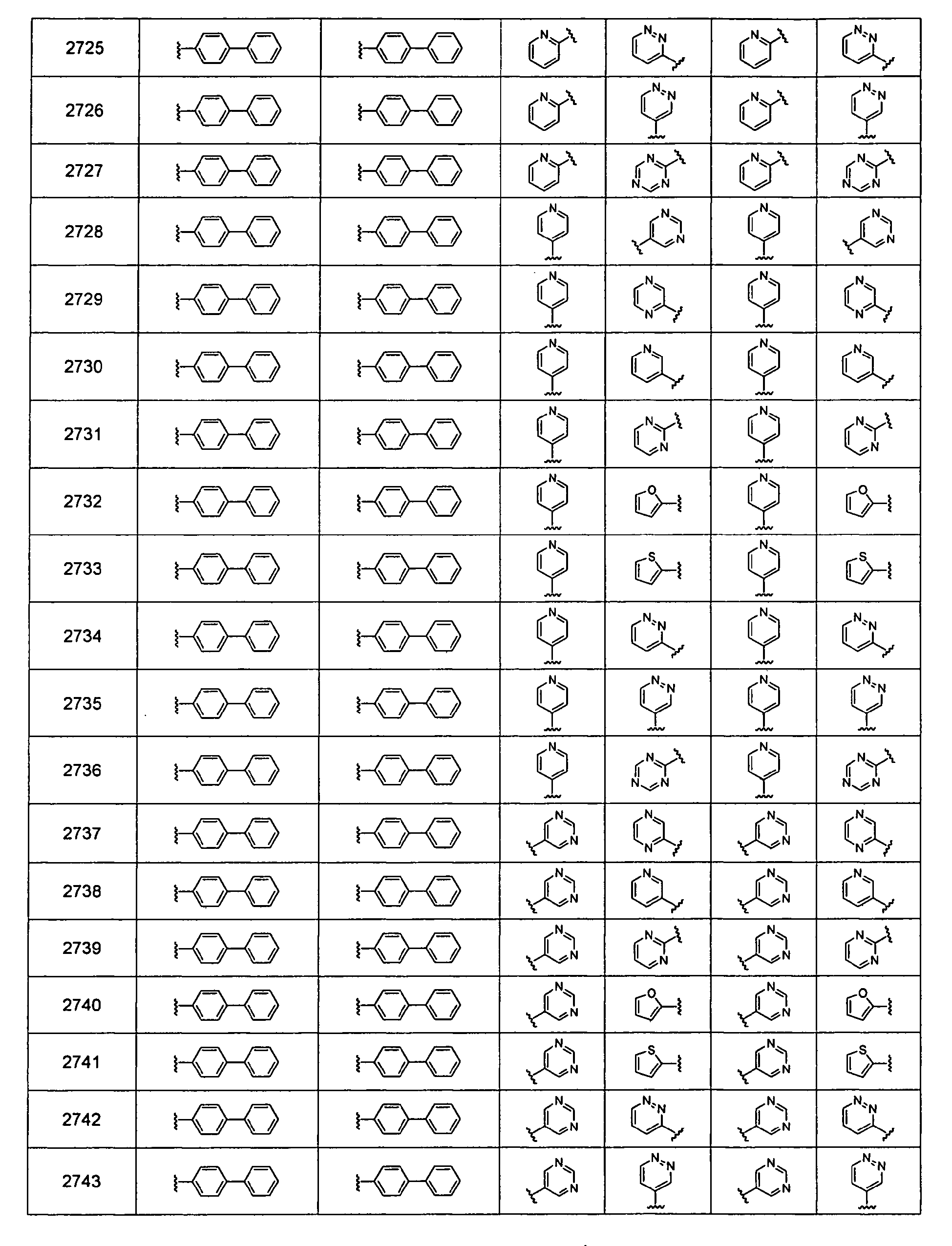

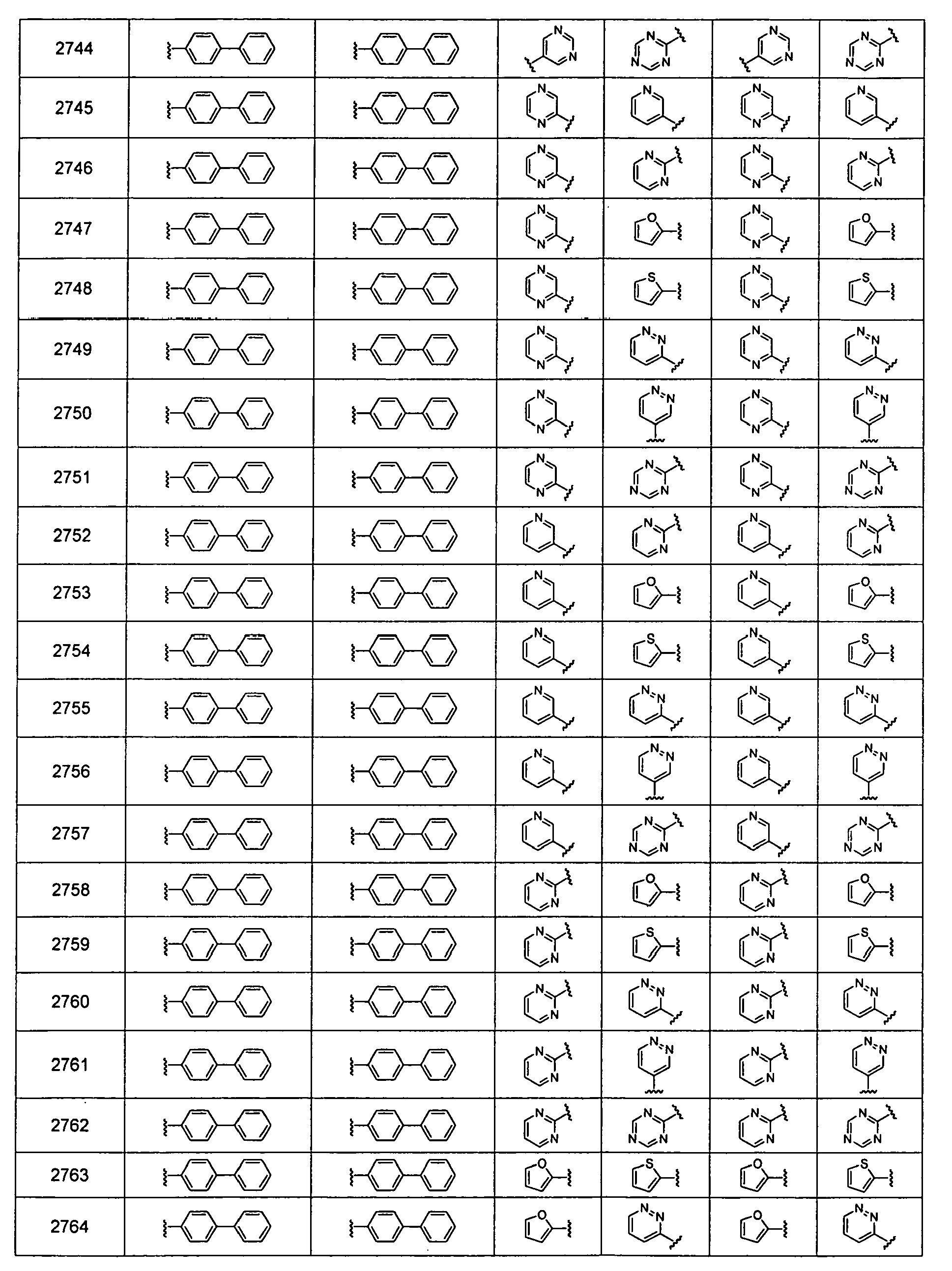

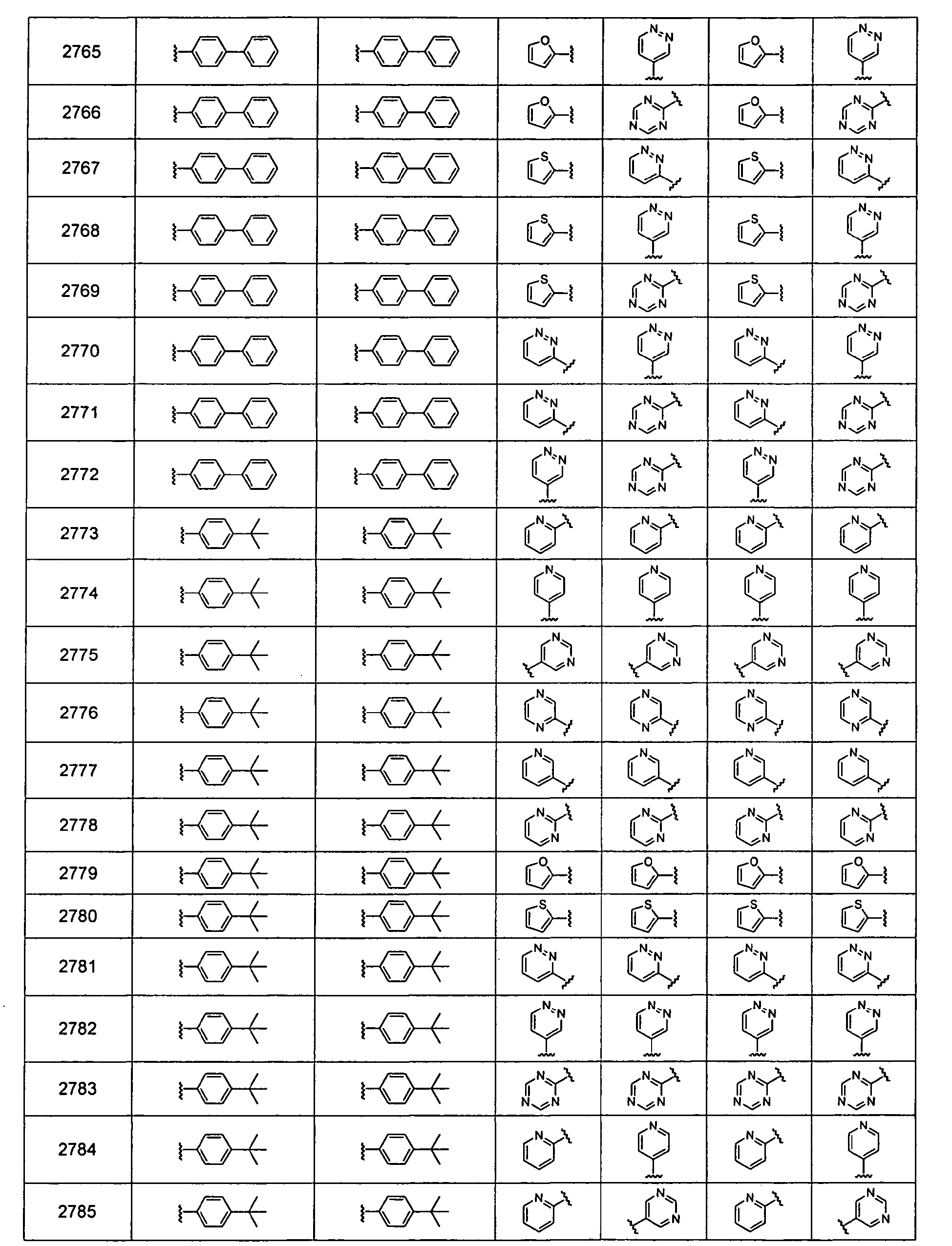

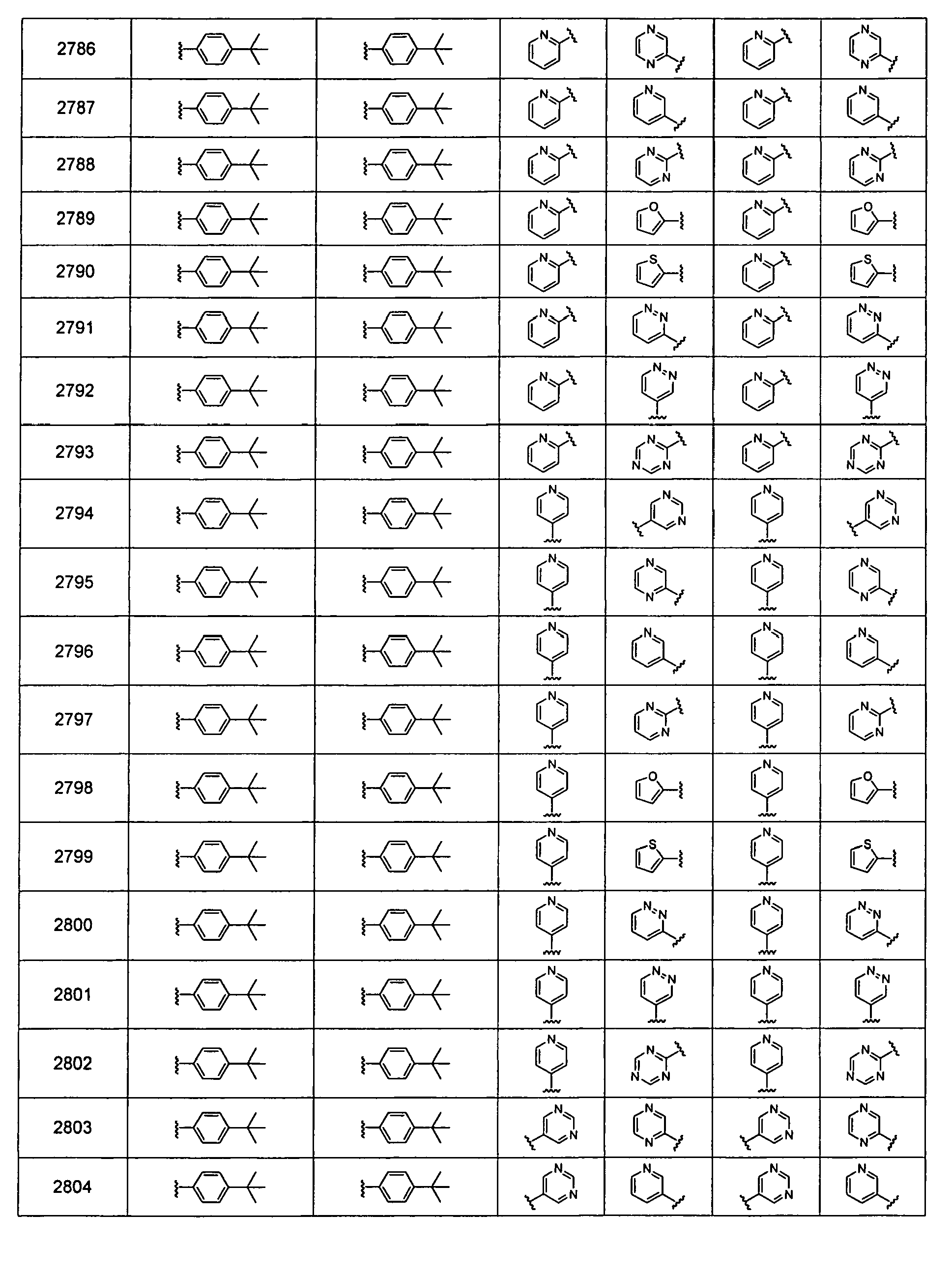

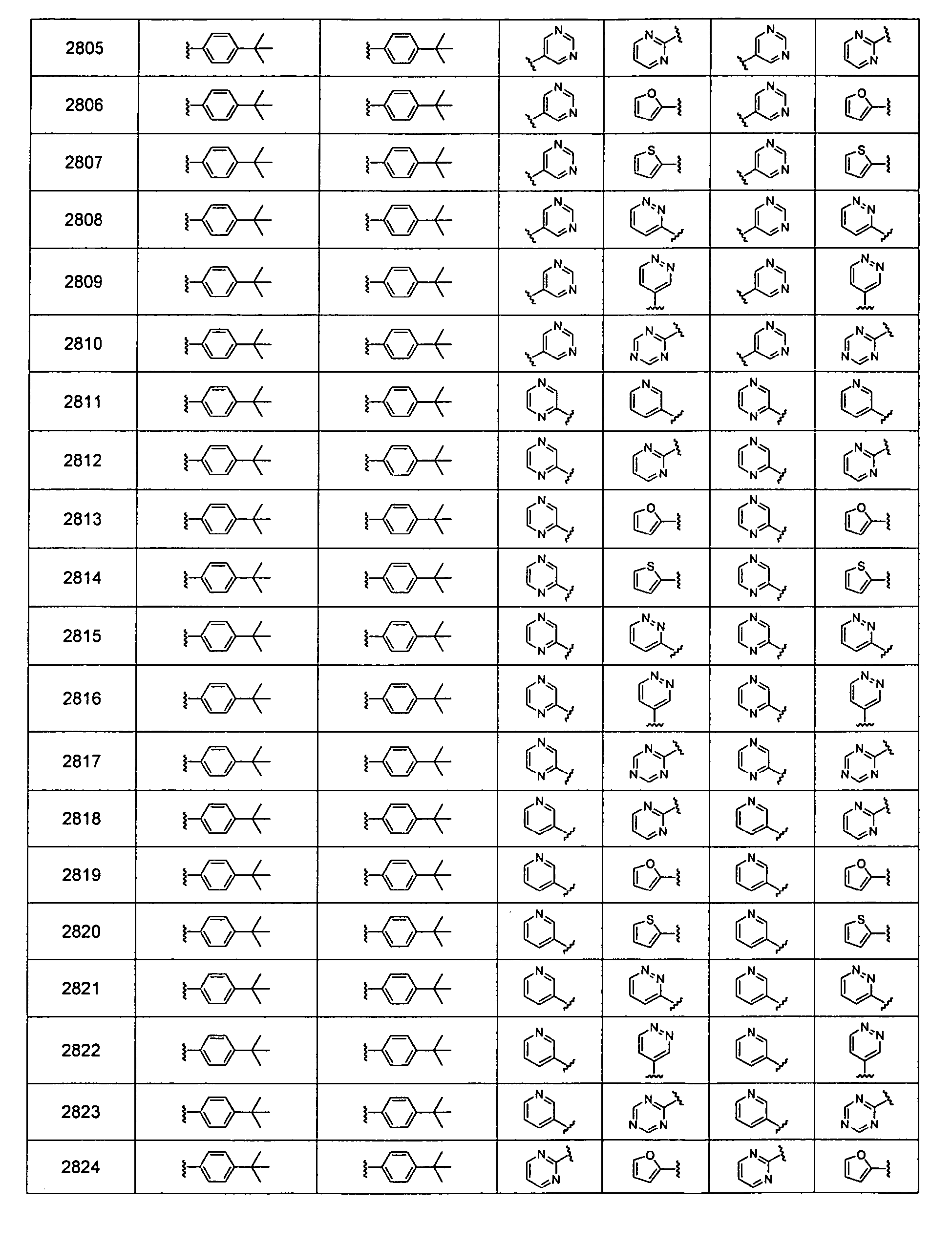

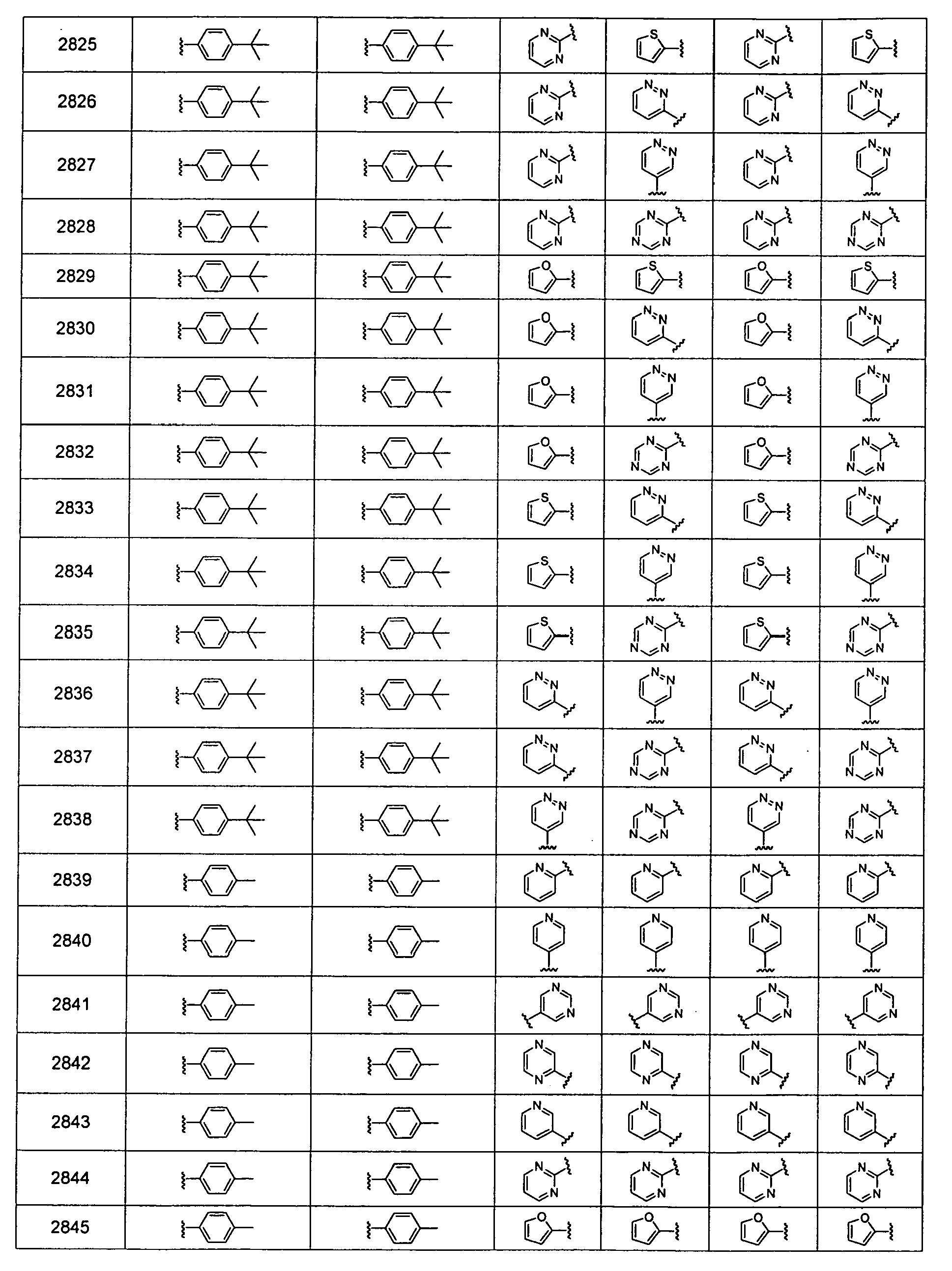

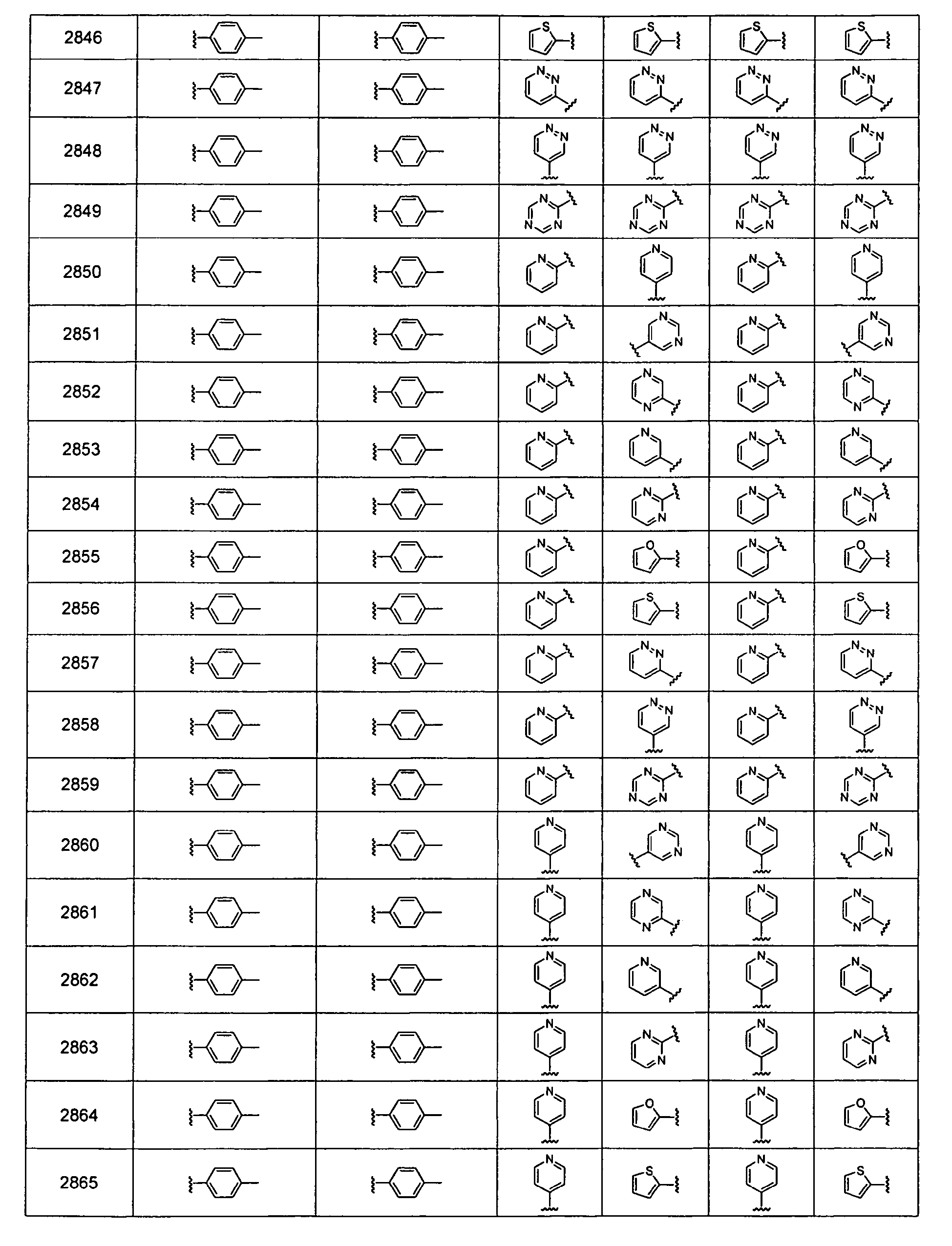

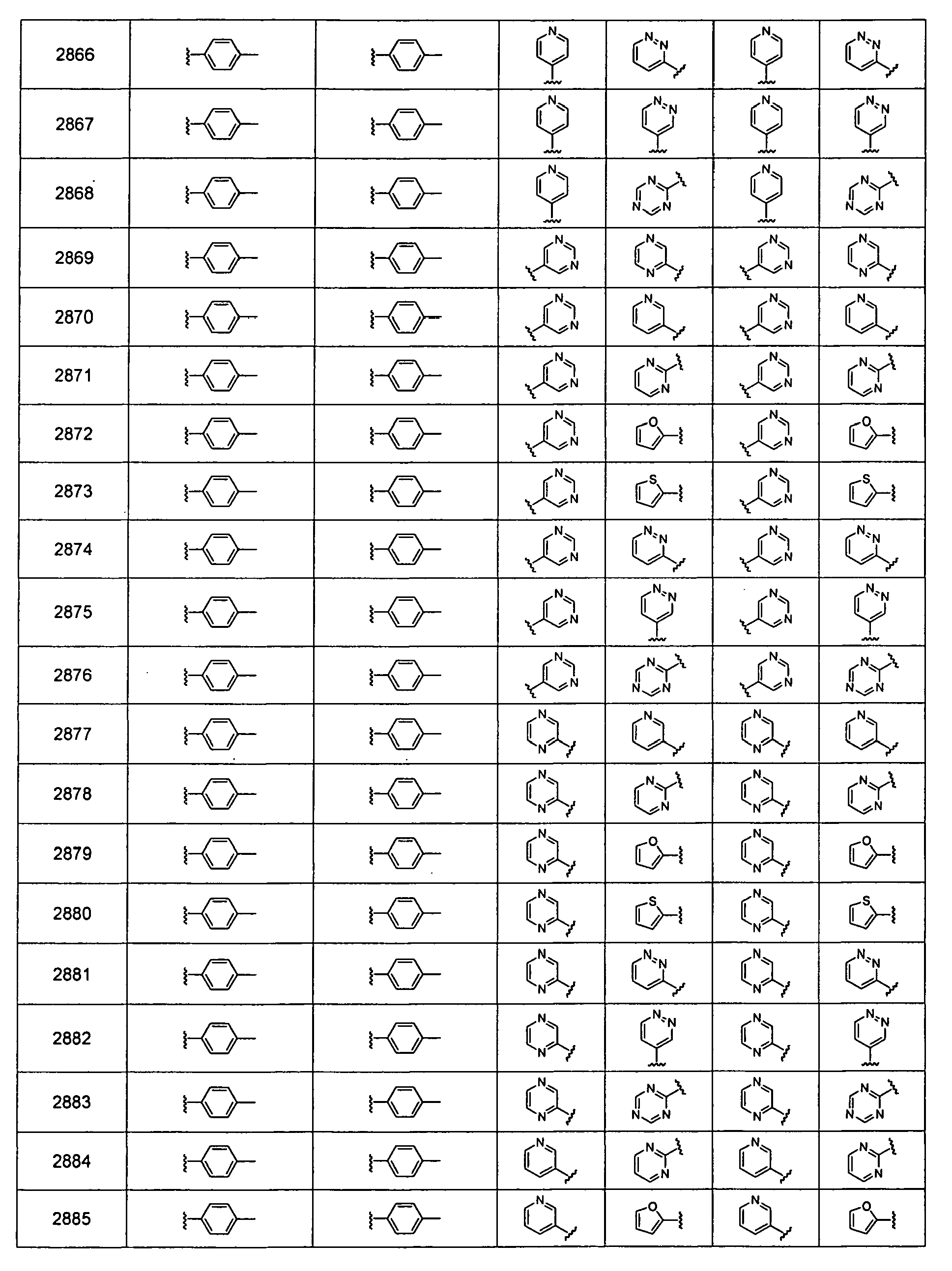

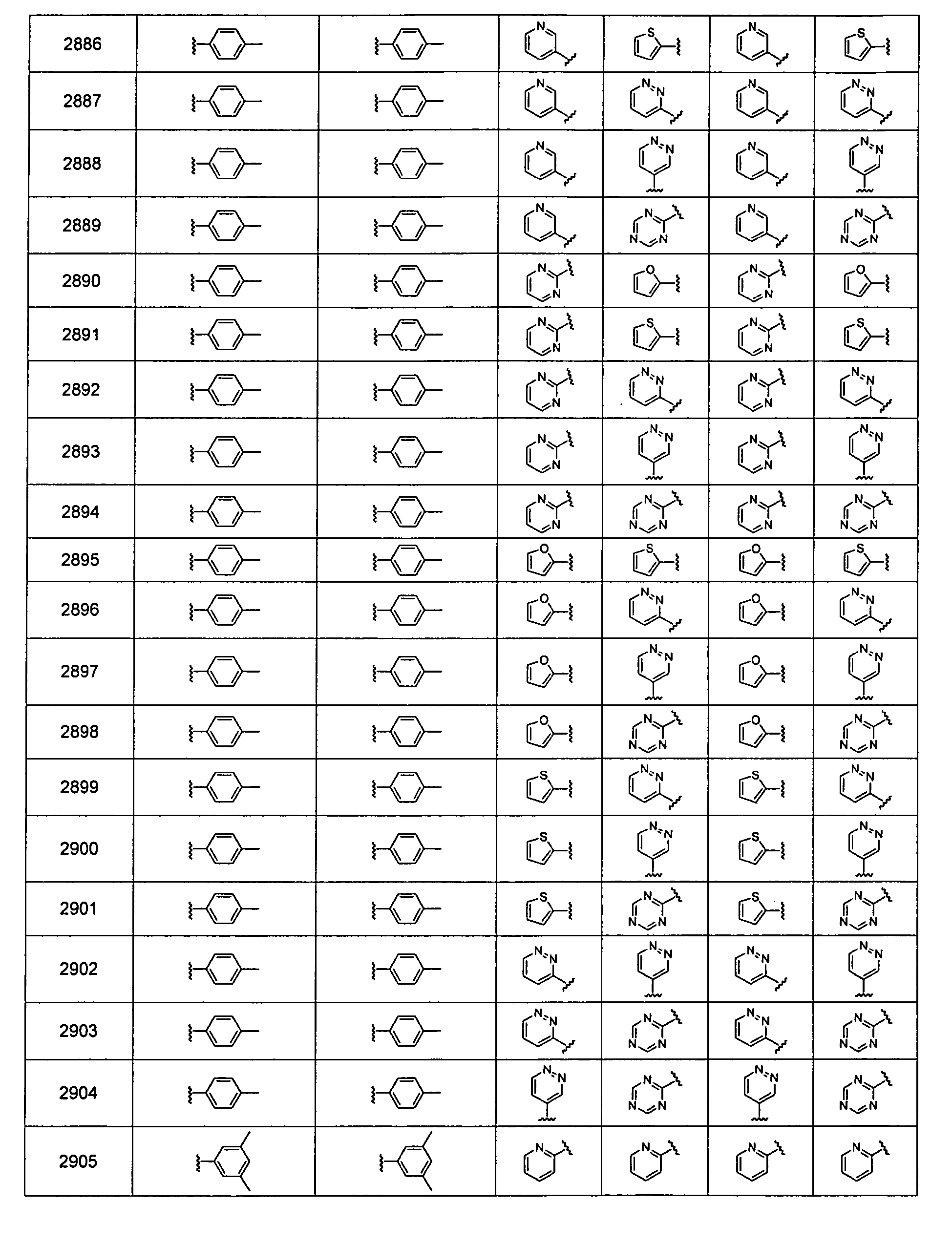

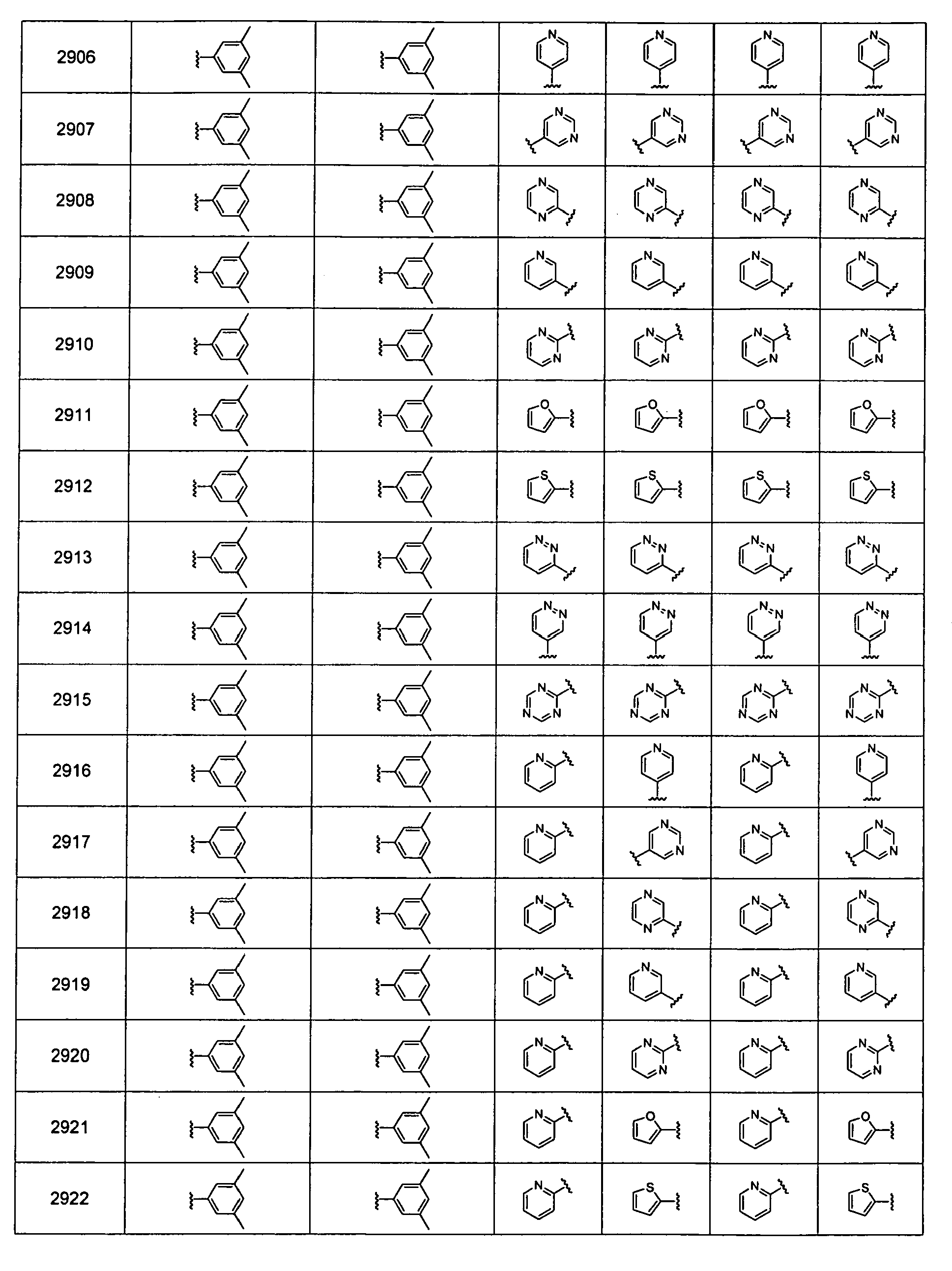

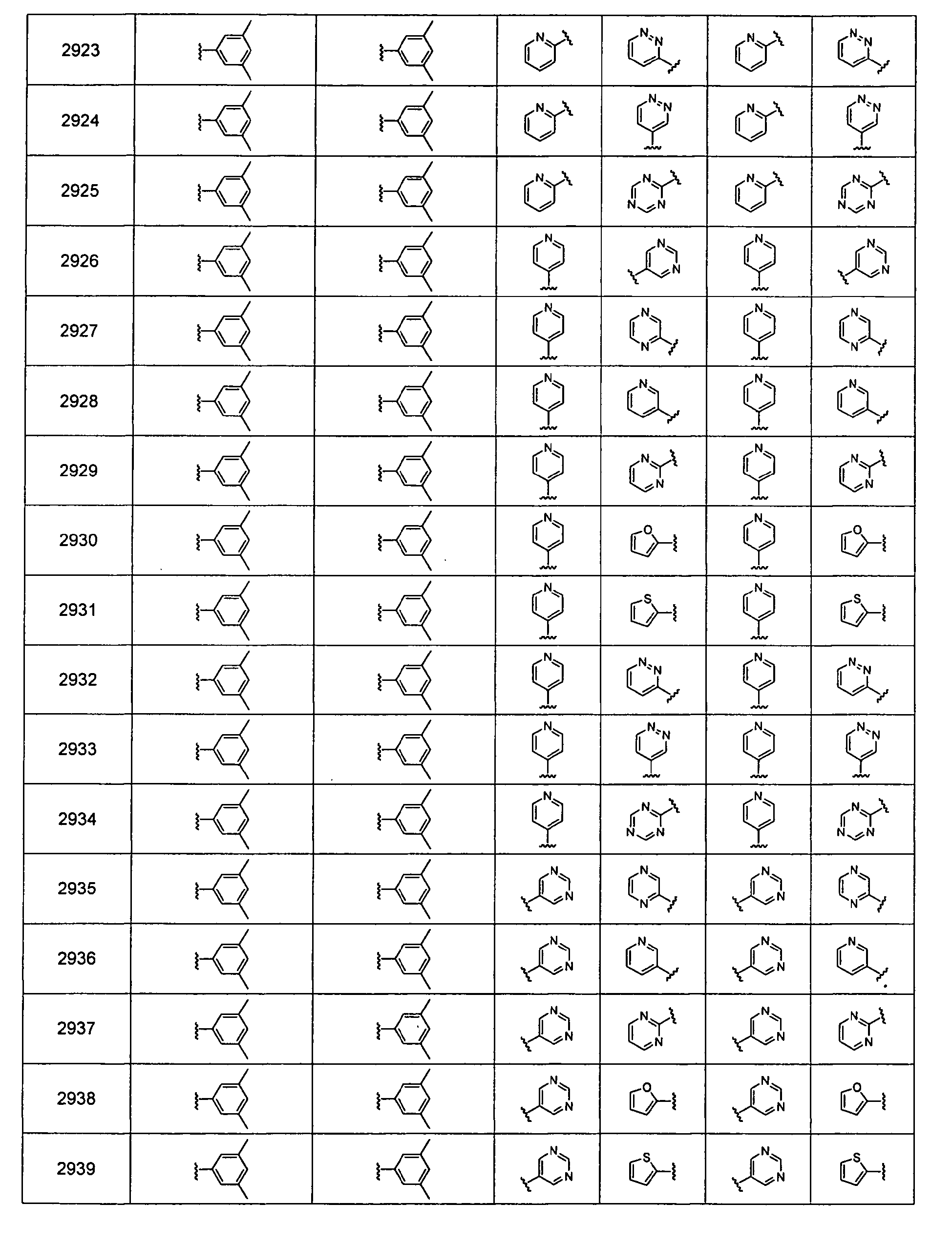

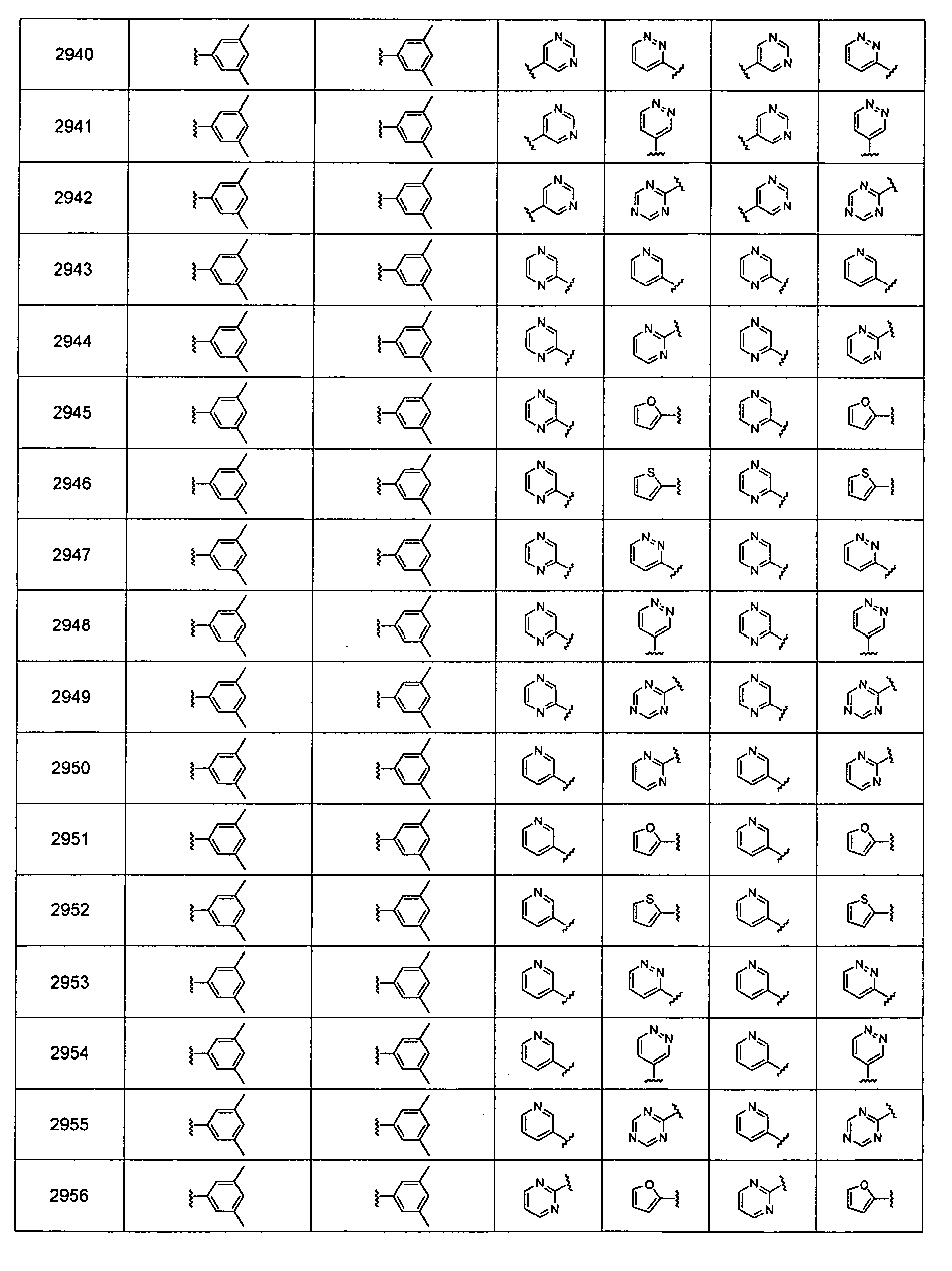

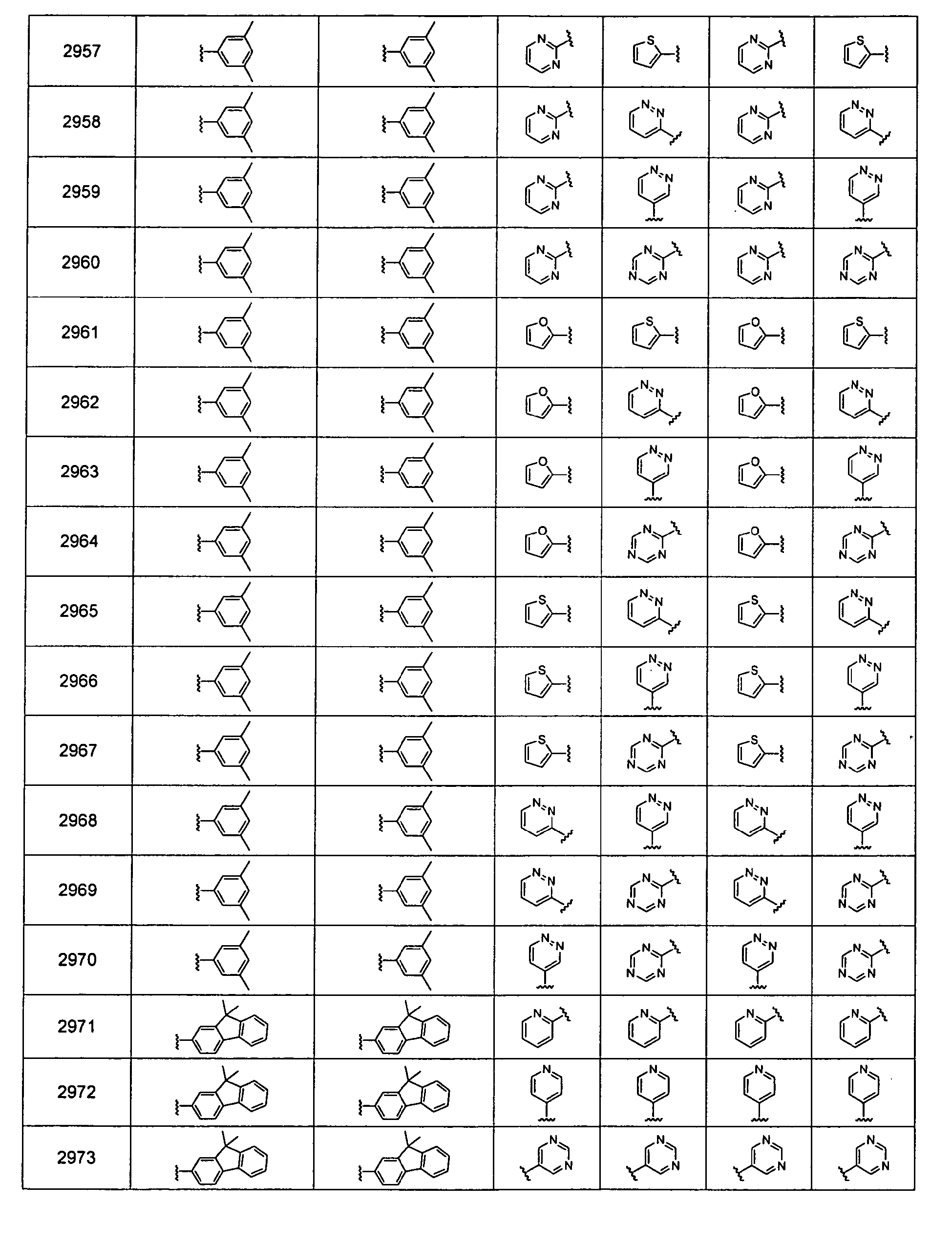

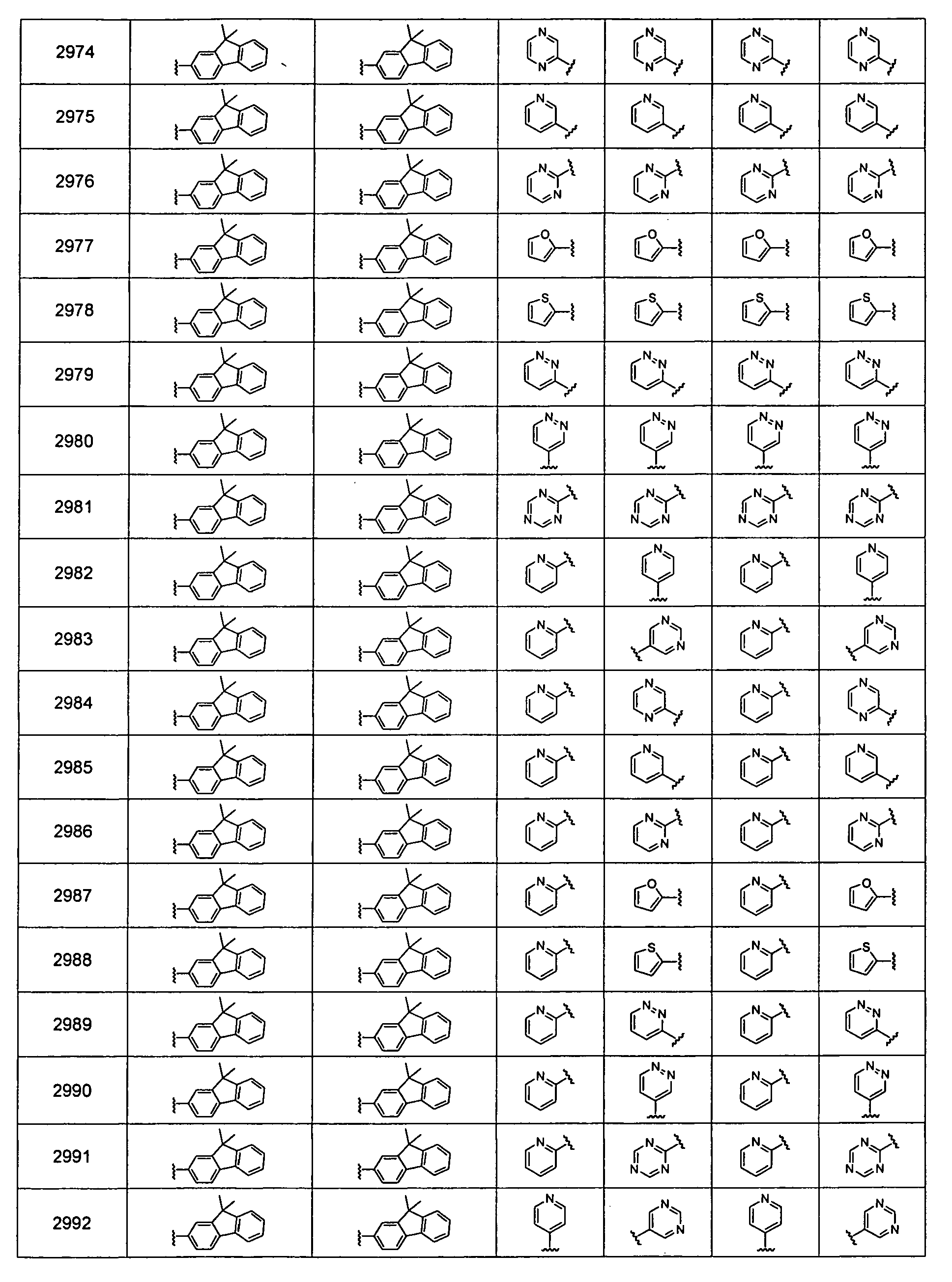

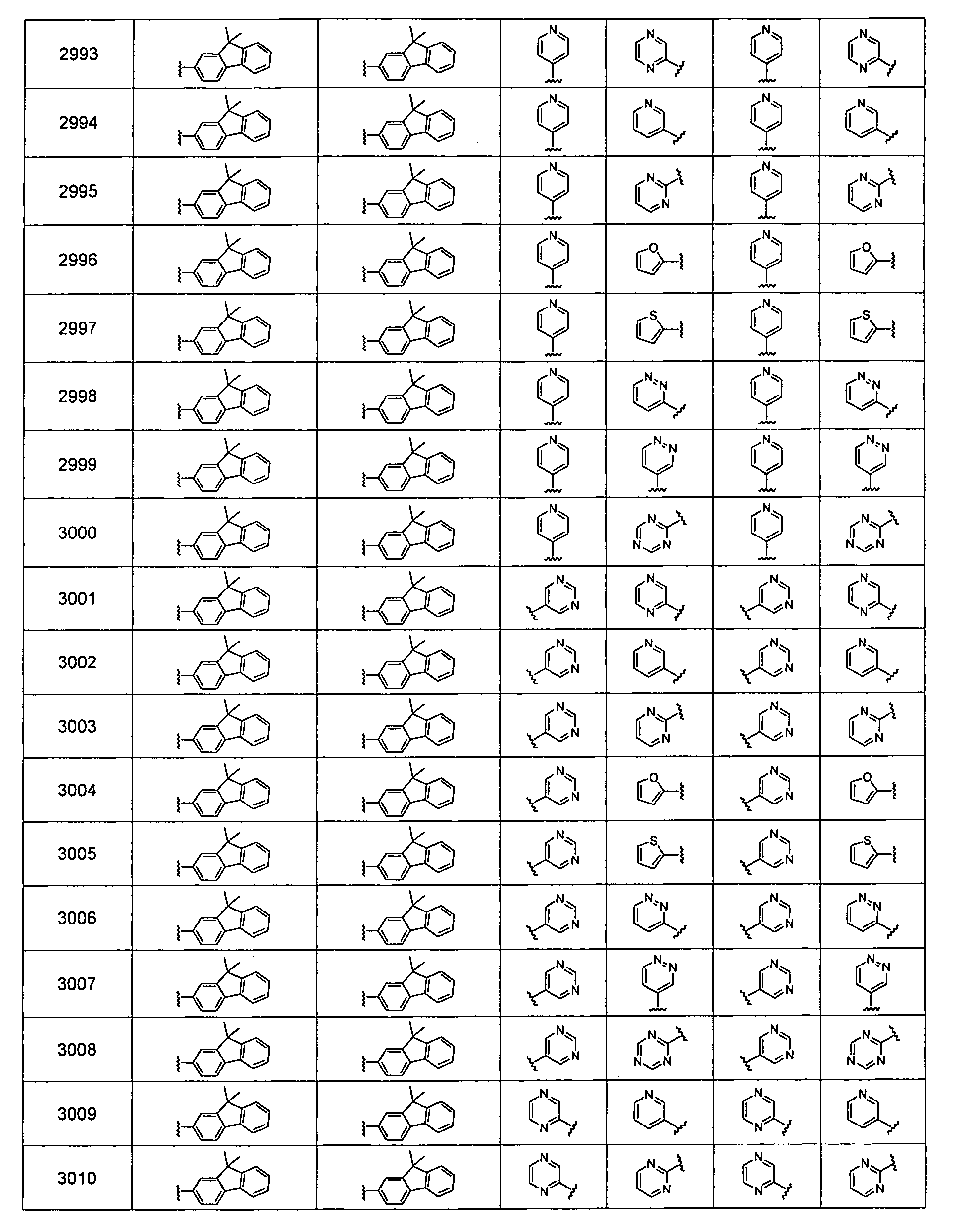

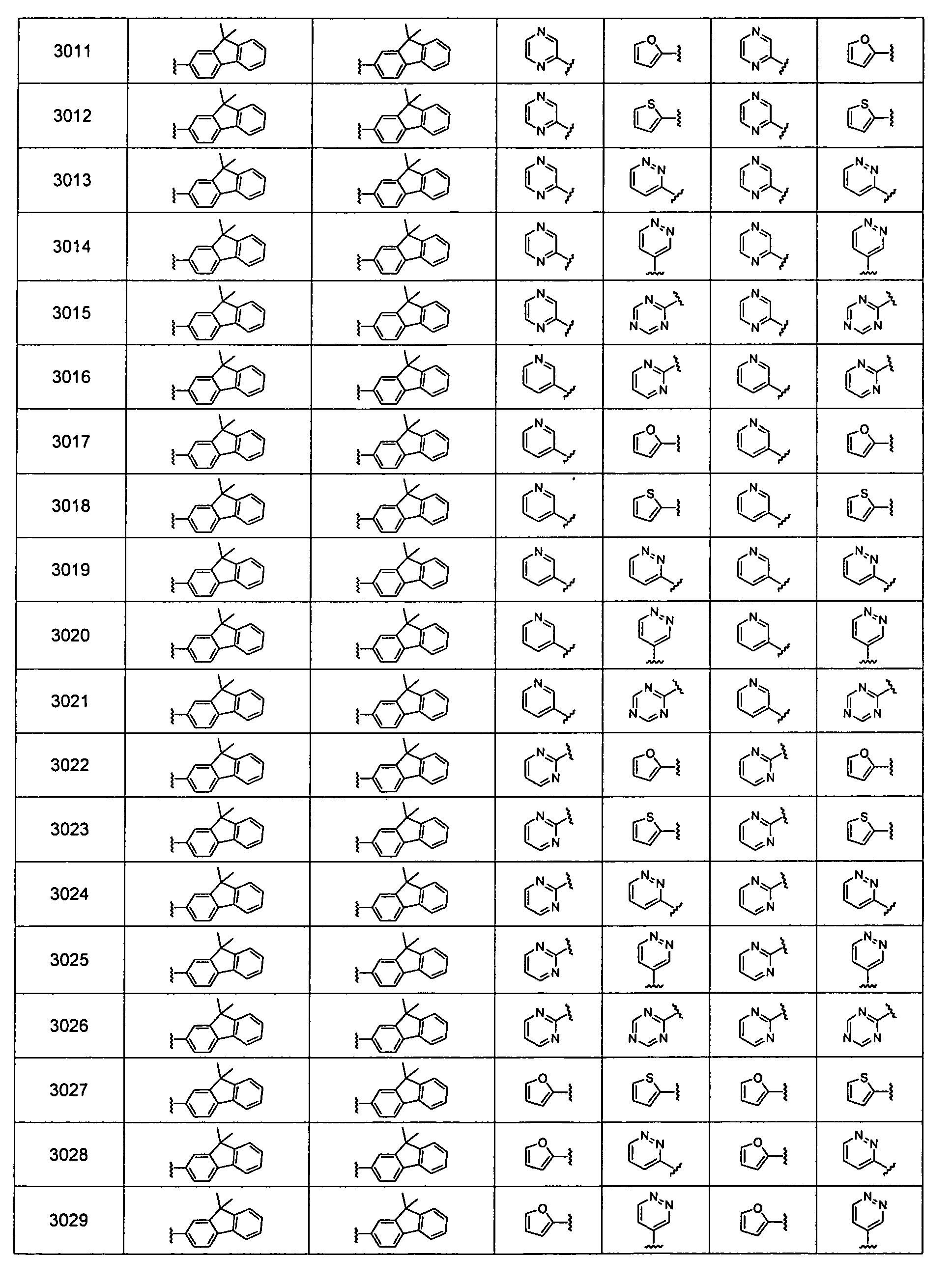

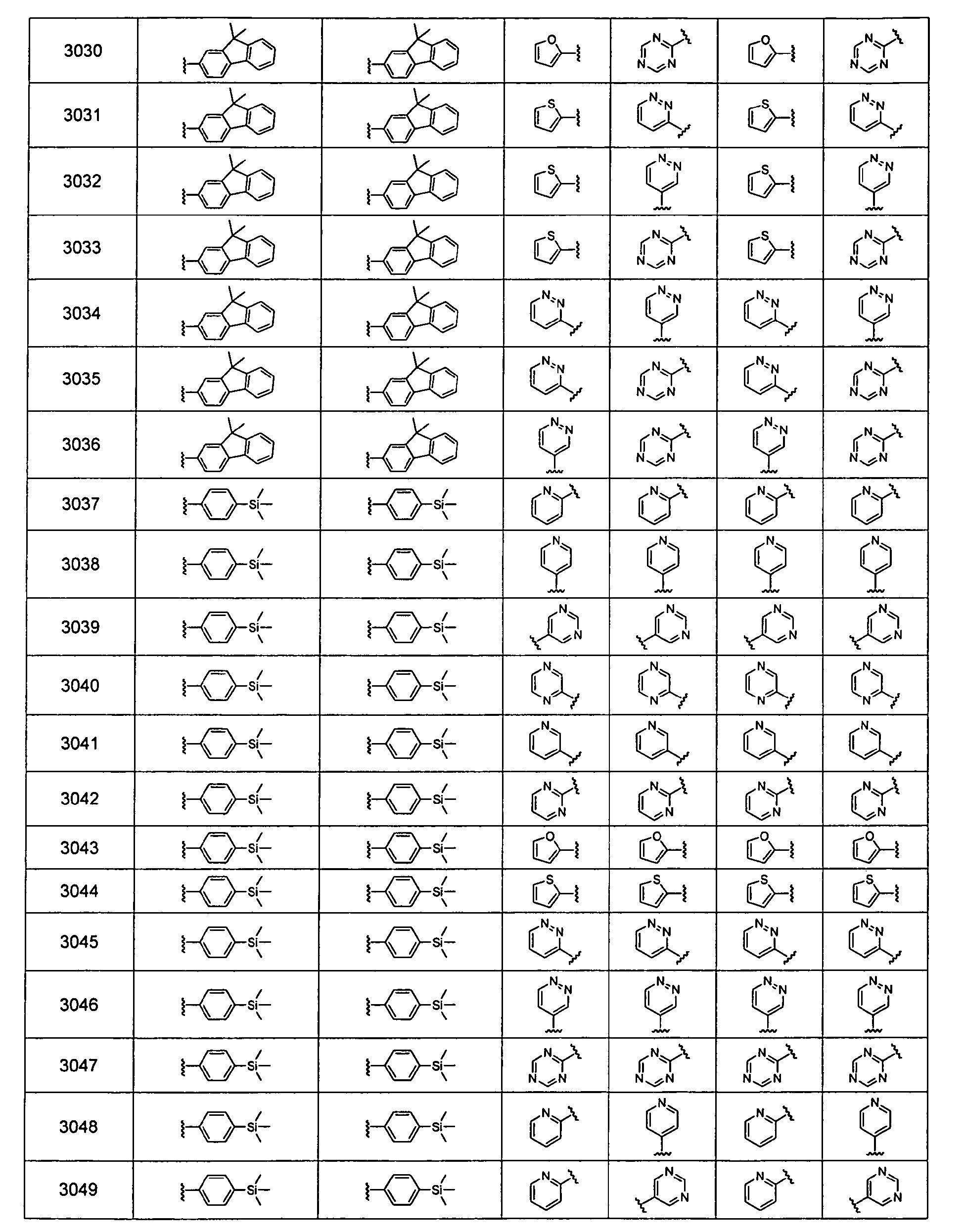

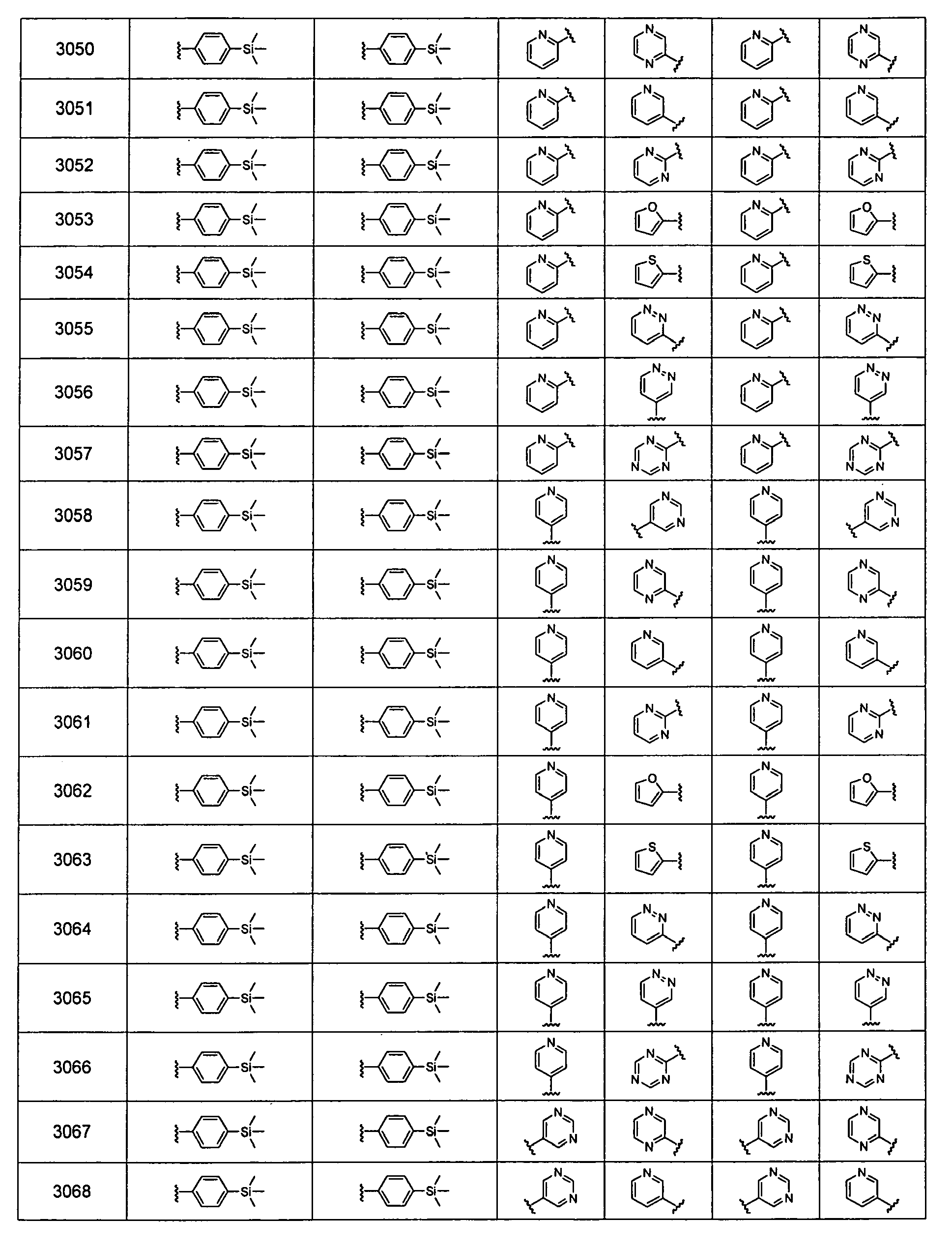

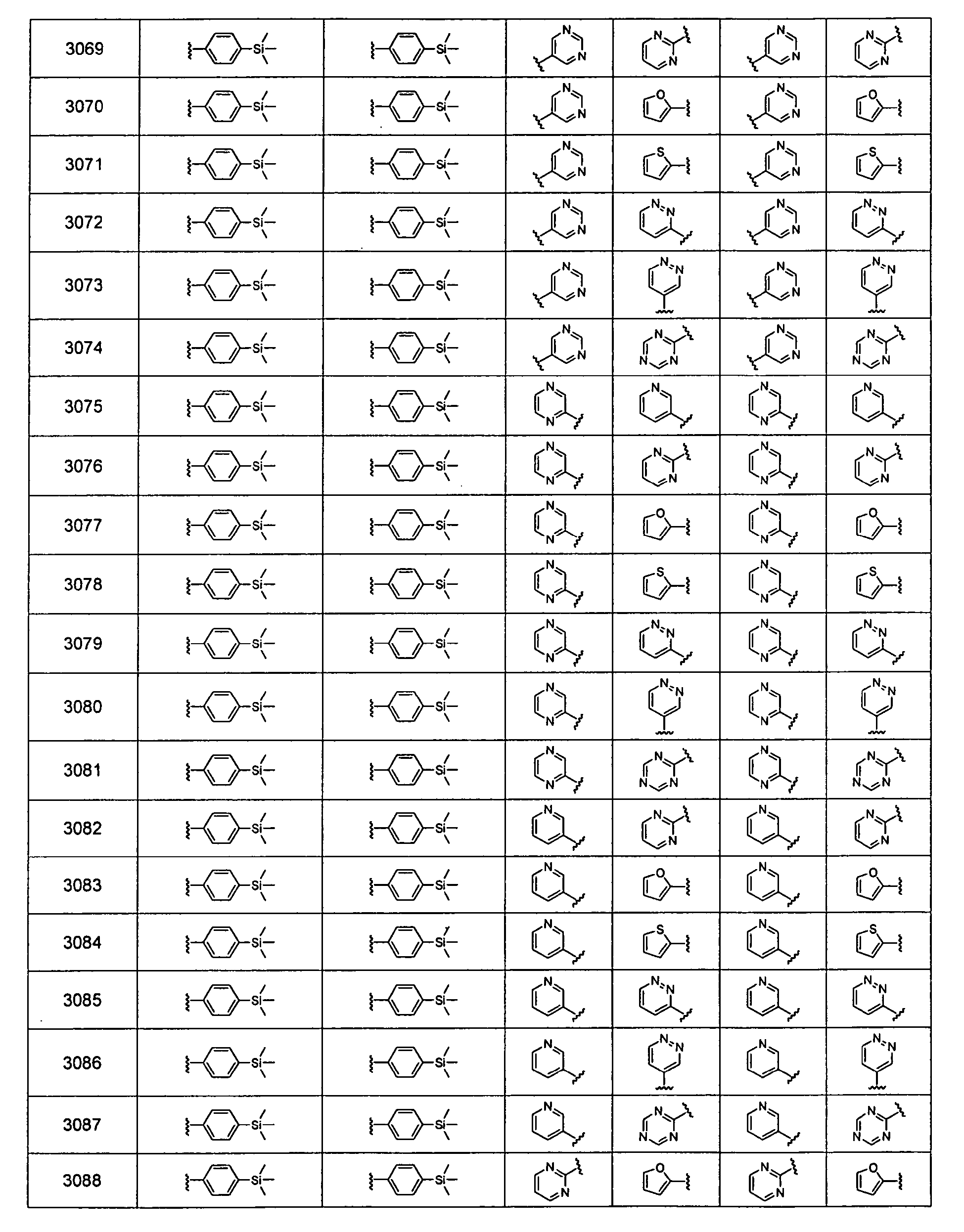

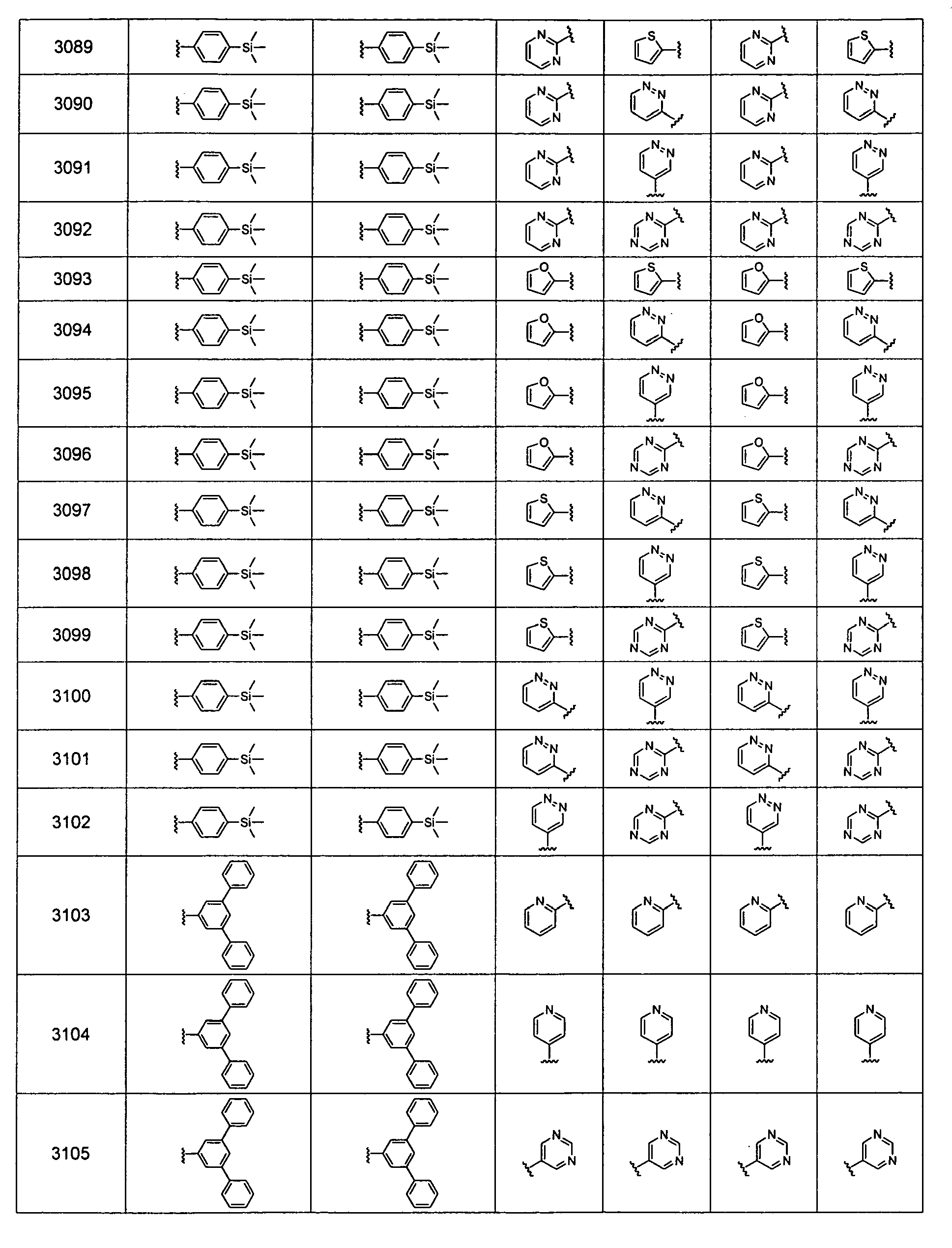

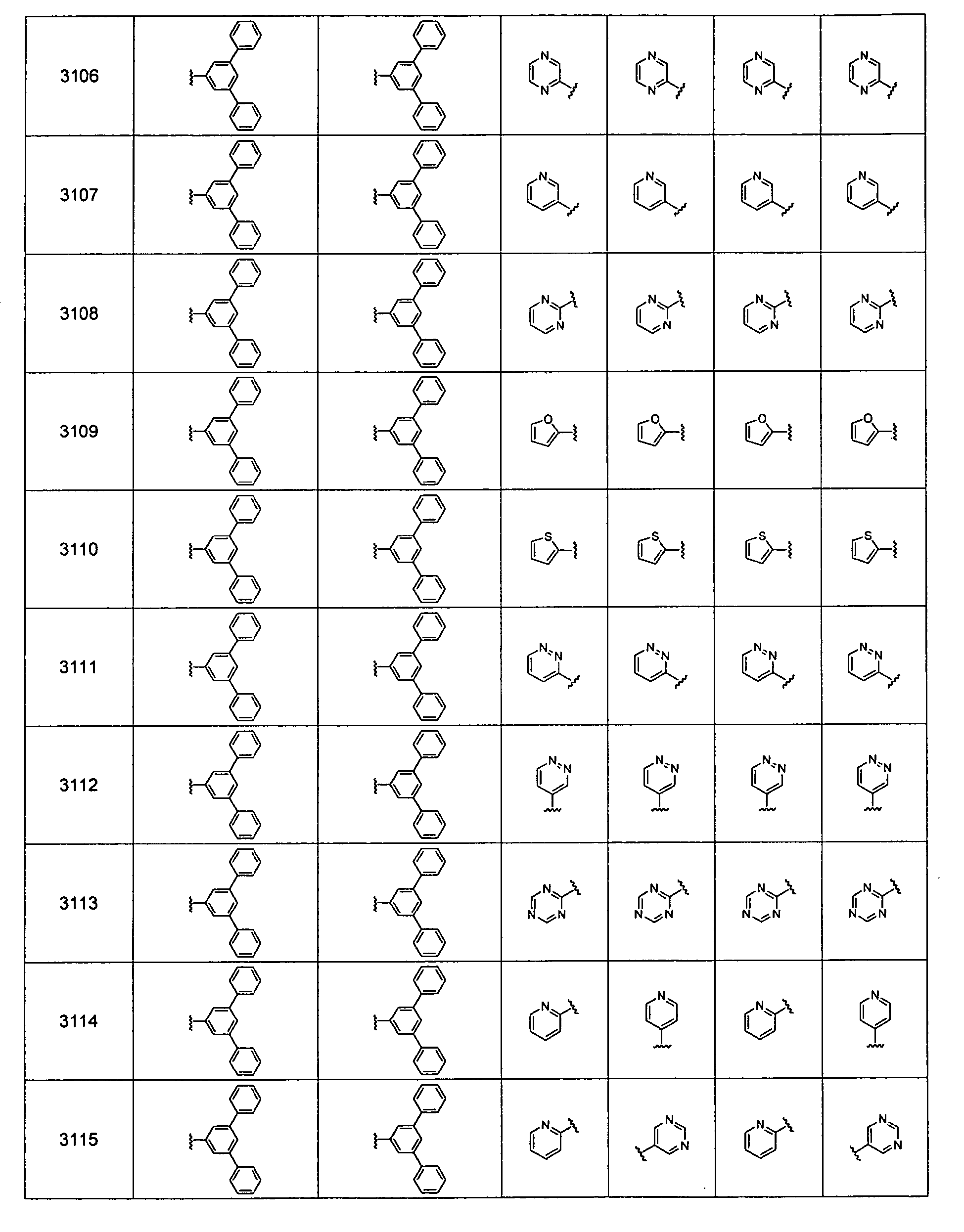

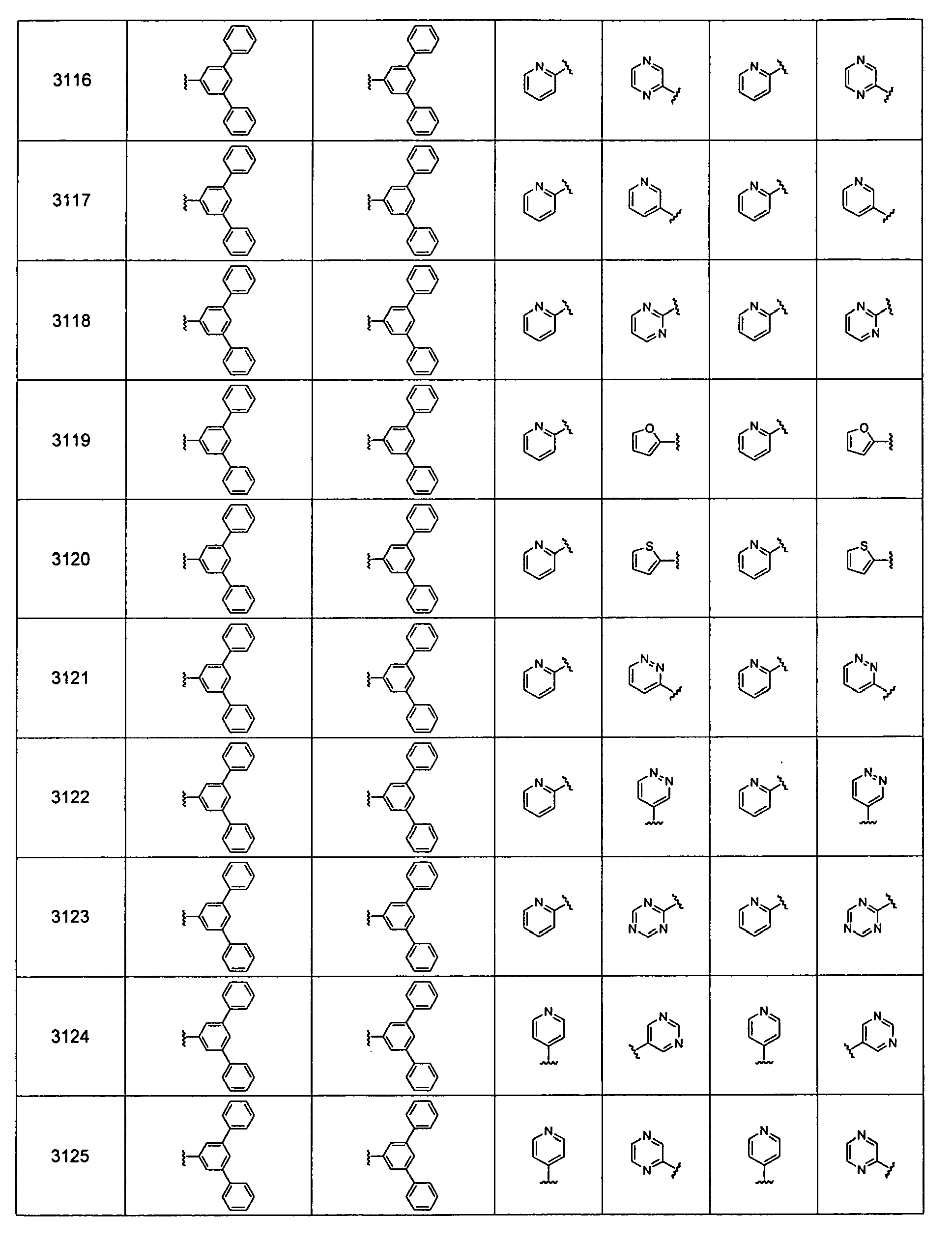

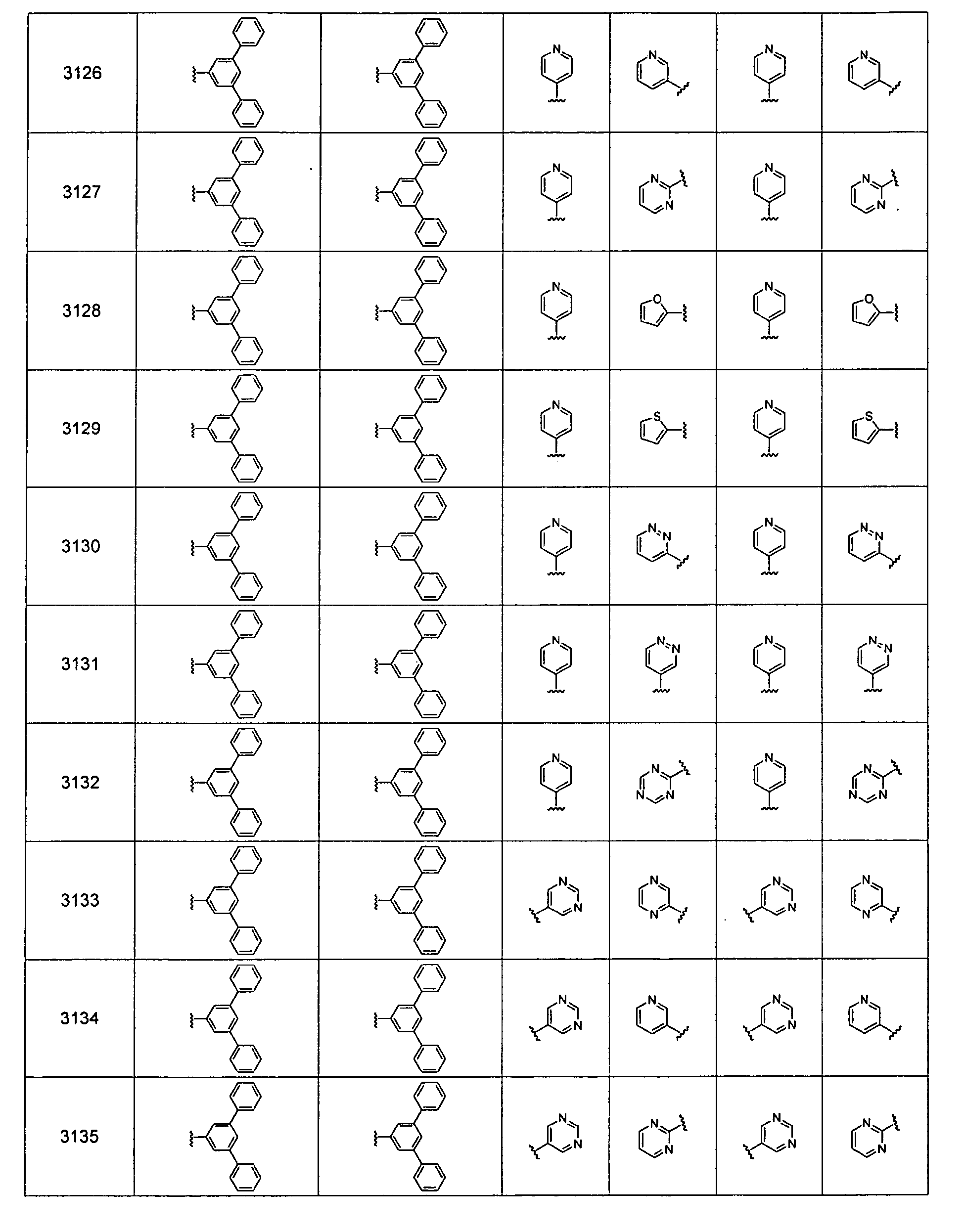

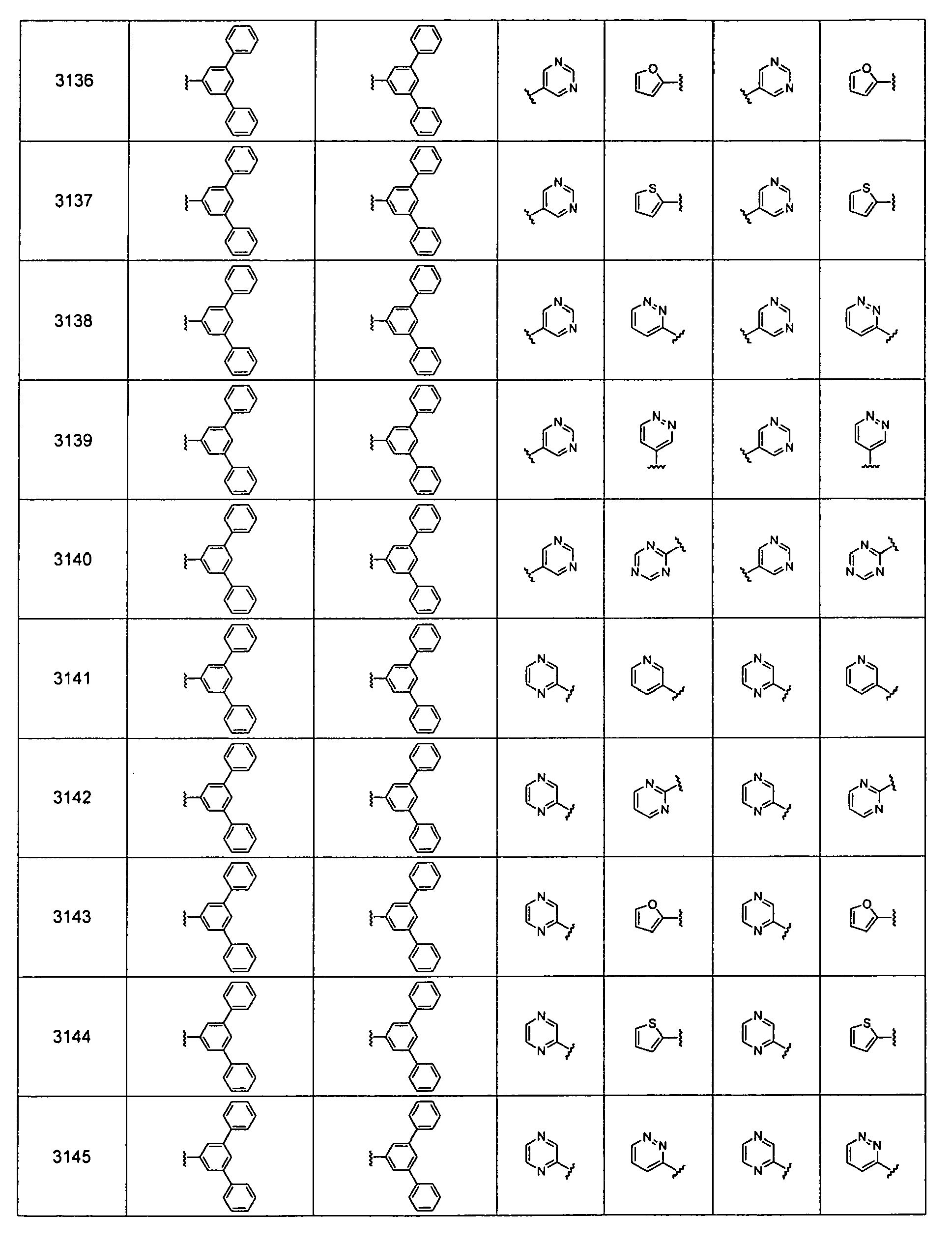

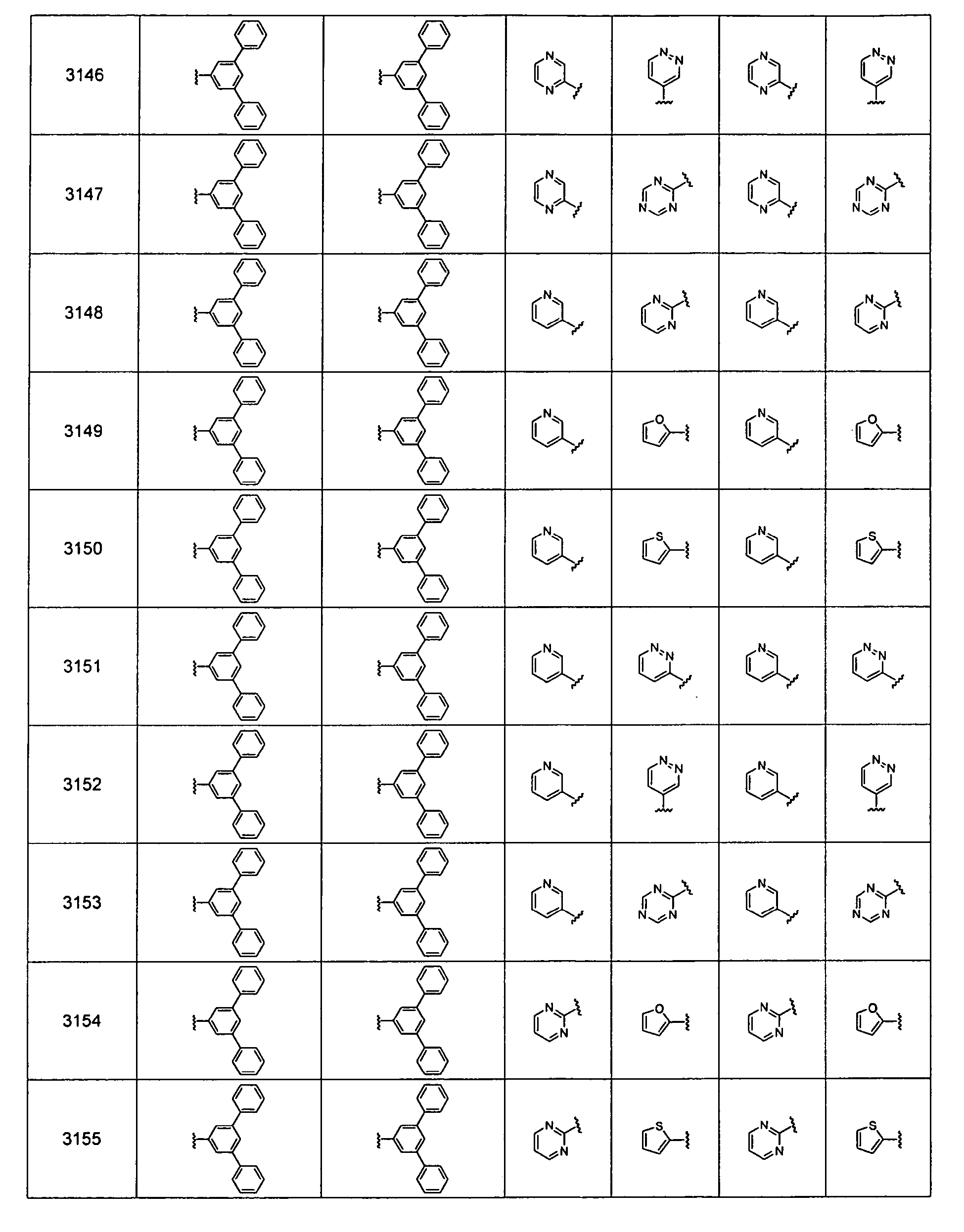

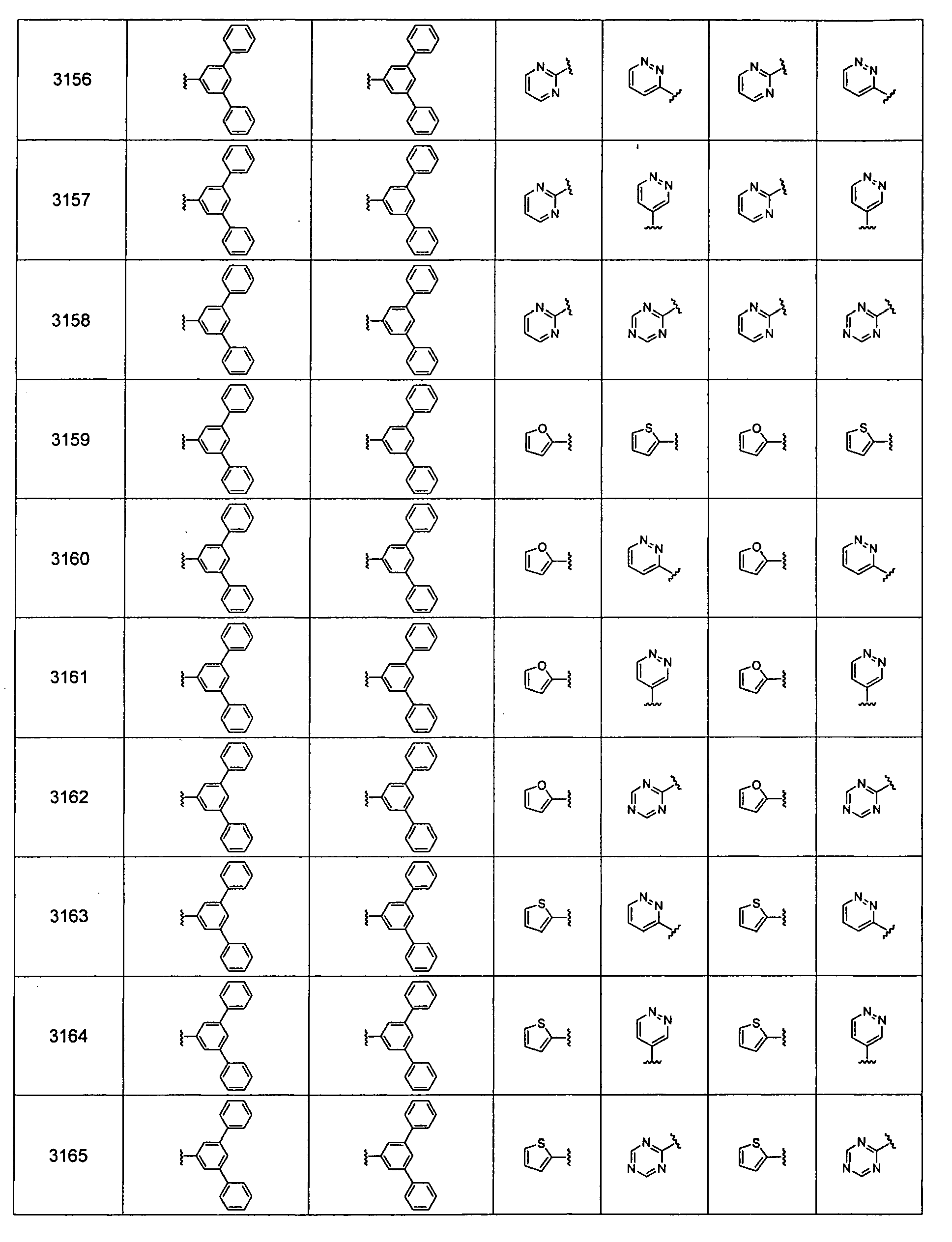

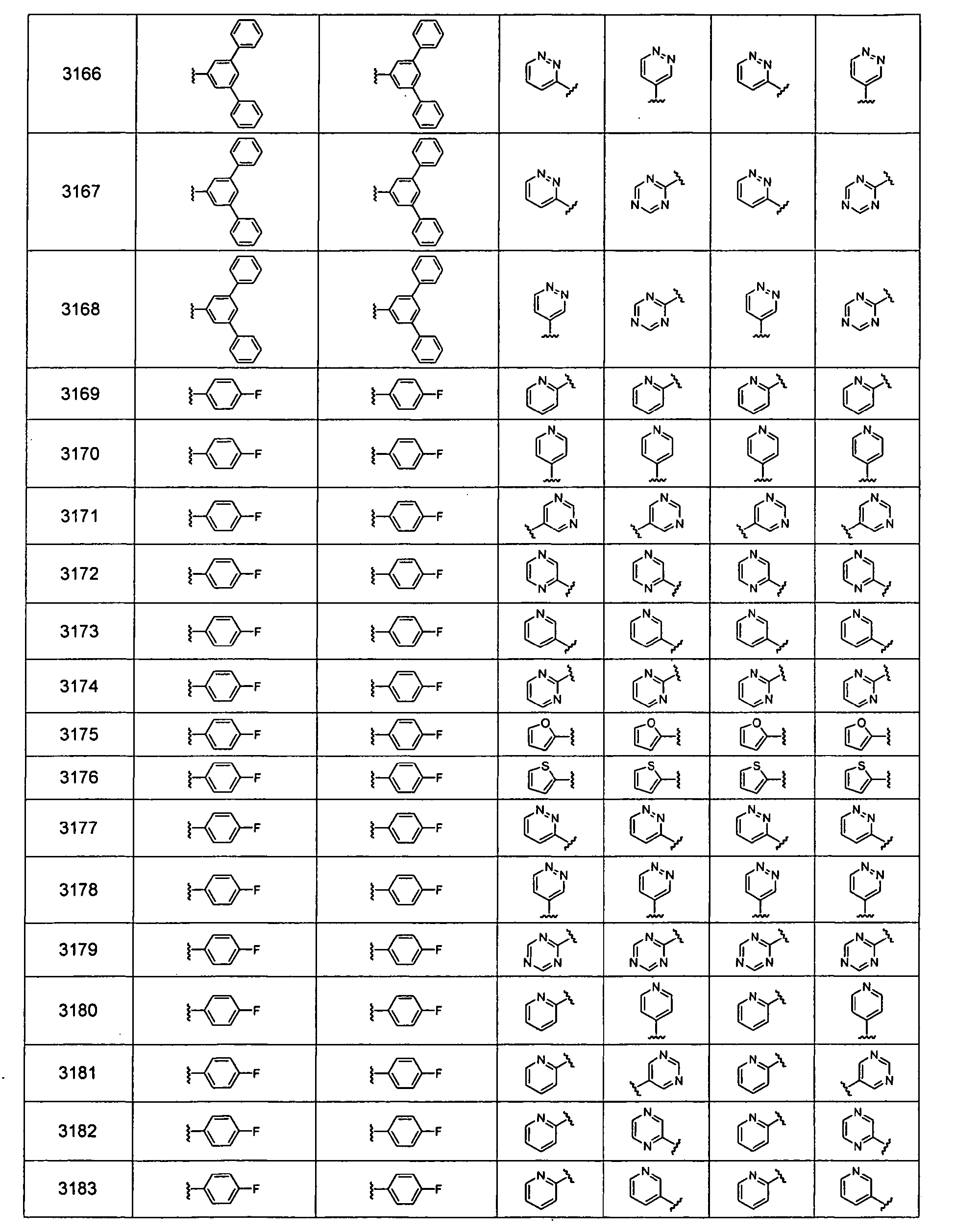

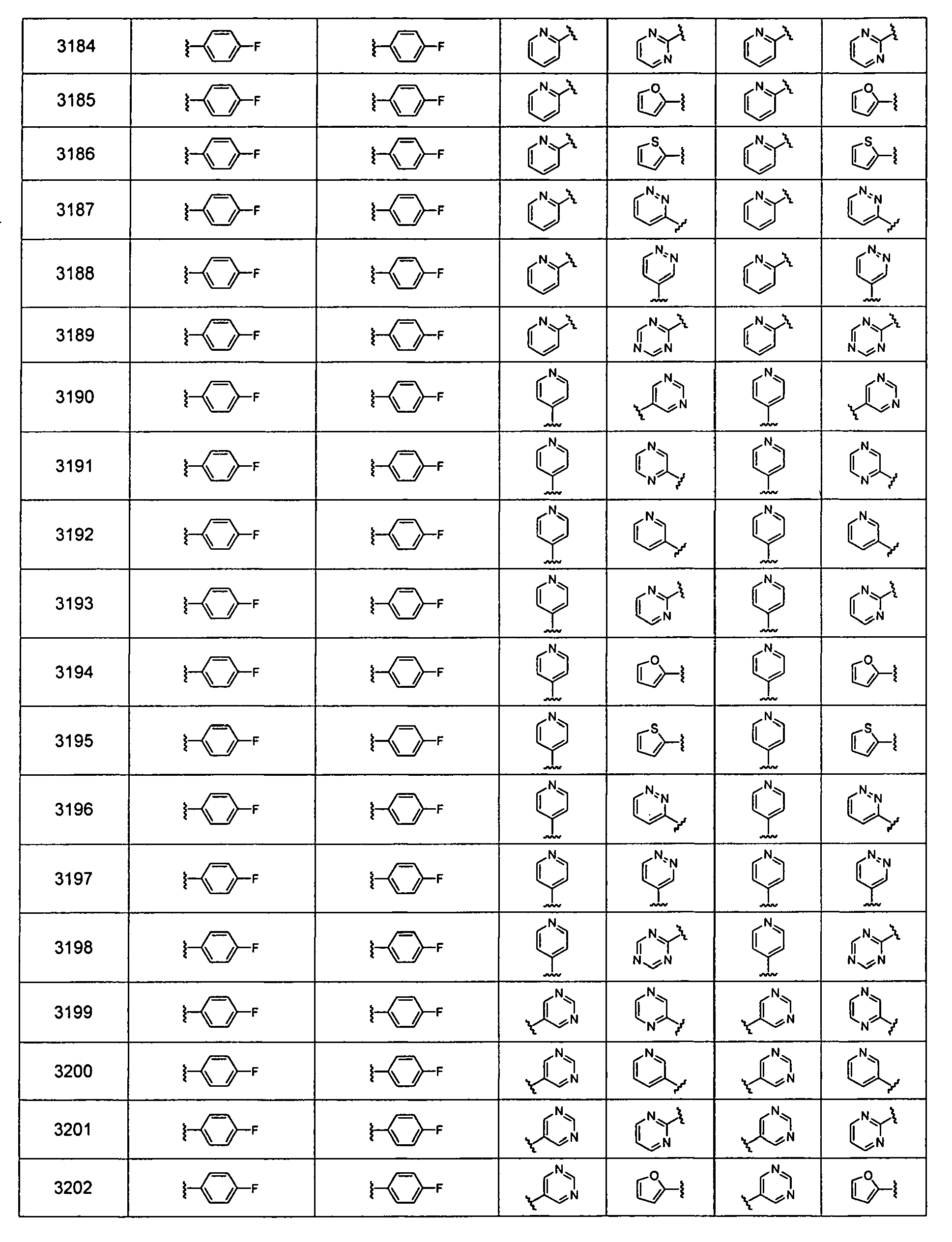

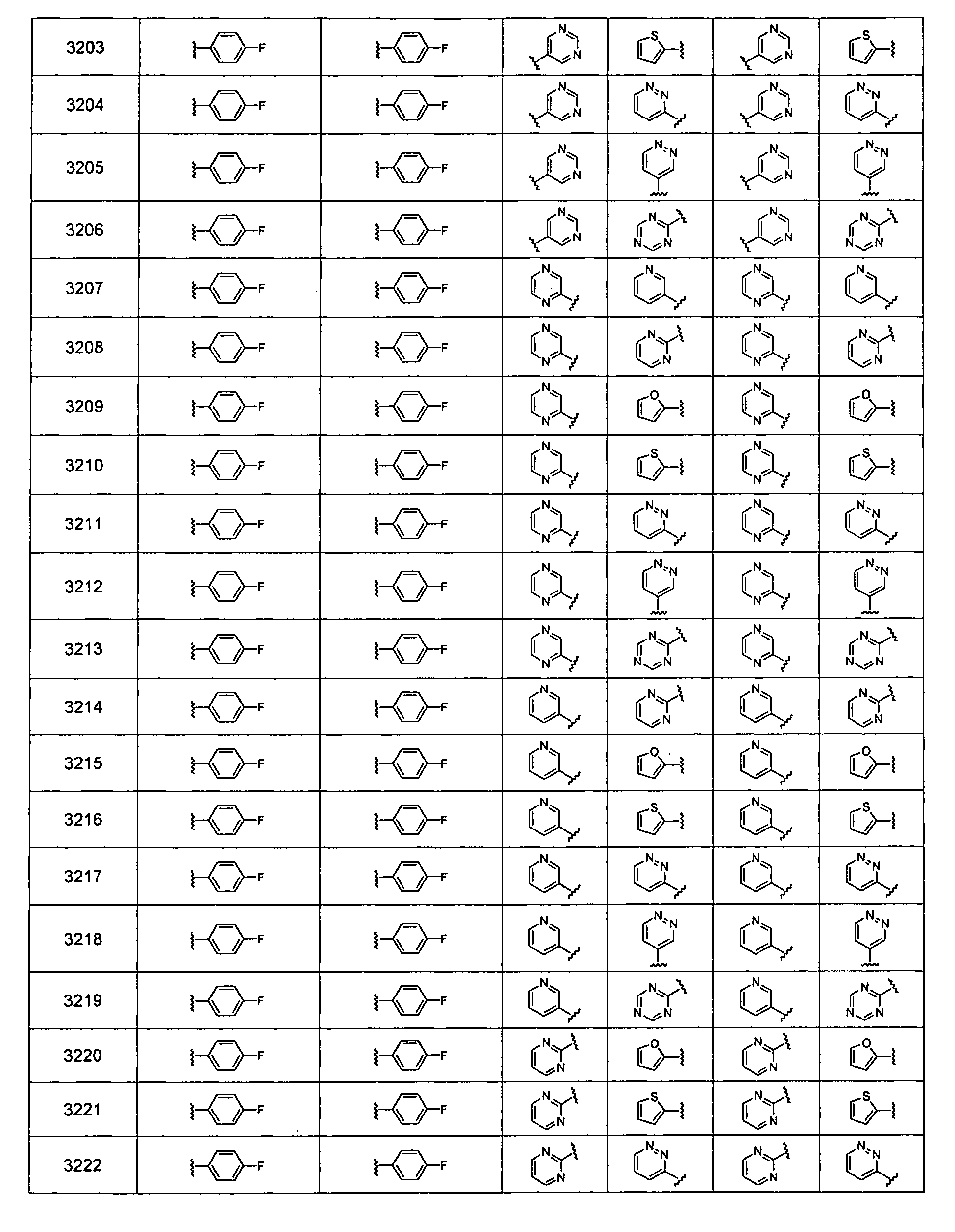

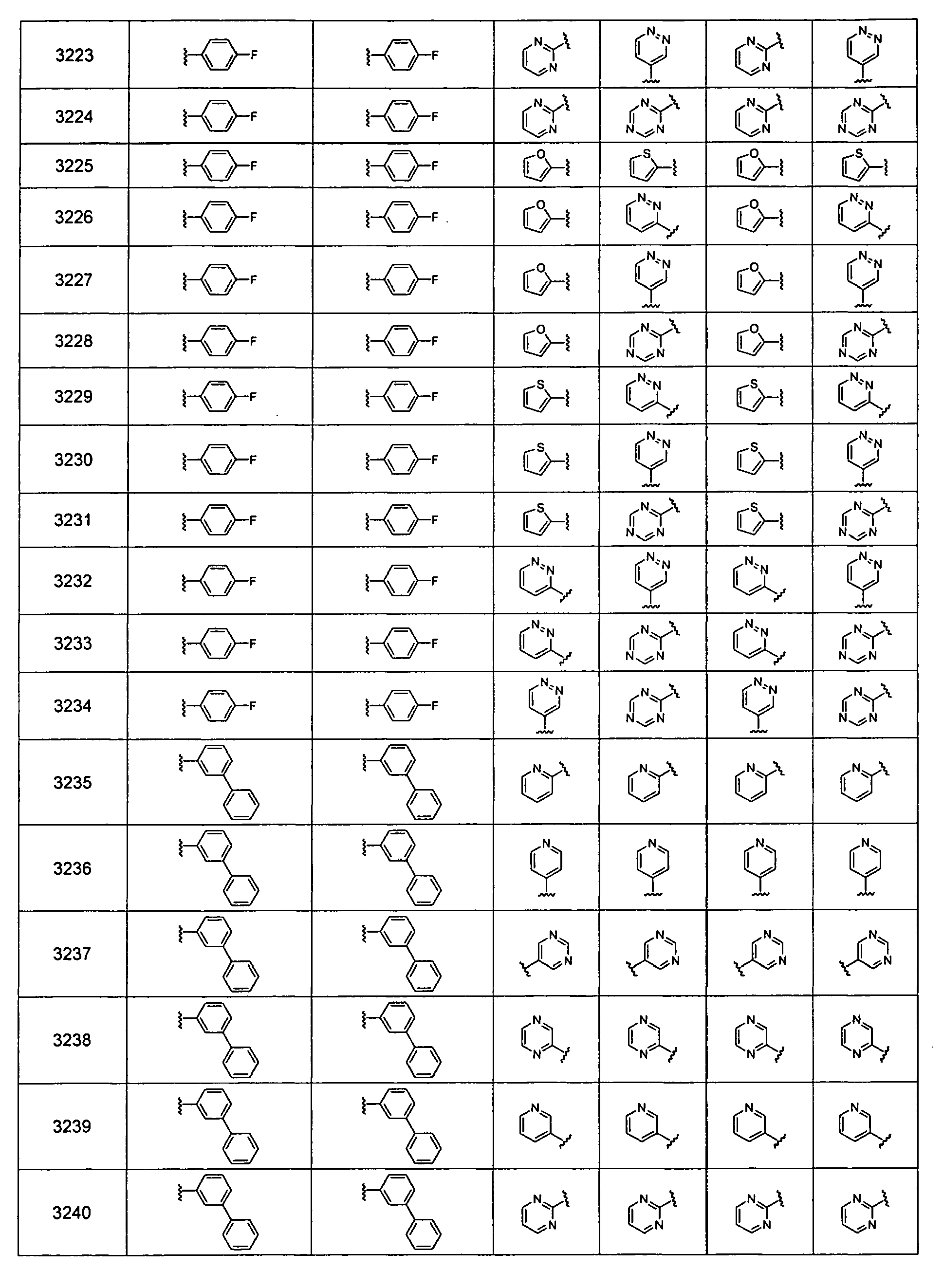

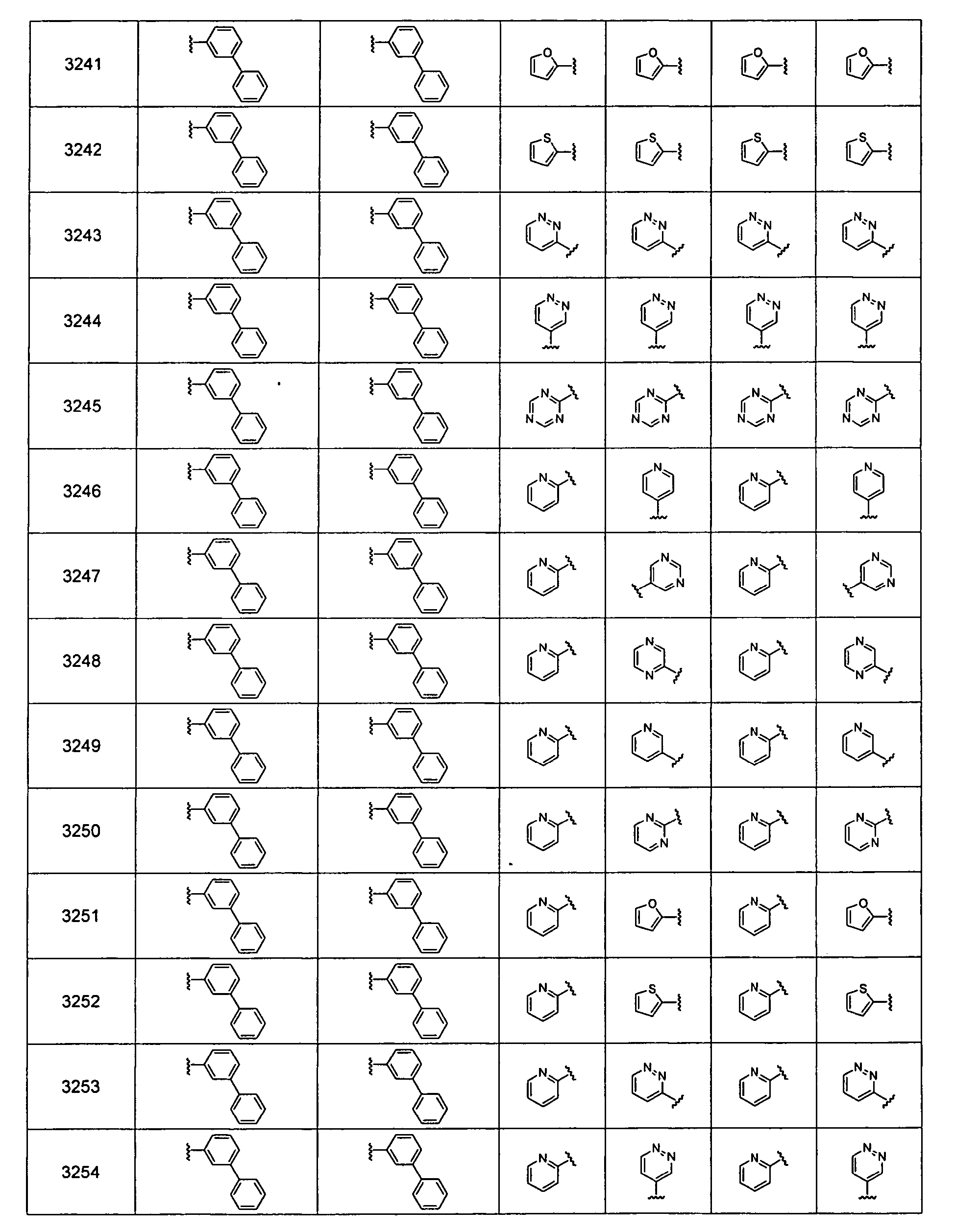

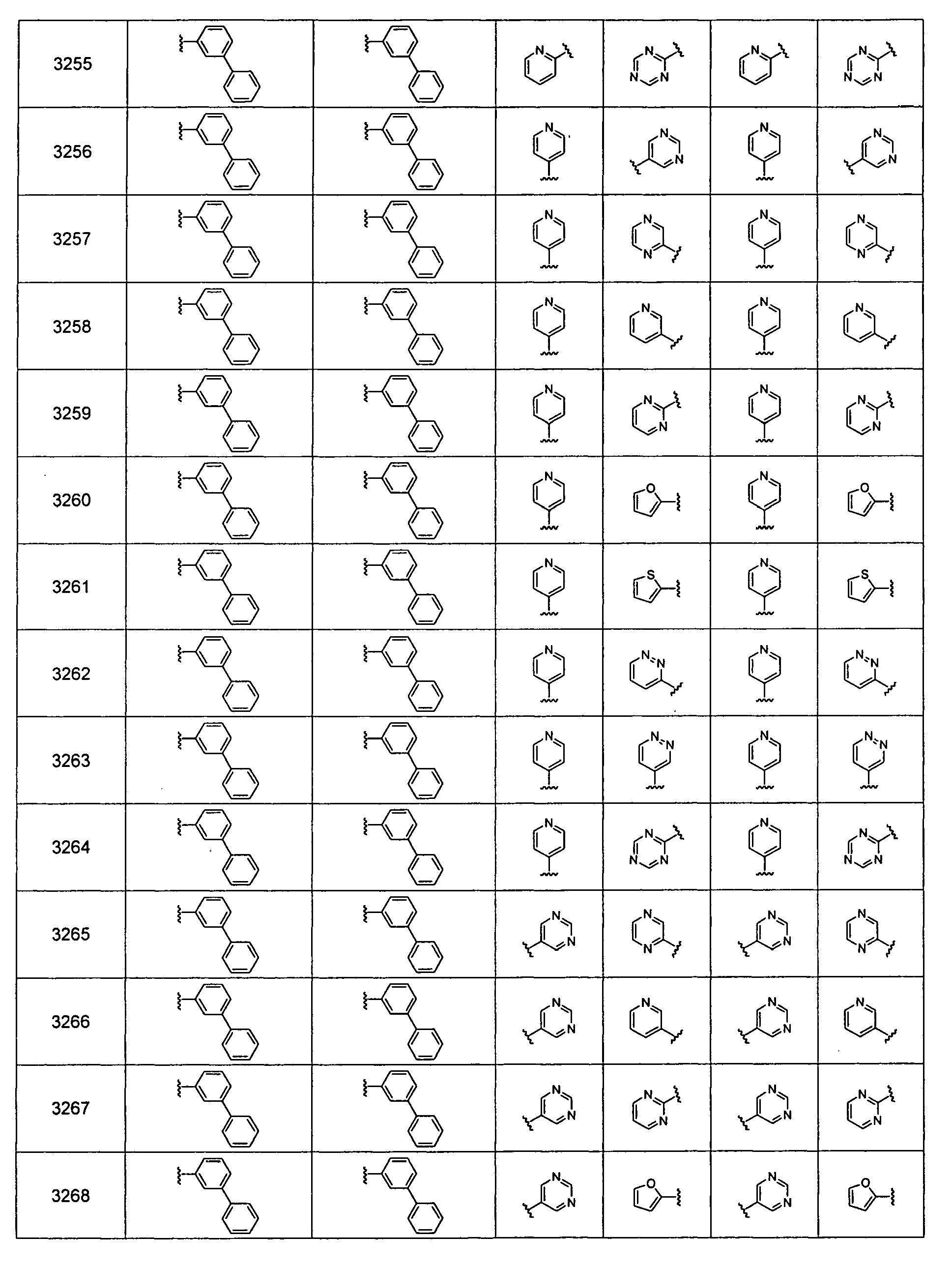

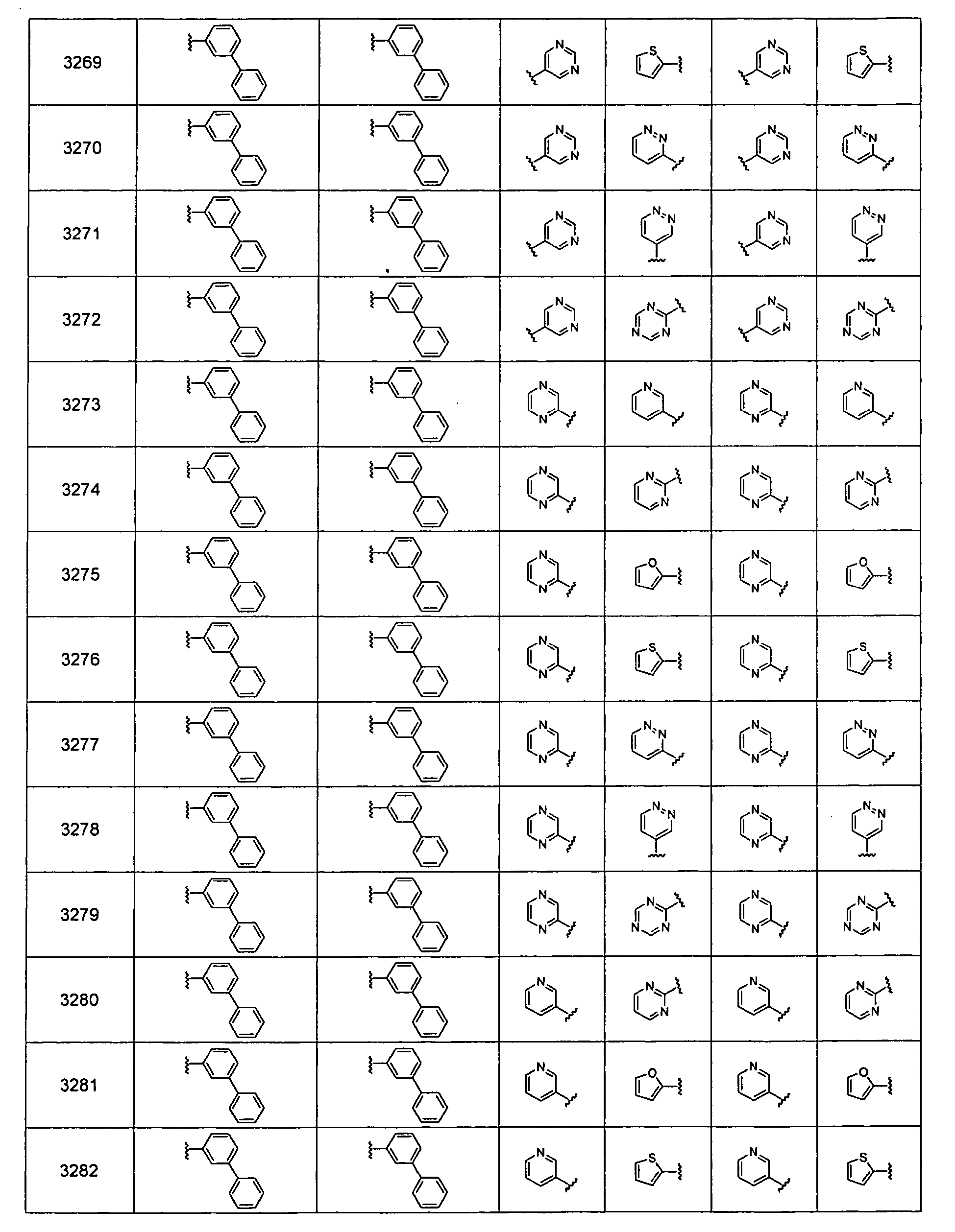

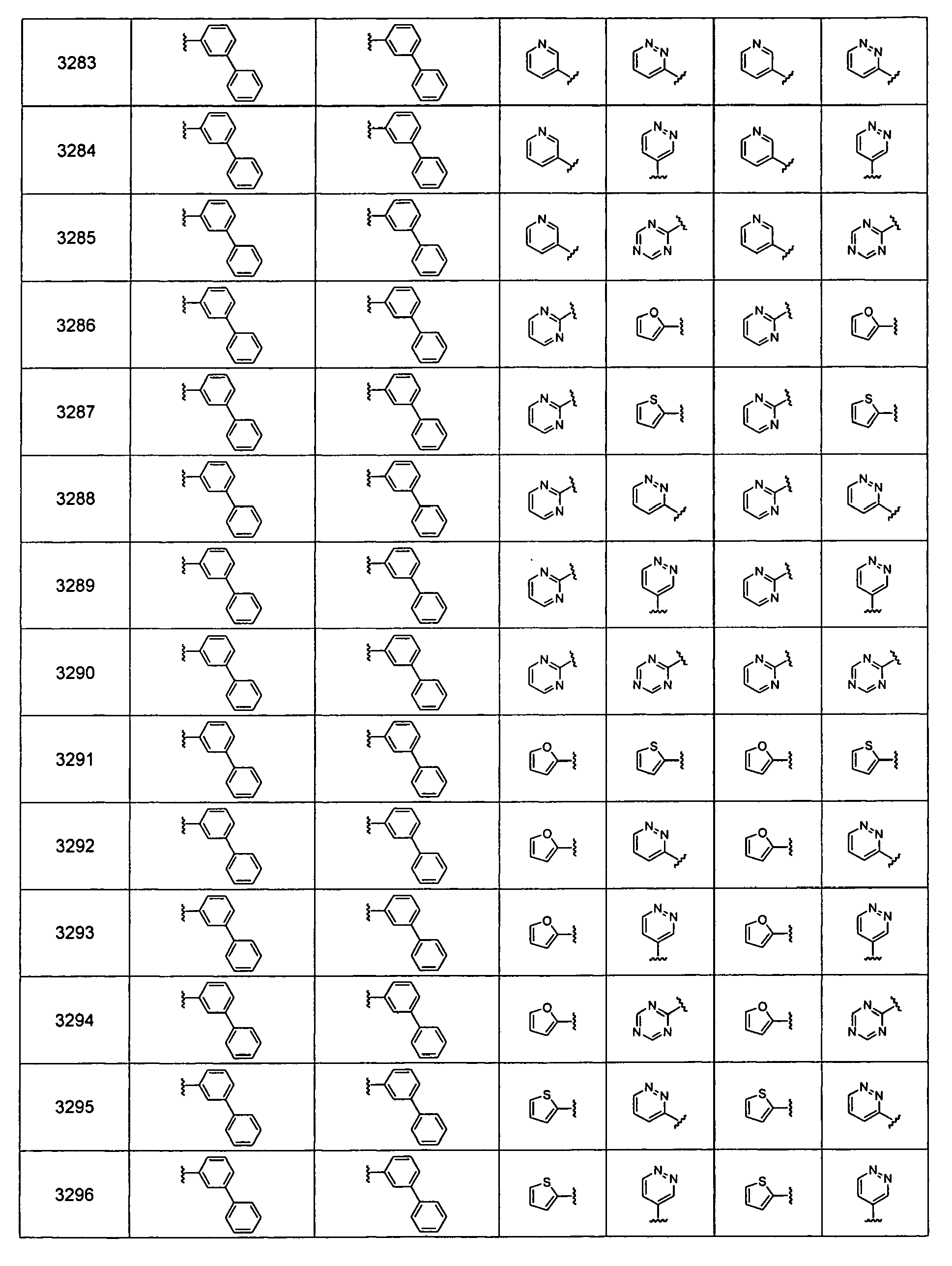

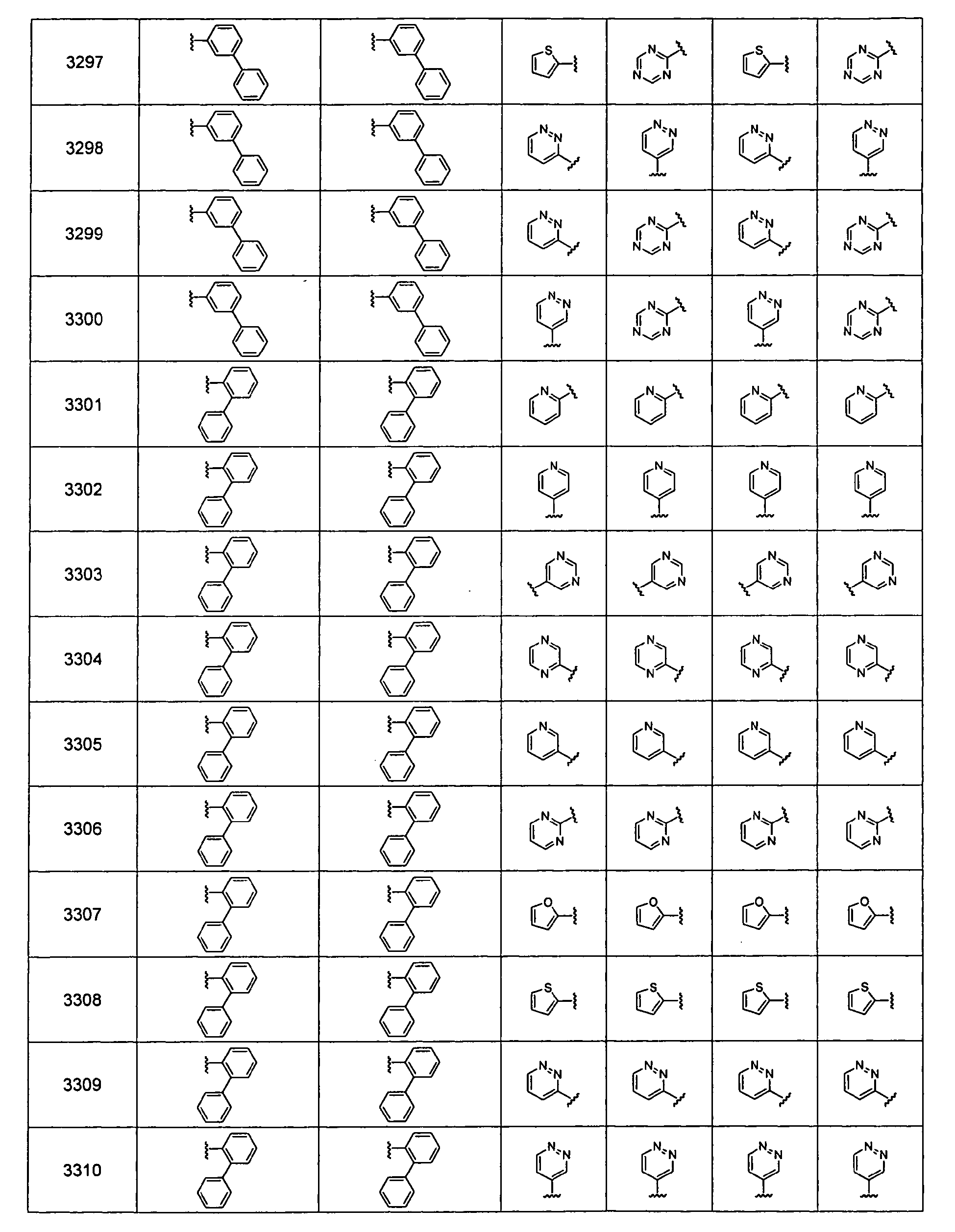

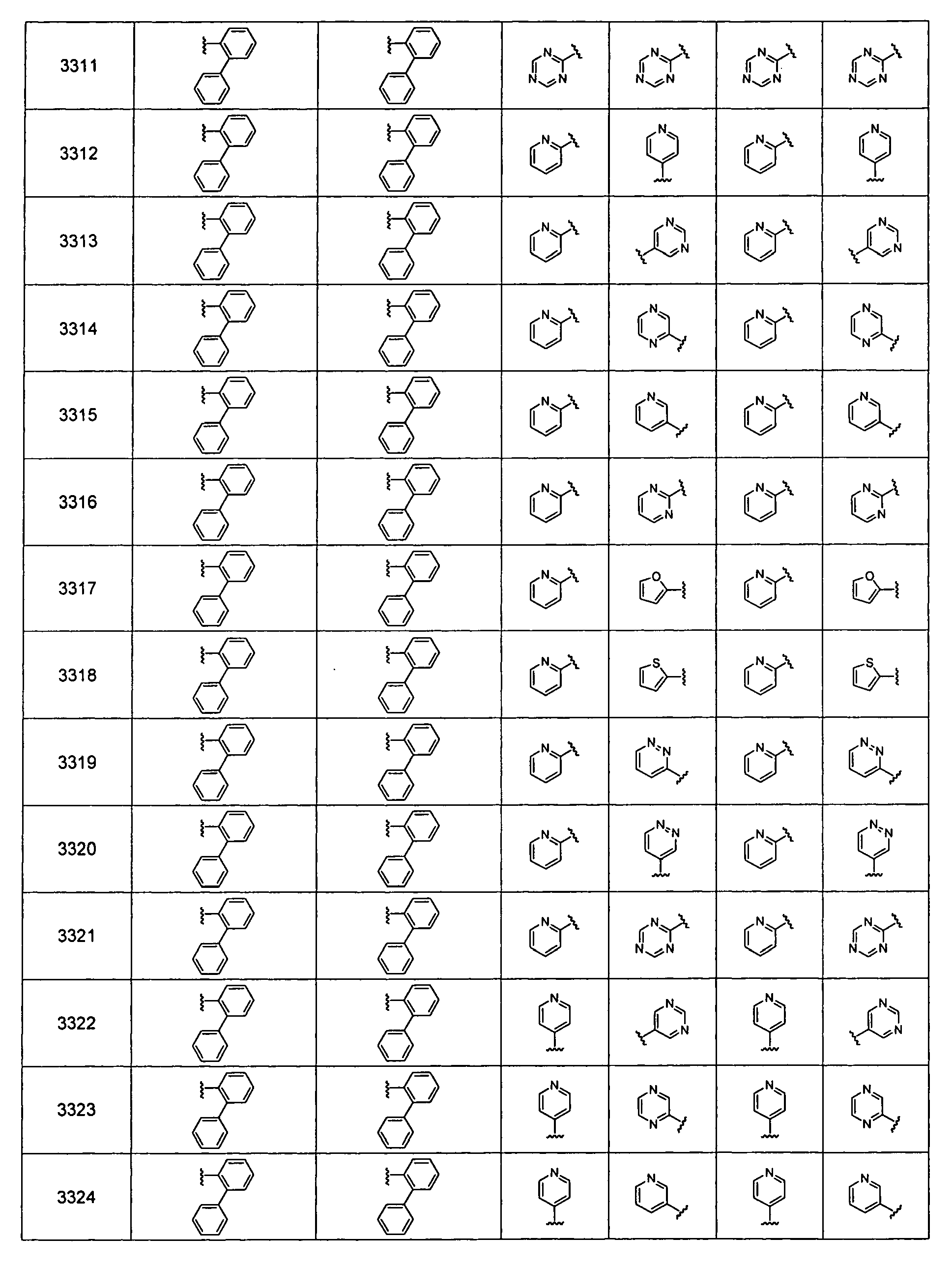

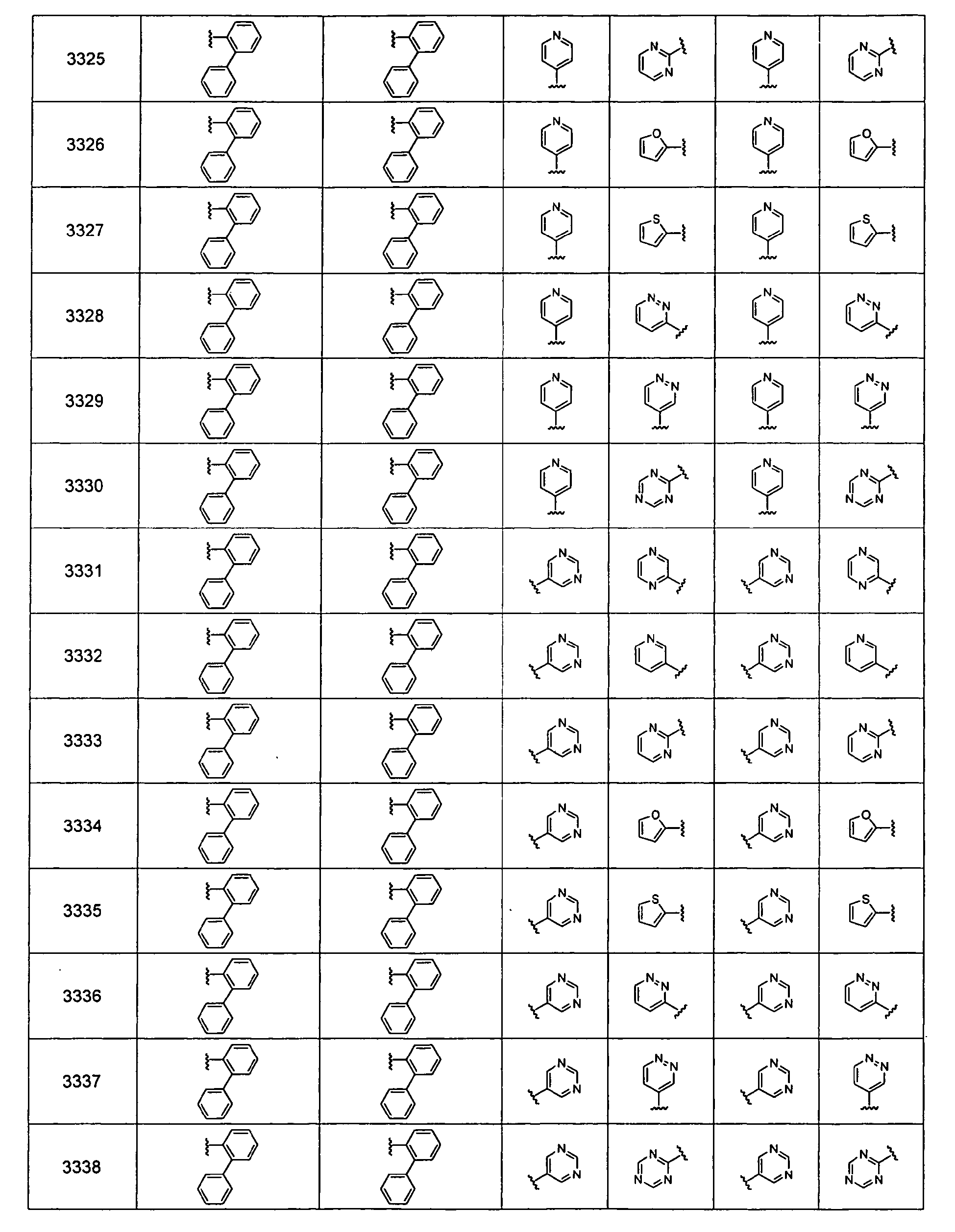

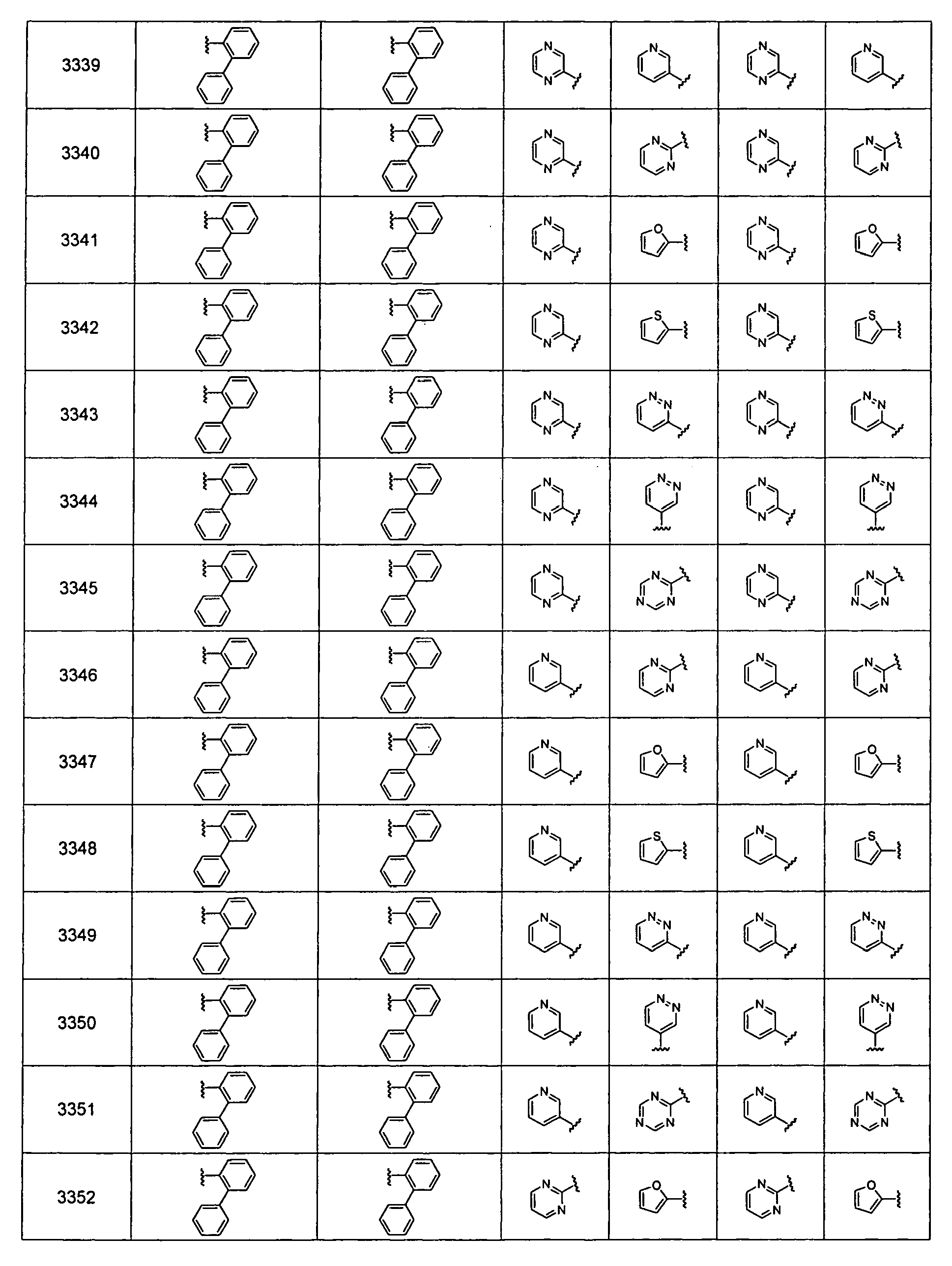

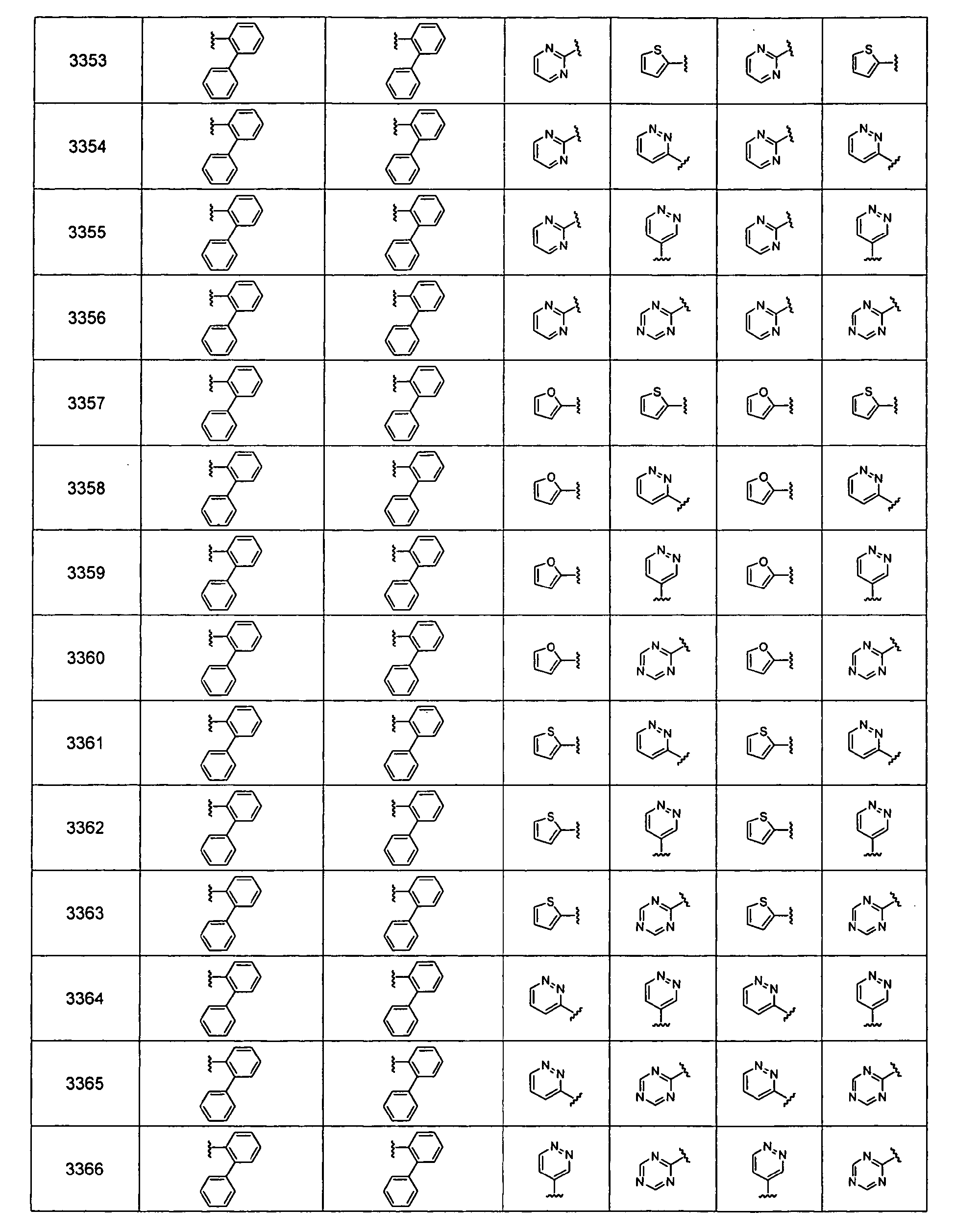

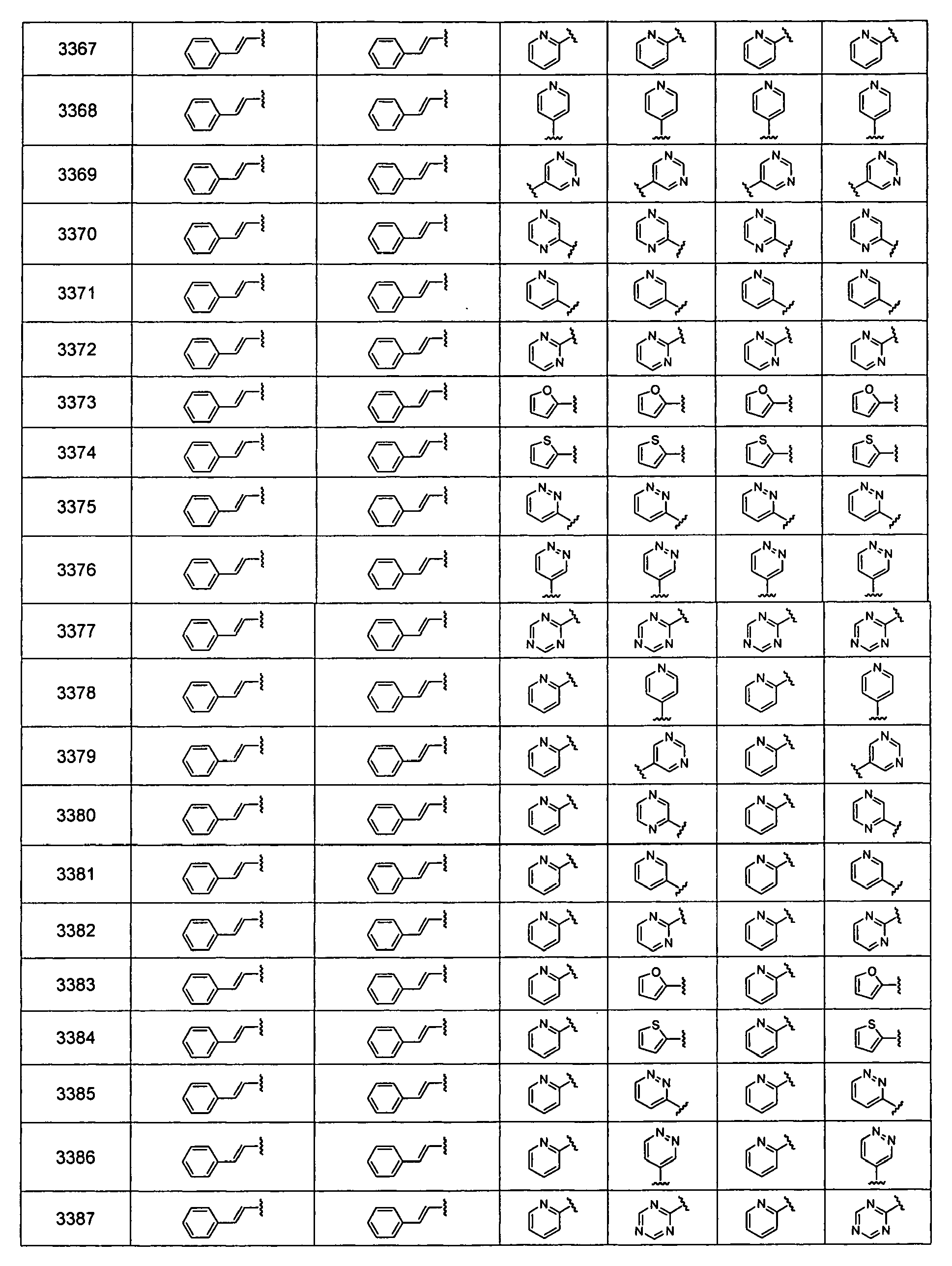

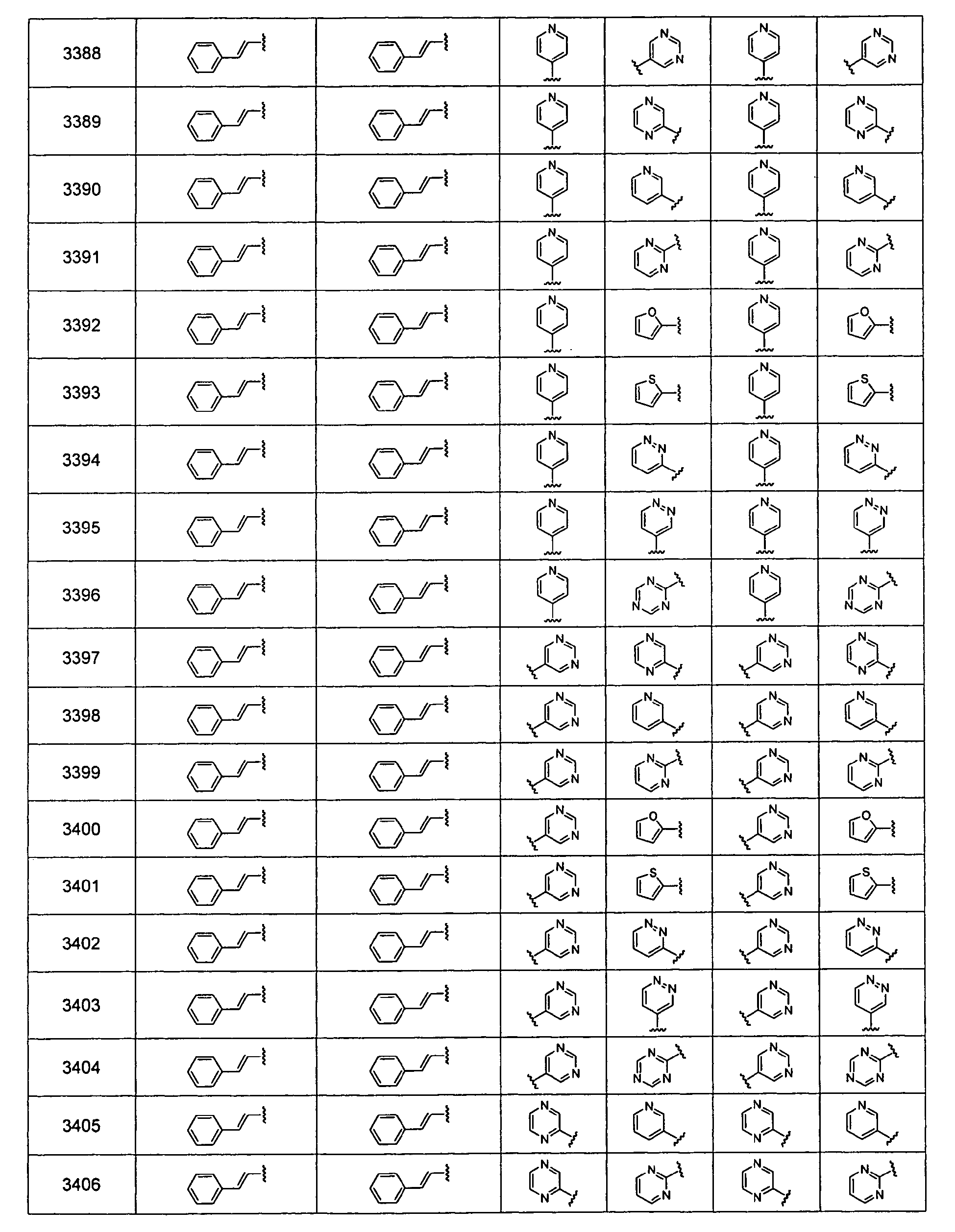

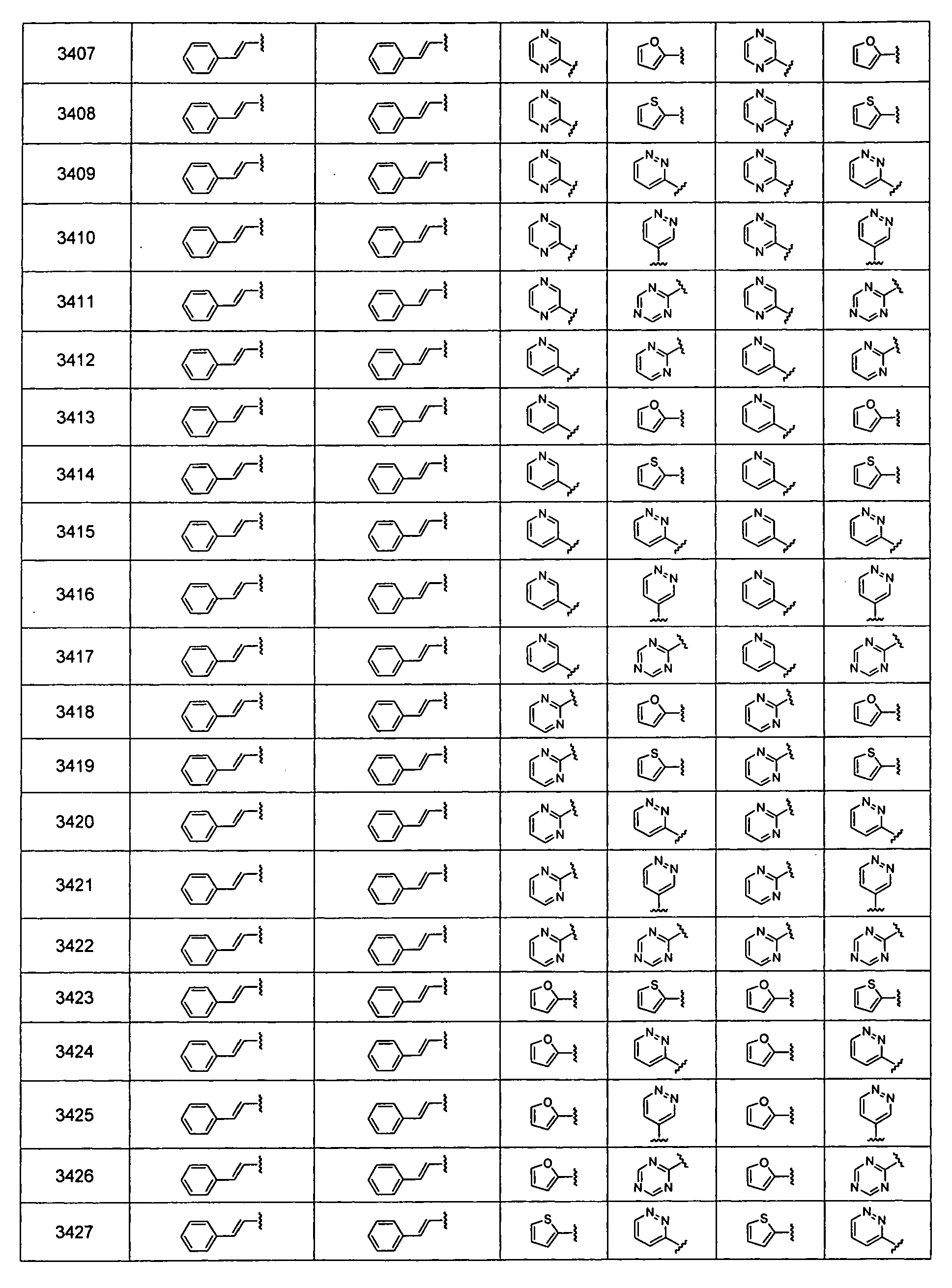

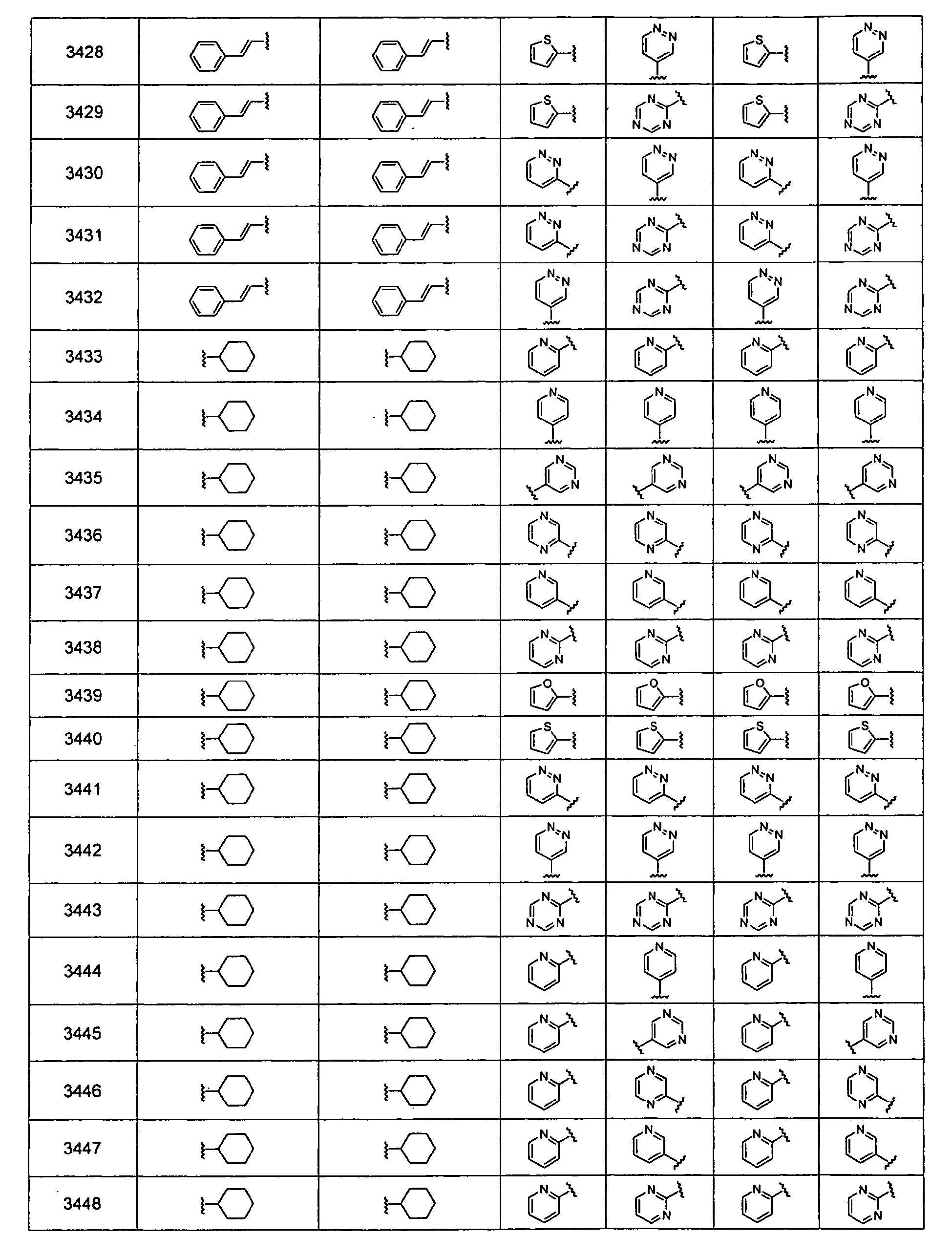

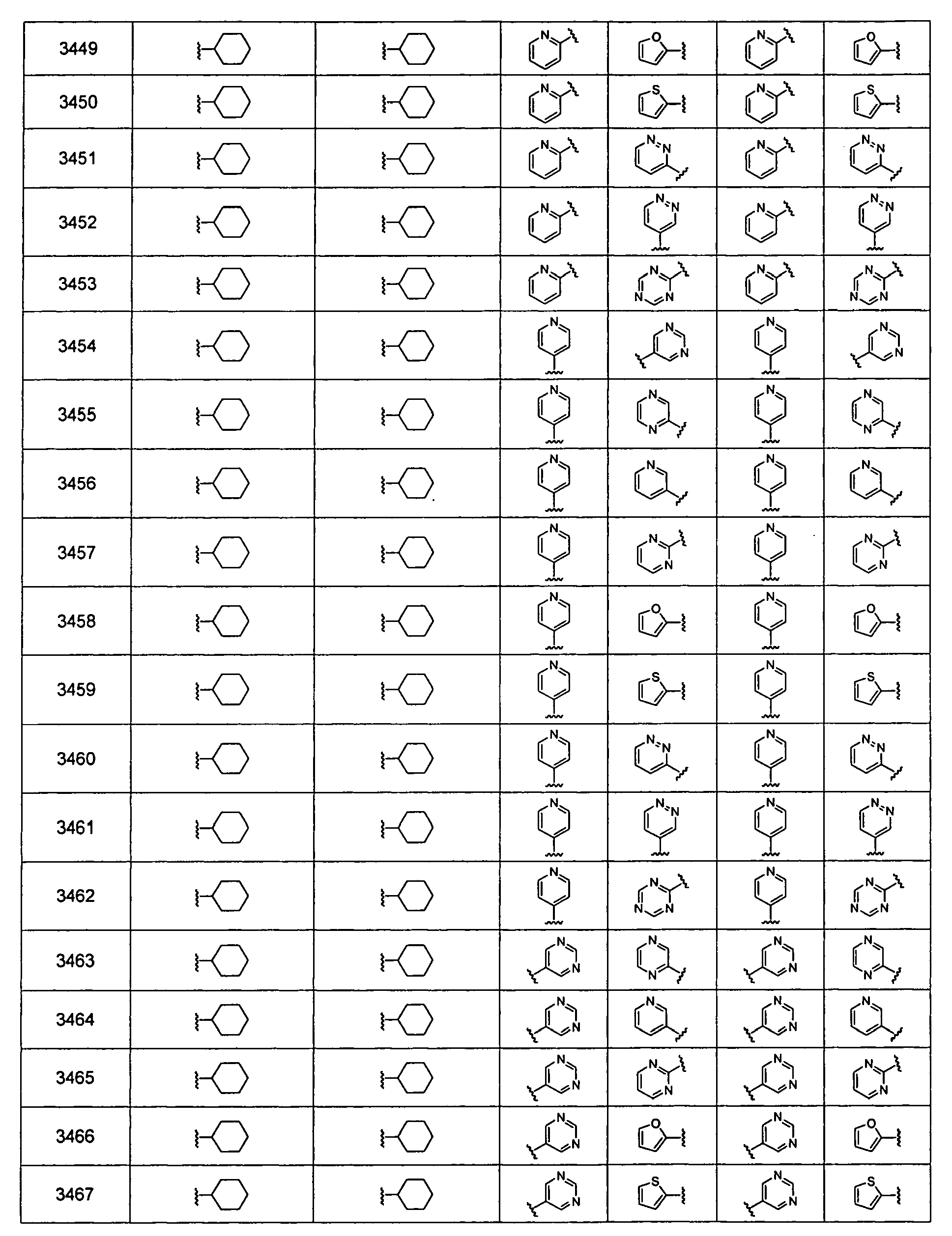

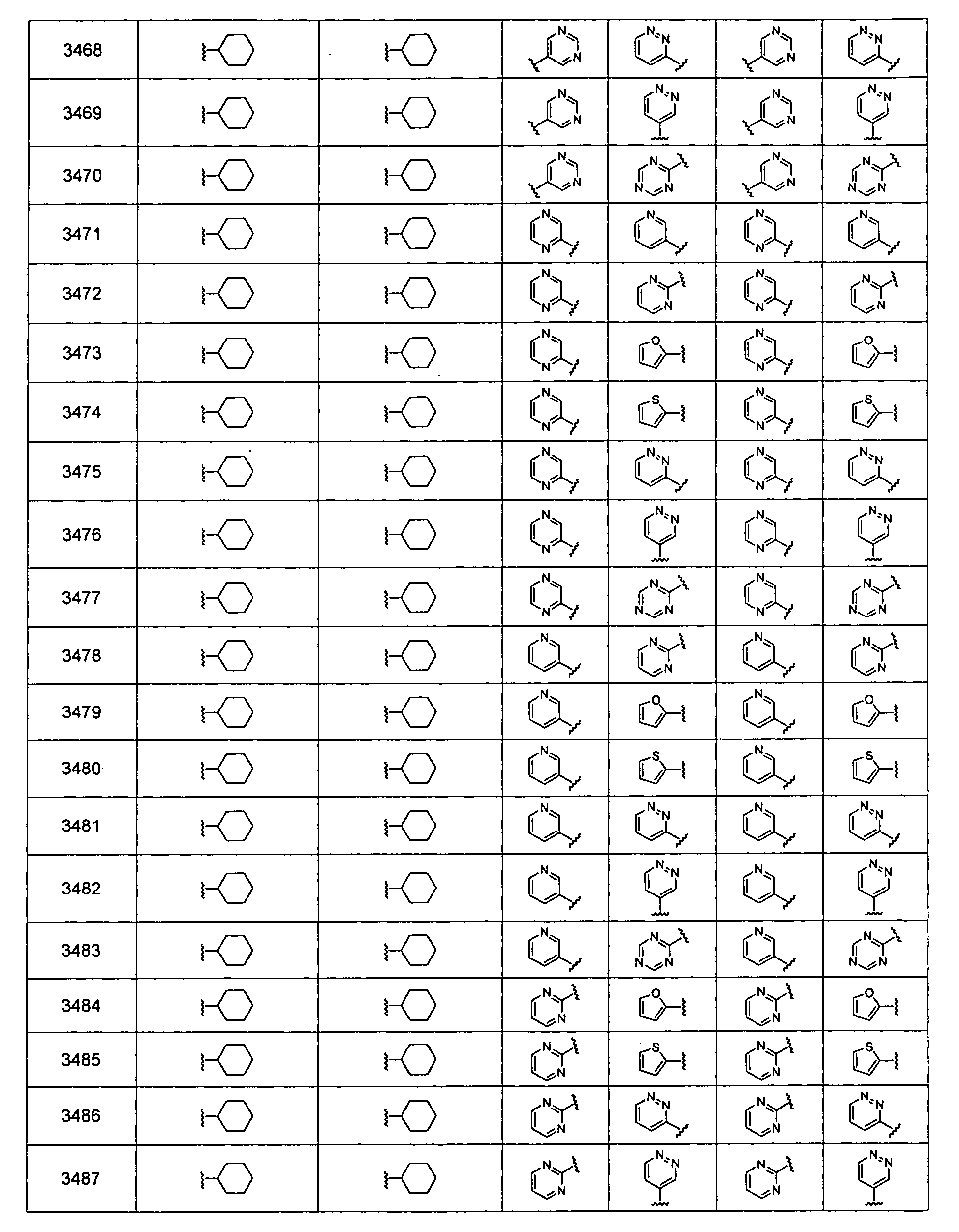

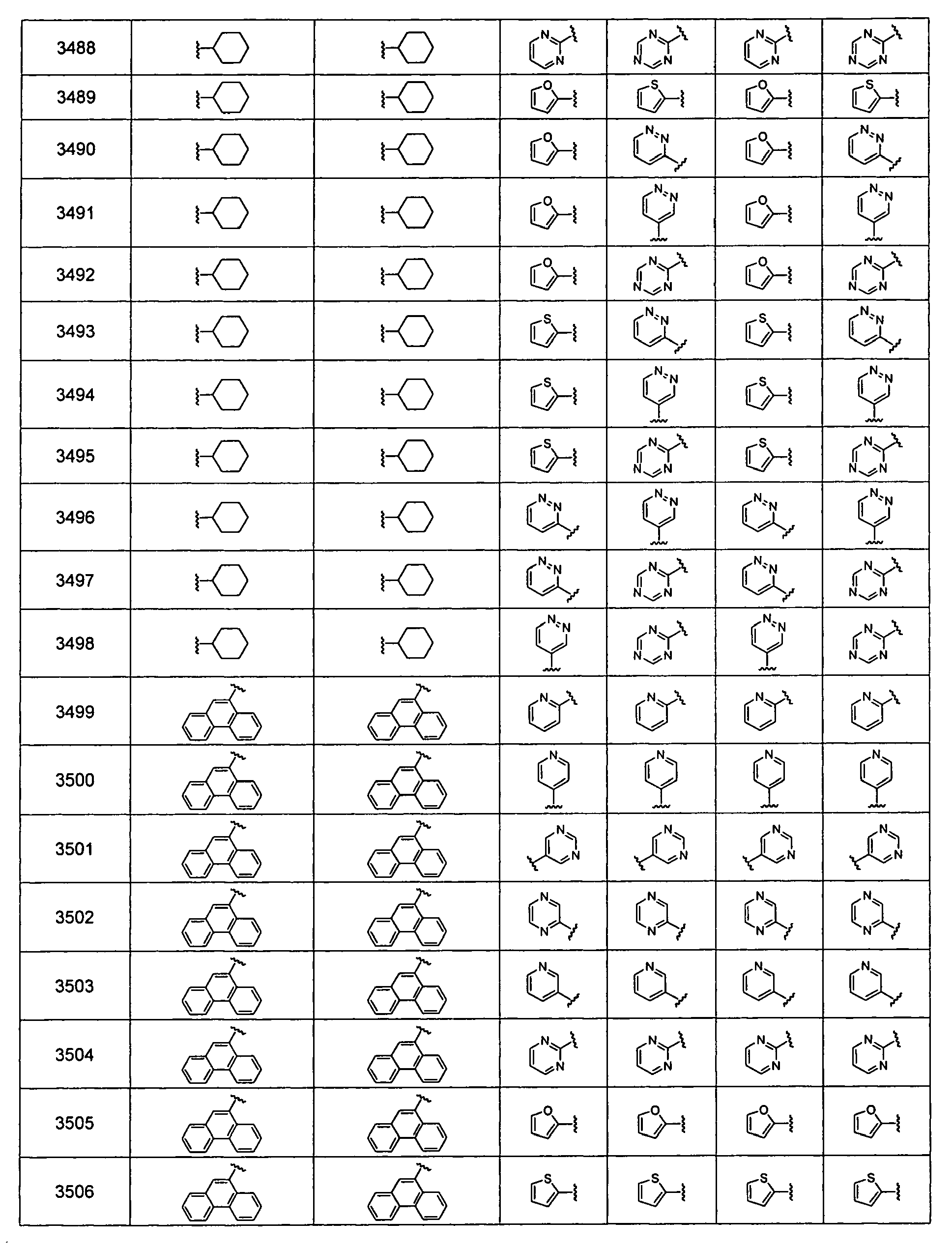

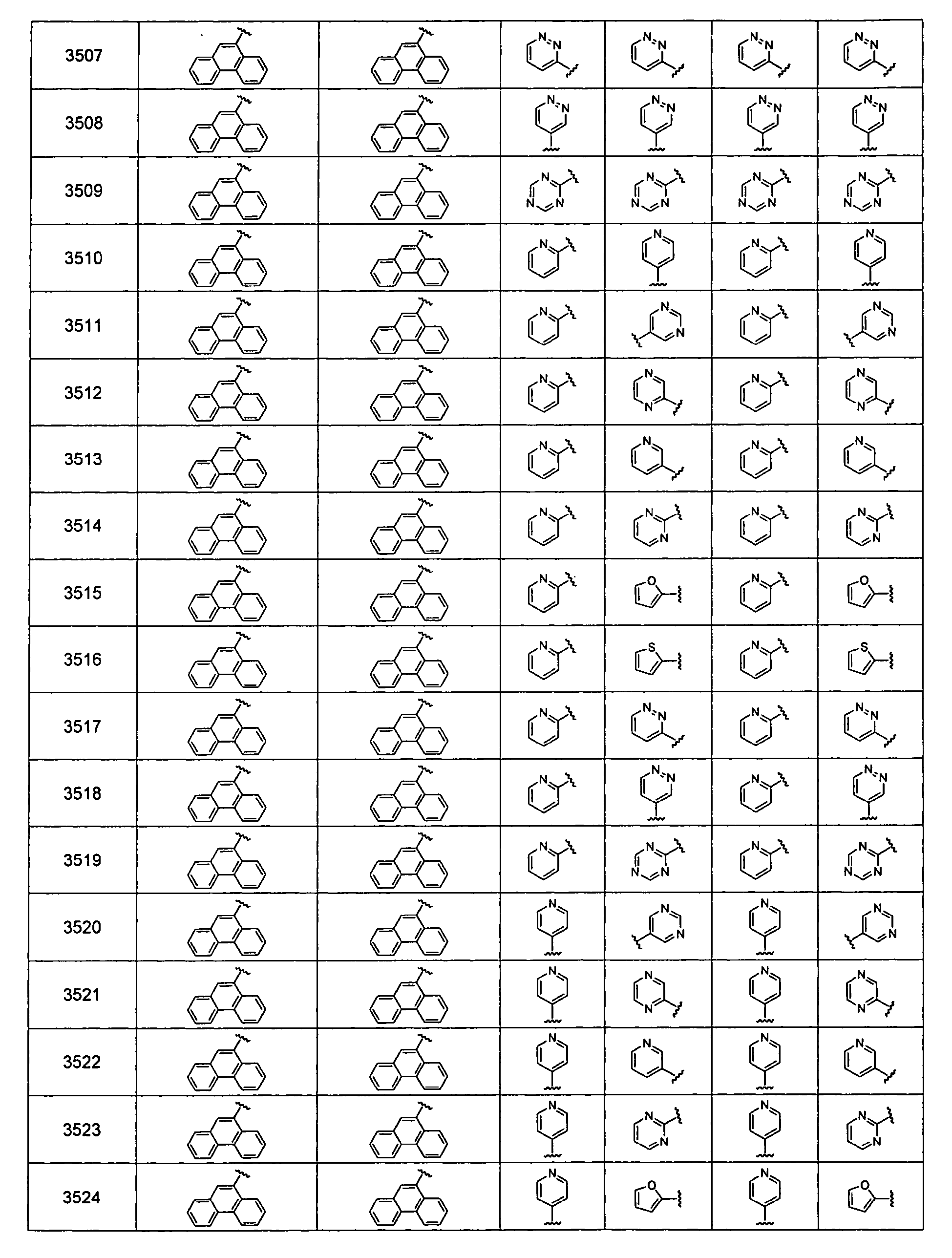

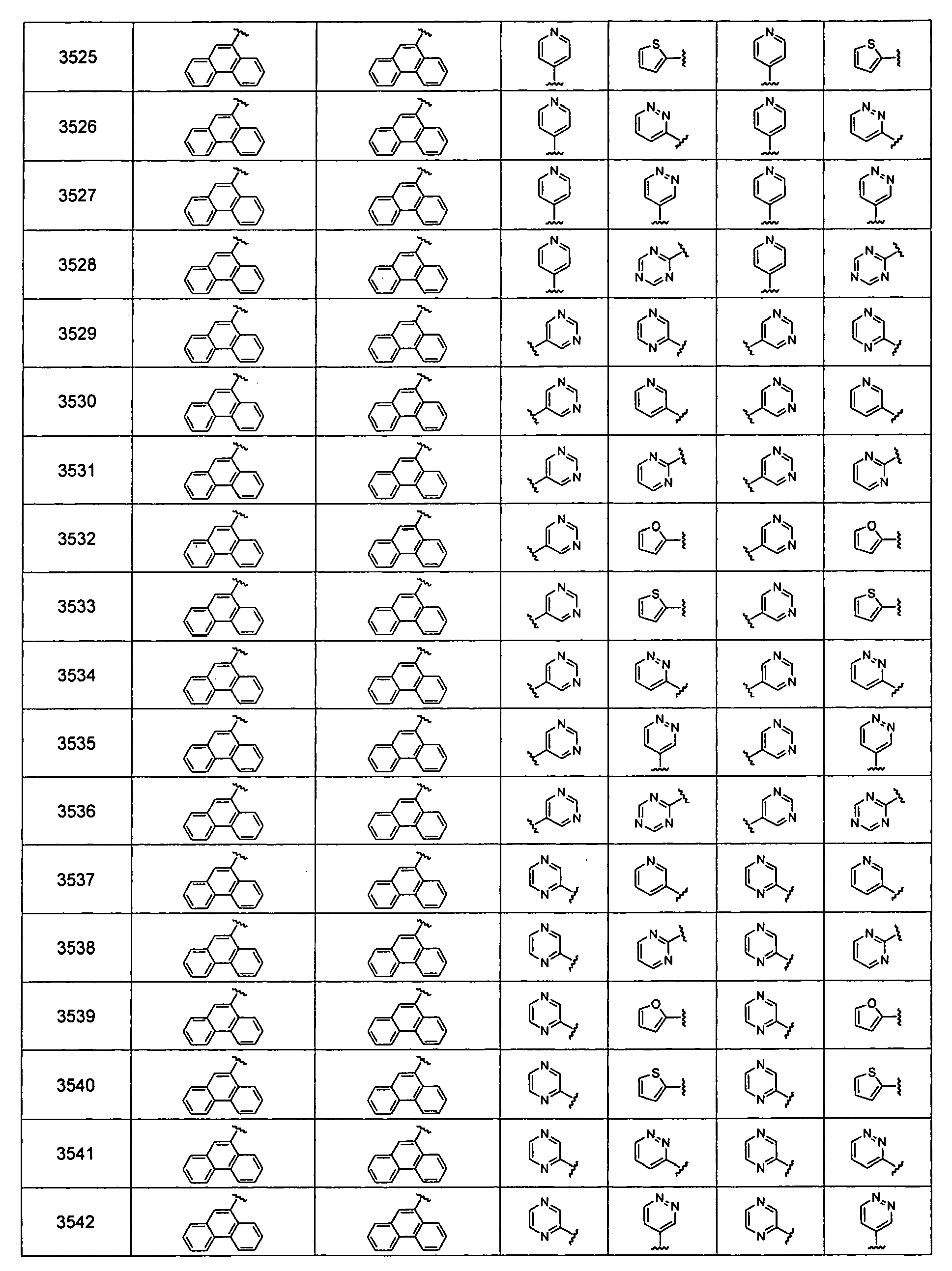

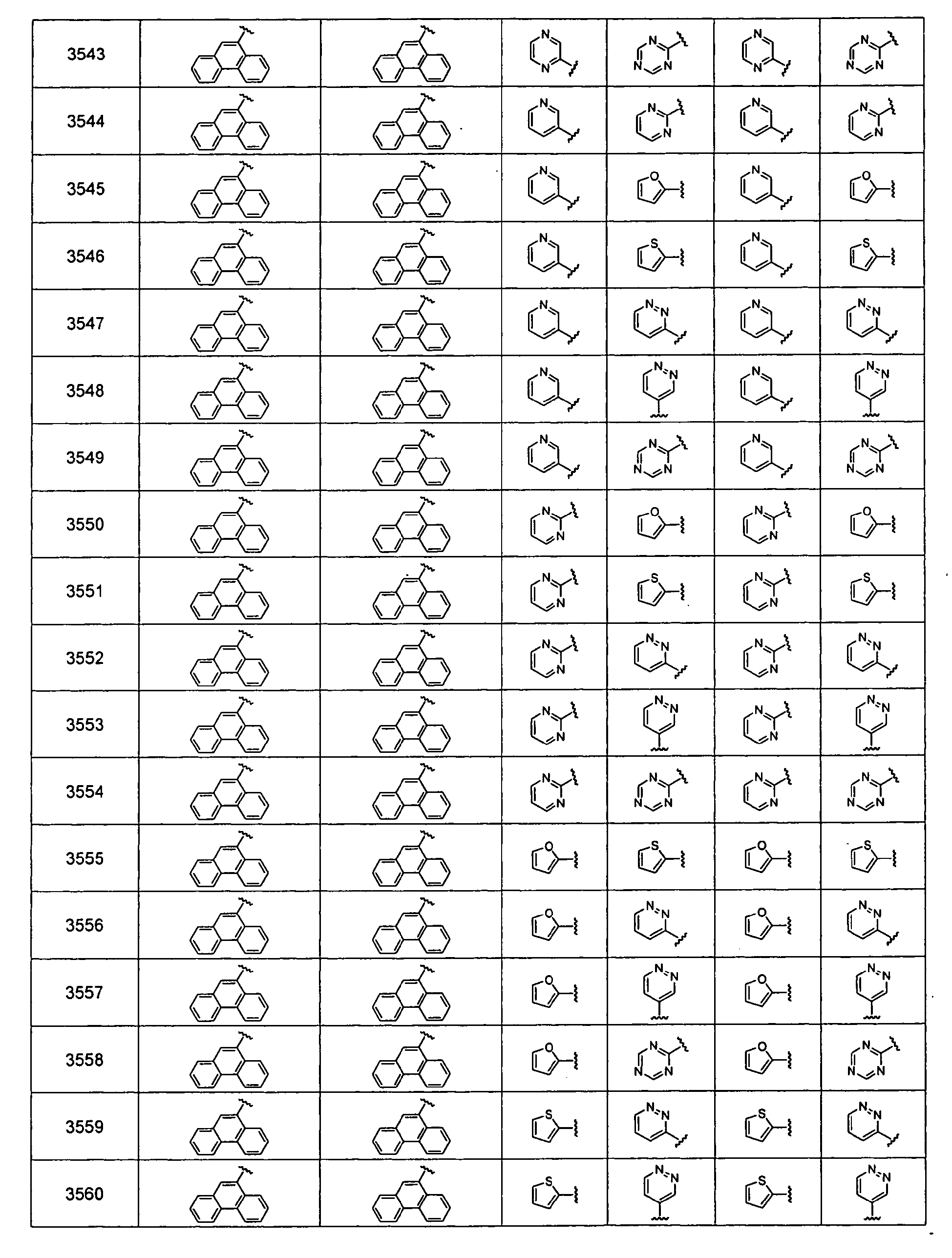

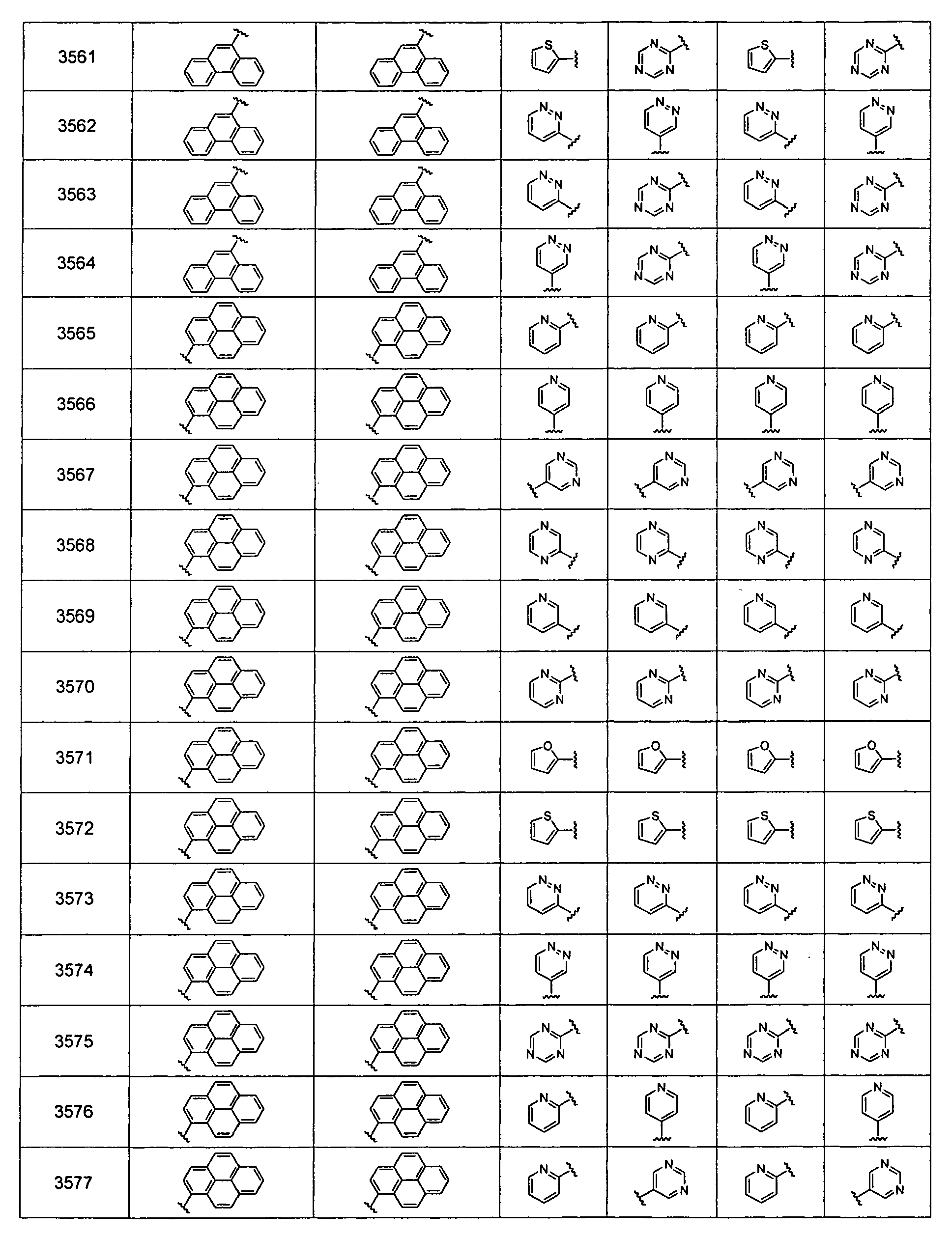

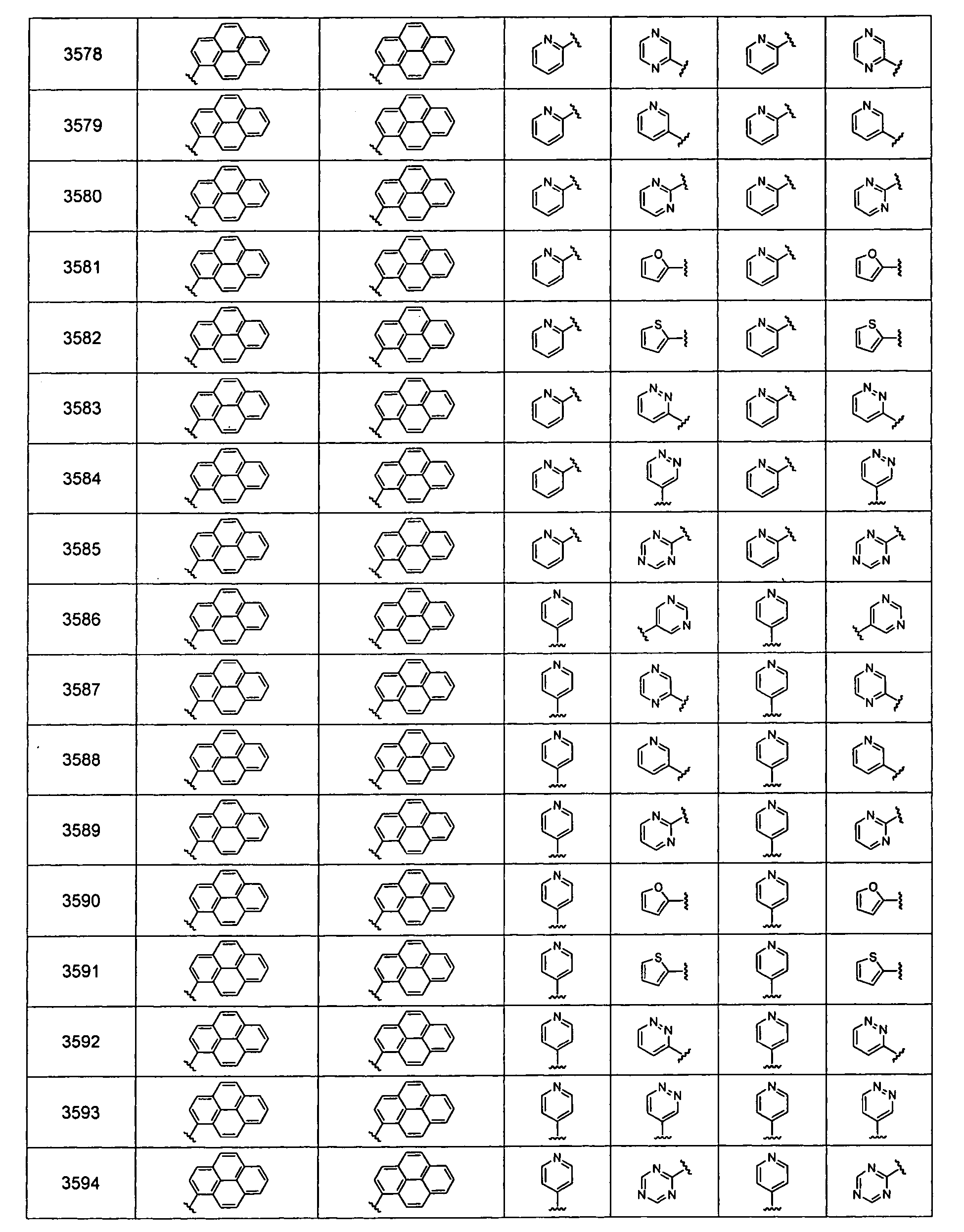

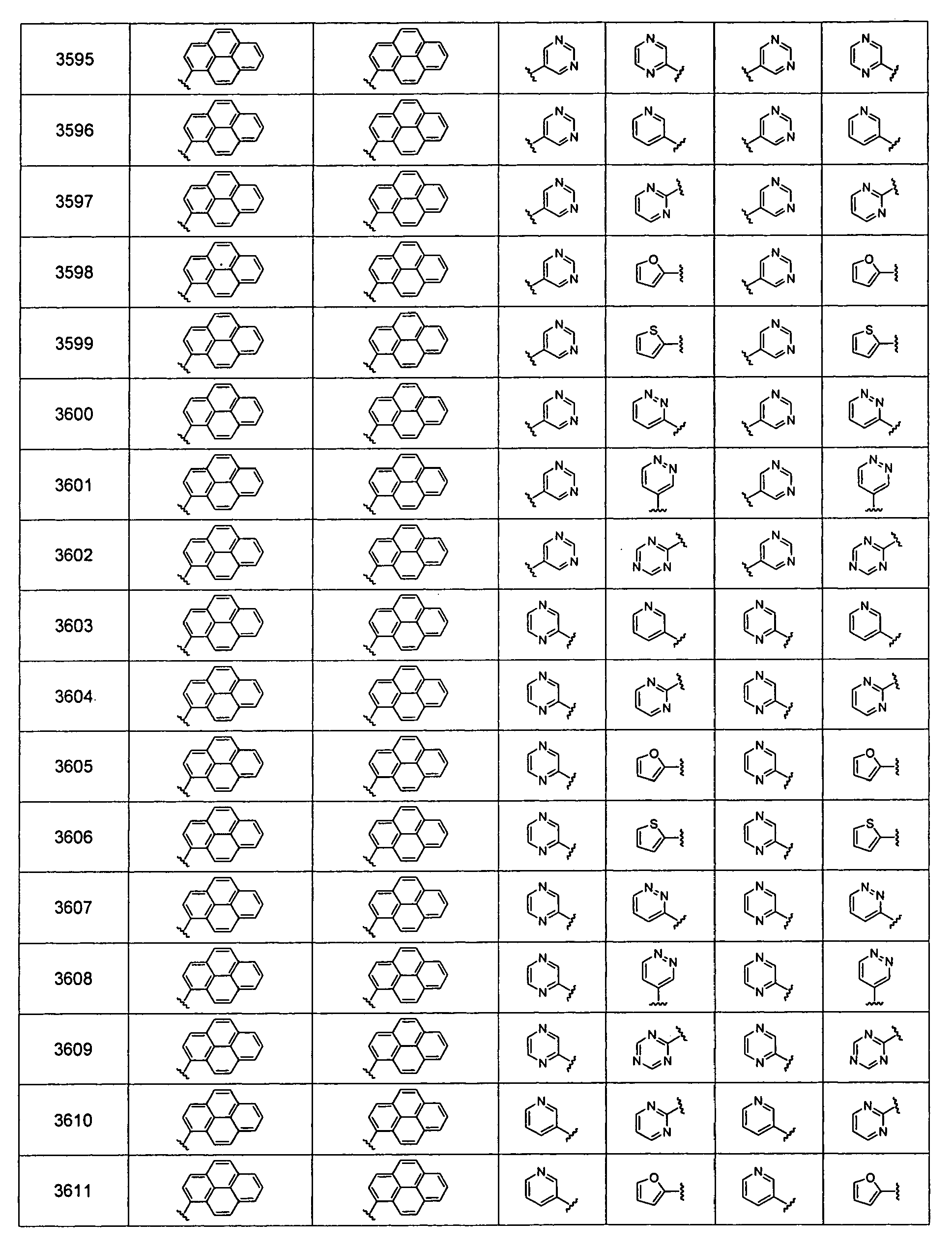

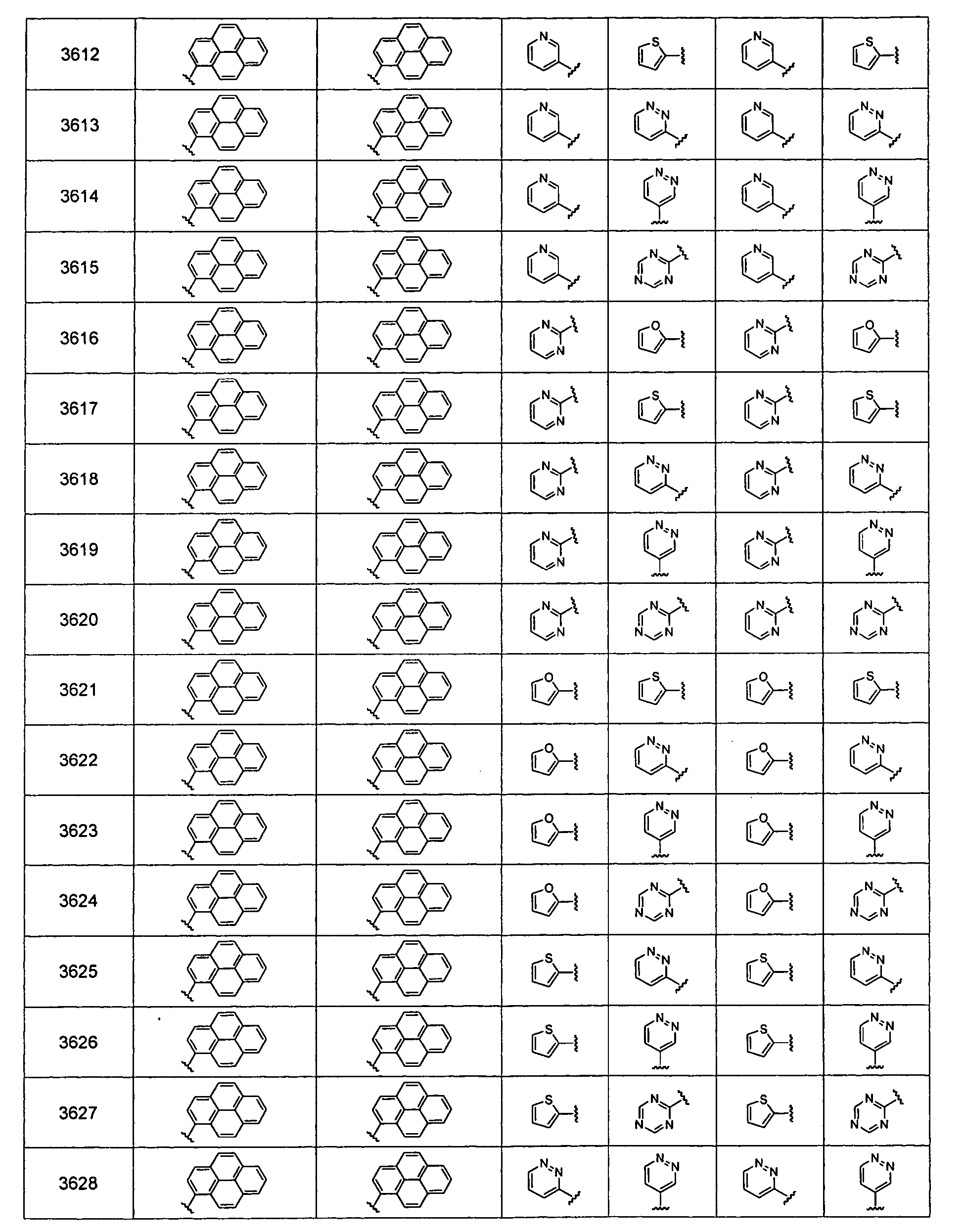

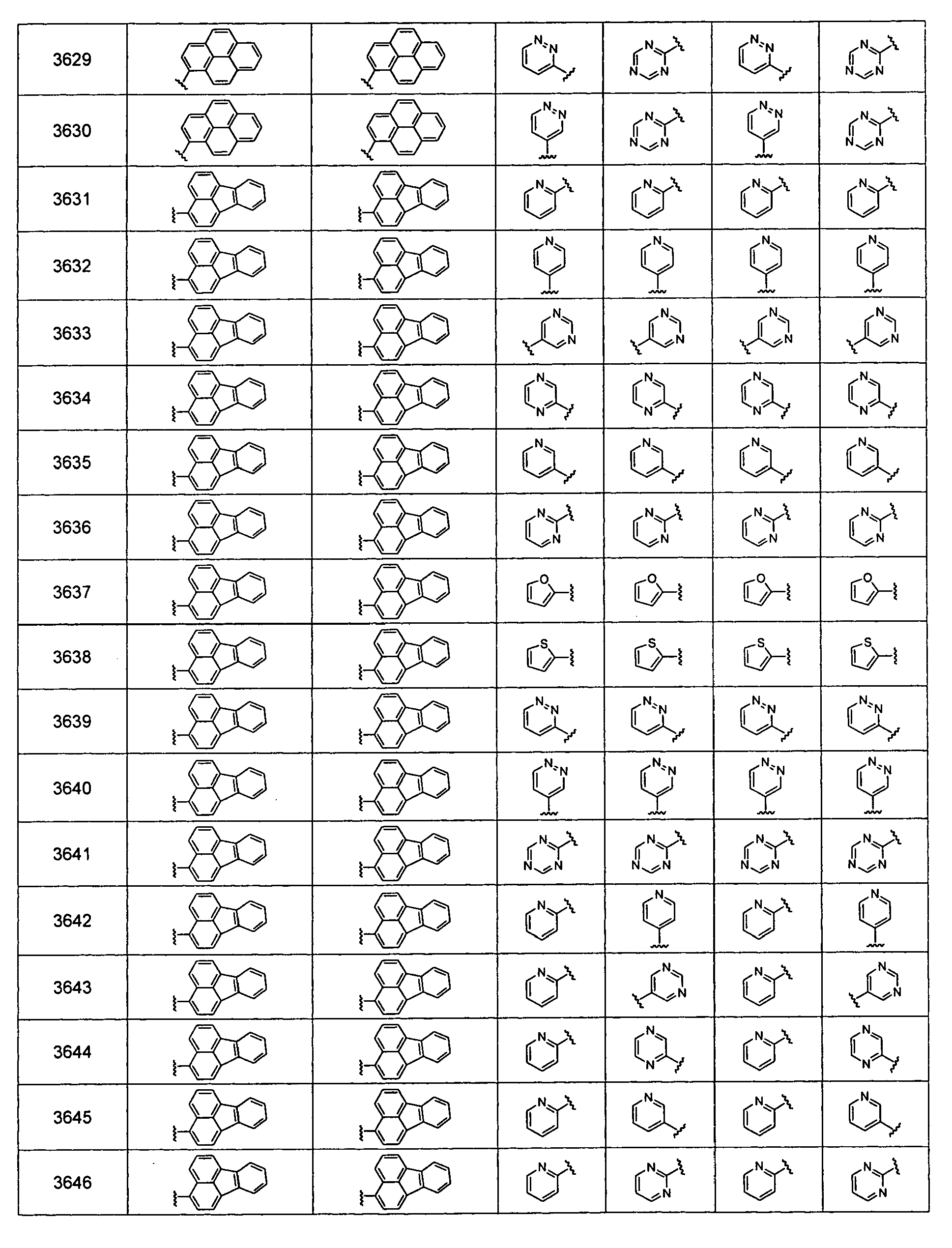

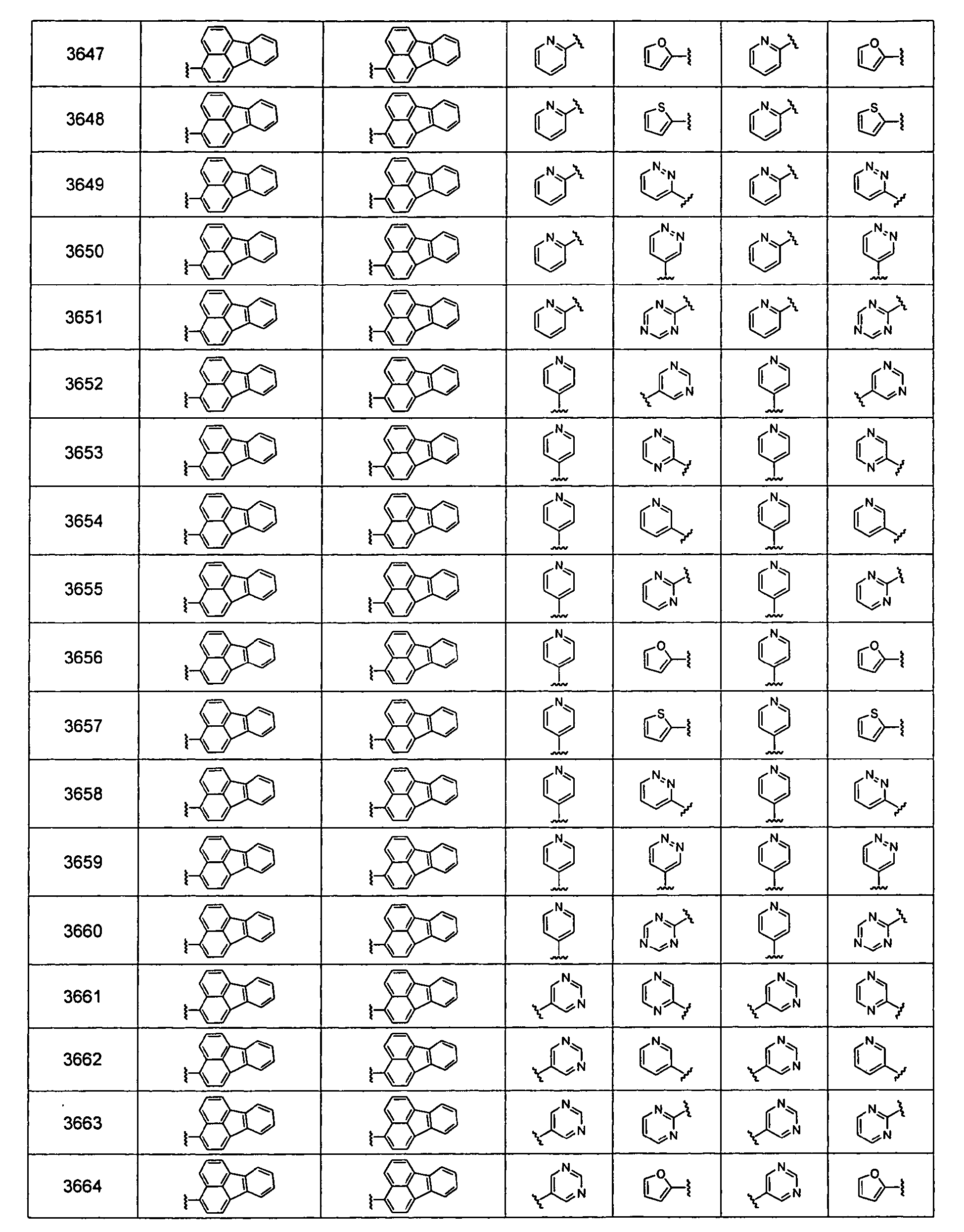

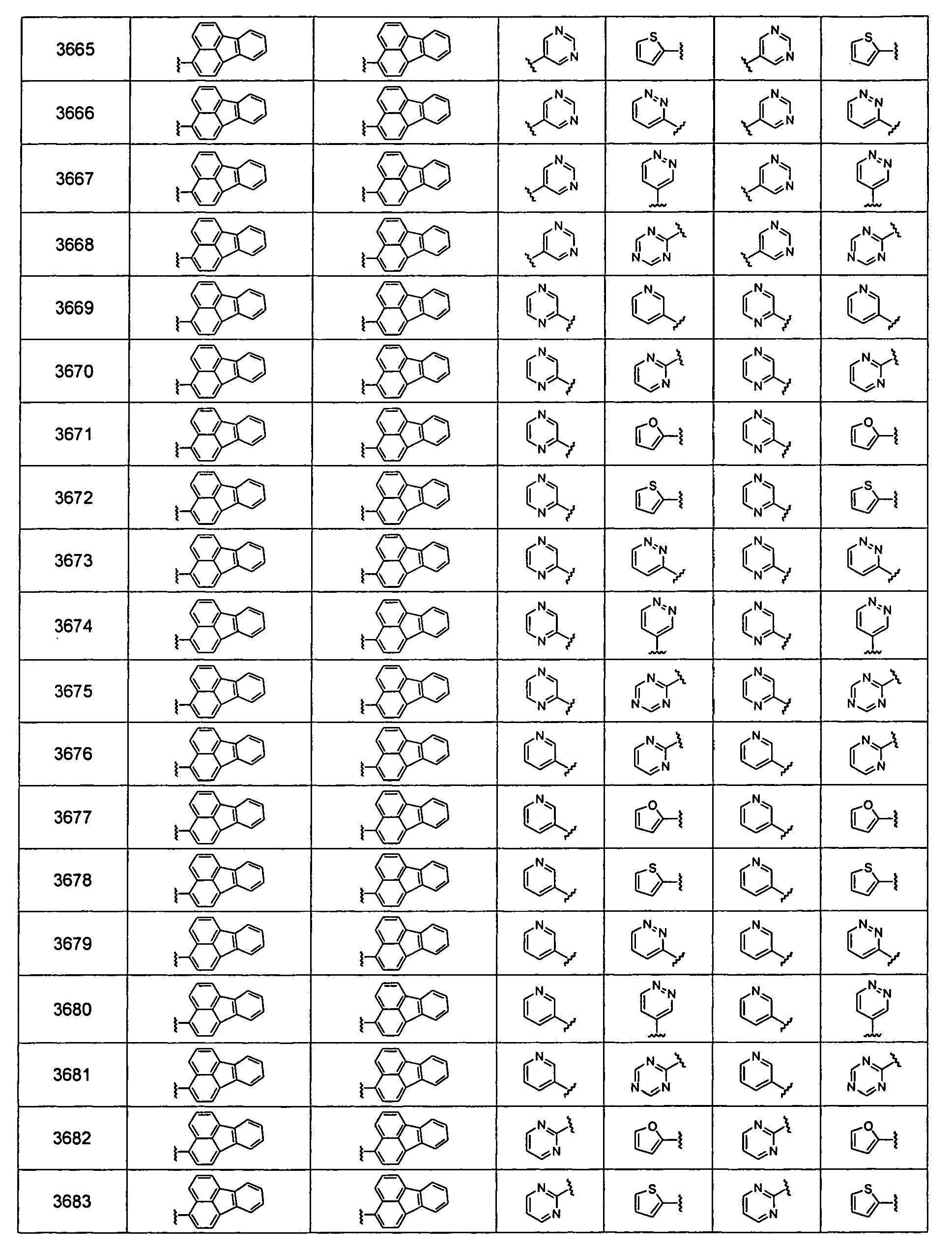

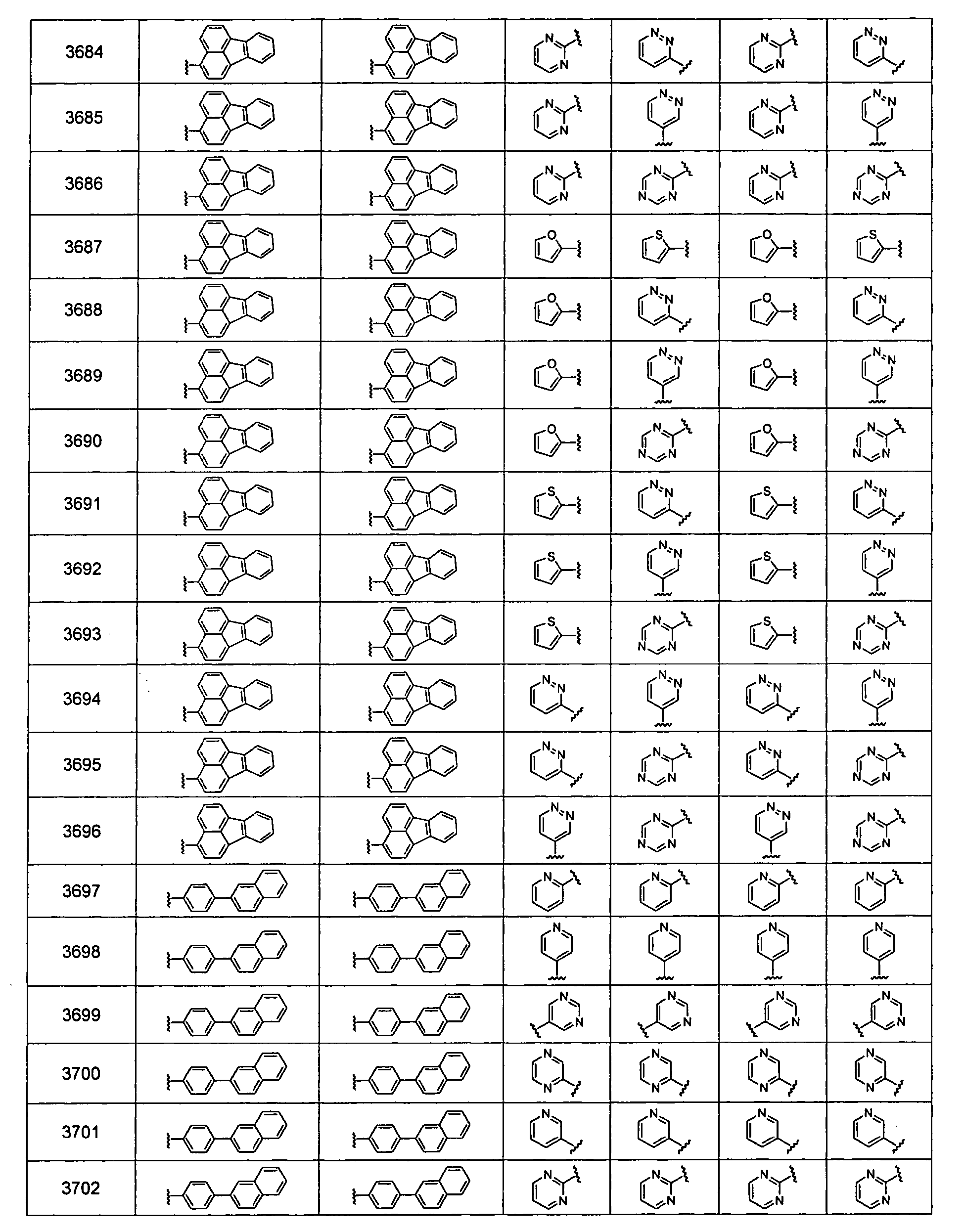

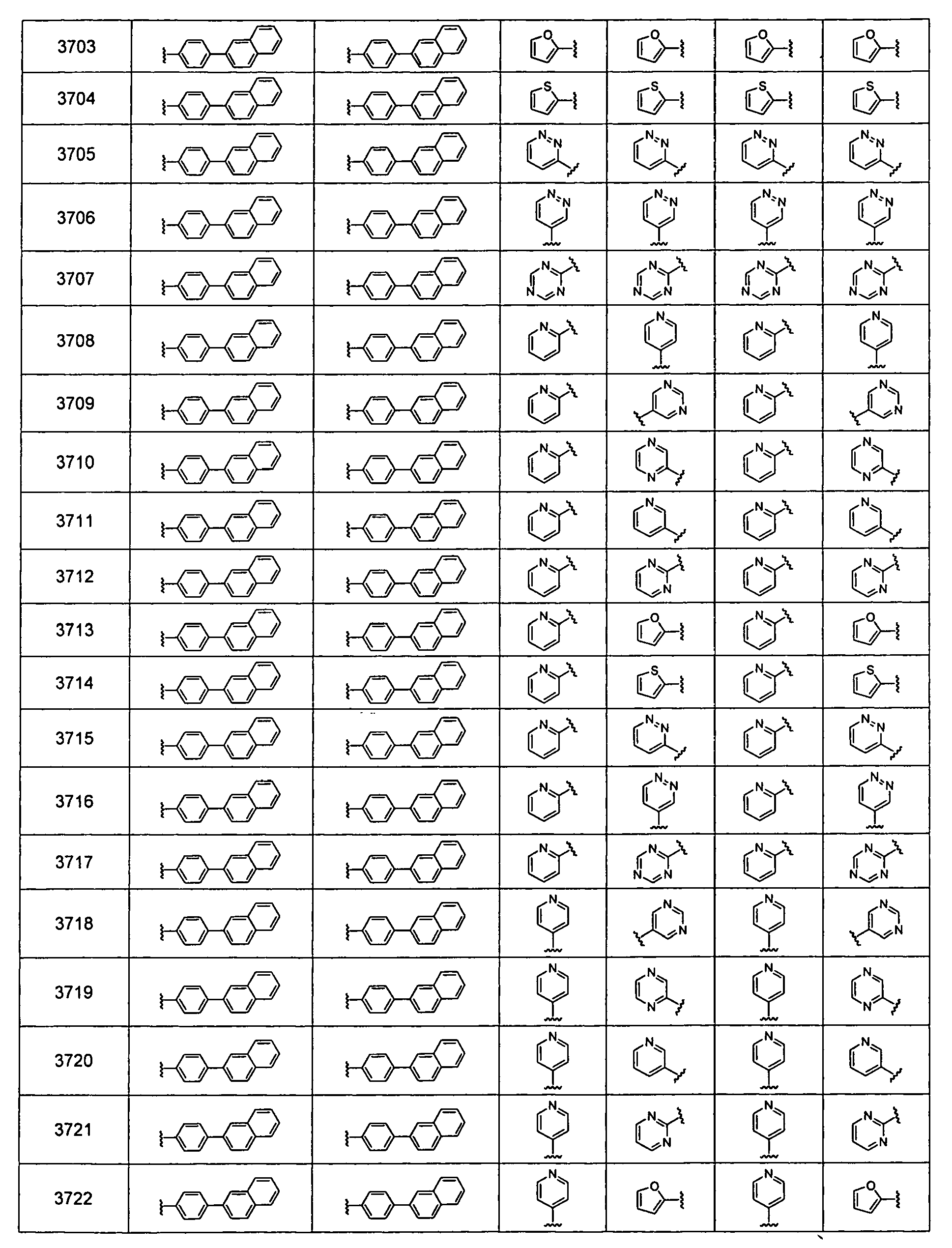

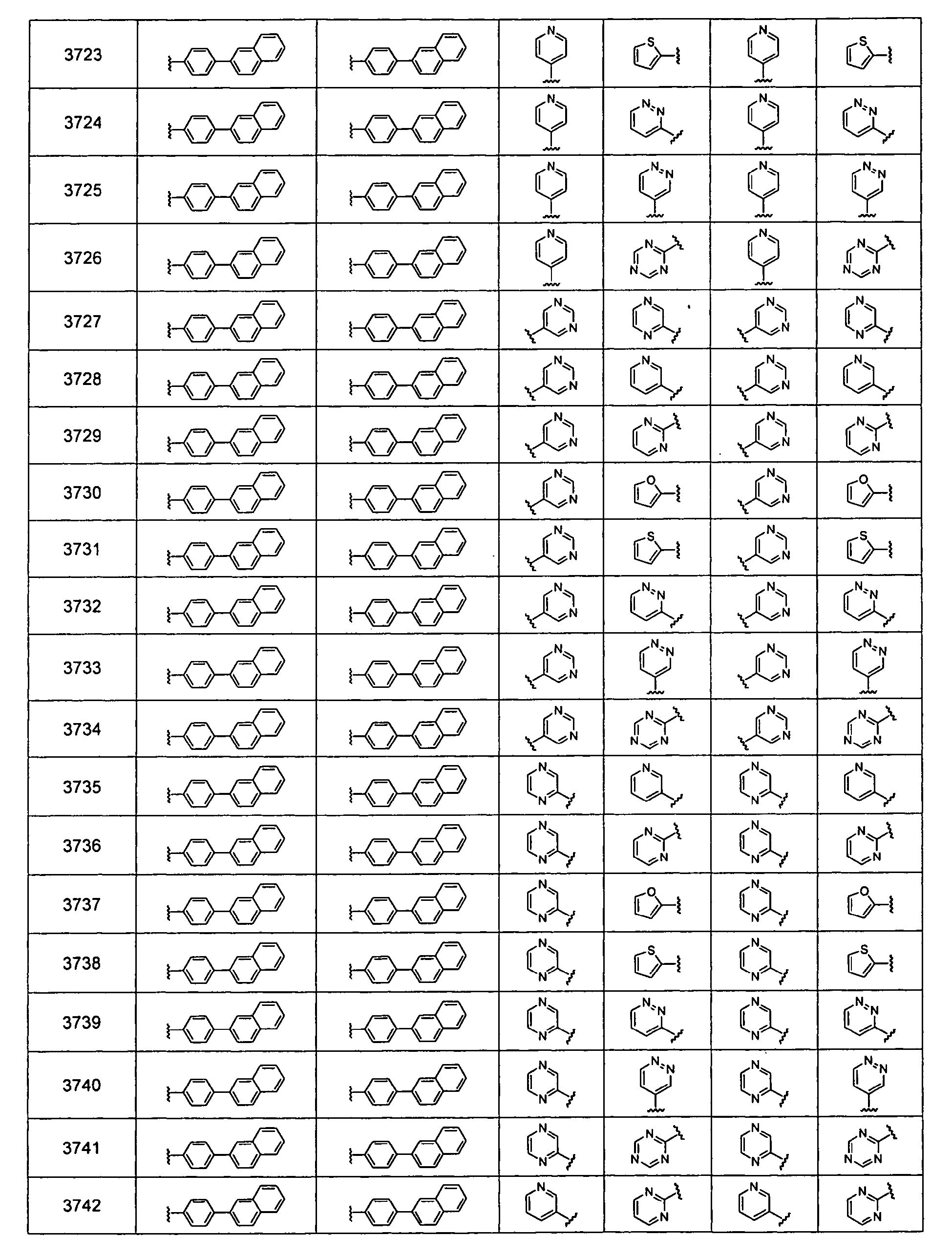

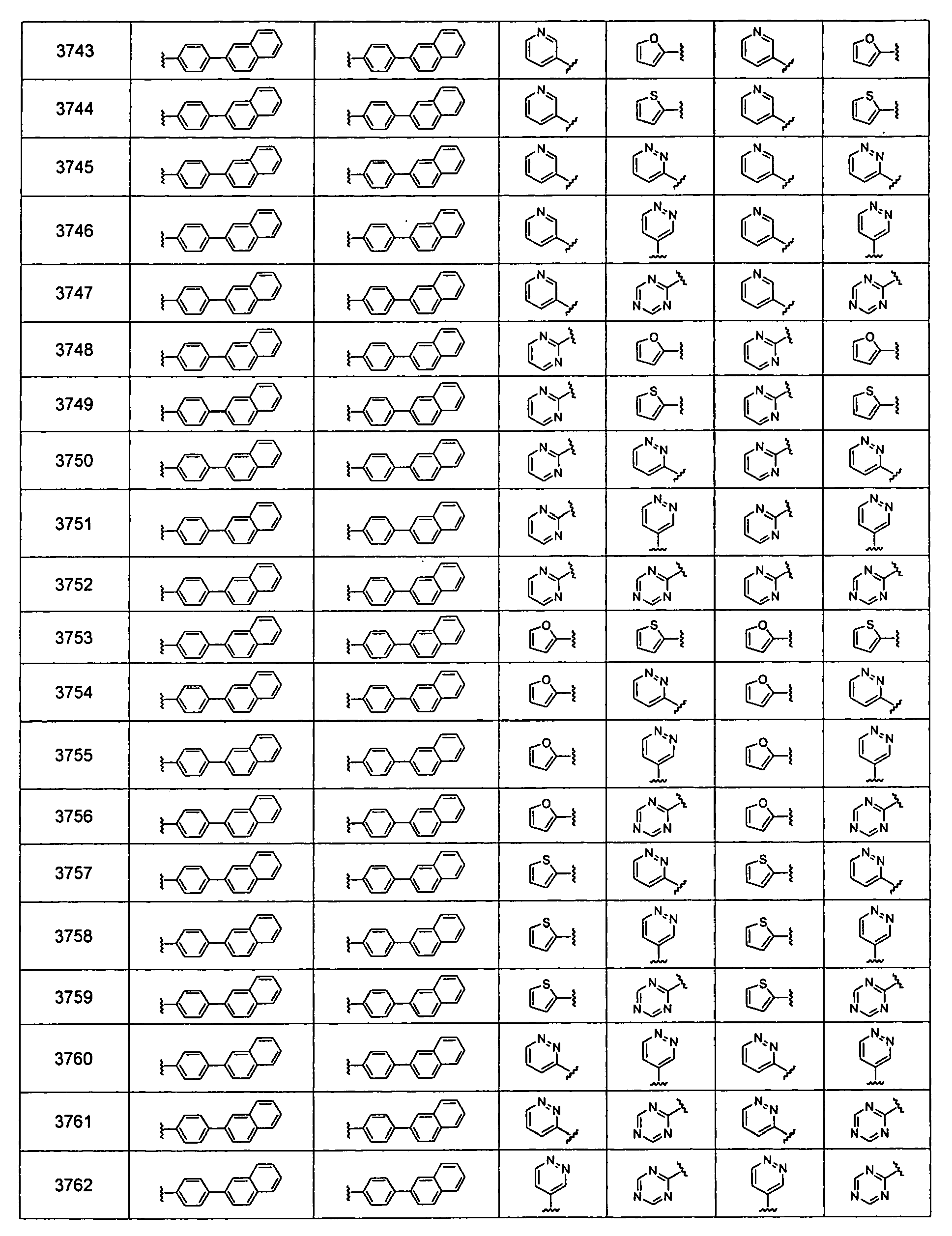

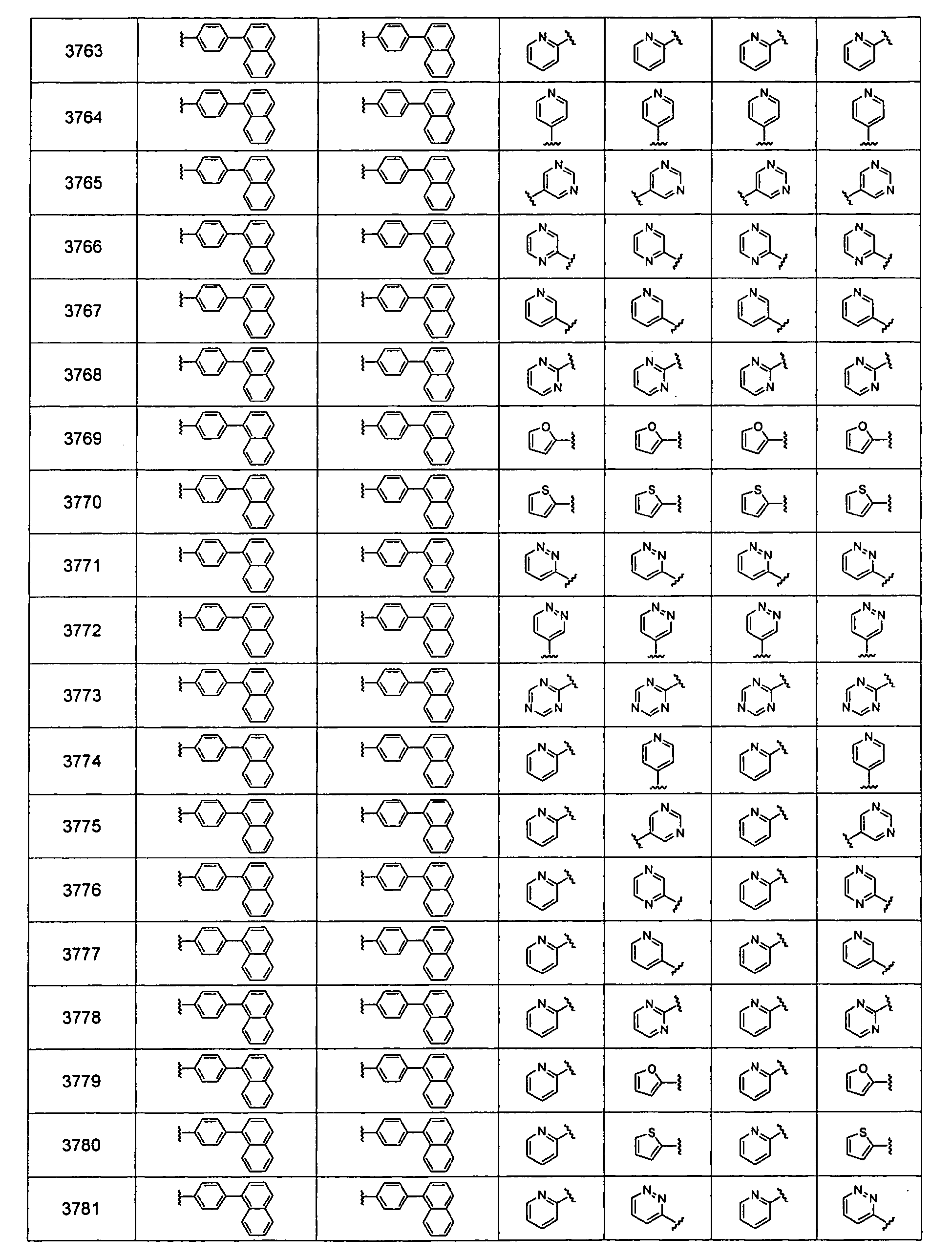

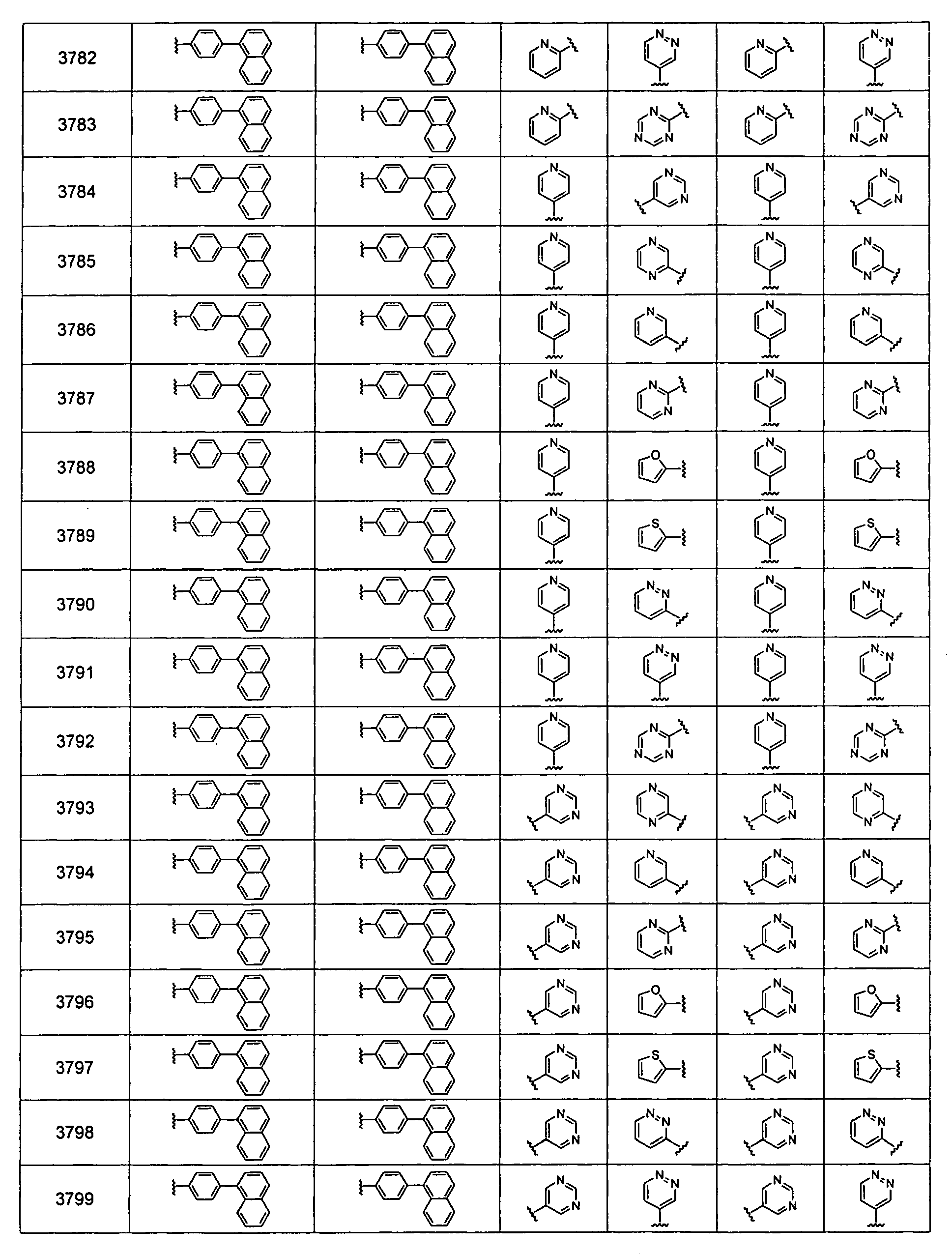

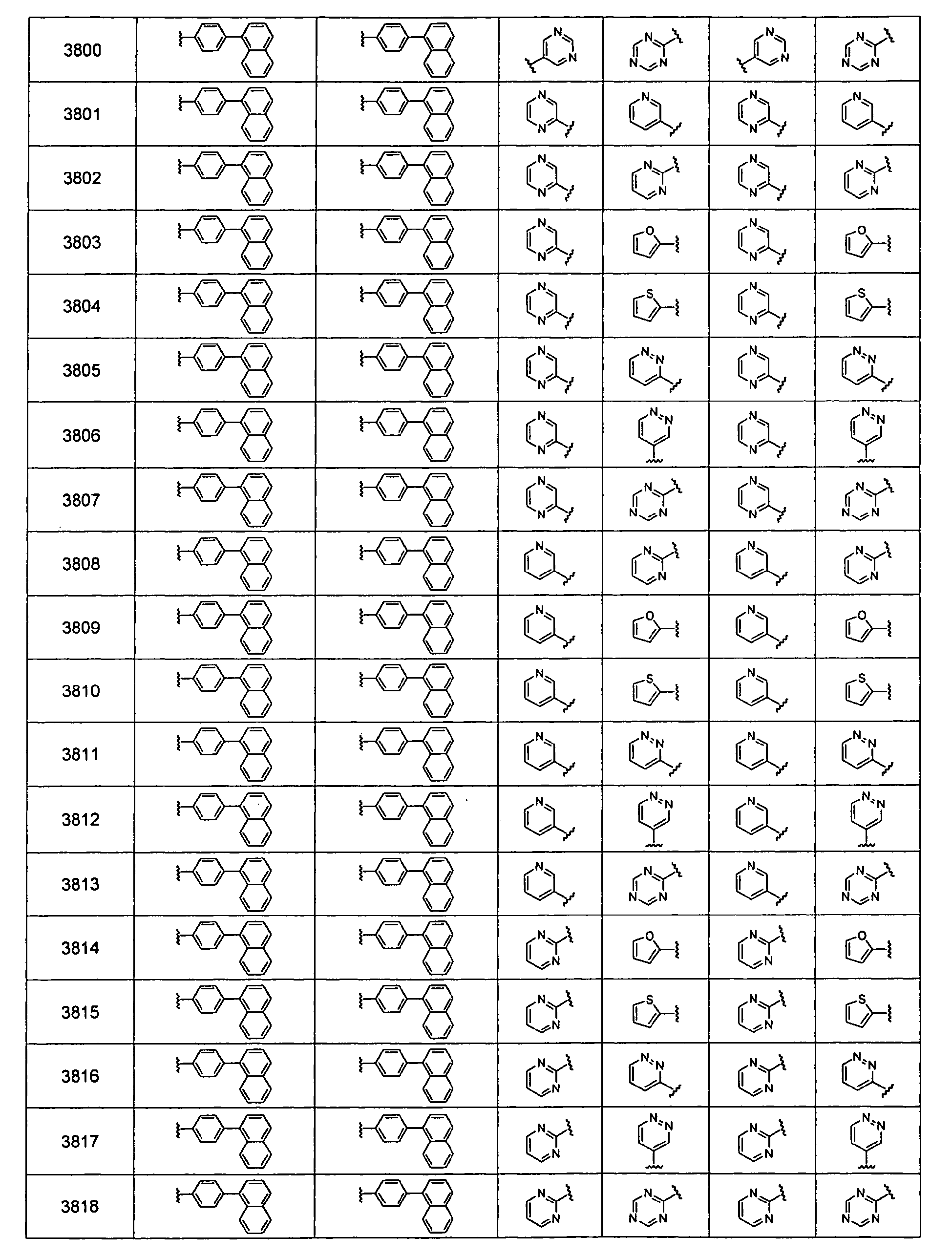

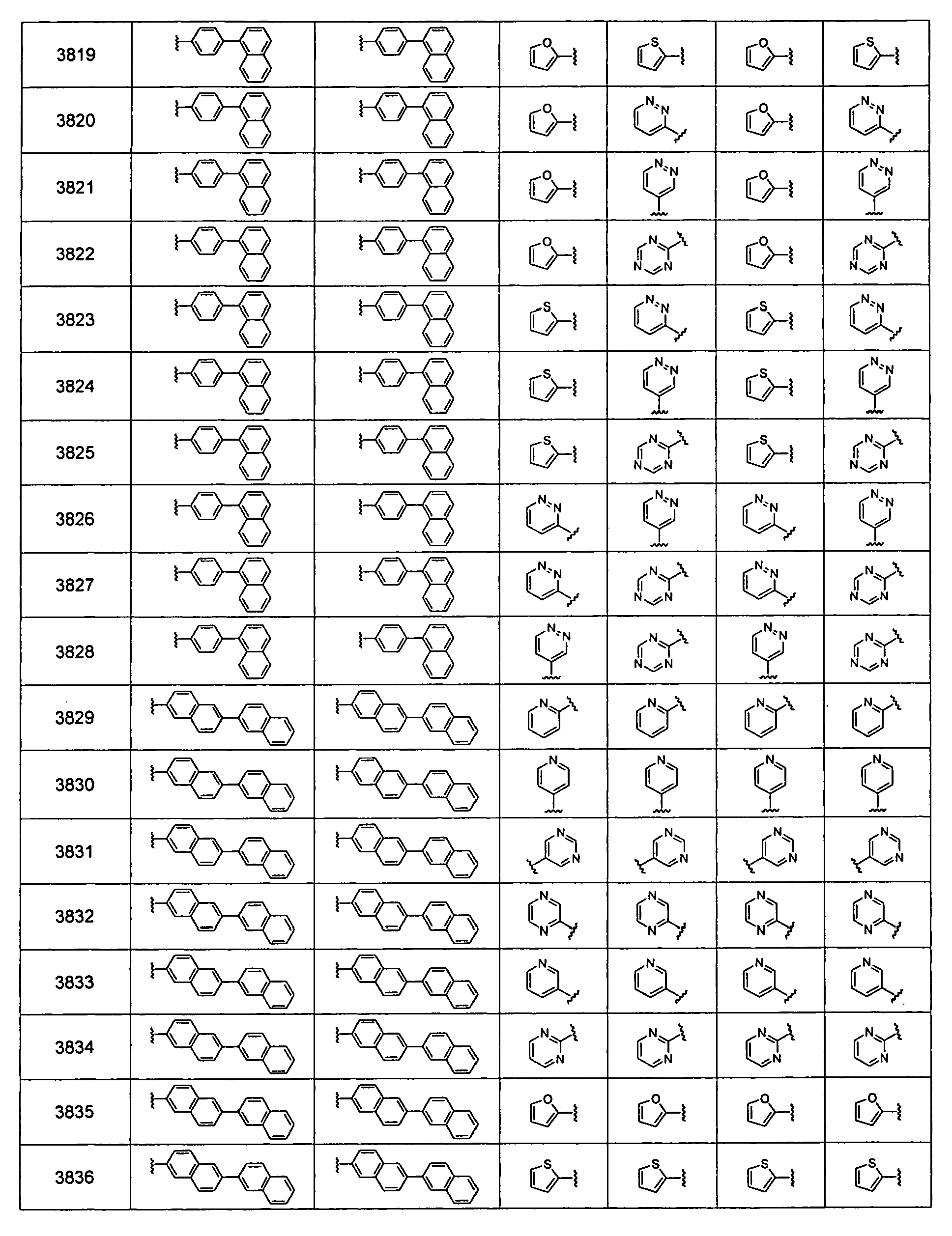

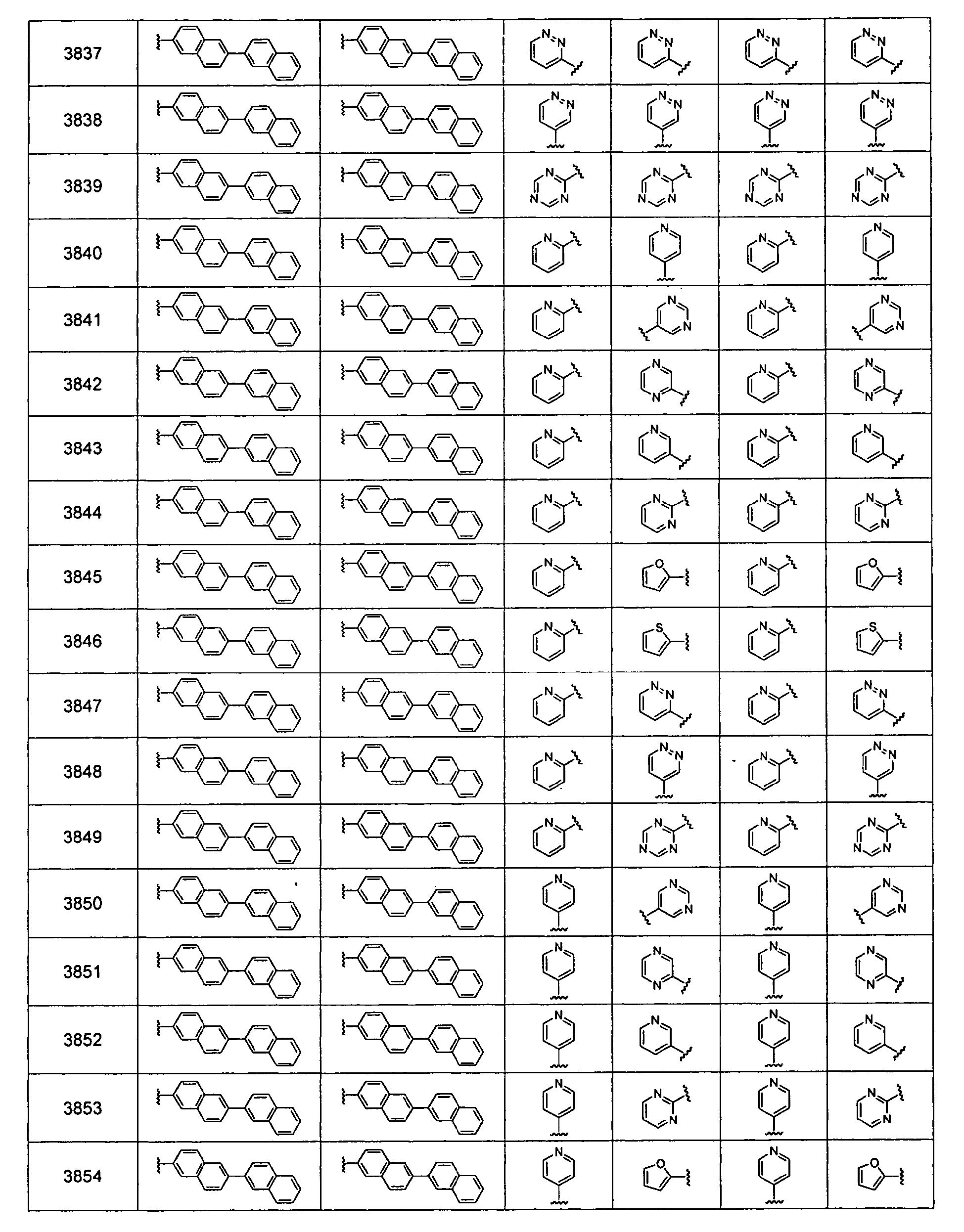

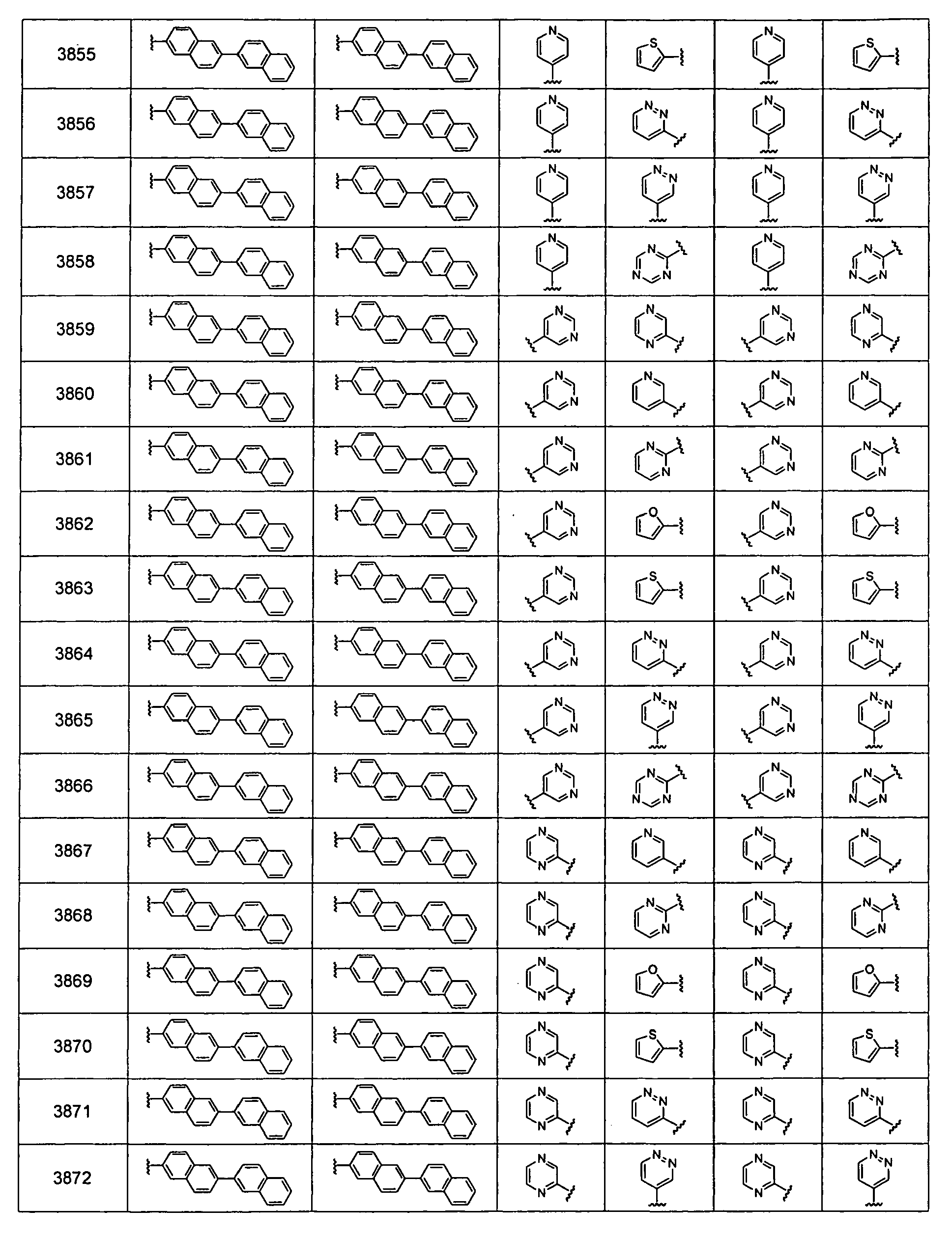

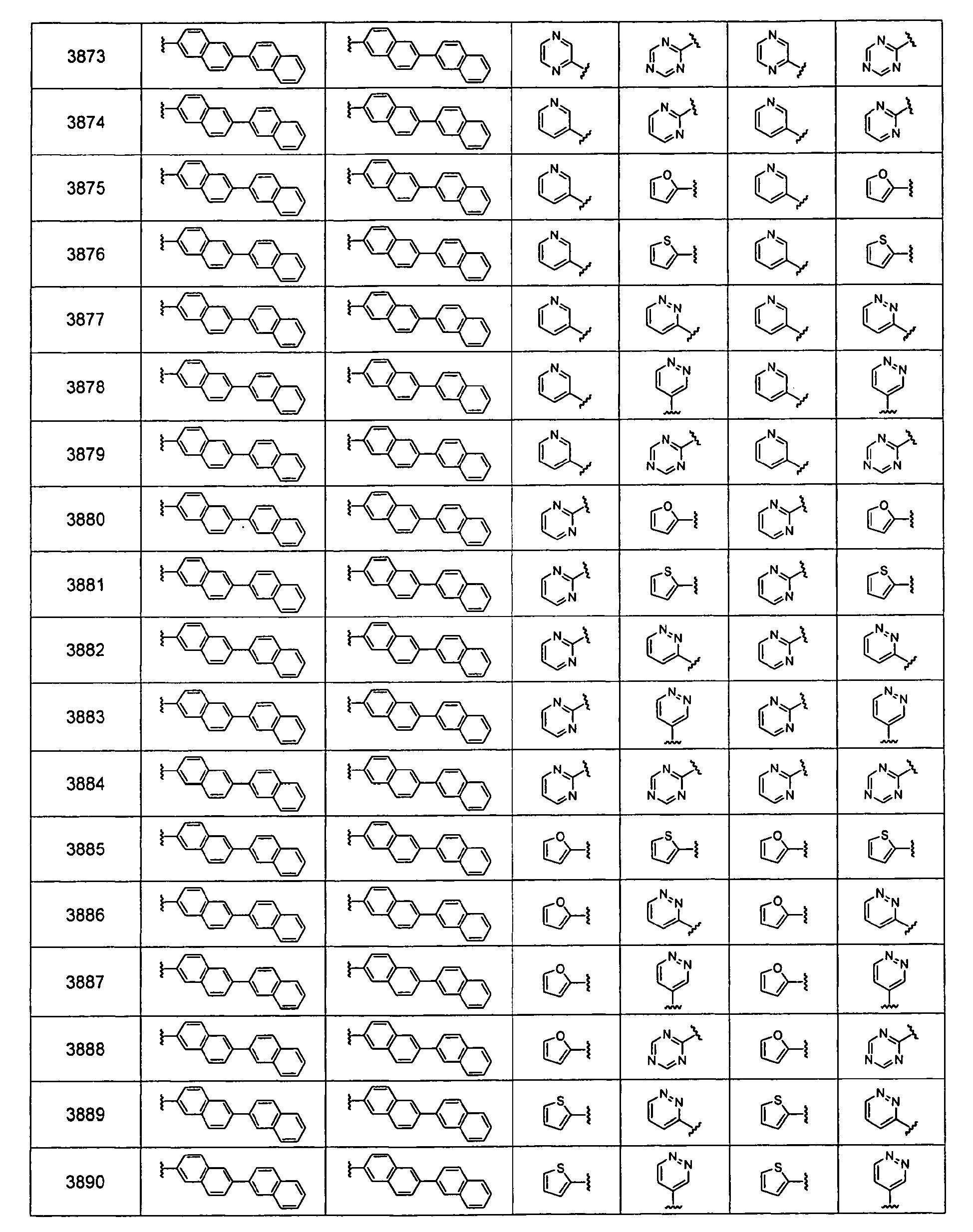

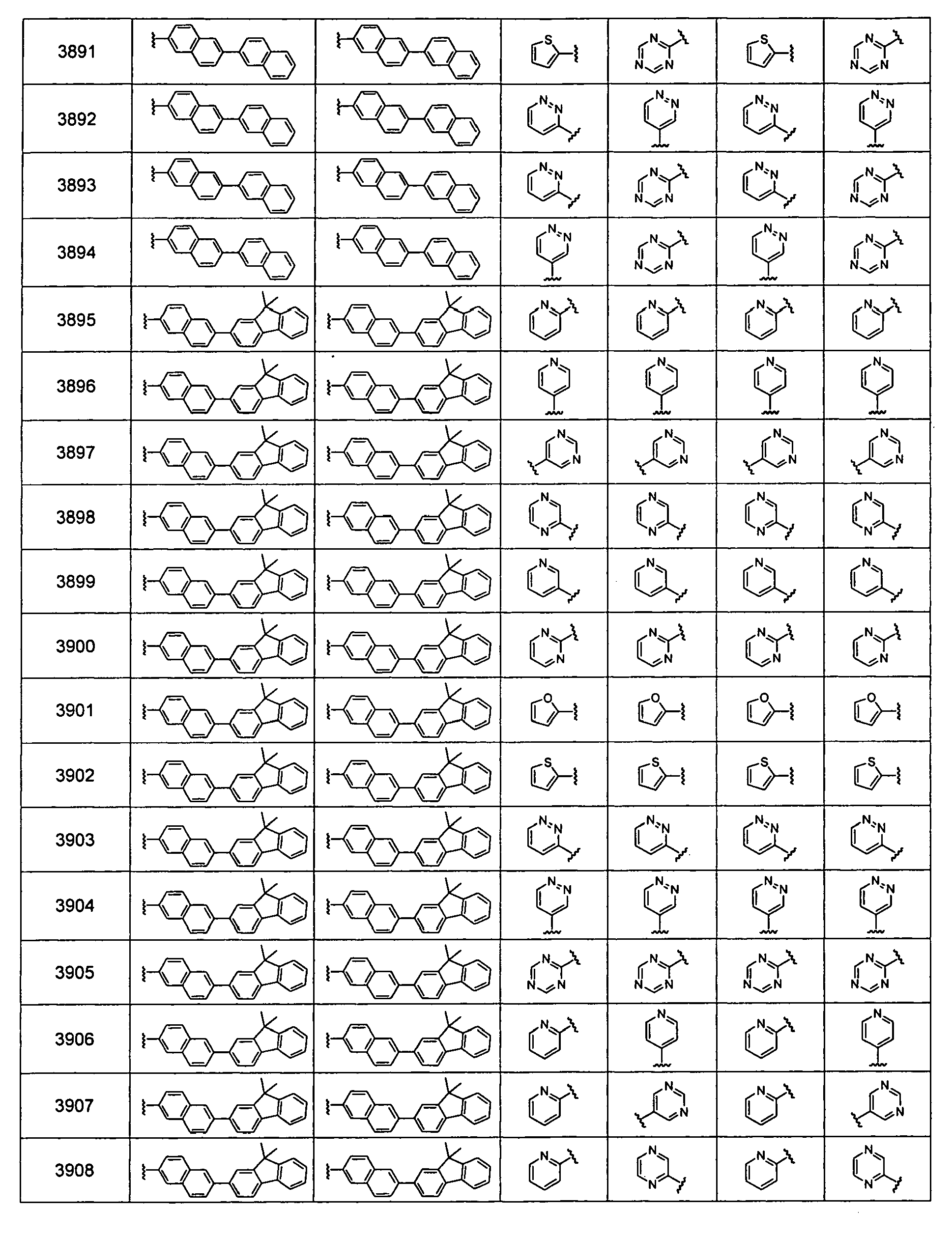

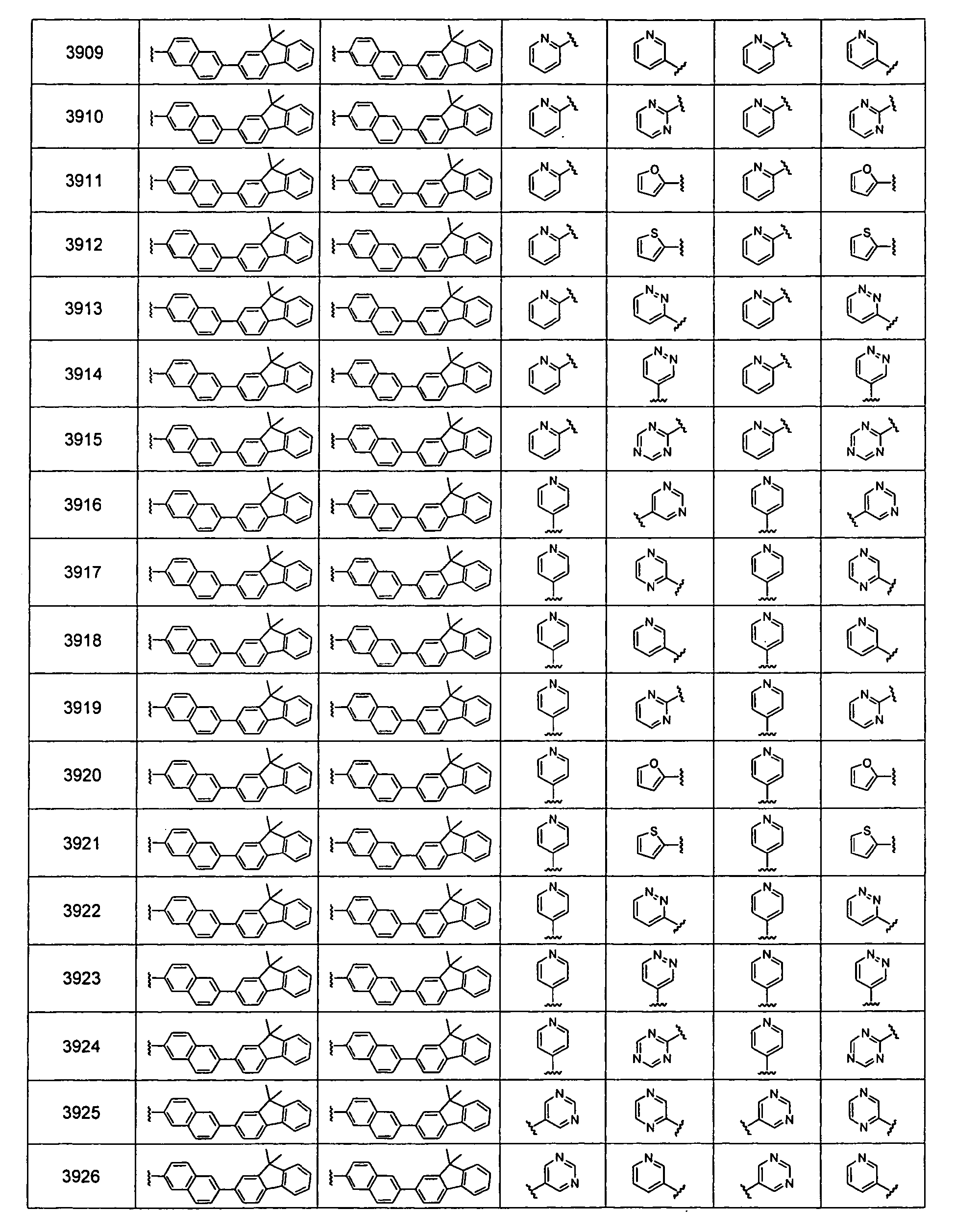

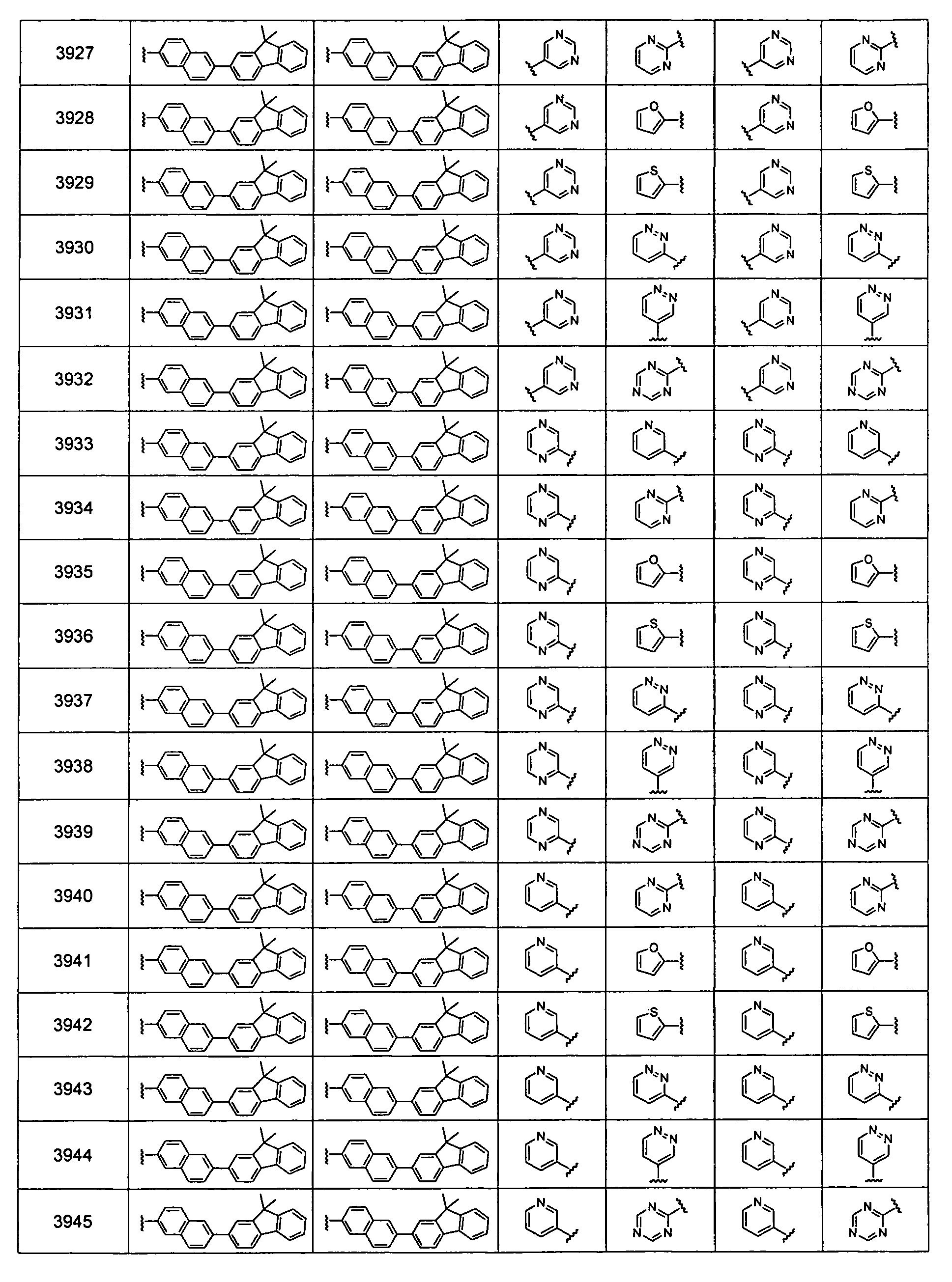

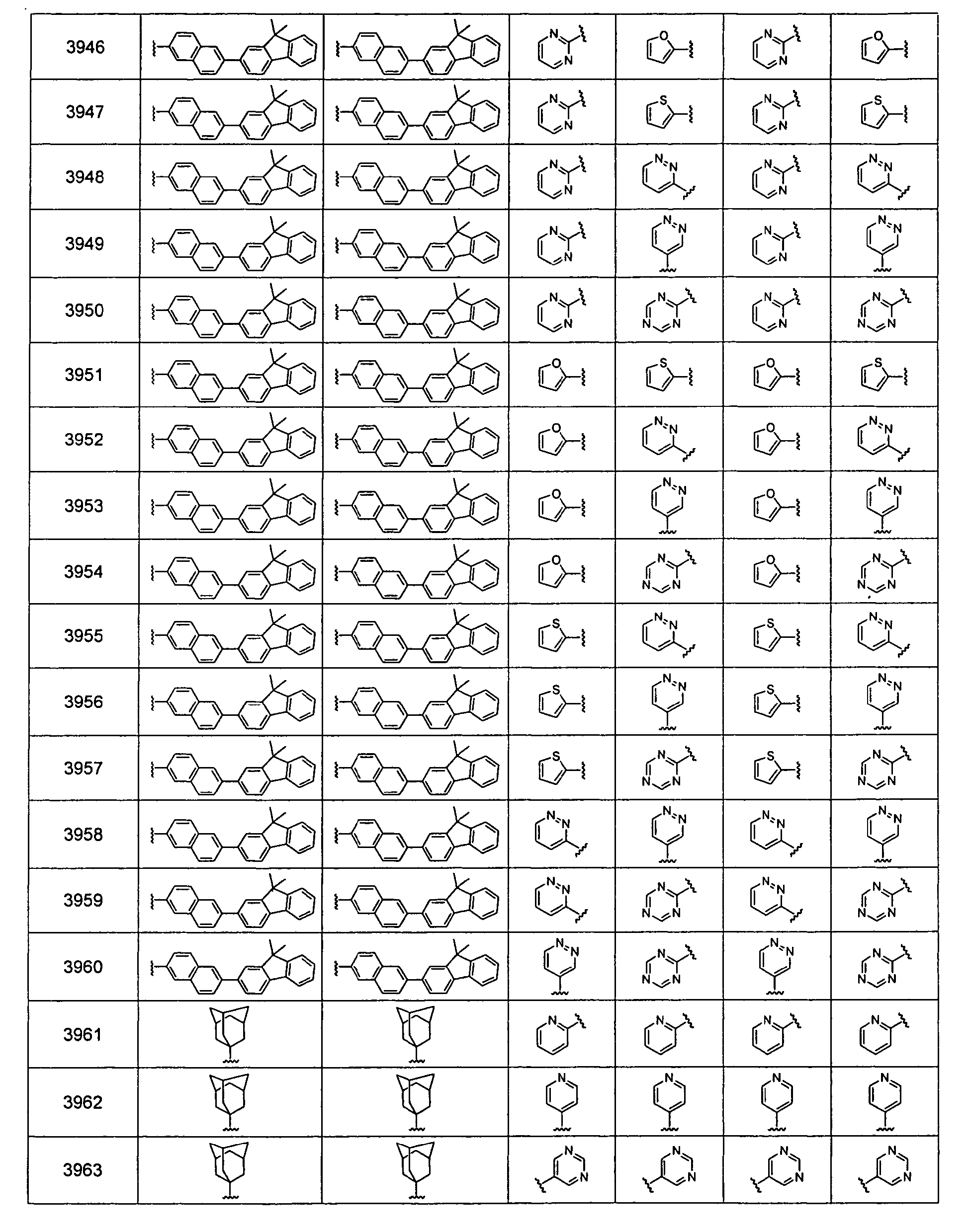

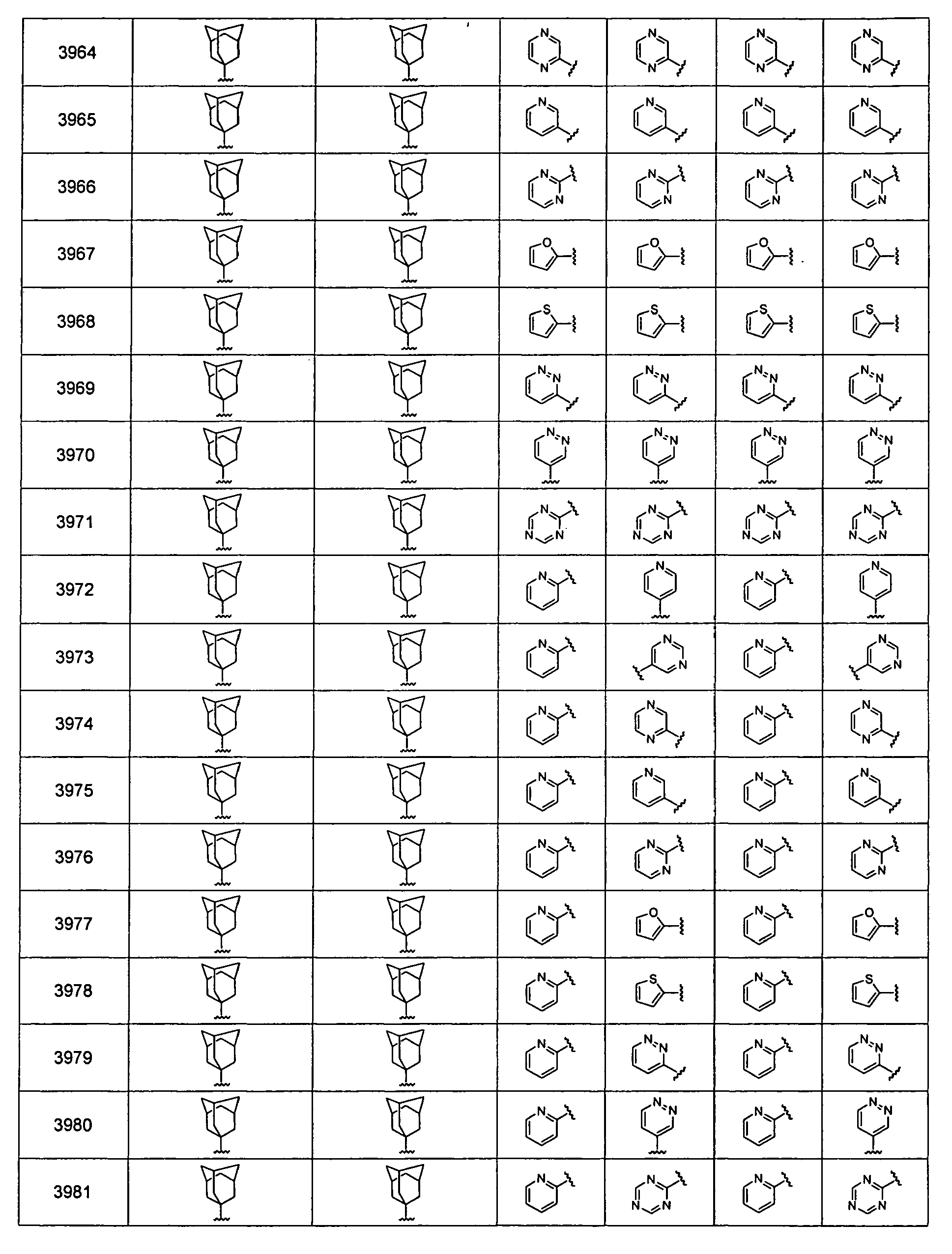

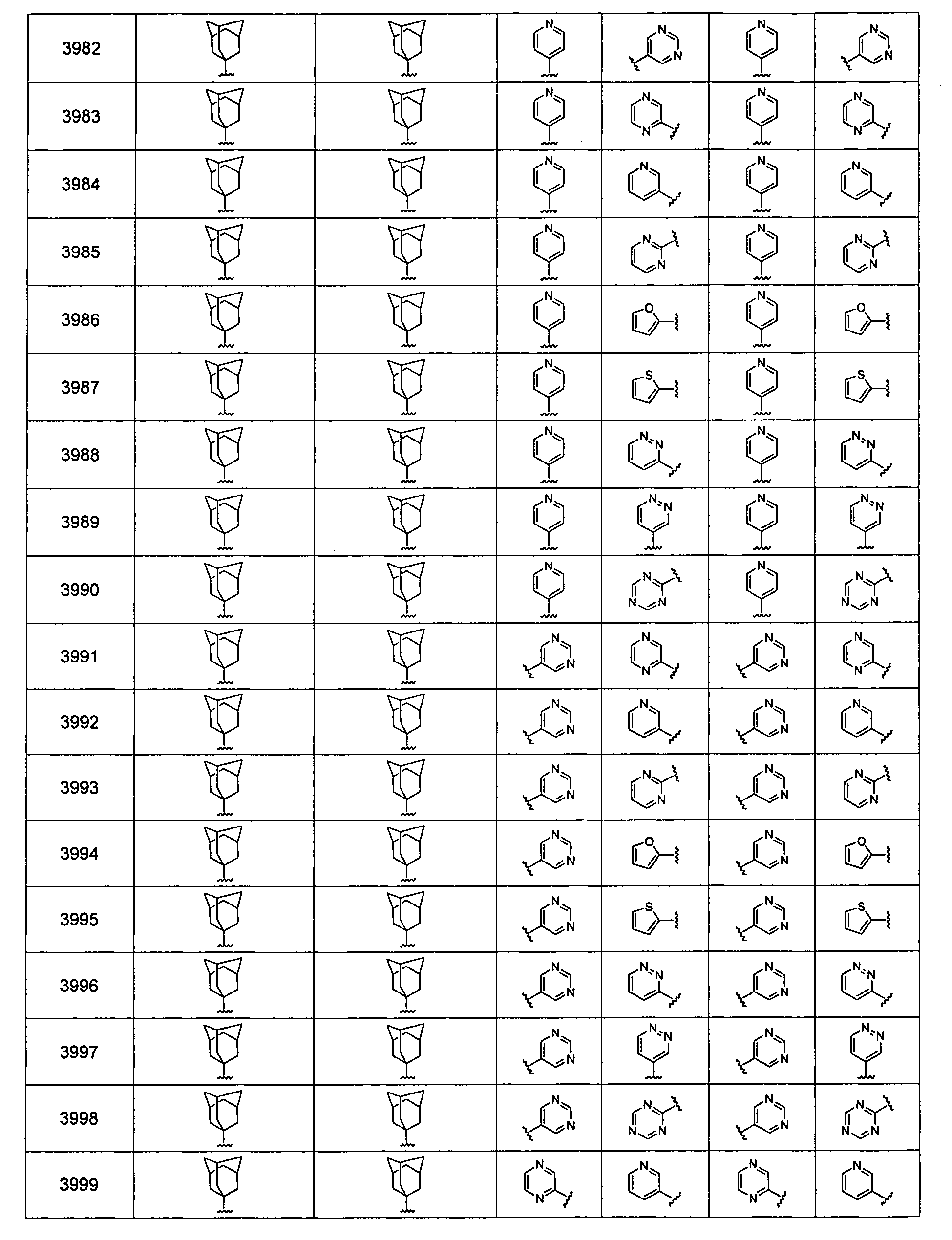

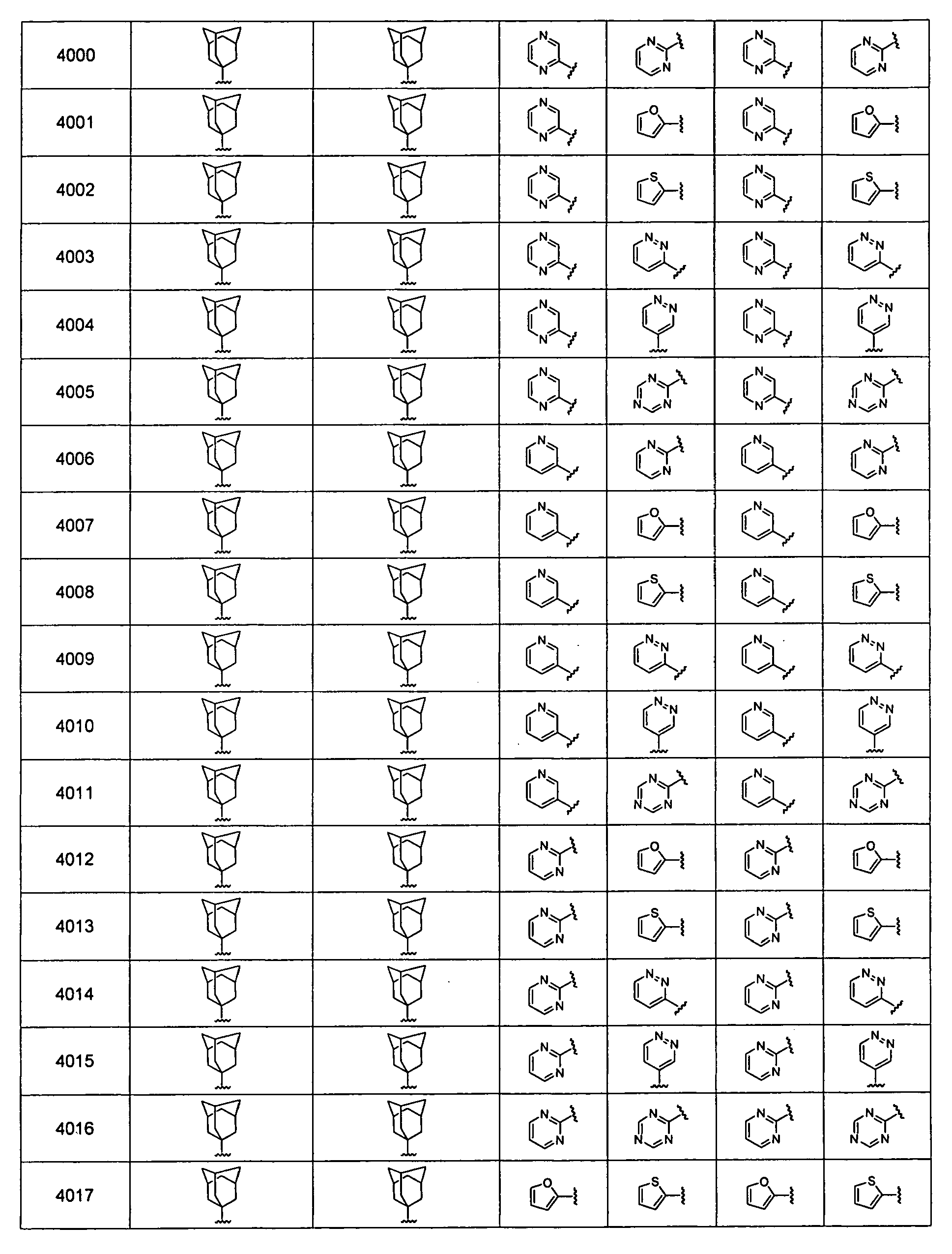

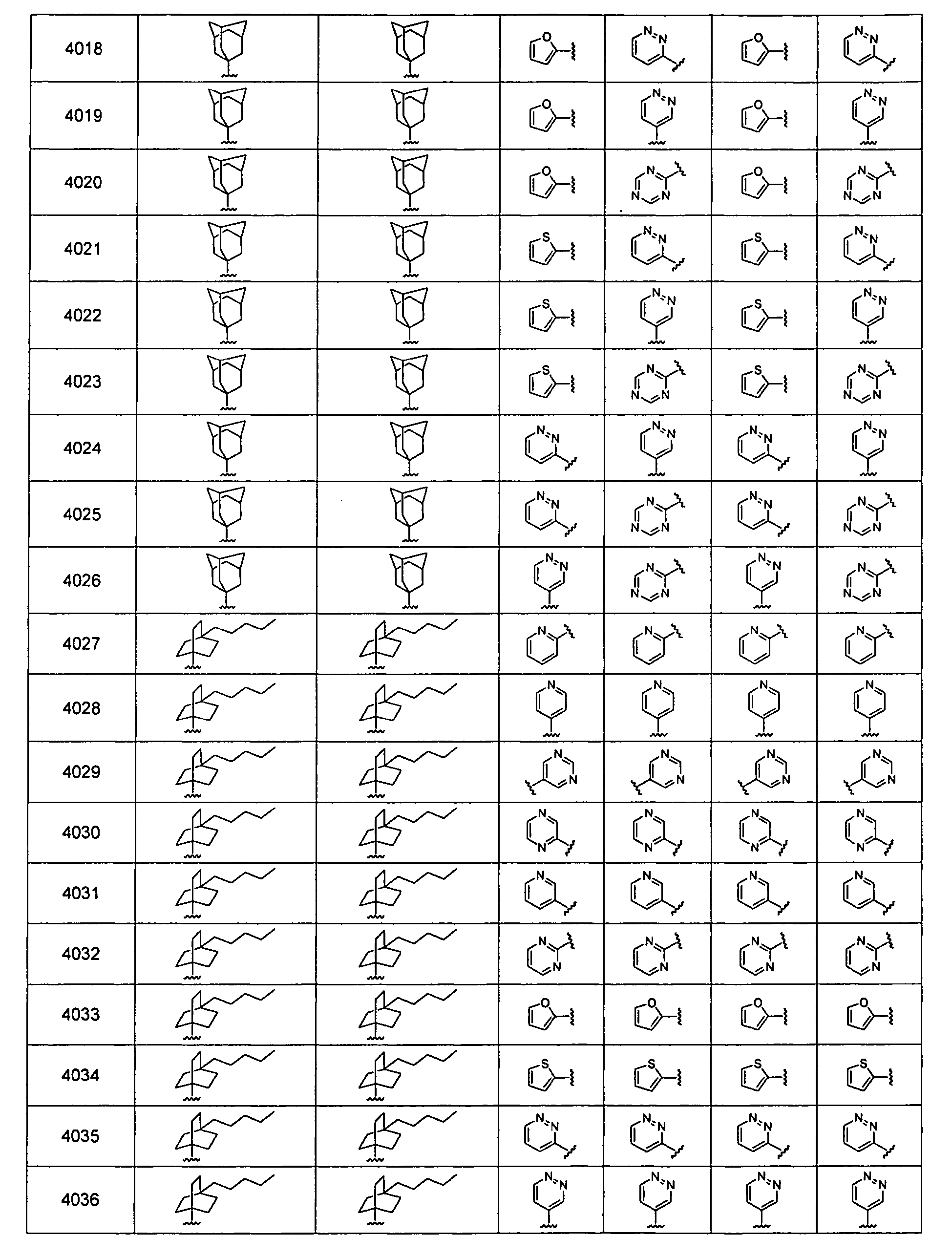

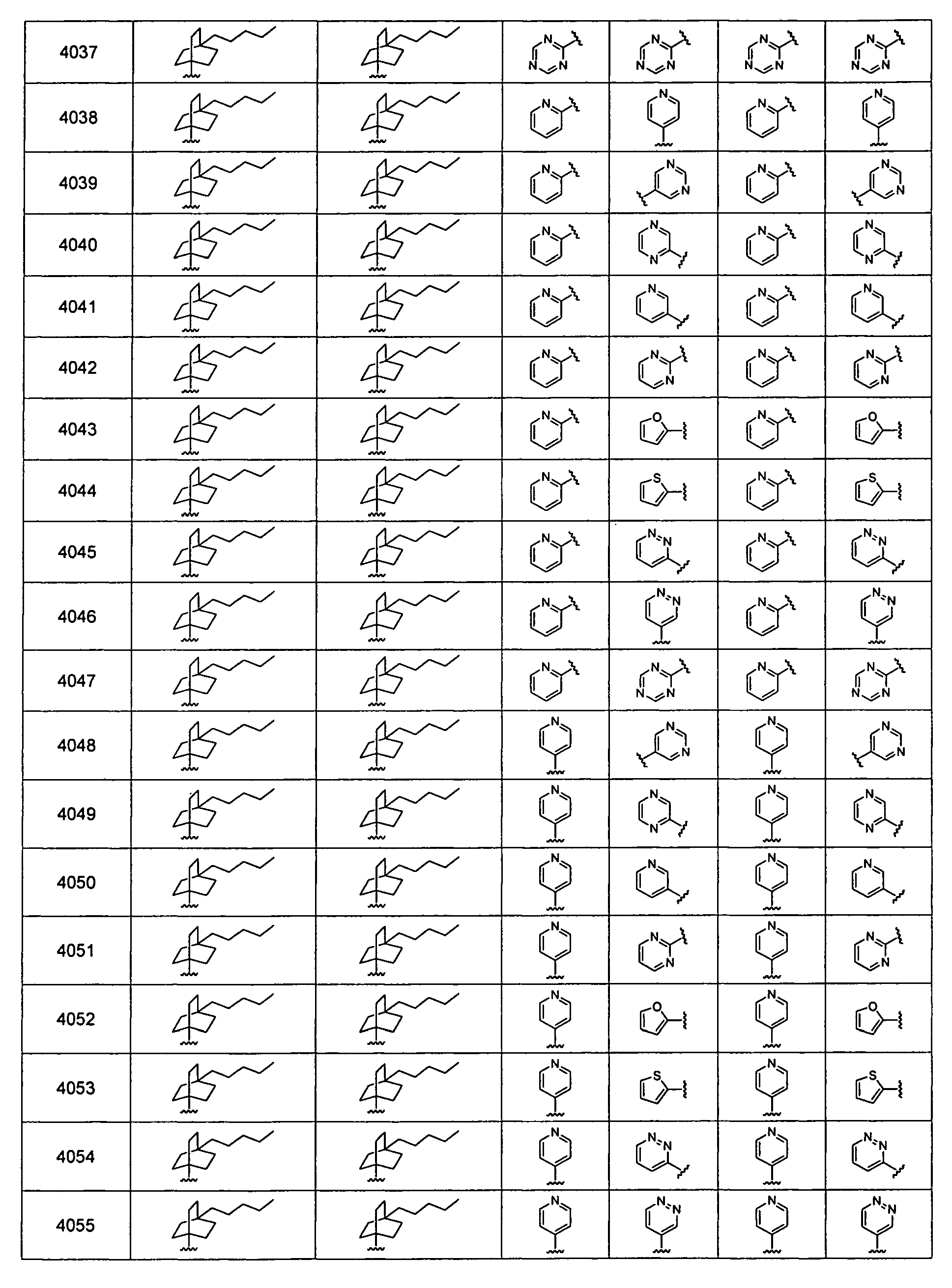

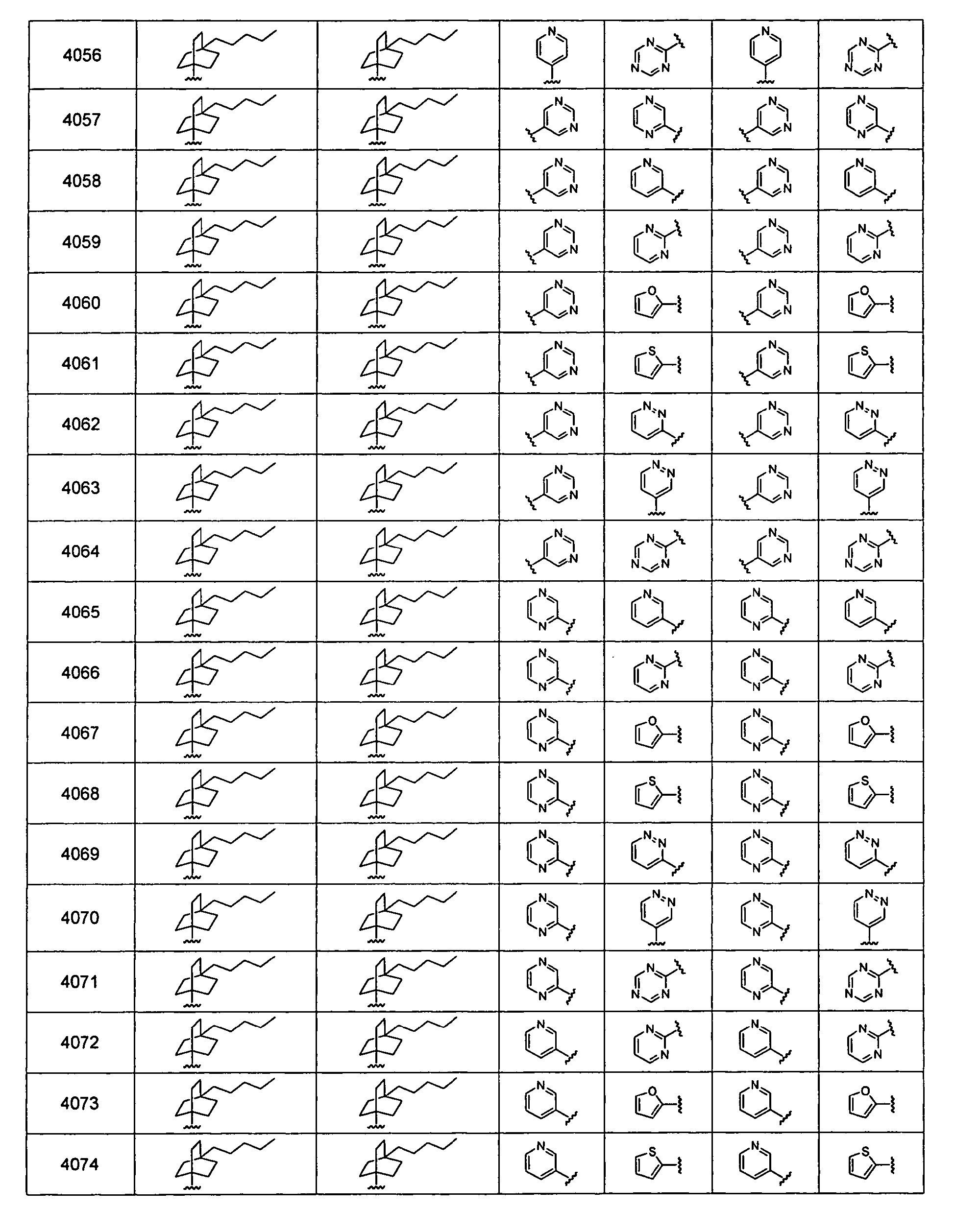

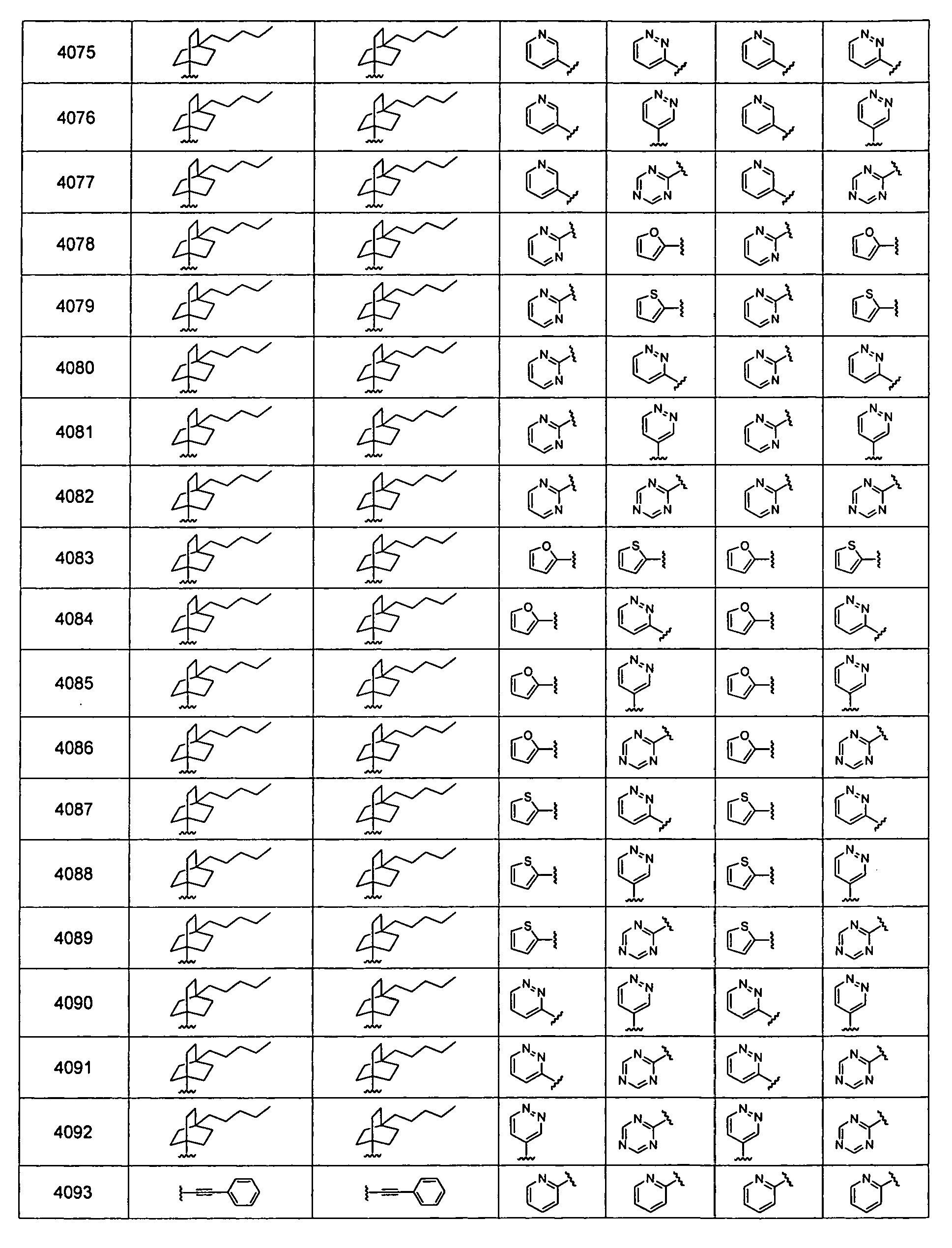

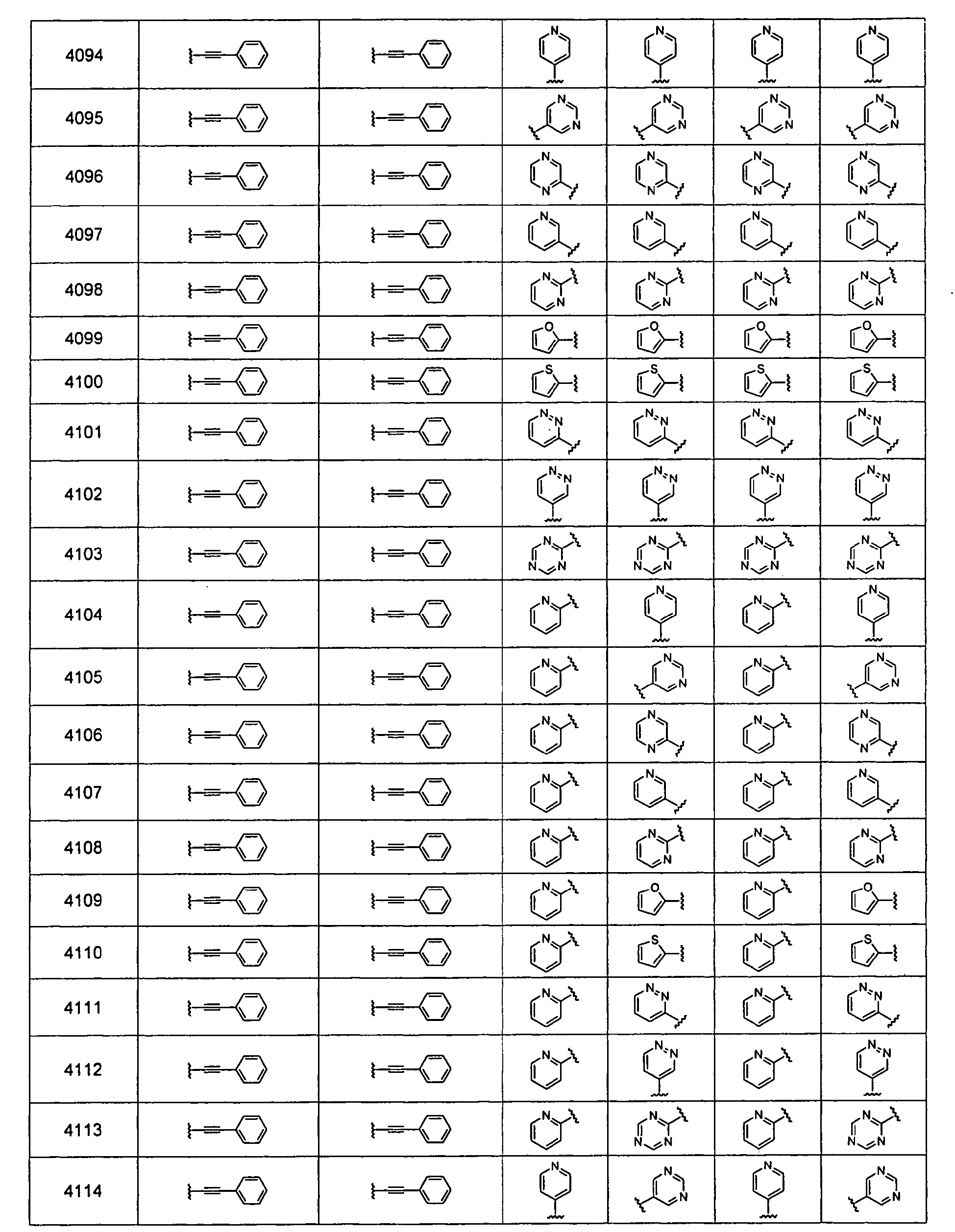

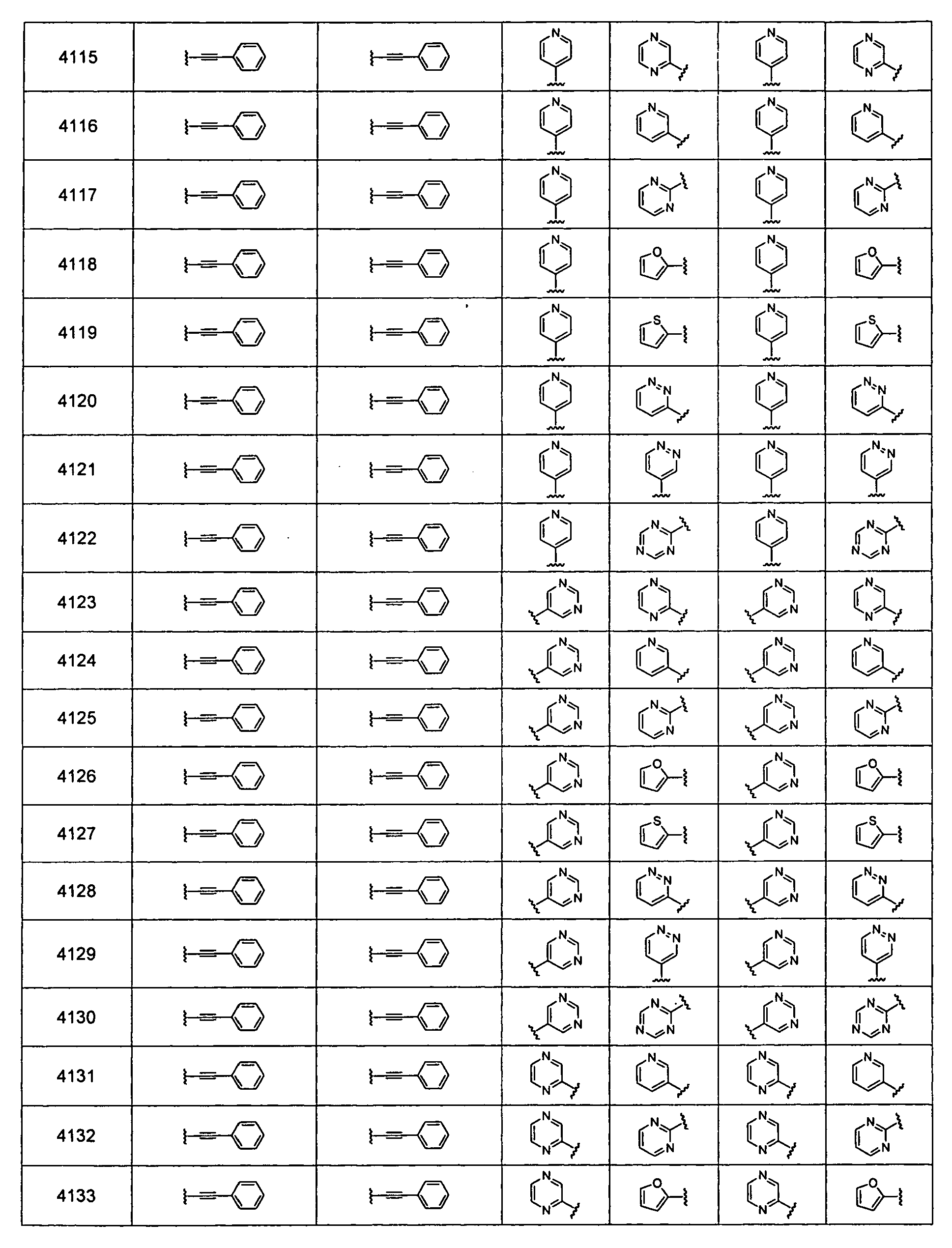

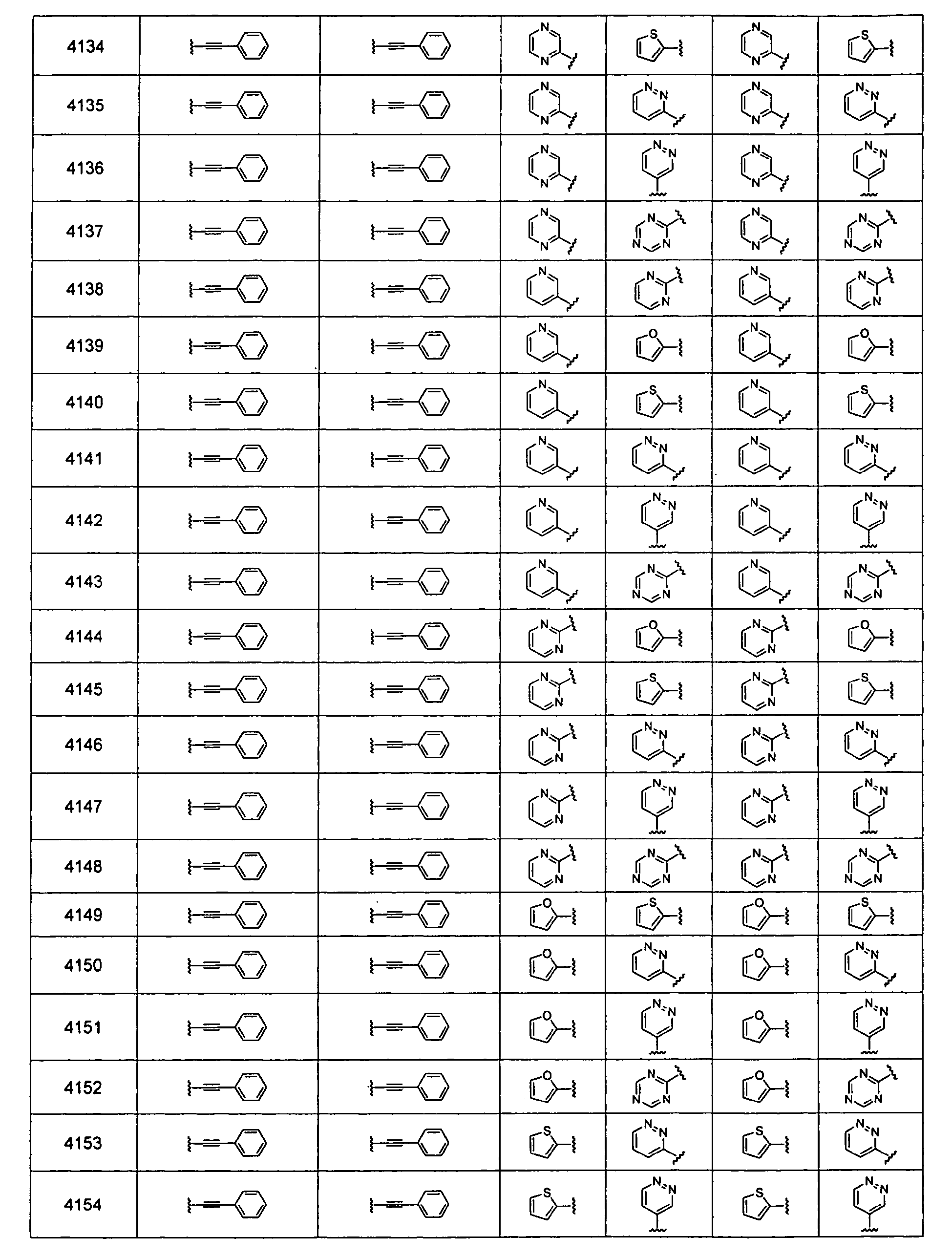

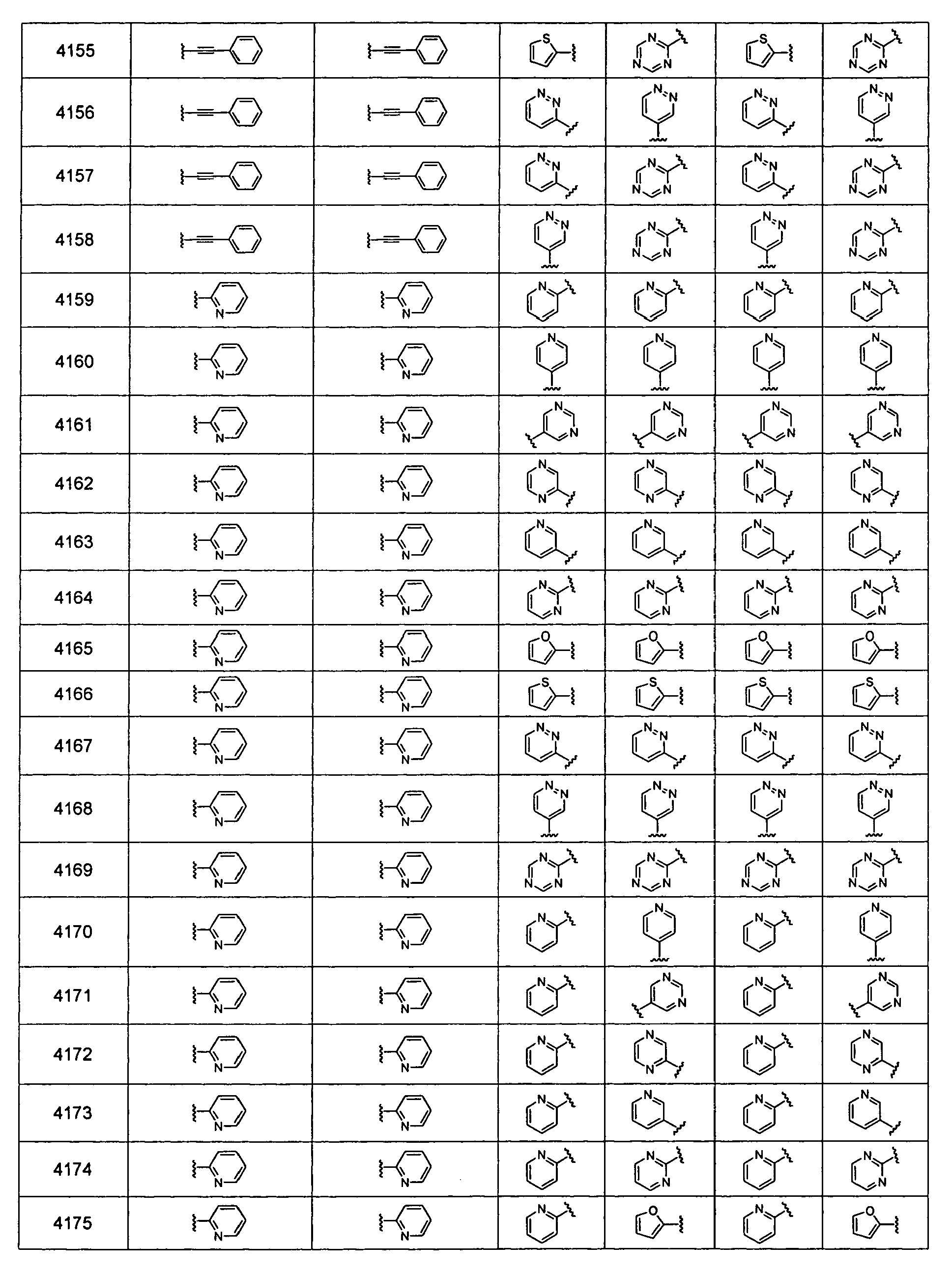

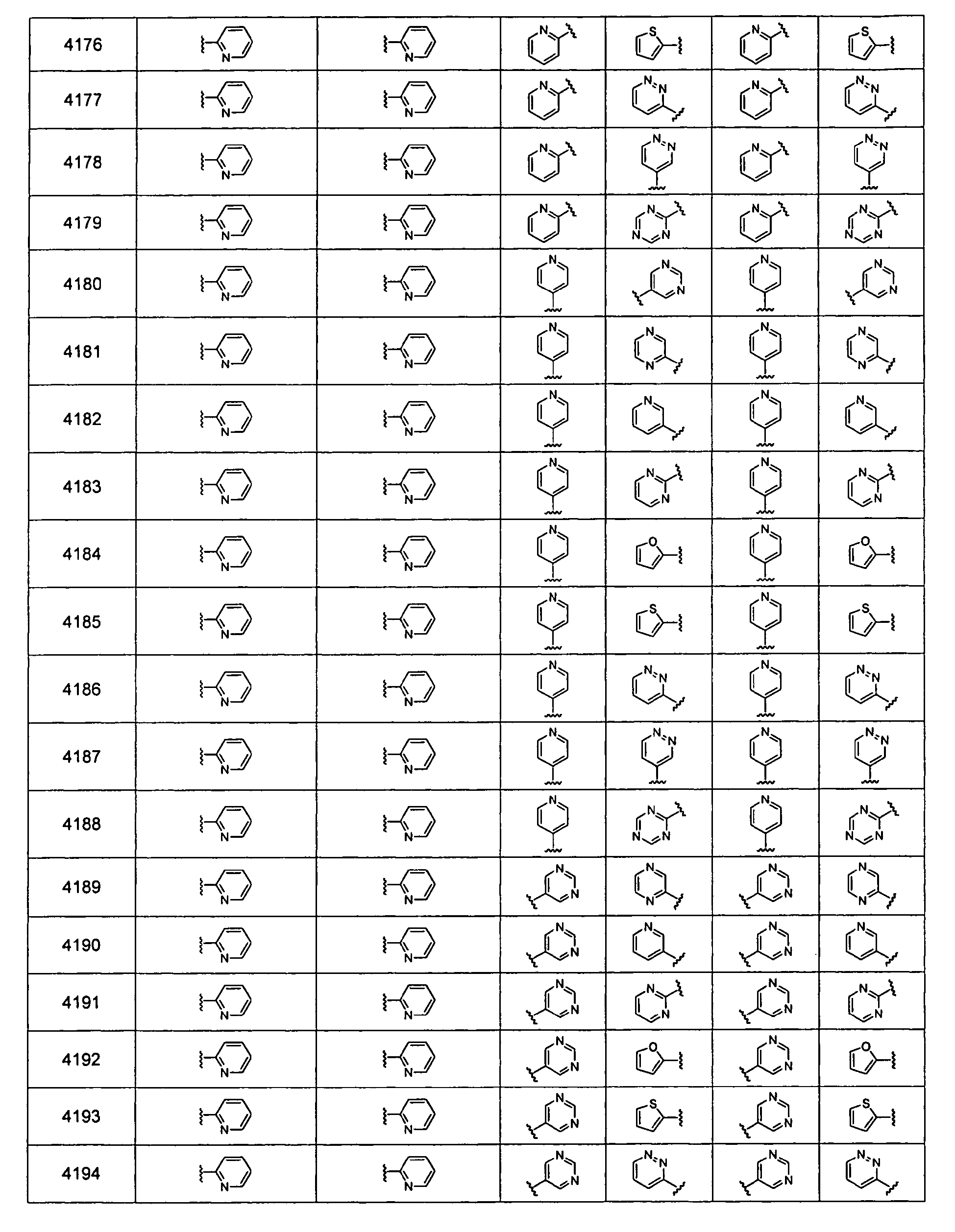

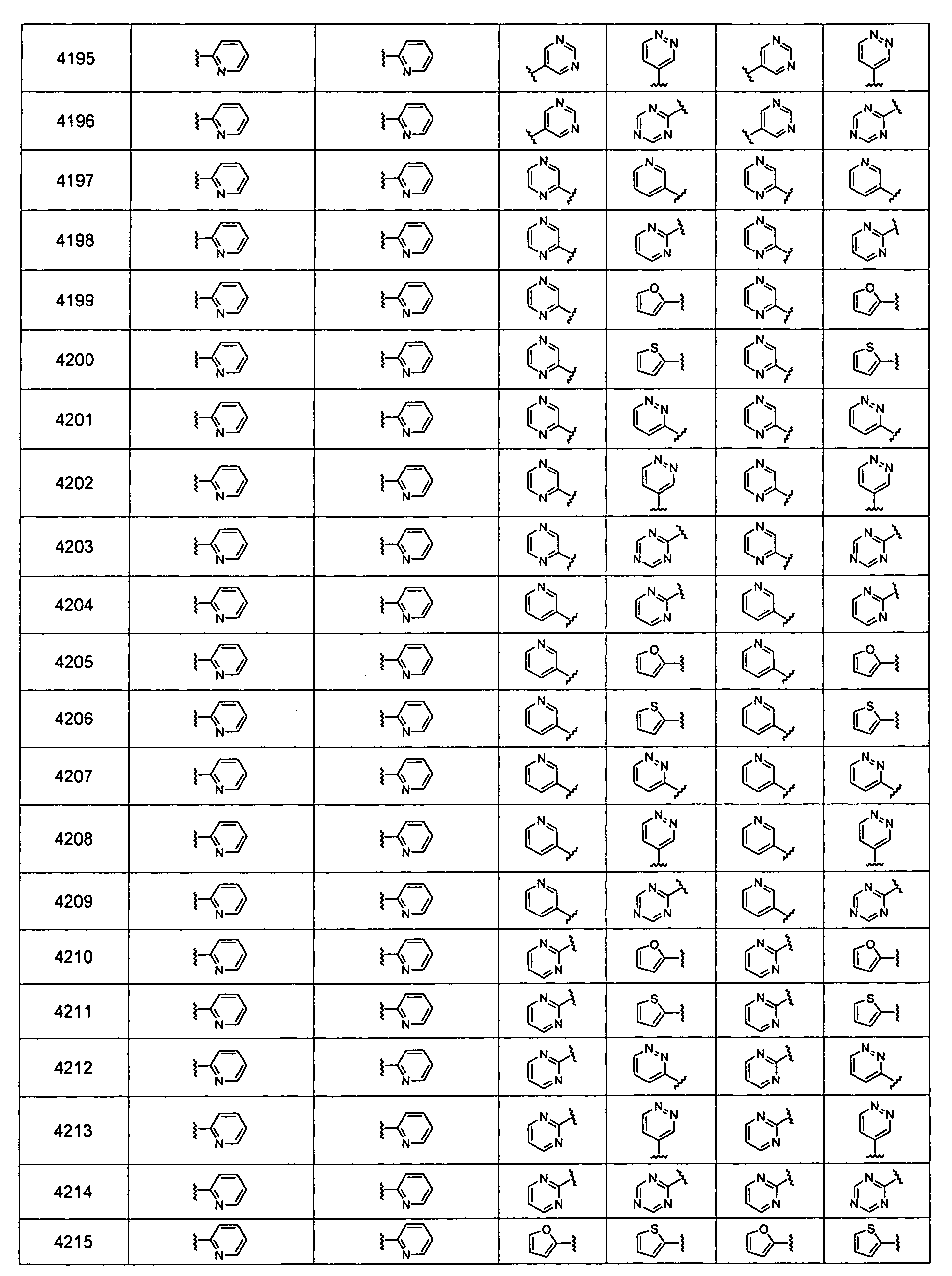

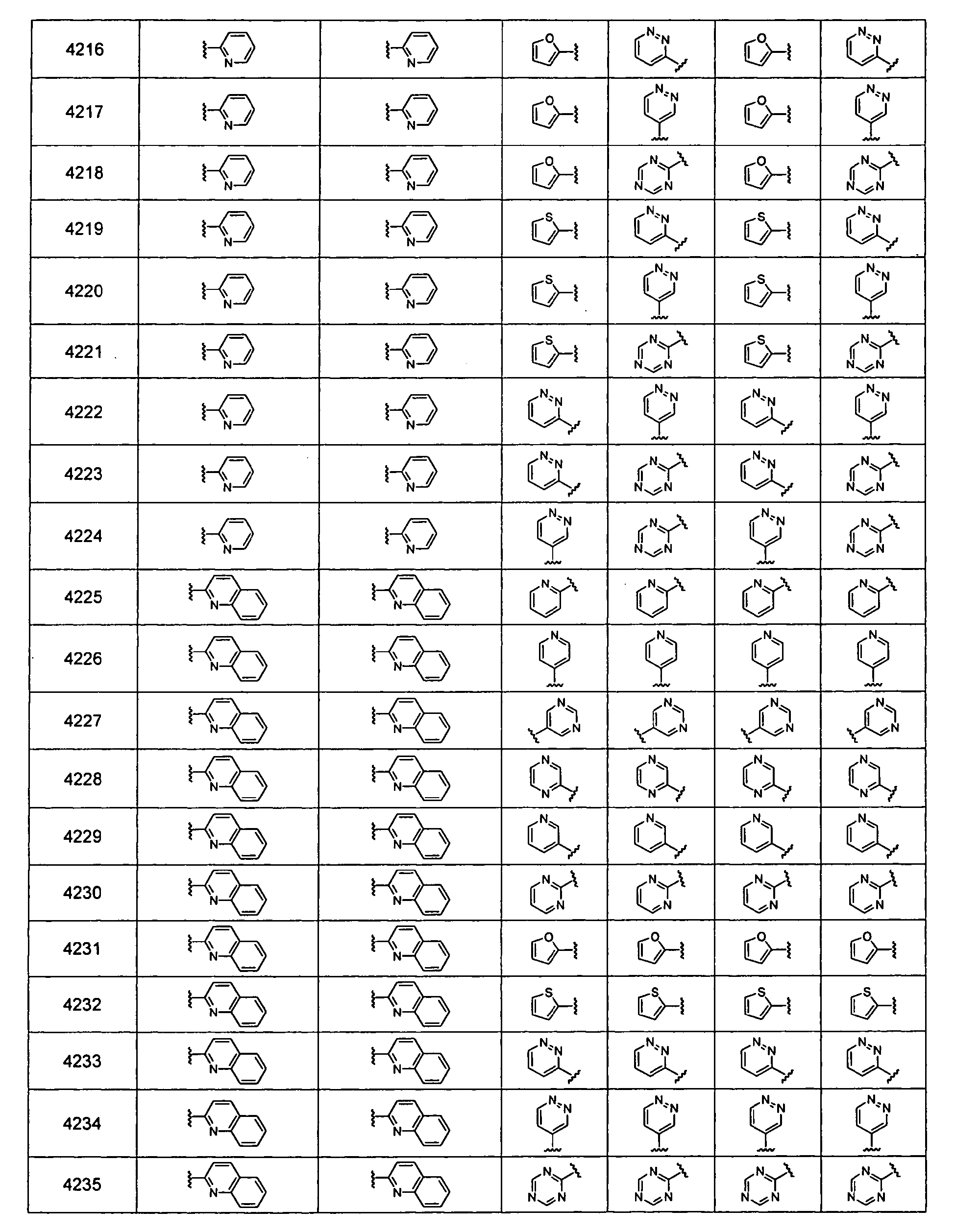

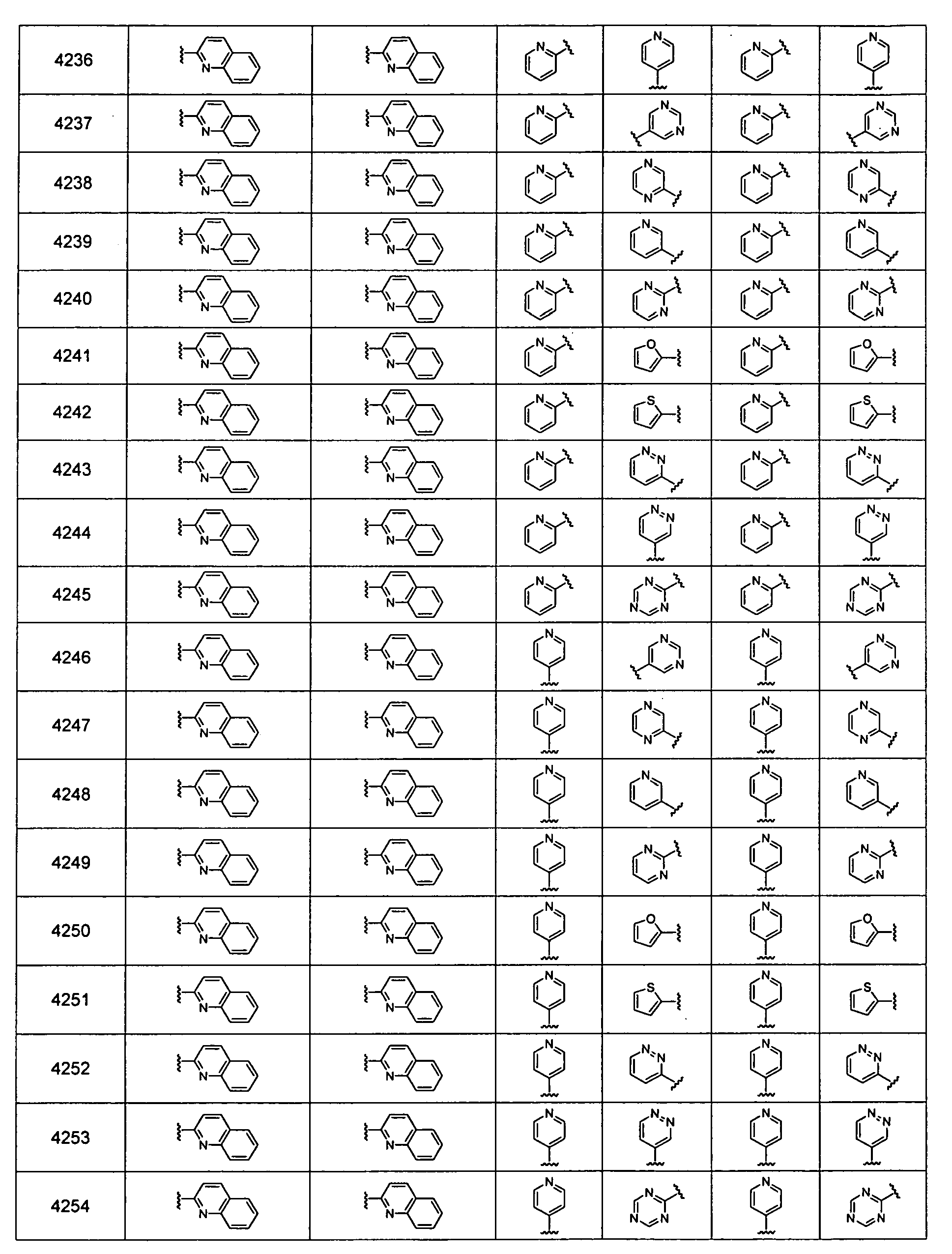

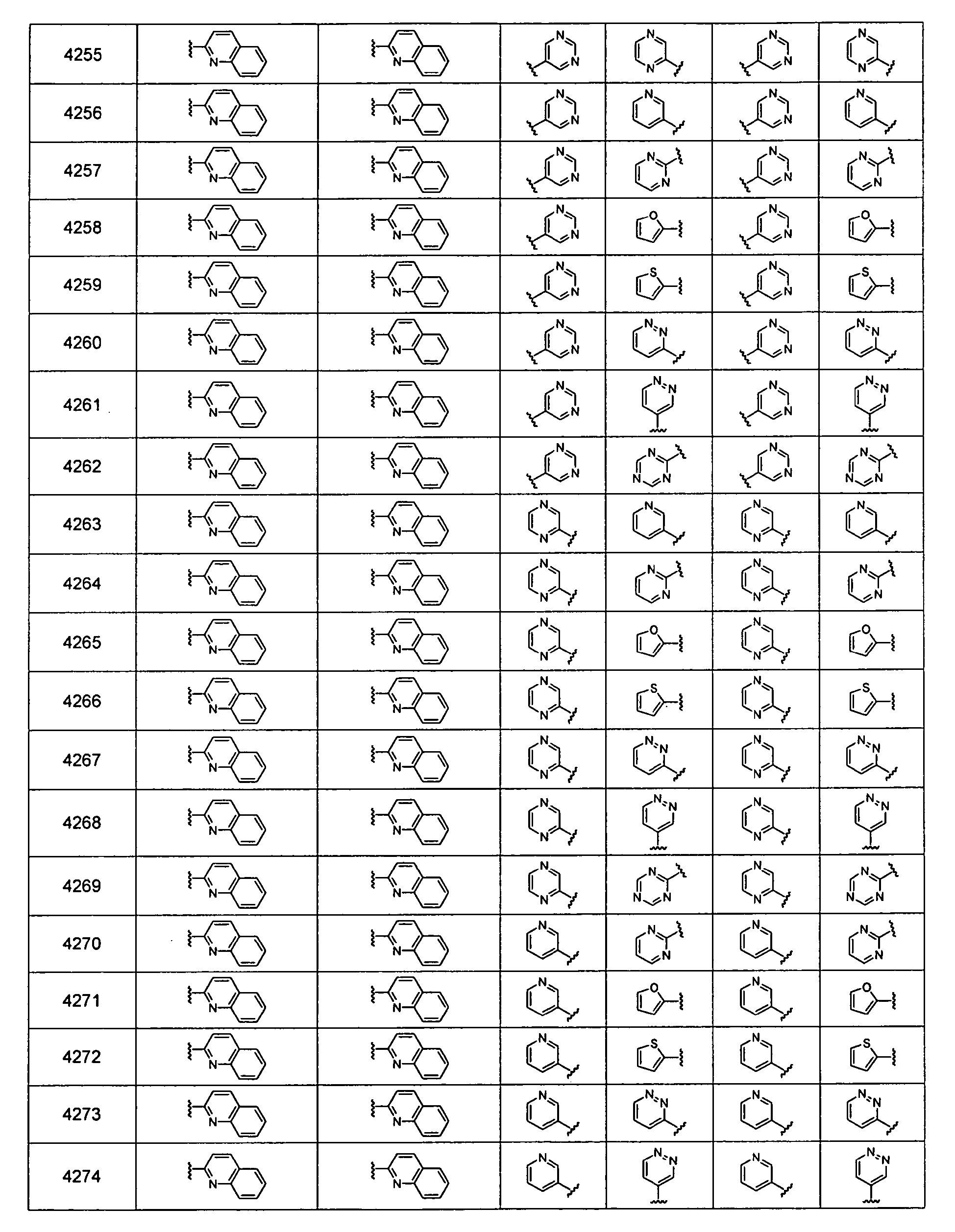

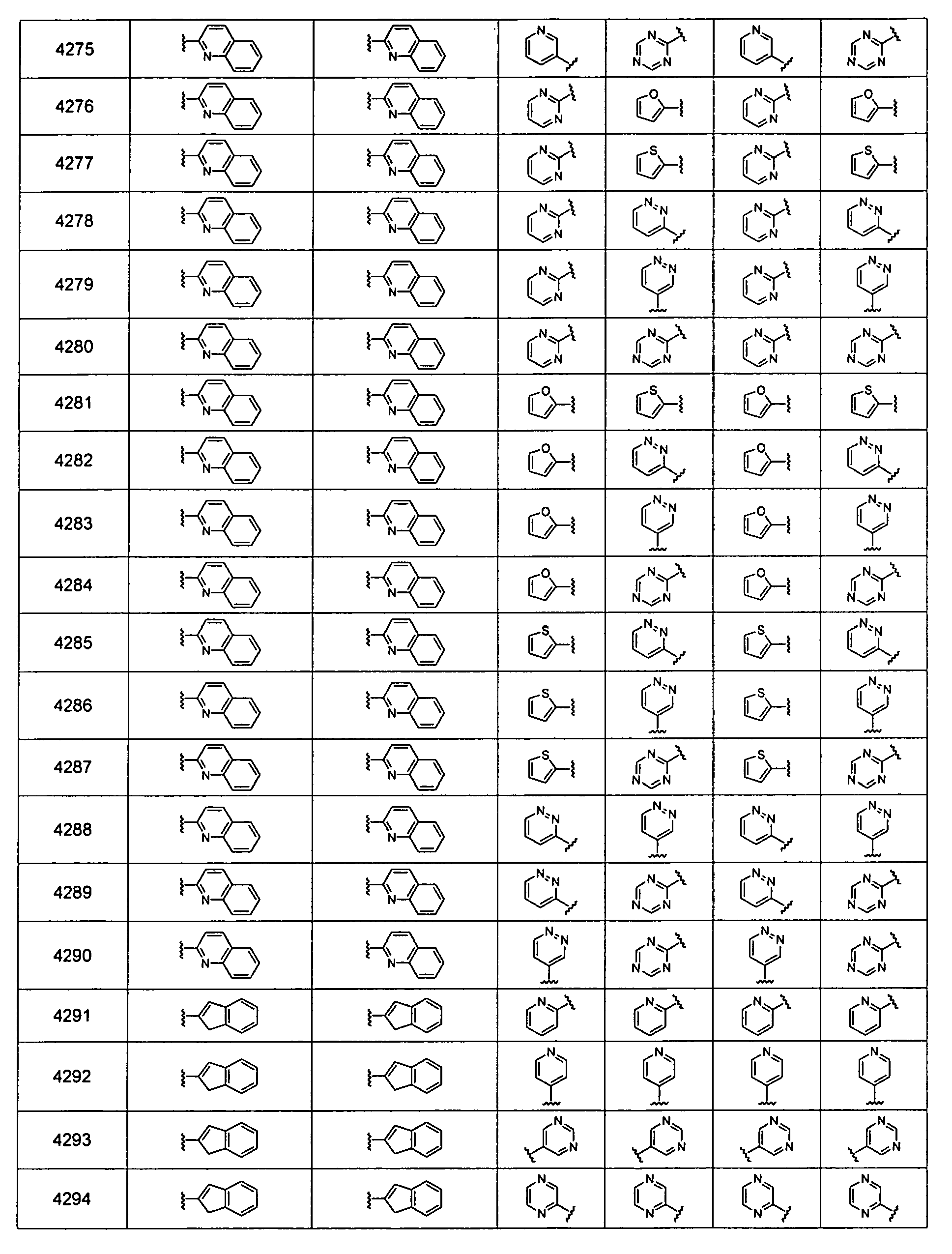

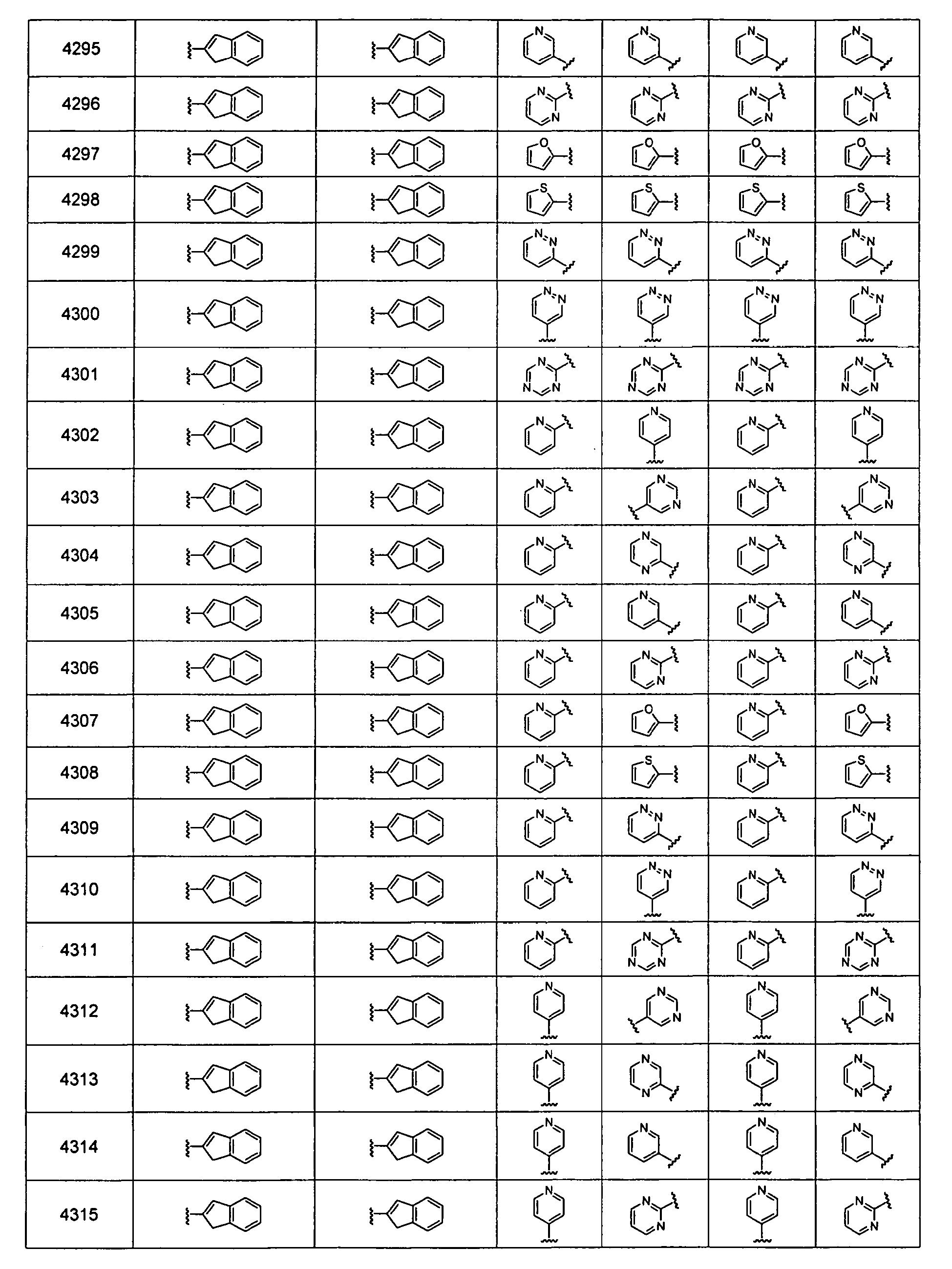

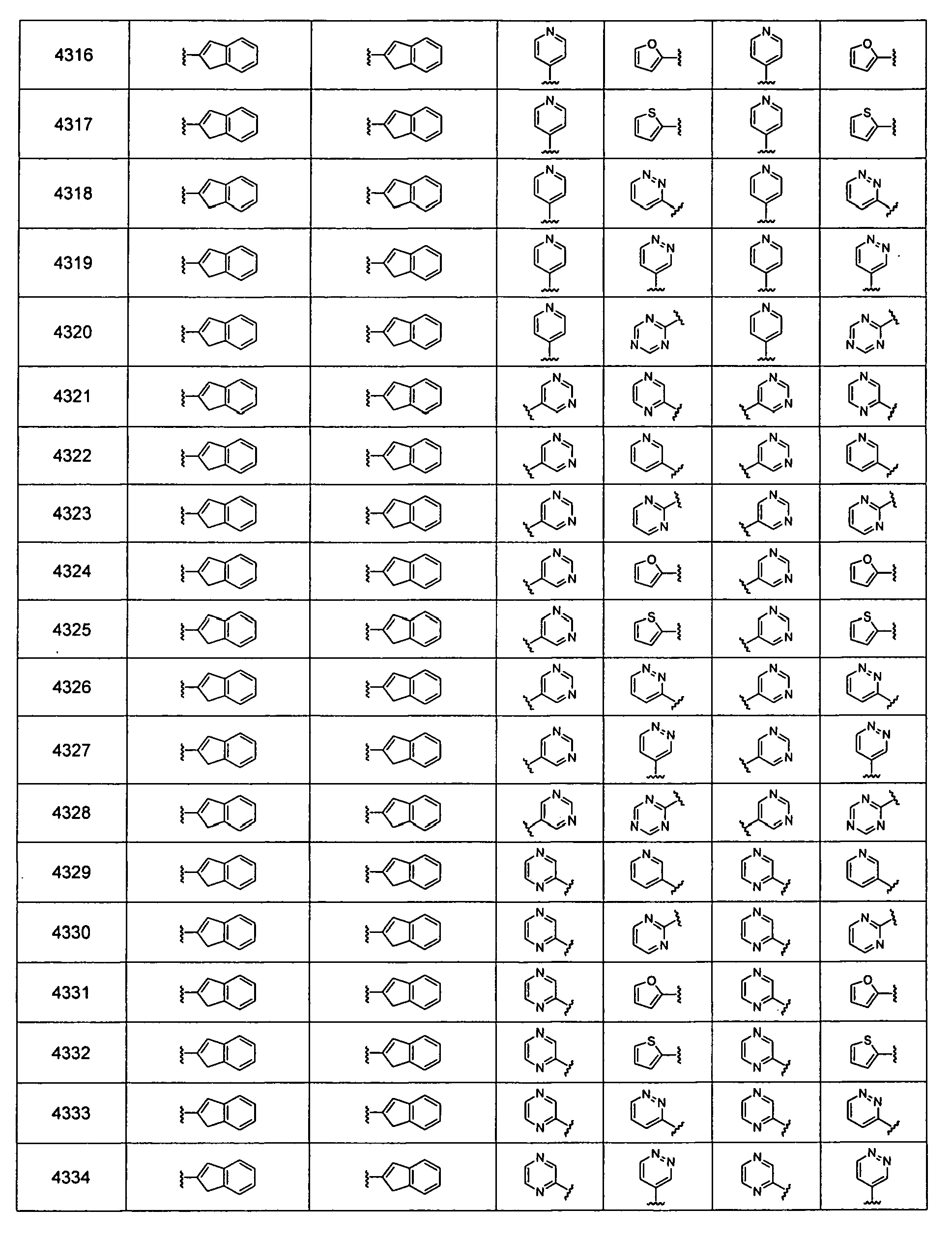

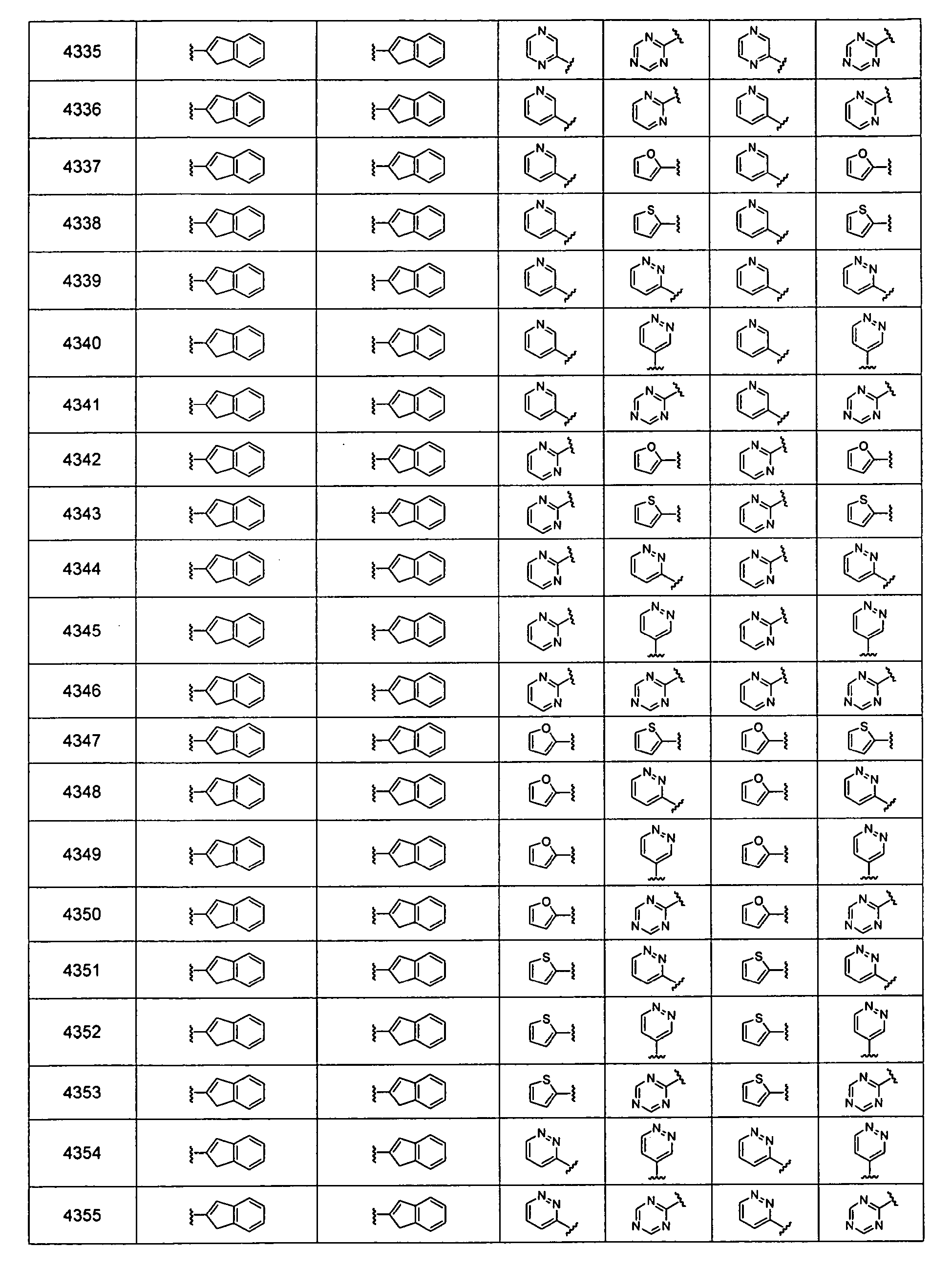

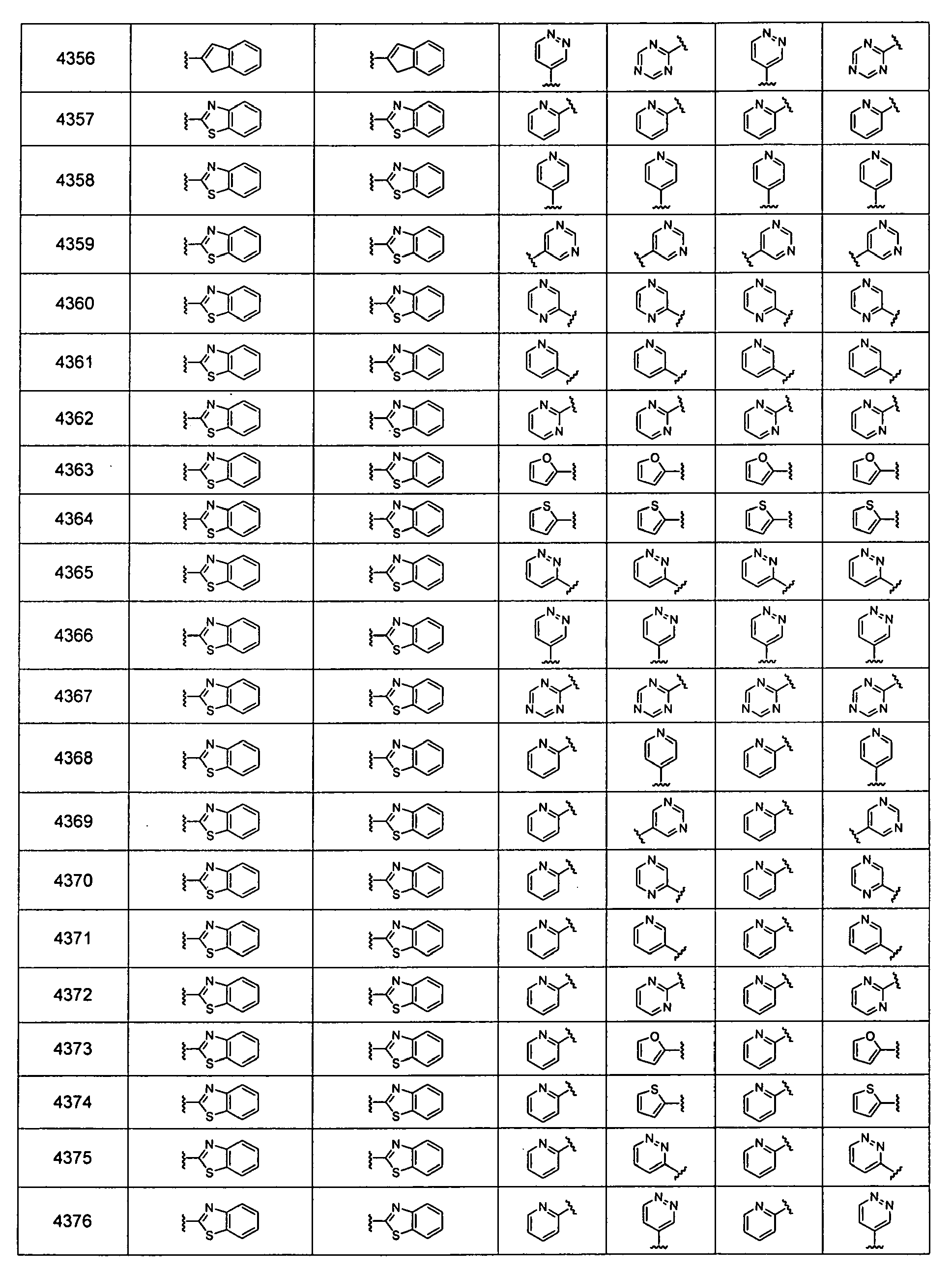

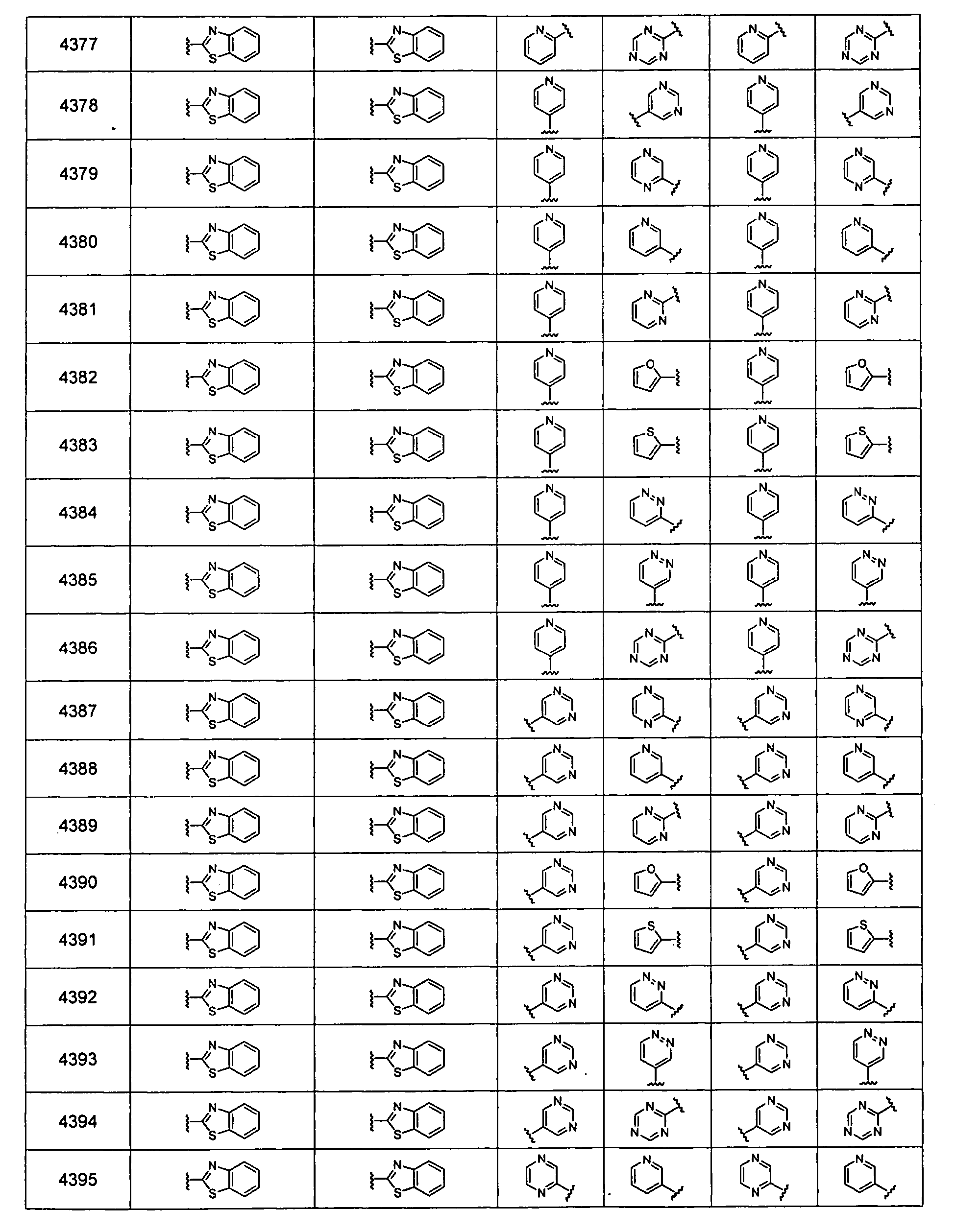

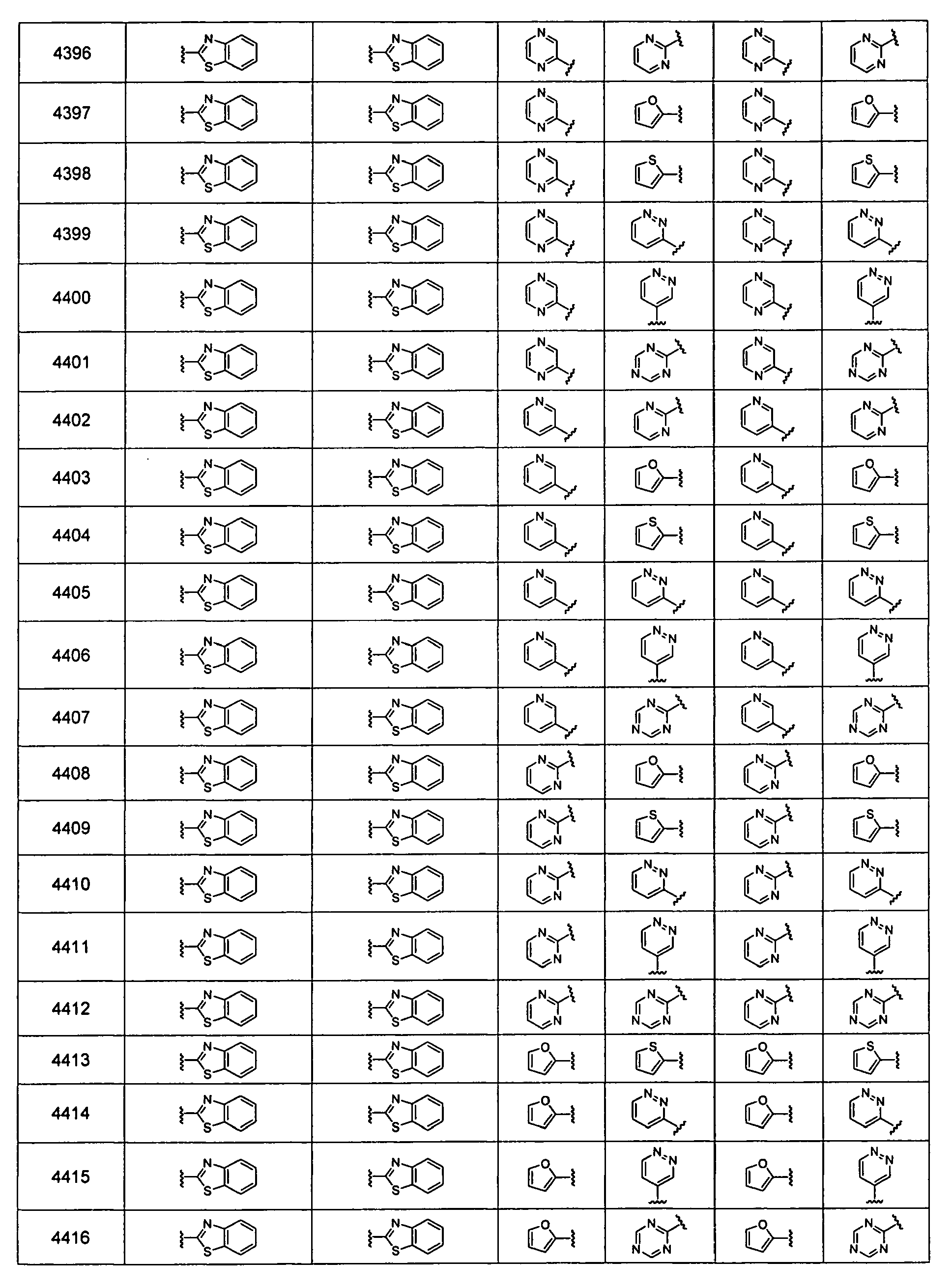

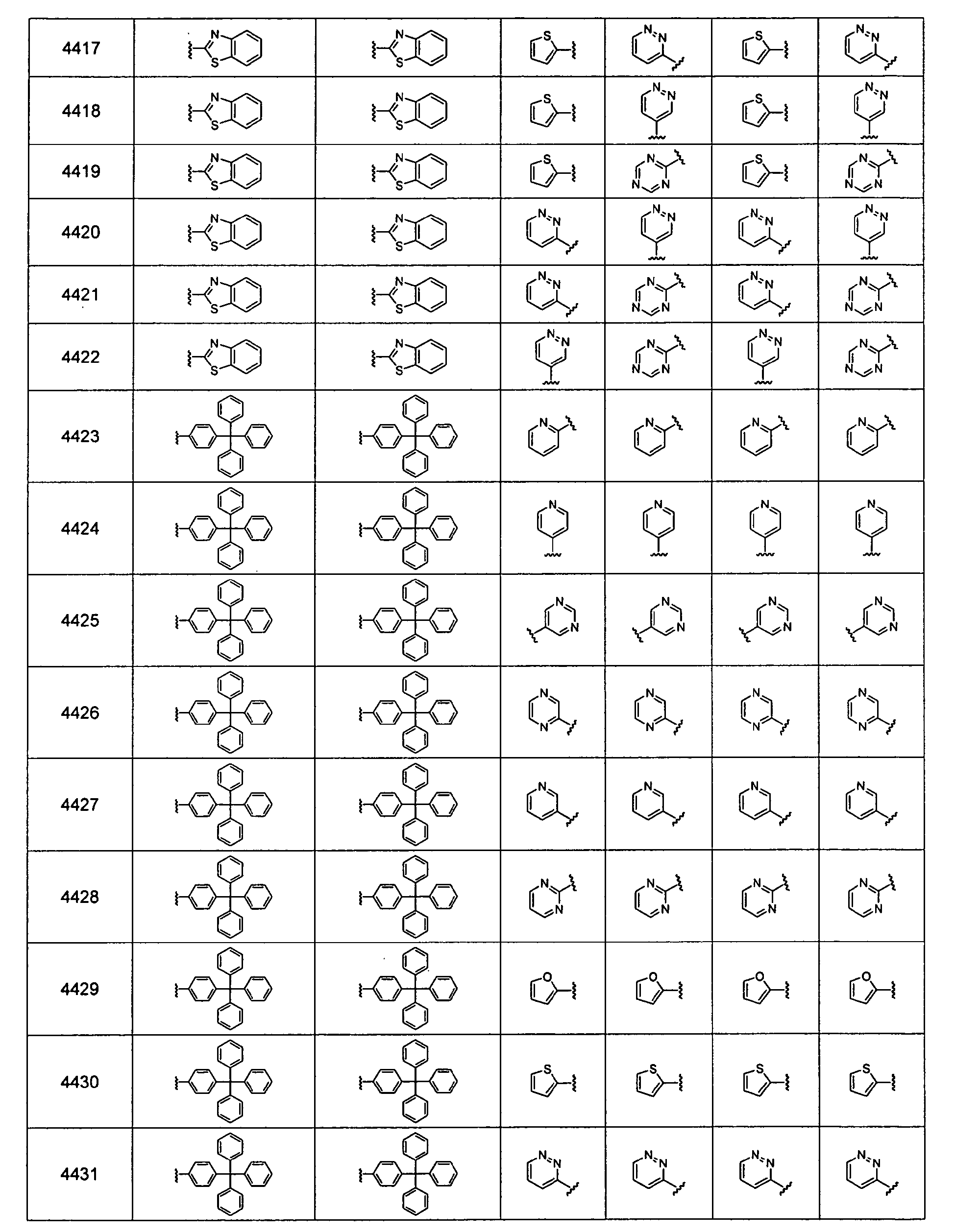

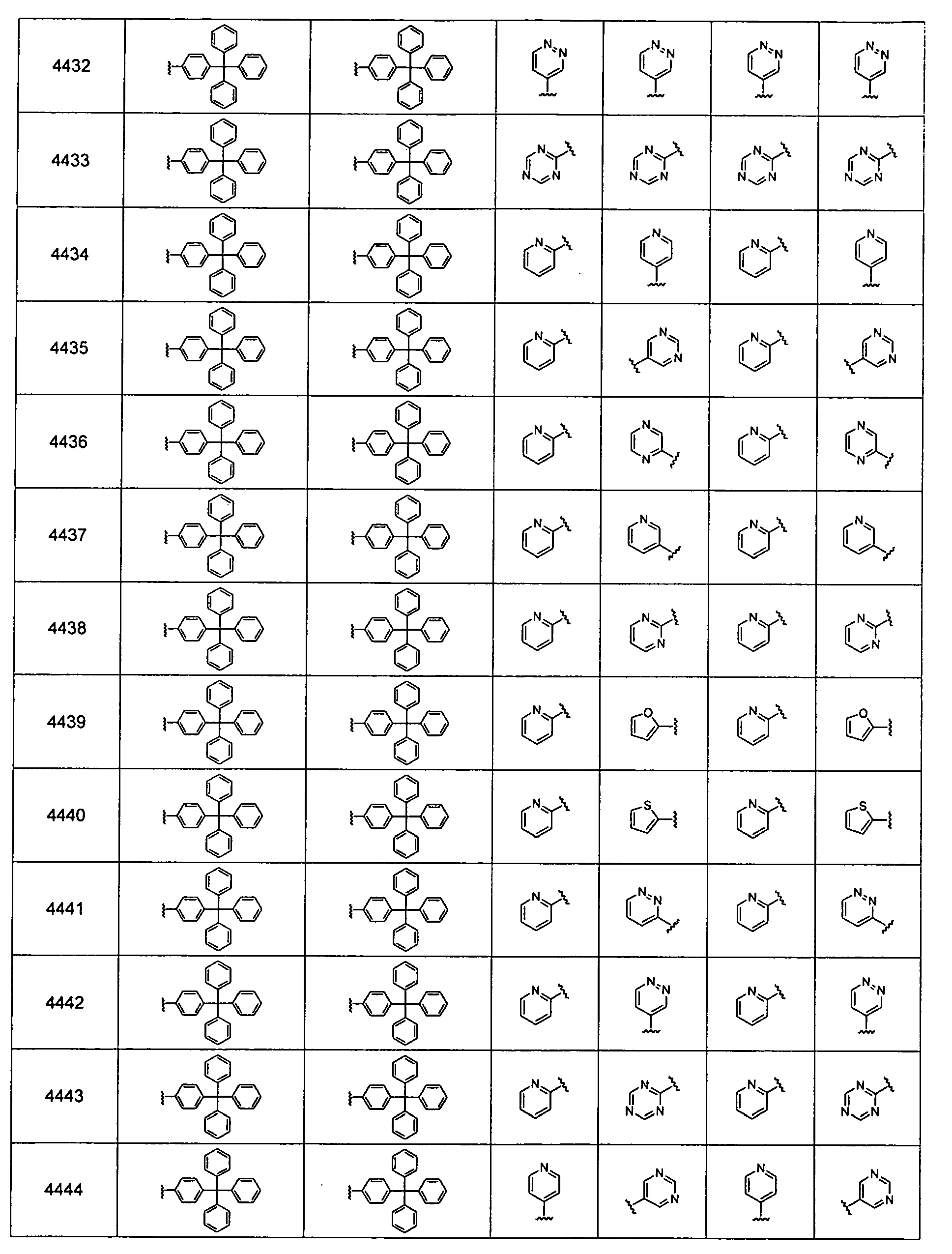

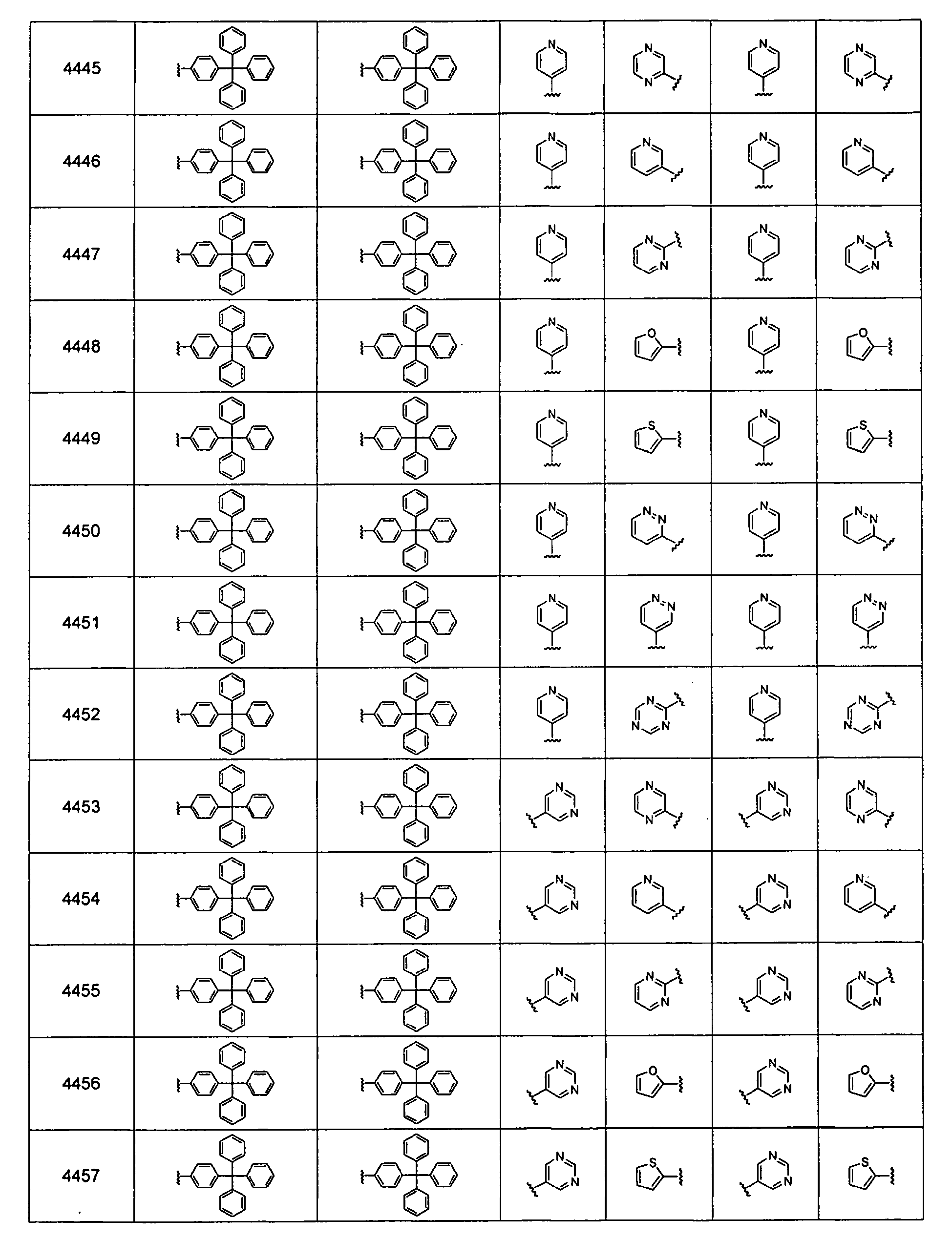

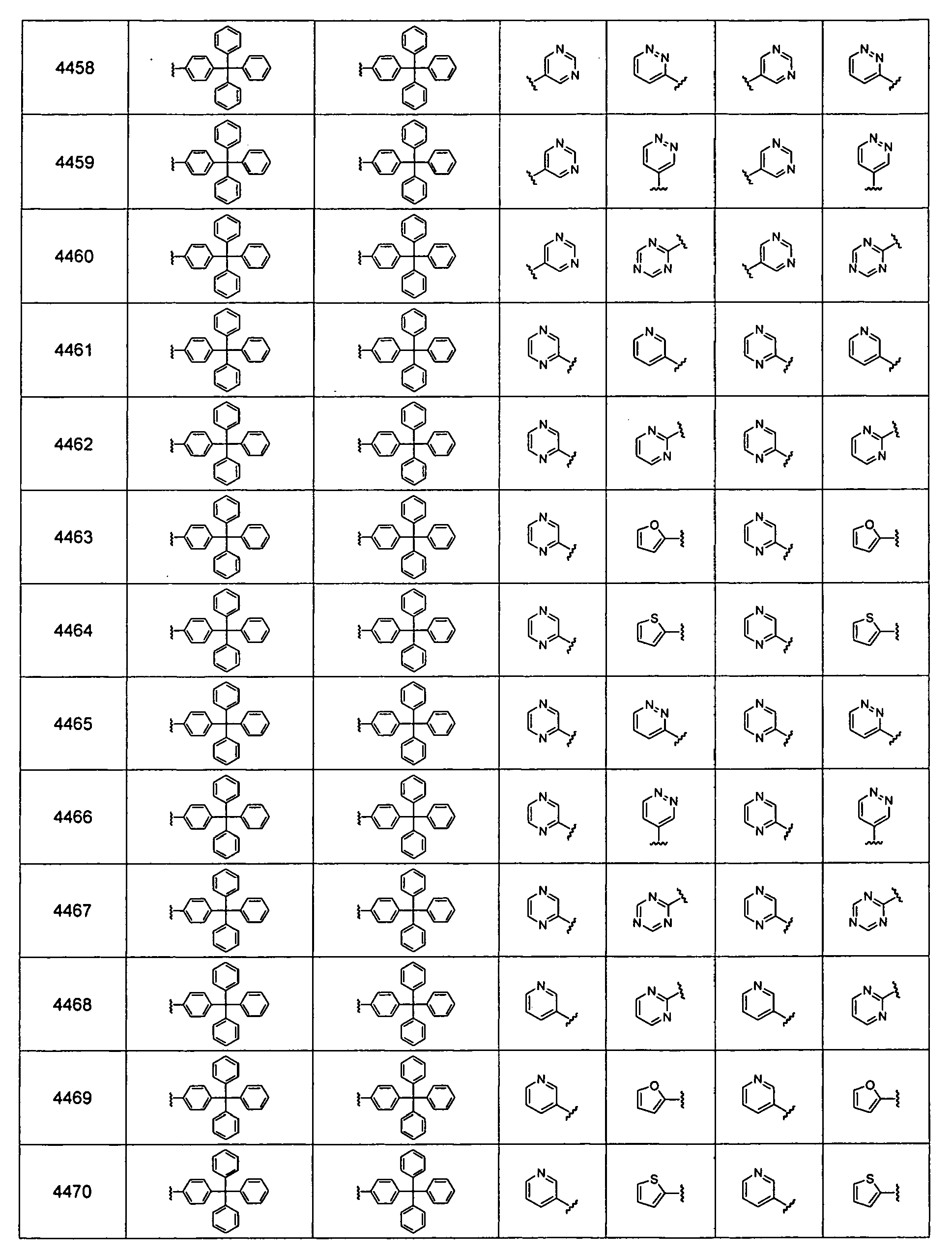

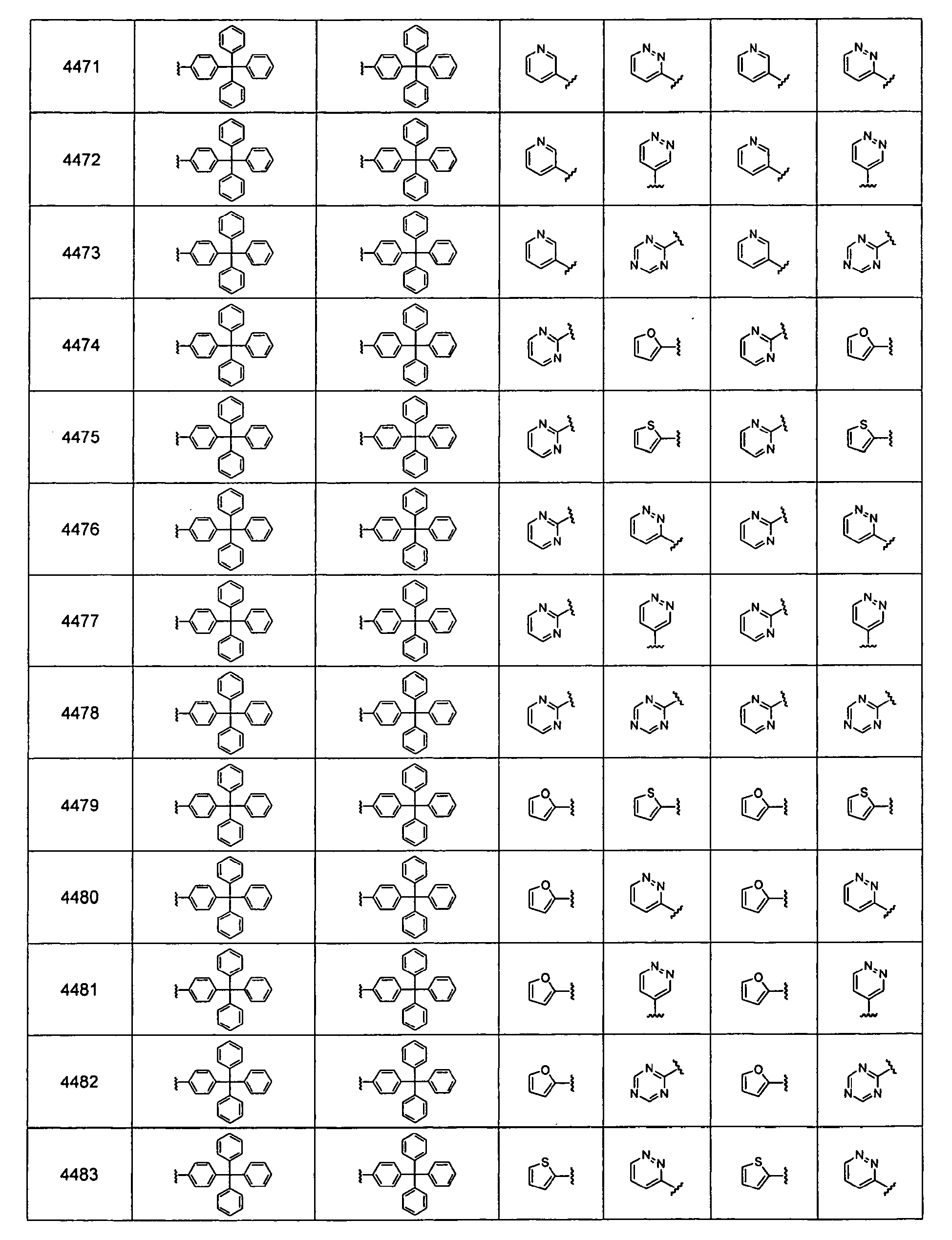

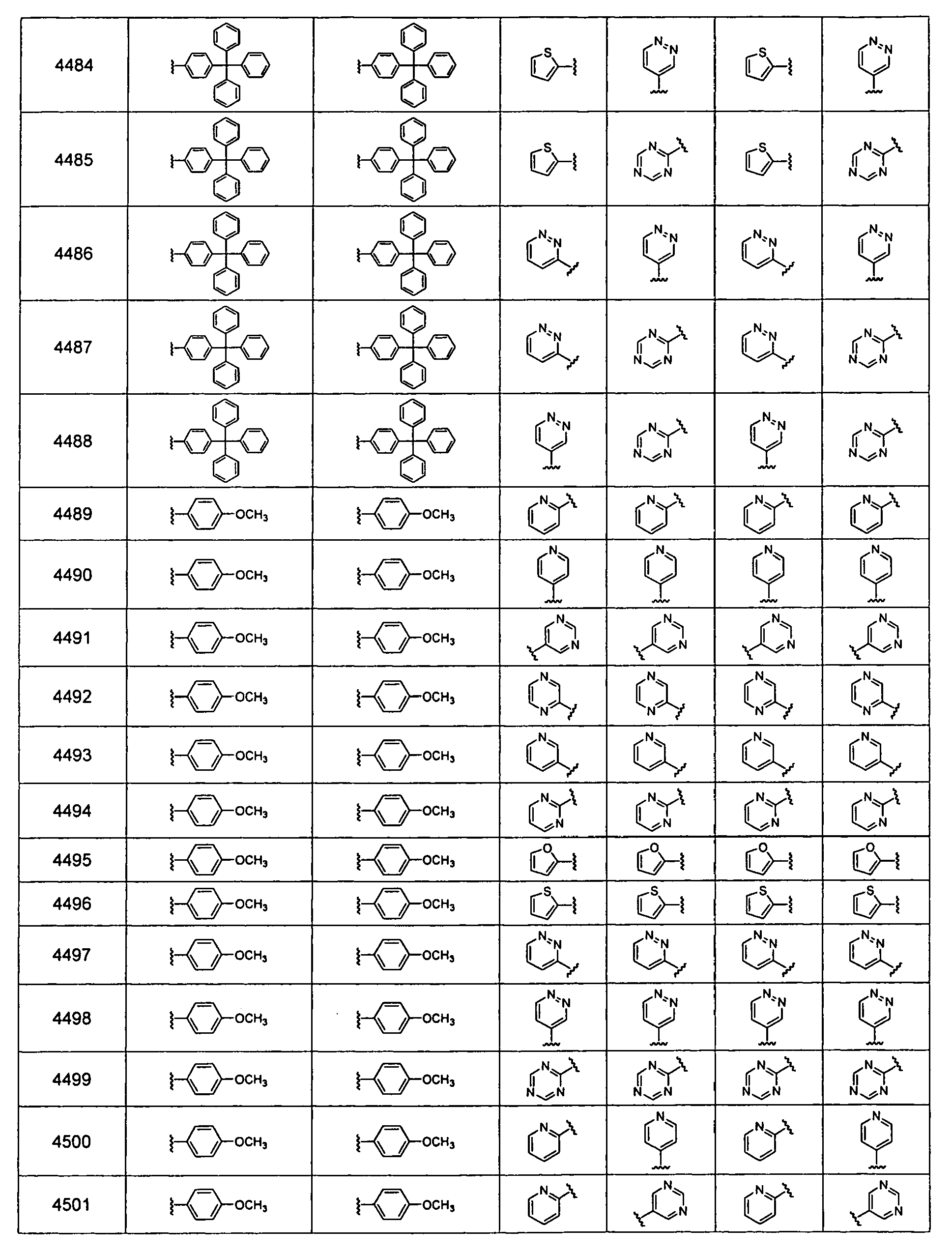

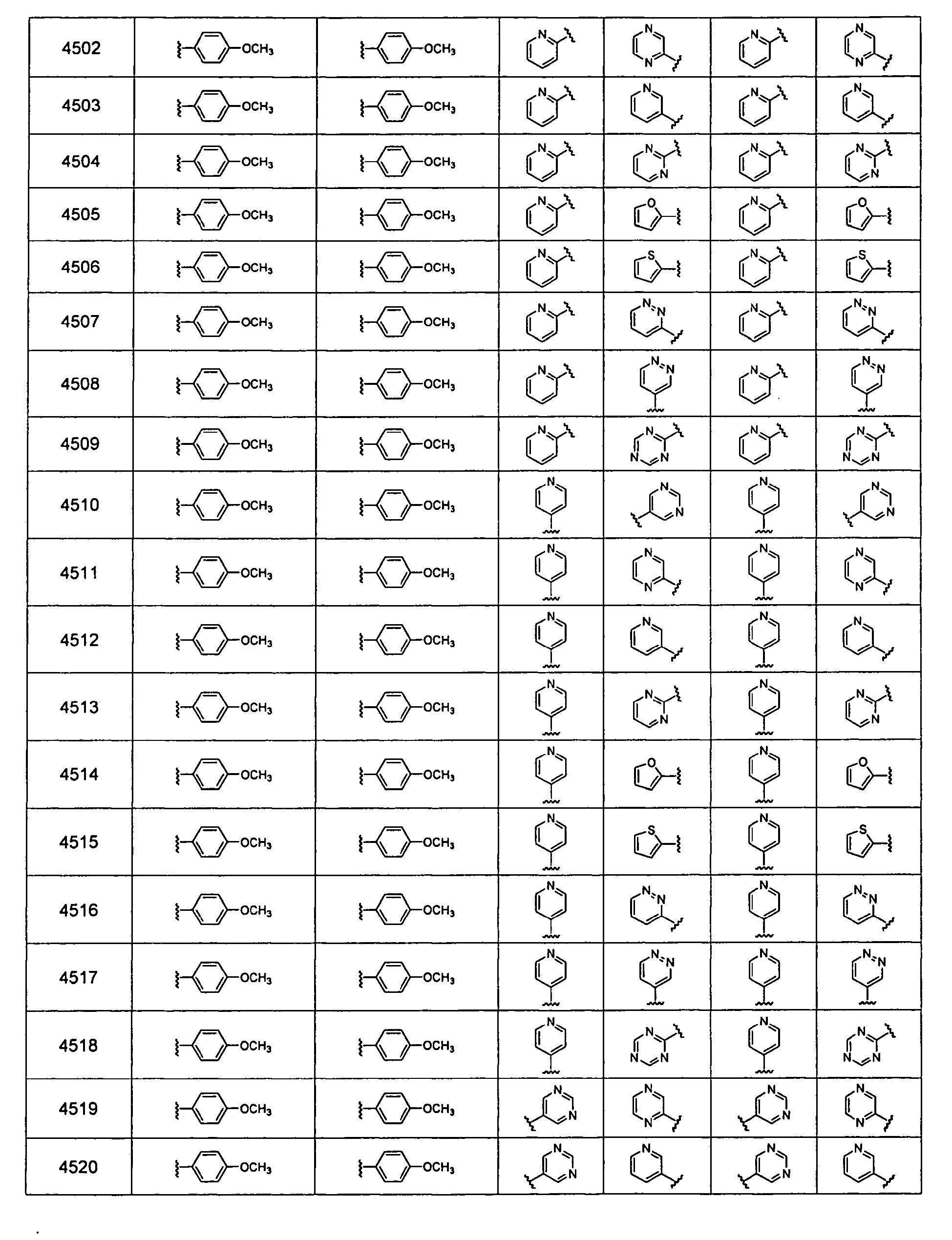

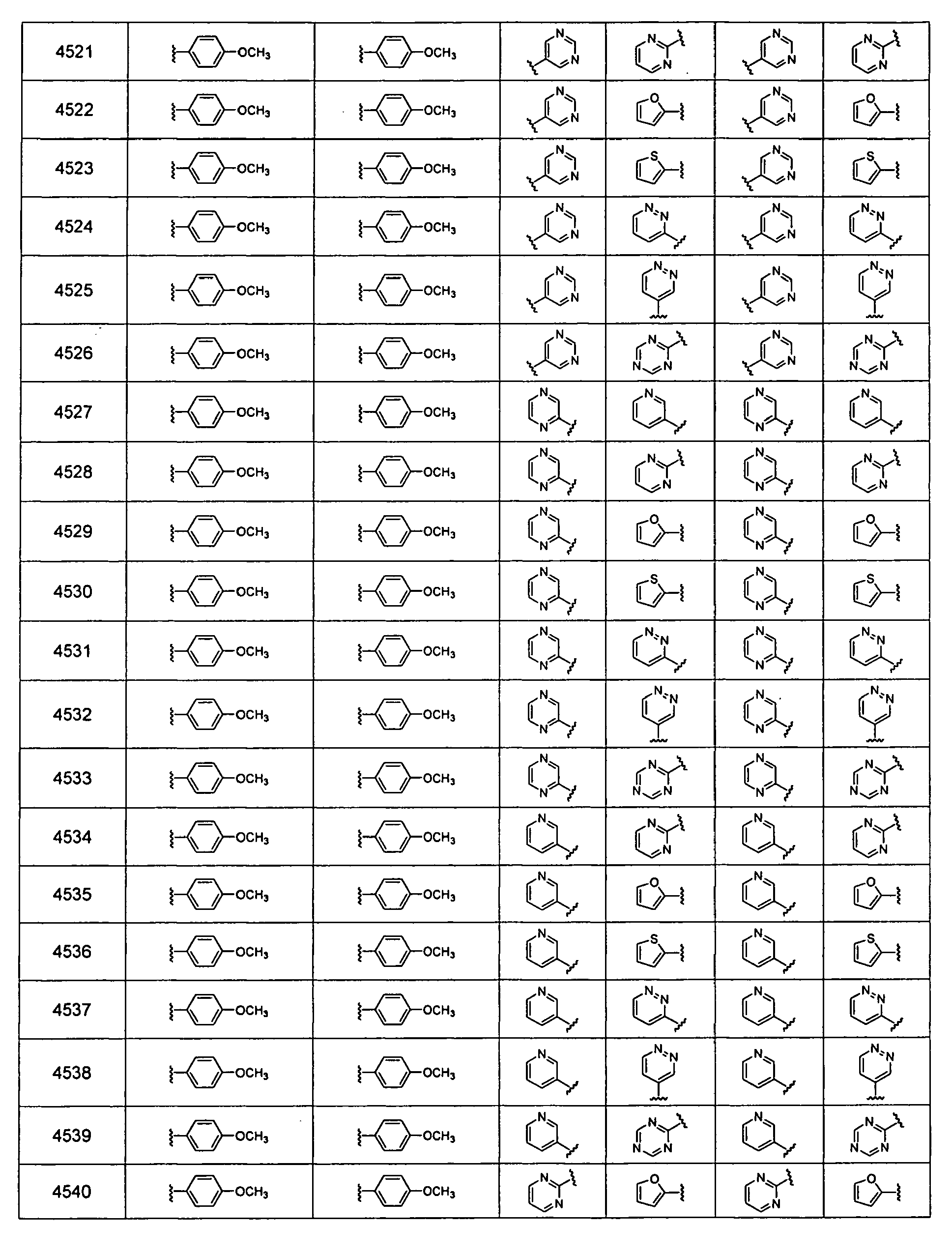

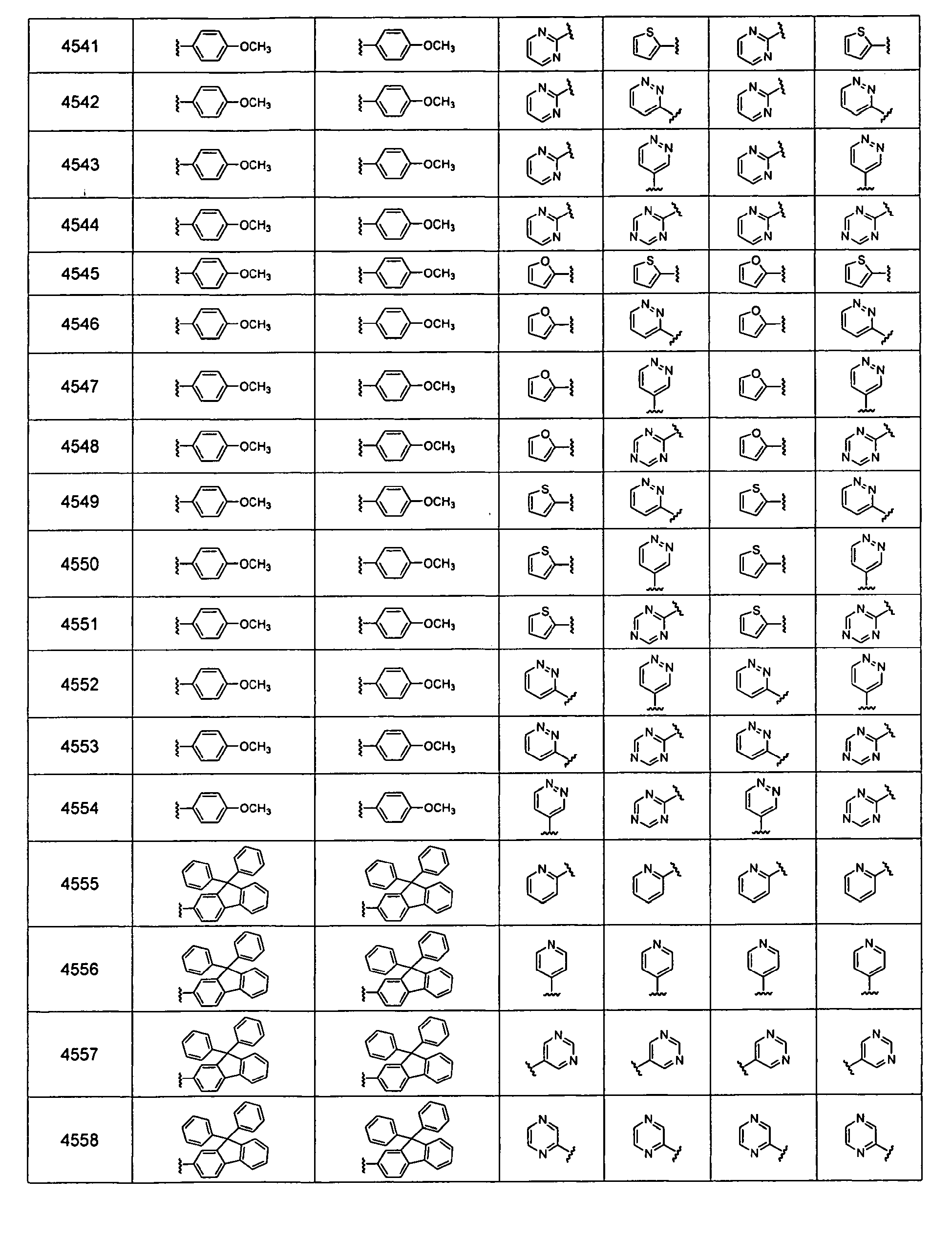

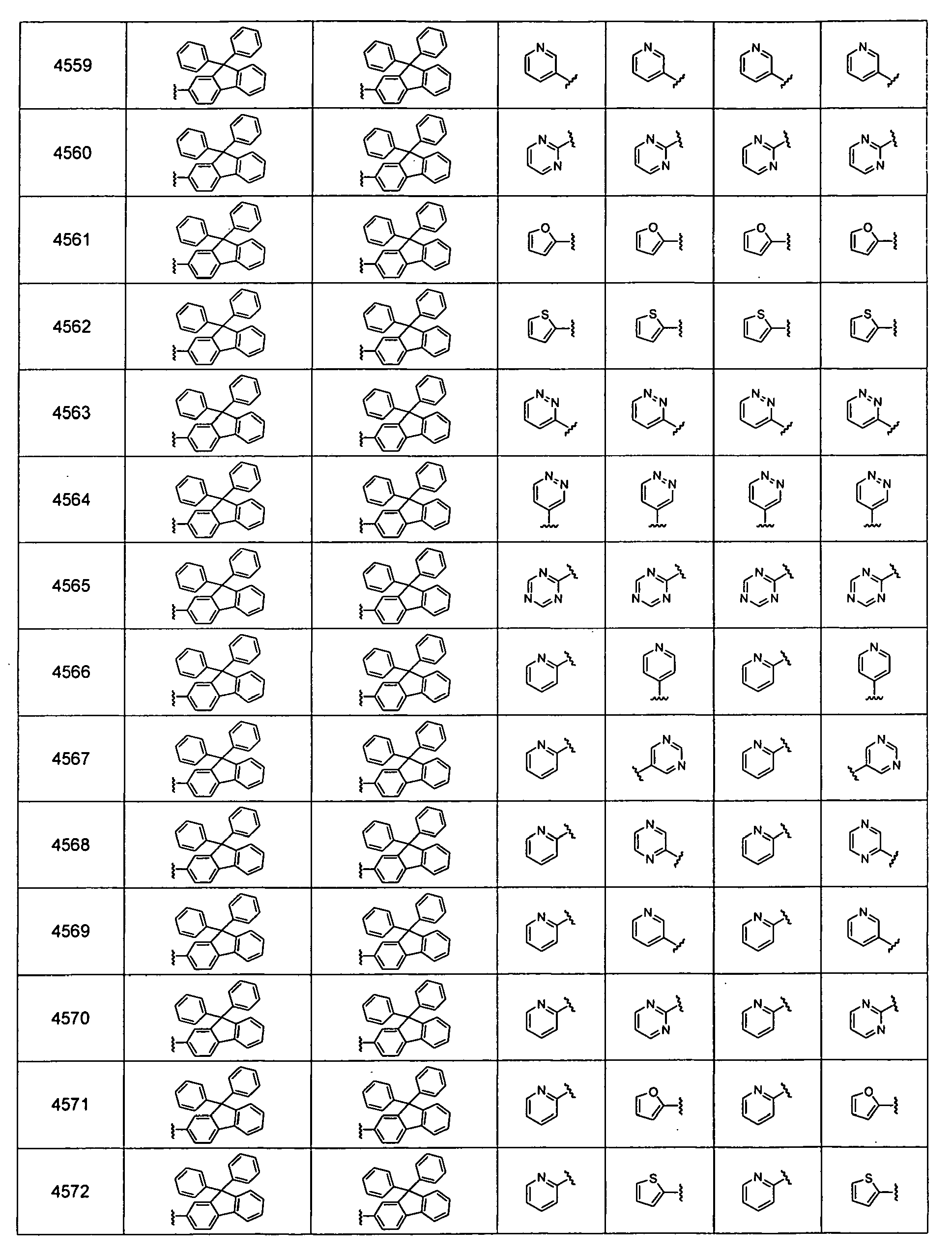

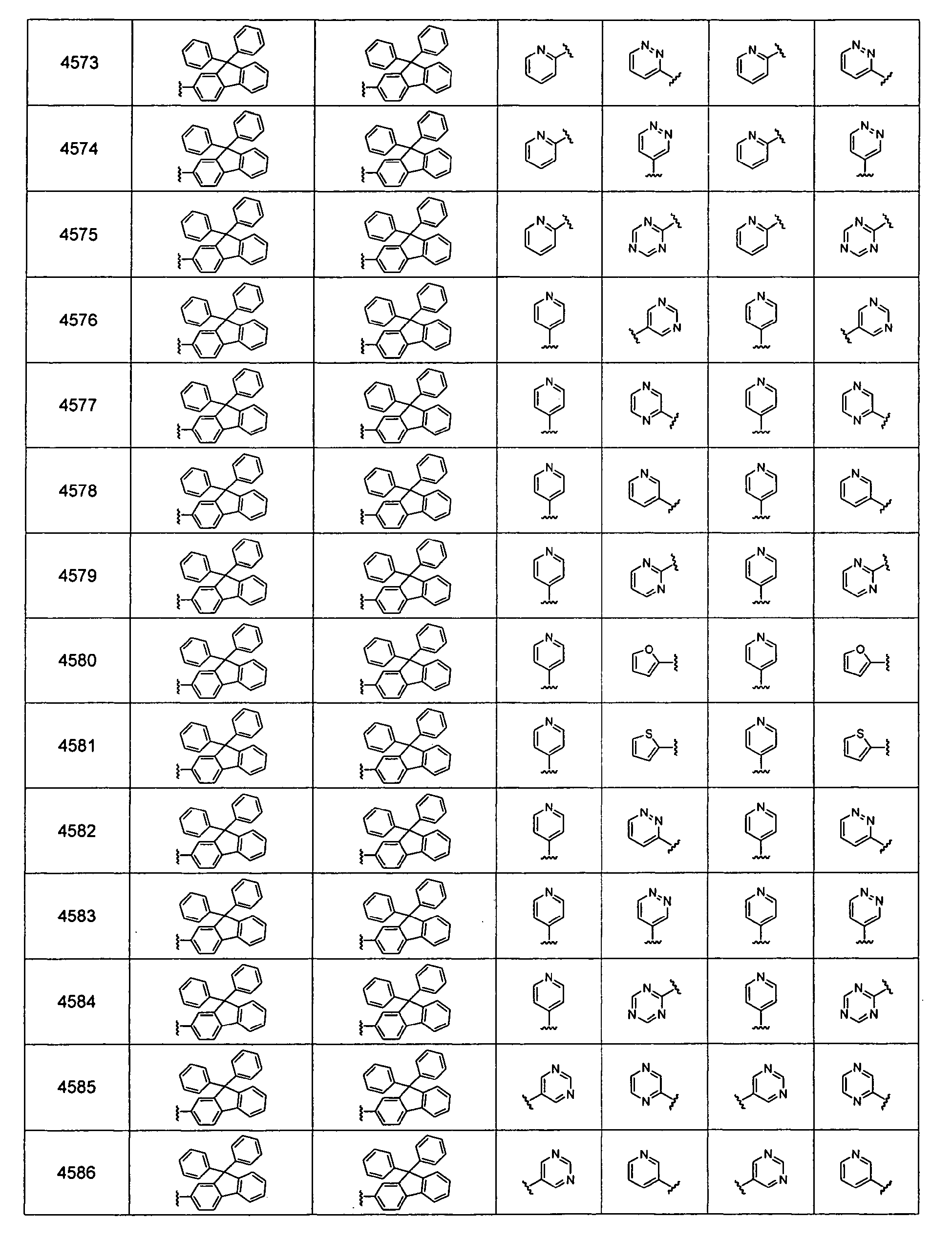

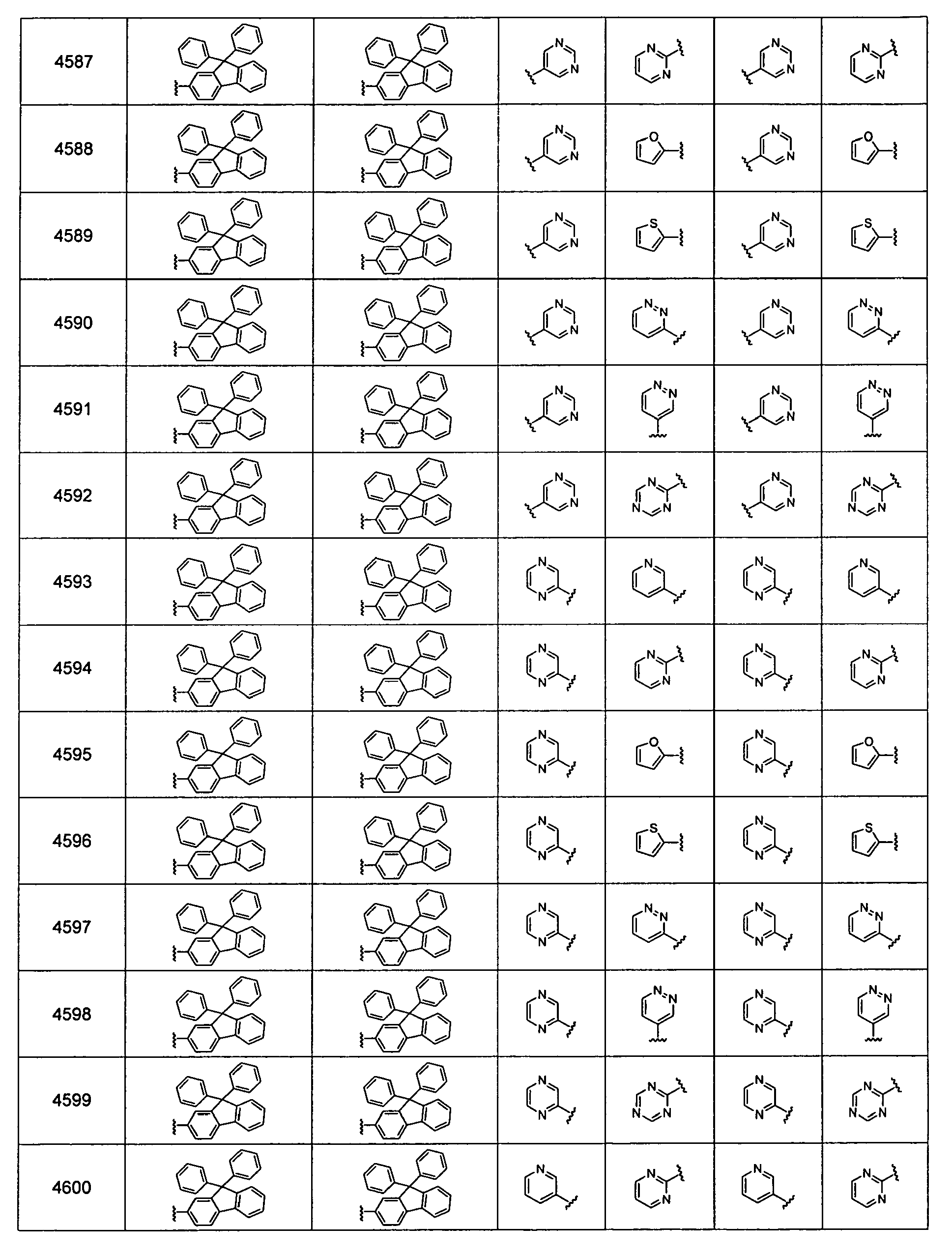

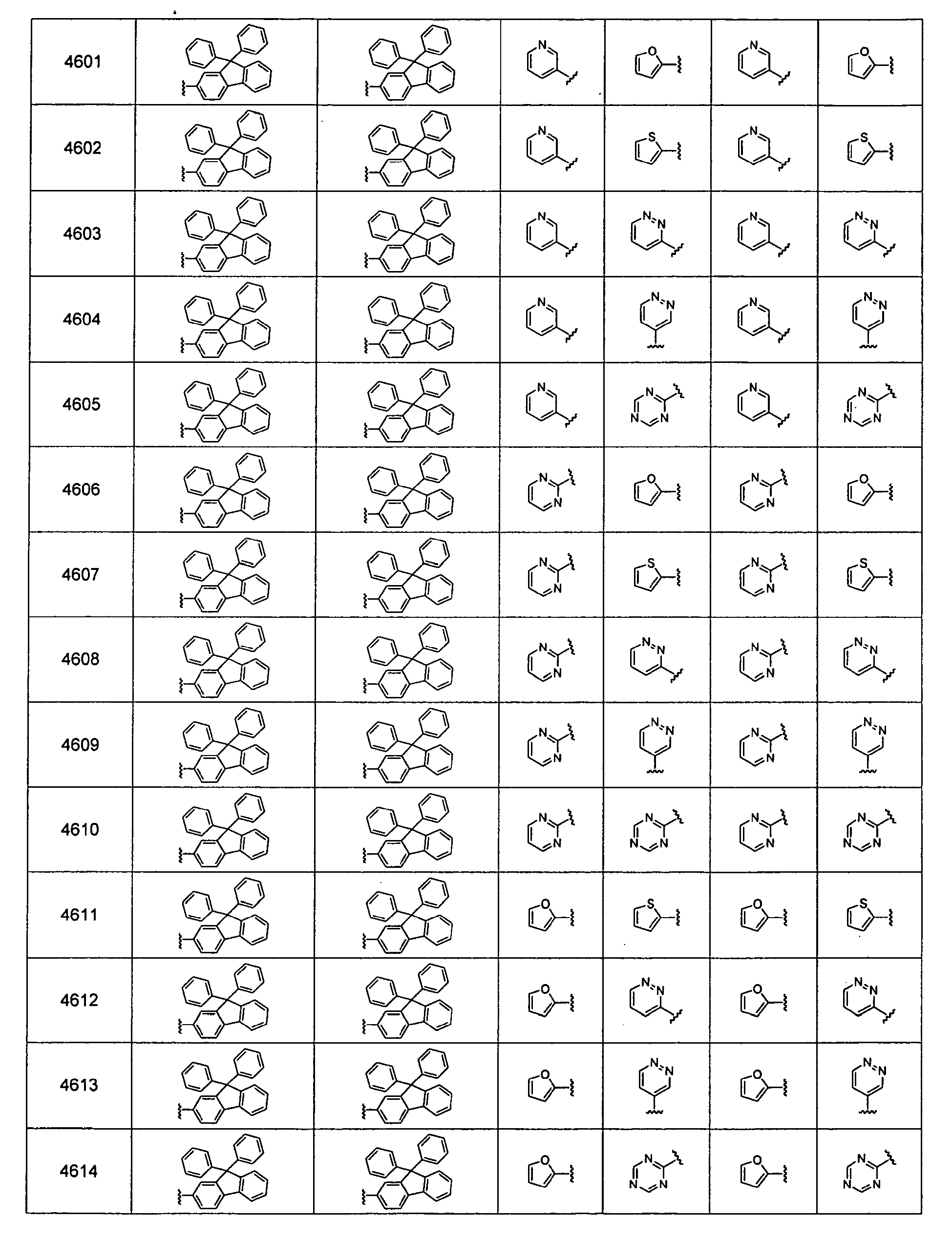

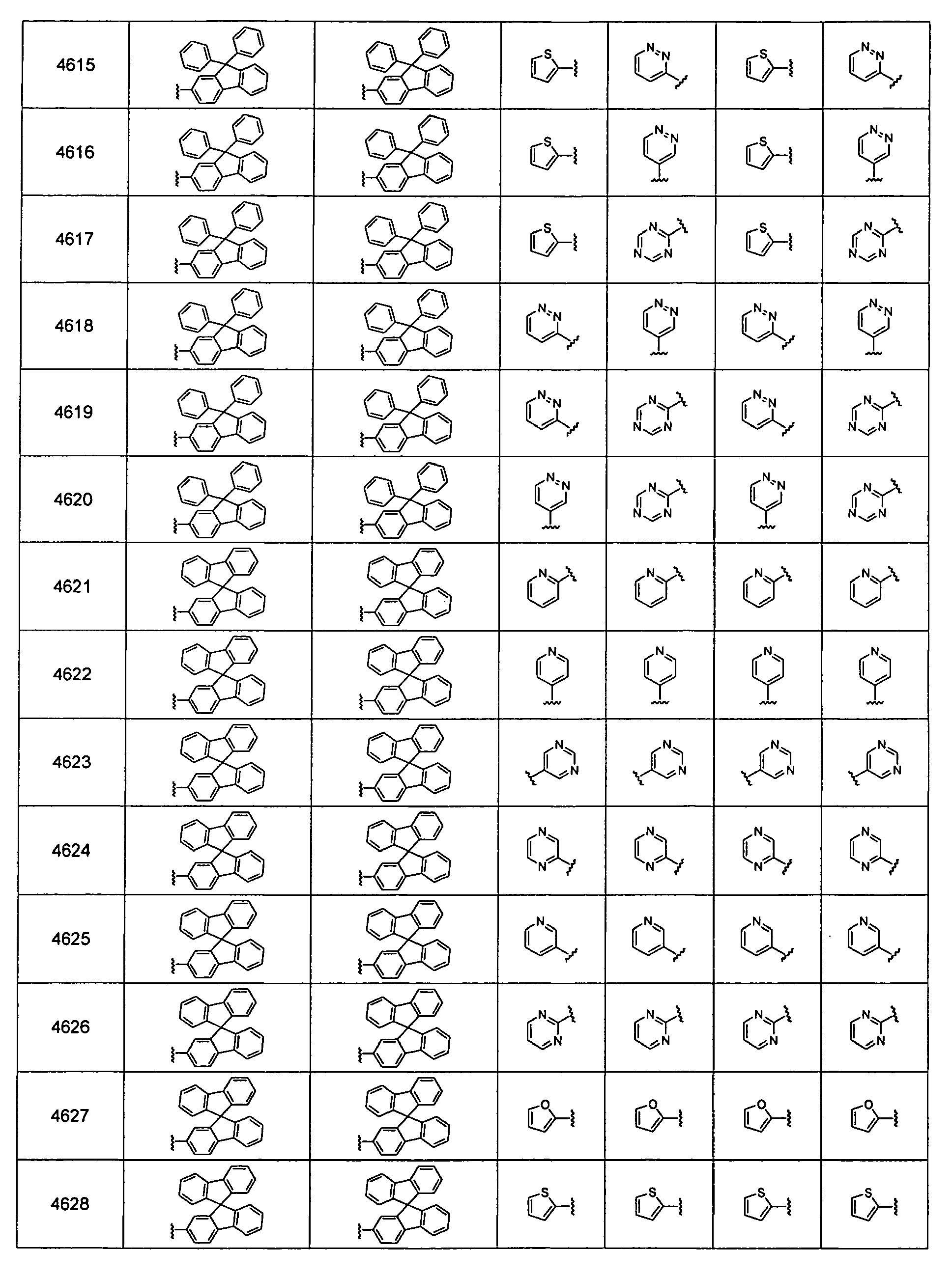

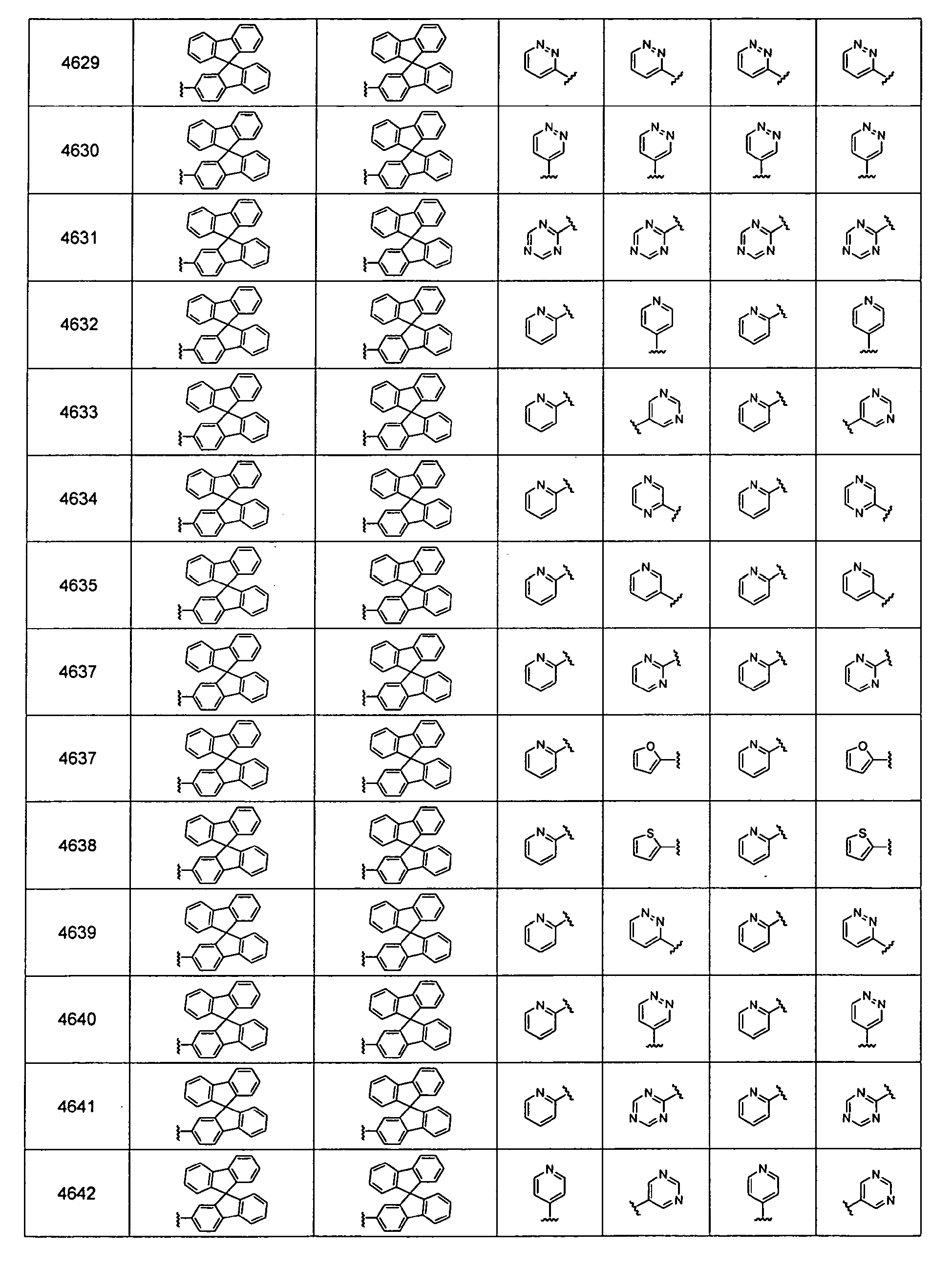

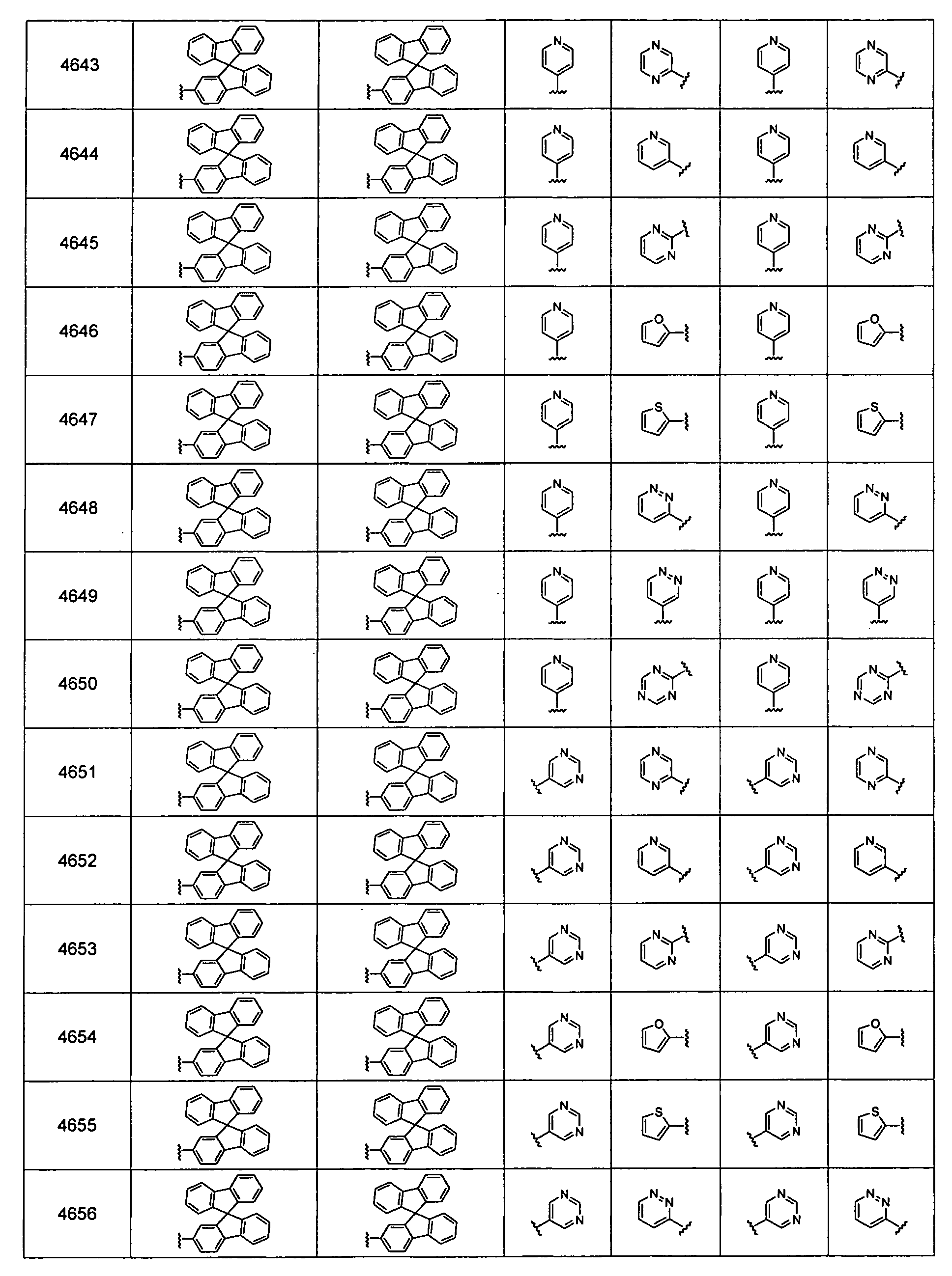

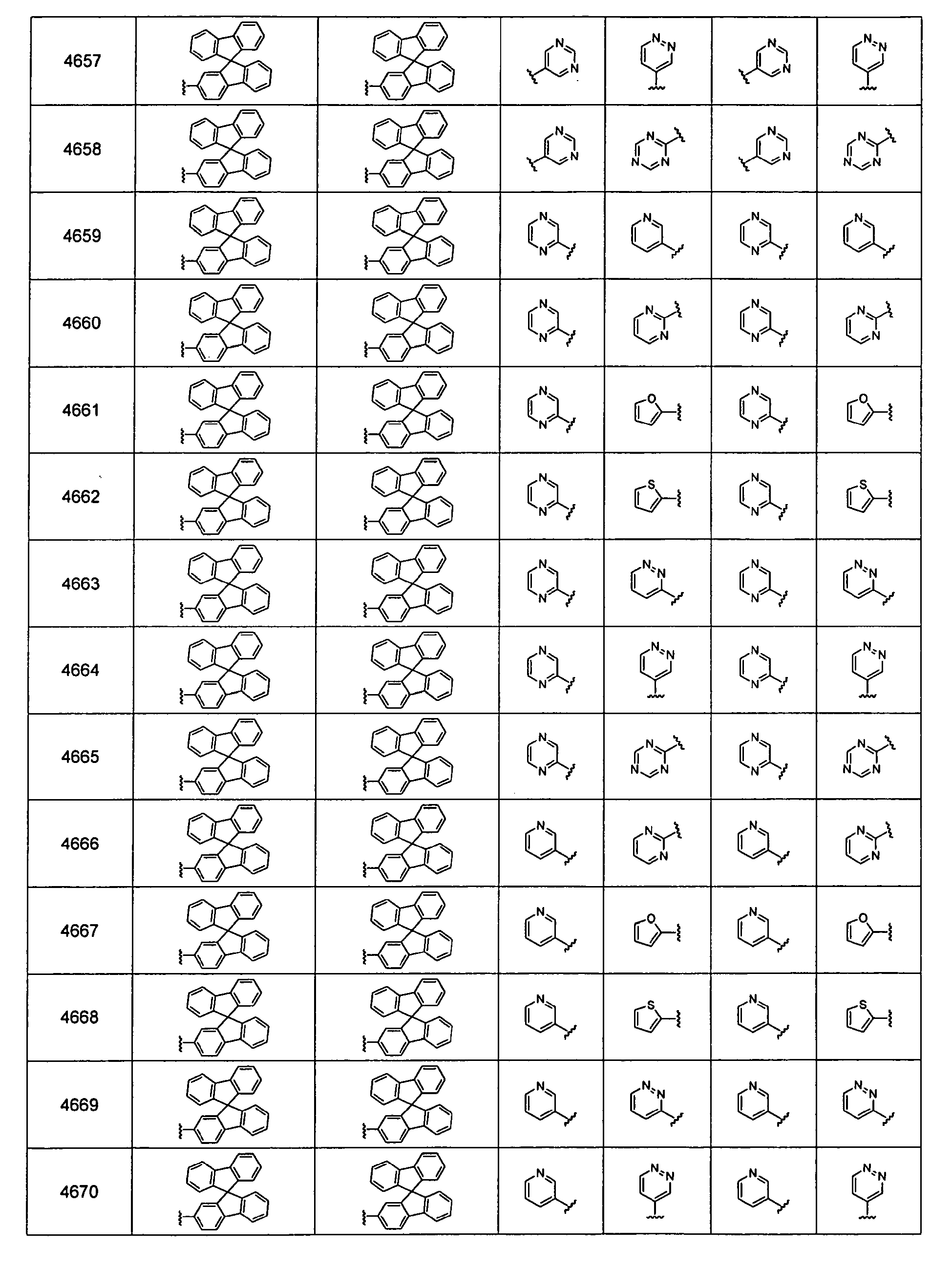

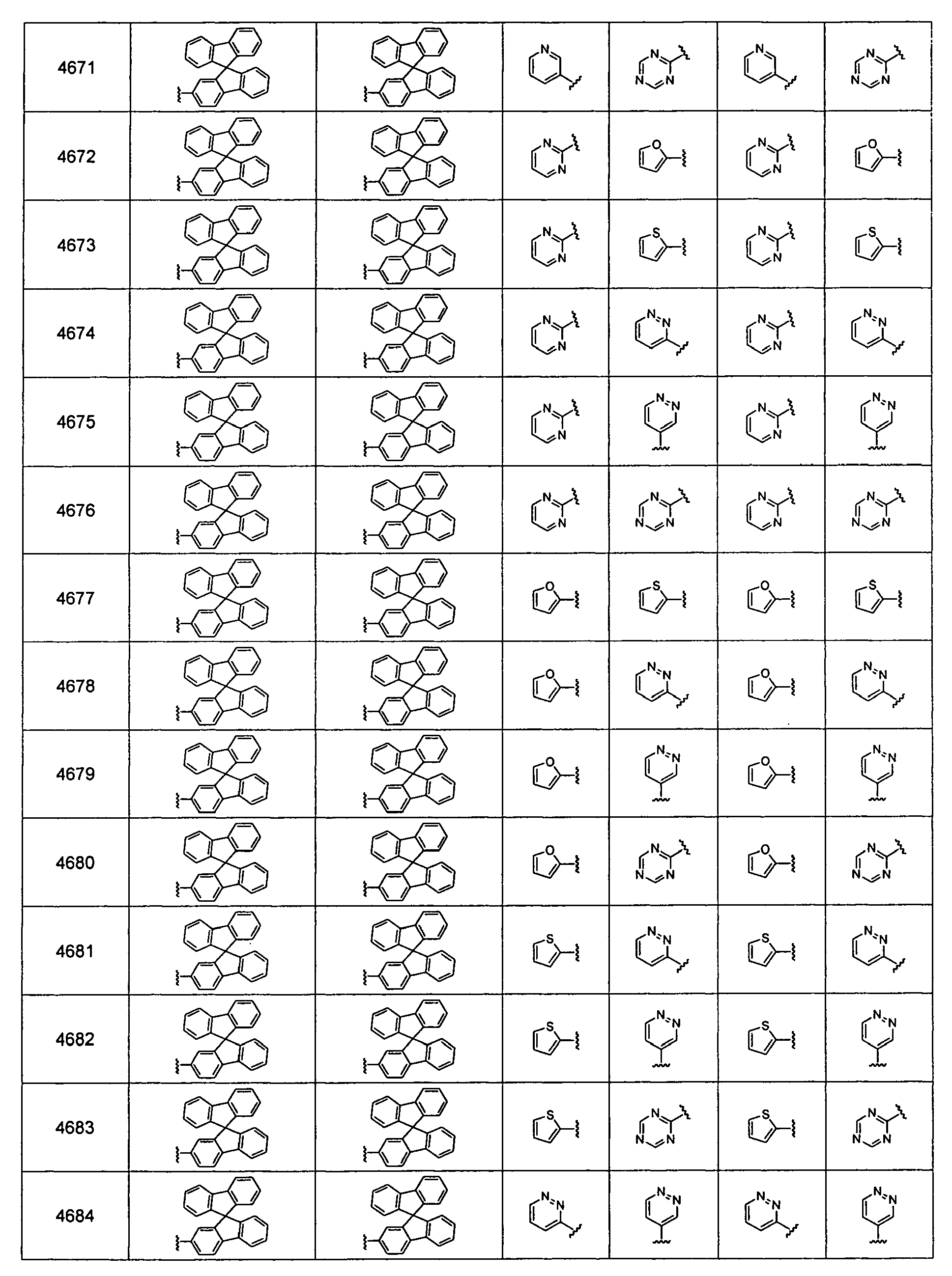

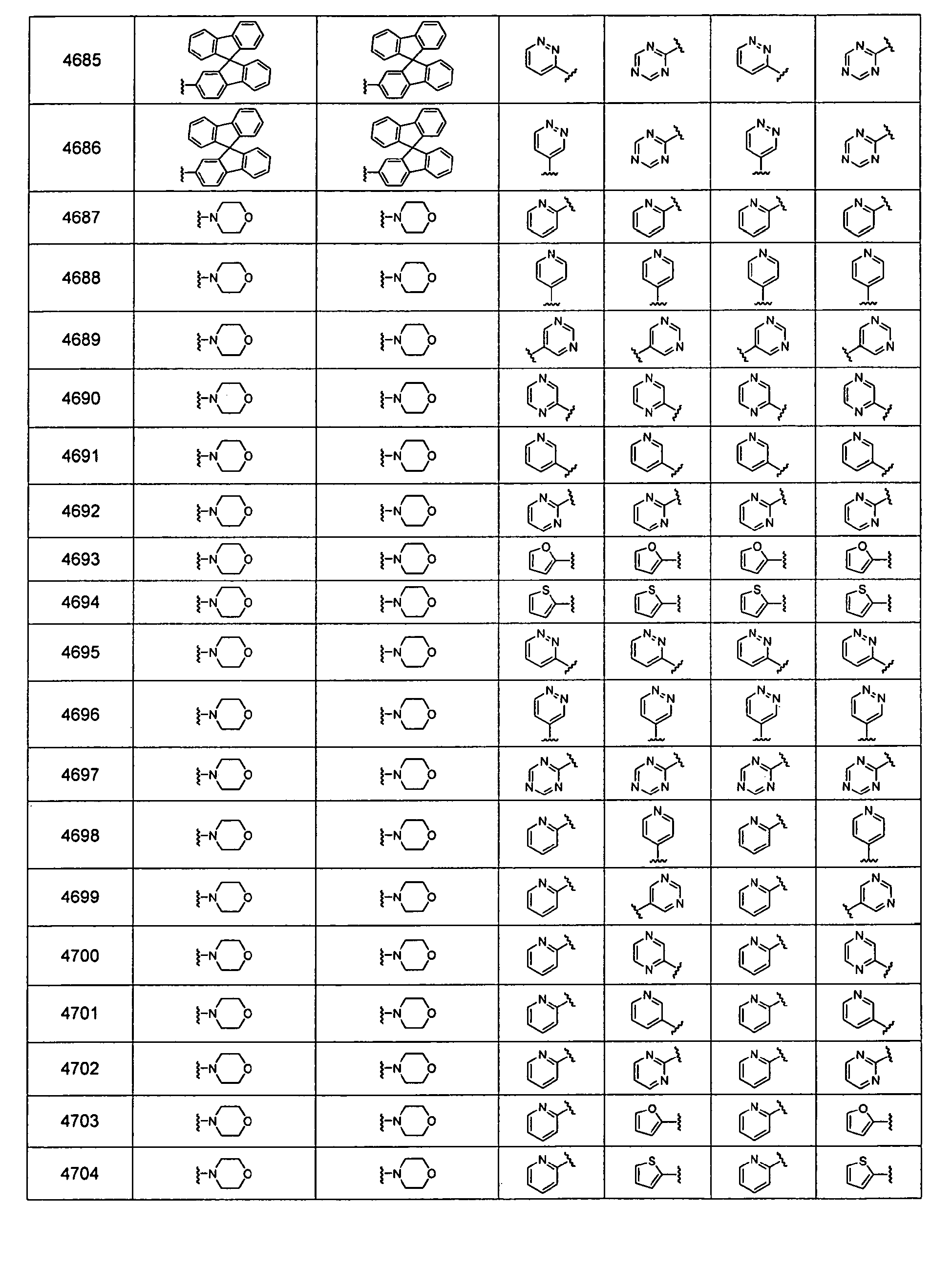

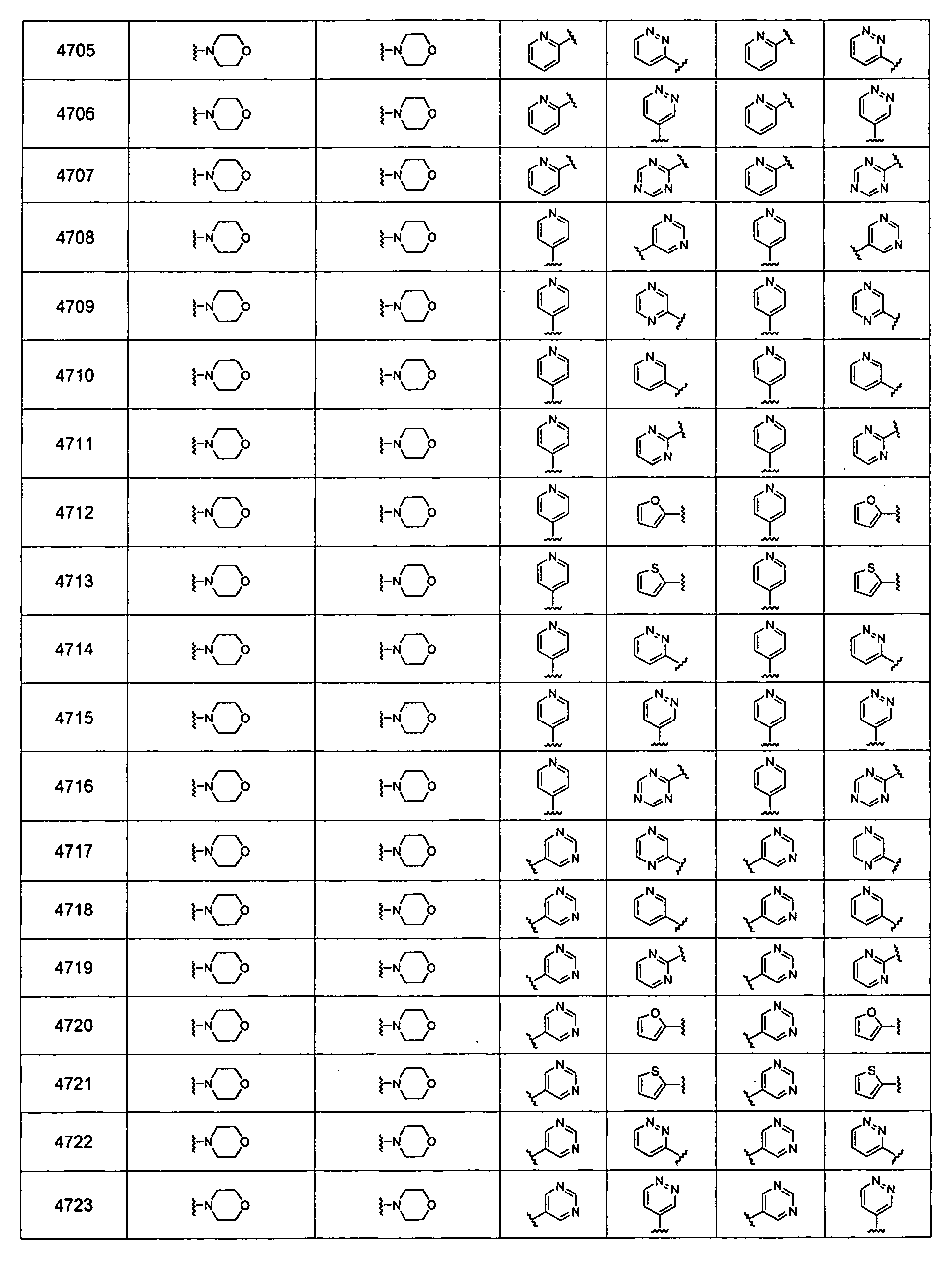

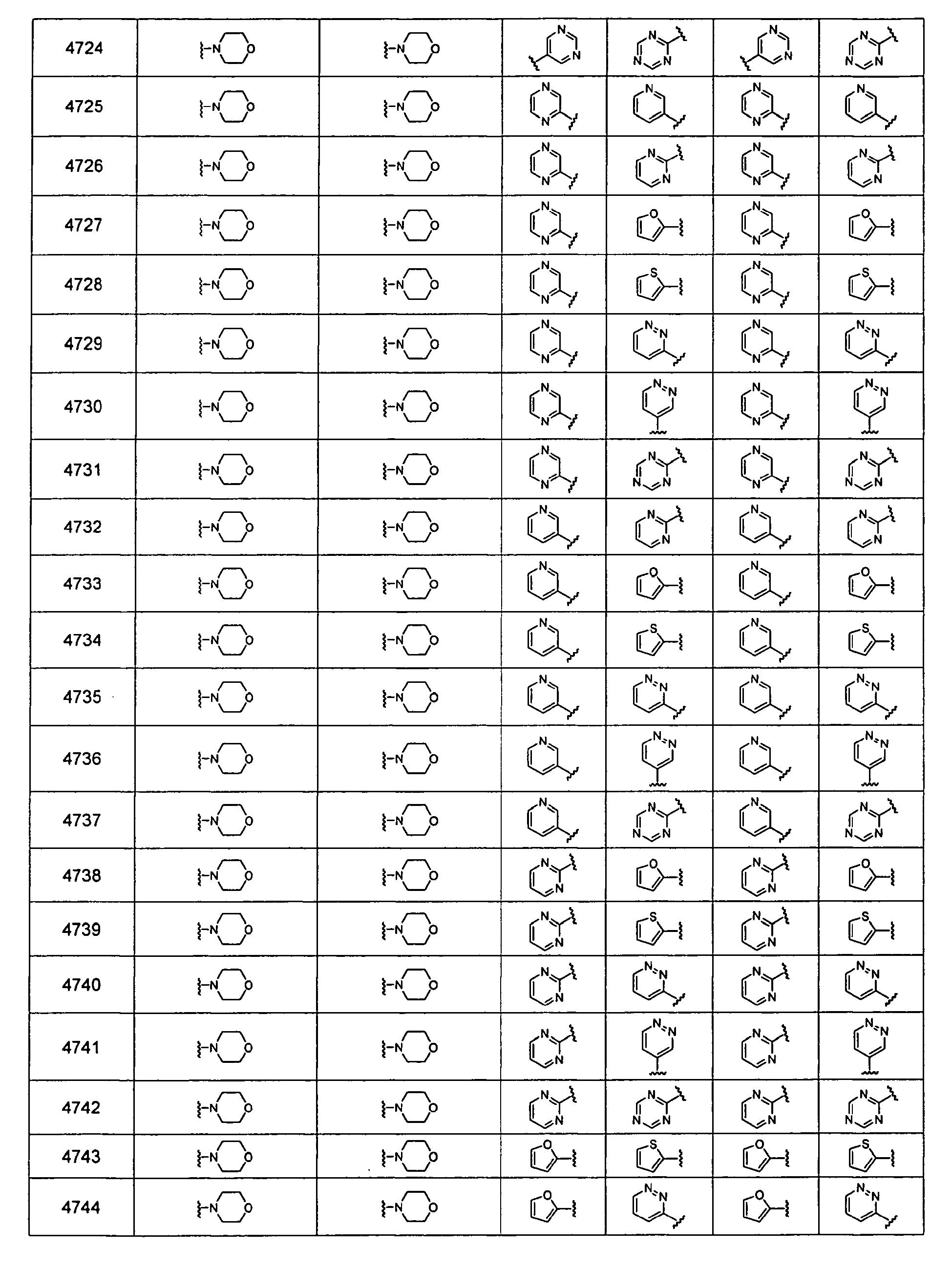

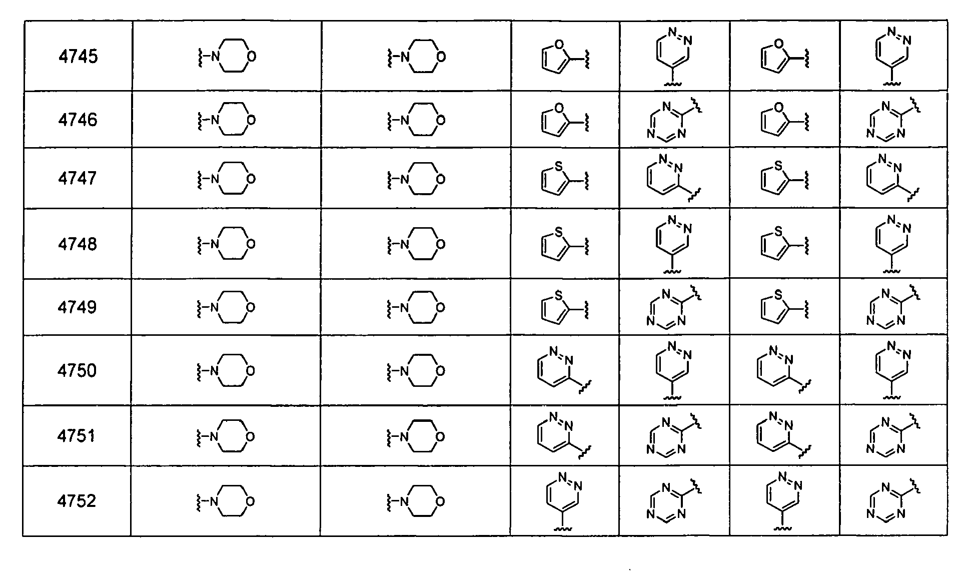

**Table 3**

| Compound | ¹H NMR(CDCl₃, 200 MHz) | MS/FAB | |
|---|---|---|---|
| | | found | calculated |
| **1** | δ= 2.64(6H, s), 6.62(4H, m), 6.7(4H, m), 6.81 (2H, m), 7.06(2H, m), 7.55(4H, m), 7.78(2H, m), 8.07(4H, m) | 544.7 | 544.2 |
| **42** | δ= 2.64(6H, s), 6.01(2H, m), 6.72(2H, m), 6.81(2H, m), 6.84(2H, m), 7.06(2H, m), 7.78(2H, m), 8.35(2H, m), 8.36(2H, m), 8.4(2H, m) | 556.7 | 556.2 |
| **67** | δ= 6.62(4H, m), 6.7(4H, m), 6.83(2H, m), 7.03(2H, m), 7.41(2H, m), 7.51(4H, m), 7.52(4H, m), 7.55(4H, m), 7.75(2H, m), 8.07(4H, m) | 668.8 | 668.3 |
| **108** | δ= 6.01(2H, m), 6.72(2H, m), 6.83(2H, m), 6.84(2H, m), 7.03(2H, m), 7.41 (2H, m), 7.51 (4H, m), 7.52(4H, m), 7.75(2H, m), 8.35(2H, m)8.36(2H, m), 8.4(2H, m) | 680.8 | 680.2 |
| **133** | δ= 1.48(18H, s), 6.62(4H, m), 6.7(4H, m), 6.81(2H, m), 7.06(2H, m), 7.55(4H, m), 7.78(2H, m), 8.07(4H, m) | 628.8 | 628.3 |
| **174** | δ= 1.48(18H, s), 6.01 (2H, m), 6.72(2H, m), 6.81(2H, m), 6.84(2H, m), 7.06(2H, m), 7.78(2H, m), 8.35(2H, m), 8.36(2H, m), 8.4(2H, m) | 640.9 | 640.2 |
| **199** | δ= 6.62(4H, m), 6.7(4H, m), 6.83(2H, m), 7.03(2H, m), 7.55(4H, m), 7.58(2H, m), 7.59(4H, m), 7.73(2H, m), 7.75(2H, m), 7.92(2H, m)8(4H, m), 8.07(4H, m) | 768.9 | 768.3 |
| **240** | δ= 6.01(2H, m), 6.72(2H, m), 6.83(2H, m), 6.84(2H, m), 7.03(2H, m), 7.58(2H, m), 7.59(4H, m), 7.73(2H, m), 7.75(2H, m), 7.92(2H, m), 8(4H, m), 8.35(2H, m), 8.36(2H, m), 8.4(2H, m) | 781.0 | 780.2 |
| **265** | δ= 6.62(4H, m), 6.7(4H, m), 6.83(2H, m), 7.03(2H, m), 7.55(8H, m), 7.61(2H, m), 7.75(2H, m), 8.04(2H, m), 8.07(4H, m), 8.08(2H, m)8.42(2H, m), 8.55(2H, m) | 768.9 | 768.3 |
| **306** | δ= 6.01(2H, m), 6.72(2H, m), 6.83(2H, m), 6.84(2H, m), 7.03(2H, m), 7.55(4H, m), 7.61(2H, m), 7.75(2H, m), 8.04(2H, m), 8.08(2H, m), 8.35(2H, m), 8.36(2H, m), 8.4(2H, m), 8.42(2H, m), 8.55(2H, m) | 781.0 | 780.2 |
| **331** | δ= 6.62(4H, m), 6.7(4H, m), 6.83(2H, m), 7.03(2H, m), 7.25(8H, m), 7.41(2H, m), 7.51(4H, m), 7.52(4H, m), 7.55(4H, m), 7.75(2H, m)8.07(4H, m) | 821.0 | 820.3 |
| **372** | δ= 6.01(2H, m), 6.72(2H, m), 6.83(2H, m), 6.84(2H, m), 7.03(2H, m), 7.25(8H, m), 7.41(2H, m), 7.51(4H, m), 7.52(4H, m), 7.75(2H, m)8.35(2H, m), 8.36(2H, m), 8.4(2H, m) | 833.0 | 832.2 |
| **397** | δ= 1.35(18H, s), 6.62(4H, m), 6.7(4H, m), 6.83(2H, m), 7.03(2H, m), 7.37(4H, m), 7.38(4H, m), 7.55(4H, m), 7.75(2H, m), 8.07(4H, m) | 781.0 | 780.4 |
| **438** | δ= 1.35(18H, s), 6.01(2H, m), 6.72(2H, m), 6.83(2H, m), 6.84(2H, m), 7.03(2H, m), 7.37(4H, m), 7.38(4H, m), 7.75(2H, m), 8.35(2H, m)8.36(2H, m), 8.4(2H, m) | 793.1 | 792.3 |
| **463** | δ= 2.34(6H, s), 6.62(4H, m), 6.7(4H, m), 6.83(2H, m), 7.03(2H, m), 7.29(4H, m), 7.33(4H, m), 7.55(4H, m), 7.75(2H, m), 8.07(4H, m) | 696.8 | 696.3 |
| **504** | δ= 2.34(6H, s), 6.01(2H, m), 6.72(2H, m), 6.83(2H, m), 6.84(2H, m), 7.03(2H, m), 7.29(4H, m), 7.33(4H, m), 7.75(2H, m), 8.35(2H, m)8.36(2H, m), 8.4(2H, m) | 708.9 | 708.2 |
| **529** | δ= 2.34(12H, s), 6.62(4H, m), 6.7(4H, m), 6.83(2H, m), 7.03(2H, m), 7.31(2H, m), 7.55(4H, m), 7.6(4H, m), 7.75(2H, m), 8.07(4H, m) | 724.9 | 724.3 |
| **570** | δ= 2.34(12H, s), 6.01(2H, m), 6.72(2H, m), 6.83(2H, m), 6.84(2H, m), 7.03(2H, m), 7.31(2H, m), 7.6(4H, m), 7.75(2H, m), 8.35(2H, m)8.36(2H, m), 8.4(2H, m) | 737.0 | 736.2 |
| **619** | δ= 1.72(12H, s), 6.83(2H, m), 6.93(2H, m), 6.99(4H, m), 7.03(2H, m), 7.28(2H, m), 7.38(2H, m), 7.55(2H, m), 7.63(2H, m), 7.75(2H, m), 7.77(2H, m), 7.87(2H, m), 7.93(2H, m), 8.45(4H, m), 8.46(4H, m) | 903.1 | 902.4 |
| **651** | δ= 1.72(12H, s), 6.01(2H, m), 6.68(2H, m), 6.72(2H, m), 6.83(2H, m), 6.84(2H, m), 7(2H, m), 7.03(2H, m), 7.28(2H, m), 7.38(2H, m), 7.55(2H, m), 7.63(2H, m), 7.75(2H, m), 7.77(2H, m), 7.87(2H, m), 7.88(2H, m)7.93(2H, m) | 889.1 | 888.3 |
| **661** | δ= 0.25(18H, s), 6.62(4H, m), 6.7(4H, m), 6.83(2H, m), 7.03(2H, m), 7.46(4H, m), 7.55(4H, m), 7.75(2H, m), 7.77(4H, m), 8.07(4H, m) | 813.2 | 812.4 |
| **685** | δ= 0.25(18H, m), 6.83(2H, m), 6.93(2H, m), 6.99(4H, m), 7.03(2H, m), 7.46(4H, m), 7.75(2H, m), 7.77(4H, m), 8.45(4H, m), 8.46(4H, m) | 815.1 | 814.3 |
| **768** | δ= 6.01(2H, m), 6.72(2H, m), 6.83(2H, m), 6.84(2H, m), 7.03(2H, m), 7.41(4H, m), 7.51(8H, m), 7.52(8H, m), 7.66(6H, m), 7.75(2H, m)8.35(2H, m), 8.36(2H, m), 8.4(2H, m) | 985.2 | 984.3 |
| **783** | δ= 6.01(2H, m), 6.68(2H, m), 6.72(2H, m), 6.83(2H, m), 6.84(2H, m), 7(2H, m), 7.03(2H, m), 7.41 (4H, m), 7.51(8H, m), 7.52(8H, m)7.66(6H, m), 7.75(2H, m), 7.88(2H, m) | 961.2 | 960.3 |
| **817** | δ= 6.83(4H, m), 6.93(2H, m), 6.99(4H, m), 7.03(2H, m), 7.3(4H, m), 7.39(4H, m), 7.75(2H, m), 8.45(4H, m), 8.46(4H, m) | 706.7 | 706.2 |
| **834** | 6= 6.01(2H, m), 6.72(2H, m), 6.83(2H, m), 6.84(2H, m), 7.03(2H, m), 7.3(4H, m), 7.39(4H, m), 7.75(2H, m), 8.35(2H, m), 8.36(2H, m)8.4(2H, m) | 716.8 | 716.2 |
| **859** | δ= 6.62(4H, m), 6.7(4H, m), 6.83(2H, m), 7.03(2H, m), 7.41(2H, m), 7.48(4H, m), 7.51 (4H, m), 7.52(4H, m), 7.55(4H, m), 7.57(2H, m)7.7(2H, m), 7.75(2H, m), 8.07(4H, m) | 821.0 | 820.3 |
| **915** | δ= 6.01(2H, m), 6.68(2H, m), 6.72(2H, m), 6.83(2H, m), 6.84(2H, m), 7(2H, m), 7.03(2H, m), 7.41(2H, m), 7.48(4H, m), 7.51 (4H, m), 7.52(4H, m), 7.57(2H, m), 7.7(2H, m), 7.75(2H, m), 7.88(2H, m) | 809.1 | 808.2 |
| **966** | δ= 6.01(2H, m), 6.72(2H, m), 6.83(2H, m), 6.84(2H, m), 7.03(2H, m), 7.41 (2H, m), 7.47(4H, m), 7.51 (4H, m), 7.75(2H, m), 7.79(4H, m), 7.85(4H, m), 8.35(2H, m), 8.36(2H, m), 8.4(2H, m) | 833.0 | 832.2 |
| **981** | δ= 6.01(2H, m), 6.68(2H, m), 6.72(2H, m), 6.83(2H, m), 6.84(2H, m), 7(2H, m), 7.03(2H, m), 7.41 (2H, m), 7.47(4H, m), 7.51 (4H, m), 7.75(2H, m), 7.79(4H, m), 7.85(4H, m), 7.88(2H, m) | 809.0 | 808.2 |
| **991** | δ= 6.62(4H, m), 6.7(4H, m), 6.83(2H, m), 6.95(4H, m), 7.03(2H, m), 7.3(2H, m), 7.45(4H, m), 7.55(4H, m), 7.72(4H, m), 7.75(2H, m)8.07(4H, m) | 720.9 | 720.3 |
| **1015** | δ= 6.83(4H, m), 6.93(2H, m), 6.95(4H, m), 6.99(4H, m), 7.03(2H, m), 7.3(2H, m), 7.45(4H, m), 7.72(4H, m), 7.75(2H, m), 8.45(4H, m)8.46(4H, m) | 722.8 | 722.3 |
| **1057** | δ= 1.48(12H, m), 1.75(8H, m), 2.72(2H, m), 6.62(4H, m), 6.7(4H, m), 6.81(2H, m), 7.06(2H, m), 7.55(4H, m), 7.78(2H, m), 8.07(4H, m) | 680.9 | 680.4 |
| **1081** | δ= 1.48(12H, m), 1.75(8H, m), 2.72(2H, m), 6.81(2H, m), 6.93(2H, m), 6.99(4H, m), 7.06(2H, m), 7.78(2H, m), 8.45(4H, m), 8.46(4H, m) | 682.9 | 682.4 |
| **1147** | δ= 6.83(2H, m), 6.93(2H, m), 6.99(4H, m), 7.03(2H, m), 7.75(2H, m), 7.82(4H, m), 7.88(4H, m), 7.93(2H, m), 8.12(4H, m), 8.45(4H, m)8.46(4H, m), 8.93(4H, m) | 871.0 | 870.3 |
| **1164** | δ= 6.01(2H, m), 6.72(2H, m), 6.83(2H, m), 6.84(2H, m), 7.03(2H, m), 7.75(2H, m), 7.82(4H, m), 7.88(4H, m), 7.93(2H, m), 8.12(4H, m), 8.35(2H, m), 8.36(2H, m), 8.4(2H, m), 8.93(4H, m) | 881.1 | 880.2 |
| **1189** | 6= 6.62(4H, m), 6.7(4H, m), 6.83(2H, m), 7.03(2H, m), 7.55(4H, m), 7.71 (8H, m), 7.75(2H, m), 7.82(2H, m), 7.88(2H, m), 8.04(2H, m)8.07(4H, m), 8.12(2H, m), 8.18(2H, m) | 917.1 | 916.3 |
| **1230** | δ= 6.01(2H, m), 6.72(2H, m), 6.83(2H, m), 6.84(2H, m), 7.03(2H, m), 7.71(8H, m), 7.75(2H, m), 7.82(2H, m), 7.88(2H, m), 8.04(2H, m), 8.12(2H, m), 8.18(2H, m), 8.35(2H, m), 8.36(2H, m), 8.4(2H, m) | 929.1 | 928.2 |
| **1255** | δ= 6.62(4H, m), 6.7(4H, m), 6.83(2H, m), 7.03(2H, m), 7.55(4H, m), 7.58(2H, m), 7.75(2H, m), 7.79(2H, m), 7.8(2H, m), 7.9(2H, m), 7.96(2H, m), 8.07(4H, m), 8.1(4H, m), 8.42(4H, m) | 917.1 | 916.3 |
| **1296** | δ= 6.01(2H, m), 6.72(2H, m), 6.83(2H, m), 6.84(2H, m), 7.03(2H, m), 7.58(2H, m), 7.75(2H, m), 7.79(2H, m), 7.8(2H, m), 7.9(2H, m), 7.96(2H, m), 8.1(4H, m), 8.35(2H, m), 8.36(2H, m), 8.4(2H, m), 8.42(4H, m) | 929.1 | 928.2 |
| **1321** | δ= 6.62(4H, m), 6.7(4H, m), 6.83(2H, m), 7.03(2H, m), 7.25(8H, m), 7.55(4H, m), 7.58(2H, m), 7.59(4H, m), 7.73(2H, m), 7.75(2H, m)7.92(2H, m), 8(4H, m), 8.07(4H, m) | 921.1 | 920.4 |
| **1345** | δ= 6.83(4H, m), 6.93(2H, m), 6.99(4H, m), 7.03(2H, m), 7.25(8H, m), 7.58(2H, m), 7.59(4H, m), 7.73(2H, m), 7.75(2H, m), 7.92(2H, m)8(4H, m), 8.45(4H, m), 8.46(4H, m) | 923.1 | 922.4 |
| **1411** | δ= 6.83(2H, m), 6.93(2H, m), 6.99(4H, m), 7.03(2H, m), 7.25(8H, m), 7.55(4H, m), 7.61(2H, m), 7.75(2H, m), 8.04(2H, m), 8.08(2H, m), 8.42(2H, m), 8.45(4H, m), 8.46(4H, m), 8.55(2H, m) | 923.1 | 922.4 |
| **1428** | δ= 6.01(2H, m), 6.72(2H, m), 6.83(2H, m), 6.84(2H, m), 7.03(2H, m), 7.25(8H, m), 7.55(4H, m), 7.61(2H, m), 7.75(2H, m), 8.04(2H, m), 8.08(2H, m), 8.35(2H, m), 8.36(2H, m), 8.4(2H, m), 8.42(2H, m), 8.55(2H, m) | 933.2 | 932.3 |
| **1453** | δ= 6.62(4H, m), 6.7(4H, m), 6.83(2H, m), 7.03(2H, m), 7.55(4H, m), 7.58(6H, m), 7.59(4H, m), 7.73(6H, m), 7.75(2H, m), 7.92(6H, m), 8(4H, m), 8.07(4H, m) | 1021.2 | 1020.4 |
| **1543** | δ= 1.72(12H, s), 6.83(2H, m), 6.93(2H, m), 6.99(4H, m), 7.03(2H, m), 7.28(2H, m), 7.38(2H, m), 7.55(2H, m), 7.58(4H, m), 7.63(2H, m), 7.73(4H, m), 7.75(2H, m), 7.77(2H, m), 7.87(2H, m), 7.92(4H, m), 7.93(2H, m)8.37(2H, m)8.46(4H, m)8.54(2H, m) | 1155.4 | 1154.5 |
| **1585** | δ= 1.71(12H, m), 2.01(12H, m), 2.02(6H, m), 6.62(4H, m), 6.7(4H, m), 6.81(2H, m), 7.06(2H, m), 7.55(4H, m), 7.78(2H, m), 8.07(4H, m) | 785.0 | 784.4 |
| **1651** | δ= 0.88(6H, m), 1.22(4H, m), 1.29(8H, m), 1.31(4H, m), 1.44(12H, m), 1.78(12H, m), 6.62(4H, m), 6.7(4H, m), 6.81 (2H, m), 7.06(2H, m), 7.55(4H, m), 7.78(2H, m), 8.07(4H, m) | 873.2 | 872.6 |
| **1675** | δ= 0.88(6H, m), 1.22(4H, m), 1.29(8H, m), 1.31(4H, m), 1.44(12H, m), 1.78(12H, m), 6.81(2H, m), 6.93(2H, m), 6.99(4H, m), 7.06(2H, m), 7.78(2H, m), 8.45(4H, m)8.46(4H, m) | 875.2 | 874.5 |
| **1717** | δ= 6.62(4H, m), 6.7(4H, m), 6.83(2H, m), 7.03(2H, m), 7.38(2H, m), 7.41(4H, m), 7.55(4H, m), 7.59(4H, m), 7.75(2H, m), 8.07(4H, m) | 716.8 | 716.3 |
| **1758** | δ= 6.01(2H, m), 6.72(2H, m), 6.83(2H, m), 6.84(2H, m), 7.03(2H, m), 7.38(2H, m), 7.41 (4H, m), 7.59(4H, m), 7.75(2H, m), 8.35(2H, m), 8.36(2H, m), 8.4(2H, m) | 728.9 | 728.2 |
| **1807** | δ= 6.83(2H, m), 6.93(2H, m), 6.99(4H, m), 7(2H, m), 7.03(2H, m), 7.26(2H, m), 7.51(2H, m), 7.75(2H, m), 8.45(4H, m), 8.46(4H, m), 8.5(2H, m) | 672.7 | 672.3 |
| **1824** | δ= 6.01(2H, m), 6.72(2H, m), 6.83(2H, m), 6.84(2H, m), 7(2H, m), 7.03(2H, m), 7.26(2H, m), 7.51(2H, m), 7.75(2H, m), 8.35(2H, m), 8.36(2H, m), 8.4(2H, m), 8.5(2H, m) | 682.8 | 682.2 |
| **1849** | δ= 6.62(4H, m), 6.7(4H, m), 6.83(2H, m), 7.03(2H, m), 7.35(2H, m), 7.55(4H, m), 7.6(2H, m), 7.75(2H, m), 7.78(2H, m), 7.98(2H, m), 8.06(2H, m), 8.07(4H, m), 8.1(2H, m) | 770.9 | 770.3 |
| **1890** | δ= 6.01(2H, m), 6.72(2H, m), 6.83(2H, m), 6.84(2H, m), 7.03(2H, m), 7.35(2H, m), 7.6(2H, m), 7.75(2H, m), 7.78(2H, m), 7.98(2H, m), 8.06(2H, m), 8.1 (2H, m), 8.35(2H, m), 8.36(2H, m), 8.4(2H, m) | 782.9 | 782.2 |
| **1939** | δ= 3.22(4H, m), 6.83(2H, m), 6.93(2H, m), 6.94(2H, m), 6.99(4H, m), 7.03(2H, m), 7.18(2H, m), 7.26(2H, m), 7.38(4H, m), 7.75(2H, m), 8.45(4H, m), 8.46(4H, m) | 746.9 | 746.3 |
| **1959** | δ= 3.22(4H, m), 6.01(2H, m), 6.72(2H, m), 6.83(2H, m), 6.84(2H, m), 6.94(2H, m), 7.03(2H, m), 7.18(2H, m), 7.26(2H, m), 7.38(4H, m), 7.75(2H, m), 8.35(2H, m), 8.36(2H, m), 8.4(2H, m) | 756.9 | 756.2 |
| **1981** | δ= 6.62(4H, m), 6.7(4H, m), 6.83(2H, m), 7.03(2H, m), 7.53(4H, m), 7.55(4H, m), 7.75(2H, m), 8.01(2H, m), 8.07(4H, m), 8.18(2H, m) | 782.9 | 782.2 |
| **2005** | δ= 6.83(2H, m), 6.93(2H, m), 6.99(4H, m), 7.03(2H, m), 7.53(4H, m), 7.75(2H, m), 8.01(2H, m), 8.18(2H, m), 8.45(4H, m), 8.46(4H, m) | 784.9 | 784.2 |
| **2071** | δ= 6.83(2H, m), 6.93(2H, m), 6.99(4H, m), 7.03(2H, m), 7.11(12H, m), 7.26(6H, m), 7.29(4H, m), 7.33(16H, m), 7.75(2H, m), 8.45(4H, m), 8.46(4H, m) | 1155.4 | 1154.5 |
| **2088** | δ= 6.01(2H, m), 6.72(2H, m), 6.83(2H, m), 6.84(2H, m), 7.03(2H, m), 7.11(12H, m), 7.26(6H, m), 7.29(4H, m), 7.33(16H, m), 7.75(2H, m), 8.35(2H, m), 8.36(2H, m), 8.4(2H, m) | 1165.5 | 1164.4 |
| **2113** | δ= 3.83(6H, m), 6.62(4H, m), 6.7(4H, m), 6.83(2H, m), 7.03(2H, m), 7.05(4H, m), 7.55(4H, m), 7.68(4H, m), 7.75(2H, m), 8.07(4H, m) | 728.8 | 728.3 |
| **2137** | δ= 3.83(6H, m), 6.83(2H, m), 6.93(2H, m), 6.99(4H, m), 7.03(2H, m), 7.05(4H, m), 7.68(4H, m), 7.75(2H, m), 8.45(4H, m), 8.46(4H, m) | 730.8 | 730.3 |
| **2203** | δ= 6.83(2H, m), 6.93(2H, m), 6.99(4H, m), 7.03(2H, m), 7.11 (8H, m), 7.26(4H, m), 7.28(2H, m), 7.33(8H, m), 7.38(2H, m), 7.55(2H, m), 7.63(2H, m), 7.75(2H, m), 7.77(2H, m), 7.87(2H, m), 7.93(2H, m), 8.45(4H, m), 8.46(4H, m) | 1151.4 | 1150.5 |
| **2220** | δ= 6.01(2H, m), 6.72(2H, m), 6.83(2H, m), 6.84(2H, m), 7.03(2H, m), 7.11(8H, m), 7.26(4H, m), 7.28(2H, m), 7.33(8H, m), 7.38(2H, m), 7.55(2H, m), 7.63(2H, m), 7.75(2H, m), 7.77(2H, m), 7.87(2H, m), 7.93(2H, m), 8.35(2H, m), 8.36(2H, m), 8.4(2H, m) | 1161.4 | 1160.4 |
| **2311** | δ= 3.18(8H, m), 3.65(8H, m), 6.62(4H, m), 6.7(4H, m), 6.81 (2H, m), 6.99(2H, m), 7.55(4H, m), 7.71(2H, m), 8.07(4H, m) | 686.8 | 686.3 |
| **2335** | δ= 3.18(8H, m), 3.65(8H, m), 6.81(2H, m), 6.93(2H, m), 6.99(6H, m), 7.71 (2H, m), 8.45(4H, m), 8.46(4H, m) | 688.8 | 688.3 |
| **2378** | δ= 2.64(6H, s), 6.81(2H, m), 6.99(8H, m), 7.06(2H, m), 7.78(2H, m), 8.46(8H, m) | 544.7 | 544.2 |
| **2402** | δ= 2.64(6H, s), 6.68(2H, m), 6.81(2H, m), 6.99(4H, m), 7(2H, m), 7.06(2H, m), 7.78(2H, m), 7.88(2H, m), 8.46(4H, m) | 522.6 | 522.2 |
| **2468** | δ= 6.68(2H, m), 6.83(2H, m), 6.99(4H, m), 7(2H, m), 7.03(2H, m), 7.41(2H, m), 7.51(4H, m), 7.52(4H, m), 7.75(2H, m), 7.88(2H, m), 8.46(4H, m) | 646.7 | 646.2 |
| **2485** | δ= 6.83(2H, m), 7.03(2H, m), 7.19(2H, m), 7.24(2H, m), 7.41(2H, m), 7.51 (4H, m), 7.52(4H, m), 7.75(2H, m), 8.35(2H, m), 8.36(2H, m), 8.4(2H, m), 8.6(2H, m) | 672.7 | 672.3 |
| **2534** | δ= 1.48(18H, s), 6.68(2H, m), 6.81 (2H, m), 6.99(4H, m), 7(2H, m), 7.06(2H, m), 7.78(2H, m), 7.88(2H, m), 8.46(4H, m) | 606.8 | 606.3 |
| **2566** | δ= 1.48(18H, s), 6.68(2H, m), 6.81(2H, m), 7(2H, m), 7.06(2H, m), 7.19(2H, m), 7.24(2H, m), 7.78(2H, m), 7.88(2H, m), 8.6(2H, m) | 608.7 | 608.3 |
| **2576** | δ= 6.83(2H, m), 6.99(8H, m), 7.03(2H, m), 7.58(2H, m), 7.59(4H, m), 7.73(2H, m), 7.75(2H, m), 7.92(2H, m), 8(4H, m), 8.46(8H, m) | 768.9 | 768.3 |
| **2632** | δ= 6.68(2H, m), 6.83(2H, m), 7(2H, m), 7.03(2H, m), 7.19(2H, m), 7.24(2H, m), 7.58(2H, m), 7.59(4H, m), 7.73(2H, m), 7.75(2H, m), 7.88(2H, m), 7.92(2H, m), 8(4H, m), 8.6(2H, m) | 748.8 | 748.3 |
| **2642** | δ= 6.83(2H, m), 6.99(8H, m), 7.03(2H, m), 7.55(4H, m), 7.61(2H, m), 7.75(2H, m), 8.04(2H, m), 8.08(2H, m), 8.42(2H, m), 8.46(8H, m), 8.55(2H, m) | 768.9 | 768.3 |
| **2666** | δ= 6.68(2H, m), 6.83(2H, m), 6.99(4H, m), 7(2H, m), 7.03(2H, m), 7.55(4H, m), 7.61(2H, m), 7.75(2H, m), 7.88(2H, m), 8.04(2H, m), 8.08(2H, m), 8.42(2H, m), 8.46(4H, m), 8.55(2H, m) | 746.9 | 746.3 |
| **2749** | δ= 6.83(2H, m), 7.03(2H, m), 7.19(2H, m), 7.24(2H, m), 7.25(8H, m), 7.41(2H, m), 7.51(4H, m), 7.52(4H, m), 7.75(2H, m), 8.35(2H, m), 8.36(2H, m), 8.4(2H, m), 8.6(2H, m) | 824.9 | 824.3 |
| **2764** | δ= 6.68(2H, m), 6.83(2H, m), 7(2H, m), 7.03(2H, m), 7.19(2H, m), 7.24(2H, m), 7.25(8H, m), 7.41(2H, m), 7.51(4H, m), 7.52(4H, m), 7.75(2H, m), 7.88(2H, m), 8.6(2H, m) | 800.9 | 800.3 |
| **2774** | δ= 1.35(18H, s), 6.83(2H, m), 6.99(8H, m), 7.03(2H, m), 7.37(4H, m), 7.38(4H, m), 7.75(2H, m), 8.46(8H, m) | 781.0 | 780.4 |
| **2830** | δ= 1.35(18H, s), 6.68(2H, m), 6.83(2H, m), 7(2H, m), 7.03(2H, m), 7.19(2H, m), 7.24(2H, m), 7.37(4H, m), 7.38(4H, m), 7.75(2H, m), 7.88(2H, m), 8.6(2H, m) | 760.9 | 760.4 |
| **2864** | δ= 2.34(6H, s), 6.68(2H, m), 6.83(2H, m), 6.99(4H, m), 7(2H, m), 7.03(2H, m), 7.29(4H, m), 7.33(4H, m), 7.75(2H, m), 7.88(2H, m), 8.46(4H, m) | 674.8 | 674.3 |
| **2881** | δ= 2.34(6H, s), 6.83(2H, m), 7.03(2H, m), 7.19(2H, m), 7.24(2H, m), 7.29(4H, m), 7.33(4H, m), 7.75(2H, m), 8.35(2H, m), 8.36(2H, m), 8.4(2H, m), 8.6(2H, m) | 700.8 | 700.3 |
| **2906** | δ= 2.34(12H, s), 6.83(2H, m), 6.99(8H, m), 7.03(2H, m), 7.31(2H, m), 7.6(4H, m), 7.75(2H, m), 8.46(8H, m) | 724.9 | 724.3 |
| **2930** | δ= 2.34(12H, s), 6.68(2H, m), 6.83(2H, m), 6.99(4H, m), 7(2H, m), 7.03(2H, m), 7.31(2H, m), 7.6(4H, m), 7.75(2H, m), 7.88(2H, m), 8.46(4H, m) | 702.8 | 702.3 |
| **2996** | δ= 1.72(12H, s), 6.68(2H, m), 6.83(2H, m), 6.99(4H, m), 7(2H, m), 7.03(2H, m), 7.28(2H, m), 7.38(2H, m), 7.55(2H, m), 7.63(2H, m), 7.75(2H, m), 7.77(2H, m), 7.87(2H, m), 7.88(2H, m), 7.93(2H, m), 8.46(4H, m) | 879.1 | 878.4 |
| **3038** | δ= 0.25(18H, s), 6.83(2H, m), 6.99(8H, m), 7.03(2H, m), 7.46(4H, m), 7.75(2H, m), 7.77(4H, m), 8.46(8H, m) | 813.2 | 812.4 |
| **3079** | δ= 0.25(18H, s), 6.83(2H, m), 7.03(2H, m), 7.19(2H, m), 7.24(2H, m), 7.46(4H, m), 7.75(2H, m), 7.77(4H, m), 8.35(2H, m), 8.36(2H, m), 8.4(2H, m), 8.6(2H, m) | 817.1 | 816.3 |
| **3145** | δ= 6.83(2H, m), 7.03(2H, m), 7.19(2H, m), 7.24(2H, m), 7.41(4H, m), 7.51 (8H, m), 7.52(8H, m), 7.66(6H, m), 7.75(2H, m), 8.35(2H, m), 8.36(2H, m), 8.4(2H, m), 8.6(2H, m) | 977.1 | 976.4 |
| **3160** | δ= 6.68(2H, m), 6.83(2H, m), 7(2H, m), 7.03(2H, m), 7.19(2H, m), 7.24(2H, m), 7.41(4H, m), 7.51(8H, m), 7.52(8H, m), 7.66(6H, m), 7.75(2H, m), 7.88(2H, m), 8.6(2H, m) | 953.1 | 952.4 |
| **3170** | δ= 6.83(2H, m), 6.99(8H, m), 7.03(2H, m), 7.3(4H, m), 7.39(4H, m), 7.75(2H, m), 8.46(8H, m) | 704.8 | 704.3 |
| **3194** | δ= 6.68(2H, m), 6.83(2H, m), 6.99(4H, m), 7(2H, m), 7.03(2H, m), 7.3(4H, m), 7.39(4H, m), 7.75(2H, m), 7.88(2H, m), 8.46(4H, m) | 682.7 | 682.2 |
| **3260** | δ= 6.68(2H, m), 6.83(2H, m), 6.99(4H, m), 7(2H, m), 7.03(2H, m), 7.41(2H, m), 7.48(4H, m), 7.51(4H, m), 7.52(4H, m), 7.57(2H, m), 7.7(2H, m), 7.75(2H, m), 7.88(2H, m), 8.46(4H, m) | 798.9 | 798.3 |
| **3277** | δ= 6.83(2H, m), 7.03(2H, m), 7.19(2H, m), 7.24(2H, m), 7.41(2H, m), 7.48(4H, m), 7.51 (4H, m), 7.52(4H, m), 7.57(2H, m), 7.7(2H, m), 7.75(2H, m), 8.35(2H, m), 8.36(2H, m), 8.4(2H, m), 8.6(2H, m) | 824.9 | 824.3 |
| **3302** | δ= 6.83(2H, m), 6.99(8H, m), 7.03(2H, m), 7.41(2H, m), 7.47(4H, m), 7.51(4H, m), 7.75(2H, m), 7.79(4H, m), 7.85(4H, m), 8.46(8H, m) | 821.0 | 820.3 |
| **3358** | δ= 6.68(2H, m), 6.83(2H, m), 7(2H, m), 7.03(2H, m), 7.19(2H, m), 7.24(2H, m), 7.41(2H, m), 7.47(4H, m), 7.51(4H, m), 7.75(2H, m), 7.79(4H, m), 7.85(4H, m), 7.88(2H, m), 8.6(2H, m) | 800.9 | 800.3 |
| **3392** | δ= 6.68(2H, m), 6.83(2H, m), 6.95(4H, m), 6.99(4H, m), 7(2H, m), 7.03(2H, m), 7.3(2H, m), 7.45(4H, m), 7.72(4H, m), 7.75(2H, m), 7.88(2H, m), 8.46(4H, m) | 698.8 | 698.3 |
| **3409** | δ= 6.83(2H, m), 6.95(4H, m), 7.03(2H, m), 7.19(2H, m), 7.24(2H, m), 7.3(2H, m), 7.45(4H, m), 7.72(4H, m), 7.75(2H, m), 8.35(2H, m), 8.36(2H, m), 8.4(2H, m), 8.6(2H, m) | 724.8 | 724.3 |
| **3458** | δ= 1.48(12H, m), 1.75(8H, m), 2.72(2H, m), 6.68(2H, m), 6.81(2H, m), 6.99(4H, m), 7(2H, m), 7.06(2H, m), 7.78(2H, m), 7.88(2H, m), 8.46(4H, m) | 658.8 | 658.3 |
| **3490** | δ= 1.48(12H, m), 1.75(8H, m), 2.72(2H, m), 6.68(2H, m), 6.81 (2H, m), 7(2H, m), 7.06(2H, m), 7.19(2H, m), 7.24(2H, m), 7.78(2H, m), 7.88(2H, m), 8.6(2H, m) | 660.8 | 660.3 |
| **3500** | 6= 6.83(2H, m), 6.99(8H, m), 7.03(2H, m), 7.75(2H, m), 7.82(4H, m), 7.88(4H, m), 7.93(2H, m), 8.12(4H, m), 8.46(8H, m), 8.93(4H, m) | 869.0 | 868.3 |
| **3556** | δ= 6.68(2H, m), 6.83(2H, m), 7(2H, m), 7.03(2H, m), 7.19(2H, m), 7.24(2H, m), 7.75(2H, m), 7.82(4H, m), 7.88(6H, m), 7.93(2H, m), 8.12(4H, m), 8.6(2H, m), 8.93(4H, m) | 849.0 | 848.3 |
| **3590** | δ= 6.68(2H, m), 6.83(2H, m), 6.99(4H, m), 7(2H, m), 7.03(2H, m), 7.71(8H, m), 7.75(2H, m), 7.82(2H, m), 7.88(4H, m), 8.04(2H, m), 8.12(2H, m), 8.18(2H, m), 8.46(4H, m) | 895.0 | 894.3 |
| **3607** | δ= 6,83(2H, m), 7.03(2H, m), 7.19(2H, m), 7.24(2H, m), 7.71(8H, m), 7.75(2H, m), 7.82(2H, m), 7.88(2H, m), 8.04(2H, m), 8.12(2H, m), 8.18(2H, m), 8.35(2H, m), 8.36(2H, m), 8.4(2H, m), 8.6(2H, m) | 921.0 | 920.3 |
| **3673** | δ= 6.83(2H, m), 7.03(2H, m), 7.19(2H, m), 7.24(2H, m), 7.58(2H, m), 7.75(2H, m), 7.79(2H, m), 7.8(2H, m), 7.9(2H, m), 7.96(2H, m), 8.1(4H, m), 8.35(2H, m), 8.36(2H, m), 8.4(2H, m), 8.42(4H, m), 8.6(2H, m) | 921.0 | 920.3 |
| **3688** | δ= 6.68(2H, m), 6.83(2H, m), 7(2H, m), 7.03(2H, m), 7.19(2H, m), 7.24(2H, m), 7.58(2H, m), 7.75(2H, m), 7.79(2H, m), 7.8(2H, m), 7.88(2H, m), 7.9(2H, m), 7.96(2H, m), 8.1(4H, m), 8.42(4H, m), 8.6(2H, m) | 897.0 | 896.3 |
| **3698** | δ= 6.83(2H, m), 6.99(8H, m), 7.03(2H, m), 7.25(8H, m), 7.58(2H, m), 7.59(4H, m), 7.73(2H, m), 7.75(2H, m), 7.92(2H, m), 8(4H, m), 8.46(8H, m) | 921.1 | 920.4 |
| **3739** | δ= 6.83(2H, m), 7.03(2H, m), 7.19(2H, m), 7.24(2H, m), 7.25(8H, m), 7.58(2H, m), 7.59(4H, m), 7.73(2H, m), 7.75(2H, m), 7.92(2H, m), 8(4H, m), 8.35(2H, m), 8.36(2H, m), 8.4(2H, m), 8.6(2H, m) | 925.1 | 924.3 |
| **3764** | δ= 6.83(2H, m), 6.99(8H, m), 7.03(2H, m), 7.25(8H, m), 7.55(4H, m), 7.61(2H, m), 7.75(2H, m), 8.04(2H, m), 8.08(2H, m), 8.42(2H, m), 8.46(8H, m), 8.55(2H, m) | 921.1 | 920.4 |
| **3788** | δ= 6.68(2H, m), 6.83(2H, m), 6.99(4H, m), 7(2H, m), 7.03(2H, m), 7.25(8H, m), 7.55(4H, m), 7.61(2H, m), 7.75(2H, m), 7.88(2H, m), 8.04(2H, m), 8.08(2H, m), 8.42(2H, m), 8.46(4H, m), 8.55(2H, m) | 899.0 | 898.3 |
| **3871** | δ= 6.83(2H, m), 7.03(2H, m), 7.19(2H, m), 7.24(2H, m), 7.58(6H, m), 7.59(4H, m), 7.73(6H, m), 7.75(2H, m), 7.92(6H, m), 8(4H, m), 8.35(2H, m), 8.36(2H, m), 8.4(2H, m), 8.6(2H, m) | 1025.2 | 1024.4 |
| **3886** | δ= 6.68(2H, m), 6.83(2H, m), 7(2H, m), 7.03(2H, m), 7.19(2H, m), 7.24(2H, m), 7.58(6H, m), 7.59(4H, m), 7.73(6H, m), 7.75(2H, m), 7.88(2H, m), 7.92(6H, m), 8(4H, m), 8.6(2H, m) | 1001.1 | 1000.4 |
| **3896** | δ= 1.72(12H, s), 6.83(2H, m), 6.99(8H, m), 7.03(2H, m), 7.28(2H, m), 7.38(2H, m), 7.55(2H, m), 7.58(4H, m), 7.63(2H, m), 7.73(4H, m), 7.75(2H, m), 7.77(2H, m), 7.87(2H, m), 7.92(4H, m), 7.93(2H, m), 8.46(8H, m) | 1153.4 | 1152.5 |
| **3937** | δ= 1.72(12H, s), 6.83(2H, m), 7.03(2H, m), 7.19(2H, m), 7.24(2H, m), 7.28(2H, m), 7.38(2H, m), 7.55(2H, m), 7.58(4H, m), 7.63(2H, m), 7.73(4H, m), 7.75(2H, m), 7.77(2H, m), 7.87(2H, m), 7.92(4H, m), 7.93(2H, m), 8.35(2H, m), 8.36(2H, m), 8.4(2H, m), 8.6(2H, m) | 1157.4 | 1156.5 |
| **3986** | δ= 1.71(12H, m), 2.01(12H, m), 2.02(6H, m), 6.68(2H, m), 6.81(2H, m), 6.99(4H, m), 7(2H, m), 7.06(2H, m), 7.78(2H, m), 7.88(2H, m), 8.46(4H, m) | 763.0 | 762.4 |
| **4018** | δ= 1.71(12H, m), 2.01(12H, m), 2.02(6H, m), 6.68(2H, m), 6.81(2H, m), 7(2H, m), 7.06(2H, m), 7.19(2H, m), 7.24(2H, m), 7.78(2H, m), 7.88(2H, m), 8.6(2H, m) | 765.0 | 764.4 |
| **4028** | δ= 0.88(6H, m), 1.22(4H, m), 1.29(8H, m), 1.31(4H, m), 1.44(12H, m), 1.78(12H, m), 6.81(2H, m), 6.99(8H, m), 7.06(2H, m), 7.78(2H, m), 8.46(8H, m) | 873.2 | 872.6 |
| **4069** | δ= 0.88(6H, m), 1.22(4H, m), 1.29(8H, m), 1.31(4H, m), 1.44(12H, m), 1.78(12H, m), 6.81(2H, m), 7.06(2H, m), 7.19(2H, m), 7.24(2H, m), 7.78(2H, m), 8.35(2H, m), 8.36(2H, m), 8.4(2H, m), 8.6(2H, m) | 877.2 | 876.5 |
| **4094** | δ= 6.83(2H, m), 6.99(8H, m), 7.03(2H, m), 7.38(2H, m), 7.41 (4H, m), 7.59(4H, m), 7.75(2H, m), 8.46(8H, m) | 716.8 | 716.3 |
| **4118** | δ= 6.68(2H, m), 6.83(2H, m), 6.99(4H, m), 7(2H, m), 7.03(2H, m), 7.38(2H, m), 7.41(4H, m), 7.59(4H, m), 7.75(2H, m), 7.88(2H, m), 8.46(4H, m) | 694.8 | 694.2 |
| **4201** | δ= 6.83(2H, m), 7(2H, m), 7.03(2H, m), 7.19(2H, m), 7.24(2H, m), 7.26(2H, m), 7.51(2H, m), 7.75(2H, m), 8.35(2H, m), 8.36(2H, m), 8.4(2H, m), 8.5(2H, m), 8.6(2H, m) | 674.7 | 674.2 |
| **4216** | δ= 6.68(2H, m), 6.83(2H, m), 7(4H, m), 7.03(2H, m), 7.19(2H, m), 7.24(2H, m), 7.26(2H, m), 7.51(2H, m), 7.75(2H, m), 7.88(2H, m), 8.5(2H, m), 8.6(2H, m) | 650.7 | 650.2 |
| **4226** | δ= 6.83(2H, m), 6.99(8H, m), 7.03(2H, m), 7.35(2H, m), 7.6(2H, m), 7.75(2H, m), 7.78(2H, m), 7.98(2H, m), 8.06(2H, m), 8.1(2H, m), 8.46(8H, m) | 770.9 | 770.3 |
| **4282** | δ= 6.68(2H, m), 6.83(2H, m), 7(2H, m), 7.03(2H, m), 7.19(2H, m), 7.24(2H, m), 7.35(2H, m), 7.6(2H, m), 7.75(2H, m), 7.78(2H, m), 7.88(2H, m), 7.98(2H, m), 8.06(2H, m), 8.1(2H, m), 8.6(2H, m) | 750.8 | 750.3 |
| **4316** | δ= 3.22(4H, s), 6.68(2H, m), 6.83(2H, m), 6.94(2H, m), 6.99(4H, m), 7(2H, m), 7.03(2H, m), 7.18(2H, m), 7.26(2H, m), 7.38(4H, m), 7.75(2H, m), 7.88(2H, m), 8.46(4H, m) | 722.8 | 722.3 |
| **4333** | δ= 3.22(4H, s), 6.83(2H, m), 6.94(2H, m), 7.03(2H, m), 7.18(2H, m), 7.19(2H, m), 7.24(2H, m), 7.26(2H, m), 7.38(4H, m), 7.75(2H, m), 8.35(2H, m), 8.36(2H, m), 8.4(2H, m), 8.6(2H, m) | 748.8 | 748.3 |
| **4358** | δ= 6.83(2H, m), 6.99(8H, m), 7.03(2H, m), 7.53(4H, m), 7.75(2H, m), 8.01(2H, m), 8.18(2H, m), 8.46(8H, m) | 782.9 | 782.2 |
| **4414** | δ= 6.68(2H, m), 6.83(2H, m), 7(2H, m), 7.03(2H, m), 7.19(2H, m), 7.24(2H, m), 7.53(4H, m), 7.75(2H, m), 7.88(2H, m), 8.01(2H, m), 8.18(2H, m), 8.6(2H, m) | 762.9 | 762.2 |
| **4448** | δ= 6.68(2H, m), 6.83(2H, m), 6.99(4H, m), 7(2H, m), 7.03(2H, m), 7.11(12H, m), 7.26(6H, m), 7.29(4H, m), 7.33(16H, m), 7.75(2H, m), 7.88(2H, m), 8.46(4H, m) | 1131.4 | 1130.5 |
| **4465** | δ= 6.83(2H, m), 7.03(2H, m), 7.11(12H, m), 7.19(2H, m), 7.24(2H, m), 7.26(6H, m), 7.29(4H, m), 7.33(16H, m), 7.75(2H, m), 8.35(2H, m), 8.36(2H, m), 8.4(2H, m), 8.6(2H, m) | 1157.4 | 1156.5 |
| **4490** | δ= 3.83(6H, s), 6.83(2H, m), 6.99(8H, m), 7.03(2H, m), 7.05(4H, m), 7.68(4H, m), 7.75(2H, m), 8.46(8H, m) | 728.8 | 728.3 |
| **4514** | δ= 3.83(6H, s), 6.68(2H, m), 6.83(2H, m), 6.99(4H, m), 7(2H, m), 7.03(2H, m), 7.05(4H, m), 7.68(4H, m), 7.75(2H, m), 7.88(2H, m), 8.46(4H, m) | 706.8 | 706.3 |
| **4580** | δ= 6.68(2H, m), 6.83(2H, m), 6.99(4H, m), 7(2H, m), 7.03(2H, m), 7.11(8H, m), 7.26(4H, m), 7.28(2H, m), 7.33(8H, m), 7.38(2H, m), 7.55(2H, m), 7.63(2H, m), 7.75(2H, m), 7.77(2H, m), 7.87(2H, m), 7.88(2H, m), 7.93(2H, m), 8.46(4H, m) | 1127.3 | 1126.4 |
| **4612** | δ= 6.68(2H, m), 6.83(2H, m), 7(2H, m), 7.03(2H, m), 7.11(8H, m), 7.19(2H, m), 7.24(2H, m), 7.26(4H, m), 7.28(2H, m), 7.33(8H, m), 7.38(2H, m), 7.55(2H, m), 7.63(2H, m), 7.75(2H, m), 7.77(2H, m), 7.87(2H, m), 7.88(2H, m), 7.93(2H, m), 8.6(2H, m) | 1129.3 | 1128.4 |
| **4712** | δ= 3.18(8H, m), 3.65(8H, m), 6.68(2H, m), 6.81(2H, m), 6.99(6H, m), 7(2H, m), 7.71 (2H, m), 7.88(2H, m), 8.46(4H, m) | 664.8 | 664.3 |
| **4729** | δ= 3.18(8H, m), 3.65(8H, m), 6.81(2H, m), 6.99(2H, m), 7.19(2H, m), 7.24(2H, m), 7.71(2H, m), 8.35(2H, m), 8.36(2H, m), 8.4(2H, m), 8.6(2H, m) | 690.8 | 690.3 |

### [Example 1] Manufacture of OLED's by using the organic electroluminescent compounds of the invention

An OLED device was manufactured by using the electroluminescent compound according to the invention.

First, a transparent electrode ITO thin film (15 Ω/□) (2) prepared from glass for OLED (1) (manufactured by Samsung-Corning) was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) (of which the structure is shown below) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injection layer (3) having 60 nm of thickness on the ITO substrate.

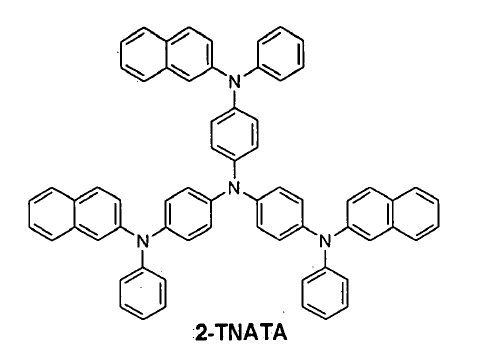

Then, to another cell of the vacuum vapor-deposit device, charged was N,N'-bis(α-naphthyl)-N,N'-diphenyl-4,4'-diamine (NPB) (of which the structure is shown below), and electric current was applied to the cell to evaporate NPB, thereby providing vapor-deposit of a hole transport layer (4) of 20 nm of thickness on the hole injection layer.

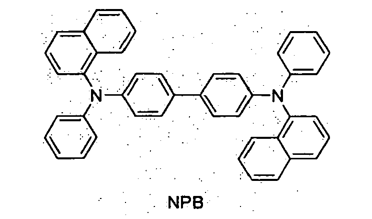

After forming the hole injection layer and hole transport layer, an electroluminescent layer was vapor-deposited as follows. To one cell of a vacuum vapor-deposit device, charged was H-62 (of which the structure is shown below) as a host, and a compound according to the invention (Compound (2717)) was charged to another cell as a dopant. Two substances were evaporated at different rates to give doping at 2 to 5% by weight on the basis of the host to vapor-deposit an electroluminescent layer (5) with a thickness of 30 nm on the hole transport layer.

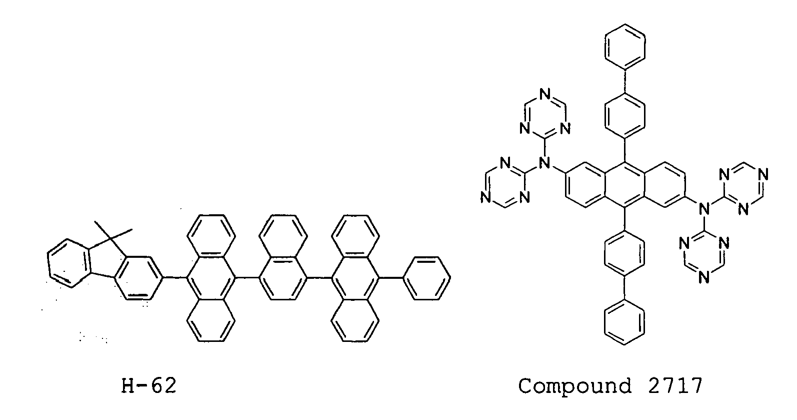

Then, tris(8-hydroxyquinoline)aluminum (III) (Alq) (of which the structure is shown below) was vapor-deposited as an electron transport layer (6) with a thickness of 20 nm, and lithium quinolate (Liq) (of which the structure shown below) was vapor-deposited as an electron injection layer (7) with a thickness of 1 to 2 nm. Thereafter, an Al cathode (8) was vapor-deposited with a thickness of 150 nm by using another vacuum vapor-deposit device to manufacture an OLED.

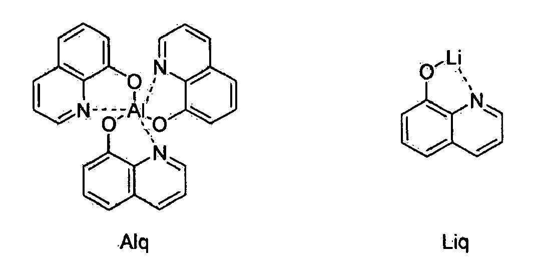

Each material employed for manufacturing an OLED was used as the electroluminescent material after purifying via vacuum sublimation under 10⁻⁶ torr.

### [Comparative Example 1] Manufacture of an OLED by using conventional electroluminescent material

After forming a hole injection layer (3) and hole transport layer (4) according to the same procedure described in Example 1, dinaphthylanthracene (DNA) was charged to one cell of said vacuum vapor-deposit device, and DSA-Ph (of which the structure is shown below) was charged to another cell. The two cells were heated at the same time to vapor-deposit DSA-Ph at a vapor-deposition rate of 2 to 5% by weight to form an electroluminescent layer (5) of 30 nm of thickness on the hole transport layer.

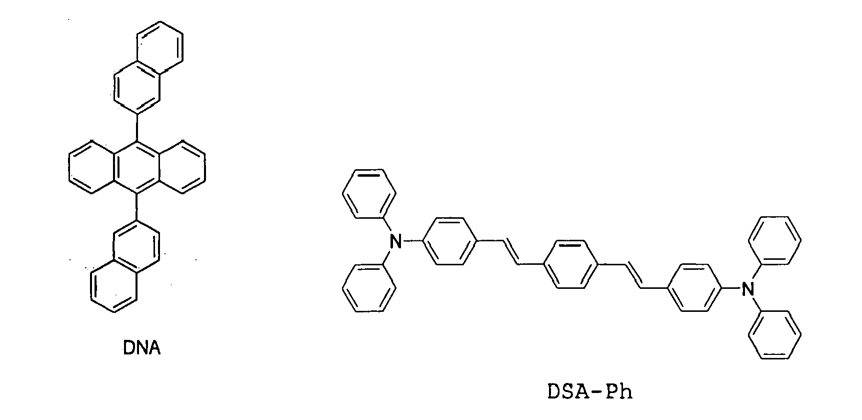

Then, an electron transport layer (6) and electron injection layer (7) were vapor-deposited according to the same procedure of Example 1, and an Al cathode (8) was vapor-deposited thereon with a thickness of 150 nm by using another vacuum vapor-deposit device to manufacture an OLED.

### [Comparative Example 2] Manufacture of an OLED by using conventional electroluminescent material

After forming a hole injection layer and hole transport layer according to the same procedure described in Example 1, tris(8-hydroxyquinoline)-aluminum (III) (Alq) was charged to another cell of said vacuum vapor-deposit device as an electroluminescent host material, while Coumarin 545T (C545T) was charged to still another cell. The two materials were evaporated at different rates to give doping, thereby vapor-depositing an electroluminescent layer with a thickness of 30 nm on the hole transport layer. The doping concentration preferably is from 1 to 3% by weight on the basis of Alq.

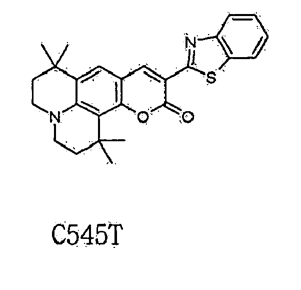

Then, an electron transport layer and an electron injection layer were vapor-deposited according to the same procedure of Example 1, and Al cathode was vapor-deposited by using another vacuum vapor-deposit device with a thickness of 150 nm, to manufacture an OLED.

### [Example 2] Electroluminescent properties of OLED's manufactured

The luminous efficiency of the OLED's comprising the organic EL compound according to the present invention (Examples 1) or conventional EL compound (Comparative Examples 1 and 2) were measured at 5,000 cd/m², and the results are shown in Table 4.

**Table 4**

| No. | Host | Dopant | Doping concentration (wt%) | Luminous efficiency (cd/A) | Color | Luminous efficiency/Y |
|---|---|---|---|---|---|---|
| | | | | @5000cd/m² | | |
| 1 | H-29 | Compound 143 | 3 | 7.2 | Blue | 51.2 |
| 2 | H-29 | Compound 270 | 3 | 7.0 | Blue | 50.2 |
| 3 | H-29 | Compound 2559 | 3 | 7.0 | Blue | 49.3 |
| 4 | H-33 | Compound 2661 | 3 | 6.9 | Blue | 48.8 |
| 5 | H-62 | Compound 2717 | 3 | 7.8 | Blue | 55.2 |
| 6 | H-5 | Compound 2499 | 3 | 20.1 | Green | - |
| 7 | H-5 | Compound 2515 | 3 | 19.8 | Green | - |
| 8 | H-29 | Compound 2765 | 3 | 21.5 | Green | - |
| 9 | H-29 | Compound 3136 | 3 | 21.3 | Green | - |
| 10 | H-29 | Compound 3995 | 3 | 19.8 | Green | - |
| 11 | H-62 | Compound 4127 | 3 | 18.2 | Green | - |
| 12 | H-62 | Compound 4206 | 3 | 20.9 | Green | - |
| Comp. 1 | DNA | DSA-Ph | 3 | 7.3 | Jade green | 35.8 |
| Comp. 2 | Alq | Compound C545T | 1 | 10.3 | Green | - |

As can be seen from Table 4, it is found that the blue OLED's employing the organic EL compounds according to the present invention exhibited comparable luminous efficiency, but far better color purity as compared to that of Comparative Example 1 employing DNA:DSA-Ph, so that the value "luminous efficiency/Y" (which has similar tendency to quantum efficiency) was much higher than that of the conventional material. Particularly, Compound (2717) showed the "luminous efficieny/Y" value enhanced by about 40% or more as compared to the conventional electroluminescent material.

Further, when the EL material according to the present invention was applied to an green electroluminescent device, the device showed the luminous efficiency by more than twice as compared to the device employing conventional Alq:C545T (Comparative Example 2), as can be seen from Table 4.

As shown above, the organic electroluminescent compounds according to the present invention can be employed as blue or green electroluminescent material of high efficiency. Furthermore, the electroluminescent devices employing the compounds as dopant material showed noticeable improvement in color purity. Those results of improvement in both color purity and luminous efficiency demonstrate advantageous properties of the EL materials according to the present invention.

## Claims

1. An organic electroluminescent compound represented by Chemical Formula (1) or Chemical Formula (2): 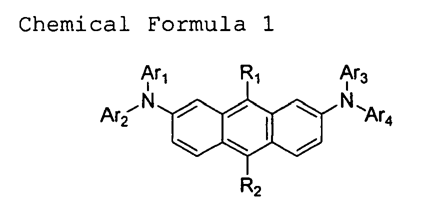 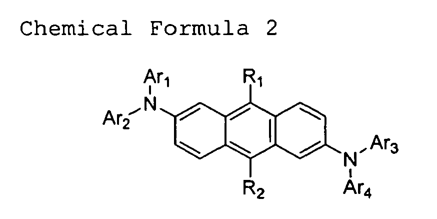 wherein,
R₁ and R₂ independently represent hydrogen, deuterium, (C1-C60)alkyl, (C2-C60)alkenyl, (C2-C60)alkynyl, (C3-C60)cycloalkyl, (C4-C60)tricycloalkyl, (C7-C60)bicycloalkyl, (C6-C60)aryl, (C4-C60)heteroaryl, 5- or 6-membered heterocycloalkyl containing one or more heteroatom(s) selected from N, O and S, spirobifluorenyl, halogen, cyano, (C1-C60)alkoxy, tri(C1-C60)alkylsilyl, di(C1-C60)alkyl(C6-C60)arylsilyl or tri(C6-C60)arylsilyl; and the alkyl, alkenyl, alkynyl, cycloalkyl, tricycloalkyl, bicycloalkyl, aryl or heteroaryl of R₁ and R₂ may be further substituted by one or more substituent(s) selected from deuterium, (C1-C60)alkyl, (C1-C60)alkoxy, halogen, tri(C1-C60)alkylsilyl, di(C1-C60)alkyl(C6-C60)arylsilyl, tri(C6-C60)arylsilyl, cyano, (C6-C60)aryl, (C6-C60)ar(C1-C60)alkyl and (C6-C60)ar(C1-C60)alkoxy; and
Ar₁ through Ar₄ independently represent 5- or 6-membered heteroaryl containing from 1 to 4 heteroatom(s) selected from N, O and S, provided that at least two of Ar₁, Ar₂, Ar₃ and Ar₄ represent pyridyl if the heteroaryl of Ar₁ through Ar₄ represents pyridyl.

2. The organic electroluminescent compound according to claim 1, wherein R₁ and R₂ independently represent methyl, ethyl, n-propyl, i-propyl, n-butyl, i-butyl, tert-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, benzyl, ethenyl, phenylethenyl, ethynyl, phenylethynyl, cyclopentyl, cyclohexyl, cycloheptyl, cyclooctyl, bicyclo[1.1.0]butyl, bicyclo[1.1.1]pentyl, bicyclo[2.1.0]pentyl, bicyclo[2.1.1]hexyl, bicyclo[3.2.0]hexyl, bicyclo[3.2.0]heptyl, bicyclo[3.1.1]heptyl, bicyclo[4.1.0]heptyl, bicyclo[2.2.1]heptyl, octahydropentalenyl, bicyclo[2.2.2]octyl, bicyclo[4.2.0]octyl, bicyclo[4.1.1]octyl, bicyclo[3.2.1]octyl, octahydro-1H-indenyl, bicyclo[5.2.0]nonyl, bicyclo[4.2.1]nonyl, bicyclo[3.3.1]nonyl, bicyclo[3.3.2]decyl, bicyclo[4.3.1]decyl, bicyclo[4.2.2]decyl, decahydronaphthalenyl, bicyclo[3.3.3]undecyl, bicyclo[4.3.2]undecyl, bicyclo[4.3.3]dodecyl, 4-pentylbicyclo[2.2.2]octyl, tricyclo[2.2.1.0]heptyl, tricyclo[5.2.1.0^{2,6}]decyl, tricyclo[5.3.1.1]dodecyl, tricyclo[5.4.0.0^{2,9}]undecyl, adamantyl, tricyclo[5.3.2.0^{4,9}]dodecyl, tricyclo[4.4.1.1^{1,5}]dodecyl, tricyclo[5.5.1.0^{3,11}]tridecyl, phenyl, naphthyl, biphenyl, indenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, tetrazolyl, pyrazinyl, pyrimidyl, quinolyl, benzofuranyl, benzothiophenyl, pyrazolyl, indolyl, carbazolyl, thiazolyl, oxazolyl, benzothiazolyl, benzoxazolyl, morpholino, thiomorpholino, spirobifluorenyl, fluoro, cyano, methoxy, ethoxy, n-propoxy, i-propoxy, n-butoxy, tert-butoxy, hexyloxy, trimethylsilyl, triethylsilyl, tripropylsilyl, tri(tert-butyl)silyl, tert-butyldimethylsilyl, dimethylphenylsilyl or triphenylsilyl; and
the phenyl, naphthyl, biphenyl, fluorenyl, phenanthryl, anthryl, fluoranthenyl, triphenylenyl, pyrenyl, chrysenyl, naphthacenyl, perylenyl, pyridyl, pyrrolyl, furanyl, thiophenyl, imidazolyl, benzimidazolyl, pyrazinyl, pyrimidyl, quinolyl, benzofuranyl, benzothiophenyl, pyrazolyl, indolyl, carbazolyl, thiazolyl, oxazolyl, benzothiazolyl or benzoxazolyl may be further substituted by one or more substituent(s) selected from methyl, ethyl, n-propyl, i-propyl, n-butyl, i-butyl, tert-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, fluoro, cyano, phenyl, naphthyl, 9,9-dimethyl-9H-fluorenyl, 9,9-diphenyl-9H-fluorenyl, anthryl, trimethylsilyl, triethylsilyl, tripropylsilyl, tri(tert-butyl)silyl, tert-butyldimethylsilyl, dimethylphenylsilyl, triphenylsilyl, triphenylmethyl and triphenylmethoxy.

3. The organic electroluminescent compound according to claim 2, wherein Ar₁ through Ar₄ independently represent 1-pyrrolyl, 2-pyrrolyl, 3-pyrrolyl, 1-pyrazolyl, 3-pyrazolyl, 4-pyrazolyl, 5-pyrazolyl, 1-imidazolyl, 2-imidazolyl, 4-imidazolyl, 5-imidazolyl, 2-thiazolyl, 4-thiazolyl, 5-thiazolyl, 3-isothiazolyl, 4-isothiazolyl, 5-isothiazolyl, 1,2,3-thiadiazol-4-yl, 1,2,3-thiadiazol-5-yl, 1,3,4-thiadiazol-2-yl, 1,3,5-thiadiazol-2-yl, 1,3,5-thiadiazol-4-yl, 2-oxazolyl, 4-oxazolyl, 5-oxazolyl, 3-isoxazolyl, 4-isoxazolyl, 5-isoxazolyl, 1,2,3-oxadiazol-4-yl, 1,2,3-oxadiazol-5-yl, 1,3,4-oxadiazol-2-yl, 1,3,5-oxadiazol-2-yl, 1,3,5-oxadiazol-4-yl, 2-furanyl, 3-furanyl, 3-furazanyl, 2-thienyl, 3-thienyl, 1H-tetrazol-5-yl, 1H-tetrazol-1-yl, 2H-tetrazol-5-yl, 2H-tetrazol-2-yl, 1H-1,2,3-triazol-1-yl, 1H-1,2,3-triazole-4-yl, 1H-1,2,3-triazol-5-yl, 1H-1,2,4-t-riazol-1-yl, 1H-1,2,4-triazol-3-yl, 1H-1,2,4-triazol-5-yl, 2H-1,2,3-triazol-2-yl, 2H-1,2,3-triazol-4-yl, 2H-1,2,3-triazol-5-yl, 2-pyridyl, 3-pyridyl, 4-pyridyl, 2-pyrimidinyl, 4-pyrimidinyl, 5-pyrimidinyl, 3-pyridazinyl, 4-pyridazinyl, 2-pyrazinyl, 1,2,3-triazin-4-yl, 1,2,3-triazin-5-yl, 1,2,4-triazin-3-yl, 1,2,4-triazin-5-yl, 1,2,4-triazin-6-yl, 1,3,5-triazin-2-yl, 1,2,3,4-tetrazin-5-yl and 1,2,3,5-tetrazin-4-yl.

4. An organic electroluminescent device which is comprised of a first electrode; a second electrode; and at least one organic layer(s) interposed between the first electrode and the second electrode; wherein the organic layer comprises an organic electroluminescent compound represented by Chemical Formula (1) or Chemical Formula (2): 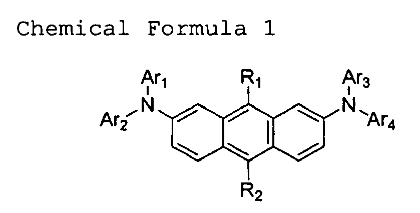 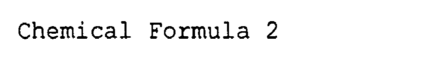 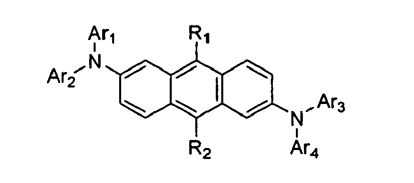 wherein,
R₁ and R₂ independently represent hydrogen, deuterium, (C1-C60)alkyl, (C2-C60)alkenyl, (C2-C60)alkynyl, (C3-C60)cycloalkyl, (C4-C60)tricycloalkyl, (C7-C60)bicycloalkyl, (C6-C60)aryl, (C4-C60)heteroaryl, 5- or 6-membered heterocycloalkyl containing one or more heteroatom(s) selected from N, O and S, spirobifluorenyl, halogen, cyano, (C1-C60)alkoxy, tri(C1-C60)alkylsilyl, di(C1-C60)alkyl(C6-C60)arylsilyl or tri(C6-C60)arylsilyl; and the alkyl, alkenyl, alkynyl, cycloalkyl, tricycloalkyl, bicycloalkyl, aryl or heteroaryl of R₁ and R₂ may be further substituted by one or more substituent(s) selected from deuterium, (C1-C60)alkyl, (C1-C60)alkoxy, halogen, tri(C1-C60)alkylsilyl, di(C1-C60)alkyl(C6-C60)arylsilyl, tri(C6-C60)arylsilyl, cyano, (C6-C60)aryl, (C6-C60)ar(C1-C60)alkyl and (C6-C60)ar(C1-C60)alkoxy; and Ar₁ through Ar₄ independently represent 5- or 6-membered heteroaryl containing from 1 to 4 heteroatom(s) selected from N, O and S, provided that at least two of Ar₁, Ar₂, Ar₃ and Ar₄ represent pyridyl if the heteroaryl of Ar₁ through Ar₄ represents pyridyl and one or more host(s) selected from the compounds represented by Chemical Formula (3) or (4):
Chemical Formula 3 (Ar₁₁)_{b}-L₁-(Ar₁₂)_{c}
Chemical Formula 4 (Ar₁₃)_{d}-L₂-(Ar₁₄)ₑ
wherein,
L₁ represents (C6-C60)arylene or (C4-C60)heteroarylene;
L₂ represents anthracenylene;
Ar₁₁ through Ar₁₉ are independently selected from hydrogen, deuterium, (C1-C60)alkyl, (C1-C60)alkoxy, halogen, (C4-C60)heteroaryl, (C5-C60)cycloalkyl and (C6-C60)aryl; and the cycloalkyl, aryl or heteroaryl of Ar₁₁ through Ar₁₄ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, deuterium, (C3-C60)cycloalkyl, halogen, cyano, tri(C1-C60)alkylsilyl, di(C1-C60)alkyl(C6-C60)arylsilyl and tri(C6-C60)arylsilyl; and b, c, d and e independently represent an integer from 0 to 4.

5. The organic electroluminescent device according to claim 4, wherein the organic layer comprises one or more compound(s) selected from a group consisting of arylamine compounds and styrylarylamine compounds.

6. The organic electroluminescent device according to claim 4, wherein the organic layer comprises one or more metal(s) selected from a group consisting of organic metals of Group 1, Group 2, 4^{th} period and 5^{th} period transition metals, lanthanide metals and d-transition elements from the Periodic Table of Elements.

7. The organic electroluminescent device according to claim 4, which is an organic electroluminescent display comprising a compound having the electroluminescent peak with wavelength of not more than 500 nm, and a compound having the electroluminescent peak with wavelength of not less than 560 nm.

8. The organic electroluminescent device according to claim 4, wherein the organic layer comprises an electroluminescent layer and a charge generating layer.

9. The organic electroluminescent device according to claim 4, wherein a mixed region of reductive dopant and organic substance, or a mixed region of oxidative dopant and organic substance is placed on the inner surface of one or both electrode(s) among the pair of electrodes.

10. An organic solar cell which comprises an organic electroluminescent compound represented by Chemical Formula
(1) or Chemical Formula (2): 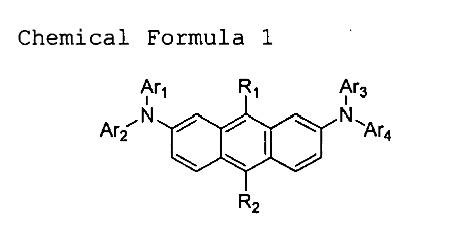 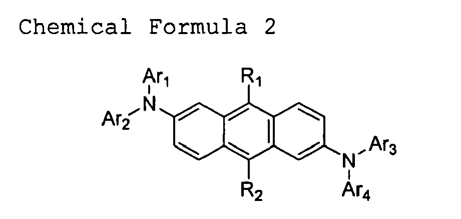
wherein,
R₁ and R₂ independently represent hydrogen, deuterium, (C1-C60)alkyl, (C2-C60)alkenyl, (C2-C60)alkynyl, (C3-C60)cycloalkyl, (C4-C60)tricycloalkyl, (C7-C60)bicycloalkyl, (C6-C60)aryl, (C4-C60)heteroaryl, 5- or 6-membered heterocycloalkyl containing one or more heteroatom(s) selected from N, O and S, spirobifluorenyl, halogen, cyano, (C1-C60)alkoxy, tri(C1-C60)alkylsilyl, di(C1-C60)alkyl(C6-C60)arylsilyl or tri(C6-C60)arylsilyl; and the alkyl, alkenyl, alkynyl, cycloalkyl, tricycloalkyl, bicycloalkyl, aryl or heteroaryl of R₁ and R₂ may be further substituted by one or more substituent(s) selected from deuterium, (C1-C60)alkyl, (C1-C60)alkoxy, halogen, tri(C1-C60)alkylsilyl, di(C1-C60)alkyl(C6-C60)arylsilyl, tri(C6-C60)arylsilyl, cyano, (C6-C60)aryl, (C6-C60)ar(C1-C60)alkyl and (C6-C60)ar(C1-C60)alkoxy; and Ar₁ through Ar₄ independently represent 5- or 6-membered heteroaryl containing from 1 to 4 heteroatom(s) selected from N, O and S, provided that at least two of Ar₁, Ar₂, Ar₃ and Ar₄ represent pyridyl if the heteroaryl of Ar₁ through Ar₄ represents pyridyl.
